(19)

**Europäisches Patentamt**

**European Patent Office**

**Office européen des brevets**

(11)  **EP 2 540 831 A2**

(12) **EUROPEAN PATENT APPLICATION**

(43) Date of publication:
**02.01.2013 Bulletin 2013/01**

(51) Int Cl.:
*C12N 15/82* <sup>(2006.01)</sup>     *A01H 5/00* <sup>(2006.01)</sup>

(21) Application number: **12178951.5**

(22) Date of filing: **17.08.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **17.08.2006 US 838415 P**

(62) Document number(s) of the earlier application(s) in accordance with Art. 76 EPC:
**07837048.3 / 2 048 939**

(71) Applicant: **Monsanto Technology, LLC
St Louis MO 63167 (US)**

(72) Inventors:
 • **Abad, Mark**
  **St. Louis, MO 63167 (US)**
 • **Adams, Tom R.**
  **St. Louis, MO 63167 (US)**
 • **Alavarez, Julie**
  **St. Louis, MO 63167 (US)**
 • **Anuradha, Mahindra**
  **St. Louis, MO 63167 (US)**
 • **Augustine, Alice Clara**
  **St. Louis, MO 63167 (US)**
 • **Badami, Pranesh**
  **St. Louis, MO 63167 (US)**
 • **Belle, Erin**
  **St. Louis, MO 63167 (US)**
 • **Bennett, Kristen**
  **St. Louis, MO 63167 (US)**
 • **Bensen, Robert**
  **St. Louis, MO 63167 (US)**
 • **Castiglioni, Paolo**
  **St. Louis, MO 63167 (US)**
 • **Cerny, R. Eric**
  **St. Louis, MO 63167 (US)**
 • **Chen, Xianfeng**
  **St. Louis, MO 63167 (US)**
 • **Chittoor, Jaishree**
  **St. Louis, MO 63167 (US)**
 • **Deeba, Farah**
  **St. Louis, MO 63167 (US)**
 • **Deikman, Jill**
  **St. Louis, MO 63167 (US)**
 • **Deng, Molian**
  **St. Louis, MO 63167 (US)**
 • **Duff, Stephen**
  **St. Louis, MO 63167 (US)**
 • **Fabri, Brandon**
  **St. Louis, MO 63167 (US)**
 • **Fenner, Jason**
  **St. Louis, MO 63167 (US)**
 • **Fernandes, Mary**
  **St. Louis, MO 63167 (US)**
 • **Gabbert, Karen**
  **St. Louis, MO 63167 (US)**
 • **Galligan, Megan**
  **St. Louis, MO 63167 (US)**
 • **Goldman, Barry**
  **St. Louis, MO 63167 (US)**
 • **Hawkins, Debbie**
  **St. Louis, MO 63167 (US)**
 • **Heard, Jacqueline**
  **St. Louis, MO 63167 (US)**
 • **Karunanandaa, Bala**
  **St. Louis, MO 63167 (US)**
 • **Ke, Dangyang**
  **St. Louis, MO 63167 (US)**
 • **Ledeaux, John**
  **St. Louis, MO 63167 (US)**
 • **Lee, Gary**
  **St. Louis, MO 63167 (US)**
 • **Madappa, Savitha**
  **St. Louis, MO 63167 (US)**
 • **Nelson, Donald**
  **St. Louis, MO 63167 (US)**
 • **Patty, Obed**
  **St. Louis, MO 63167 (US)**
 • **Qi, Qungang**
  **St. Louis, MO 63167 (US)**
 • **Rajani, M.S.**
  **St. Louis, MO 63167 (US)**
 • **Ramachandra, Dhanalakshmi**
  **St. Louis, MO 63167 (US)**
 • **Ramamohan, G.**
  **St. Louis, MO 63167 (US)**
 • **Ruff, Thomas**
  **St. Louis, MO 63167 (US)**
 • **Sanders, Rick**
  **St. Louis, MO 63167 (US)**
 • **Sangeetha, S.**
  **St. Louis, MO 63167 (US)**
 • **Savage, Thomas J.**
  **St. Louis, MO 63167 (US)**
 • **Savidge, Beth**
  **St. Louis, MO 63167 (US)**

**(Cont. next page)**

EP 2 540 831 A2

- **Shobha, Char**
  St. Louis, MO 63167 (US)
- **Sudarshana, Padmini**
  St. Louis, MO 63167 (US)
- **Suma, S. Navarathna**
  St. Louis, MO 63167 (US)
- **Sun, Jindong**
  St. Louis, MO 63167 (US)
- **Thompson, Rebecca**
  St. Louis, MO 63167 (US)
- **Val, Dale**
  St. Louis, MO 63167 (US)
- **Venkatachalayya, Srikantha**
  St. Louis, MO 63167 (US)
- **Venkatesh, T.**
  St. Louis, MO 63167 (US)

(74) Representative: **von Kreisler Selting Werner**
**Deichmannhaus am Dom**
**Bahnhofsvorplatz 1**
**50667 Köln (DE)**

Remarks:
•The complete document including Reference Tables and the Sequence Listing can be downloaded from the EPO website
•This application was filed on 02-08-2012 as a divisional application to the application mentioned under INID code 62.
•Claims filed after the date of filing of the application/after the date of receipt of the divisional application (Rule 68(4) EPC).

(54)     **Transgenic plants with enhanced agronomic traits**

(57)     This invention provides transgenic plant cells with recombinant DNA for expression of proteins that are useful for imparting enhanced agronomic trait(s) to transgenic crop plants. This invention also provides transgenic plants and progeny seed comprising the transgenic plant cells where the plants are selected for having an enhanced trait selected from the group of traits consisting of enhanced water use efficiency, enhanced cold tolerance, increased yield, enhanced nitrogen use efficiency, enhanced seed protein and enhanced seed oil. Also disclosed are methods for manufacturing transgenic seed and plants with enhanced traits.

**Description**

**Cross Reference to Related Applications**

This application claims benefit under 35USC § 119(e) of United States provisional application Serial No. 60/838,415, filed 08/17/2006, herein incorporated by reference.

**Incorporation Of Sequence Listing**

Two copies of the sequence listing (Copy I and Copy 2) and a computer readable form (CRF) of the sequence listing, all on CD-Rs, each containing the text file named 38-21(54146)B_seqListing.txt, which is 103,067,648 bytes (measured in MS-WINDOWS), were created on August 16, 2007 and are herein incorporated by reference.

**Incorporation Of Table**

Two copies of Table 9 (Copy 1 and Copy 2) and a computer readable form (CRF), all on CD-Rs, each containing the file named 38-21(54146)B_table9.txt, which is 319,488 bytes (measured in MS-WINDOWS), were created on August 16, 2007, and comprise 74 pages when viewed in MS Word, are herein incorporated by reference.

**Field Of The Invention**

Disclosed herein are inventions in the field of plant genetics and developmental biology. More specifically, the present inventions provide plant cells with recombinant DNA for providing an enhanced trait in a transgenic plant, plants comprising such cells, seed and pollen derived from such plants, methods of making and using such cells, plants, seeds and pollen.

**Background Of The Invention**

Transgenic plants with improved agronomic traits such as yield, environmental stress tolerance, pest resistance, herbicide tolerance, improved seed compositions, and the like are desired by both farmers and consumers. Although considerable efforts in plant breeding have provided significant gains in desired traits, the ability to introduce specific DNA into plant genomes provides further opportunities for generation of plants with improved and/or unique traits. Merely introducing recombinant DNA into a plant genome doesn't always produce a transgenic plant with an enhanced agronomic trait. Methods to select individual transgenic events from a population are required to identify those transgenic events that are characterized by the enhanced agronomic trait.

**Summary Of The Invention**

This invention provides plant cell nuclei with recombinant DNA that imparts enhanced agronomic traits in transgenic plants having the nuclei in their cells, e.g. enhanced water use efficiency, enhanced cold tolerance, increased yield, enhanced nitrogen use efficiency, enhanced seed protein or enhanced seed oil. Such recombinant DNA in a plant cell nuclus of this invention is provided in as a construct comprising a promoter that is functional in plant cells and that is operably linked to DNA that encodes a protein. Such DNA in the construct is sometimes defined by protein domains of an encoded protein targeted for production or suppression., e.g. a "Pfam domain module" (as defined herein below) from the group of Pfam domain modules identified in Table 9. Alternatively, e.g. where a Pfam domain module is not available, such DNA in the construct is defined a consensus amino acid sequence of an encoded protein that is targeted for production e.g. a protein having amino acid sequence with at least 90% identity to a consensus amino acid sequence in the group of SEQ ID NO: 30328, and SEQ ID NO: 30377 through SEQ ID NO: 30418. Alternatively, in other cases where neither a Pfam domain module nor a consensus amino acid sequence is available, such DNA in the construct is defined by the sequence of a specific encoded and/or its homologous proteins.

Other aspects of the invention are specifically directed to transgenic plant cells comprising the recombinant DNA ofthe invention, transgenic plants comprising a plurality of such plant cells, progeny transgenic seed, embryo and transgenic pollen from such plants. Such plant cells are selected from a population of transgenic plants regenerated from plant cells transformed with recombinant DNA and that express the protein by screening transgenic plants in the population for an enhanced trait as compared to control plants that do not have said recombinant DNA, where the enhanced trait is selected from group of enhanced traits consisting of enhanced water use efficiency, enhanced cold tolerance, increased yield, enhanced nitrogen use efficiency, enhanced seed protein and enhanced seed oil.

In yet another aspect of the invention the plant cells, plants, seeds, embryo and pollen further comprise DNA expressing a protein that provides tolerance from exposure to an herbicide applied at levels that are lethal to a wild type of said plant cell. Such tolerance is especially useful not only as an advantageous trait in such plants but is also useful in a selection step in the methods of the invention. In aspects of the invention the agent of such herbicide is a glyphosate, dicamba, or glufosinate compound.

Yet other aspects of the invention provide transgenic plants which are homozygous for the recombinant DNA and transgenic seed of the invention from corn, soybean, cotton, canola, alfalfa, wheat or rice plants.

This invention also provides methods for manufacturing non-natural, transgenic seed that can be used to produce a crop of transgenic plants with an enhanced trait resulting from expression of stably-integrated, recombinant DNA in the nucleus of the plant cells. More specifically the method comprises (a) screening a population of plants for an enhanced trait and recombinant DNA, where individual plants in the population can exhibit the trait at a level less than, essentially the same as or greater than the level that the trait is exhibited in control plants which do not express the recombinant DNA; (b) selecting from the population one or more plants that exhibit the trait at a level greater than the level that said trait is exhibited in control plants and (c) collecting seed from a selected plant. Such method further comprises steps (d) verifying that the recombinant DNA is stably integrated in said selected plants; and (e) analyzing tissue of a selected plant to determine the production of a protein having the function of a protein encoded by a recombinant DNA with a sequence of one of SEQ ID NO: 1-358; In one aspect of the invention the plants in the population further comprise DNA expressing a protein that provides tolerance to exposure to an herbicide applied at levels that are lethal to wild type plant cells and where the selecting is effected by treating the population with the herbicide, e.g. a glyphosate, dicamba, or glufosinate compound. In another aspect of the invention the transgenic plants are selected by identifying plants with the enhanced trait. The methods are especially useful for manufacturing corn, soybean, cotton, alfalfa, wheat or rice seed selected as having one of the enhanced traits described above.

Another aspect of the invention provides a method of producing hybrid corn seed comprising acquiring hybrid corn seed from a herbicide tolerant corn plant which also has stably-integrated, recombinant DNA comprising a promoter that is (a) functional in plant cells and (b) is operably linked to DNA that encodes a protein. Such protein is defined by protein domains of an encoded protein targeted for production or suppression., e.g. a "Pfam domain module" (as defined herein below) from the group of Pfam domain modules identified in Table 9. Alternatively, e.g. where a Pfam domain module is not available, such protein is defined by a consensus amino acid sequence of an encoded protein that is targeted for production e.g. a protein having amino acid sequence with at least 90% identity to a consensus amino acid sequence in the group of SEQ ID NO: 30328, and SEQ ID NO: 30377 through SEQ ID NO: 30418. Alternatively, in other cases where neither a Pfam domain module nor a consensus amino acid sequence is available, such DNA in the construct is defined by the sequence of a specific encoded and/or its homologous proteins. The methods further comprise producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA; selecting corn plants which are homozygous and hemizygous for said recombinant DNA by treating with an herbicide; collecting seed from herbicide-treated-surviving corn plants and planting said seed to produce further progeny corn plants; repeating the selecting and collecting steps at least once to produce an inbred corn line; and crossing the inbred corn line with a second corn line to produce hybrid seed.

**Brief Description Of The Drawings**

Figure 1 is a consensus amino acid sequence of SEQ ID NO: 561 and its homologs.

Figures 2-5 are plasmid maps.

**Detailed Description Of The Invention**

In the attached sequence listing:

SEQ ID NO:1-358 are nucleotide sequences of the coding strand of DNA for "genes" used in the recombinant DNA imparting an enhanced trait in plant cells, i.e. each represents a coding sequence for a protein;

SEQ ID NO: 359-716 are amino acid sequences of the cognate protein of the "genes" with nucleotide coding sequences 1-358;

SEQ ID NO: 717-30327 are amino acid sequences of homologous proteins;

SEQ ID NO: 30328 is a consensus sequence of SEQ ID NO: 561 and its homologs;

SEQ ID NO: 30329 is the nucleotide sequence of a plasmid base vector pMON93039 useful for corn transformation;

SEQ ID NO: 30330 is the nucleotide sequence of a plasmid base vector pMON92705 useful for corn transformation;

SEQ ID NO: 30331 is the nucleotide sequence of a plasmid base vector pMON82053 useful for soybean and canola transformation;

SEQ ID NO: 30332-30375 are nucleotide sequences of the regulatory elements in base vectors;

SEQ ID NO: 30376 is the nucleotide sequence of a plasmid base vector pMON99053 useful for cotton transformation; and

SEQ ID NO: 30377-30418 are consensus sequences.

Table 1 lists the protein SEQ ID Nos and their corresponding consensus SEQ ID Nos.

Table 1

| PEP SEQ ID NO | Gene ID | Consensus SEQ ID NO |
|---|---|---|
| 371 | PHE0002860 7494 | 30377 |
| 372 | PHE0002860 8694 | 30378 |
| 378 | PHE0004013 9281 | 30379 |
| 401 | PHE0004780 5752 | 30380 |
| 402 | PHE0004782 5754 | 30381 |
| 420 | PHE0004859 5896 | 30382 |
| 421 | PHE0004859 5917 | 30383 |
| 427 | PHE0004889 7961 | 30384 |
| 436 | PHE0004903 5960 | 30385 |
| 446 | PHE0004948 6003 | 30386 |
| 470 | PHE0006047 7234 | 30387 |
| 471 | PHE0006047 8766 | 30388 |
| 474 | PHE0006049 7107 | 30389 |
| 480 | PHE0006062 7058 | 30390 |
| 485 | PHE0006072 7071 | 30391 |
| 486 | PHE0006074 7060 | 30392 |
| 487 | PHE0006076 7052 | 30393 |
| 488 | PHE0006076 7331 | 30394 |
| 514 | PHE0006176 7147 | 30395 |
| 544 | PHE0006286 7314 | 30396 |
| 545 | PHE0006286 8011 | 30397 |
| 546 | PHE0006288 7310 | 30398 |
| 547 | PHE0006288 8023 | 30399 |
| 558 | PHE0006346 8132 | 30400 |
| 561 | PHE0006351 8200 | 30328 |
| 562 | PHE0006353 8098 | 30401 |
| 563 | PHE0006355 8084 | 30402 |
| 567 | PHE0006378 7667 | 30403 |
| 568 | PHE0006378 8715 | 30404 |
| 615 | PHE0006593 8245 | 30405 |
| 616 | PHE0006593 8256 | 30406 |
| 654 | PHE0006740 8446 | 30407 |

(continued)

| PEP SEQ ID NO | Gene ID | Consensus SEQ ID NO |
|---|---|---|
| 655 | PHE0006740 8596 | 30408 |
| 679 | PHE0006616 8560 | 30409 |
| 680 | PHE0006844 8839 | 30410 |
| 683 | PHE0006908 9016 | 30411 |
| 699 | PHE0006941 9117 | 30412 |
| 707 | PHE0006954 9154 | 30413 |
| 708 | PHE0006954 9161 | 30414 |
| 712 | PHE0006970 9141 | 30415 |
| 714 | PHE0006986 9183 | 30416 |
| 715 | PHE0006992 9140 | 30417 |
| 716 | PHE0006992 9184 | 30418 |

As used herein a "plant cell" means a plant cell that is transformed with stably-integrated, non-natural, recombinant DNA, e.g. by *Agrobacterium*-mediated transformation or by baombardment using microparticles coated with recombinant DNA or other means. A plant cell of this invention can be an originally-transformed plant cell that exists as a microorganism or as a progeny plant cell that is regenerated into differentiated tissue, e.g. into a transgenic plant with stably-integrated, non-natural recombinant DNA, or seed or pollen derived from a progeny transgenic plant.

As used herein a "transgenic plant" means a plant whose genome has been altered by the stable integration of recombinant DNA. A transgenic plant includes a plant regenerated from an originally-transformed plant cell and progeny transgenic plants from later generations or crosses of a transformed plant.

As used herein "recombinant DNA" means DNA which has been a genetically engineered and constructed outside of a cell including DNA containing naturally occurring DNA or cDNA or synthetic DNA.

As used herein "consensus sequence" means an artificial sequence of amino acids in a conserved region of an alignment of amino acid sequences of homologous proteins, e.g. as determined by a CLUSTALW alignment of amino acid sequence of homolog proteins.

As used herein "homolog" means a protein in a group of proteins that perform the same biological function, e.g. proteins that belong to the same Pfam protein family and that provide a common enhanced trait in transgenic plants of this invention. Homologs are expressed by homologous genes. Homologous genes include naturally occurring alleles and artificially-created variants. Degeneracy of the genetic code provides the possibility to substitute at least one base of the protein encoding sequence of a gene with a different base without causing the amino acid sequence ofthe polypeptide produced from the gene to be changed. Hence, a polynucleotide useful in the present invention may have any base sequence that has been changed from SEQ ID NO:1 through SEQ ID NO: 358 substitution in accordance with degeneracy of the genetic code. Homologs are proteins that, when optimally aligned, have at least 60% identity, more preferably about 70% or higher, more preferably at least 80% and even more preferably at least 90% identity over the full length of a protein identified as being associated with imparting an enhanced trait when expressed in plant cells. Homologs include proteins with an amino acid sequence that has at least 90% identity to a consensus amino acid sequence of proteins and homologs disclosed herein.

Homologs are be identified by comparison of amino acid sequence, e.g. manually or by use of a computer-based tool using known homology-based search algorithms such as those commonly known and referred to as BLAST, FASTA, and Smith-Waterman. A local sequence alignment program, e.g. BLAST, can be used to search a database of sequences to find similar sequences, and the summary Expectation value (E-value) used to measure the sequence base similarity. As a protein hit with the best E-value for a particular organism may not necessarily be an ortholog or the only ortholog, a reciprocal query is used in the present invention to filter hit sequences with significant E-values for ortholog identification. The reciprocal query entails search of the significant hits against a database of amino acid sequences from the base organism that are similar to the sequence of the query protein. A hit is a likely ortholog, when the reciprocal query's best hit is the query protein itself or a protein encoded by a duplicated gene after speciation. A further aspect of the invention comprises functional homolog proteins that differ in one or more amino acids from those of disclosed protein as the result of conservative amino acid substitutions, for example substitutions are among: acidic (negatively charged) amino acids such as aspartic acid and glutamic acid; basic (positively charged) amino acids such as arginine, histidine, and lysine; neutral polar amino acids such as glycine, serine, threonine, cysteine, tyrosine, asparagine, and glutamine; neutral nonpolar (hydrophobic) amino acids such as alanine, leucine, isoleucine, valine, proline, phenylalanine, tryptophan, and methionine; amino acids having aliphatic side chains such as glycine, alanine, valine, leucine, and isoleucine; amino acids having aliphatic-hydroxyl side chains such as serine and threonine; amino acids having amide-containing side chains such as asparagine and glutamine; amino acids.having aromatic side chains such as phenylalanine, tyrosine, and tryptophan; amino acids having basic side chains such as lysine, arginine, and histidine; amino acids having sulfur-containing side chains such as cysteine and methionine; naturally conservative amino acids such as valine-leucine, valine-isoleucine, phenylalanine-tyrosine, lysine-arginine, alanine-valine, aspartic acid-glutamic acid, and asparagine-glutamine. A further aspect of the homologs encoded by DNA useful in the transgenic plants of the invention are those proteins that differ from a disclosed protein as the result of deletion or insertion of one or more amino acids in a native sequence.

As used herein, "percent identity" means the extent to which two optimally aligned DNA or protein segments are invariant throughout a window of alignment of components, for example nucleotide sequence or amino acid sequence. An "identity fraction" for aligned segments of a test sequence and a reference sequence is the number of identical components that are shared by sequences of the two aligned segments divided by the total number of sequence components in the reference segment over a window of alignment which is the smaller of the full test sequence or the full reference sequence. "Percent identity" ("% identity") is the identity fraction times 100.

The "Pfam" database is a large collection of multiple sequence alignments and hidden Markov models covering many common protein families, e.g. Pfam version 19.0 (December 2005) contains alignments and models for 8183 protein families and is based on the Swissprot 47.0 and SP-TrEMBL 30.0 protein sequence databases. See S.R. Eddy, "Profile Hidden Markov Models", Bioinformatics 14:755-763, 1998. The Pfam database is currently maintained and updated by the Pfam Consortium. The alignments represent some evolutionary conserved structure that has implications for the protein's function. Profile hidden Markov models (profile HMMs) built from the protein family alignments are useful for automatically recognizing that a new protein belongs to an existing protein family even if the homology by alignment appears to be low.

A "Pfam domain module" is a representation of Pfam domains in a protein, in order from N terminus to C terminus. In a Pfam domain module individual Pfam domains are separated by double colons "::". The order and copy number of the Pfam domains from N to C terminus are attributes of a Pfam domain module. Although the copy number of repetitive domains is important, varying copy number often enables a similar function. Thus, a Pfam domain module with multiple copies of a domain should define an equivalent Pfam domain module with variance in the number of multiple copies. A Pfam domain module is not specific for distance between adjacent domains, but contemplates natural distances and variations in distance that provide equivalent funtion. The Pfam database contains both narrowly- and broadly-defined domains, leading to identification of overlapping domains on some proteins. A Pfam domain module is characterized by non-overlapping domains. Where there is overlap, the domain having a function that is more closely associated with the function of the protein (based on the E value of the Pfam match) is selected.

Once one DNA is identified as encoding a protein which imparts an enhanced trait when expressed in transgenic plants, other DNA encoding proteins with the same Pfam domain module are identified by querying the amino acid sequence of protein encoded by candidate DNA against the Hidden Markov Models which characterizes the Pfam domains using HMMER software, publically available through Pfam consortium. Candidate proteins meeting the same Pfam domain module are in the protein family and have cognate DNA that is useful in constructing recombinant DNA for the use in the plant cells of this invention. Hidden Markov Model databases for use with HMMER software in identifying DNA expressing protein with a common Pfam domain module for recombinant DNA in the plant cells of this invention are also available through Pfam Consortium.

Version 19.0 of the HMMER software and Pfam databases were used to identify known domains in the proteins corresponding to amino acid sequence of SEQ ID NO: 359 through SEQ ID NO: 716. All DNA encoding proteins that have scores higher than the gathering cutoff disclosed in Table 16 by Pfam analysis disclosed herein can be used in recombinant DNA of the plant cells of this invention, e.g. for selecting transgenic plants having enhanced agronomic traits. The relevant Pfams modules for use in this invention, as more specifically disclosed below, are Gp_dh_N::Gp_dh_C, Mg_chelatase::VWA, zf-CCCH::zf-CCCH::zf-CCCH::zf-CCCH::zf-CCCH, WD40, tRNA-synt_2b::HGTP_anticodon, RNase_PH::RNase_PH_C, F-box::Kelch_1::Kelch_1, Peptidase_C54, Iso_dh, Metallophos, OTU, Rotamase, Sugar_tr, Glyoxalase::Glyoxalase, Ras, Brix, S6PP::S6PP_C, PsbR, Pkinase, p450, PP2C, CH::EB1, DUF537, Histone, PPR::PPR::PPR::PPR::PPR, TFIIS_M::TFIIS_C, DUF751, RRM_1::RRM_1, ETC_C1_NDUFA4, SRF-TF, CCT, Globin::FAD_binding_6::NAD_binding_1, FAE1_CUT1_RppA::ACP_syn_III_C, Frataxin_Cyay, F-box::LRR_2, Tryp_alpha_amyl, PFK::PFK, Dehydrin, RLI::Fer4::ABC_tran::ABC_tran, CTP_transf_2, GTP_EFTU::GTP_EFTU_D2::GTP_EFTU_D3, PfkB, IPT, TPR_1::TPR_2::TPR_1::TPR_2::TPR_1::TPR_1::TPR_1::TPR_1::TPR_1, Globin, Porphobil_deam::Porphobil_deamC, NB-ARC::LRR_1::LRR_1::LRR_1, Bromodomain, DUF1365, PTS_2-RNA, Pkinase::UBA::KA1, MATH::BTB, DUF6::TPT, Cyctin_N::Cyctin_C, zf-AN1, Methyltransf_6, Thioredoxin, DNA_photolyase::FAD_binding_7, vATP-synt_E, Bac_globin, B_lectin::S_locus_glycop::PAN_2::Pkinase_Tyr, Sigma70_r2::Sigma70_r3::Sigma70_r4, Ribosomal_L10, zf-C3HC4::WD40::WD40::WD40, PGM_PMM_I::PGM_PMM_II::PGM_PMM_III::PGM_PMM_IV, Hydrolase, Peptidase_C1, DS, Carotene_hydrox, Aa_trans, Mov34, zf-MYND::UCH, Heme_oxygenase, S6PP, SSB, Peptidase_M16::Peptidase_M16_C, Bet_v_I, Auxin_inducible, Response_reg, Di19, DUF125, GDC-P, Pyr_redox_2:: Fer2_BFD::NIR_SIR_ferr::NIR_SIR, KOW::eIF-5a, MtN3_slv::MtN3_slv, Ribul_P_3_epim, NPH3, DnaJ::DnaJ_C, UQ_ con, RRM_1::RRM_1::RRM_1, F-box, CoA_binding::Ligase_CoA, adh_short, Ribosomal_L22, AA_permease, Acyltransferase, AMPKBI, RRM_1, Chalcone, GATase_2::Asn_synthase, Peptidase_M24. DUF498, DAGAT, PFK, DUF1677, Glyco_transf_43, zf-DNL, DHBP_synthase::GTP_cyclohydro2, PseudoU_synth_2, Glyoxalase, DUF21:: CBS, Ribosomal_S30AE, Glycolytic, Chloroa_b-bind, ZF-HD_dimer, Usp, Ferrochelatase, Pyridoxal_deC, Glyco_ transf_8, Pyr_redox_2::Glutaredoxin, Epimerase, UPF0113, RNase_PH, AIG1, Phi_1, CorA, HD::RelA-SpoT, P-II, GSHPx, PGAM, PGI, DUF868, Lung_7-TM_R, F-box::FBA_1, TPP_enzyme_N::TPP_enzyme_M::TPP_enzyme_C, DnaJ:: zf-CSL, DEAD::Helicase_C, 20G-FeII_Oxy, HMGL-like::LeuA_dimer, VQ, DUF298, DREPP, ketoacyl-synt::Ketoacyl-synt_C, THF_DHG_CYH::THF_DHG_CYH_C, DNA_pol_E_B, UPF0051, Pkinase;;efhand::efhand::efhand::efhand, malic::Malic_M, ThiF, Transket_pyr::Transketolase_C, Ribosomal_L37ae, PEPcase, Glyco_hydro_32N::Glyco_hydro_ 32C, GASA, DnaJ, AA_kinase::ACT::ACT, Pkinase_Tyr, Cupin_1, zf-LSD1::zf-LSD1::zf-LSD1, Cupin_3, GAF::HisKA:: HATPase_c::Response reg, Methyltransf_12::Mg-por_mtran_C, DUF516, PTR2, Ammonium_transp, eIF-5a, ECH, Aldedh, zf-C3HC4, SAM_decarbox, X8, Mg_chelatase, PurA, Ribosomal_S6e, Molybdop_Fe4S4::Molybdopterin:: Molydop_binding, CP12, Biotin_lipoyl::E3_binding::2-oxoacid_dh, NOI, Tubulin::Tubulin_C, V-SNARE, AP2, ELFV_ dehydrog_N::ELFV_dehydrog, Ribosomal_L32e, and FAD_binding_3.

As used herein "promoter" means regulatory DNA for initializing transcription. A "plant promoter" is a promoter capable of initiating transcription in plant cells whether or not its origin is a plant cell, e.g. is it well known that *Agrobacterium* promoters are functional in plant cells. Thus, plant promoters include promoter DNA obtained from plants, plant viruses and bacteria such as *Agrobacterium* and *Bradyrhizobium* bacteria. Examples of promoters under developmental control include promoters that preferentially initiate transcription in certain tissues, such as leaves, roots, or seeds. Such promoters are referred to as "tissue preferred". Promoters that initiate transcription only in certain tissues are referred to as "tissue specific". A "cell type" specific promoter primarily drives expression in certain cell types in one or more organs, for example, vascular cells in roots or leaves. An "inducible" or "repressible" promoter is a promoter which is under environmental control. Examples of environmental conditions that may effect transcription by inducible promoters include anaerobic conditions, or certain chemicals, or the presence of light. Tissue specific, tissue preferred, cell type specific, and inducible promoters constitute the class of "non-constitutive" promoters. A "constitutive" promoter is a promoter which is active under most conditions.

As used herein "operably linked" means the association of two or more DNA fragments in a DNA construct so that the function of one, e.g. protein-encoding DNA, is controlled by the other, e.g. a promoter.

As used herein "expressed" means produced, e.g. a protein is expressed in a plant cell when its cognate DNA is transcribed to mRNA that is translated to the protein.

As used herein a "control plant" means a plant that does not contain the recombinant DNA that expressed a protein that impart an enhanced trait. A control plant is to identify and select a transgenic plant that has an enhance trait. A suitable control plant can be a nontransgenic plant of the parental line used to generate a transgenic plant, i.e. devoid of recombinant DNA. A suitable control plant may in some cases be a progeny of a hemizygous transgenic plant line that is does not contain the recombinant DNA, known as a negative segregant.

As used herein an "enhanced trait" means a characteristic of a transgenic plant that includes, but is not limited to, an enhance agronomic trait characterized by enhanced plant morphology, physiology, growth and development, yield, nutritional enhancement, disease or pest resistance, or environmental or chemical tolerance. In more specific aspects of this invention enhanced trait is selected from group of enhanced traits consisting of enhanced water use efficiency, enhanced cold tolerance, increased yield, enhanced nitrogen use efficiency, enhanced seed protein and enhanced seed oil. In an important aspect of the invention the enhanced trait is enhanced yield including increased yield under non-stress conditions and increased yield under environmental stress conditions. Stress conditions may include, for example, drought, shade, fungal disease, viral disease, bacterial disease, insect infestation, nematode infestation, cold temperature exposure, heat exposure, osmotic stress, reduced nitrogen nutrient availability, reduced phosphorus nutrient availability and high plant density. "Yield" can be affected by many properties including without limitation, plant height, pod number, pod position on the plant, number of internodes, incidence of pod shatter, grain size, efficiency of nodulation and nitrogen fixation, efficiency of nutrient assimilation, resistance to biotic and abiotic stress, carbon assimilation, plant architecture, resistance to lodging, percent seed germination, seedling vigor, and juvenile traits. Yield can also affected by efficiency of germination (including germination in stressed conditions), growth rate (including growth rate in stressed conditions), ear number, seed number per ear, seed size, composition of seed (starch, oil, protein) and characteristics of seed fill.

Increased yield of a transgenic plant of the present invention can be measured in a number of ways, including test weight, seed number per plant, seed weight, seed number per unit area (i.e. seeds, or weight of seeds, per acre), bushels per acre, tonnes per acre, tons per acre, kilo per hectare. For example, maize yield may be measured as production of shelled corn kernels per unit of production area, for example in bushels per acre or metric tons per hectare, often reported on a moisture adjusted basis, for example at 15.5 percent moisture. Increased yield may result from improved utilization of key biochemical compounds, such as nitrogen, phosphorous and carbohydrate, or from improved responses to environmental stresses, such as cold, heat, drought, salt, and attack by pests or pathogens. Recombinant DNA used in this invention can also be used to provide plants having improved growth and development, and ultimately increased yield, as the result of modified expression of plant growth regulators or modification of cell cycle or photosynthesis pathways. Also of interest is the generation of transgenic plants that demonstrate enhanced yield with respect to a seed component that may or may not correspond to an increase in overall plant yield. Such properties include enhancements in seed oil, seed molecules such as tocopherol, protein and starch, or oil particular oil components as may be manifest by an alterations in the ratios of seed components.

A subset of the nucleic molecules of this invention includes fragments of the disclosed recombinant DNA consisting of oligonucleotides of at least 15, preferably at least 16 or 17, more preferably at least 18 or 19, and even more preferably at least 20 or more, consecutive nucleotides. Such oligonucleotides are fragments of the larger molecules having a sequence selected from the group consisting of SEQ ID NO:1 through SEQ ID NO: 358, and find use, for example as probes and primers for detection of the polynucleotides of the present invention.

DNA constructs are assembled using methods well known to persons of ordinary skill in the art and typically comprise a promoter operably linked to DNA, the expression of which provides the enhanced agronomic trait. Other construct components may include additional regulatory elements, such as 5' leasders and introns for enhancing transcription, 3' untranslated regions (such as polyadenylation signals and sites), DNA for transit or signal peptides.

Numerous promoters that are active in plant cells have been described in the literature. These include promoters present in plant genomes as well as promoters from other sources, including nopaline synthase (NOS) promoter and octopine synthase (OCS) promoters carried on tumor-inducing plasmids of *Agrobacterium tumefaciens,* caulimovirus promoters such as the cauliflower mosaic virus. For instance, see U.S. Patents No. 5,858,742 and 5,322,938, which disclose versions ofthe constitutive promoter derived from cauliflower mosaic virus (CaMV35S), U.S. Patent 5,641,876, which discloses a rice actin promoter, U.S. Patent Application Publication 2002/0192813A1, which discloses 5', 3' and intron elements useful in the design of effective plant expression vectors, U.S. patent application Serial No. 09/757,089, which discloses a maize chloroplast aldolase promoter, U.S. patent application Serial No. 08/706,946, which discloses a rice glutelin promoter, U.S. patent application Serial No.09/757,089, which discloses a maize aldolase (FDA) promoter, and U.S. patent application Serial No.60/310, 370, which discloses a maize nicotianamine synthase promoter, all of which are incorporated herein by reference. These and numerous other promoters that function in plant cells are known to those skilled in the art and available for use in recombinant polynucleotides of the present invention to provide for expression of desired genes in transgenic plant cells.

In other aspects of the invention, preferential expression in plant green tissues is desired. Promoters of interest for such uses include those from genes such as Arabidopsis thaliana ribulose-1,5-bisphosphate carboxylase (Rubisco) small subunit (Fischhoff et al. (1992) Plant Mol Biol. 20:81-93), aldolase and pyruvate orthophosphate dikinase (PPDK) (Taniguchi et al. (2000) Plant Cell Physiol. 41(1):42-48).

Furthermore, the promoters may be altered to contain multiple "enhancer sequences" to assist in elevating gene expression. Such enhancers are known in the art. By including an enhancer sequence with such constructs, the expression of the selected protein may be enhanced. These enhancers often are found 5' to the start of transcription in a promoter that functions in eukaryotic cells, but can often be inserted upstream (5') or downstream (3') to the coding sequence. In some instances, these 5' enhancing elements are introns. Particularly useful as enhancers are the 5' introns of the rice actin 1 (see US Patent 5,641,876)and rice actin 2 genes, the maize alcohol dehydrogenase gene intron, the maize heat shock protein 70 gene intron (U.S. Patent 5,593,874) and the maize shrunken 1 gene.

In other aspects of the invention, sufficient expression in plant seed tissues is desired to effect improvements in seed composition. Exemplary promoters for use for seed composition modification include promoters from seed genes such as napin (U.S. 5,420,034), maize L3 oleosin (U.S. 6,433,252), zein Z27 (Russell et al. (1997) Transgenic Res. 6 (2):157-166), globulin 1 (Belanger et al (1991) Genetics 129:863-872), glutelin 1 (Russell (1997) *supra*), and peroxiredoxin antioxidant (Per1) (Stacy et al. (1996) Plant Mol Biol. 31(6):1205-1216).

Recombinant DNA constructs prepared in accordance with the invention will also generally include a 3' element that typically contains a polyadenylation signal and site. Well-known 3' elements include those from *Agrobacterium tumefaciens* genes such as *nos 3', tml 3', tmr 3', tms 3', ocs 3', tr7 3'*, for example disclosed in U.S. 6,090,627, incorporated herein by reference; 3' elements from plant genes such as wheat (*Triticum aesevitum*) heat shock protein 17 (*Hsp17 3'*), a wheat ubiquitin gene, a wheat fructose-1,6-biphosphatase gene, a rice glutelin gene a rice lactate dehydrogenase gene and a rice beta-tubulin gene, all of which are disclosed in U.S. published patent application 2002/0192813 A1, incorporated herein by reference; and the pea (*Pisum sativum*) ribulose biphosphate carboxylase gene (*rbs 3')*, and 3' elements from the genes within the host plant.

Constructs and vectors may also include a transit peptide for targeting of a gene target to a plant organelle, particularly to a chloroplast, leucoplast or other plastid organelle. For descriptions of the use of chloroplast transit peptides see U.S. Patent 5, 188,642 and U.S. Patent No. 5,728,925, incorporated herein by reference. For description ofthe transit peptide region of an Arabidopsis EPSPS gene useful in the present invention, see Klee, H.J. et al (MGG (1987) 210:437-442).

Transgenic plants comprising or derived from plant cells of this invention transformed with recombinant DNA can be further enhanced with stacked traits, e.g. a crop plant having an enhanced trait resulting from expression of DNA disclosed herein in combination with herbicide and/or pest resistance traits. For example, genes of the current invention can be stacked with other traits of agronomic interest, such as a trait providing herbicide resistance, or insect resistance, such as using a gene from *Bacillus thuringensis* to provide resistance against lepidopteran, coliopteran, homopteran, hemiopteran, and other insects. Herbicides for which transgenic plant tolerance has been demonstrated and the method of the present invention can be applied include, but are not limited to, glyphosate, dicamba, glufosinate, sulfonylurea, bromoxynil and norflurazon herbicides. Polynucleotide molecules encoding proteins involved in herbicide tolerance are well-known in the art and include, but are not limited to, a polynucleotide molecule encoding 5-enolpyruvylshikimate-3-phosphate synthase (EPSPS) disclosed in U.S. Patent 5,094,945; 5,627,061; 5,633,435 and 6,040,497 for imparting glyphosate tolerance; polynucleotide molecules encoding a glyphosate oxidoreductase (GOX) disclosed in U.S. Patent 5,463,175 and a glyphosate-N-acetyl transferase (GAT) disclosed in U.S. Patent Application publication 2003/0083480 A1 also for imparting glyphosate tolerance; dicamba monooxygenase disclosed in U.S. Patent Application publication 2003/0135879 A1 for imparting dicamba tolerance; a polynucleotide molecule encoding bromoxynil nitrilase (*Bxn*) disclosed in U.S. Patent 4,810,648 for imparting bromoxynil tolerance; a polynucleotide molecule encoding phytoene desaturase (*crtl*) described in Misawa et al, (1993) Plant J. 4:833-840 and Misawa et al, (1994) Plant J. 6:481-489 for norflurazon tolerance; a polynucleotide molecule encoding acetohydroxyacid synthase (AHAS, *aka* ALS) described in Sathasiivan et al. (1990) Nucl. Acids Res. 18:2188-2193 for imparting tolerance to sulfonylurea herbicides; polynucleotide molecules known as *bar* genes disclosed in DeBlock, et al. (1987) EMBO J. 6:2513-2519 for imparting glufosinate and bialaphos tolerance; polynucleotide molecules disclosed in U.S. Patent Application Publication 2003/010609 A1 for imparting N-amino methyl phosphonic acid tolerance; polynucleotide molecules disclosed in U.S. Patent 6,107,549 for impartinig pyridine herbicide resistance; molecules and methods for imparting tolerance to multiple herbicides such as glyphosate, atrazine, ALS inhibitors, isoxoflutole and glufosinate herbicides are disclosed in U.S. Patent 6,376,754 and U.S. Patent Application Publication 2002/0112260, all of said U.S. Patents and Patent Application Publications are incorporated herein by reference. Molecules and methods for imparting insect/nematode/virus resistance is disclosed in U.S. Patents 5,250,515; 5,880,275; 6,506,599; 5,986,175 and U.S. Patent Application Publication 2003/0150017 A1, all of which are incorporated herein by reference.

**Plant Cell Transformation Methods**

Numerous methods for transforming plant cells with recombinant DNA are known in the art and may be used in the present invention. Two commonly used methods for plant transformation are *Agrobacterium*-mediated transformation and microprojectile bombardment. Microprojectile bombardment methods are illustrated in U.S. Patents 5,015,580 (soybean); 5,550,318 (corn); 5,538,880 (corn); 5,914,451 (soybean); 6,160,208 (corn); 6,399,861 (corn); 6,153,812 (wheat) and 6,365,807 (rice) and *Agrobacterium*-mediated transformation is described in U.S. Patents 5,159,135 (cotton); 5,824,877 (soybean); 5,463,174 (canola); 5,591,616 (corn); 6,384,301 (soybean), 7,026,528 (wheat) and 6329571 (rice), all of which are incorporated herein by reference. For *Agrobacterium tumefaciens* based plant transformation systems, additional elements present on transformation constructs will include T-DNA left and right border sequences to facilitate incorporation of the recombinant polynucleotide into the plant genome.

In general it is useful to introduce recombinant DNA randomly, i.e. at a non-specific location, in the genome of a target plant line. In special cases it may be useful to target recombinant DNA insertion in order to achieve site-specific integration, for example, to replace an existing gene in the genome, to use an existing promoter in the plant genome, or to insert a recombinant polynucleotide at a predetermined site known to be active for gene expression. Several site specific recombination systems exist which are known to function in plants including cre-lox as disclosed in U.S. Patent 4,959,317 and FLP-FRT as disclosed in U.S. Patent 5,527,695, both incorporated herein by reference.

Transformation methods of this invention are preferably practiced in tissue culture on media and in a controlled environment. "Media" refers to the numerous nutrient mixtures that are used to grow cells *in vitro,* that is, outside of the intact living organism. Recipient cell targets include, but are not limited to, meristem cells, hypocotyls, calli, immature embryos and gametic cells such as microspores, pollen, sperm and egg cells. It is contemplated that any cell from which a fertile plant may be regenerated is useful as a recipient cell. Callus may be initiated from tissue sources including, but not limited to, immature embryos, hypocotyls, seedling apical meristems, microspores and the like. Cells capable of proliferating as callus are also recipient cells for genetic transformation. Practical transformation methods and materials for making transgenic plants of this invention, for example various media and recipient target cells, transformation of immature embryo cells and subsequent regeneration of fertile transgenic plants are disclosed in U.S. Patents 6,194,636 and 6,232,526, which are incorporated herein by reference.

The seeds of transgenic plants can be harvested from fertile transgenic plants and be used to grow progeny generations of transformed plants of this invention including hybrid plants line for selection of plants having an enhanced trait. In addition to direct transformation of a plant with a recombinant DNA, transgenic plants can be prepared by crossing a first plant having a recombinant DNA with a second plant lacking the DNA. For example, recombinant DNA can be introduced into a first plant line that is amenable to transformation to produce a transgenic plant which can be crossed with a second plant line to introgress the recombinant DNA into the second plant line. A transgenic plant with recombinant DNA providing an enhanced trait, e.g. enhanced yield, can be crossed with transgenic plant line having other recombinant DNA that confers another trait, for example herbicide resistance or pest resistance, to produce progeny plants having recombinant DNA that confers both traits. Typically, in such breeding for combining traits the transgenic plant donating the additional trait is a male line and the transgenic plant carrying the base traits is the female line. The progeny of this cross will segregate such that some of the plants will carry the DNA for both parental traits and some will carry DNA for one parental trait; such plants can be identified by markers associated with parental recombinant DNA, e.g. marker identification by analysis for recombinant DNA or, in the case where a selectable marker is linked to the recombinant, by application of the selecting agent such as a herbicide for use with a herbicide tolerance marker, or by selection for the enhanced trait. Progeny plants carrying DNA for both parental traits can be crossed back into the female parent line multiple times, for example usually 6 to 8 generations, to produce a progeny plant with substantially the same genotype as one original transgenic parental line but for the recombinant DNA of the other transgenic parental line

In the practice of transformation DNA is typically introduced into only a small percentage of target plant cells in any one transformation experiment. Marker genes are used to provide an efficient system for identification of those cells that are stably transformed by receiving and integrating a recombinant DNA molecule into their genomes. Preferred marker genes provide selective markers which confer resistance to a selective agent, such as an antibiotic or a herbicide. Any of the herbicides to which plants of this invention may be resistant are useful agents for selective markers. Potentially transformed cells are exposed to the selective agent. In the population of surviving cells will be those cells where, generally, the resistance-conferring gene is integrated and expressed at sufficient levels to permit cell survival. Cells may be tested further to confirm stable integration of the exogenous DNA. Commonly used selective marker genes include those conferring resistance to antibiotics such as kanamycin and paromomycin (*nptII*), hygromycin B (*aph IV*), spectinomycin *(aad*A) and gentamycin *(aac3* and *aac*C4) or resistance to herbicides such as glufosinate (*bar* or *pat*), dicamba (DMO) and glyphosate (*aroA* or EPSPS). Examples of such selectable markers are illustrated in U.S. Patents 5,550,318; 5,633,435; 5,780,708 and 6,118,047, all of which are incorporated herein by reference. Selectable markers which provide an ability to visually identify transformants can also be employed, for example, a gene expressing a colored or fluorescent protein such as a luciferase or green fluorescent protein (GFP) or a gene expressing a *beta*-glucuronidase or *uid*A gene (GUS) for which various chromogenic substrates are known.

Plant cells that survive exposure to the selective agent, or plant cells that have been scored positive in a screening assay, may be cultured in regeneration media and allowed to mature into plants. Developing plantlets regenerated from transformed plant cells can be transferred to plant growth mix, and hardened off, for example, in an environmentally controlled chamber at about 85% relative humidity, 600 ppm $CO_2$, and 25-250 microcinsteins m$^{-2}$ s$^{-1}$ of light, prior to transfer to a greenhouse or growth chamber for maturation. Plants are regenerated from about 6 weeks to 10 months after a transformant is identified, depending on the initial tissue, and plant species. Plants may be pollinated using conventional plant breeding methods known to those of skill in the art and seed produced, for example self-pollination is commonly used with transgenic corn. The regenerated transformed plant or its progeny seed or plants can be tested for expression of the recombinant DNA and selected for the presence of enhanced agronomic trait.

**Transgenic Plants and Seeds**

Transgenic plants derived from the plant cells of this invention are grown to generate transgenic plants having an enhanced trait as compared to a control plant and produce transgenic seed and haploid pollen of this invention. Such plants with enhanced traits are identified by selection of transformed plants or progeny seed for the enhanced trait. For efficiency a selection method is designed to evaluate multiple transgenic plants (events) comprising the recombinant DNA , for example multiple plants from 2 to 20 or more transgenic events. Transgenic plants grown from transgenic seed provided herein demonstrate improved agronomic traits that contribute to increased yield or other trait that provides increased plant value, including, for example, improved seed quality. Of particular interest are plants having enhanced water use efficiency, enhanced cold tolerance, increased yield, enhanced nitrogen use efficiency, enhanced seed protein and enhanced seed oil.

Table 2 provides a list of protein encoding DNA ("genes") that are useful as recombinant DNA for production of transgenic plants with enhanced agronomic trait, the elements of Table 2 are described by reference to: "PEP SEQ ID NO" identifies an amino acid sequence from SEQ ID NO: 359 to 716. "NUC SEQ ID NO" identifies a DNA sequence from SEQ ID NO: 1 to 358. "BV id" is a reference to the identifying number in Table 4 of base vectors used for construction of the transformation vectors of the recombinant DNA. Construction of plant transformation constructs is illustrated in Example 1.

"Gene Name" which is a common name for protein encoded by the recombinant DNA. "Annotation" refers to a description of the top hit protein obtained from an amino acid sequence query of each PEP SEQ ID NO to Gen Bank database of the National Center for Biotechnology Information (ncbi). More particularly, "gi" is the GenBank ID number for the top BLAST hit;

"description" refers to the description of the top BLAST hit ;

"% id" refers to the percentage of identically matched amino acid residues along the length of the portion of the sequences which is aligned by BLAST (-F T) between the sequence of interest provided herein and the hit sequence in GenBank;

Table 2.

| nuc seq ID NO | pep seq ID NO | Gene ID | BV id | Gene Name | Annotation | | |
|---|---|---|---|---|---|---|---|
| | | | | | % id | GenBank id | desciption |
| 1 | 359 | PHE0001295_ 7469 | 4 | ricecryptochrome 1-AB073546 | 95 | gi\|50909767\| | ref\|XP_466372.1\|cryptoch rome 1a [Oryza sativa (japonica cultivar-group)] |
| 2 | 360 | PHE0002129_ 8308 | 16 | Nostoc sp. PCC 7120 phosphoenolpyruvate carboxylase | 93 | gi\|17133998\| | ref\|NP_488901.1\| phosphoenolpyruvate carboxylase [Nostoc sp. PCC 7120] |
| 3 | 361 | PHE0002132_ 4965 | 4 | maizephosphoenolpyruvate carboxylase kinase 2 | 80 | gi\|59803710\| | gb\|AAX07936.1\|phospho enolpyruvate carboxylase kinase 2 [Zea mays] |
| 4 | 362 | PHE0002132_ 8653 | 19 | maizephosphoenolpyruvate carboxylase kinase 2 | 80 | gi\|59803710\| | gb\|AAX07936.1\|phospho enolpyruvate carboxylase kinase 2 [Zea mays] |
| 5 | 363 | PHE0002133_ 7497 | 4 | maize phosphoenopyruvate carboxylase kinase 3 | 82 | gi\|59803708\| | gb\|AAX07935.1\|phospho enolpyruvate carboxylase kinase I [Zea mays] |
| 6 | 364 | PHE0002693_ 8516 | 17 | wheat geranylgeranyl reductase like I sequence | 93 | gi\|23397035\| | gb\|AAN31803.1\|putative geranylgeranyl reductase [Arabidopsis thaliana] |
| 7 | 365 | PHE0002777_ 7490 | 4 | maize ferrochelatase-I like 2 sequence | 85 | gi\|50725080\| | dbj\|BAD33213.1\|putative ferrochelatase [Oryza sativa (japonica cultivar-group)] |
| 8 | 366 | PHE0002777_ 8472 | 6 | maize ferrochelatase-I like 2 sequence | 85 | gi\|50725080\| | dbj\|BAD33213.1\|putative ferrochelatase [Oryza sativa (japonica cultivar-group)] |
| 9 | 367 | PHE0002777_ 8726 | 19 | maize ferrochelatase-I like 2 sequence | 85 | gi\|50725080\| | dbj\|BAD33213.1\|putative ferrochelatase [Oryza sativa (japonica cultivar-group)] |
| 10 | 368 | PHE0002779_ 7478 | 4 | soybean phosphoglucomutase like I sequence | 89 | gi\|6272281\| | emb\|CAB60127.1\|cytosolic phosphoglucomutase [Pisum sativum] |
| 11 | 369 | PHE0002810_ 5803 | 9 | maize cytochrome P450 monooxygenase (CYP71B3) like 4 sequence | 92 | gi\|1870201\| | emb\|CAA72208.1\|cytochrome p450 [Zea mays] emb\|CAA57423.1\| cytochrome P450 [Zea mays] |
| 12 | 370 | PHE0002857_ 7502 | 4 | Zea Mays putative low molecular early light-inducible protein | 61 | gi\|50934635\| | ref\|XP_476845.1\|putative low molecular mass early light-induced protein, chloroplast precursor (ELIP) [Oryza sativa (japonica cultivar-group)] |

(continued)

| nuc seq ID NO | pep seq ID NO | Gene ID | BV id | Gene Name | Annotation | | |
|---|---|---|---|---|---|---|---|
| | | | | | % id | GenBank id | desciption |
| 13 | 371 | PHE0002860_ 7494 | 4 | Zea Mays Unknown protein | 25 | gi|15237638| | ref|NP_201222.1|unknown protein [Arabidopsis thaliana] |
| 14 | 372 | PHE0002860_ 8694 | 19 | Zea Mays Unknown protein | 25 | gi|15237638| | ref|NP_201222.1|unknown protein [Arabidopsis thaliana] |
| 15 | 373 | PHE0003814_ 7802 | 17 | rice PsbS like | 85 | gi|34908652| | ref|NP_915673.1|putative pltotosystem II subunit (22KDa) precursor [Oryza sativa (japonica cultivar-group)] |
| 16 | 374 | PHE0003838_ 5934 | 1 | soy G2604 like1 | 79 | gi|18398482| | |AAQ55219.1| LSD1-like [Arabidopsis thaliana] |
| 17 | 375 | PHE0003845_ 5806 | 11 | Arabidopsis DWF4 | 88 | gi|15229822| | emb|CAB62435.1| steroid 22-alpha-hydroxylase (DWF4) [Arabidopsis thaliana] |
| 18 | 376 | PHE0003845_ 7028 | 17 | Arabidopsis DWF4 | 88 | gi|15229822| | ref|NP_190635.1|DWF4 (DWARF 4) [Arabidopsis thaliana] |
| 19 | 377 | PHE0003845_ 7413 | 2 | Arabidopsis DWF4 | 88 | gi|15229822| | ref|NP_190635.1|DWF4 (DWARF 4) [Arabidopsis thaliana] |
| 20 | 378 | PHE0004013_ 9281 | 9 | ARGOS-like | 68 | gi|62734659| | gb|AAX96768.1|expressed protein [Oryza sativa (japonica cultivar-group)] g |
| 21 | 379 | PHE0004021_ 4654 | 10 | Galdieria sulphuraria asparagine synthetase | 45 | gi|83769256| | dbj|BAE59393.1|unnamed protein product [Aspergillus oryzae] |
| 22 | 380 | PHE0004143_ 7850 | 1 | Arabidopsis putative glutathione peroxidase | 100 | gi|7267860| | emb|CAB78203.1|phosph olipid hydroperoxide glutathione peroxidase [Arabidopsis thaliana] |
| 23 | 381 | PHE0004143_ 8160 | 19 | Arabidopsis putative glutathione peroxidase | 92 | gi|30681827| | ref|NP_192897.2|ATGPX 6 (GLUTATHIONE PEROXIDASE 6); glutathione peroxidase [Arabidopsis thaliana] |
| 24 | 382 | PHE0004311_ 5022 | 1 | Arabidopsis aminopeptidase P | 99 | gi|24209881| | gb|AAN41402.1|aminope ptidase P [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 25 | 383 | PHE0004398_ 5136 | 4 | rice NPK1 kinase domain only | 95 | gi\|50900060\| | ref\|XF_450818.1\|putative protein kinase [Oryza sativa (japonica cultivar-group)] |
| 26 | 384 | PHE0004398_ 5757 | 13 | rice NPK1 kinase domain only | 95 | gi\|50900060\| | ref\|XP_450818.1\|putative protein kinase [Oryza sativa (japonica cultivar-group)] |
| 27 | 385 | PHE0004473_ 5214 | 4 | Arabidopsis putative histone H2A | 69 | gi\|15241016\| | ref\|NP_198119.1\|DNA binding [Arabidopsis thaliana] |
| 28 | 386 | PHE0004473_ 8803 | 19 | Arabidopsis putative histone H2A | 69 | gi\|15241016\| | ref\|TNP_198119.1\|DNA binding [Arabidopsis thaliana] |
| 29 | 387 | PHE0004503_ 5244 | 4 | Arabidopsis nodulin MtN3 family protein | 100 | gi\|15240040\| | emb\|CAC05445.1\| senescence-associated protein (SAG29) [Arabidopsis thaliana] |
| 30 | 388 | PHE0004503_ 8801 | 19 | Arabidopsis nodulin MtN3 family protein | 100 | gi\|15240040\| | emb\|CAC05445.1\| senescence-associated protein (SAG29) [Arabidopsis thaliana] |
| 31 | 389 | PHE0004641_ 5519 | 9 | corn photosynthetic NADP-dependent malic enzyme | 90 | gi\|126737\| | gb\|AAA33487.1\| NADP-dependent malic enzyme (EC 1.1.1.40) |
| 32 | 390 | PHE0004642_ 5520 | 9 | corn non-photosynthetic NADP-dependent malic enzyme | 85 | gi\|37147841\| | gb\|AAQ88396.1\|non-photosynthetic NADP-malic enzyme [Zea mays] |
| 33 | 391 | PHE0004670_ 6044 | 9 | Arabidopsis putative glutathione peroxidase | 100 | gi\|15225103\| | ref\|NP_180715.1\|ATGPX 2 (GLUTATHIONE PEROXIDASE 2); glutathione peroxidase |
| 34 | 392 | PHE0004683_ 8693 | 19 | Arabidopsis SUMO activating enzyme 1a | 100 | gi\|18416454\| | gb\|AAN15413.1\| ubiquitin activating enzyme - like protein |
| 35 | 393 | PHE0004742_ 5691 | 13 | rice putative CRT/DRE binding factor | 67 | gi\|50948187\| | ref\|XP_483621.1\|putative CRT/DRE binding factor [Oryza sativa (japonica cultivar-group)] |
| 36 | 394 | PHE0004747_ 5708 | 16 | Xenorhabdus nematophilus MMSDH-like | 75 | gi\|87120270\| | ref\|ZP_01076165.1\|methy Imalonate-semialdehyde dehydrogenase [Marinomonas sp. MED121] |

(continued)

| nuc seq ID NO | pep seq ID NO | Gene ID | BV id | Gene Name | Annotation | | |
|---|---|---|---|---|---|---|---|
| | | | | | % id | GenBank id | desciption |
| 37 | 395 | PHE0004761_ 5728 | 4 | Arabidopsis transducin family protein / WD-40 repeat family protein | 92 | gi\|15218889\| | ref\|NP_174226.1\|nucleotide binding [Arabidopsis thaliana] |
| 38 | 396 | PHE0004762_ 5729 | 4 | Arabidopsis F-box family protein | 89 | gi\|56790216\| | dbj\|BAB09749.1\| unnamed protein product [Arabidopsis thaliana] |
| 39 | 397 | PHE0004762_ 7997 | 19 | Arabidopsis F-box family protein | 89 | gi\|56790216\| | dbj\|BAB09749.1\| unnamed protein product [Arabidopsis thaliana] |
| 40 | 398 | PHE0004766_ 5733 | 4 | soy ATP-binding-cassette transporter | 76 | gi\|15234447\| | gb\|AAD03441.1\| contains similarity to Guillardia theta ABC transporter (GB: AF041468) [Arabidopsis thaliana] |
| 41 | 399 | PHE0004779_ 5749 | 4 | Arabidopsis ammonium transporter | 89 | gi\|15230092\| | ref\|NP_189073.1\|ATAMT 1;3; ammonium transporter [Arabidopsis thaliana] |
| 42 | 400 | PHE0004779_ 8394 | 1 | Arabidopsis ammonium transporter | 89 | gi\|15230092\| | ref\|NP_189073.1\|ATAMT 1;3; ammonium transporter [Arabidopsis thaliana] |
| 43 | 401 | PHE0004780_ 5752 | 4 | Arabidopsisexpressed protein | 92 | gi\|21536499\| | gb\|AAM60831.1 \|unknown [Arabidopsis thaliana] |
| 44 | 402 | PHE0004782_ 5754 | 4 | Arabidopsis hypothetical protein | 65 | gi\|18404589\| | ref\|NP_565874.1\|EMB15 13; copper ion transporter [Arabidopsis thaliana] |
| 45 | 403 | PHE0004784_ 5760 | 1 | soy S-adenosylmethionine decarboxylase | 72 | gi\|21239731\| | gb\|AAM44307.1\|Sadenosylmethionine decarboxylase [x Citrofortunella mitis] |
| 46 | 404 | PHE0004787_ 7988 | 19 | rice Nitrogen regulatory protein P-II | 73 | gi\|50878396\| | gb\|AAT85171.1\|putative P-II nitrogen sensing protein |
| 47 | 405 | PHE0004791_ 5771 | 4 | Xenorhabdus nematophila Flavohemoprotein | 73 | gi\|36786606\| | emb\|CAE15666.1\|Flavohe moprotein (hemoglobin-like protein) (flavohemoglobin) (dihydropteridine reductase) (ferrisiderophore reductase B) (nitric oxide dioxygenase) (NOD) [Photorhabdus luminescens subsp. laumondii TTO1] |

| nuc seq ID NO | pep seq ID NO | Gene ID | BV id | Gene Name | Annotation | | |
|---|---|---|---|---|---|---|---|
| | | | | | % id | GenBank id | desciption |
| 48 | 406 | PHE0004805_ 5791 | 17 | corn hypothetical protein | 51 | gi\|50509855\| | dbj\|BAD32027.1\|unknown protein [Oryza sativa (japonica cultivar-group)] |
| 49 | 407 | PHE0004806_ 5792 | 17 | rice OTU-like cysteine protease-like | 90 | gi\|50915926\| | ref\|XP_468427.1\|OTU-like cysteine protease-like [Oryza sativa (japonica cultivar-group)] |
| 50 | 408 | PHE0004807_ 5793 | 17 | corn cleavage stimulation factor 64 | 97 | gi\|62733690\| | gb\|AAX95801.1\|RNA recognition motif. (a.k.a. RRM, RBD, or RNP domain), putative [Oryza sativa (japonica cultivar-proup)] |
| 51 | 409 | PHE0004808_ 5794 | 17 | corn cysteine proteinase | 63 | gi\|2224810\| | emb\|CAB09698.1\|cysteine proteinase [Hordeum vulgare subsp. vulgare] |
| 52 | 410 | PHE0004809_ 5795 | 17 | corn MRT4577_261462 putative protein phosphatase 2C | 71 | gi\|34898886\| | ref\|NP_910789.1\|putative protein phosphatase 2C (PP2C) [Oryza sativa (japonica cultivar-group)] |
| 53 | 411 | PHE0004810_ 5796 | 17 | rice MRT4577_41500 putative calcium-dependent protein kinase | 90 | gi\|50948089\| | ref\|XP_483572.1\|putative calcium-dependent protein kinase [Oryza sativa (japonica cultivar-group)] |
| 54 | 412 | PHE0004811_ 5798 | 17 | rice MRT4577_35987 C3HC4-type RING finger | 78 | gi\|50911677\| | ref\|XP_467246.1\|zinc finger (C3HC4-type RING finger)-like [Oryza sativa (japonica cultivar-group)] |
| 55 | 413 | PHE0004812_ 5799 | 17 | rice MRT4577_148933 plastid sigma factor SIG5 | 80 | gi\|30698518\| | dbj\|BAC76607.1\|plastid sigma factor SIG5 [Oryza sativa (japonica cultivar-group)] |
| 56 | 414 | PHE0004813_ 5800 | 17 | corn putative zinc finger protein | 85 | gi\|57900442\| | sp\|Q5JLB5\|ZFNL2_ORY SA Zinc finger CCCH type domain containing protein ZFN-like 2 |
| 57 | 415 | PHE0004815_ 5802 | 17 | corn protein kinase family protein | 51 | gi\|15237684\| | ref\|NP_200660.1\|ATP binding / kinase/ protein kinase/ protein serine/threonine kinase/ protein-tyrosine kinase [Arabidopsis thaliana |

| nuc seq ID NO | pep seq ID NO | Gene ID | BV id | Gene Name | Annotation | | |
|---|---|---|---|---|---|---|---|
| | | | | | % id | GenBank id | desciption |
| 58 | 416 | PHE0004827_ 5825 | 4 | corn phosphate-induced protein 1-like (EXORDIUM) | 74 | gi|50912943| | ref|XP_467879.1|putative phi-1 [Oryza sativa (japonica cultivar-group)] |
| 59 | 417 | PHE0004830_ 5828 | 4 | Arabidopsis putative RelA/ SpoT protein | 91 | gi|15231772| | ref|NP_188021.1|RSH2 (RELA-SPOT HOMOLOG); catalytic [Arabidopsis thaliana] dbj|BAB02337.1| unnamed protein product [Arabidopsis thaliana] |
| 60 | 418 | PHE0004845_ 5852 | 4 | Arabidopsis Beta carotene hydroxilase | 96 | gi|15235959| | ref|NP_194300.1|BETA-OHASE 1 (BETA-HYDROXYLASE 1); ] |
| 61 | 419 | PHE0004856_ 7855 | 1 | Arabidopsis phototropic-responsive NPH3 family protein | 93 | gi|22331730| | ref|NP_190653.1|protein binding I signal transducer [Arabidopsis thaliana] |
| 62 | 420 | PHE0004859_ 5896 | 4 | Arabidopsis thaliana Glutamine dumper 1 | 77 | gi|15236062| | ref|NP_194901.1|GDU1 (GLUTAMINE DUMPER 1) [Arabidopsis thaliana] |
| 63 | 421 | PHE0004859_ 5917 | 8 | Arabidopsis thaliana Glutamine dumper 1 | 77 | gi[15236062| | ref|NP_194901.1|GDU1 (GLUTAMINE DUMPER 1) [Arabidopsis thaliana] |
| 64 | 422 | PHE0004883_ 5935 | 1 | Arabidopsis serine/ threonine protein kinase | 89 | gi|15240864| | ref|NP_198641.1|ATP binding / kinase/ protein kinase/ protein serine/threonine kinase/ protein-tyrosine kinase [Arabidopsis thaliana] |
| 65 | 423 | PHE0004886_ 5938 | 4 | Arabidopsis thaliana GEK1 | 94 | gi|4406770| | gb|AAD20081.1|unknown protein [Arabidopsis thaliana] |
| 66 | 424 | PHE0004887_ 5939 | 4 | Zea mays GEK1-like | 74 | gi|50944457| | ref|XP_481756.1|putative GEKO1 [Oryza sativa (japonica cultivar-group)] |
| 67 | 425 | PHE0004887_ 5940 | 16 | Zea mays GEK1-like | 74 | gi|50944457| | ref|XP_481756.1|putative GEKO1 [Oryza sativa (japonica cultivar-group)] |
| 68 | 426 | PHE0004887_ 8704 | 19 | Zea mays GEK1-like | 74 | gi|50944457| | ref|XP_481756.1|putative GEKO1 [Oryza sativa (japonica cultivar-group)] |
| 69 | 427 | PHE0004889_ 7961 | 19 | Corn OsRAA1-like | 75 | gi|34902924| | dbj|BAB07982.1|FPF1 protein-like [Oryza sativa (japonica cultivar-group)]. |

| nuc seq ID NO | pep seq ID NO | Gene ID | BV id | Gene Name | Annotation | | |
|---|---|---|---|---|---|---|---|
| | | | | | % id | GenBank id | desciption |
| 70 | 428 | PHE0004894_ 5948 | 17 | corn plastid division protein FtsZ | 79 | gi\|50929441\| | gb\|AAK64282.1\| plastid division protein FtsZ [Oryza sativa] |
| 71 | 429 | PHE0004894_ 5950 | 10 | corn plastid division protein FtsZ | 79 | gi\|50929441\| | gb\|AAK64282.1\| plastid division protein FtsZ [Oryza sativa] |
| 72 | 430 | PHE0004894_ 5951 | 4 | corn plastid division protein FtsZ | 79 | gi\|50929441\| | gb\|AAK64282.1\| plastid division protein FtsZ [Oryza sativa] |
| 73 | 431 | PHE0004895_ 5952 | 4 | comdeoxyhypusine synthase 3 | 79 | gi\|1019423\| | gb\|AAC49075.1\|deoxyhy pusine synthase |
| 74 | 432 | PHE0004895_ 7135 | 15 | corn deoxyhypusine synthase 3 | 79 | gi\|1019423\| | gb\|AAC49075.1\|deoxyhy pusine synthase |
| 75 | 433 | PHE0004895_ 7137 | 17 | corn deoxyhypusine synthase 3 | 79 | gi\|1019423\| | gb\|AAC49075.1\|deoxyhy pusine synthase |
| 76 | 434 | PHE000489S_ 8610 | 19 | corn deoxyhypusine synthase 3 | 79 | gi\|1019423\| | gb\|AAC49075.1\|deoxyhy pusine synthase |
| 77 | 435 | PHE0004902_ 5959 | 4 | Glycine max soy type-A response regulator | 59 | gi\|33330864\| | gb\|AAQ10675.1\|type-A response regulator [Catharanthus roseus] |
| 78 | 436 | PHE0004903_ 5960 | 4 | Arabidopsis expressed protein | 82 | gi\|18412607\| | ref\|NP_565228.1\|unknown protein [Arabidopsis thaliana] |
| 79 | 437 | PHE0004905_ 5962 | 4 | Arabidopsis calcium-dependent protein kinase | 86 | gi\|15236560\| | ref\|NP_194096.1\|CDPK6 (CALCIUM-DEPENDENT PROTEIN KINASE 6); |
| 80 | 438 | PHE0004909_ 5966 | 4 | Arabidopsis protein kinase family protein | 83 | gi\|42561860\| | ref\|NP_172415.2\|ATP binding / kinase/ protein kinase/ protein serine/threonine kinase/ protein-tyrosine kinase |
| | | | | | | | [Arabidopsis thaliana] |
| 81 | 439 | PHE0004911_ 5968 | 4 | Arabidopsis thioredoxin family protein | 91 | gi\|15235475\| | ref\|NP_195437.1\|HCF164 ;thiol-disulfide exchange intermediate [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 82 | 440 | PHE0004912_ 5969 | 4 | Arabidopsis putative serine/ threonine protein kinase | 87 | gi|15235432| | ref|NP_192172.1|ATP binding / kinase/ protein kinase/ protein serine/threonine kinase/ protein-tyrosine kinase [Ambidopsis thaliana] |
| 83 | 441 | PHE0004918_ 5975 | 4 | Arabidopsis expressed protein | 90 | gi|42571697| | ref|NP_973939.1|unknown protein [Arabidopsis thaliana] |
| 84 | 442 | PHE0004921_ 5979 | 4 | corn hypothetical protein | 80 | gi|50917557| | ref|XP_469175.1|hypothetical protein [Oryza sativa (japonica cultivar-group)] |
| 85 | 443 | PHE0004928_ 5986 | 4 | Arabidopsis putative peptidyl-prolyl cis-trans isomerase | 100 | gi|15227956| | gb|AAL07163.1| putative peptidyl-prolyl cis-trans isomerase [Arabidopsis thaliana] |
| 86 | 444 | PHE0004932_ 6045 | 9 | Arabidopsis PUR alpha-1 protein | 92 | gi|30685174| | ref|NP_850182.1|PUR ALPHA-1; nucleic acid binding [Arabidopsis thaliana]] |
| 87 | 445 | PHE0004941_ 5997 | 4 | Arabidopsis dehydrin | 42 | gi|30693389| | sp|P25863|XERO1_ARATH Dehydrin Xero 1 gb|AAB00375.1| dehydrin |
| 88 | 446 | PHE0004948_ 6003 | 9 | corn PUR alpha-1 protein | 97 | gi|34902984| | ref|NP_912839.1|unnamed protein product [Oryza sativa (japonica cultivar-group)] |
| 89 | 447 | PHE0004966_ 6028 | 4 | Arabidopsis sugar transporter family protein | 97 | gi|56381949| | ref|NP_200733.2| carbohydrate transporter/ sugar porter [Arabidopsis thaliana] |
| 90 | 448 | PHE0004968_ 6030 | 4 | Arabidopsis RNase L inhibitor-like protein | 100 | gi|22328793| | gb|AAN15617.1| RNase L inhibitor-like protein [Arabidopsis thaliana] |
| 91 | 449 | PHE0004977_ 6043 | 9 | Mortierella ramanniana diacylglycerol acyltransferase type 2A | 95 | gi|15099959| | gb|AAK84179.1|diacylgly cerol acyltransferase type 2A [Mortierella ramanniana] |
| 92 | 450 | PHE0004979_ 6047 | 3 | yeast SUC2 | 100 | gi|50554053| | ref|XP_504435.1|YIXPR2 :SUC2 [Yarrowia lipolytica] |
| 93 | 451 | PHE0004984_ 7235 | 4 | Arabidopsis putative aspartate kinase | 100 | gi|15232838| | ref|NP_186851.1|amino acid binding / aspartate |

| nuc seq ID NO | pep seq ID NO | Gene ID | BV id | Gene Name | Annotation | | |
|---|---|---|---|---|---|---|---|
| | | | | | % id | GenBank id | desciption |
| | | | | | | | kinase [Arabidopsis thaliana] |
| 94 | 452 | PHE0004984_ 8782 | 19 | Arabidopsis putative aspartate kinase | 100 | gi|15232838| | ref|NP_186851.1|amino acid binding / aspartate kinase [Arabidopsis thaliana] |
| 95 | 453 | PHE0004989_ 8115 | 19 | Arabidopsis CBS domain-containing protein | 96 | gi|42569036| | ref|NF_179058.3|unknown protein [Arabidopsis thaliana] |
| 96 | 454 | PHE0004991_ 8092 | 19 | Arabidopsis auxin-responsive family protein | 77 | gt|15241259| | ref|NP_199889.1|unknown protein [Arabidopsis thaliana] |
| 97 | 455 | PHE0004993_ 6062 | 4 | soy putative protein | 50 | gi|18423511| | ref|NP_568793.1)unknown protein [Arabidopsis thaliana] |
| 98 | 456 | PHE0004993_ 8014 | 19 | soy putative protein | 50 | gi|18423511| | ref|NP_568793.1|unknown protein [Arabidopsis thaliana] |
| 99 | 457 | PHE0004993_ 8682 | 19 | soy putative protein | 50 | gi|18423511| | ref|NP_568793.1|unknown protein [Arabidopsis thaliana] |
| 100 | 458 | PHE0005002_ 6071 | 4 | corn putative magnesium-protoporphyrin IX methyltransferase | 79 | gi|33321009| | gb|AAQ06256.1|putative magnesium-protoporphyrin IX methyltransferase [Sorghum bicolor] |
| 101 | 459 | PHE0005003_ 7032 | 4 | corn putative porphobilinogen deaminase | 83 | gi[50905547] | ref|XP_464262.1|putative porphobilinogen deaminase [Oryza sativa (japonica cultivar-group)] |
| 102 | 460 | PHE0005008_ 6077 | 4 | Arabidopsis two-component responsive regulator family protein | 91 | gi|30679083| | ref|NP_850511.1|ARR22 (ARABIDOPSIS RESPONSE REGULATOR 22); two-component response regulator [Arabidopsis thaliana] |
| 103 | 461 | PHE0005009_ 6078 | 4 | Arabidopsis ubiquitin-conjugating enzyme | 100 | gi|22331064| | ref|NP_566459.2|FUS9 (FUSCA 9); ubiquitin conjugating enzyme [Arabidopsis thaliana] |
| 104 | 462 | PHE0005010_ 6079 | 4 | corn ETCHED1 protein | 94 | gi|48596293| | emb|CAD45039.1|ETCH ED1 protein [Zea mays] |

| nuc seq ID NO | pep seq ID NO | Gene ID | BV id | Gene Name | Annotation | | |
|---|---|---|---|---|---|---|---|
| | | | | | % id | GenBank id | desciption |
| 105 | 463 | PHE0006003_ 7195 | 13 | rice OSISAP1 | 71 | gi\|37548823\| | gb\|AAN15744.1\|multiple stress-associated zinc-finger protein |
| 106 | 464 | PHE0006003_ 7205 | 1 | rice OSISAP1 | 71 | gi\|37548823\| | gb\|AAN15744.1\|multiple stress-associated zinc-finger protein |
| 107 | 465 | PHE0006018_ 7098 | 4 | corn translational elongation factor EF-TuM | 90 | gi\|11181616\| | gb\|AAG32661.1\|translatio nal elongation factor EF-TuM [Zea mays] |
| 108 | 466 | PHE0066021_ 7077 | 4 | rice root specific pathogenesis-related protein 10 | 100 | gi\|38678114\| | dbj\|BAD03969.1\|root specific pathogenesis-related protein 10 [Oryza sativa (japonica cultivar-group)] |
| 109 | 467 | PHE0006021_ 8737 | 19 | rice root specific pathogenesis-related protein 10 | 100 | gi\|38678114\| | dbj\|BAD03969.1\|root specific pathogenesis-related protein 10 |
| 110 | 468 | PHE0006043_ 7080 | 4 | Arabidopsis glycosyl transferase family 8 protein | 100 | gi\|18409445\| | ref\|NP_564983.1\|transfera se, transferring glycosyl groups / transferase, transferring hexosyl groups |
| 111 | 469 | PHE0006043_ 8788 | 19 | Arabidopsis glycosyl transferase family 8 protein | 100 | gi\|18409445\| | ref\|NP_564983.1\|transfera se, transferring glycosyl groups / transferase, transferring hexosyl groups |
| 112 | 470 | PHE0006047_ 7234 | 4 | soy hydroperoxide lyase | 69 | gi\|5830465\| | emb\|CAB54847.1\|hydrop eroxide lyase [Medicago sativa] |
| 113 | 471 | PHE0006047_ 8766 | 19 | soy hydroperoxide lyase | 69 | gi\|5830465\| | emb\|CAB54847.1\|hydrop eroxide lyase [Medicago sativa] |
| 114 | 472 | PHE0006048_ 7094 | 4 | soy serine/threonine/tyrosi ne kinase | 90 | gi\|13124865\| | gb\|AAK11734.1\|serine/thr eonine/ tyrosine kinase [Arachis hypogaea] |
| 115 | 473 | PHE0006048_ 8785 | 19 | soy serine/threonine/tyrosi ne kinase | 90 | gi\|13124865\| | gb\|AAK11734.1\|serine/thr eonine/ tyrosine kinase [Arachis hypogaea] |
| 116 | 474 | PHE0006049_ 7107 | 4 | soy putative non-green plastid inner envelope membrane protein | 42 | gi\|18404784\| | gb\|AAC67363.2\| putative non-green plastid inner envelope membrane protein [Arabidopsis thaliana] |

(continued)

| nuc seq ID NO | pep seq ID NO | Gene ID | BV id | Gene Name | Annotation | | |
|---|---|---|---|---|---|---|---|
| | | | | | % id | GenBank id | desciption |
| 117 | 475 | PHE0006051_ 7097 | 4 | Arabidopsis ubiquitin carboxyl-terminal hydrolase family protein | 91 | gi\|15238468\| | ref\|NP_201348.1\|cysteine -type endopeptidase/ ubiquitin thiolesterase [Arabidopsis thaliana] |
| 118 | 476 | PHE0006054_ 7095 | 4 | Arabidopsis GTP-binding protein | 92 | gi\|30680751\| | dbj\|BAB11522.1\| GTP-binding protein [Arabidopsis thaliana] |
| 119 | 477 | PHE0006054_ 8779 | 19 | Arabidopsis GTP-binding protein | 92 | gi\|30680751\| | dbj\|BAB11522.1\| GTP-binding protein [Arabidopsis thaliana] |
| 120 | 478 | PHE0006059_ 7042 | 4 | corn heat-shock protein | 74 | gi\|51964000\| | ref\|XP_465165.1\| putative DnaJ-like protein [Oryza sativa (japonica cultivar-group)] |
| 121 | 479 | PHE0006061_ 7051 | 4 | corn calcineurin-like phosphoesterase family protein | 94 | gi\|50251955\| | dbj\|BAD27890.1\|putative vacuolar protein sorting; Vps29p [Oryza sativa (japonica cultivar-group)] |
| 122 | 480 | PHE0006062_ 7058 | 4 | corn putative ATP synthase | 70 | gi\|50905037\| | ref\|XP_464007.1\|putative ATP synthase [Oryza sativa (japonica cultivar-group)] |
| 123 | 481 | PHE0006063_ 7049 | 4 | corn putative pyruvate dehydrogenase E1 beta subunit | 85 | gi\|77557068\| | gb\|ABA99864.1\|pyruvate dehydrogenase E1 beta subunit [Oryza sativa (japonica cultivar-group)] |
| 124 | 482 | PHE0006068_ 7064 | 4 | corn protein kinase family protein | 80 | gi\|50924460\| | ref\|XP_472590.1\|OSJNBa 0006B20.13 [Oryza sativa (japonica cultivar-group)] |
| 125 | 483 | PHE0006069_ 7065 | 4 | corn unknown protein | 75 | gi\|50924572\| | ref\|XP_472645.1\|OSJNBa 0027P08.10 [Oryza sativa (japonica cultivar-group)] |
| 126 | 484 | PHE0006071_ 7068 | 4 | corn pentatricopeptide (PPR) repeat-containing protein | 44 | gi\|50905575\| | ref\|XP_464276.1\|putative pentatricopeptide (PPR) repeat-containing protein [Oryza sativa (japonica cultivar-group)] |
| 127 | 485 | PHE0006072_ 7071 | 4 | corn unknown protein | 53 | gi\|77554714\| | gb\|ABA97510.1\|transposo n protein, putative, CACTA, En/Spm sub-class [Oryza sativa (japonica cultivar-group)] |

| nuc seq ID NO | pep seq ID NO | Gene ID | BV id | Gene Name | Annotation | | |
|---|---|---|---|---|---|---|---|
| | | | | | % id | GenBank id | desciption |
| 128 | 486 | PHE0006074_ 7060 | 4 | corn putative polyprotein | 44 | gi\|18568267\| | gb\|AAL75999.1\|putative polyprotein [Zea mays] |
| 129 | 487 | PHE0006076_ 7052 | 4 | Arabidopsis Clavata3 / ESR-Related-3 | 100 | gi\|18390629\| | ref\|NP_563763.1\|CLE3 (CLAVATA3/ESR-RELATED 3); receptor binding [Arabidopsis thaliana] |
| 130 | 488 | PHE0006076_ 7331 | 1 | Arabidopsis Clavata3 / ESR-Related-3 | 100 | gi\|18390629\| | ref\|NP_563763.1\|CLE3 (CLAVATA3/ESR-RELATED 3); receptor binding [Arabidopsis thaliana] |
| 131 | 489 | PHE0006077_ 7045 | 4 | Arabidopsis gibberellin-regulated protein 4 | 100 | gi\|15242249\| | sp\|P46690\|GASA4_ARA TH Gibberellin-regulated protein 4 precursor |
| 132 | 490 | PHE0006077_ 7343 | 1 | Arabidopsis gibberellin-regulated protein 4 | 100 | gi\|15242249\| | emb\|CAA66909.1\| GASA4 [Arabidopsis thaliana] sp\|P46690\|GASA4_ARA TH Gibberellin-regulated protein 4 precursor |
| 133 | 491 | PHE0006079_ 7044 | 4 | Arabidopsis sucrose-phosphatase | 100 | gi\|18409555\| | ref\|NP_566964.1\|SPP2 (sucrose-phosphatase 2); catalytic/ sucrose-phosphatase [Arabidopsis thaliana] |
| 134 | 492 | PHE0006079_ 7337 | 1 | Arabidopsis sucrose-phosphatase | 100 | gi\|18409555\| | ref\|NP_566964.1\|SPP2 (sucrose-phosphatase 2); catalytic/ sucrose-phosphatase [Arabidopsis thaliana] |
| 135 | 493 | PHE0006082_ 7330 | 1 | soy stress-induced protein stil-like protein | 66 | gi\|79325071\| | emb\|CAB78283.1\|stress-induced protein stil-like protein [Arabidopsis thaliana] |
| 136 | 494 | PHE0006088_ 7063 | 14 | CTP-RtACL | 70 | gi\|71004972\| | ref\|XP_757152.1\|hypothetical protein UM01005.1 [Ustilago maydis 521] |
| 137 | 495 | PHE0006089_ 7061 | 4 | Arabidopsis brix domain-containing protein | 94 | gi\|18404250\| | ref\|NP_564618.1\|unknown protein [Arabidopsis thaliana] |
| 138 | 496 | PHE0006089_ 7334 | 1 | Arabidopsis brix domain-containing protein | 94 | gi\|18404250\| | ref\|NP_564618.1\|unknown protein [Arabidopsis thaliana] |
| 139 | 497 | PHE0006091_ 7074 | 4 | Arabidopsis putative elongation factor | 94 | gi\|15224901\| | ref\|NP_181390.1\|DNA binding / transcription factor [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 140 | 498 | PHE0006091_ 7341 | 1 | Arabidopsis putative elongation factor | 94 | gi\|15224901\| | ref\|NP-181390.1\|DNA binding / transcription factor [Arabidopsis thaliana] |
| 141 | 499 | PHE0006092_ 7062 | 4 | Arabidopsis oligouridylate-binding protein | 90 | gi\|30695647\| | ref\|NP_849806.1\|mRNA 3'-UTR binding [Arabidopsis thaliana] |
| 142 | 500 | PHE0006092_ 7336 | 1 | Arabidopsis oligouridylate-binding protein | 90 | gi\|30695647\| | ref\|NP_849806.1\|mRNA 3'-UTR binding [Arabidopsis thaliana] |
| 143 | 501 | PHE0006093_ 7066 | 4 | Arabidopsis putative tRNA 2"phosphotransferase | 84 | gi\|2583121\| | gb\|AAB82630.1\|unknown protein [Arabidopsis thaliana] |
| 144 | 502 | PHE0006093_ 7327 | 1 | Arabidopsis putative tRNA 2"phosphotransferase | 84 | gi\|2583121\| | gb\|AAB82630.1\|unknown protein [Arabidopsis thaliana] |
| 145 | 503 | PHE0006094_ 7231 | 4 | Arabidopsis chalcone flavanone isomerase | 100 | gi\|15233190\| | ref\|NP_191072.1\|TT5 (TRANSPARENT TESTA 5); chalcone isomerase [Arabidopsis thaliana] |
| 146 | 504 | PHE0006094_ 7333 | 1 | Arabidopsis chalcone flavanone isomerase | 100 | gi\|15233190\| | ref\|NP_191072.1\|TT5 (TRANSPARENT TESTA 5); chalcone isomerase [Arabidopsis thaliana] |
| 147 | 505 | PHE0006154_ 7204 | 14 | E. coli ATP-dependent phosphofructokinase B (pfkB) | 100 | gi\|85675091\| | dbj\|BAA15500.2\|6-phosphofructokinase II [Escherichia coli W3110] |
| 148 | 506 | PHE0006160_ 7265 | 14 | pyrophosphate-dependent phosphofructokinase (PPi-PFK) | 92 | gi\|1346693\| | sp\|P29495\|PFP_PROFRP yrophosphate-fructose 6-phosphate 1-phosphotransferase |
| 149 | 507 | PHE0006160_ 7286 | 9 | pyrophosphate-dependent phosphofructokinase (PPi-PFK) | 92 | gi\|1346693\| | gb\|AAA25675.1\| pyrophosphate-frustose 6-phosphate 1-phosphotransferase |
| 150 | 508 | PHE0006160_ 8851 | 14 | pyrophosphate-dependent phosphofructokinase (PPi-PFK) | 92 | gi\|1346693\| | sp\|P29495\|PFP_PROFRP yrophosphate--fructose 6-phosphate 1-phosphotransferase |

(continued)

| nuc seq ID NO | pep seq ID NO | Gene ID | BV id | Gene Name | Annotation | | |
|---|---|---|---|---|---|---|---|
| | | | | | % id | GenBank id | desciption |
| 151 | 509 | PHE0006161_ 7215 | 9 | ATP-dependent phosphofructokinase 1 (Pfk-1) | 96 | gi|2956754| | sp|042938|K6PF_SCHPO 6-phosphofructokinase (Phosphofructokinase) (Phosphohexokinase) (6PF-1-K) |
| 152 | 510 | PHE0006161_ 7221 | 14 | ATP-dependent phosphofructokinase 1 (Pfk-1) | 97 | gi|2956754| | sp|O42938|K6PF_SCHPO 6-phosphofructokinase (Phosphofructokinase) (Phosphohexokinase) (6PF-1-K) |
| 153 | 511 | PHE0006173_ 7211 | 12 | Glycine max ribosomal protein S6 | 81 | gi|44662864| | gb|AAS47511. 1 |ribosoma l protein S6 [Glycine max] |
| 154 | 512 | PHE0006174_ 7208 | 12 | Yeast NSR I | 62 | gi|1323271| | ref|NP_011675.1| Nucleolar protein that binds nuclear localization sequences, required for pre-rRNA processing and ribosome biogenesis; Nsr1p [Saccharomyces cerevisiae] |
| 155 | 513 | PHE0006175_ 7210 | 4 | Corn eIF-5A-2 | 87 | gi|34915268| | ref|NP_919091.1|putative translation initiation factor 5A [Oryza sativa (japonica cultivar-group)] |
| 156 | 514 | PHE0006176_ 7147 | 9 | EEM1 | | | |
| 157 | 515 | PHE0006178_ 7139 | 4 | Corn eIF-5A-3 | 63 | gi|4204352| | gb|AAD10697.1|eIF-5A [Candida albicans] |
| 158 | 516 | PHE0006178_ 8626 | 19 | Corn eIF-5A-3 | 63 | gi|4204352| | gb|AAD10697.1|eIF-5A [Candida albicans] sp|O94083|IF5A_CANAL Eukaryotic translation initiation factor SA (eIF-SA) (eIF-4D) |
| 159 | 517 | PHE0006184_ 7245 | 9 | EEM11 | 58 | gi|50924850| | ref|XP_472770.1B1358B 12.19 [Oryza sativa (japonica cultivar-group)] |
| 160 | 518 | PHE0006201_ 7184 | 14 | ZmKASICTP-AtKAS | 94 | gi|22325473| | ref|NP_178533.2|catalytic / fatty-acid synthase [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 161 | 519 | PHE0006201_ 7187 | 9 | ZmKASICTP-AtKAS | 94 | gi|22325473| | ref|NP_178533.2|catalytic / fatty-acid synthase [Arabidopsis thaliana] |
| 162 | 520 | PHE0006202_ 7182 | 4 | MAML-4 (At1g18500) | 94 | gi|42562149| | ref|NP_173285.2|2 isopropylmalate synthase/ catalytic/ transferase, transferring acyl groups, acyl groups converted into alkyl on transfer [Arabidopsis thaliana] |
| 163 | 521 | PHE0006204_ 7183 | 17 | soy Cyclin D | 45 | gi|15236274| | ref|NP_192236.1|CYCD6; 1; cyclin-dependent protein kinase [Arabidopsis thaliana] |
| 164 | 522 | PHE0006204_ 7189 | 4 | soy Cyclin D | 45 | gi|15236274| | ref|NP_192236.1|CYCD6; 1; cyclin-dependent protein kinase [Arabidopsis thaliana |
| 165 | 523 | PHE0006204_ 8634 | 19 | soy Cyclin D | 45 | gi|15236274| | ref|NP_1922361|CYCD6; 1; cyclin-dependent protein kinase [Arabidopsis thaliana] |
| 166 | 524 | PHE0006208_ 7223 | 4 | rice Microtubule-associated EB 1 | 92 | gi|50929089| | ref|XP_474072.1|OSJNBb 0079B02.14 [Oryza sativa (japonica cultivar-group)] |
| 167 | 525 | PHE0006209_ 7991 | 19 | rice 2-isopropylmalate synthase | 96 | gi|77548611| | gb|ABA91408.1|2-isopropylmalate synthase [Oryza sativa (japonica cultivar-group)] |
| 168 | 526 | PHE0006212_ 7196 | 4 | corn Heme oxygenase-like | 89 | gi|51090890| | dbj|BAD35463.1|putative heme oxygenase 1 [Oryza sativa (japonica cultivar-group)] |
| 169 | 527 | PHE0006213_ 7198 | 4 | corn ATG4a-like | 69 | gi|50929729| | gb|ABB77259.1| autophagy 4 [Oryza sativa (indica cultivar-group)] |
| 170 | 528 | PHE0006214_ 7213 | 17 | corn Cyclin D | 61 | gi|50508578| | dbj|BAD30903.1|putative cyclin D1 [Oryza sativa (japonica cultivar-group)] |
| 171 | 529 | PHE0006214_ 7219 | 4 | corn Cyclin D | 61 | gi|50508578| | dbj|BAD30903.1|putative cyclin DI [Oryza sativa (japonica cultivar-group)] |

| nuc seq ID NO | pep seq ID NO | Gene ID | BV id | Gene Name | Annotation | | |
|---|---|---|---|---|---|---|---|
| | | | | | % id | GenBank id | desciption |
| 172 | 530 | PHE0006215_ 7280 | 9 | ATP-dependent phosphofructokinase 1 (Pfk-1) | 92 | gi\|396136\| | emb\|CAA50526.1\|6-phosphofructokinase [Lactobacillus delbrueckii] |
| 173 | 531 | PHE0006221_ 7201 | 13 | OsNTRC | 94 | gi\|34576294\| | emb\|CAE46765.1\|NADP H thioredoxin reductase [Oryza sativa (japonica cultivar-group)] |
| 174 | 532 | PHE0006221_ 7241 | 5 | OsNTRC | 94 | gi\|34576294\| | emb\|CAE46765.1\|NADP H thioredoxin reductase [Oryza sativa (japonica cultivar-group)] |
| 175 | 533 | PHE0006221_ 7937 | 19 | OsNTRC | 94 | gi\|34576294\| | emb\|CAE46765.1\|NADP H thioredoxin reductase [Oryza sativa (japonica cultivar-group)] |
| 176 | 534 | PHE0006227_ 7282 | 1 | ADR1 | 100 | gi\|30692890\| | emb\|CAE46486.1\|CC-NBS-LRR disease resistance protein [Arabidopsis thaliana] |
| 177 | 535 | PHE0006232_ 7454 | 4 | rice Kinase | 95 | gi\|34896978\| | ref\|NP_909835.1\|putative receptor-like kinase [Oryza sativa (japonica cultivar-group)] |
| 178 | 536 | PHE0006232_ 8756 | 19 | rice Kinase | 95 | gi\|34896978\| | ref\|NP_909835.1\|putative receptor-like kinase [Oryza sativa (japonica cultivar-groups |
| 179 | 537 | PHE0006233_ 7220 | 4 | corn bchl | 88 | gi\|70905055\| | gb\|AAZ14053.1\|magnesiu m chelatase subunit I precursor [zea mays] |
| 180 | 538 | PHE0006234_ 7281 | 4 | bchD -Mg Chelatase | 87 | gi\|30680676\| | ref\|NP_563821.2\|PDE166 ; magnesium chelatase/ nucleoside-triphosphatase/ nucleotide binding [Arabidopsis thaliana] |
| 181 | 539 | PHE0006254_ 7312 | 1 | glycosyl hydroxylase | 90 | gi\|15238600\| | ref\|NP_198423.1\|unknown protein [Arabidopsis thaliana] |
| 182 | 540 | PHE0006263_ 7271 | 9 | OsDGAT2 | 95 | gi\|50912089\| | ref\|XP_467452.1\|putative mono- or diacylglycerol acyltransferase [Oryza sativa (japonica cultivar-group)] |

EP 2 540 831 A2

(continued)

| nuc seq ID NO | pep seq ID NO | Gene ID | BV id | Gene Name | Annotation | | |
|---|---|---|---|---|---|---|---|
| | | | | | % id | GenBank id | desciption |
| 183 | 541 | PHE0006264_ 7285 | 9 | NcDGAT2 | 100 | gi\|38567182\| | emb\|CAE76475.1\|related to diacylglycerol acyltransferase type 2a [Neurospora crassa] |
| 184 | 542 | PHE0006265_ 7990 | 19 | Arabidopsis thaliana cultivar Col-0 heme oxygenase 1 (HO1) gene, | 100 | gi\|15225771\| | ref\|NP_180235.\|HY1 (HEME OXYGENASE 1) [Arabidopsis thaliana] |
| 185 | 543 | PHE0006281_ 7526 | 7 | AtETR | 95 | gi\|30697334\| | ref\|NP_176808.3\|ETR1 (ETHYLENE RESPONSE 1); two-component response regulator [Arabidopsis thaliana] |
| 186 | 544 | PHE0006286_ 7314 | 1 | Arabidopsis allene oxide synthase (AOS) / hydroperoxide dehydrase / cyt | 97 | gi\|15239032\| | ref\|NP_199079.1 \|AOS (ALLENE OXIDE SYNTHASE); hydro-lyase/ oxygen binding [Arabidopsis thaliana] |
| 187 | 545 | PHE0006286_ 8011 | 19 | Arabidopsisallene oxide synthase (AOS) / hydroperoxide dehydrase / cyt | 97 | gi\|15239032\| | ref\|NP_199079.1\|AOS (ALLENE OXIDE SYNTHASE); hydro-lyase/ oxygen binding [Arabidopsis thaliana] |
| 188 | 546 | PHE0006288_ 7310 | 1 | Arabidopsis unknown protein | 80 | gi\|15219363\| | ref\|NP_173123.1\| unknown protein [Arabidopsis thaliana] |
| 189 | 547 | PHE0006288_ 8023 | 19 | Arabidopsis unknown protein | 80 | gi\|15219363\| | ref\|NP_173123.1\|unknown protein [Arabidopsis thaliana] |
| 190 | 548 | PHE0006296_ 7515 | 9 | EEM9 | 87 | gi\|63087722\| | emb\|CA193176.1\|glycosyl transferase [Zea mays] |
| 191 | 549 | PHE0006309_ 7570 | 4 | E. coli Glyoxalase 1 | 100 | gi\|24052010\| | gb\|AAN43259.1\|lactoylgl utathione lyase [Shigella flexneri 2a str. 301] |
| 192 | 550 | PHE0006309_ 8148 | 19 | E. coli Glyoxalase 1 | 100 | gi\|24052010\| | gb\|AAN43259.1\|lactoylgl utathione lyase [Shigella flexneri 2a str. 301] dbj\|BAE76494.1\| glyoxalase 1, Ni-dependent [Escherichia coli W3110] |

| nuc seq ID NO | pep seq ID NO | Gene ID | BV id | Gene Name | Annotation | | |
|---|---|---|---|---|---|---|---|
| | | | | | % id | GenBank id | desciption |
| 193 | 551 | PHE0006309_ 8620 | 19 | E. coli Glyoxalase I | 100 | gi\|240520101\| | gb\|AAN43259.1\|lactoylgl utathione lyase [Shigella flexneri 2a str. 301] dbj\|BAE76494.1\| glyoxalase 1, Ni-dependent [Escherichia coli W3110] |
| 194 | 552 | PHE0006310_ 7574 | 12 | rice BWMK1 | 95 | gi\|6689924\| | gb\|AAF23902.1\|MAP kinase homolog [Oryza sativa] |
| 195 | 553 | PHE0006311_ 7976 | 19 | AtRrp4p | 96 | gi\|15218790\| | ref\|NP_171835.1\|RNA binding / exonuclease [Arabidopsis thaliana] |
| 196 | 554 | PHE0006312_ 7579 | 4 | yeast Nip7p | 100 | gi\|45270008\| | ref\|NP_015113.1\| Nucleolar protein required for 60S ribosome subunit biogenesis, constituent of 66S pre-ribosomal particles; physically interacts with Nop8p and the exosome subunit Rrp43p; Nip7p [Saccharomyces cerevisiae] |
| 197 | 555 | PHE0006312_ 8644 | 19 | yeast Nip7p | 100 | gi\|45270008\| | ref\|NP_015113.1\| Nucleolar protein required for 60S ribosome subunit biogenesis, constituent of 66S pre-ribosomal particles; physically interacts with Nop8p and the exosome subunit Rrp43p; Nip7p [Saccharomyces cerevisiae] |
| 198 | 556 | PHE0006342_ 8182 | 19 | 1 | 91 | gi\|6320905\| | ref\|NP_010984.1\|One of two redundant DL-glycerol-3-phosphatases (RHR2/GPP1 encodes the other) involved in glycerol biosynthesis; induced in response to hyperosmotic stress and oxidative stress, and during the diauxic transition; Hor2p [Saccharomyces, cerevisiae] |

| nuc seq ID NO | pep seq ID NO | Gene ID | BV id | Gene Name | Annotation | | |
|---|---|---|---|---|---|---|---|
| | | | | | % id | GenBank id | desciption |
| 199 | 557 | PHE0006344_ 8188 | 19 | SIB1 (SIGMA FACTOR BINDING PROTEIN 1); binding | 89 | gi\|15228994\| | ref\|NP_191230.1\|SIB1 (SIGMA FACTOR BINDING PROTEIN 1); binding [Arabidopsis thaliana] |
| 200 | 558 | PHE0006346_ 8132 | 19 | | 100 | gi\|18410491\| | ref\|NP_565076.1\|unknown protein [Arabidopsis thaliana] |
| 201 | 559 | PHE0006348_ 8203 | 19 | antiporter/ glucose-6-phosphate transporte | 92 | gi\|18407336\| | ref\|NP_564785.1\|antiporter/ glucose-6-phosphate transporter [Arabidopsis thaliana] |
| 202 | 560 | PHE0006349_ 8204 | 19 | | 100 | gi\|45270642\| | sp\|P40001\|YEA8_YEAST Hypothetical 14.0 kDa protein in GCN4-WBP 1 intergenic region |
| 203 | 561 | PHE0006351_ 8200 | 19 | rice hypothetical protein | 58 | gi\|34897644\| | ref\|NP_910168.1\|hypothet ical protein [Oryza sativa] |
| 204 | 562 | PHE0006353_ 8098 | 19 | | 91 | gi\|18418660\| | ref\|NP_567982.1\|unknown protein [Arabidopsis thaliana] |
| 205 | 563 | PHE0006355_ 8084 | 19 | Arabidopsis unknown protein | 84 | gi\|18403871\| | ref\|NP_564601.1\|unknown protein [Arabidopsis thaliana] |
| 206 | 564 | PHE0006356_ 8103 | 19 | | 78 | gi\|15240413\| | ref\|NP_198048.1\|unknown protein [Arabidopsis thaliana] |
| 207 | 565 | PHE0006377_ 7592 | 4 | Saccharomyces cerevisiae Rrp44p | 91 | gi\|14588945\| | emb\|CAC42984.1 \|rRNA processing protein [Saccharomyces |
| | | | | | | | cerevisiae] |
| 208 | 566 | PHE0006377_ 8683 | 19 | Saccharomyces cerevisiae Rrp44p | 91 | gi\|14588945\| | sp\|P25359\|RRP43_YEAST Exosome complex exonuclease RRP43 (Ribosomal RNA-processing protein 43) |
| 209 | 567 | PHE0006378_ 7667 | 4 | Saccharomyces cerevisiae Rrp40p | 94 | gi\|51013303\| | gb\|AAT92945.1\|YOL142 W [Saccharomyces cerevisiae] |
| 210 | 568 | PHE0006378_ 8715 | 19 | Saccharomyces cerevisiae Rrp40p | 94 | gi\|51013303\| | gb\|AAT92945.1\|YOL142 W [Saccharomyces cerevisiae] |

| nuc seq ID NO | pep seq ID NO | Gene ID | BV id | Gene Name | Annotation | | |
|---|---|---|---|---|---|---|---|
| | | | | | % id | GenBank id | desciption |
| 211 | 569 | PHE0006380_ 7658 | 4 | Saccharomyces cerevisiae Rrp46p | 100 | gi|1323143| | sp|P53256|RRP46_YEAST Exosome complex exonuclease RRP46 (Ribosomal RNA-processing protein 46) |
| 212 | 570 | PHE0006380_ 8719 | 19 | Saccharomyces cerevisiae Rrp46p | 100 | gi|1323143| | sp|P53256|RRP46_YEAST Exosome complex exonuclease RRP46 (Ribosomal RNA-processing protein 46) |
| 213 | 571 | PHE0006381_ 7655 | 4 | Saccharomyces cerevisiae mtr3p | 88 | gi|1045263| | ref|NP_011674.1|3'5' exoribonuclease, exosome subunit; nucleolar protein involved in export of mRNA and ribosomal subunits; homologous to the E. coli exonuclease RNase PH; Mtr3p [Saccharomyces cerevisiae] |
| 214 | 572 | PHE0006381_ 8695 | 19 | Saccharomyces cerevisiae mtr3p | 88 | gi|1045263| | ref|NP_011674.1|3'5' exoribonuclease, exosome subunit; nucleolar protein involved in export of mRNA and ribosomal subunits; homologous to the E. coli exonuclease RNase PH; Mtr3p [Saccharomyces cerevisiae] |
| 215 | 573 | PHE0006382_ 7652 | 4 | PHE0006382_Sacchar omyces cerevisiae SK18 | 100 | gi|6321225| | ref|NP_011302.1|Protein involved in exosome mediated 3' to 5' mRNA degradation and translation inhibition of non-poly(A) mRNAs as well as double-strand break formation during meiotic recombination; required for repressing |
| | | | | | | | propagation of dsRNA viruses; Ski8p [Saccharomyces cerevisiae] |

| nuc seq ID NO | pep seq ID NO | Gene ID | BV id | Gene Name | Annotation | | |
|---|---|---|---|---|---|---|---|
| | | | | | % id | GenBank id | desciption |
| 216 | 574 | PHE0006382_ 8.678 | 19 | Saccharomyces cerevisiae SKI8 | 100 | gi\|6321225\| | ref\|NP_011302.1\|Protein involved in exosome mediated 3' to 5' mRNA degradation and translation inhibition of non-poly(A) mRNAs as well as double-strand break formation during meiotic recombination; required for repressing propagation of dsRNA viruses; Ski8p [Saccharomyces cerevisiae] |
| 217 | 575 | PHE0006425_ 7646 | 4 | corn CAT2 | 86 | gi\|77556625\| | gb\|ABA99421.1\|Amino acid permease [Oryza sativa (japonica cultivar-group)] |
| 218 | 576 | PHE0006426_ 8056 | 19 | corn CATS | 76 | gi\|50881438\| | gb\|AAT85283.1\|amino acid permease domain containing protein [Oryza sativa (japonica cultivar-group)] |
| 219 | 577 | PHE0006428_ 7651 | 4 | Oryza sativa hemoglobin 2 | 100 | gi\|34902308\| | ref\|NP_912500.1\|Putative non-symbiotic hemoglobin 2 (rHb2) [Oryza sativa (japonica cultivar-group)] |
| 220 | 578 | PHE0006429_ 7671 | 4 | Oryza sativa hemoglobin 1 | 100 | gi\|50920543\| | ref\|XP_470632.1\|Putative Non-symbiotic hemoglobin 1 [Oryza sativa (japonica cultivar-group)] |
| 221 | 579 | PHE0006433_ 8307 | 19 | Pseudouridine synthase | 90 | gi\|56744228\| | ref\|NP_190794.2\| RNA binding / pseudouridine synthase/ pseudouridylate synthase [Arabidopsis thaliana] |
| 222 | 580 | PHE0006439_ 8108 | 19 | corn putative RNA-binding protein | 56 | gi\|15231311\| | ref\|NP_190188.1\|RNA binding / nucleic acid binding [Arabidopsis thaliana] |
| 223 | 581 | PHE0006449_ 7865 | 1 | dihydrolipoamide acetyltransferase | 90 | gi\|17741871\| / | ref\|NP_533968.1\| dihydrolipoamide acetyltransferase [Agrobacterium tumefaciens str. C58] |

| nuc seq ID NO | pep seq ID NO | Gene ID | BV id | Gene Name | Annotation | | |
|---|---|---|---|---|---|---|---|
| | | | | | % id | GenBank id | desciption |
| 224 | 582 | PHE0006449_ 8165 | 19 | lipoamide acyltransferase component of branched-chain alpha-keto acid dehydrogenase complex E2 | 90 | gi\|17741871\| | gb\|AAL44284.1\|lipoamid e acyltransferase component of branched-chain alpha-keto acid dehydrogenase complex E2 [Agrobacterium tumefaciens str. C58] |
| 225 | 583 | PHE0006450_ 7624 | 1 | FtsZ1 | 83 | gi\|7672161\| | emb\|CAB89287.1\|chlorop last FtsZ-like protein [Nicotiana tabacum] |
| 226 | 584 | PHE0006464_ 8089 | 19 | corn unnamed protein product | 56 | gi\|34904362\| | dbj\|BAA96588.1\| plasma membrane polypeptide -like [Oryza sativa (japonica cultivar-group)] sp\|P83649\|SRS1_ORYSA Salt-stress root protein RS1 |
| 227 | 585 | PHE0006468_ 7903 | 1 | | 100 | gi\|18401231\| | ref\|NP_566558.1\|unknown protein [Arabidopsis thaliana] |
| 228 | 586 | PHE0006477_ 7809 | 17 | AtPsbR | 81 | gi\|15219268\| | sp\|P27202\|PSBR_ARATH Photosystem II 10 kDa polypeptide, chloroplast precursor |
| 229 | 587 | PHE0006478_ 8190 | 19 | adenosylmethionine:2-demethylmenaquinone methyltransferase-like protein | 94 | gi\|34910724\| | ref\|NP_916709.1\|S-adenosylmethionine: 2-demethylmenaquinone methyltransferase-like protein [Oryza sativa (japonica cultivar-group)] |
| 230 | 588 | PHE0006497_ 8355 | 19 | Arabidopsis unknown protein | 72 | gi\|15238921\| | ref\|NP_196661.1\|unknown protein [Arabidopsis thaliana] |
| 231 | 589 | PHE0006498_ 7795 | 18 | soy Glutamate Decarboxylase | 100 | gi\|32493114\| | gb\|AAP85548.1\|putative glutamate decarboxylase [Glycine max] |
| 232 | 590 | PHE0006498_ 7796 | 2 | soy Glutamate Decarboxylase | 100 | gi\|32493114\| | gb\|AAP85548.1\|putative glutamate decarboxylase [Glycine max] |
| 233 | 591 | PHE0006505_ 7871 | 1 | soy Thioredoxin X | 68 | gi\|18403021\| | ref\|NP_564566.1\|ATHX; electron transporter/ thiol-disulfide exchange intermediate [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 234 | 592 | PHE0006506_ 7818 | 1 | Arabidopsis SRK2C like | 100 | gi\|42572557\| | ref\|NP_974374.1\|AKIN11 ; protein kinase [Arabidopsis thaliana] |
| 235 | 593 | PHE0006514_ 7926 | 10 | Truncated Beta GDH | 99 | gi\|18273\| | emb\|CAA41636.1\|glutamate dehydrogenase (NADP+) [Chlorella sorokiniana] |
| 236 | 594 | PHE0006516_ 7866 | 17 | Corn Magnesium transporter | 80 | gi\|78708975\| | gb\|ABB47950.1\|CorA-like Mg2+ transporter protein, putative [Oryza sativa (japonica cultivar-group)] |
| 237 | 595 | PHE0006516_ 7882 | 8 | Corn Magnesium transporter | 80 | gi\|78708975\| | gb\|ABB47950.1\|CorA-like Mg2+ transporter protein, putative [Oryza sativa (japonica cultivar-group)] |
| 238 | 596 | PHE0006516_ 7887 | 16 | Corn Magnesium transporter | 80 | gi\|78708975\| | gb\|ABB47950.1\|CorA-like Mg2+ transporter protein, putative [Oryza sativa (japonica cultivar-group)] |
| 239 | 597 | PHE0006516_ 8363 | 19 | Corn Magnesium transporter | 80 | gi\|78708975\| | gb\|ABB47950.1\|CorA-like Mg2+ transporter protein, putative [Oryza sativa (japonica cultivar-group)] |
| 240 | 598 | PHE0006517_ 7858 | 16 | Rice Magnesium transporter | 95 | gi\|78708975\| | gb\|ABB47950.1\|CorA-like Mg2+ transporter protein, putative [Oryza sativa (japonica cultivar-group)] |
| 241 | 599 | PHE0006517_ 7879 | 17 | Rice Magnesium transporter | 95 | gi\|78708975\| | gb\|ABB47950.1\|CorA-like Mg2+ transporter protein, putative [Oryza sativa (japonica cultivar-group)] |
| 242 | 600 | PHE0006517_ 7897 | 8 | Rice Magnesium transporter | 95 | gi\|78708975\| | gb\|ABB47950.1\|CorA-like Mg2+ transporter protein, putative [Oryza sativa (japonica cultivar-group)] |
| 243 | 601 | PHE0006521_ 7840 | 15 | Anabaena SPP | 100 | gi\|4594809\| | emb\|CAC43285.1\|sucrose -phosphate phosphatase [Nostoc sp. PCC 7120] |
| 244 | 602 | PHE0006545_ 8320 | 9 | ARG1-like protein | 79 | gi\|51535811\| | dbj\|BAD37896.1\|ARG1-like protein [Oryza sativa (japonica cultivar-group)] |

(continued)

| nuc seq ID NO | pep seq ID NO | Gene ID | BV id | Gene Name | % id | GenBank id | Annotation desciption |
|---|---|---|---|---|---|---|---|
| 245 | 603 | PHE0006549_8255 | 19 | methylenetetrahydrofo late dehydrogenase (NADP+) | 83 | gi|54291831| | gb|AAV32199.1|unknown protein [Oryza sativa (japonica cultivar-group)] |
| 246 | 604 | PHE0006555_8283 | 19 | G3PB_PEA Glyceraldehyde 3-phosphate dehydrogenase B, chloroplast | 84 | gi|50948907| | ref|XP_493811.1|EST C74302(E30840) corresponds to a region of the predicted gene.~similar to glyceraldehyde-3-phosphate dehydrogenase. (M64118) [Oryza sativa (japonica cultivar-group)] |
| 247 | 605 | PHE0006559_8227 | 16 | phosphoenolpyruvate carboxylase | 98 | gi|34904868| | ref|NP_913781.1|phospho enolpyruvate carboxylase [Oryza sativa (japonica cultivar-group)] |
| 248 | 606 | PHE0006564_8298 | 17 | comasparagine synthetase 2 | 95 | gi|28395526| | gb|AAO39048.1|asparagine synthetase 2 [Hordeum vulgare] |
| 249 | 607 | PHE0006565_8300 | 17 | corn glutamine-dependent asparagines synthetase | 97 | gi|53680379| | gb|AAU89392.1|glutamin e-dependentasparagine synthetase [Triticum aestivum] |
| 250 | 608 | PHE0006571_8279 | 19 | Arabidopsis thaliana phosphoenolpyruvate carboxylase kinase | 95 | gi|15223870| | gb|AAN12902.1|putative calcium-dependent protein kinase [Arabidopsis thaliana] |
| 251 | 609 | PHE0006574_8224 | 19 | Glyoxalase I | 100 | gi|984219| | sp|Q09751|LGUL_SCHP O Lactoylglutathione lyase (Methylglyoxalase) (Aldoketomutase) (Glyoxalase I) |
| 252 | 610 | PHE0006586_8271 | 19 | Arabidopsis mitochondrial frataxin-like | 83 | gi|51860727| | gb|AAU11485.1|mitochon drial frataxin-like [Arabidopsis thaliana] |
| 253 | 611 | PHE0006587_8277 | 19 | Arabidopsis CP12 domain-containing protein | 100 | gi|15228752| | gb|AAM45071.1| putative CP12 protein precursor [Arabidopsis thaliana] |
| 254 | 612 | PHE0006590_8258 | 19 | Arabidopsis thioredoxin M-type 1 | 89 | gi|30678634| | ref|NP_849585.1|ATHM1 ; electron transporter/ thiol-disulfide exchange intermediate [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 255 | 613 | PHE0006591_ 8264 | 19 | Arabidopsis thioredoxin M-type 2 | 94 | gi\|15236327\| | ref\|NP_192261.1\|ATHM2 ; electron transporter/ thiol-disulfide exchange intermediate [Arabidopsis thaliana] |
| 256 | 614 | PHE0006592_ 8278 | 19 | thioredoxin M-type 4 | 80 | gi\|15232567\| | ref\|NP_188155.1\|ATHM4 ; electron transporter/ thiol-disulfide exchange intermediate [Arabidopsis thaliana] |
| 257 | 615 | PHE0006593_ 8245 | 1 | Arabidopsis putative protein | 100 | gi\|15236013\| | ref\|NP_193460.1\|unknown protein [Arabidopsis thaliana] |
| 258 | 616 | PHE0006593_ 8256 | 19 | Arabidopsisputative protein | 100 | gi\|15236013\| | ref\|NP_193460.1\|unknown protein [Arabidopsis thaliana] |
| 259 | 617 | PHE0006595_ 8250 | 1 | Arabidopsis unknown protein | 100 | gi\|18417658\| | ref\|NP_567853.1\|unknown protein [Arabidopsis thaliana] |
| 260 | 618 | PHE0006595_ 8265 | 19 | Arabidopsis unknown protein | 100 | gi\|18417658\| | ref\|NP_567853.1\|unknown protein [Arabidopsis thaliana] |
| 261 | 619 | PHE0006596_ 8236 | 1 | Arabidopsis hypothetical protein | 93 | gi\|15224138\| | ref\|NP_179417.1\|nucleoti dyltransferase [Arabidopsis thaliana] |
| 262 | 620 | PHE0006596_ 8257 | 19 | Arabidopsis hypothetical protein | 93 | gi\|15224138\| | ref\|NP_179417.1\|nucleoti dyltransferase [Arabidopsis thaliana] |
| 263 | 621 | PHE0006597_ 8242 | 1 | Arabidopsis putative serine/ threonine protein kinase | 94 | gi\|22330852\| | ref\|NP_187165.2\|ATP binding / kinase/ protein kinase/ protein serine/threonine kinase/ protein-tyrosine kinase [Arabidopsis thaliana] |
| 264 | 622 | PHE0006598_ 8240 | 1 | Arabidopsis drought-responsive family protein | 77 | gi\|22137252\| | gb\|AAM91471.1\|AT3g06 760/F3 E22_10 [Arabidopsis thaliana] gb\|AAL67123.1\| AT3g06760/F3E22_10 [Arabidopsis thaliana] |
| 265 | 623 | PHE0006598_ 8268 | 19 | Arabidopsis drought-responsive family protein | 77 | gi\|22137252\| | gb\|AAM91471.1\|AT3g06 760/F3E22_10 [Arabidopsis thaliana] |
| 266 | 624 | PHE0006599_ 8230 | 1 | Arabidopsis zinc finger homeobox family protein | 79 | gi\|18410665\| | ref\|NP_565088.1\|transcrip tion factor [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 267 | 625 | PHE0006599_ 8262 | 19 | Arabidopsis zinc finger homeobox family protein | 79 | gi|18410665| | ref|NP_565088.1|transcrip tion factor [Arabidopsis thaliana] |
| 268 | 626 | PHE0006600_ 8249 | 1 | Xenorhabdus putative tartrate dehydrogenase | 83 | gi|75208732| | ref|ZP_00709024.1|COG0 473: Isocitrate/isopropylmalate dehydrogenase [Escherichia coli B171] |
| 269 | 627 | PHE0006607_ 8231 | 1 | Arabidopsis MADS-box family protein | 92 | gi|15219825| | ref|NP_176285.1|AGL56; DNA binding / transcription factor [Arabidopsis thaliana] |
| 270 | 628 | PHE0006609_ 8234 | 1 | Soy Glutathione Peroxidase | 71 | gi|18407538| | ref|NP_566128.1|ATGPX 4 (GLUTATHIONE PEROXIDASE 4); glutathione peroxidase [Arabidopsis thaliana] |
| 271 | 629 | PHE0006610_ 8239 | 1 | Soy Glutathione Peroxidase | 75 | gi|2632109| | emb|CAA0414.1|phosph olipid glutathione peroxidase [Pisum sativum] |
| 272 | 630 | PHE0006613_ 8238 | 1 | Soy Glutathione Peroxidase | 64 | gi|11544696| | emb|CAC17628.1|putative phospholipid hydroperoxide glutathione peroxidase [Oryza sativa (japonica cultivar-group)] |
| 273 | 631 | PHE0006617_ 8463 | 19 | corn oxalate oxidase | 86 | gi|6996619| | gb|AAF34811.1|oxalate oxidase [Triticum aestivum] |
| 274 | 632 | PHE0006620_ 8462 | 19 | corn NADPH-dependent reductase | 96 | gi|78172239| | gb|ABB29303.1|NADPH-dependent reductase [Zea mays] |
| 275 | 633 | PHE0006648_ 8356 | 19 | corn putative protease inhibitor | 44 | gi|50919133| | ref|XP_469963.1|putative protease inhibitor [Oryza sativa (japonica cultivar-group)] |
| 276 | 634 | PHE0006666_ 8414 | 19 | corn putative plastidic aldolase | 93 | gi|34895322| | ref|NP_909004.1|putative plastidic aldolase [Oryza sativa (japonica cultivar-group)] |
| 277 | 635 | PHE0006669_ 8357 | 14 | Schizosaccharomyces pombe ATP-PFK | 97 | gi|2956754| | sp|O42938|K6PF_SCHPO 6-phosphofructokinase (Phosphofructokinase) (Phosphohexokinase) (6PF-I-K) |

| nuc seq ID NO | pep seq ID NO | Gene ID | BV id | Gene Name | Annotation | | |
|---|---|---|---|---|---|---|---|
| | | | | | % id | GenBank id | desciption |
| 278 | 636 | PHE0006670_ 8346 | 21 | E. coli ATP-dependent phosphofructokinase B with CTP2 | 100 | gi\|85675091\| | dbj\|BAA15500.2\|6-phosphofructokinase II [Escherichia coli W3110] |
| 279 | 637 | PHE0006673_ 8992 | 17 | Arabidopsis peptide transporter | 88 | gi\|18409391\| | ref\|NP_564979.1\|transporter [Arabidopsis thaliana] |
| 280 | 638 | PHE0006676_ 8410 | 19 | corn pyruvate dehydrogenase E1 beta subunit | 94 | gi\|3850999\| | gb\|AAC72192.1\|pyruvate dehydrogenase E1 beta subunit isoform I [Zea mays] |
| 281 | 639 | PHE0006684_ 8413 | 19 | corn probable 60S acidic ribosomal protein | 88 | gi\|77548268\| | gb\|ABA91065.1\|ribosoma I protein L10, putative [Oryza sativa (japonica cultivar-group)] |
| 282 | 640 | PHE0006685_ 8415 | 19 | corn Ribosomal protein L1p/L10e family | 63 | gi\|50932757\| | ref\|XP_475906.1\|unknown protein [Oryza sativa (japonica cultivar-group)] |
| 283 | 641 | PHE0006686_ 8416 | 19 | corn ribosomal protein L17.2, cytosolic | 98 | gi\|3914685\| | sp\|O48557\|RL17_MAIZE 60S ribosomal protein L17 gb\|AAB88619.1\| ribosomal protein L17 [Zea mays] |
| 284 | 642 | PHE0006687_ 8471 | 19 | corn ribosomal protein L32-like protein | 98 | gi\|15236757\| | sp\|P49211\|RL32A_ARAT H 60S ribosomal protein L32-1 |
| 285 | 643 | PHE0006706_ 8434 | 1 | soy PDH45 (DNA helicase 45) | 100 | gi\|15223841\| | ref\|NP_175549.1\|ATP binding / ATP-dependent helicase/ helicase/ nucleic acid binding [Arabidopsis thaliana] |
| 286 | 644 | PHE0006709_ 8432 | 1 | Corn protein similar to nodulin Mt N3 Protein | 80 | gi\|34912462\| | ref\|NP_917578.1\|MtN3-like protein [Oryza sativa (japonica cultivar-group)] dbj\|BAB92465.1\|senescence-associated protein-like [Oryza sativa (japonica cultivar-group)] |
| 287 | 645 | PHE0006715_ 8477 | 1 | AKIN beta2 | 100 | gi\|56744220\| | sp\|Q9SCY5\|K1NB2_ARA TH SNF1-related protein kinase regulatory beta subunit 2 (AKIN beta2) (AKINB2) |

(continued)

| nuc seq ID NO | pep seq ID NO | Gene ID | BV id | Gene Name | Annotation | | |
|---|---|---|---|---|---|---|---|
| | | | | | % id | GenBank id | desciption |
| 288 | 646 | PHE0006716_ 8482 | 1 | putative nitrate-induced NOI protein | 78 | gi\|20465673\| | gb\|AAM20305.1\|putative nitrate-induced NOI protein [Arabidopsis thaliana] |
| 289 | 647 | PHE0006727_ 8435 | 1 | corn NADH-ubiquinone oxidoreductase-related-like protein | 91 | gi\|50509945\| | dbj\|BAD30267.1\|NADH-ubiquinone oxidoreductase-related-like protein [Oryza sativa (japonica cultivar-group)] |
| 290 | 648 | PHE0006727_ 8595 | 19 | NADH-ubiquinone oxidoreductase-related-like protein | 91 | gi\|50509945\| | dbj\|BAD30267.1\|NADH-ubiquinone oxidoreductase-related-like protein [Oryza sativa (japonica cultivar-group)] |
| 291 | 649 | PHE0006728_ 8430 | 1 | Arabidopsis RNA recognition motif (RRM)-containing protein | 95 | gi\|15231193\| | ref\|NP_190149.1\|RNA binding / nucleic acid binding / ubiquitin-protein ligase/ zinc ion binding [Arabidopsis thaliana] |
| 292 | 650 | PHE0006729_ 8433 | 1 | corn DNAJ heat shock N-terminal domain-containing protein-like | 67 | gi\|51536221\| | dbj\|BAD38392.1\|DNAJ heat shock N-terminal domain-containing protein-like [Oryza sativa (japonica cultivar-group)] |
| 293 | 651 | PHE0006730_ 8428 | 1 | corn membrane protein PTM 1-like | 77 | gi\|51091402\| | dbj\|BAD36145.1\|membrane protein PTM1-like [Oryza sativa (japonica cultivar-group)] |
| 294 | 652 | PHE0006737_ 8455 | 1 | Arabidopsis putative oxidoreductase | 100 | gi\|15221544\| | gb\|AAK64077.1\|putative oxidoreductase [Arabidopsis thaliana] |
| 295 | 653 | PHE0006737_ 8527 | 19 | Arabidopsis putative oxidoreductase | 100 | gi\|15221544\| | gb\|AAK25895.1\|putative oxidoreductase [Arabidopsis thaliana] |
| 296 | 654 | PHE0006740_ 8446 | 1 | corn unknown protein | 70 | gi\|50931847\| | ref\|XP_475451.1\|unknown protein [Oryza sativa (japonica cultivar-group)] |
| 297 | 655 | PHE0006740_ 8596 | 19 | corn unknown protein | 70 | gi\|50931847\| | ref\|XP_475451.1\|unknown protein [Oryza sativa (japonica cultivar-group)] |
| 298 | 656 | PHE0006741_ 8448 | 1 | Arabidopsis unknown protein | 90 | gi\|30678912\| | ref\|NP_566212.2\|ATBPM 4; protein binding [Arabidopsis thaliana] |
| 299 | 657 | PHE0006741_ 8589 | 19 | Arabidopsis unknown protein | 90 | gi\|30678912\| | ref\|NP_566212.2\|ATBPM 4; protein binding [arabidopsis thaliana] |

EP 2 540 831 A2

| nuc seq ID NO | pep seq ID NO | Gene ID | BV id | Gene Name | Annotation | | |
|---|---|---|---|---|---|---|---|
| | | | | | % id | GenBank id | desciption |
| 300 | 658 | PHE0006742_ 8440 | 1 | Pseudomonas fluorescens Glucose-6-phosphate isomerase | 98 | gi\|77385037\| | ref\|YP_350541.1\| glucose-6-phosphate isomerase [Pseudomonas fluorescens PfO-1] |
| 301 | 659 | PHE0006742_ 8591 | 19 | Pseudomonas fluorescens Glucose-6-phosphate isomerase | 98 | gi\|77385037\| | gb\|ABA76550.1\|Glucose-6-phosphate isomerase [Pseudomonas fluorescens PfO-1] |
| 302 | 660 | PHE0006744_ 8449 | 1 | Arabidopsis ribitol dehydrogenase-like | 95 | gi\|18423110\| | ref\|NP_568721.1\|oxidored uctase [Arabidopsis thaliana] gb\|AAM13036.1\|ribitol dehydrogenase-like [Arabidopsis thaliana] |
| 303 | 661 | PHE0006745_ 8590 | 19 | corn putative 28 kDa Golgi SNARE protein | 81 | gi\|50904959\| | ref\|XP_463968.1\|putative 28 kDa Golgi SNARE protein [Oryza sativa (japonica cultivar-group)] |
| 304 | 662 | PHE0006746_ 8453 | 1 | Arabidopsis CGPG6223 sugar-porter family protein 1 | 93 | gi\|18421106\| | ref\|NP_568493.1\|SFP1 (sugar-porter family protein 1); carbohydrate transporter/ sugar porter [Arabidopsis thaliana] |
| 305 | 663 | PHE0006750_ 8523 | 12 | Arabidopsis Cop 1 | 100 | gi\|15225760\| | ref\|NP_180854.1\|COP1 (CONSTITUTIVE PHOTOMORPHOGENIC 1) [Arabidopsis thaliana] |
| 306 | 664 | PHE0006757_ 8530 | 12 | Arabidopsis phospholipid/ glycerol acyltransferase family protein | 92 | gi\|18390661\| | ref\|NP_563768.1\|ATGPA T1/GPAT1; 1-acylglycerol-3-phosphate O-acyltransferase/ acyltransferase [Arabidopsis thaliana] |
| 307 | 665 | PHE0006760_ 8529 | 19 | Arabidopsis vacuolar ATP synthase subunit E / V-ATPase E subunit / vacuolar prot | 100 | gi\|15237054\| | ref\|NP_192853.1\|TUF (TUFF) [Arabidopsis thaliana] emb\|CAB81216.1\| H+-transporting ATPase chain E, vacuolar [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 308 | 666 | PHE0006765_ 8536 | 20 | Arabidopsis IMB I | 92 | gi\|30686240\| | ref\|NP_181036.2\|IMB1 (IMBIBITION-INDUCIBLE 1); DNA binding [Arabidopsis thaliana] |
| 309 | 667 | PHE0006766_ 8867 | 9 | AGRtu.Isopentyl transferase-0:2:1 | 94 | gi\|15163474\| | gb\|AAK90970.1\|AGR_pT i_50p [Agrobacterium tumefaciens str. C58] |
| 310 | 668 | PHE0006769_ 8865 | 14 | Pyruvate oxidase (POXB) | 97 | gi\|1651398\| | dbj\|BAA35585.1\|pyruvate dehydrogenase (pyruvate oxidase), thiamin-dependent, FAD-binding [Escherichia coli W3110] |
| 311 | 669 | PHE0006770_ 8553 | 19 | corn PDH45 | 99 | gi\|84322402\| | gb\|ABC55720.1\|putative RH2 protein [Zea mays] |
| 312 | 670 | PHE0006770_ 8568 | 13 | corn PDH45 | 99 | gi\|84322402\| | gb\|ABC55720.1\|putative RH2 protein [Zea mays] |
| 313 | 671 | PHE0006771_ 8551 | 19 | Arabidopsis HIC (High CO2 | 94 | gi\|15226428\| | gb\|AAC69929.1\| putative beta-ketoacyl-CoA synthase [Arabidopsis thaliana] |
| 314 | 672 | PHE0006775_ 8548 | 19 | RabG3e/Rab7 | 100 | gi\|15222098\| | ref\|NF_175355.1\|GTP binding [Arabidopsis thaliana] |
| 315 | 673 | PHE0006775_ 8555 | 13 | RabG3e/Rab7 | 100 | g\|15222098\| | ref\|NP_175355.1\|GTP binding [Arabidopsis thaliana] |
| 316 | 674 | PHE0006788_ 8581 | 13 | Lycopersicon esculentum non specific lipid transfer protein | 72 | gi\|71360930\| | emb\|CAJ19706.1\|non-specific lipid transfer protein [Lycopersicon esculentum] |
| 317 | 675 | PHE0006793_ 8580 | 13 | Arabidopsis cytochrome P450 | 97 | gi\|15236789\| | ref\|NP_191946.1\|CYP86 A2; oxygen binding [Arabidopsis thaliana] emb\|CAB80794.1\| probable cytochrome P450 [Arabidopsis thaliana] |
| 318 | 676 | PHE0006794_ 8578 | 13 | Arabidopsis single-strand-binding family protein | 100 | gi\|30681642\| | ref\|NP_192844.2\|single-stranded DNA binding [Arabidopsis thaliana] |
| 319 | 677 | PHE0006805_ 8531 | 12 | E.coli yfla | 100 | gi\|24053042\| | gb\|AAN44153.1\|putative yhbH sigma 54 modulator [Shigella flexneri 2a str. 301] |

| nuc seq ID NO | pep seq ID NO | Gene ID | BV id | Gene Name | Annotation | | |
|---|---|---|---|---|---|---|---|
| | | | | | % id | GenBank id | desciption |
| 320 | 678 | PHE0006811_ 8506 | 19 | Cyanoglobin | 91 | gi\|1653074\| | dbj\|BAA17991.1\|cyanogl obin [Synechocystis sp. PCC 6803] |
| 321 | 679 | PHE0006816_ 8560 | 19 | Corn HO2-Like | 71 | gi\|14485573\| | gb\|AAK63011.1\|heme oxygenase 2 [Sorghum bicolor] |
| 322 | 680 | PHE0006844_ 8839 | 22 | Arabidopsis RNA-binding protein-like protein | 100 | gi\|15232735\| | ref\|NP_190300.1\|ubiquiti n-protein ligase/ zinc ion binding [Arabidopsis thaliana] |
| 323 | 681 | PHE0006847_ 8860 | 19 | Agrobacterium 3,4-dihydroxy-2-butanone-4-phoshate synthase/GTP cyclohyd | 100 | gi\|17739122\| | gb\|AAL41769.1\|3,4-dihydroxy-2-butanone-4-phoshate synthase/GTP cyclohydrolase II [Agrobacterium tumefaciens str. C58] |
| 324 | 682 | PHE0006870_ 8846 | 19 | 60S ribosomal protein L37a | 100 | gi\|18411538\| | gb\|AAM65721.1\|60S ribosomal protein L37a [Arabidopsis thaliana] |
| 325 | 683 | PHE0006908_ 9016 | 19 | Thioredoxin_MON_Z M33301 | 67 | gi\|10178282\| | emb\|CAC08340.1\|putative protein [Arabidopsis thaliana] |
| 326 | 684 | PHE0006909_ 9003 | 19 | A1ZM000889_at_Cup in_3 | 75 | gi\|50924572\| | ref\|XP_472645.1\|OSJNBa 0027P08.10 [Oryza sativa (japonica cultivar-group)] |
| 327 | 685 | PHE0006910_ 9019 | 19 | A1ZM009835_at_Mo v34 | 75 | gi\|55773965\| | dbj\|BAD72492.1\|ALM beta-like [Oryza sativa (japonica cultivar-group)] |
| 328 | 686 | PHE0006912_ 9000 | 19 | A1ZM000998_a_at_p utative enoyl-CoA hydratase | 79 | gi\|50911385\| | ref\|XP_467100.1\|putative enoyl-CoA hydratase [Oryza sativa (japonica cultivar-group)] |
| 329 | 687 | PHE0006919_ 9008 | 19 | putative mitochondrial processing peptidase alpha subuunit, mitoch | 76 | gi\|57899480\| | dbj\|BAD86941.1\|putative mitochondrial processing peptidase [Oryza sativa (japonica cultivar-group)] |
| 330 | 688 | PHE0006929_ 9151 | 22 | COP1-interacting protein C1P8 | 46 | gi\|15238295\| | ref\|NF_201297.1\|C1P8 (COP 1-INTERACTING PROTEIN 8); protein binding / zinc ion binding [Arabidopsis thaliana] |

43

EP 2 540 831 A2

| nuc seq ID NO | pep seq ID NO | Gene ID | BV id | Gene Name | Annotation | | |
|---|---|---|---|---|---|---|---|
| | | | | | % id | GenBank id | desciption |
| 331 | 689 | PHE0006929_ 9185 | 19 | COP 1-interacting protein C1P8 | 46 | gi\|15238295\| | ref\|NP_201297.1\|C1P8 (COP1-INTERACTING PROTEIN 8); protein binding / zinc ion binding [Arabidopsis thaliana] |
| 332 | 690 | PHE0006931_ 9148 | 22 | glycine dehydrogenase subunit I | 93 | gi\|10175436\| | dbj\|BAB06534.1\|glycine dehydrogenase subunit 1 [Bacillus halodurans C-125] |
| 333 | 691 | PHE0006931_ 9168 | 19 | glycine dehydrogenase subunit 1 | 93 | gi\|10175436\| | dbj\|BAB06534.1\|glycine dehydrogenase subunit 1[Bacillus halodurans C-125] |
| 334 | 692 | PHE0006932_ 9147 | 22 | expressed protein | 100 | gi\|18406559\| | ref\|NP_566020.1\|unknown protein [Arabidopsis thaliana] |
| 335 | 693 | PHE0006932_ 9174 | 19 | Unknown protein | 100 | gi\|18406559\| | ref\|NP_566020.1\|unknown protein [Arabidopsis thaliana] |
| 336 | 694 | PHE0006933_ 9139 | 22 | short-chain dehydrogenase/ reducta se (SDR) family protein_CGPG5025 | 92 | gi\|30686197\| | ref\|NP_849428.1\|oxidored uctase [Arabidopsis thaliana] |
| 337 | 695 | PHE0006934_ 9145 | 22 | OsPol delta small subunit | 91 | gi\|9188572\| | dbj\|BAA99574.1\|OsPol delta small subunit [Oryza sativa (japonica cultivar-group)] |
| 338 | 696 | PHE0006937_ 9126 | 22 | putative leucine zipper protein | 77 | gi\|51535194\| | dbj\|BAD38167.1\|putative leucine zipper protein [Oryza sativa (japonica cultivar-group)] |
| 339 | 697 | PHE0006938_ 9149 | 22 | TPA: actin-related protein 8B | 98 | gi\|30696705\| | ref\|NP_568836.2\|ATARP 8 (ACTIN-RELATED PROTEIN 8); structural constituent of cytoskeleton [Arabidopsis thaliana] |
| 340 | 698 | PHE0006940_ 9122 | 22 | NADP-dependent glyceraldehyde-3-phosphate dehydrogenase | 100 | gi\|10174856\| | dbj\|BAB05956.1\|NADP-dependent glyceraldehyde-3-phosphate dehydrogenase [Bacillus halodurans C-125] |

| nuc seq ID NO | pep seq ID NO | Gene ID | BV id | Gene Name | Annotation | | |
|---|---|---|---|---|---|---|---|
| | | | | | % id | GenBank id | desciption |
| 341 | 699 | PHE0006941_ 9117 | 22 | Unknown protein | 90 | gi\|18401933\| | ref\|NP_564515.1\|unknown protein [Arabidopsis thaliana] |
| 342 | 700 | PHE0006943_ 9124 | 22 | amino acid transporter-like protein 2 | 94 | gi\|18395471\| | ref\|NP_564217.1\|AATL2 (AMINO ACID TRANSPORTER-LIKE PROTEIN 2); amino acid permease [Arabidopsis thaliana] |
| 343 | 701 | PHE0006948_ 9125 | 22 | Similar to glycine-rich RNA-binding proteins _CGPG4960 | 100 | gi\|15221825\| | ref\|NP_173298.1\|RNA binding / nucleic acid binding [Arabidopsis thaliana] |
| 344 | 702 | PHE0006948_ 9160 | 19 | glycine-rich RNA-binding proteins | 100 | gi\|15221825\| | ref\|NP_173298.1\|RNA binding / nucleic acid binding [Arabidopsis thaliana] |
| 345 | 703 | PHE0006949_ 9133 | 22 | PROBABLE SUCCINATE-SEMIALDEHYDE DEHYDROGENASE [NADP] PROTEIN | 97 | gi\|15072942\| | ref\|NP_384120.1\| PROBABLE SUCCINATE-SEMIALDEHYDE DEHYDROGENASE [NADP+] PROTEIN [Sinorhizobium meliloti 1021] |
| 346 | 704 | PHE0006949_ 9179 | 19 | PROBABLE SUCCINATE-SEMIALDEHYDE DEHYDROGENASE [NADP] PROTEIN | 97 | gi\|15072942\| | emb\|CAC41401.1\|PROB ABLE SUCCINATE-SEMIALDEHYDE DEHYDROGENASE [NADP+] PROTEIN [Sinorhizobium meliloti] |
| 347 | 705 | PHE0006952_ 9233 | 22 | Gpm1p with ctp | 95 | gi\|407495\| | ref\|NP_012770.1\| Tetrameric phosphoglycerate mutase, [Saccharomyces cerevisiae] |
| 348 | 706 | PHE0006953_ 9121 | 22 | universal stress protein (USP) family protein | 93 | gi\|30678807\| | ref\|NF_850506.1\|unknown protein [Arabidopsis thaliana] |
| 349 | 707 | PHE0006954_ 9154 | 22 | unnamed protein product | 80 | gi\|22327694\| | ref\|NP_680415.1\|unknown protein [Arabidopsis thaliana] |
| 350 | 708 | PHE0006954_ 9161 | 19 | unnamed protein product | 80 | gi\|22327694\| | ref\|NP_680415.1\|unknown protein [Arabidopsis thaliana] |

| nuc seq ID NO | pep seq ID NO | Gene ID | BV id | Gene Name | Annotation | | |
|---|---|---|---|---|---|---|---|
| | | | | | % id | GenBank id | desciption |
| 351 | 709 | PHE0006962_ 9114 | 15 | nitrate reductase | 94 | gi\|85675313\| | dbj\|BAA15989.2\|nitrate reductase, periplasmic, large subunit [Escherichia coli W3110] |
| 352 | 710 | PHE0006963_ 9131 | 15 | nitrite reductase | 97 | gi\|85676675\| | dbj\|BAE77925.1\|nitrite reductase, large subunit, NAD(P)H-binding [Escherichia coli W3110] |
| 353 | 711 | PHE0006965_ 9119 | 17 | glutaminyl-tRNA synthetase | 86 | gi\|77554943\| | gb\|ABA97739.1\|prolyl-tRNA synthetase [Oryza sativa (japonica cultivar-group)] |
| 354 | 712 | PHE0006970_ 9141 | 19 | DN A binding / transcription factor | 100 | gi\|15242227\| | ref\|NP_197020.1\|DNA binding / transcription factor [Arabidopsis thaliana] |
| 355 | 713 | PHE0006977_ 9163 | 19 | ribulose-phosphate 3-epimerase | 96 | gi\|15221735\| | ref\|NP_176518.1\|ribulose-phosphate 3-epimerase [Arabidopsis thaliana] |
| 356 | 714 | PHE0006986_ 9183 | 19 | Unknown protein | 82 | gi\|50932819\| | ref\|XP_475937.1\|unknown protein [Oryza sativa (japonica cultivar-group)] |
| 357 | 715 | PHE0006992_ 9140 | 22 | unknown protein | 93 | gi\|15234800\| | ref\|NP_194792.1\|unknown protein [Arabidopsis thaliana] |
| 358 | 716 | PHE0006992_ 9184 | 19 | Unknown protein | 93 | gi\|15234800\| | ref\|NP_194792.1\|unknown protein [Arabidopsis thaliana] |

**Selection methods for transgenic plants with enhanced agronomic trait**

Within a population of transgenic plants regenerated from plant cells transformed with the recombinant DNA many plants that survive to fertile transgenic plants that produce seeds and progeny plants will not exhibit an enhanced agronomic trait. Selection from the population is necessary to identify one or more transgenic plant cells that can provide plants with the enhanced trait. Transgenic plants having enhanced traits are selected from populations of plants regenerated or derived from plant cells transformed as described herein by evaluating the plants in a variety of assays to detect an enhanced trait, e.g. enhanced water use efficiency, enhanced cold tolerance, increased yield, enhanced nitrogen use efficiency, enhanced seed protein and enhanced seed oil. These assays also may take many forms including, but not limited to, direct screening for the trait in a greenhouse or field trial or by screening for a surrogate trait. Such analyses can be directed to detecting changes in the chemical composition, biomass, physiological properties, morphology of the plant. Changes in chemical compositions such as nutritional composition of grain can be detected by analysis of the seed composition and content of protein, free amino acids, oil, free fatty acids, starch or tocopherols. Changes in biomass characteristics can be made on greenhouse or field grown plants and can include plant height, stem diameter, root and shoot dry weights; and, for corn plants, ear length and diameter. Changes in physiological properties can be identified by evaluating responses to stress conditions, for example assays using imposed stress conditions such as water deficit, nitrogen deficiency, cold growing conditions, pathogen or insect attack or light deficiency, or increased plant density. Changes in morphology can be measured by visual observation of tendency ofa transformed plant with an enhanced agronomic trait to also appear to be a normal plant as compared to changes toward bushy, taller, thicker, narrower leaves, striped leaves, knotted trait, chlorosis, albino, anthocyanin production, or altered tassels, ears or roots. Other selection properties include days to pollen shed, days to silking, leaf extension rate, chlorophyll content, leaf temperature, stand, seedling vigor, internode length, plant height, leaf number, leaf area, tillering, brace roots, stay green, stalk lodging, root lodging, plant health, barreness/prolificacy, green snap, and pest resistance. In addition, phenotypic characteristics of harvested grain may be evaluated, including number of kernels per row on the ear, number of rows of kernels on the ear, kernel abortion, kernel weight, kernel size, kernel density and physical grain quality. Although the plant cells and methods of this invention can be applied to any plant cell, plant, seed or pollen, e.g. any fruit, vegetable, grass, tree or ornamental plant, the various aspects of the invention are preferably applied to corn, soybean, cotton, canola, alfalfa, wheat and rice plants. In many cases the invention is applied to corn plants that are inherently resistant to disease from the Mal de Rio Cuarto virus or the *Puccina sorghi* fungus or both.

The following examples are included to demonstrate aspects of the invention, those of skill in the art should, in light of the present disclosure, appreciate that many changes can be made in the specific aspects which are disclosed and still obtain a like or similar results without departing from the spirit and scope of the invention.

**Example 1. Plant Expression Constructs**

This example illustrates the construction of plasmids for transferring recombinant DNA into plant cells which can be regenerated into transgenic plants of this invention

A. Plant expression constructs for corn transformation

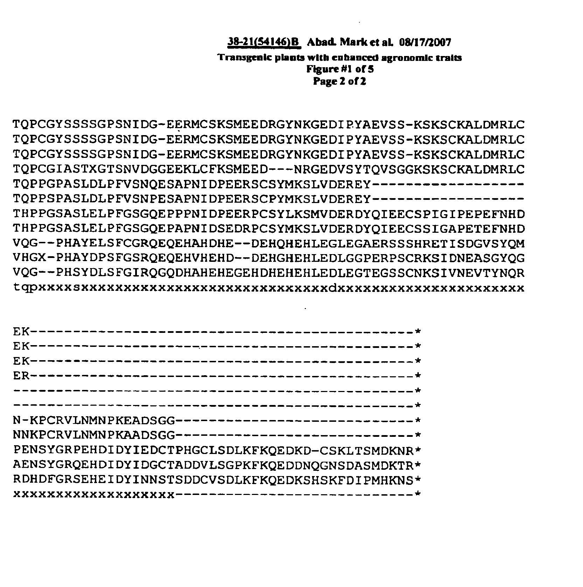

A base corn plant transformation vector pMON93039, as set forth in SEQ ID NO: 30329, illustrated in Table 3 and Figure 2, was fabricated for use in preparing recombinant DNA for *Agrobacterium*-mediated transformation into corn tissue.

Table 3.

| function | name | annotation | Coordinates of SEQ ID NO: 30329 |
|---|---|---|---|
| Agro transforamtion | B-AGRtu.right border | Agro right border sequence, essential for transfer of T-DNA. | 11364-11720 |

(continued)

| function | name | annotation | Coordinates of SEQ ID NO: 30329 |
|---|---|---|---|
| Gene of interest expression cassette | E-Os.Act1 | upstream promoter region of the rice actin 1 gene | 19-775 |
| | E-CaMV.35S.2xA1-B3 | duplicated35S A1-B3 domain without TATA box | 788-1120 |
| | P-Os.Act1 | promoter region of the rice actin 1 gene | 1125-1204 |
| | L-Ta.Lhcb1 | 5' untranslated leader of wheat major chlorophyll a/b binding protein | 1210-1270 |
| | I-Os.Act1 | first intron and flanking UTR exon sequences from the rice actin 1 gene | 1287-1766 |
| | T-St.Pis4 | 3' non-translated region of the potato proteinase inhibitor II gene which functions to direct polyadenylation of the mRNA | 1838-2780 |
| Plant selectable marker expression | P-Os.Act1 | Promoter from the rice actin 1 gene | 2830-3670 |
| | L-Os.Act1 | first exon of the rice actin 1 gene | 3671-3750 |
| cassette | I-Os.Act1 | first intron and flanking UTR exon sequences from the rice actin 1 gene | 3751-4228 |
| | TS-At.ShkG-CTP2 | Transit peptide region of Arabidopsis EPSPS | 4238-4465 |
| | CR-AGRtu.aroA-CP4.nat | Synthetic CP4 coding region with dicot preferred codon usage. | 4466-5833 |
| | T-AGRtu.nos | A 3' non-translated region of the nopaline synthase gene of Agrobacterium tumefaciens Ti plasmid which functions to direct polyadenylation of the mRNA. | 5849-6101 |
| Agro transformation | B-AGRtu.left border | Agro left border sequence, essential for transfer of T-DNA. | 6168-6609 |

(continued)

| function | name | annotation | Coordinates of SEQ ID NO: 30329 |
|---|---|---|---|
| Maintenance in E. coli | OR-Ec.oriV-RK2 | The vegetative origin of replication from plasmid RK2. | 6696-7092 |
| | CR-Ec.rop | Coding region for repressor of primer from the ColE1 plasmid. Expression of this gene product interferes with primer binding at the origin of replication, keeping plasmid copy number low. | 8601-8792 |
| | OR-Ec.ori-ColE1 | The minimal origin of replication from the E. coli plasmid ColE1. | 9220-9808 |
| | P-Ec.aadA-SPC/STR | romoter for Tn7 adenylyltransferase (AAD (3")) | 10339-10380 |
| | CR-Ec.aadA-SPC/STR | Coding region for Tn7 adenylyltransferase (AAD (3")) conferring spectinomycin and streptomycin resistance. | 10381-11169 |
| | T-Ec.aadA-SPC/STR | 3' UTR from the Tn7 adenylyltransferase (AAD (3")) gene ofE. coli. | 11170-11227 |

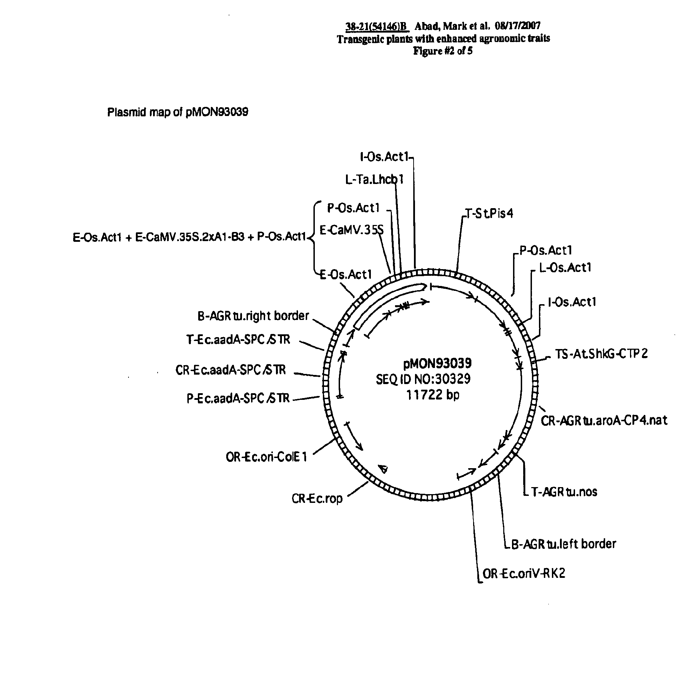

Another embobiment of corn plant transformation base vector is pMON92705, as set forth in SEQ ID NO: 30330, illustrated in Table 4 and Figure 3, which was fabricated for use in preparing recombinant DNA for *Agrobacterium*-mediated transformation into corn tissue.

Table 4.

| function | name | annotation | Coordinates of SEQ ID NO: 30330 |
|---|---|---|---|
| Agro transforamtion | B-AGRtu.right border | Agro right border sequence, essential for transfer of T-DNA. | 5206-5526 |
| Gene of interest expression cassette | P-Os.Act1 | Promoter from the rice actin 1 gene | 5580-6423 |
| | L-Os.Act1 | 5'UTR of riceAct1 gene | 6424-6503 |
| | I-Os.Act1 | Intron from the rice actin 1 gene | 6504-6980 |
| | T-St.Pis4 | 3' non-translated region of the potato proteinase inhibitor II gene which functions to direct polyadenylation of the mRNA | 7055-7997 |

(continued)

| function | name | annotation | Coordinates of SEQ ID NO: 30330 |
|---|---|---|---|
| Plant selectable marker expression cassette | P-Os.Act1 | Promoter from the rice actin 1 gene | 8047-8887 |
| | L-Os.Act1 | first exon of the rice actin 1 gene | 8888-8967 |
| | I-Os.Act1 | first intron and flanking UTR exon sequences from the rice actin 1 gene | 8968-9445 |
| | TS-At.ShkG-CTP2 | Transit peptide region of Arabidopsis EPSPS | 9455-9682 |
| | CR-AGRtu.aroA-CP4.nat | Synthetic CP4 coding region with dicot preferred codon usage. | 9683-11050 |
| | T-AGRtu.nos | A 3' non-translated region of the nopaline synthase gene of Agrobacterium tumefaciens Ti plasmid which functions to direct polyadenylation of the mRNA. | 11066-11318 |
| Agro transformation | B-AGRtu.left border | Agro left border sequence, essential for transfer of T-DNA. | 10-451 |
| Maintenance in E. coli | OR-Ec.oriV-RK2 | The vegetative origin of replication from plasmid RK2. | 538-934 |
| | CR-Ec.rop | Coding region for repressor of primer from the ColE1 plasmid. Expression of this gene product interferes with primer binding at the origin of replication, keeping plasmid copy number low. | 2443-2634 |
| | OR-Ec.ori-ColE1 | The minimal origin of replication from the E. coli plasmid ColE1. | 3062-3650 |
| | P-Ec.aadA-SPC/STR | romoter for Tn7 adenylyltransferase (AAD (3")) | 4181-4222 |
| | CR-Ec.aadA-SPC/STR | Coding region for Tn7 adenylyltransferase (AAD (3")) conferring spectinomycin and streptomycin resistance. | 4223-5011 |
| | T-Ec.aadA-SPC/STR | 3' UTR from the Tn7 adenylyltransferase (AAD (3")) gene of E. coli. | 5012-5562 |

Other base vectors similar to those described above were also constructed as listed in Table 5. See Table 5 for a summary of base vector plasmids and base vector ID's which are referenced in Table 2. Also see Table 5 for a summary of regulatory elements used in the gene expression cassette for these base vectors and SEQ ID NOs for elements.

Primers for PCR amplification of protein coding nucleotides of recombinant DNA are designed at or near the start and stop codons of the coding sequence, in order to eliminate most of the 5' and 3' untranslated regions. Each recombinant DNA coding for a protein identified in Table 2 is amplified by PCR prior to insertion into the insertion site within the gene of interest expression cassette of one of the base vectors as referenced in Table 2.

Table 5

| Base Vector ID | Base Vector for Corn |
|---|---|
| 4 | pMON92705 |
| 5 | pMON92708 |
| 6 | pMON92709 |
| 7 | pMON92713 |
| 8 | nMON92714 |
| 9 | pMON92715 |
| 10 | pMON92716 |
| 11 | pMON92717 |
| 12 | pMON92718 |
| 13 | pMON92719 |
| 14 | pMON92721 |
| 15 | pMON92722 |
| 16 | pMON92723 |
| 17 | pMON92724 |
| 19 | pMON93039 |
| 20 | pMON93043 |
| 21 | pMON94781 |
| Base Vector ID | Base Vector for Soybean |
| 1 | pMON82053 |
| 2 | pMON92671 |
| 3 | pMON92672 |
| 18 | pMON93007 |
| 22 | pMON99006 |

Table 6

| vector | promoter | SEQ ID NO | leader | SEQ ID NO | intron | SEQ ID NO |
|---|---|---|---|---|---|---|
| pMON82053 | P-CaMV.35S-enh | 30332 | NONE | / | NONE | / |
| pMON92671 | P-At. SAMS3 | 30333 | L-At.SAMS3 | 30352 | I-At.SAMS3 | 30371 |
| pMON92672 | P-At.Stm | 30334 | L-At.Stm | 30353 | NONE | / |
| pMON92705 | P-Os.Act1 | 30335 | L-Os.Act1 | 30354 | I-Os.Act1 | 30372 |
| pMON92708 | P-Zm.CA4H | 30336 | L-Zm.CA4H | 30355 | NONE | / |

(continued)

| vector | promoter | SEQ ID NO | leader | SEQ ID NO | intron | SEQ ID NO |
|---|---|---|---|---|---|---|
| pMON92709 | P-Os.GT1 | 30337 | L-Os.GT1 | 30356 | I-Zm.DnaK | 30373 |
| pMON92713 | P-Zm.P39486 | 30338 | L-Zm.39486 | 30357 | I-Zm.DnaK | 30373 |
| pMON92714 | P-RTBV | 30339 | L-RTBV | 30358 | I-Zm.DnaK | 30373 |
| pMON92715 | P-Hv. Per1 | 30340 | L-Hv.Per1 | 30359 | I-Zm.DnaK | 30373 |
| pMON92716 | P-Zm.FDA | 30341 | L-Zm.FDA | 30360 | I-Zm.DnaK | 30373 |
| pMON92717 | P-At.SAMS3 | 30334 | L-At.SAMS3 | 30352 | I-At.SAMS3 | 30371 |
| pMON92718 | P-Zm.CLK1 | 30342 | L-Zm.Cik1 | 30361 | I-Zm.Cik1 | 30374 |
| pMON92719 | P-Zm.RAB17 | 30343 | L-Zm.RAB17 | 30362 | I-Zm.DnaK | 30373 |
| pMON92721 | P-Zm.SzeinC1 | 30344 | L-Zm.SzeinC1 | 30363 | I-Zm.DnaK | 303773 |
| pMON92722 | P-CaMV.35S-enh | 30345 | L-CaMV.35S | 30364 | I-Zm.DnaK | 30373 |
| pMON92723 | P-Zm.Nicotianamine Synthase | 30346 | L-Zm.NAS2 | 30365 | I-Zm.DnaK | 30373 |
| pMON92724 | P-Zm.-636aldolase-0:1:2 + P-Zm.PPDK | 30347 | L-Zm.PPDK | 30366 | I-Zm.DnaK | 30373 |
| pMON93007 | P-At.rd29a | 30348 | L-Atrd29a | 30367 | NONE | / |
| pMON93039 | E-Os.Act1 + E-CaMV.35S.2xA1-B3 + P-Os.Act1 | 30349 | L-Ta.Lhcb1 | 30368 | I-Os.Act1 | 30375 |
| pMON93043 | P-Zm.EM | 30350 | L-Zm.EM | 30369 | I-Zm.DnaK | 30373 |
| pMON94781 | P-Zm.Brittle-2 | 30351 | L-Zm.Brittle-2 | 30370 | I-Zm.DnaK | 30373 |
| pMON99006 | P-CaMV.35S-enh | 30332 | NONE | / | NONE | / |

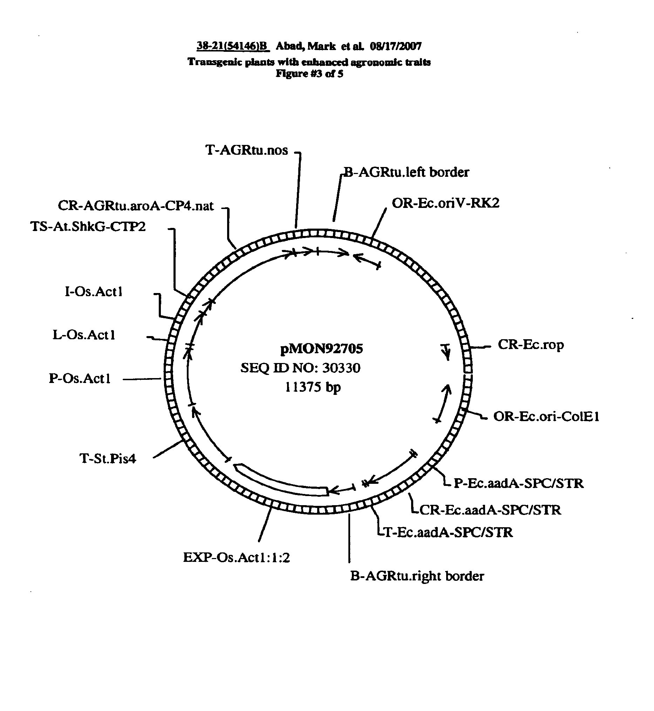

B. Plasmids for use in transformation of soybean were also prepared. Elements of an exemplary common expression vector plasmid pMON82053 are shown in Table 7 below and Figure 4.

Table 7

| function | name | annotation | Coordinates of SEQ ID NO: 30331 |
|---|---|---|---|
| Agro transforamtion | B-AGRtu.left border | Agro left border sequence, essential for transfer of T-DNA. | 6144-6585 |
| Plant selectable marker expression cassette | P-At.Act7 | Promoter from the arabidopsis actin 7 gene | |
| | L-At.Act7 | 5'UTR of Arabidopsis Act7 gene | |
| | I-At.Act7 | Intron from the Arabidopsis actin7 gene | 6624-7861 |
| | TS-At.ShkG-CTP2 | Transit peptide region of Arabidopsis EPSPS | 7864-8091 |

(continued)

| function | name | annotation | Coordinates of SEQ ID NO: 30331 |
|---|---|---|---|
| | CR-AGRtu.aroA-CP4.nno At | Synthetic CP4 coding region with dicot preferred codon usage. | 8092-9459 |
| | T-AGRtu.nos | A 3' non-translated region of the nopaline synthase gene of Agrobacterium tumefaciens Ti plasmid which functions to direct polyadenylation of the mRNA. | 9466-9718 |
| | P-CaMV.35S-enh | Promoter for 35S RNA from CaMV containing a duplication of the -90 to -350 region. | 1-613 |
| Gene of interest expression cassette | T-Gb.E6-3b | 3' untranslated region from the fiber protein E6 gene of sea-island cotton; | 688-1002 |
| Agro transformation | B-AGRtu.right border | Agro right border sequence, essential for transfer of T-DNA. | 1033-1389 |
| | OR-Ec.oriV-RK2 | The vegetative origin of replication from plasmid RK2. | 5661-6057 |
| | CR-Ec.rop | Coding region for repressor of primer from the ColE1 plasmid. Expression of this gene product interferes with primer binding at the origin of replication, keeping plasmid copy number low. | 3961-4152 |
| | OR-Ec.ori-ColE1 | The minimal origin of replication from the E. coli plasmid ColE1. | 2945-3533 |
| | P-Ec.aadA-SPC/STR | romoter for Tn7 adenylyltransferase (AAD (3")) | 2373-2414 |
| | CR-Ec.aadA-SPC/STR | Coding region for Tn7 adenylyltransferase (AAD (3")) conferring spectinomycin and streptomycin resistance. | 1584-2372 |
| Maintenance in E. coli | T-Ec.aadA-SPC/STR | 3' UTR from the Tn7 adenylyltransferase (AAD (3")) gene of E. coli. | 1526-1583 |

Primers for PCR amplification of protein coding nucleotides of recombinant DNA are designed at or near the start and stop codons of the coding sequence, in order to eliminate most of the 5' and 3' untranslated regions. Each recombinant DNA coding for a protein identified in Table 2 is amplified by PCR prior to insertion into the insertion site within the gene of interest expression cassette of one of the base vectors as referenced in Table 2.

C. Cotton transformation vector

Plasmids for use in transformation of cotton are also prepared. Elements of an exemplary common expression vector plasmid pMON99053 are shown in Table 8 below and

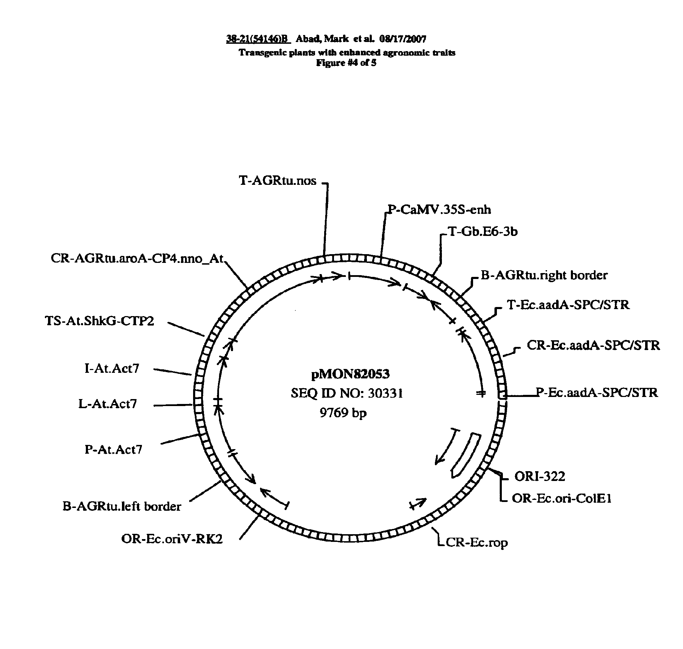

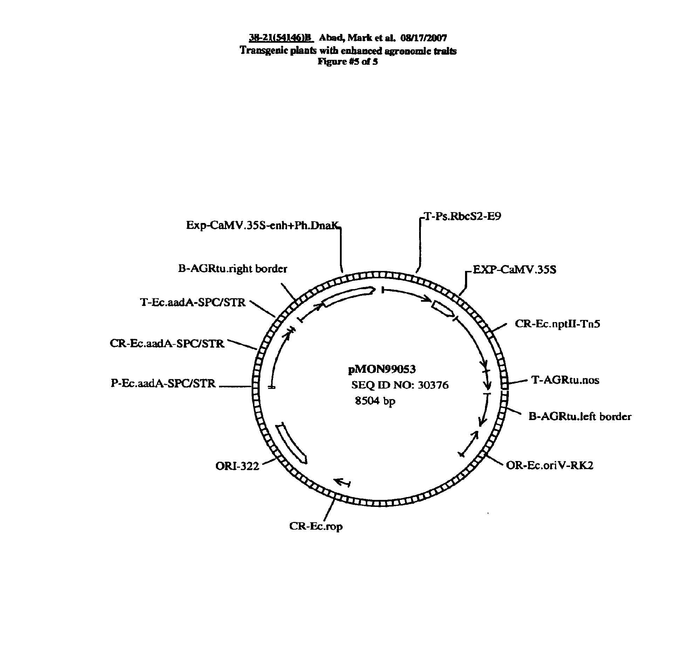

Figure 5. Primers for PCR amplification of protein coding nucleotides of recombinant DNA are designed at or near the start and stop codons of the coding sequence, in order to eliminate most of the 5' and 3' untranslated regions. Each recombinant DNA coding for a protein identified in Table 2 is amplified by PCR prior to insertion into the insertion site within the gene of interest expression cassette of one of the base vectors as referenced in Table 2.

Table 8.

| function | name | annotation | Coordinates of SEQ ID NO: 30376 |
|---|---|---|---|
| Agro transforamtion | B-AGRtu.right border | Agro right border sequence, essential for transfer of T-DNA. | 11364-11720 |
| Gene of interest expression cassette | Exp-CaMV.35S-enh+ph.DnaK | Enhanced version of the 35S RNA promoter from CaMV plus the petunia hsp70 5' untranslated region | 7794-8497 |
| | T-Ps.RbcS2-E9 | The 3' non-translated region of the pea RbcS2 gene which functions to direct polyadenylation of the mRNA. | 67-699 |
| Plant selectable marker expression cassette | Exp-CaMV.35S | Promoter from the rice actin 1 gene | 730-1053 |
| | CR-Ec.nptII-Tn5 | first exon of the rice actin 1 gene | 1087-1881 |
| | T-AGRtu.nos | A 3' non-translated region of the nopaline synthase gene of Agrobacterium tumefaciens Ti plasmid which functions to direct polyadenylation of the mRNA. | 1913-2165 |
| Agro transformation | B-AGRtu.left border | Agro left border sequence, essential for transfer of T-DNA. | 2211-2652 |
| Maintenance in E. coli | OR-Ec.oriV-RK2 | The vegetative origin of replication from plasmid RK2. | 2739-3135 |

(continued)

| function | name | annotation | Coordinates of SEQ ID NO: 30376 |
|---|---|---|---|
| | CR-Ec.rop | Coding region for repressor of primer from the ColE1 plasmid. Expression of this gene product interferes with primer binding at the origin of replication, keeping plasmid copy number low. | 4644-4835 |
| | OR-Ec.ori-ColE1 | The minimal origin of replication from the E. coli plasmid ColE1. | 5263-5851 |
| | P-Ec.aadA-SPC/STR | romoter for Tn7 adenylyltransferase (AAD (3")) | 6382-6423 |
| | CR-Ec.aadA-SPC/STR | Coding region for Tn7 adenylyltransferase (AAD (3")) conferring spectinomycin and streptomycin resistance. | 6424-7212 |
| | T-Ec.aadA-SPC/STR | 3' UTR from the Tn7 adenylyltransferase (AAD (3")) gene of E. coli. | 7213-7270 |

## Example 2. Corn Transformation

This example illustrates plant cell transformation methods useful in producing transgenic corn plant cells, plants, seeds and pollen of this invention and the production and identification of transgenic corn plants and seed with an enhanced trait, i.e. enhanced water use efficiency, enhanced cold tolerance, increased yield, enhanced nitrogen use efficiency, enhanced seed protein and enhanced seed oil. Plasmid vectors were prepared by cloning DNA identified in Table 2 in the identified base vectors for use in corn transformation of corn plant cells to produce transgenic corn plants and progeny plants, seed and pollen.

For Agrobacterium-mediated transformation of corn embryo cells corn plants of a readily transformable line (designated LH59) is grown in the greenhouse and ears harvested when the embryos are 1.5 to 2.0 mm in length. Ears are surface sterilized by spraying or soaking the ears in 80% ethanol, followed by air drying. Immature embryos are isolated from individual kernels on surface sterilized ears. Prior to inoculation of maize cells, Agrobacterium cells are grown overnight at room temperature. Immature maize embryo cells are inoculated with Agrobacterium shortly after excision, and incubated at room temperature with Agrobacterium for 5-20 minutes. Immature embryo plant cells are then co-cultured with Agrobacterium for 1 to 3 days at 23°C in the dark. Co-cultured embryos are transferred to selection media and cultured for approximately two weeks to allow embryogenic callus to develop. Embryogenic callus is transferred to culture medium containing 100 mg/L paromomycin and subcultured at about two week intervals. Transformed plant cells are recovered 6 to 8 weeks after initiation of selection.

For Agrobacterium-mediated transformation of maize callus immature embryos are cultured for approximately 8-21 days after excision to allow callus to develop. Callus is then incubated for about 30 minutes at room temperature with the Agrobacterium suspension, followed by removal of the liquid by aspiration. The callus and Agrobacterium are co-cultured without selection for 3-6 days followed by selection on paromomycin for approximately 6 weeks, with biweekly transfers to fresh media, and paromomycin resistant callus identified as containing the recombinant DNA in an expression cassette.

For transformation by microprojectile bombardment immature maize embryos are isolated and cultured 3-4 days prior to bombardment. Prior to microprojectile bombardment, a suspension of gold particles is prepared onto which the desired recombinant DNA expression cassettes are precipitated. DNA is introduced into maize cells as described in U.S. Patents 5,550,318 and 6,399,861 using the electric discharge particle acceleration gene delivery device. Following microprojectile bombardment, tissue is cultured in the dark at 27 degrees C. Additional transformation methods and materials for making transgenic plants of this invention, for example, various media and recipient target cells, transformation of immature embryos and subsequence regeneration of fertile transgenic plants are disclosed in U.S. Patents 6,194,636 and 6,232,526 and U.S. Patent application Serial No. 09/757,089, which are incorporated herein by reference.

To regenerate transgenic corn plants a callus of transgenic plant cells resulting from transformation is placed on media to initiate shoot development in plantlets which are transferred to potting soil for initial growth in a growth chamber at 26 degrees C followed by a mist bench before transplanting to 5 inch pots where plants are grown to maturity. The regenerated plants are self fertilized and seed is harvested for use in one or more methods to select seed, seedlings or progeny second generation transgenic plants (R2 plants) or hybrids, e.g. by selecting transgenic plants exhibiting an enhanced trait as compared to a control plant.

Transgenic corn plant cells are transformed with recombinant DNA from each of the genes identified in Table 2. Progeny transgenic plants and seed of the transformed plant cells are screened for enhanced water use efficiency, enhanced cold tolerance, increased yield, enhanced nitrogen use efficiency, enhanced seed protein and enhanced seed oil as reported in Example 7.

### Example 3. Soybean transformation

This example illustrates plant transformation useful in producing the transgenic soybean plants of this invention and the production and identification of transgenic seed for transgenic soybean having enhanced water use efficiency, enhanced cold tolerance, increased yield, enhanced nitrogen use efficiency, enhanced seed protein and enhanced seed oil.

For *Agrobacterium* mediated transformation, soybean seeds are germinated overnight and the meristem explants excised. The meristems and the explants are placed in a wounding vessel. Soybean explants and induced *Agrobacterium* cells from a strain containing plasmid DNA with the gene of interest cassette and a plant selectable marker cassette are mixed no later than 14 hours from the time of initiation of seed germination and wounded using sonication. Following wounding, explants are placed in co-culture for 2-5 days at which point they are transferred to selection media for 6-8 weeks to allow selection and growth of transgenic shoots. Trait positive shoots are harvested approximately 6-8 weeks and placed into selective rooting media for 2-3 weeks. Shoots producing roots are transferred to the greenhouse and potted in soil. Shoots that remain healthy on selection, but do not produce roots are transferred to non-selective rooting media for an additional two weeks. Roots from any shoots that produce roots off selection are tested for expression of the plant selectable marker before they are transferred to the greenhouse and potted in soil. Additionally, a DNA construct can be transferred into the genome of a soybean cell by particle bombardment and the cell regenerated into a fertile soybean plant as described in U.S. Patent 5,015,580, herein incorporated by reference.

Transgenic soybean plant cells are transformed with recombinant DNA from each of the genes identified in Table 2. Progeny transgenic plants and seed of the transformed plant cells are screened for enhanced water use efficiency, enhanced cold tolerance, increased yield, enhanced nitrogen use efficiency, enhanced seed protein and enhanced seed oil as reported in Example 7.

### Example 4. Cotton transgenic plants with enhanced agronomic traits

Cotton transformation is performed as generally described in WO0036911 and in U.S. Pat. No. 5,846,797. Transgenic cotton plants containing each of the recombinant DNA having a sequence of SEQ ID NO: 1 through SEQ ID NO: 358 are obtained by transforming with recombinant DNA from each of the genes identified in Table 2. Progeny transgenic plants are selected from a population of transgenic cotton events under specified growing conditions and are compared with control cotton plants. Control cotton plants are substantially the same cotton genotype but without the recombinant DNA, for example, either a parental cotton plant of the same genotype that was not transformed with the identical recombinant DNA or a negative isoline of the transformed plant. Additionally, a commercial cotton cultivar adapted to the geographical region and cultivation conditions, i.e. cotton variety ST474, cotton variety FM 958, and cotton variety Siokra L-23, are used to compare the relative performance of the transgenic cotton plants containing the recombinant DNA. The specified culture conditions are growing a first set of transgenic and control plants under "wet" conditions, i.e. irrigated in the range of 85 to 100 percent of evapotranspiration to provide leaf water potential of -14 to - 18 bars, and growing a second set of transgenic and control plants under "dry" conditions, i.e. irrigated in the range of 40 to 60 percent of evapotranspiration to provide a leaf water potential of -21 to -25 bars. Pest control, such as weed and insect control is applied equally to both wet and dry treatments as needed. Data gathered during the trial includes weather records throughout the growing season including detailed records of rainfall; soil characterization information; any herbicide or insecticide applications; any gross agronomic differences observed such as leaf morphology, branching habit, leaf color, time to flowering, and fruiting pattern; plant height at various points during the trial; stand density; node and fruit number including node above white flower and node above crack boll measurements; and visual wilt scoring.

Cotton boll samples are taken and analyzed for lint fraction and fiber quality. The cotton is harvested at the normal harvest timeframe for the trial area. Enhanced water use efficiency is indicated by increased yield, improved relative water content, enhanced leaf water potential, increased biomass, enhanced leaf extension rates, and improved fiber parameters.

The transgenic cotton plants of this invention are identified from among the transgenic cotton plants by agronomic trait screening as having increased yield and enhanced water use efficiency.

**Example 5. Canola transformation**

This example illustrates plant transformation useful in producing the transgenic canola plants of this invention and the production and identification of transgenic seed for transgenic canola having enhanced water use efficiency, enhanced cold tolerance, increased yield, enhanced nitrogen use efficiency, enhanced seed protein and enhanced seed oil.

Tissues from *in vitro* grown canola seedlings are prepared and inoculated with overnight-grown *Agrobacterium* cells containing plasmid DNA with the gene of interest cassette and a plant selectable marker cassette. Following co-cultivation with *Agrobacterium,* the infected tissues are allowed to grow on selection to promote growth of transgenic shoots, followed by growth of roots from the transgenic shoots. The selected plantlets are then transferred to the greenhouse and potted in soil. Molecular characterization are performed to confirm the presence of the gene of interest, and its expression in transgenic plants and progenies. Progeny transgenic plants are selected from a population of transgenic canola events under specified growing conditions and are compared with control canola plants. Control canola plants are substantially the same canola genotype but without the recombinant DNA, for example, either a parental canola plant of the same genotype that is not transformed with the identical recombinant DNA or a negative isoline of the transformed plant

Transgenic canola plant cells are transformed with recombinant DNA from each of the genes identified in Table 2. Transgenic progeny plants and seed of the transformed plant cells are screened for enhanced water use efficiency, enhanced cold tolerance, increased yield, enhanced nitrogen use efficiency, enhanced seed protein and enhanced seed oil as reported in Example 7.

**Example 6. Homolog Identification**

This example illustrates the identification of homologs of proteins encoded by the DNA identified in Table 2 which is used to provide transgenic seed and plants having enhanced agronomic traits. From the sequence of the homologs, homologous DNA sequence can be identified for preparing additional transgenic seeds and plants of this invention with enhanced agronomic traits.

An "All Protein Database" was constructed of known protein sequences using a proprietary sequence database and the National Center for Biotechnology Information (NCB1) non-redundant amino acid database (nr.aa). For each organism from which a polynucleotide sequence provided herein was obtained, an "Organism Protein Database" was constructed of known protein sequences of the organism; it is a subset of the All Protein Database based on the NCBI taxonomy ID for the organism.

The All Protein Database was queried using amino acid sequences provided herein as SEQ ID NO: 359 through SEQ ID NO: 716 using NCBI "blastp" program with E-value cutoff of 1e-8. Up to 1000 top hits were kept, and separated by organism names. For each organism other than that of the query sequence, a list was kept for hits from the query organism itself with a more significant E-value than the best hit of the organism. The list contains likely duplicated genes of the polynucleotides provided herein, and is referred to as the Core List. Another list was kept for all the hits from each organism, sorted by E-value, and referred to as the Hit List.

The Organism Protein Database was queried using polypeptide sequences provided herein as SEQ ID NO: 359 through SEQ ID NO: 716 using NCBI "blastp" program with E-value cutoff of 1e-4. Up to 1000 top hits were kept. A BLAST searchable database was constructed based on these hits, and is referred to as "SubDB". SubDB was queried with each sequence in the Hit List using NCBI "blastp" program with E-value cutoff of 1e-8. The hit with the best E-value was compared with the Core List from the corresponding organism. The hit is deemed a likely ortholog if it belongs to the Core List, otherwise it is deemed not a likely ortholog and there is no further search of sequences in the Hit List for the same organism. Homologs from a large number of distinct organisms were identified and are reported by amino acid sequences of SEQ ID NO: 717 through SEQ ID NO: 30327. These relationship of proteins of SEQ ID NO: 358 through 716 and homologs of SEQ ID NO: 717 through 30327 is identified in Table 9. The source organism for each homolog is found in the Sequence Listing.

**Example 7. Selection of transgenic plants with enhanced agronomic trait(s)**

This example illustrates identification of plant cells of the invention by screening derived plants and seeds for enhanced trait. Transgenic corn seed and plants with recombinant DNA identified in Table 2 are prepared by plant cells transformed with DNA that is stably integrated into the genome of the corn cell. Transgenic corn plant cells are transformed with recombinant DNA from each of the genes identified in Table 1. Progeny transgenic plants and seed of the transformed plant cells are screened for enhanced water use efficiency, enhanced cold tolerance, increased yield, enhanced nitrogen use efficiency, enhanced seed protein and enhanced seed oil as compared to control plants.

**A. Selection for enhanced Nitrogen Use Efficiency**

The physiological efficacy of transgenic corn plants (tested as hybrids) can be tested for nitrogen use efficiency (NUE) traits in a high-throughput nitrogen (N) selection method. The collected data are compared to the measurements from wildtype controls using a statistical model to determine if the changes are due to the transgene. Raw data were analyzed by SAS software. Results shown herein are the comparison of transgenic plants relative to the wildtype controls.

(1) Media Preparation for Planting a NUE Protocol

Planting materials used: Metro Mix 200 (vendor: Hummert) Cat. # 10-0325, Scotts Micro Max Nutrients (vendor: Hummert) Cat. # 07-6330, OS 4 1/3" x 3 7/8" pots (vendor: Hummert) Cat. # 16-1415, OS trays (vendor: Hummert) Cat. # 16-1515, Hoagland's macronutrients solution, Plastic 5" stakes (vendor: Hummert) yellow Cat. # 49-1569, white Cat. # 49-1505, Labels with numbers indicating material contained in pots. Fill 500 pots to rim with Metro Mix 200 to a weight of ~140g/pot. Pots are filled uniformly by using a balancer. Add 0.4g of Micro Max nutrients to each pot. Stir ingredients with spatula to a depth of 3 inches while preventing material loss.

(2) Planting a NUE selection in the Greenhouse

(a) Seed Germination - Each pot is lightly atered twice using reverse osmosis purified water. The first watering is scheduled to occur just before planting; and the second watering, after the seed has been planted in the pot. Ten Seeds of each entry (1 seed per pot) are planted to select eight healthy uniform seedlings. Additional wild type controls are planted for use as border rows. Alternatively, 15 seeds of each entry (1 seed per pot) are planted to select 12 healthy uniform seedlings (this larger number of plantings is used for the second, or confirmation, planting). Place pots on each of the 12 shelves in the Conviron growth chamber for seven days. This is done to allow more uniform germination and early seedling growth. The following growth chamber settings are 25° C/day and 22° C/ night, 14 hours light and ten hours dark, humidity ~ 80%, and light intensity ~350 $\mu$mol/m$^2$/s (at pot level). Watering is done via capillary matting similar to greenhouse benches with duration of ten minutes three times a day.

(b) Seedling transfer - After seven days, the best eight or 12 seedlings for the first or confirmation pass runs, respectively, are chosen and transferred to greenhouse benches. The pots are spaced eight inches apart (center to center) and are positioned on the benches using the spacing patterns printed on the capillary matting. The Vattex matting creates a 384-position grid, randomizing all range, row combinations. Additional pots of controls are placed along the outside of the experimental block to reduce border effects.

Plants are allowed to grow for 28 days under the low N run or for 23 days under the high N run. The macronutrients are dispensed in the form of a macronutrient solution (see composition below) containing precise amounts of N added (2mM $NH_4NO_3$ for limiting N selection and 20mM $NH_4NO_3$ for high N selection runs). Each pot is manually dispensed 100ml of nutrient solution three times a week on alternate days starting at eight and ten days after planting for high N and low N runs, respectively. On the day of nutrient application, two 20 min waterings at 05:00 and 13:00 are skipped. The vattex matting should be changed every third run to avoid N accumulation and buildup of root matter. Table 10 shows the amount of nutrients in the nutrient solution for either the low or high nitrogen selection.

Table 10

| Nutrient Stock | 2mM NH$_4$NO$_3$ (Low Nitrogen Growth Condition, Low N) | 20mM NH$_4$NO$_3$ (high Nitrogen Growth Condition, High N) |
|---|---|---|
| | mL/L | mL/L |
| 1 M NH$_4$N0$_3$ | 2 | 20 |
| 1 M KH$_2$PO$_4$ | 0.5 | 0.5 |
| 1 M MgSO$_4$.7H$_2$O | 2 | 2 |
| 1 M CaCl$_2$ | 2.5 | 2.5 |
| 1 M K$_2$SO$_4$ | 1 | 1 |
| Note: Adjust pH to 5.6 with HCl or KOH | | |

(c) Harvest Measurements and Data Collection - After 28 days of plant growth for low N runs and 23 days of plant growth for high N runs, the following measurements are taken (phenocodes in parentheses): total shoot fresh mass (g) (SFM) measured by Sartorius electronic balance, V6 leaf chlorophyll measured by Minolta SPAD meter (relative units) (LC), V6 leaf area (cm$^2$) (LA) measured by a Li-Cor leaf area meter, V6 leaf fresh mass (g) (LFM) measured by Sartorius electronic balance, and V6 leaf dry mass (g) (LDM) measured by Sartorius electronic balance. Raw data were analyzed by SAS software. Results shown are the comparison of transgenic plants relative to the wildtype controls.

To take a leaf reading, samples were excised from the V6 leaf. Since chlorophyll meter readings of corn leaves are affected by the part of the leaf and the position of the leaf on the plant that is sampled, SPAD meter readings were done on leaf six of the plants. Three measurements per leaf were taken, of which the first reading was taken from a point one-half the distance between the leaf tip and the collar and halfway from the leaf margin to the midrib while two were taken toward the leaf tip. The measurements were restricted in the area from 1/2 to 3/4 of the total length of the leaf (from the base) with approximately equal spacing between them. The average of the three measurements was taken from the SPAD machine.

Leaf fresh mass is recorded for an excised V6 leaf, the leaf is placed into a paper bag. The paper bags containing the leaves are then placed into a forced air oven at 80° C for 3 days. After 3 days, the paper bags are removed from the oven and the leaf dry mass measurements are taken.

From the collected data, two derived measurements are made: (1)Leaf chlorophyll area (LCA), which is a product of V6 relative chlorophyll content and its leaf area (relative units). Leaf chlorophyll area= leaf chlorophyll X leaf area. This parameter gives an indication of the spread of chlorophyll over the entire leaf area; (2)specific leaf area (LSA) is calculated as the ratio of V6 leaf area to its dry mass (cm$^2$/g dry mass), a parameter also recognized as a measure of NUE.

Nitrogen use field efficacy assay

Level I. Transgenic plants provided by the present invention are planted in field without any nitrogen source being applied. Transgenic plants and control plants are grouped by genotype and construct with controls arranged randomly within genotype blocks. Each type of transgenic plants are tested by 3 replications and across 5 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

Level II. Transgenic plants provided by the present invention are planted in field with three levels of nitrogen (N) fertilizer being applied, i.e. low level (0 N), medium level (80 lb/ac) and high level (180 lb/ac). Liquid 28% or 32% UAN (Urea, Ammonium Nitrogen) are used as the N source and apply by broadcast boom and incorporate with a field cultivator with rear rolling basket in the same direction as intended crop rows. Although there is no N applied to the 0 N treatment the soil should still be disturbed in the same fashion as the treated area. Transgenic plants and control plants are grouped by genotype and construct with controls arranged randomly within genotype blocks. Each type of transgenic plants is tested by 3 replications and across 4 locations. Nitrogen levels in the fields are analyzed in early April pre-planting by collecting 30 sample soil cores from 0-24" and 24 to 48" soil layer. Soil samples are analyzed for nitrate-nitrogen, phosphorus(P), Potassium(K), organic matter and pH to provide baseline values. P, K and micronutrients are applied based upon soil test recommendations.

**B. Selection for increased yield**

Many transgenic plants of this invention exhibit improved yield as compared to a control plant. Improved yield can result from enhanced seed sink potential, i.e. the number and size of endosperm cells or kernels and/or enhanced sink strength, i.e. the rate of starch biosynthesis. Sink potential can be established very early during kernel development, as endosperm cell number and size are determined within the first few days after pollination.

Much of the increase in corn yield of the past several decades has resulted from an increase in planting density. During that period, corn yield has been increasing at a rate of 2.1 bushels/acre/year, but the planting density has increased at a rate of 250 plants/acre/year. A characteristic of modern hybrid corn is the ability of these varieties to be planted at high density. Many studies have shown that a higher than current planting density should result in more biomass production, but current germplasm does not perform well at these higher densities. One approach to increasing yield is to increase harvest index (HI), the proportion of biomass that is allocated to the kernel compared to total biomass, in high density plantings.

Effective yield selection of enhanced yielding transgenic corn events uses hybrid progeny of the transgenic event over multiple locations with plants grown under optimal production management practices, and maximum pest control. A useful target for improved yield is a 5% to 10% increase in yield as compared to yield produced by plants grown from seed for a control plant, Selection methods may be applied in multiple and diverse geographic locations, for example up to 16 or more locations, over one or more plating seasons, for example at least two planting seasons to statistically distinguish yield improvement from natural environmental effects. It is to plant multiple transgenic plants, positive and negative control plants, and pollinator plants in standard plots, for example 2 row plots, 20 feet long by 5 feet wide with 30 inches distance between rows and a 3 foot alley between ranges. Transgenic events can be grouped by recombinant DNA constructs with groups randomly placed in the field. A pollinator plot ofa high quality corn line is planted for every two plots to allow open pollination when using male sterile transgenic events. A useful planting density is about 30,000 plants/acre. High planting density is greater than 30,000 plants/acre, preferably about 40,000 plants/acre, more preferably about 42,000 plants/acre, most preferably about 45,000 plants/acre. Surrogate indicators for yield improvement include source capacity (biomass), source output (sucrose and photosynthesis), sink components (kernel size, ear size, starch in the seed), development (light response, height, density tolerance), maturity, early flowering trait and physiological responses to high density planting, for example at 45,000 plants per acre, for example as illustrated in Table 11 and 12.

Table 11

| Timing | Evaluation | Description | comments |
|---|---|---|---|
| V2-3 | Early stand | Can be taken any time after germination and prior to removal of any plants. | |
| Pollen shed | GDU to 50% shed | GDU to 50% plants shedding 50% tassel. | |
| Silking | GDU to 50% silk | GDU to 50% plants showing silks. | |
| Maturity | Plant height | Height from soil surface to flag leaf attachment (inches). | 10 plants per plot - Yield team assistance |
| Maturity | Ear height | Height from soil surface to primary ear attachment node. | 10 plants per plot - Yield team assistance |
| Maturity | Leaves above ear | visual scores: erect, size, rolling | |
| Maturity | Tassel size | Visual scores +/- vs. WT | |
| Pre-Harvest | Final Stand | Final stand count prior to harvest, exclude tillers | |
| Pre-Harvest | Stalk lodging | No. of stalks broken below the primary ear attachment. Exclude leaning tillers | |
| Pre-Harvest | Root lodging | No. of stalks leaning >45° angle from perpendicular. | |

(continued)

| Timing | Evaluation | Description | comments |
|---|---|---|---|
| Pre-Harvest | Stay green | After physiological maturity and when differences among genotypes are evident: Scale 1 (90-100% tissue green) - 9 (0-19% tissue green). | |
| Harvest | Grain Yield | Grain yield/plot (Shell weight) | |

**Table 12**

| Timing | Evaluation | Description |
|---|---|---|
| V8 - V12 | Chlorophyll | |
| V12 - VT | Ear leaf area | |
| V15 - 15DAP | Chl fluorescence | |
| V15 - 15DAP | CER | |
| 15 - 25 DAP | Carbohydrates | sucrose, starch |
| Pre-Harvest | 1st internode diameter | |
| Pre-Harvest | Base 3 internode diameter | |
| Pre-Harvest | Ear internode diameter | |
| Maturity | Ear traits | diameter, length, kernel number, kernel weight |

Electron transport rates (ETR) and CO2 exchange rates (CER): ETR and CER are measured with Li6400LCF (Licor, Lincoln, NE) around V9-R1 stages. Leaf chlorophyll fluorescence is a quick way to monitor the source activity and is reported to be highly correlated with $CO_2$ assimilation under varies conditions (Photosyn Research, 37: 89-102). The youngest fully expanded leaf or 2 leaves above the ear leaf is measured with actinic light 1500 (with 10% blue light) micromol m$^{-2}$ s$^{-1}$, 28oC, CO2 levels 450ppm. Ten plants are measured in each event. There are 2 readings for each plant.

A hand-held chlorophyll meter SPAD-502 (Minolta - Japan) is used to measure the total chlorophyll level on live transgenic plants and the wild type counterparts a. Three trifoliates from each plant are analyzed, and each trifoliate were analyzed three times. Then 9 data points are averaged to obtain the chlorophyll level. The number of analyzed plants of each genotype ranges from 5 to 8.

When selecting for yield improvement a useful statistical measurement approach comprises three components, i.e. modeling spatial autocorrelation of the test field separately for each location, adjusting traits of recombinant DNA events for spatial dependence for each location, and conducting an across location analysis. The first step in modeling spatial autocorrelation is estimating the covariance parameters of the semivariogram. A spherical covariance model is assumed to model the spatial autocorrelation. Because of the size and nature of the trial, it is likely that the spatial autocorrelation may change. Therefore, anisotropy is also assumed along with spherical covariance structure. The following set of equations describes the statistical form of the anisotropic spherical covariance model.

$$C(h;\theta) = \nu I(h=0) + \sigma^2 \left(1 - \frac{3}{2}h + \frac{1}{2}h^3\right) I(h<1),$$

where $I(\bullet)$ is the indicator function, $h = \sqrt{\dot{x}^2 + \dot{y}^2}$, and

$$\dot{x} = [\cos(\rho\pi/180)(x_1 - x_2) - \sin(\rho\pi/180)(y_1 - y_2)]/\omega_x$$

$$\dot{y} = [\sin(\rho\pi/180)(x_1 - x_2) + \cos(\rho\pi/180)(y_1 - y_2)]/\omega_y$$

where $s_1 = (x_1, y_1)$ are the spatial coordinates of one location and $s_2 = (x_2, y_2)$ are the spatial coordinates of the second location. There are 5 covariance parameters, $\theta = (v, \sigma^2, \rho, \omega_n, \omega_j)$, where $v$ is the nugget effect, $\sigma^2$ is the partial sill, $\rho$ is a rotation in degrees clockwise from north, $\omega_n$ is a scaling parameter for the minor axis and $\omega_j$ is a scaling parameter for the major axis of an anisotropical ellipse of equal covariance. The five covariance parameters that defines the spatial trend will then be estimated by using data from heavily replicated pollinator plots via restricted maximum likelihood approach. In a multi-location field trial, spatial trend are modeled separately for each location.

After obtaining the variance parameters of the model, a variance-covariance structure is generated for the data set to be analyzed. This variance-covariance structure contains spatial information required to adjust yield data for spatial dependence. In this case, a nested model that best represents the treatment and experimental design of the study is used along with the variance-covariance structure to adjust the yield data. During this process the nursery or the seed batch effects can also be modeled and estimated to adjust the yields for any yield parity caused by seed batch differences. After spatially adjusted data from different locations are generated, all adjusted data is combined and analyzed assuming locations as replications. In this analysis, intra and inter-location variances are combined to estimate the standard error of yield from transgenic plants and control plants. Relative mean comparisons are used to indicate statistically significant yield improvements.

**C. Selection for enhanced water use efficiency (WUE)**

Described in this example is a high-throughput method for greenhouse selection of transgenic corn plants to wild type corn plants (tested as inbreds or hybrids) for water use efficiency. This selection process imposes 3 drought/re-water cycles on plants over a total period of 15 days after an initial stress free growth period of 11 days. Each cycle consists of 5 days, with no water being applied for the first four days and a water quenching on the 5th day of the cycle. The primary phenotypes analyzed by the selection method are the changes in plant growth rate as determined by height and biomass during a vegetative drought treatment. The hydration status of the shoot tissues following the drought is also measured. The plant height are measured at three time points. The first is taken just prior to the onset drought when the plant is 11 days old, which is the shoot initial height (S1H). The plant height is also measured halfway throughout the drought/re-water regimen, on day 18 after planting, to give rise to the shoot mid-drought height (SMH). Upon the completion of the final drought cycle on day 26 after planting, the shoot portion of the plant is harvested and measured for a final height, which is the shoot wilt height (SWH) and also measured for shoot wilted biomass (SWM). The shoot is placed in water at 40 degree Celsius in the dark. Three days later, the shoot is weighted to give rise to the shoot turgid weight (STM). After drying in an oven for four days, the shoots are weighted for shoot dry biomass (SDM). The shoot average height (SAH) is the mean plant height across the 3 height measurements. The procedure described above may be adjusted for +/- ~ one day for each step given the situation.

To correct for slight differences between plants, a size corrected growth value is derived from SIH and SWH. This is the Relative Growth Rate (RGR). Relative Growth Rate (RGR) is calculated for each shoot using the formula [RGR% = (SWH-SIH)/((SWH+SIH)/2)*100]. Relative water content (RWC) is a measurement of how much (%) of the plant was water at harvest. Water Content (RWC) is calculated for each shoot using the formula [RWC% = (SWM-SDM) /(STM-SDM)*100]. Fully watered corn plants of this age run around 98% RWC.

**D. Selection for Growth Under Cold Stress**

(1) Cold germination assay - Three sets of seeds are used for the assay. The first set consists of positive transgenic events (F1 hybrid) where the genes of the present invention are expressed in the seed. The second seed set is nontransgenic, wild-type negative control made from the same genotype as the transgenic events. The third set consisted of two cold tolerant and one cold sensitive commercial check lines of corn. All seeds are treated with a fungicide " Captan" (MAESTRO® 80DF Fungicide, Arvesta Corporation, San Francisco, CA, USA). 0.43 mL Captan is applied per 45 g of corn seeds by mixing it well and drying the fungicide prior to the experiment.

Corn kernels are placed embryo side down on blotter paper within an individual cell (8.9 x 8.9 cm) of a germination tray (54 x 36 cm). Ten seeds from an event are placed into one cell of the germination tray. Each tray can hold 21 transgenic events and 3 replicates of wildtype (LH244SDms+LH59), which is randomized in a complete block design. For every event there are five replications (five trays). The trays are placed at 9.7C for 24 days (no light) in a Convrion growth chamber *(Conviron Model PGV36, Controlled Environments, Winnipeg, Canada)*. Two hundred and fifty millilters of deionized water are added to each germination tray. Germination counts are taken 10th, 11th, 12th, 13th, 14th, 17th, 19th, 21st, and 24th day after start date of the experiment. Seeds are considered germinated if the emerged radicle size is 1 cm. From the germination counts germination index is calculated.
*The germination index is calculated as per:*

$$\textit{Germination index} = (\Sigma \ ([T+1-n_i] * [P_i - P_{i-1}]))/T$$

Where T is the total number of days for which the germination assay is performed. The number of days after planting is defined by n. "i" indicated the number of times the germination had been counted, including the current day. P is the percentage of seeds germinated during any given rating. Statistical differences are calculated between transgenic events and wild type control. After statistical analysis, the events that show a statistical significance at the p level of less than 0.1 relative to wild-type controls will advance to a secondary cold selection. The secondary cold screen is conducted in the same manner of the primary selection only increasing the number of repetitions to ten. Statistical analysis of the data from the secondary selection is conducted to identify the events that show a statistical significance at the p level of less than 0.05 relative to wild-type controls.

(2) Cold Shock assay - The experimental set-up for the cold shock assay is the same as described in the above cold germination assay except seeds were grown in potted media for the cold shock assay.

The desired numbers of 2.5" square plastic pots are placed on flats (n=32, 4x8). Pots were filled with Metro Mix 200 soil-less media containing 19:6:12 fertilizer (6 lbs/cubic yard) (Metro Mix, Pots and Flat are obtained from Hummert International, Earth City, MO). After planting seeds, pots are placed in a growth chamber set at 23° C, relative humidity of 65% with 12 hour day and night photoperiod (300 uE/m2-min). Planted seeds are watered for 20 minute every other day by sub-irrigation and flats were rotated every third day in a growth chamber for growing corn seedlings. On the 10th day after planting the transgenic positive and wild-type negative (WT) plants are positioned in flats in an alternating pattern. Chlorophyll fluorescence of plants is measured on the 10th day during the dark period of growth by using a PAM-2000 portable fluorometer as per the manufacturer's instructions (Walz, Germany). After chlorophyll measurements, leaf samples from each event are collected for confirming the expression of genes of the present invention. For expression analysis six V1 leaf tips from each selection are randomly harvested. The flats are moved to a growth chamber set at 5° C. All other conditions such as humidity, day/night cycle and light intensity are held constant in the growth chamber. The flats are sub-irrigated every day after transfer to the cold temperature. On the 4th day chlorophyll fluorescence is measured. Plants are transferred to normal growth conditions after six days of cold shock treatment and allowed to recover for the next three days. During this recovery period the length of the V3 leaf is measured on the 1st and 3rd days. After two days of recovery V2 leaf damage is determined visually by estimating percent of green V2 leaf.

Statistical differences in V3 leaf growth, V2 leaf necrosis and fluorescence during pre- shock and cold shock can be used for estimation of cold shock damage on corn plants.

(3) Early seedling growth assay - Three sets of seeds are used for the experiment. The first set consists of positive transgenic events (F1 hybrid) where the genes of the present invention are expressed in the seed. The second seed set is nontransgenic, wild-type negative control made from the same genotype as the transgenic events. The third seed set consists of two cold tolerant and two cold sensitive commercial check lines of corn. All seeds are treated with a fungicide " Captan", (3a,4,7,a-tetrahydro-2-[(trichloromethly)thio]-1H-isoindole-1,3(2H)-dione, Drex Chemical Co. Memphis, TN). Captan (0.43 mL) was applied per 45 g of corn seeds by mixing it well and drying the fungicide prior to the experiment.

Seeds are grown in germination paper for the early seedling growth assay. Three 12"x18" pieces of germination paper (Anchor Paper #SD7606) are used for each entry in the test (three repetitions per transgenic event). The papers are wetted in a solution of 0.5% $KNO_3$ and 0.1% Thyram.

For each paper fifteen seeds are placed on the line evenly spaced down the length of the paper. The fifteen seeds are positioned on the paper such that the radical would grow downward, for example longer distance to the paper's edge. The wet paper is rolled up starting from one of the short ends. The paper is rolled evenly and tight enough to hold the seeds in place. The roll is secured into place with two large paper clips, one at the top and one at the bottom. The rolls are incubated in a growth chamber at 23° C for three days in a randomized complete block design within an appropriate container. The chamber is set for 65% humidity with no light cycle. For the cold stress treatment the rolls are then incubated in a growth chamber at 12° C for twelve days. The chamber is set for 65% humidity with no light cycle.

After the cold treatment the germination papers are unrolled and the seeds that did not germinate are discarded. The lengths of the radicle and coleoptile for each seed are measured through an automated imaging program that automatically collects and processes the images. The imaging program automatically measures the shoot length, root length, and whole seedling length of every individual seedling and then calculates the average of each roll.

After statistical analysis, the events that show a statistical significance at the p level of less than 0.1 relative to wild-type controls will advance to a secondary cold selection. The secondary cold selection is conducted in the same manner of the primary selection only increasing the number of repetitions to five. Statistical analysis of the data from the secondary selection is conducted to identify the events that show a statistical significance at the p level of less than 0.05 relative to wild-type controls.

4. Cold field efficacy trial

This example sets forth a cold field efficacy trial to identify gene constructs that confer enhanced cold vigor at germination and early seedling growth under early spring planting field conditions in conventional-till and simulated no-till environments. Seeds are planted into the ground around two weeks before local farmers are beginning to plant corn so that a significant cold stress is exerted onto the crop, named as cold treatment. Seeds also are planted under local optimal planting conditions such that the crop has little or no exposure to cold condition, named as normal treatment. The cold field efficacy trials are carried out in five locations, including Glyndon MN, Mason MI, Monmouth IL, Dayton IA, Mystic CT. At each location, seeds are planted under both cold and normal conditions with 3 repetitions per treatment, 20 kernels per row and single row per plot. Seeds are planted 1.5 to 2 inch deep into soil to avoid muddy conditions. Two temperature monitors are set up at each location to monitor both air and soil temperature daily.

Seed emergence is defined as the point when the growing shoot breaks the soil surface. The number of emerged seedling in each plot is counted everyday from the day the earliest plot begins to emerge until no significant changes in emergence occur. In addition, for each planting date, the latest date when emergence is 0 in all plots is also recorded. Seedling vigor is also rated at V3-V4 stage before the average of corn plant height reaches 10 inches, with 1=excellent early growth, 5=Average growth and 9=poor growth. Days to 50% emergence, maximum percent emergence and seedling vigor are calculated using SAS software for the data within each location or across all locations.

E. Screens for transgenic plant seeds with increased protein and/or oil levels

This example sets forth a high-throughput selection for identifying plant seeds with improvement in seed composition using the Infratec 1200 series Grain Analyzer, which is a near-infrared transmittance spectrometer used to determine the composition of a bulk seed sample. Near infrared analysis is a non-destructive, high-throughput method that can analyze multiple traits in a single sample scan.. An NIR calibration for the analytes of interest is used to predict the values of an unknown sample. The NIR spectrum is obtained for the sample and compared to the calibration using a complex chemometric software package that provides a predicted values as well as information on how well the sample fits in the calibration.

Infratec Model 1221, 1225, or 1227 with transport module by Foss North America is used with cuvette, item # 1000-4033, Foss North America or for small samples with small cell cuvette, Foss standard cuvette modified by Leon Girard Co. Corn and soy check samples of varying composition maintained in check cell cuvettes are supplied by Leon Girard Co. NIT collection software is provided by Maximum Consulting Inc.Software. Calculations are performed automatically by the software. Seed samples are received in packets or containers with barcode labels from the customer. The seed is poured into the cuvettes and analyzed as received.

Table 13.

| Typical sample(s): | Whole grain corn and soybean seeds |
|---|---|
| Analytical time to run method: | Less than 0.75 min per sample |
| Total elapsed time per run: | 1.5 minute per sample |
| Typical and minimum sample size: | Corn typical: 50cc; minimum 30cc<br>Soybean typical: 50cc; minimum 5cc |
| Typical analytical range: | Determined in part by the specific calibration.<br>Corn - moisture 5-15%, oil 5-20%, protein 5-30%, starch 50-75%, and density 1.0-1.3%.<br>Soybean - moisture 5-15%, oil 15-25%, and protein 35-50%. |

**Example 8. Consensus sequence**

This example illustrates the identification of consensus amino acid sequence for the proteins and homologs encoded by DNA that is used to prepare the transgenic seed and plants of this invention having enhanced agronomic traits.

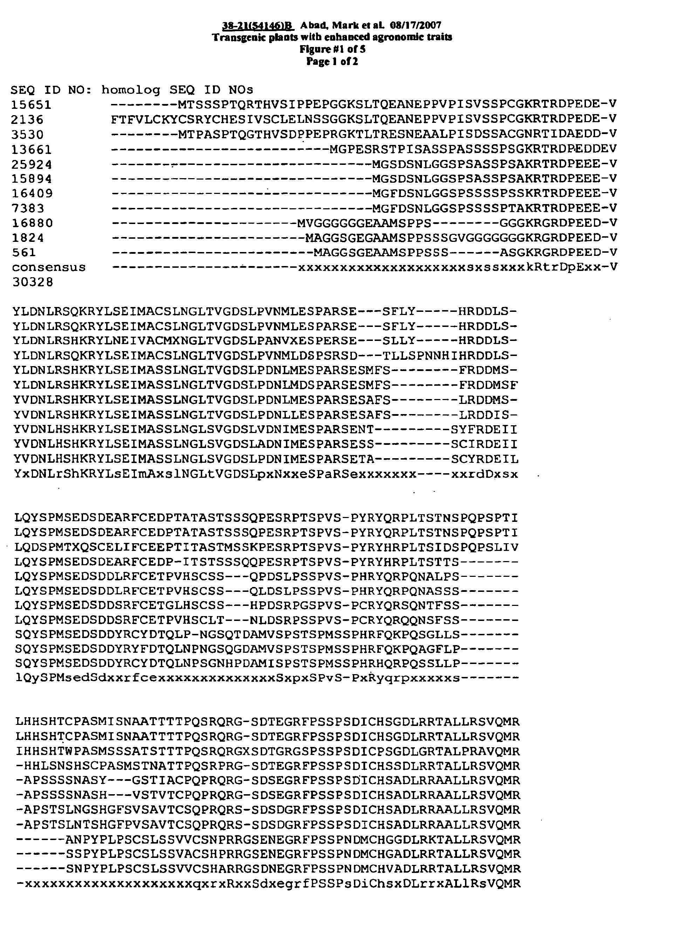

ClustalW program was selected for multiple sequence alignments of the amino acid sequence of SEQ ID NO: 561 and its 10 homologs. Three major factors affecting the sequence alignments dramatically are (1) protein weight matrices; (2) gap open penalty; (3) gap extension penalty. Protein weight matrices available for ClustalW program include Blosum, Pam and Gonnet series. Those parameters with gap open penalty and gap extension penalty were extensively tested. On the basis of the test results, Blosum weight matrix, gap open penalty of 10 and gap extension penalty of 1 were chosen for multiple sequence alignment. Figure 1 shows the sequences of SEQ ID NO: 561, its homologs and the consensus sequence (SEQ ID NO: 30328) at the end. The symbols for consensus sequence are (1) uppercase letters for 100% identity in all positions of multiple sequence alignment output; (2) lowercase letters for >=70% identity; symbol; (3) "X" indicated <70% identity; (4) dashes "-" meaning that gaps were in >=70% sequences.

The consensus amino acid sequence can be used to identify DNA corresponding to the full scope of this invention that is useful in providing transgenic plants, for example corn and soybean plants with enhanced agronomic traits, for example improved nitrogen use efficiency, improved yield, improved water use efficiency and/or improved growth under cold stress, due to the expression in the plants of DNA encoding a protein with amino acid sequence identical to the consensus amino acid sequence.

**Example 9. Identification of amino acid domain by Pfam analysis**

This example illustrates the identification of domain and domain module by Pfam analysis.

The amino acid sequence of the expressed proteins that were shown to be associated with an enhanced trait were analyzed for Pfam protein family against the current Pfam collection of multiple sequence alignments and hidden Markov models using the HMMER software. The Pfam domain modules and individual protein domain for the proteins of SEQ ID NO: 359 through 716 are shown in Table 14 and Table 15 respectively. The Hidden Markov model databases for the identified protein families are also allowing identification of other homologous proteins and their cognate encoding DNA to enable the full breadth of the invention for a person of ordinary skill in the art. Certain proteins are identified by a single Pfam domain and others by multiple Pfam domains. For instance, t For instance, the protein with amino acids of SEQ ID NO: 417 is characterized by two Pfam domains, i.e. HD and RelA_Spot.

In Table 15 "score" is the gathering score for the Hidden Markov Model of the domain which exceeds the gathering cutoff reported in Table 16.

Table 14

| PEP SEQ ID NO | Gene ID | Pfam domain module | domain coordinates |
|---|---|---|---|
| 591 | PHE0006505_7871.pep | Thioredoxin | 69-174 |
| 541 | PHE0006264_7285.pep | DAGAT | 48-349 |
| 359 | PHE0001295_7469.pep | DNA_photolyase::FAD_binding_7 | 18-190::223-501 |
| 665 | PHE0006760_8529.pep | vATP-synt_E | 16-225 |
| 639 | PHE0006684_8413.pep | Ribosomal_L10 | 19-123 |
| 645 | PHE0006715_8477.pep | AMPKBI | 197-287 |
| 626 | PHE0006600_8249.pep | Iso_dh | 6-355 |
| 484 | PHE0006071_7068.pep | PPR::PPR::PPR::PPR::PPR | 30-63::64-98::99-132::138-172::173-207 |
| 417 | PHE0004830_5828.pep | HD::RelA_SpoT | 233-337::427-537 |
| 576 | PHE0006426_8056.pep | AA_permease | 2-454 |
| 571 | PHE0006381_7655.pep | RNase_PH | 42-186 |
| 570 | PHE0006380_8719.pep | RNase_PH::RNase_PH_C | 1-126::129-201 |
| 713 | PHE0006977_9163-pep | Ribul_P_3_epim | 7-207 |
| 466 | PHE0006021_7077.pep | Bet_v_I | 1-155 |
| 596 | PHE0006516_7887.pep | CorA | 90-474 |
| 632 | PHE0006620_8462.pep | Epimerase | 13-259 |
| 631 | PHE0006617_8463.pep | Cupin_1 | 65-215 |
| 585 | PHE0006468_7903.pep | F-box::FBA_1 | 2-49::209-387 |
| 424 | PHE0004887_5939.pep | DUF516 | 49-310 |
| 478 | PHE0006059_7042.pep | DnaJ::DnaJ_C | 4-67::222-344 |

(continued)

| PEP SEQ ID NO | Gene ID | Pfam domain module | domain coordinates |
|---|---|---|---|
| 671 | PHE0006771_8551.pep | FAE1_CUT1_RppA::ACP_syn_III _C | 52-341::356-439 |
| 606 | PHE0006564_8298.pep | GATase_2::Asn_synthase | 2-161::209-450 |
| 695 | PHE0006934_9145.pep | DNA_pol_E_B | 178-389 |
| 391 | PHE0004670_6044.pep | GSHPx | 9-117 |
| 647 | PHE0006727_8435.pep | ETC_C1_NDUFA4 | 54-156 |
| 441 | PHE0004918_5975.pep | DUF1365 | 44-255 |
| 704 | PHE0006949_9179.pep | Aldedh | 19-478 |
| 409 | PHE0004808_5794.pep | Peptidase_C1 | 8-205 |
| 582 | PHE0006449_8165.pep | Biotin_lipoyl::E3_binding::2-oxoacid_dh | 92-165::229-265::281-512 |
| 538 | PHE0006234_7281.pep | Mg_chelatase::VWA | 85-295::559-754 |
| 383 | PHE0004398_5136.pep | Pkinase | 7-269 |
| 687 | PHE0006919_9008.pep | Peptidase_M16::Peptidase_M16_ C | 80-226::231-417 |
| 379 | PHE0004021_4654.pep | GATase_2::Asn_synthase | 2-173::227-460 |
| 387 | PHE0004503_5244.pep | MtN3_slv::MtN3_slv | 12-99::134-220 |
| 648 | PHE0006727_8595.pep | ETC_C1_NDUFA4 | 54-156 |
| 601 | PHE0006521_7840.pep | S6PP | 2-247 |
| 569 | PHE0006380_7658.pep | RNase_PH::RNase_PH_C | 1-126::129-201 |
| 525 | PHE0006209_7991.pep | HMGL-like::LeuA_dimer | 28-305::398-542 |
| 556 | PHE0006342_8182.pep | Hydrolase | 12-200 |
| 550 | PHE0006309_8148.pep | Glyoxalase | 2-123 |
| 515 | PHE0006178_7139.pep | eIF-5a | 82-149 |
| 453 | PHE0004989_8115.pep | DUF21::CBS | 14-191::210-325 |
| 443 | PHE0004928_5986.pep | Rotamase | 11-119 |
| 681 | PHE0006847_8860.pep | DHBP_synthase::GTP_cyclohydro 2 | 8-203::208-366 |
| 510 | PHE0006161_7221.pep | PFK::PFK | 195-506::585-877 |
| 468 | PHE0006043_7080.pep | Glyco_transf_8 | 83-345 |
| 710 | PHE0006963_9131.pep | Pyr_redox_2::Fer2_BFD::NIR_SIR _ferr::NIR_SIR | 5-287::422-474::556-623:: 631-777 |
| 565 | PHE0006377_7592.pep | RNase_PH::RNase_PH_C | 48-244::322-384 |
| 535 | PHE0006232_7454.pep | B_lectin::S_locus_glycop::PAN_2: :Pkinase_Tyr | 74-187::201-327::344-411:: 552-824 |
| 494 | PHE0006088_7063.pep | CoA_binding::Ligase_CoA | 634-743::784-929 |
| 438 | PHE0004909_5966.pep | Pkinase | 157-428 |
| 619 | PHE0006596_8236.pep | CTP_transf_2 | 19-159 |
| 536 | PHE0006232_8756.pep | B_lectin::S_locus_glycop::PAN_2: :Pkinase_Tyr | 74-187::201-327::344-411:: 552-824 |

(continued)

| PEP SEQ ID NO | Gene ID | Pfam domain module | domain coordinates |
|---|---|---|---|
| 407 | PHE0004806_5792.pep | OTU | 156-268 |
| 502 | PHE0006093_7327.pep | PTS_2-RNA | 48-239 |
| 411 | PHE0004810_5796.pep | Pkinase::efhand::efhand:: efhand:: efhand | 77-358::405-433::441-469:: 477-505::508-536 |
| 586 | PHE0006477_7809.pep | PsbR | 42-140 |
| 507 | PHE0006160_7286.pep | PFK | 3-309 |
| 362 | PHE0002132_8653.pep | Pkinase | 9-285 |
| 505 | PHE0006154_7204.pep | PfkB | 7-299 |
| 700 | PHE0006943_9124.pep | Aa_trans | 29-428 |
| 479 | PHE0006061_7051.pep | Metallophos | 2-120 |
| 492 | PHE0006079_7337.pep | S6PP::S6PP_C | 8-262::263-395 |
| 661 | PHE0006745_8590.pep | V-SNARE | 71-221 |
| 653 | PHE0006737_8527.pep | 2OG-FeII_Oxy | 217-317 |
| 688 | PHE0006929_9151.pep | zf-C3HC4 | 259-299 |
| 422 | PHE0004883_5935.pep | Pkinase | 314-581 |
| 686 | PHE0006912_9000.pep | ECH | 57-226 |
| 685 | PHE0006910_9019.pep | Mov34 | 92-201 |
| 674 | PHE0006788_8581.pep | Tryp_alpha_amyl | 27-110 |
| 599 | PHE0006517_7879.pep | CorA | 81-456 |
| 429 | PHE0004894_5950.pep | Tubulin::Tubulin_C | 52-245::247-369 |
| 439 | PHE0004911_5968.pep | Thioredoxin | 120-227 |
| 666 | PHE0006765_8536.pep | Bromodomain | 110-199 |
| 489 | PHE0006077_7045.pep | GASA | 5-106 |
| 590 | PHE0006498_7796.pep | Pyridoxal_deC | 34-381 |
| 396 | PHE0004762_5729.pep | F-box::LRR_2 | 62-108::314-340 |
| 369 | PHE0002810_5803.pep | p450 | 59-531 |
| 432 | PHE0004895_7135.pep | DS | 44-349 |
| 448 | PHE0004968_6030.pep | RLI::Fer4::ABC_tran::ABC_tran | 6-37::48-71::103-292::374-544 |
| 477 | PHE0006054_8779.pep | AIG1 | 39-236 |
| 380 | PHE0004143_7850.pep | GSHPx | 21-129 |
| 491 | PHE0006079_7044.pep | S6PP::S6PP_C | 8-262: :263-395 |
| 442 | PHE0004921_5979.pep | DUF1677 | 3-107 |
| 412 | PHE0004811_5798.pep | zf-C3HC4 | 164-205 |
| 469 | PHE0006043_8788.pep | Glyco_transf_8 | 83-345 |
| 435 | PHE0004902_5959.pep | Response_reg | 21-146 |
| 437 | PHE0004905_5962.pep | Pkinase | 78-336 |
| 635 | PHE0006669_8357.pep | PFK::PFK | 271-582::661-953 |
| 364 | PHE0002693_8516.pep | FAD_binding_3 | 55-374 |

(continued)

| PEP SEQ ID NO | Gene ID | Pfam domain module | domain coordinates |
|---|---|---|---|
| 454 | PHE0004991_8092.pep | Auxin_inducible | 19-119 |
| 460 | PHE0005008_6077.pep | Response_reg | 22-137 |
| 425 | PHE0004887_5940.pep | DUF516 | 49-310 |
| 467 | PHE0006021_8737.pep | Bet_v_I | 1-155 |
| 511 | PHE0006173_7211.pep | Ribosomal_S6e | 1-129 |
| 361 | PHE0002132_4965.pep | Pkinase | 9-285 |
| 587 | PHE0006478_8190.pep | Methyltransf_6 | 4-161 |
| 526 | PHE0006212_7196.pep | Heme_oxygenase | 74-278 |
| 431 | PHE0004895_5952.pep | DS | 44-349 |
| 709 | PHE0006962_9114.pep | Molybdop_Fe4S4:: Molybdopterin:: Molydop_ binding | 39-93::96-568::714-822 |
| 696 | PHE0006937_9126.pep | DUF298 | 127-242 |
| 610 | PHE0006586_8271.pep | Frataxin_Cyay | 76-187 |
| 385 | PHE0004473_5214.pep | Histone | 28-101 |
| 483 | PHE0006069_7065.pep | Cupin_3 | 62-137 |
| 659 | PHE0006742_8591.pep | PGI | 55-545 |
| 637 | PHE0006673_8992.pep | PTR2 | 123-530 |
| 604 | PHE0006555_8283.pep | Gp_dh_N::Gp_dh_C | 83-236::241-398 |
| 475 | PHE0006051_7097.pep | zf-MYND::UCH | 57-94::326-630 |
| 638 | PHE0006676_8410.pep | Transket_pyr::Transketolase_C | 39-215::232-354 |
| 612 | PHE0006590_8258.pep | Thioredoxin | 75-178 |
| 539 | PHE0006254_7312.pep | X8 | 29-115 |
| 513 | PHE0006175_7210.pep | KOW::eIF-5a | 27-63::85-154 |
| 428 | PHE0004894_5948.pep | Tubulin::Tubulin_C | 52-245::247-369 |
| 532 | PHE0006221_7241.pep | Pyr_redox_2::Glutaredoxin | 44-329::405-467 |
| 501 | PHE0006093_7066.pep | PTS_2-RNA | 48-239 |
| 609 | PHE0006574_8224.pep | Glyoxalase::Glyoxalase | 11-150::166-298 |
| 543 | PHE0006281_7526.pep | GAF::HisKA::HATPase_c:: Response_reg | 158-307::343-408::455-582:: 610-726 |
| 497 | PHE0006091_7074.pep | TFIIS_M::TFIIS_C | 206-327::338-376 |
| 520 | PHE0006202_7182.pep | HMGL-Nke::LeuA_dimer | 97-374::467-612 |
| 462 | PHE0005010_6079.pep | zf-DNL | 96-159 |
| 373 | PHE0003814_7802.pep | Chloroa_b-bind | 66-217 |
| 530 | PHE0006215_7280.pep | PFK | 2-277 |
| 493 | PWE0006082_7330.pep | TPR_1::TPR_2::TPR_1::TPR_ 2::T PR_1::TPR_1::TPR_1:: TPR_1::TP R_1 | 2-35::36-69::70-103::257-290:: 291-324::332-369::396-429:: 430-463::464-497 |
| 368 | PHE0002779_7478.pep | PGM_PMM_I::PGM_PMM_II:: PG M_PMM_III::PGM_PMM_IV | 16-165::199-314::316-439:: 477-571 |

(continued)

| PEP SEQ ID NO | Gene ID | Pfam domain module | domain coordinates |
|---|---|---|---|
| 593 | PHE0006514_7926.pep | ELFV_dehydrog_N::ELFV_dehydrog | 57-187::202-447 |
| 548 | PHE0006296_7515.pep | Glyco_transf_43 | 89-312 |
| 691 | PHE0006931_9168.pep | GDC-P | 3-443 |
| 621 | PHE0006597_8242.pep | Pkinase | 143-409 |
| 628 | PHE0006609_8234.pep | GSHPx | 12-120 |
| 630 | PHE0006613_8238.pep | GSHPx | 12-120 |
| 504 | PHE0006094_7333.pep | Chalcone | 14-225 |
| 634 | PHE0006666_8414_pep | Glycolytic | 43-387 |
| 698 | PHE0006940_9122.pep | Aldedh | 18-477 |
| 617 | PHE0006595_8250.pep | DUF537 | 18-156 |
| 527 | PHE0006213_7198.pep | Peptidase_C54 | 142-436 |
| 399 | PHE0004779_5749.pep | Ammonium_transp | 47-471 |
| 419 | PHE0004856_7855.pep | NPH3 | 193-435 |
| 549 | PHE0006309_7570pep | Glyoxalase | 2-123 |
| 451 | PHE0004984_7235.pep | AA_kinase::ACT::ACT | 83-366::403-478::479-546 |
| 588 | PHE0006497_8355.pep | DUF868 | 28-304 |
| 677 | PHE0006805_8531.pep | Ribosomal_S30AE | 2-95 |
| 574 | PHE0006382_8678.pep | WD40 | 282-319 |
| 705 | PHE0006952_9233.pep | PGAM | 91-277 |
| 652 | PHE0006737_8455pep | 20G-Fell_Oxy | 217-317 |
| 367 | PHE0002777_8726.pep | Fercochelatase | 108-432 |
| 405 | PHE0004791_5771.pep | Globin::FAD_binding_6::NAD_binding_1 | 7-131::154-254::263-373 |
| 575 | PHE0006425_7646.pep | AA_permease | 36-537 |
| 578 | PHE0006429_7671.pep | Globin | 18-158 |
| 522 | PHE0006204_7189.pep | Cyclin_N::Cyclin_C | 18-151::153-284 |
| 684 | PHE0006909_9003.pep | Cupin_3 | 62-137 |
| 559 | PHE0006348_8203.pep | DUF6::TPT | 106-231::240-385 |
| 650 | PHE0006729_8433.pep | DnaJ::zf-CSL | 12-81::96-174 |
| 703 | PHE0006949_9133.pep | Aldedh | 19-478 |
| 533 | PHE0006221_7937.pep | Pyr_redox_2::Glutaredoxin | 44-329::405-467 |
| 458 | PHE0005002_6071.pep | Methyltransf_12::Mg-por_mtran_C | 155-252::223-319 |
| 690 | PHE0006931_9148.pep | GDC-P | 3-443 |
| 416 | PHE0004827_5825.pep | Phi_1 | 40-315 |
| 583 | PHE0006450_7624pep | Tubulin::Tubulin_C | 57-250::252-369 |
| 605 | PHE0006559_8227.pep | PEPcase | 1-948 |
| 433 | PHE0004895_7137.pep | DS | 44-349 |

(continued)

| PEP SEQ ID NO | Gene ID | Pfam domain module | domain coordinates |
|---|---|---|---|
| 669 | PHE0006770_8553.pep | DEAD::Helicase_C | 58-224::292-368 |
| 667 | PHE0006766_8867.pep | IPT | 1-235 |
| 594 | PHE0006516_7866.pep | CorA | 90-474 |
| 473 | PHE0006048_8785.pep | Pkinase_Tyr | 135-389 |
| 374 | PHE0003838_5934.pep | zf-LSD1::zf-LSD1::zf-LSD1 | 28-52::67-91::105-129 |
| 464 | PHE0006003_7205.pep | zf-AN1 | 105-145 |
| 618 | PHE0006595_8265.pep | DUF537 | 18-156 |
| 365 | PHE0002777_7490.pep | Ferrochelatase | 108-432 |
| 629 | PHE0006610_8239.pep | GSHPx | 77-185 |
| 531 | PHE0006221_7201.pep | Pyr_redox_2::Glutaredoxin | 44-329::405-467 |
| 476 | PHE0006054_7095.pep | AIG1 | 39-236 |
| 393 | PHE0004742_5691.pep | AP2 | 43-108 |
| 404 | PHE0004787_7988.pep | P-II | 85-187 |
| 410 | PHE0004809_5795.pep | PP2C | 46-324 |
| 434 | PHE0004895_8610.pep | DS | 44-349 |
| 415 | PHE0004815_5802.pep | Pkinase_Tyr | 334-585 |
| 613 | PHE0006591_8264.pep | Thioredoxin | 81-184 |
| 455 | PHE0004993_6062.pep | CCT | 237-275 |
| 689 | PHE0006929_9185.pep | zf-C3HC4 | 259-299 |
| 375 | PHE0003845_5806.pep | p450 | 40-509 |
| 498 | PHE0006091_7341.pep | TFIIS_M::TFIIS_C | 206-327::338-376 |
| 592 | PHE0006506_7818.pep | Pkinase::UBA::KA1 | 20-272::294-333::463-511 |
| 384 | PHE0004398_5757.pep | Pkinase | 7-269 |
| 557 | PHE0006344_8188.pep | VQ | 44-74 |
| 702 | PHE0006948_9160.pep | RRM_1 | 38-109 |
| 682 | PHE0006870_8846.pep | Ribosomal_L37ae | 2-91 |
| 423 | PHE0004886_5938.pep | DUF516 | 49-313 |
| 589 | PHE0006498_7795.pep | Pyridoxal_deC | 34-381 |
| 602 | PHE0006545_8320.pep | DnaJ | 31-93 |
| 641 | PHE0006686_8416.pep | Ribosomal_L22 | 17-153 |
| 572 | PHE0006381_8695pep | RNase_PH | 42-186 |
| 529 | PHE0006214_7219.pep | Cyclin_N::Cyclin_C | 32-158::160-289 |
| 581 | PHE0006449_7865.pep | Biotin_lipoyl::E3_binding::2-oxoacid_dh | 92-165::229-265::281-512 |
| 607 | PHE0006565_8300.pep | GATase_2::Asn_synthase | 2-161::209-450 |
| 664 | PHE0006757_8530.pep | Acyltransferase | 375-496 |
| 603 | PHE0006549_8255.pep | THF_DHG_CYH::THF_DHG_CYH _C | 3-120::123-290 |

(continued)

| PEP SEQ ID NO | Gene ID | Pfam domain module | domain coordinates |
|---|---|---|---|
| 658 | PHE0006742_8440.pep | PGI | 55-545 |
| 524 | PHE0006208_7223.pep | CH::EB1 | 19-120::204-251 |
| 657 | PHE0006741_8589.pep | MATH::BTB | 53-182::206-328 |
| 408 | PHE0004807_5793.pep | RRM_1 | 13-84 |
| 456 | PHE0004993_8014.pep | CCT | 237-275 |
| 693 | PHE0006932_9174.pep | DUF498 | 56-164 |
| 414 | PHE0004813_5800.pep | zf-CCCH::zf-CCCH::zf-CCCH::zf-CCCH::zf-CCCH | 74-100::119-145::165-191::317-343::363-389 |
| 512 | PHE0006174_7208.pep | RRM_1::RRM_1 | 170-241::269-340 |
| 670 | PHE0006770_8568.pep | DEAD::Helicase_C | 58-224::292-368 |
| 506 | PHE0006160_7265.pep | PFK | 3-309 |
| 646 | PHE0006716_8482.pep | NOI | 1-72 |
| 392 | PHE0004683_8693.pep | ThiF | 30-167 |
| 500 | PHE0006092_7336.pep | RRM_1::RRM_1::RRM_1 | 65-132::150-225::275-343 |
| 388 | PHE0004503_8801.pep | MtN3_slv::MtN3_slv | 12-99::134-220 |
| 452 | PHE0004984_8782.pep | AA_kinase::ACT::ACT | 83-366::403-478::479-546 |
| 516 | PHE0006178_8626.pep | eIF-5a | 82-149 |
| 521 | PHE0006204_7183.pep | Cyclin_N::Cyclin_C | 18-151::153-284 |
| 636 | PHE0006670_8346.pep | PfkB | 83-375 |
| 366 | PHE0002777_8472.pep | Ferrochelatase | 108-432 |
| 376 | PHE0003845_7028.pep | p450 | 40-509 |
| 598 | PHE0006517_7858.pep | CorA | 81-456 |
| 651 | PHE0006730_8428.pep | Lung_7.TM_R | 168-423 |
| 381 | PHE0004143_8160.pep | GSHPx | 23-131 |
| 426 | PHE0004887_8704.pep | DUF516 | 49-310 |
| 440 | PHE0004912_5969.pep | Pkinase | 162-434 |
| 620 | PHE0006596_8257.pep | CTP_transf_2 | 19-159 |
| 692 | PHE0006932_9147.pep | DUF498 | 56-164 |
| 418 | PHE0004845_5852.pep | Carotene_hydrox | 136-295 |
| 406 | PHE0004805_5791.pep | DUF751 | 125-188 |
| 540 | PHE0006263_7271.pep | DAGAT | 48-324 |
| 398 | PHE0004766_5733.pep | UPF0051 | 275-515 |
| 643 | PHE0006706_8434.pep | DEAD::Helicase_C | 46-212::280-356 |
| 495 | PHE0006089_7061.pep | Brix | 60-255 |
| 519 | PHE0006201_7187.pep | ketoacyl-synt::Ketoacyl-synt_C | 47-309::317-477 |
| 447 | PHE0004966_6028.pep | Sugar_tr | 101-556 |
| 577 | PHE0006428_7651.pep | Globin | 21-161 |
| 449 | PHE0004977_6043.pep | DAGAT | 54-352 |

(continued)

| PEP SEQ ID NO | Gene ID | Pfam domain module | domain coordinates |
|---|---|---|---|
| 509 | PHE0006161_7215.pep | PFK::PFK | 195-506::585-877 |
| 706 | PHE0006953_9121.pep | Usp | 3-157 |
| 413 | PHE0004812_5799.pep | Sigma70_r2::Sigma70_r3:: Sigma70_r4 | 267-340::343-424::436-489 |
| 389 | PHE0004641_5519.pep | malic::Malic_M | 162-350::352-605 |
| 668 | PHE0006769_8865.pep | TPP_enzyme_N::TPP_ enzyme_M ::TPP_enzyme_C | 3-172::190-336::379-525 |
| 496 | PHE0006089_7334.pep | Brix | 60-255 |
| 649 | PHE0006728_8430.pep | RRM_1 | 111-190 |
| 663 | PHE0006750_8523.pep | zf-C3HC4::WD40::WD40:: WD40 | 52-89::454-492::496-534:: 540-576 |
| 463 | PHE0006003_7195.pep | zf-AN1 | 105-145 |
| 597 | PHE0006516_8363.pep | CorA | 90-474 |
| 461 | PHE0005009_6078.pep | UQ_con | 40-177 |
| 534 | PHE0006227_7282.pep | NB-ARC::LRR_1::LRR_1:: LRR_1 | 152-421::652-674::676-698:: 700-722 |
| 551 | PHE0006309_8620.pep | Glyoxalase | 2-123 |
| 554 | PHE0006312_7579.pep | UPF0113 | 1-175 |
| 608 | PHE0006571_8279.pep | Pkinase | 15-273 |
| 382 | PHE0004311_5022.pep | Peptidase_M24 | 354-577 |
| 472 | PHE0006048_7094.pep | Pkinase_Tyr | 135-389 |
| 397 | PHE0004762_7997.pep | F-box::LRR_2 | 62-108::314-340 |
| 390 | PHE0004642_5520.pep | malic::Malic_M | 170-358::360-613 |
| 518 | PHE0006201_7184.pep | ketoacyl-synt::Ketoacyl-synt_C | 47-309::317-477 |
| 662 | PHE0006746_8453.pep | Sugar_tr | 33-464 |
| 528 | PHE0006214_7213.pep | Cyclin_N::Cyclin_C | 32-158::160-289 |
| 503 | PHE0006094_7231.pep | Chalcone | 14-225 |
| 445 | PHE0004941_5997.pep | Dehydrin | 14-128 |
| 370 | PHE0002857_7502.pep | Chloroa_b-bind | 66-183 |
| 627 | PHE0006607_8231.pep | SRF-TF | 11-66 |
| 676 | PHE0006794_8578.pep | SSB | 71-182 |
| 600 | PHE0006517_7897.pep | CorA | 81-456 |
| 694 | PHE0006933_9139.pep | adh_short | 30-212 |
| 555 | PHE0006312_8644.pep | UPF0113 | 1-175 |
| 624 | PHE0006599_8230pep | ZF-HD_dimer | 34-93 |
| 580 | PHE0006439_8108.pep | RRM_1 | 23-94 |
| 430 | PHE0004894_5951.pep | Tubulin::Tubulin_C | 52-245::247-369 |
| 377 | PHE0003845_7413.pep | p450 | 40-509 |
| 482 | PHE0006068_7064.pep | Pkinase | 2-222 |

(continued)

| PEP SEQ ID NO | Gene ID | Pfam domain module | domain coordinates |
|---|---|---|---|
| 656 | PHE0006741_8448.pep | AAATH::BTB | 53-182::206-328 |
| 579 | PHE0006433_8307.pep | PseudoU_synth_2 | 105-284 |
| 675 | PWE0006793_8580pep | p450 | 27-508 |
| 508 | PHE0006160_8851.pep | PFK | 3-309 |
| 595 | PHE0006516_7882.pep | CorA | 90-474 |
| 614 | PHE0006592_8278.pep | Thioredoxin | 87-190 |
| 633 | PHE0006648_8356.pep | Tryp_alpha_amyl | 36-114 |
| 678 | PHE0006811_8506.pep | Bac_globin | 3-122 |
| 672 | PHE0006775_8548.pep | Ras | 10-178 |
| 403 | PHE0004784_5760.pep | SAM_decarbox | 3-333 |
| 542 | PHE0006265_7990.pep | Heme_oxygenase | 88-279 |
| 566 | PHE0006377_8683.pep | RNase_PH::RNase_PH_C | 48-244::322-384 |
| 459 | PHE0005003_7032.pep | Porphobil_deam::Porphobil_ deam C | 47-263::271-347 |
| 450 | PHE0004979_6047.pep | Glyco_hydro_32N::Glyco_ hydro_3 2C | 32-342::395-492 |
| 363 | PHE0002133_7497.pep | Pkinase | 13-273 |
| 644 | PHE0006709_8432.pep | MtN3_slv::MtN3_slv | 9-98::132-218 |
| 490 | PHE0006077_7343.pep | GASA | 5-106 |
| 537 | PHE0006233_7220.pep | Mg_chelatase | 87-295 |
| 552 | PHE0006310_7574.pep | Pkinase | 13-304 |
| 623 | PHE0006598_8268.pep | Di19 | 11-219 |
| 564 | PHE0006356_8103.pep | F-box::Kelch_1::Kelch_1 | 42-89::180-225::227-282 |
| 673 | PHE0006775_8555.pep | Ras | 10-178 |
| 394 | PHE0004747_5708.pep | Aldedh | 99-565 |
| 523 | PHE0006204_8634.pep | Cyclin_N::Cyclin_C | 18-151::153-284 |
| 697 | PHE0006938_9149.pep | F-box | 41-88 |
| 465 | PHE0006018_7098.pep | GTP_EFTU::GTP_EFTU_D2:: GT P_EFTU_D3 | 64-260::281-352::357-451 |
| 642 | PHE0006687_8471.pep | Ribosomal_L32e | 14-123 |
| 611 | PHE0006587_8277.pep | CP12 | 60-131 |
| 622 | PHE0006598_8240.pep | Di19 | 11-219 |
| 625 | PHE0006599_8262.pep | ZF-HD_dimer | 34-93 |
| 457 | PHE0004993_8682.pep | CCT | 237-275 |
| 584 | PHE0006464_8089.pep | DREPP | 2-203 |
| 573 | PHE0006382_7652.pep | WD40 | 282-319 |
| 701 | PHE0006948_9125.pep | RRM_1 | 38-109 |
| 499 | PHE0006092_7062.pep | RRM_1::RRM_1::RRM_1 | 65-132::150-225::275-343 |
| 481 | PHE0006063_7049.pep | Transket_pyr::Transketolase_C | 76-252::265-387 |

(continued)

| PEP SEQ ID NO | Gene ID | Pfam domain module | domain coordinates |
|---|---|---|---|
| 400 | PHE0004779_8394.pep | Ammonium_transp | 47-471 |
| 711 | PHE0006965_9119.pep | tRNA-synt_2b::HGTP_ anticodon | 67-243::312-409 |
| 517 | PHE0006184_7245.pep | DUF125 | 34-247 |
| 360 | PHE0002129_8308.pep | PEPcase | 3-982 |
| 444 | PHE0004932_6045.pep | PurA | 28-275 |
| 386 | PHE0004473_8803.pep | Histone | 28-101 |
| 660 | PHE0006744_8449.pep | adh_short | 37-225 |

Table 15.

| PEP SEQ ID NO | GENE ID | Pfam domain name | begin | stop | score | E-value |
|---|---|---|---|---|---|---|
| 359 | PHE0001295_7469 pep | DNA_photolyase | 18 | 190 | 254.3 | 2.30E-73 |
| 359 | PHE0001295_7469.pep | FAD_binding_7 | 223 | 501 | 503 | 3.20E-148 |
| 360 | PHE0002129_8308pep | PEPcase | 3 | 982 | 425.8 | 5.30E-125 |
| 361 | PHE0002132_4965.pep | Pkinase | 9 | 285 | 234.9 | 1.60E-67 |
| 362 | PHE0002132_8653.pep | Pkinase | 9 | 285 | 234.9 | 1.60E-67 |
| 363 | PHE0002133_7497.pep | Pkinase | 13 | 273 | 288.2 | 1.40E-83 |
| 364 | PHE0002693_8516.pep | FAD binding_3 | 55 | 374 | -131.4 | 0.0029 |
| 365 | PHE0002777_7490 pep | Ferrochelatase | 108 | 432 | 598.9 | 4.30E-177 |
| 366 | PHE0002777_8472.pep | Ferrochelatase | 108 | 432 | 598.9 | 4.30E-177 |
| 367 | PHE0002777_8726.pep | Ferrochelatase | 108 | 432 | 598.9 | 4.30E-177 |
| 368 | PHE0002779_7478.pep | PGM PMM_I | 16 | 165 | 154.6 | 2.40E-43 |
| 368 | PHE0002779_7478.pep | PGM PMM_II | 199 | 314 | 108.5 | 1.80E-29 |
| 368 | PHE0002779_7478.pep | PGM PMM_III | 316 | 439 | 136.8 | 5.30E-38 |
| 368 | PHE0002779_7478.pep | PGM PMM_IV | 477 | 571 | 77.3 | 4.30E-20 |
| 369 | PHE0002810_5803.pep | p450 | 59 | 531 | 323.8 | 2.80E-94 |
| 370 | PHE0002857_7502.pep | Chloroa_b-bind | 66 | 183 | -26.1 | 0.0016 |
| 373 | PHE0003814_7802.pep | Chloroa_b-bind | 66 | 217 | 66.4 | 8.20E-17 |
| 374 | PHE0003838_5934.pep | zf-LSD1 | 28 | 52 | 40.3 | 6.10E-09 |
| 374 | PHE0003838_5934.pep | zf-LSD1 | 67 | 91 | 54.8 | 2.60E-13 |
| 374 | PHE0003838_5934.pep | zf-LSD1 | 105 | 129 | 54.1 | 4.30E-13 |
| 375 | PHE0003845_5806.pep | p450 | 40 | 509 | 127.1 | 4.50E-35 |
| 376 | PHE0003845_7028.pep | p450 | 40 | 509 | 127.1 | 4.50E-35 |
| 377 | PHE0003845_7413.pep | p450 | 40 | 509 | 127.1 | 4.50E-35 |
| 379 | PHE0004021_4654.pep | GATase 2 | 2 | 173 | -29 | 7.60E-09 |
| 379 | PHE0004021_4654.pep | Asn synthase | 227 | 460 | 295.7 | 7.70E-86 |
| 380 | PHE0004143_7850.pep | Redoxin | 12 | 176 | 4.9 | 0.0016 |
| 380 | PHE0004143_7850.pep | GSHPx | 21 | 129 | 246.5 | 5.10E-71 |

(continued)

| PEP SEQ ID NO | GENE ID | Pfam domain name | begin | stop | score | E-value |
|---|---|---|---|---|---|---|
| 381 | PHE0004143_8160.pep | Redoxin | 14 | 178 | 4.9 | 0.0016 |
| 381 | PHE0004143_8160.pep | GSHPx | 23 | 131 | 246.5 | 5.10E-71 |
| 382 | PHE0004311_5022.pep | Peptidase M24 | 354 | 577 | 12.2 | 3.20E-09 |
| 383 | PHE0004398_5136-pep | Pkinase | 7 | 269 | 341.5 | 1.30E-99 |
| 383 | PHE0004398_5136.pep | Pkinase, Tyr | 7 | 269 | 155.9 | 9.80E-44 |
| 384 | PHE0004398_5757.pep | Pkinase | 7 | 269 | 341.5 | 1.30E-99 |
| 384 | PHE0004398_5757.pep | Pkinase Tyr | 7 | 269 | 155.9 | 9.80E-44 |
| 385 | PHE0004473_5214.pep | Histone | 28 | 101 | 104.2 | 3.60E-28 |
| 386 | PHE0004473_8803.pep | Histone | 28 | 101 | 104.2 | 3.60E-28 |
| 387 | PHE0004503_5244.pep | MtN3 slv | 12 | 99 | 135.1 | 1.80E-37 |
| 387 | PHE0004503_5244.pep | MtN3 slv | 134 | 220 | 135.4 | 1.40E-37 |
| 388 | PHE0004503_8801.pep | MtN3 slv | 12 | 99 | 135.1 | 1.80E-37 |
| 388 | PHE0004503_8801.pep | MtN3 slv | 134 | 220 | 135.4 | 1.40E-37 |
| 389 | PHE0004641_5519.pep | malic | 162 | 350 | 392.4 | 6.10E-115 |
| 389 | PHE0004641_5519 pep | Malic M | 352 | 605 | 486.9 | 2.20E-143 |
| 390 | PHE0004642_5520.pep | malic | 170 | 358 | 402.6 | 5.40E-118 |
| 390 | PHE0004642_5520 pep | Malic M | 360 | 613 | 483.8 | 1.90E-142 |
| 391 | PHE0004670_6044.pep | GSHPx | 9 | 117 | 230.9 | 2.60E-66 |
| 392 | PHE0004683_8693.pep | ThiF | 30 | 167 | -12.5 | 3.60E-05 |
| 393 | PHE0004742_5691.pep | AP2 | 43 | 108 | 93.1 | 7.50E-25 |
| 394 | PHE0004747_5708.pep | Aldedh | 99 | 565 | 568.6 | 5.60E-168 |
| 396 | PHE0004762_5729.pep | F-box | 62 | 108 | 15.1 | 0.22 |
| 396 | PHE0004762_5729.pep | LRR_2 | 314 | 340 | 17.1 | 0.058 |
| 397 | PHE0004762_7997.pep | F-box | 62 | 108 | 15.1 | 0.22 |
| 397 | PHE0004762_7997.pep | LRR_2 | 314 | 340 | 17.1 | 0.058 |
| 398 | PHE0004766_5733.pep | UPF0051 | 275 | 515 | 465.7 | 5.30E-137 |
| 399 | PHE0004779_5749.pep | Ammonium_transp | 47 | 471 | 644.8 | 6.60E-191 |
| 400 | PHE0004779_8394.pep | Ammonium_transp | 47 | 471 | 644.8 | 6.60E-191 |
| 403 | PHE0004784_5760.pep | SAM decarbox | 3 | 333 | 694.5 | 7.10E-206 |
| 404 | PHE0004787_7988.pep | P-II | 85 | 187 | 176 | 8.50E-50 |
| 405 | PHE0004791_5771.pep | Globin | 7 | 131 | 80.7 | 4.20E-21 |
| 405 | PHE0004791_5771.pep | FAD binding_6 | 154 | 254 | 44.1 | 4.20E-10 |
| 405 | PHE0004791_5771.pep | NAD binding_1 | 263 | 373 | 45 | 2.40E-10 |
| 406 | PHE0004805_5791.pep | DUF751 | 125 | 188 | 62.4 | 1.40E-15 |
| 407 | PHE0004806_5792.pep | OTU | 156 | 268 | 141.1 | 2.60E-39 |
| 408 | PHE0004807_5793.pep | RRM_1 | 13 | 84 | 114.1 | 3.80E-31 |
| 409 | PHE0004808_5794.pep | Peptidase_C1 | 8 | 205 | 327.1 | 2.80E-95 |
| 410 | PHE0004809_5795.pep | PP2C | 46 | 324 | 111.1 | 3.00E-30 |

(continued)

| PEP SEQ ID NO | GENE ID | Pfam domain name | begin | stop | score | E-value |
|---|---|---|---|---|---|---|
| 411 | PHE0004810_5796.pep | Pkinase | 77 | 358 | 332.4 | 7.00E-97 |
| 411 | PHE0004810_5796.pep | efhand | 405 | 433 | 26 | 0.00012 |
| 411 | PHE0004810_5796.pep | efhand | 441 | 469 | 26.3 | 9.80E-05 |
| 411 | PHE0004810_5796.pep | efhand | 477 | 505 | 21 | 0.0038 |
| 411 | PHE0004810_5796.pep | efhand | 508 | 536 | 34.1 | 4.40E-07 |
| 412 | PHE0004811_5798.pep | zf-C3HC4 | 164 | 205 | 37.6 | 3.90E-08 |
| 413 | PHE0004812_5799.pep | sigma70_r2 | 267 | 340 | 49.2 | 1.30E-11 |
| 413 | PHE0004812_5799.pep | Sigma70_r3 | 343 | 424 | 52.3 | 1.50E-12 |
| 413 | PHE0004812_5799.pep | Sigma70_r4 | 436 | 489 | 66.5 | 7.70E-17 |
| 414 | PHE0004813_5800.pep | zf-CCCH | 74 | 100 | 42.6 | 1.20E-09 |
| 414 | PHE0004813_5800 pep | zf-CCCH | 119 | 145 | 42.4 | 1.40E-09 |
| 414 | PHE0004813_5800.pep | zf-CCCH | 165 | 191 | 38 | 2.90E-08 |
| 414 | PHE0004813_5800.pep | zf-CCCH | 317 | 343 | 46.8 | 6.70E-11 |
| 414 | PHE0004813_5800.pep | zf-CCCH | 363 | 389 | 48.2 | 2.60E-11 |
| 415 | PHE0004815_5802.pep | Pkinase | 334 | 585 | 35.3 | 4.90E-10 |
| 415 | PHE0004815_5802.pep | Pkinase_Tyr | 334 | 585 | 77.5 | 1.60E-20 |
| 416 | PHE0004827_5825.pep | Phi_1 | 40 | 315 | 567.5 | 1.20E-167 |
| 417 | PHE0004830_5828.pep | HD | 233 | 337 | 53.6 | 6.10E-13 |
| 417 | PHE0004830_5828.pep | RelA SpoT | 427 | 537 | 165 | 1.80E-46 |
| 418 | PHE0004845_5852.pep | Carotene hydrox | 136 | 295 | 339.2 | 6.30E-99 |
| 419 | PHE0004856_7855.pep | NPH3 | 193 | 435 | 469.9 | 2.90E-138 |
| 422 | PHE0004883_5935.pep | Pkinase | 314 | 581 | 148.8 | 1.30E-41 |
| 422 | PHE0004883_5935.pep | Pkinase Tyr | 315 | 581 | 104 | 4.10E-28 |
| 423 | PHE0004886_5938.pep | DUF516 | 49 | 313 | 561.2 | 9.10E-166 |
| 424 | PHE0004887_5939.pep | DUF516 | 49 | 310 | 356.9 | 2.90E-104 |
| 425 | PHE0004887_5940.pep | DUF516 | 49 | 310 | 356.9 | 2.90E-104 |
| 426 | PHE0004887_8704.pep | DUF516 | 49 | 310 | 356.9 | 2.90E-104 |
| 428 | PHE0004894_5948.pep | Tubulin | 52 | 245 | 339.5 | 5.20E-99 |
| 428 | PHE0004894_5948.pep | Tubulin C | 247 | 369 | 96.5 | 7.20E-26 |
| 429 | PHE0004894_5950.pep | Tubulin | 52 | 245 | 339.5 | 5.20E-99 |
| 429 | PHE0004894_5950.pep | Tubulin C | 247 | 369 | 96.5 | 7.20E-26 |
| 430 | PHE0004894_5951.pep | Tubulin | 52 | 245 | 339.5 | 5.20E-99 |
| 430 | PHE0004894_5951.pep | Tubulin C | 247 | 369 | 96.5 | 7.20E-26 |
| 431 | PHE0004895_5952.pep | DS | 44 | 349 | 713.1 | 1.80E-211 |
| 432 | PHE0004895_7135.pep | DS | 44 | 349 | 713.1 | 1.80E-211 |
| 433 | PHE0004895_7137.pep | DS | 44 | 349 | 713.1 | 1.80E-211 |
| 434 | PHE0004895_8610.pep | DS | 44 | 349 | 713.1 | 1.80E-211 |
| 435 | PHE0004902_5959.pep | Response_reg | 21 | 146 | 77.4 | 4.00E-20 |

(continued)

| PEP SEQ ID NO | GENE ID | Pfam domain name | begin | stop | score | E-value |
|---|---|---|---|---|---|---|
| 437 | PHE0004905_5962.pep | Pkinase | 78 | 336 | 354.5 | 1.50E-103 |
| 438 | PHE0004909_5966.pep | Pkinase | 157 | 428 | 148.5 | 1.60E-41 |
| 438 | PHE0004909_5966.pep | Pkinase_Tyr | 157 | 428 | 139.3 | 9.50E-39 |
| 439 | PHE0004911_5968.pep | Thioredoxin | 120 | 227 | 56.1 | 1.10E-13 |
| 440 | PHE0004912_5969.pep | Pkinase | 162 | 434 | 125.2 | 1.70E-34 |
| 440 | PHE0004912_5969.pep | Pkinase_Tyr | 162 | 434 | 115.7 | 1.20E-31 |
| 441 | PHE0004918_5975.pep | DUF1365 | 44 | 255 | 407.9 | 1.30E-119 |
| 442 | PHE0004921_5979.pep | DUF1677 | 3 | 107 | 192-2 | 1.20E-54 |
| 443 | PHE0004928_5986.pep | Rotamase | 11 | 119 | 143.6 | 4.90E-40 |
| 444 | PHE0004932_6045.pep | PurA | 28 | 275 | 44.4 | 5.80E-12 |
| 445 | PHE0004941_5997.pep | Dehydrin | 14 | 128 | 165.3 | 1.40E-46 |
| 447 | PHE0004966_6028.pep | Sugar_tr | 101 | 556 | 315.4 | 9.20E-92 |
| 447 | PHE0004966_6028.pep | MFS_1 | 105 | 515 | 81.2 | 2.90E-21 |
| 448 | PHE0004968_6030.pep | RL1 | 6 | 37 | 55.4 | 1.70E-13 |
| 448 | PHE0004968_6030.pep | Fer4 | 48 | 71 | 39.3 | 1.20E-08 |
| 448 | PHE0004968_6030.pep | ABC_tran | 103 | 292 | 96.8 | 5.90E-26 |
| 448 | PHE0004968_6030.pep | ABC_tran | 374 | 544 | 85.8 | 1.30E-22 |
| 449 | PHE0004977_6043.pep | DAGAT | 54 | 352 | 405.8 | 5.80E-119 |
| 450 | PHE0004979_6047.pep | Glyco_hydro_32N | 32 | 342 | 430.7 | 1.80E-126 |
| 450 | PHE0004979_6047.pep | Glyco_hydro_32C | 395 | 492 | 42.6 | 1.20E-09 |
| 451 | PHE0004984_7235.pep | AA_kinase | 83 | 366 | 224.7 | 1.80E-64 |
| 451 | PHE0004984_7235.pep | ACT | 403 | 478 | 28.1 | 2.80E-05 |
| 451 | PHE0004984_7235.pep | ACT | 479 | 546 | 24 | 0.0005 |
| 452 | PHE0004984_8782.pep | AA_kinase | 83 | 366 | 224.7 | 1.80E-64 |
| 452 | PHE0004984_8782.pep | ACT | 403 | 478 | 28.1 | 2.80E-05 |
| 452 | PHE0004984_8782.pep | ACT | 479 | 546 | 24 | 0.0005 |
| 453 | PHE0004989_8115.pep | DUF21 | 14 | 191 | 174.7 | 2.10E-49 |
| 453 | PHE0004989_8115.pep | CBS | 210 | 325 | 33.9 | 5.30E-07 |
| 454 | PHE0004991_8092.pep | Auxin_inducible | 19 | 119 | 55.4 | 1.70E-13 |
| 455 | PHE0004993_6062.pep | CCT | 237 | 275 | 74 | 4.40E-19 |
| 456 | PHE0004993_8014.pep | CCT | 237 | 275 | 74 | 4.40E-19 |
| 457 | PHE0004993_8682.pep | CCT | 237 | 275 | 74 | 4.40E-19 |
| 458 | PHE0005002_6071.pep | Methyltransf_11 | 155 | 252 | 44.7 | 3.00E-10 |
| 458 | PHE0005002_6071.pep | Methyltransf_12 | 155 | 252 | 59.7 | 8.50E-15 |
| 458 | PHE0005002_6071.pep | Mg-por_mtran_C | 223 | 319 | 198.4 | 1.50E-56 |
| 459 | PHE0005003_7032.pep | Porphobil_deam | 47 | 263 | 451.7 | 8.90E-133 |
| 459 | PHE0005003_7032.pep | Porphobil_deamC | 271 | 347 | 100.4 | 4.80E-27 |
| 460 | PHE0005008_6077.pep | Response_reg | 22 | 137 | 70.4 | 5.10E-18 |

(continued)

| PEP SEQ ID NO | GENE ID | Pfam domain name | begin | stop | score | E-value |
|---|---|---|---|---|---|---|
| 461 | PHE0005009_6078.pep | UQ_con | 40 | 177 | 216.1 | 7.20E-62 |
| 462 | PHE0005010_6079.pep | zf-DNL | 96 | 159 | 102.2 | 1.40E-27 |
| 463 | PHE0006003_7195.pep | zf-AN1 | 105 | 145 | 73.2 | 7.60E-19 |
| 464 | PHE0006003_7205.pep | zf-AN1 | 105 | 145 | 73.2 | 7.60E-19 |
| 465 | PHE0006018_7098.pep | GTP_EFTU | 64 | 260 | 334.6 | 1.60E-97 |
| 465 | PHE0006018_7098.pep | GTP EFTU D2 | 281 | 352 | 87 | 5.30E-23 |
| 465 | PHE0006018 7098.pep | GTP EFTU D3 | 357 | 451 | 186.5 | 5.80E-53 |
| 466 | PHE0006021 7077.pep | Bet_v_I | 1 | 155 | 11.1 | 6.70E-07 |
| 467 | PHE0006021 8737.pep | Bet_v_I | 1 | 155 | 11.1 | 6.70E-07 |
| 468 | PHE0006043_7080.pep | Glyco transf 8 | 83 | 345 | 341.2 | 1.60E-99 |
| 469 | PHE0006043 8788.pep | Glyco, transf 8 | 83 | 345 | 341.2 | 1.60E-99 |
| 472 | PHE0006048 7094 pep | Pkinase | 135 | 389 | 219.6 | 6.60E-63 |
| 472 | PHE0006048 7094.pep | Pkinase Tyr | 135 | 389 | 257.1 | 3.40E-74 |
| 473 | PHE0006048 8785.pep | Pkinase | 135 | 389 | 219.6 | 6.60E-63 |
| 473 | PHE0006048 8785.pep | Pkinase Tyr | 135 | 389 | 257.1 | 3.40E-74 |
| 475 | PHE0006051 7097.pep | zf-MYND | 57 | 94 | 50.2 | 6.20E-12 |
| 475 | PHE0006051 7097.pep | UCH | 326 | 630 | 176.1 | 8.20E-50 |
| 476 | PHE0006054 7095.pep | AIG1 | 39 | 236 | 212.9 | 6.60E-61 |
| 476 | PHE0006054 7095.pep | MMR HSR1 | 39 | 154 | 31.5 | 1.20E-06 |
| 477 | PHE0006054 8779.pep | AIG1 | 39 | 236 | 212.9 | 6.60E-61 |
| 477 | PHE0006054 8779.pep | MMR HSR1 | 39 | 154 | 31.5 | 1.20E-06 |
| 478 | PHE0006059 7042.pep | DnaJ | 4 | 67 | 144.7 | 2.20E-40 |
| 478 | PHE0006059 7042.pep | DnaJ C | 222 | 344 | 47 | 5.90E-11 |
| 479 | PHE00060617051.pep | Metallophos | 2 | 120 | 28.2 | 2.60E-05 |
| 481 | PHE0006063 7049.pep | Transket pyr | 76 | 252 | 249.7 | 5.40E-72 |
| 481 | PHE0006063 7049.pep | Transketolase C | 265 | 387 | 161.6 | 1.80E-45 |
| 482 | PHE0006068 7064.pep | Pkinase Tyr | 1 | 222 | 68.5 | 7.00E-20 |
| 482 | PHE0006068 7064.pep | Pkinase | 2 | 222 | 219.7 | 5.90E-63 |
| 483 | PHE0006069 7065.pep | Cupin- 3 | 62 | 137 | 130 | 6.10E-36 |
| 484 | PHE0006071 7068.pep | PPR | 30 | 63 | 4.4 | 2.1 |
| 484 | PHE0006071 7068.pep | PPR | 64 | 98 | 19.6 | 0.011 |
| 484 | PHE0006071 7068.pep | PPR | 99 | 132 | 18.3 | 0.025 |
| 484 | PHE0006071 7068.pep | PPR | 138 | 172 | 29.2 | 1.40E-05 |
| 484 | PHE0006071 7068.pep | PPR | 173 | 207 | 39.2 | 1.30E-08 |
| 489 | PHE0006077 7045.pep | GASA | 5 | 106 | 226.6 | 5.10E-65 |
| 490 | PHE0006077 7343 pep | GASA | 5 | 106 | 226.6 | 5.10E-65 |
| 491 | PHE0006079 7044.pep | S6PP | 8 | 262 | 519.2 | 4.00E-153 |
| 491 | PHE0006079 7044.pep | Hydrolase 3 | 12 | 257 | -20.6 | 5.00E-06 |

(continued)

| PEP SEQ ID NO | GENE ID | Pfam domain name | begin | stop | score | E-value |
|---|---|---|---|---|---|---|
| 491 | PHE0006079 7044.pep | S6PP C | 263 | 395 | 320.8 | 2.20E-93 |
| 492 | PHE0006079 7337.pep | S6PP | 8 | 262 | 519.2 | 4.00E-153 |
| 492 | PHE0006079 7337.pep | Hydrolase 3 | 12 | 257 | -20.6 | 5.00E-06 |
| 492 | PHE0006079 7337.pep | S6PP C | 263 | 395 | 320.8 | 2.20E-93 |
| 493 | PHE0006082 7330.pep | TPR 1 | 2 | 35 | 28.1 | 2.80E-05 |
| 493 | PHE0006082 7330.pep | TPR 2 | 2 | 35 | 27.1 | 5.70E-05 |
| 493 | PHE0006082 7330.pep | TPR 2 | 36 | 69 | 22.3 | 0.0016 |
| 493 | PHE0006082 7330.pep | TPR 1 | 36 | 69 | 15.7 | 0.066 |
| 493 | PHE0006082 7330.pep | TPR 1 | 70 | 103 | 40.8 | 4.40E-09 |
| 493 | PHE0006082 7330.pep | TPR 2 | 70 | 103 | 32.2 | 1.70E-06 |
| 493 | PHE0006082 7330.pep | TPR 1 | 255 | 290 | 25.7 | 0.00015 |
| 493 | PHE0006082 7330.pep | TPR 2 | 257 | 290 | 26 | 0.00013 |
| 493 | PHE0006082 7330.pep | TPR 1 | 291 | 324 | 30.4 | 5.90E-06 |
| 493 | PHE0006082 7330.pep | TPR 2 | 291 | 324 | 21.2 | 0.0034 |
| 493 | PHE0006082 7330.pep | TPR 1 | 332 | 369 | 19.6 | 0.01 |
| 493 | PHE0006082 7330.pep | TPR 1 | 396 | 429 | 26.1 | 0.00012 |
| 493 | PHE0006082 7330 pep | TPR 2 | 396 | 429 | 20.9 | 0.0042 |
| 493 | PHE0006082 7330.pep | TPR 1 | 430 | 463 | 35.5 | 1.70E-07 |
| 493 | PHE0006082 7330.pep | TPR 2 | 430 | 463 | 23.6 | 0.00062 |
| 493 | PHE0006082 7330 pep | TPR 3 | 461 | 497 | 16.6 | 0.066 |
| 493 | PHE0006082 7330.pep | TPR 1 | 464 | 497 | 33.3 | 7.70E-07 |
| 493 | PHE0006082 7330.pep | TPR 2 | 464 | 497 | 24.5 | 0.00034 |
| 494 | PHE0006088 7063.pep | CoA binding | 634 | 743 | 52.5 | 1.30E-12 |
| 494 | PHE0006088 7063.pep | Ligase_CoA | 784 | 929 | 149.1 | 1.10E-41 |
| 495 | PHE0006089 7061.pep | Brix | 60 | 255 | 136.1 | 8.50E-38 |
| 496 | PHE0006089 7334 pep | Brix | 60 | 255 | 136.1 | 8.50E-38 |
| 497 | PHE0006091 7074.pep | TFIIS M | 206 | 327 | 203.5 | 4.50E-58 |
| 497 | PHE0006091 7074.pep | TFIIS C | 338 | 376 | 83.3 | 7.00E-22 |
| 498 | PHE0006091 7341.pep | TFIIS M | 206 | 327 | 203.5 | 4.50E-58 |
| 498 | PHE0006091 7341.pep | TFIIS C | 338 | 376 | 83.3 | 7.00E-22 |
| 499 | PHE0006092 7062.pep | RRM 1 | 65 | 132 | 43 | 9.10E-10 |
| 499 | PHE0006092 7062 pep | RRM 1 | 150 | 225 | 83.8 | 4.80E-22 |
| 499 | PHE0006092 7062.pep | RRM 1 | 275 | 343 | 53.1 | 8.60E-13 |
| 500 | PHE0006092, 7336.pep | RRM 1 | 65 | 132 | 43 | 9.10E-10 |
| 500 | PHE0006092 7336.pep | RRM 1 | 150 | 225 | 83.8 | 4.80E-22 |
| 500 | PHE0006092 7336 pep | RRM 1 | 275 | 343 | 53.1 | 8.60E-13 |
| 501 | PHE0006093 7066.pep | PTS 2-RNA | 48 | 239 | 409.9 | 3.30E-120 |
| 502 | PHE0006093 7327.pep | PTS 2-RNA | 48 | 239 | 409.9 | 3.30E-120 |

(continued)

| PEP SEQ ID NO | GENE ID | Pfam domain name | begin | stop | score | E-value |
|---|---|---|---|---|---|---|
| 503 | PHE0006094 7231.pep | Chalcone | 14 | 225 | 498.4 | 7.50E-147 |
| 504 | PHE0006094 7333.pep | Chalcone | 14 | 225 | 498.4 | 7.50E-147 |
| 505 | PHE0006154 7204.pep | PfkB | 7 | 299 | 260.8 | 2.50E-75 |
| 506 | PHE0006160 7265.pep | PFK | 3 | 309 | -70.5 | 1.40E-08 |
| 507 | PHE0006160 7286.pep | PFK | 3 | 309 | -70.5 | 1.40E-08 |
| 508 | PHE0006160 8851.pep | PFK | 3 | 309 | -70.5 | 1.40E-08 |
| 509 | PHE0006161 7215.pep | PFK | 195 | 506 | 618.8 | 4.20E-183 |
| 509 | PHE0006161 7215.pep | PFK | 585 | 877 | 69.6 | 9.40E-18 |
| 510 | PHE0006161 7221.pep | PFK | 195 | 506 | 621.1 | 8.90E-184 |
| 510 | PHE0006161 7221.pep | PFK | 585 | 877 | 69.6 | 9.40E-18 |
| 511 | PHE0006173 7211.pep | Ribosomal S6e | 1 | 129 | 266.2 | 6.00E-77 |
| 512 | PHE0006174 7208.pep | RRM 1 | 170 | 241 | 103.1 | 7.30E-28 |
| 512 | PHE0006174 7208 pep | RRM 1 | 269 | 340 | 101.6 | 2.10E-27 |
| 513 | PHE0006175 7210.pep | KOW | 27 | 63 | 31.3 | 3.10E-06 |
| 513 | PHE0006175 7210.pep | eIF-5a | 85 | 154 | 122.5 | 1.10E-33 |
| 515 | PHE0006178 7139.pep | eIF-5a | 82 | 149 | 97.8 | 3.00E-26 |
| 516 | PHE0006178 8626.pep | eIF-5a | 82 | 149 | 97.8 | 3.00E-26 |
| 517 | PHE0006184 7245.pep | DUF125 | 34 | 247 | 255.9 | 7.60E-74 |
| 518 | PHE0006201 7184.pep | ketoacyl-synt | 47 | 309 | 215.6 | 1.00E-61 |
| 518 | PHE0006201 7184.pep | Ketoacyl-synt C | 317 | 477 | 227.3 | 3.00E-65 |
| 519 | PHE0006201 7187.pep | ketoacyl-synt | 47 | 309 | 215.6 | 1.00E-61 |
| 519 | PHE0006201 7187.pep | Ketoacyl-synt C | 317 | 477 | 227.3 | 3.00E-65 |
| 520 | PHE0006202 7182.pep | HMGL-like | 97 | 374 | 377.5 | 1.90E-110 |
| 520 | PHE0006202 7182.pep | LeuA dimer | 467 | 612 | 180.7 | 3.20E-51 |
| 521 | PHE0006204 7183.pep | Cyclin_N | 18 | 151 | 53.6 | 6.20E-13 |
| 521 | PHE0006204 7183.pep | Cyclin C | 153 | 284 | 17.1 | 0.00056 |
| 522 | PHE0006204 7189.pep | Cyclin N | 18 | 151 | 53.6 | 6.20E-13 |
| 522 | PHE0006204 7189.pep | Cyclin C | 153 | 284 | 17.1 | 0.00056 |
| 523 | PHE0006204 8634.pep | Cyclin N | 18 | 151 | 53.6 | 6.20E-13 |
| 523 | PHE0006204 8634.pep | Cyclin_C | 153 | 284 | 17.1 | 0.00056 |
| 524 | PHE0006208 7223.pep | CH | 19 | 120 | 54.8 | 2.50E-13 |
| 524 | PHE0006208 7223.pep | EB1 | 204 | 251 | 79.3 | 1.10E-20 |
| 525 | PHE0006209 7991.pep | HMGL-like | 28 | 305 | 371.7 | 1.00E-108 |
| 525 | PHE0006209 7991.pep | LeuA dimer | 398 | 542 | 165.2 | 1.50E-46 |
| 526 | PHE0006212 7196.pep | Heme oxygenase | 74 | 278 | -13.8 | 4-40E-06 |
| 527 | PHE0006213 7198.pep | Peptidase C54 | 142 | 436 | 555.2 | 6.10E-164 |
| 528 | PHE0006214 7213.pep | Cyclin_N | 32 | 158 | 43.2 | 7.80E-10 |
| 528 | PHE0006214 7213.pep | Cyclin C | 160 | 289 | -2.8 | 0.029 |

(continued)

| PEP SEQ ID NO | GENE ID | Pfam domain name | begin | stop | score | E-value |
|---|---|---|---|---|---|---|
| 529 | PHE0006214 7219.pep | Cyclin N | 32 | 158 | 43.2 | 7.80E-10 |
| 529 | PHE0006214 7219.pep | Cyclin C | 160 | 289 | -2.8 | 0.029 |
| 530 | PHE0006215 7280.pep | PFK | 2 | 277 | 650.2 | 1.50E-192 |
| 531 | PHE0006221 7201.pep | Pyr redox 2 | 44 | 329 | 169.5 | 7.60E-48 |
| 531 | PHE0006221 7201.pep | Pyr redox | 190 | 284 | 94.9 | 2.20E-25 |
| 531 | PHE0006221 7201.pep | Thioredoxin | 384 | 487 | 22.9 | 2.10E-06 |
| 531 | PHE0006221 7201.pep | Gtutaredoxin | 405 | 467 | 35 | 2.40E-07 |
| 532 | PHE0006221 7241.pep | Pyr redox, 2 | 44 | 329 | 169.5 | 7.60E-48 |
| 532 | PHE0006221 7241.pep | Pyr redox | 190 | 284 | 94.9 | 2.20E-25 |
| 532 | PHE0006221 7241.pep | Thioredoxin | 384 | 487 | 22.9 | 2.10E-06 |
| 532 | PHE0006221 7241.pep | Glutaredoxin | 405 | 467 | 35 | 2.40E-07 |
| 533 | PHE0006221 7937.pep | Pyr redox 2 | 44 | 329 | 169.5 | 7.60E-48 |
| 533 | PHE0006221 7937.pep | Pyr redox | 190 | 284 | 94.9 | 2.20E-25 |
| 533 | PHE0006221 7937.pep | Thioredoxin | 384 | 487 | 22.9 | 2.10E-06 |
| 533 | PHE0006221 7937.pep | Glutaredoxin | 405 | 467 | 35 | 2.40E-07 |
| 534 | PHE0006227 7282.pep | NB-ARC | 152 | 421 | 72.5 | 1.20E-18 |
| 534 | PHE0006227 7282.pep | LRR 1 | 652 | 674 | 9.6 | 4.2 |
| 534 | PHE0006227 7282.pep | LRR 1 | 676 | 698 | 8.1 | 7.8 |
| 534 | PHE0006227 7282.pep | LRR 1 | 700 | 722 | 10.3 | 3 |
| 535 | PHE0006232 7454.pep | B lectin | 74 | 187 | 129.9 | 6.60E-36 |
| 535 | PHE0006232 7454.pep | S locus_glycop | 201 | 327 | 180.6 | 3.60E-51 |
| 535 | PHE0006232 7454.pep | PAN 2 | 344 | 411 | 108.4 | 2.00E-29 |
| 535 | PHE0006232 7454.pep | Pkinase | 552 | 823 | 142.3 | 1.20E-39 |
| 535 | PHE0006232 7454.pep | Pkinase Tyr | 552 | 824 | 143.8 | 4.30E-40 |
| 536 | PHE0006232 8756.pep | B lectin | 74 | 187 | 129.9 | 6.60E-36 |
| 536 | PHE0006232 8756.pep | S locus qlycop | 201 | 327 | 180.6 | 3.60E-51 |
| 536 | PHE0006232 8756.pep | PAN 2 | 344 | 411 | 108.4 | 2.00E-29 |
| 536 | PHE0006232 8756.pep | Pkinase Tyr | 552 | 824 | 143.8 | 4.30E-40 |
| 536 | PHE0006232 8756.pep | Pkinase | 552 | 823 | 142.3 | 1.20E-39 |
| 537 | PHE0006233 7220.pep | Mg chelatase | 87 | 295 | -123 | 0.00014 |
| 538 | PHE0006234 7281.pep | Mg chelatase | 85 | 295 | -105.3 | 5.10E-06 |
| 538 | PHE0006234 7281.pep | VWA | 559 | 754 | -2.7 | 0.0079 |
| 539 | PHE0006254 7312.pep | X8 | 29 | 115 | 168.4 | 1.70E-47 |
| 540 | PHE0006263 7271.pep | DAGAT | 48 | 324 | 279.6 | 5.70E-81 |
| 541 | PHE0006264 7285.pep | DAGAT | 48 | 349 | 391.3 | 1.30E-114 |
| 542 | PHE0006265 7990.pep | Heme oxygenase | 88 | 279 | -46.8 | 0.00098 |
| 543 | PHE0006281 7526.pep | GAF | 158 | 307 | 83.2 | 7.40E-22 |
| 543 | PHE0006281 7526.pep | HisKA | 343 | 408 | 84.2 | 3.60E-22 |

(continued)

| PEP SEQ ID NO | GENE ID | Pfam domain name | begin | stop | score | E-value |
|---|---|---|---|---|---|---|
| 543 | PHE0006281 7526.pep | HATPase c | 455 | 582 | 126.8 | 5.50E-35 |
| 543 | PHE00062817526.pep | Response req | 610 | 726 | 51.5 | 2.60E-12 |
| 548 | PHE0006296 7515 pep | Glyco transf 43 | 89 | 312 | 227.4 | 2.90E-65 |
| 549 | PHE0006309 7570 pep | Glyoxalase | 2 | 123 | 153.5 | 4.90E-43 |
| 550 | PHE0006309 8148.pep | Glyoxalase | 2 | 123 | 153.5 | 4.90E-43 |
| 551 | PHE0006309 8620.pep | Glyoxalase | 2 | 123 | 153.5 | 4.90E-43 |
| 552 | PHE0006310 7574.pep | Pkinase | 13 | 304 | 294.9 | 1.40E-85 |
| 554 | PHE0006312 7579.pep | UPF0113 | 1 | 175 | 14.8 | 1.10E-06 |
| 555 | PHE0006312 8644.pep | UPF0113 | 1 | 175 | 14.8 | 1.10E-06 |
| 556 | PHE0006342 8182.pep | Hydrolase | 12 | 200 | 106.3 | 8.10E-29 |
| 557 | PHE0006344 8188.pep | VQ | 44 | 74 | 46 | 1.10E-10 |
| 559 | PHE0006348 8203.pep | UAA | 97 | 388 | -141.8 | 0.0019 |
| 559 | PHE0006348 8203.pep | DUF6 | 106 | 231 | 27.4 | 4.60E-05 |
| 559 | PHE0006348 8203.pep | TPT | 240 | 385 | 193.4 | 5.10E-55 |
| 564 | PHE0006356 8103.pep | F-box | 42 | 89 | 41.3 | 3.00E-09 |
| 564 | PHE0006356 8103.pep | Kelch 1 | 180 | 225 | 43.1 | 8.70E-10 |
| 564 | PHE0006356 8103.pep | Kelch 2 | 180 | 225 | 22.6 | 0.0012 |
| 564 | PHE0006356 8103.pep | Kelch 1 | 227 | 282 | 21.5 | 0.0027 |
| 565 | PHE0006377 7592.pep | RNase PH | 48 | 244 | 131.7 | 1.80E-36 |
| 565 | PHE0006377 7592.pep | RNase PH C | 322 | 384 | 36.2 | 1.00E-07 |
| 566 | PHE0006377 8683.pep | RNase PH | 48 | 244 | 131.7 | 1.80E-36 |
| 566 | PHE0006377 8683.pep | RNase PH C | 322 | 384 | 36.2 | 1.00E-07 |
| 569 | PHE0006380 7658.pep | RNase PH | 1 | 126 | 104.2 | 3.60E-28 |
| 569 | PHE0006380 7658.pep | RNase PH C | 129 | 201 | 61.5 | 2.40E-15 |
| 570 | PHE0006380 8719.pep | RNase PH | 1 | 126 | 104.2 | 3.60E-28 |
| 570 | PHE0006380 8719.pep | RNase PH C | 129 | 201 | 61.5 | 2.40E-15 |
| 571 | PHE00006381 7655.pep | RNase PH | 42 | 186 | 112.2 | 1.40E-30 |
| 572 | PHE0006381 8695.pep | RNase PH | 42 | 186 | 112.2 | 1.40E-30 |
| 573 | PHE0006382 7652.pep | WD40 | 282 | 319 | 33.7 | 5.70E-07 |
| 574 | PHE0006382 8678 pep | WD40 | 282 | 319 | 33.7 | 5.70E-07 |
| 575 | PHE0006425 7646.pep | AA permease | 36 | 537 | 76.4 | 8.40E-20 |
| 576 | PHE0006426 8056.pep | AA permease | 2 | 454 | -41.6 | 2.20E-05 |
| 577 | PHE0006428 7651.pep | Globin | 21 | 161 | 110.6 | 4.10E-30 |
| 578 | PHE0006429 7671.pep | Globin | 18 | 158 | 110.2 | 5.60E-30 |
| 579 | PHE0006433 8307 pep | PseudoU synth 2 | 105 | 284 | 150.2 | 5.00E-42 |
| 580 | PHE0006439 8108.pep | RRM 1 | 23 | 94 | 105.3 | 1.70E-28 |
| 581 | PHE0006449 7865.pep | Biotin, lipoyl | 92 | 165 | 76.9 | 6.00E-20 |
| 581 | PHE0006449 7865.pep | E3 binding | 229 | 265 | 50.4 | 5.70E-12 |

(continued)

| PEP SEQ ID NO | GENE ID | Pfam domain name | begin | stop | score | E-value |
|---|---|---|---|---|---|---|
| 581 | PHE0006449 7865.pep | 2-oxoacid dh | 281 | 512 | 373.8 | 2.40E-109 |
| 582 | PHE0006449 8165.pep | Biotin lipoyl | 92 | 165 | 76.9 | 6.00E-20 |
| 582 | PHE0006449 8165.pep | E3 binding | 229 | 265 | 50.4 | 5.70E-12 |
| 582 | PHE0006449 8165.pep | 2-oxoacid dh | 281 | 512 | 373.8 | 2.40E-109 |
| 583 | PHE0006450 7624.pep | Tubulin | 57 | 250 | 351.6 | 1.20E-102 |
| 583 | PHE0006450 7624.pep | Tubulin C | 252 | 369 | 102.7 | 9.80E-28 |
| 584 | PHE0006464 8089.pep | DREPP | 2 | 203 | 280.3 | 3.30E-81 |
| 585 | PHE0006468 7903.pep | F-box | 2 | 49 | 42.7 | 1.20E-09 |
| 585 | PHE0006468 7903.pep | FBA 1 | 209 | 387 | 311.9 | 1.00E-90 |
| 586 | PHE0006477 7809.pep | PsbR | 42 | 140 | 242.1 | 1.10E-69 |
| 587 | PHE0006478 8190.pep | Methyltransf 6 | 4 | 161 | 171.6 | 1.70E-48 |
| 588 | PHE0006497 8355.pep | DUF868 | 28 | 304 | 175.5 | 1.20E-49 |
| 589 | PHE0006498 7795.pep | Pyridoxal deC | 34 | 381 | 515 | 7.40E-152 |
| 590 | PHE0006498 7796.pep | Pyridoxal deC | 34 | 381 | 515 | 7.40E-152 |
| 591 | PHE0006505 7871.pep | Thioredoxin | 69 | 174 | 120.9 | 3.30E-33 |
| 592 | PHE0006506 7818.pep | Pkinase Tyr | 20 | 270 | 87.1 | 4.90E-23 |
| 592 | PHE0006506 7818.pep | Pkinase | 20 | 272 | 381.9 | 9.00E-112 |
| 592 | PHE0006506 7818.pep | UBA | 294 | 333 | 35.7 | 1.50E-07 |
| 592 | PHE0006506 7818.pep | KA1 | 463 | 511 | 95.6 | 1.40E-25 |
| 593 | PHE0006514 7926.pep | ELFV dehydrog N | 57 | 187 | 298.9 | 8.40E-87 |
| 593 | PHE0006514 7926.pep | ELFV dehydrog | 202 | 447 | 469.7 | 3.30E-138 |
| 594 | PHE0006516 7866.pep | CorA | 90 | 474 | 403.1 | 3.80E-118 |
| 595 | PHE0006516 7882.pep | CorA | 90 | 474 | 403.1 | 3.80E-118 |
| 596 | PHE0006516 7887 pep | CorA | 90 | 474 | 403.1 | 3.80E-118 |
| 597 | PHE0006516 8363.pep | CorA | 90 | 474 | 403.1 | 3.80E-118 |
| 598 | PHE0006517 7858.pep | CorA | 81 | 456 | 344 | 2.30E-100 |
| 599 | PHE0006517 7879 pep | CorA | 81 | 456 | 344 | 2.30E-100 |
| 600 | PHE0006517 7897.pep | CorA | 81 | 456 | 344 | 2.30E-100 |
| 601 | PHE0006521 7840.pep | S6PP | 2 | 247 | 493.5 | 2.30E-145 |
| 601 | PHE0006521 7840.pep | Hydrolase 3 | 6 | 242 | -20.7 | 5.00E-06 |
| 602 | PHE0006545 8320.pep | DnaJ | 31 | 93 | 128.9 | 1.30E-35 |
| 603 | PHE0006549 8255.pep | THF DHG CYH | 3 | 120 | 222.5 | 8.50E-64 |
| 603 | PHE0006549 8255.pep | THF DHG CYH C | 123 | 290 | 366.5 | 3.90E-107 |
| 604 | PHE0006555 8283.pep | Gp dh N | 83 | 236 | 280.2 | 3.80E-81 |
| 604 | PHE0006555 8283.pep | Gp dh C | 241 | 398 | 333 | 4.80E-97 |
| 605 | PHE0006559 8227.pep | PEPcase | 1 | 948 | 2506 | 0 |
| 606 | PHE0006564 8298.pep | GATase 2 | 2 | 161 | 98.9 | 1.40E-26 |
| 606 | PHE0006564 8298 pep | Asn synthase | 209 | 450 | 340.2 | 3.20E-99 |

(continued)

| PEP SEQ ID NO | GENE ID | Pfam domain name | begin | stop | score | E-value |
|---|---|---|---|---|---|---|
| 607 | PHE0006565 8300.pep | GATase 2 | 2 | 161 | 102.1 | 1.50E-27 |
| 607 | PHE0006565 8300.pep | Asn synthase | 209 | 450 | 325.3 | 9.70E-95 |
| 608 | PHE0006571 8279.pep | Pkinase | 15 | 273 | 308.5 | 1.10E-89 |
| 609 | PHE0006574 8224.pep | Glyoxalase | 11 | 150 | 144.4 | 2.80E-40 |
| 609 | PHE0006574 8224.pep | Glyoxalase | 166 | 298 | 109.9 | 6.70E-30 |
| 610 | PHE0006586 8271 pep | Frataxin Cyay | 76 | 187 | 128.3 | 2.00E-35 |
| 611 | PHE0006587 8277.pep | CP12 | 60 | 131 | 155.6 | 1.20E-43 |
| 612 | PHE0006590 8258.pep | Thioredoxin | 75 | 178 | 170.4 | 4.10E-48 |
| 613 | PHE0006591 8264.pep | Thioredoxin | 81 | 184 | 162.9 | 7.80E-46 |
| 614 | PHE0006592 8278.pep | Thioredoxin | 87 | 190 | 157.4 | 3.50E-44 |
| 617 | PHE0006595 8250.pep | DUF537 | 18 | 156 | 206.9 | 4.30E-59 |
| 618 | PHE0006595 8265.pep | DUF537 | 18 | 156 | 206.9 | 4.30E-59 |
| 619 | PHE0006596 8236.pep | CTP transf 2 | 19 | 159 | 48.1 | 2.80E-11 |
| 620 | PHE0006596 8257.pep | CTP transf 2 | 19 | 159 | 48.1 | 2.80E-11 |
| 621 | PHE0006597 8242.pep | Pkinase | 143 | 409 | 98.7 | 1.60E-26 |
| 621 | PHE0006597 8242.pep | Pkinase Tyr | 143 | 409 | 97.2 | 4.50E-26 |
| 622 | PHE0006598 8240.pep | Di19 | 11 | 219 | 487.1 | 1.90E-143 |
| 623 | PHE0006598 8268.pep | Di19 | 11 | 219 | 487.1 | 1.90E-143 |
| 624 | PHE0006599 8230.pep | ZF-HD dimer | 34 | 93 | 141.6 | 2.00E-39 |
| 625 | PHE0006599 8262.pep | ZF-HD dimer | 34 | 93 | 141.6 | 2.00E-39 |
| 626 | PHE0006600 8249.pep | Iso dh | 6 | 355 | 346.2 | 4.90E-101 |
| 627 | PHE0006607 8231.pep | SRF-TF | 11 | 66 | 24.4 | 0.00036 |
| 628 | PHE0006609 8234.pep | GSHPx | 12 | 120 | 221.4 | 1.80E-63 |
| 629 | PHE0006610 8239.pep | GSHPx | 77 | 185 | 234.2 | 2.60E-67 |
| 630 | PHE0006613 8238.pep | GSHPx | 12 | 120 | 200.6 | 3.30E-57 |
| 631 | PHE0006617 8463.pep | Cupin 1 | 65 | 215 | 171.2 | 2.40E-48 |
| 631 | PHE0006617 8463.pep | Cupin 2 | 100 | 177 | 26.7 | 7.30E-05 |
| 632 | PHE0006620 8462.pep | Epimerase | 13 | 259 | 78.2 | 2.40E-20 |
| 632 | PHE0006620 8462.pep | NmrA | 13 | 313 | -88.1 | 0.004 |
| 632 | PHE0006620 8462.pep | 3Beta HSD | 14 | 287 | -65.2 | 3.00E-08 |
| 632 | PHE0006620 8462.pep | NAD biding 4 | 15 | 243 | -13.5 | 8.50E-08 |
| 633 | PHE0006648, 8356.pep | Tryp alpha amyl | 36 | 114 | 56 | 1.20E-13 |
| 634 | PHE0006666 8414.pep | Glycolytic | 43 | 387 | 859 | 2.20E-255 |
| 635 | PHE0006669 8357.pep | PFK | 271 | 582 | 621.1 | 8.90E-184 |
| 635 | PHE0006669 8357.pep | PFK | 661 | 953 | 69.6 | 9.40E-18 |
| 636 | PHE0006670 8346.pep | PfkB | 83 | 375 | 260.8 | 2.50E-75 |
| 637 | PHE0006673 8992.pep | PTR2 | 123 | 530 | 305.8 | 7.40E-89 |
| 638 | PHE0006676 8410.pep | Transket pyr | 39 | 215 | 267.6 | 2.30E-77 |

(continued)

| PEP SEQ ID NO | GENE ID | Pfam domain name | begin | stop | score | E-value |
|---|---|---|---|---|---|---|
| 638 | PHE0006676 8410.pep | Transketolase C | 232 | 354 | 203.9 | 3.50E-58 |
| 639 | PHE0006684 8413.pep | Ribosomal L10 | 19 | 123 | 4.8 | 0.0008 |
| 641 | PHE0006686 8416.pep | Ribosomal L22 | 17 | 153 | 267.2 | 3.00E-77 |
| 642 | PHE0006687 8471.pep | Ribosomal L32e | 14 | 123 | 200.6 | 3.30E-57 |
| 643 | PHE0006706 8434.pep | DEAD | 46 | 212 | 190.3 | 4.20E-54 |
| 643 | PHE0006706 8434.pep | Helicase C | 280 | 356 | 128.7 | 1.50E-35 |
| 644 | PHE0006709 8432.pep | MtN3 slv | 9 | 98 | 96.7 | 6.30E-26 |
| 644 | PHE0006709 8432-pep | MtN3 slv | 132 | 218 | 116.8 | 5.80E-32 |
| 645 | PHE0006715 8477.pep | AMPKBI | 197 | 287 | 161.8 | 1.60E-45 |
| 646 | PHE0006716 8482.pep | NOI | 1 | 72 | 159.7 | 7.10E-45 |
| 647 | PHE0006727 8435.pep | ETC C1 NDUFA4 | 54 | 156 | 168.1 | 2.00E-47 |
| 648 | PHE0006727 8595.pep | ETC C1 NDUFA4 | 54 | 156 | 168.1 | 2.00E-47 |
| 649 | PHE0006728 8430.pep | RRM 1 | 111 | 190 | 32.8 | 1.10E-06 |
| 650 | PHE0006729 8433.pep | DnaJ | 12 | 81 | 66.1 | 1.00E-16 |
| 650 | PHE0006729 8433.pep | zf-CSL | 96 | 174 | 25.2 | 0.00021 |
| 651 | PHE0006730 8428.pep | Lung 7-TM R | 168 | 423 | 385.2 | 8.80E-113 |
| 652 | PHE0006737 8455.pep | 20G-Fell Oxy | 217 | 317 | 139.1 | 1.10E-38 |
| 653 | PHE0006737 8527.pep | 2OG-Fell Oxy | 217 | 317 | 139.1 | 1.10E-38 |
| 656 | PHE0006741 8448.pep | MATH | 53 | 182 | 61.8 | 2.10E-15 |
| 656 | PHE0006741 8448.pep | BTB | 206 | 328 | 86.7 | 6.70E-23 |
| 657 | PHE0006741 8589.pep | MATH | 53 | 182 | 61.8 | 2.10E-15 |
| 657 | PHE0006741 8589.pep | BTB | 206 | 328 | 86.7 | 6.70E-23 |
| 658 | PHE0006742 8440.pep | PGI | 55 | 545 | 770.4 | 1.00E-228 |
| 659 | PHE0006742 8591.pep | PGI | 55 | 545 | 770.4 | 1.00E-228 |
| 660 | PHE0006744 8449.pep | adh short | 37 | 225 | 8.4 | 1.00E-06 |
| 661 | PHE0006745 8590.pep | V-SNARE | 71 | 221 | 154.6 | 2.40E-43 |
| 662 | PHE0006746 8453.pep | Sugar tr | 33 | 464 | 275.2 | 1.10E-79 |
| 662 | PHE0006746 8453.pep | MFS 1 | 38 | 424 | 80 | 6.90E-21 |
| 663 | PHE0006750 8523.pep | zf-C3HC4 | 52 | 89 | 35.8 | 1.40E-07 |
| 663 | PHE0006750 8523.pep | WD40 | 454 | 492 | 34 | 4.90E-07 |
| 663 | PHE0006750 8523.pep | WD40 | 496 | 534 | 22.1 | 0.0018 |
| 663 | PHE0006750 8523.pep | WD40 | 540 | 576 | 38.3 | 2.50E-08 |
| 664 | PHE0006757 8530.pep | Acyltransferase | 375 | 496 | 38.8 | 1.70E-08 |
| 665 | PHE0006760 8529.pep | vATP-synt E | 16 | 225 | 389.4 | 4.90E-114 |
| 666 | PHE0006765 8536.pep | Bromodomain | 110 | 199 | 136.3 | 7.50E-38 |
| 667 | PHE0006766 8867.pep | IPT | 1 | 235 | 515.1 | 6.90E-152 |
| 668 | PHE0006769 8865.pep | TPP enzyme N | 3 | 172 | 280.5 | 2.90E-81 |
| 668 | PHE0006769 8865.pep | TPP enzyme M | 190 | 336 | 193 | 6.40E-55 |

(continued)

| PEP SEQ ID NO | GENE ID | Pfam domain name | begin | stop | score | E-value |
|---|---|---|---|---|---|---|
| 668 | PHE0006769 8865.pep | TPP enzyme C | 379 | 525 | 201.8 | 1.50E-57 |
| 669 | PHE0006770 8553.pep | DEAD | 58 | 224 | 201.3 | 2.10E-57 |
| 669 | PHE0006770 8553.pep | Helicase C | 292 | 368 | 128.3 | 2.00E-35 |
| 670 | PHE0006770 8568.pep | DEAD | 58 | 224 | 201.3 | 2.10E-57 |
| 670 | PHE0006770 8568.pep | Helicase C | 292 | 368 | 128.3 | 2.00E-35 |
| 671 | PHE0006771 8551.pep | FAE1 CUT1 RppA | 52 | 341 | 682.1 | 3.70E-202 |
| 671 | PHE0006771 8551.pep | Chal sti synt C | 298 | 441 | 13.6 | 0.00012 |
| 671 | PHE0006771 8551 .pep | ACP, syn III C | 356 | 439 | 6 | 3.10E-06 |
| 672 | PHE0006775 8548.pep | Miro | 9 | 128 | 68.3 | 2.20E-17 |
| 672 | PHE0006775 8548.pep | Ras | 10 | 178 | 279.3 | 7.00E-81 |
| 673 | PHE0006775 8555.pep | Miro | 9 | 128 | 68.3 | 2.20E-17 |
| 673 | PHE0006775 8555.pep | Ras | 10 | 178 | 279.3 | 7.00E-81 |
| 674 | PHE0006788 8581.pep | Tryp alpha, amyl | 27 | 110 | 118.8 | 1.50E-32 |
| 675 | PHE0006793 8580.pep | p450 | 27 | 508 | 156.6 | 5.80E-44 |
| 676 | PHE0006794 8578.pep | SSB | 71 | 182 | 121.5 | 2.20E-33 |
| 677 | PHE0006805 8531.pep | Ribosomal S30AE | 2 | 95 | 173.7 | 4.20E-49 |
| 678 | PHE0006811 8506.pep | Bac globin | 3 | 122 | 108.1 | 2.30E-29 |
| 681 | PHE0006847 8860.pep | DHBP synthase | 8 | 203 | 370.6 | 2.20E-108 |
| 681 | PHE0006847 8860.pep | GTP cyclohydro2 | 208 | 366 | -2.3 | 3.80E-10 |
| 682 | PHE0006870 8846.pep | Ribosomal L37ae | 2 | 91 | 220.3 | 3.90E-63 |
| 684 | PHE0006909 9003.pep | Cupin 3 | 62 | 137 | 130 | 6.10E-36 |
| 685 | PHE0006910 9019 .pep | Mov34 | 92 | 201 | 58 | 2.80E-14 |
| 686 | PHE0006912 9000.pep | ECH | 57 | 226 | 208.9 | 1.10E-59 |
| 687 | PHE0006919 9008.pep | Peptidase M16 | 80 | 226 | 191.7 | 1.70E-54 |
| 687 | PHE0006919 9008.pep | Peptidase M16 C | 231 | 417 | 152.9 | 7.70E-43 |
| 688 | PHE0006929 9151.pep | zf-C3HC4 | 259 | 299 | 47.2 | 5.00E-11 |
| 689 | PHE0006929 9185.pep | zf-C3HC4 | 259 | 299 | 47.2 | 5.00E-11 |
| 690 | PHE0006931 9148.pep | GDC-P | 3 | 443 | 700.9 | 8.30E-208 |
| 691 | PHE0006931 9168.pep | GDC-P | 3 | 443 | 700.9 | 8.30E-208 |
| 692 | PHE0006932 9147.pep | DUF498 | 56 | 164 | 153.1 | 6.90E-43 |
| 693 | PHE0006932 9174.pep | DUF498 | 56 | 164 | 153.1 | 6.90E-43 |
| 694 | PHE0006933 9139.pep | adh short | 30 | 212 | 5.7 | 1.50E-06 |
| 695 | PHE0006934 9145.pep | DNA pol E B | 178 | 389 | 249.9 | 5.00E-72 |
| 696 | PHE0006937 9126.pep | DUF298 | 127 | 242 | 222.4 | 9.20E-64 |
| 697 | PHE0006938 9149.pep | F-box | 41 | 88 | 34.3 | 3.80E-07 |
| 698 | PHE0006940 9122.pep | Aldedh | 18 | 477 | 674.3 | 8.70E-200 |
| 700 | PHE0006943 9124.pep | Aa trans | 29 | 428 | 516.5 | 2.80E-152 |
| 701 | PHE0006948 9125.pep | RRM 1 | 38 | 109 | 98.5 | 1.80E-26 |

(continued)

| PEP SEQ ID NO | GENE ID | Pfam domain name | begin | stop | score | E-value |
|---|---|---|---|---|---|---|
| 702 | PHE0006948 9160.pep | RRM 1 | 38 | 109 | 98.5 | 1.80E-26 |
| 703 | PHE0006949 9133.pep | Aldedh | 19 | 478 | 778.3 | 4.10E-231 |
| 704 | PHE0006949 9179.pep | Aldedh | 19 | 478 | 778.3 | 4.10E-231 |
| 705 | PHE0006952 9233.pep | PGAM | 91 | 277 | 153.2 | 6.30E-43 |
| 706 | PHE0006953 9121.pep | Usp | 3 | 157 | 85.3 | 1.80E-22 |
| 709 | PHE0006962 9114.pep | Molybdop Fe4S4 | 39 | 93 | 88.2 | 2.40E-23 |
| 709 | PHE0006962 9114.pep | Molybdopterin | 96 | 568 | 478.2 | 9.20E-141 |
| 709 | PHE0006962_9114.pep | Molydop_binding | 714 | 822 | 121.4 | 2.40E-33 |
| 710 | PHE0006963_9131.pep | Pyr_redox 2 | 5 | 287 | 191.2 | 2.30E-54 |
| 710 | PHE0006963 9131.pep | Pyr_redox | 147 | 242 | 100.4 | 4.80E-27 |
| 710 | PHE0006963_9131.pep | Fer2_BFD | 422 | 474 | 92.1 | 1.50E-24 |
| 710 | PHE0006963_9131.pep | NIR_SIR_ferr | 556 | 623 | 82 | 1.70E-21 |
| 710 | PHE0006963_9131.pep | NIR_SIR | 631 | 777 | 166.4 | 6.70E-47 |
| 711 | PHE0006965_9119.pep | tRNA-synt_2b | 67 | 243 | 57.5 | 3.90E-14 |
| 711 | PHE0006965_9119.pep | HGTP_anticodon | 312 | 409 | 100 | 6.40E-27 |
| 713 | PHE0006977_9163.pep | Ribul_P_3_epim | 7 | 207 | 332.8 | 5.30E-97 |

Table 16.

| Pfam domain name | accession number | gathering cutoff | domain description |
|---|---|---|---|
| 2-Hacid dh | PF00389.18 | 13.2 | D-isomer specific 2-hydroxyacid dehydrogenase, catalytic domain |
| 2-Hacid dh C | PF02826.6 | -75.7 | D-isomer specific 2-hydroxyacid dehydrogenase, NAD binding domain |
| 3Beta_HSD | PF01073.8 | -135.9 | 3-beta hydroxysteroid dehydrogenase/ isomerase family |
| 3 5 exonuc | PF01612.10 | -32 | 3'-5' exonuclease |
| AAA | PF00004.17 | 10 | ATPase family associated with various cellular activities (AAA) |
| AA kinase | PF00696.16 | -40 | Amino acid kinase family |
| AA_permease | PF00324.10 | -120.8 | Amino acid permease |
| ABC1 | PF03109.6 | -27.6 | ABC1 family |
| ABC tran | PF00005.14 | 8.6 | ABC transporter |
| ADH_N | PF08240.1 | -14.5 | Alcohol dehydrogenase GroES-like domain |
| ADH zinc N | PF00107.15 | 23.8 | Zinc-binding dehydrogenase |
| AMP-binding | PF00501.15 | 0 | AMP-binding enzyme |
| AMPKBI | PF04739.4 | 25 | 5'-AMP-activated protein kinase, beta subunit, complex-interacting region |
| AP2 | PF00847.9 | 0 | AP2 domain |
| APS kinase | PF01583.9 | 25 | Adenylyisulphate kinase |

(continued)

| Pfam domain name | accession number | gathering cutoff | domain description |
|---|---|---|---|
| ARID | PF01388.10 | -8 | ARID/BRIGHT DNA binding domain |
| AT_hook | PF02178.7 | 14.2 | AT hook motif |
| AUX_IAA | PF02309.6 | -83 | AUX/IAA family |
| Aa trans | PF01490.7 | -128.4 | Transmembrane amino acid transporter protein |
| Abhydrolase_1 | PF00561.9 | 5.5 | alpha/beta hydrolase fold |
| Acetyltransf_1 | PF00583.12 | 18.6 | Acetyltransferase (GNAT) family |
| Acyltransferase | PF01553.10 | 6 | Acyltransferase |
| Aldedh | PF00171.11 | -295 | Aldehyde dehydrogenase family |
| Aldo_ket_red | PF00248.10 | -97 | Aldo/keto reductase family |
| Alpha-amylase | PF00128.11 | -93 | Alpha amylase, catalytic domain |
| Alpha_adaptinC2 | PF02883.9 | -12 | Adaptin C-terminal domain |
| Aminotran_1_2 | PF00155.9 | -57.5 | Aminotransferase class I and II |
| Aminotran 3 | PF00202.10 | -207.6 | Aminotransferase class-III |
| Aminotran_5 | PF00266.8 | -92.9 | Aminotransferase class-V |
| Ammonium_transp | PF00909.10 | -144 | Ammonium Transporter Family |
| Ank | PF00023.17 | 21.6 | Ankyrin repeat |
| Annexin | PF00191.8 | 8 | Annexin |
| ArfGap | PF01412.8 | -17 | Putative GTPase activating protein for Arf |
| Asn synthase | PF00733.10 | -52.8 | Asparagine synthase |
| Asp | PF00026.13 | -186.1 | Eukaryotic aspartyl protease |
| Auxin inducible | PF02519.4 | -15 | Auxin responsive protein |
| Auxin_resp | PF06507.3 | 25 | Auxin response factor |
| B12D | PF06522.1 | 25 | B12D protein |
| B3 | PF02362.11 | 26.5 | B3 DNA binding domain |
| B56 | PF01603.8 | -210 | Protein phosphatase 2A regulatory B subunit (B56 family) |
| BAH | PF01426.6 | 7 | BAH domain |
| BRO1 | PF03097.6 | 25 | BRO1-like domain |
| BURP | PF03181.5 | -52 | BURP domain |
| Bromodomain | PF00439.13 | 8.9 | Bromodomain |
| CAF1 | PF04857.8 | -100.5 | CAF1 family ribonuclease |
| CBFD NFYB HMF | PF00808.12 | 18.4 | Histone-like transcription factor (CBF/NF-Y) and archaeal histone |
| CBS | PF00571.16 | 15.8 . | CBS domain pair |
| CCT | PF06203.3 | 25 | CCT motif |
| CH | PF00307.18 | 22.5 | Calponin homology (CH) domain |
| CMAS | PF02353.9 | -177.9 | Cyclopropane-fatty-acyl-phospholipid synthase |
| CN hydrolase | PF00795.11 | -13.9 | Carbon-nitrogen hydrolase |

(continued)

| Pfam domain name | accession number | gathering cutoff | domain description |
|---|---|---|---|
| CTP synth N | PF06418.2 | 25 | CTP synthase N-terminus |
| CTP_transf_2 | PF01467.15 | -11.8 | Cytidylyltransferase |
| Carb kinase | PF01256.7 | -66.3 | Carbohydrate kinase |
| Catalase | PF00199.8 | -229 | Catalase |
| Cation efflux | PF01545.10 | -95.7 | Cation efflux family |
| Chal_sti_synt_C | PF02797.5 | -6.1 | Chalcone and stilbene synthases, C-terminal domain |
| Chromo | PF00385.11 | 27.5 | 'chromo' (CHRromatin Organisation MOdifier) domain |
| Citrate_synt | PF00285.10 | -101.5 | Citrate synthase |
| CobW_C | PF07683.3 | 18 | Cobalamin synthesis protein cobW C-terminal domain |
| ComA | PF02679.5 | 25 | (2R)-phospho-3-sulfolactate synthase (ComA) |
| CorA | PF01544.8 | -61.3 | CorA-like Mg2+ transporter protein |
| Cpn10 | PF00166.11 | -7.8 | Chaperonin 10 Kd subunit |
| Cpn60 TCP1 | PF00118.13 | -223.4 | TCP-1/cpn60 chaperonin family |
| Cu-oxidase | PF00394.11 | -18.9 | Multicopper oxidase |
| Cu-oxidase 2 | PF07731.3 | -5.8 | Multicopper oxidase |
| Cu-oxidase 3 | PF07732.4 | 10 | Multicopper oxidase |
| Cyclin_C | PF02984.7 | -13 | Cyclin, C-terminal domain |
| Cyclin_N | PF00134.12 | -14.7 | Cyclin, N-terminal domain |
| Cyclotide | PF03784.3 | 25 | Cyclotide family |
| Cys_Met_Meta_PP | PF01053.9 | -278.4 | Cys/Met metabolism PLP-dependent enzyme |
| Cystatin | PF00031.10 | 17.5 | Cystatin domain |
| DAO | PF01266.11 | -36.5 | FAD dependent oxidoreductase |
| DNA_photolyase | PF00875.7 | -10 | DNA photolyase |
| DSPc | PF00782.9 | -21.8 | Dual specificity phosphatase, catalytic domain |
| DUF125 | PF01988.8 | -10.1 | Integral membrane protein DUF125 |
| DUF1423 | PF07227.1 | 25 | Protein of unknown function (DUF1423) |
| DUF1530 | PF07060.1 | 25 | ProFAR isomerase associated |
| DUF1685 | PF07939.1 | 25 | Protein of unknown function (DUF1685) |
| DUF246 | PF03138.4 | -15 | Plant protein family |
| DUF250 | PF03151.6 | 125 | Domain of unknown function, DUF250 |
| DUF296 | PF03479.4 | -11 | Domain of unknown function (DUF296) |
| DUF393 | PF04134.2 | 25 | Protein of unknown function, DUF393 |
| DUF581 | PF04570.4 | -3.1 | Protein of unknown function (DUF581) |
| DUF6 | PF00892.9 | 30 | Integral membrane protein DUF6 |
| DUF641 | PF04859.2 | 25 | Plant protein of unknown function (DUF641) |
| DUF760 | PF05542.1 | 25 | Protein of unknown function (DUF760) |

(continued)

| Pfam domain name | accession number | gathering cutoff | domain description |
|---|---|---|---|
| DUF788 | PF05620.1 | 25 | Protein of unknown function (DUF788) |
| Dehvdrin | PF00257.8 | -4.4 | Dehydrin |
| Di19 | PF05605.2 | 25 | Drought induced 19 protein (Di19) |
| Dirigent | PF03018.4 | 25 | Dirigent-like protein |
| DnaJ | PF00226.18 | -8 | DnaJ domain |
| E1_dh | PF00676.9 | -90 | Dehydropenase E1 component |
| E2F TDP . | PF02319.9 | 17 | E2F/DP family winged-helix DNA-binding domain |
| EB1 | PF03271.6 | 25 | EB1-like C-terminal motif |
| EF1_GNE | PF00736.8 | 20 | EF-1 guanine nucleotide exchange domain |
| ELFV_dehydrog | PF00208.10 | -27 | Glutamate/Leucine/Phenylalanine/Valine dehydrogenase |
| ELFV dehvdrog N | PF02812.7 | 31.8 | Glu/Leu/Phe/Val dehydrogenase, dimerisation domain |
| ERO1 | PF04137.5 | -179.5 | Endoplasmic Reticulum Oxidoreductin I (ERO1) |
| ERp29 | PF07749.2 | 10.5 | Endoplasmic reticulum protein ERp29, C-terminal domain |
| Epimerase | PF01370.10 | -46.3 | NAD dependent epimerase/dehydratase |
|  |  |  | family |
| F-box | PF00646.20 | 12.4 | F-box domain |
| FAD_binding_3 | PF01494.8 | -136.6 | FAD binding domain |
| FAD_binding_4 | PFO1565.12 | -8.1 | FAD binding domain |
| FAD_binding_7 | PF03441.3 | 25 | FAD binding domain of DNA photolyase |
| FAE_3-kCoA_syn1 | PF07168.1 | 25 | Fatty acid elongase 3-ketoacyl-CoA synthase 1 |
| FA desaturase | PF00487.13 | -46 | Fatty acid desaturase |
| FBA_1 | PF07734.2 | -39.4 | F-box associated |
| FBPase | PF00316.9 | -170.3 | Fructose-1-6-bisphosphatase |
| FGGY N | PF00370.10 | -104.7 | FGGY family of carbohydrate kinases, N-terminal domain |
| FHA | PF00498.13 | 25 | FHA domain |
| Fer4 | PF00037.14 | 8 | 4Fe-4S binding domain |
| GAF | PF01590.14 | 23 | GAF domain |
| GAT | PF03127.4 | -7 | GAT domain |
| GATA | PF00320.15 | 28.5 | GATA zinc finger |
| GATase | PF00117.15 | -38.1 | Glutamine amidotransferase class-I |
| GATase_2 | PF00310.10 | -106.2 | Glutamine amidotransferases class-II |
| GFO_IDH_MocA | PF01408.11 | -7.2 | Oxidoreductase family, NAD-binding Rossmann fold |
| GFO IDH MocA C | PF02894.7 | 6 | Oxidoreductase family, C-terminal alpha/beta domain |

(continued)

| Pfam domain name | accession number | gathering cutoff | domain description |
|---|---|---|---|
| GH3 | PF03321.3 | -336 | GH3 auxin-responsive promoter |
| GIDA | PF01134.11 | -226.7 | Glucose inhibited division protein A |
| GRAS | PF03514.4 | -78 | GRAS family transcription factor |
| GRIM-19 | PF06212.1 | 25 | GRIM-19 protein |
| GSHPx | PF00255.9 | -16 | Glutathione peroxidase |
| GST C | PF00043.13 | 22.3 | Glutathione S-transferase, C-terminal domain |
| GST N | PF02798.8 | 14.6 | Glutathione S-transferase, N-terminal domain |
| GTP EFTU | PF00009.14 | 8 | Elongation factor Tu GTP binding domain |
| GTP EFTU D2 | PF03144.13 | 25 | Elongation factor Tu domain 2 |
| GTP EFTU D3 | PF03143.6 | 14.3 | Elongation factor Tu C-terminal domain |
| Gamma-thionin | PF00304.10 | 9.6 | Gamma-thionin family |
| Gln-synt_C | PF00120.13 | -124 | Glutamine synthetase, catalytic domain |
| Gln-synt_N | PF03951.8 | 9 | Glutamine synthetase, beta-Grasp domain |
| Globin | PF00042.11 | -8.8 | Globin |
| Glyco_hydro_1 | PF00232.8 | -301.8 | Glycosyl hydrolase family 1 |
| Glyco_hydro_14 | PF01373.7 | -231.4 | Glycosyl hydrolase family 14 |
| Glyco_hydro_16 | PF00722.9 | -65 | Glycosyl hydrolases family 16 |
| Glyco_hydro_38 | Glycosyl PF01074.11 | -125.3 | Glycosyl hydrolases family 38 N-terminal domain |
| Glyco_hydro_38C | PF07748.2 | -93.1 | Glycosyl hydrolases family 38 C-terminal domain |
| Glyco_transf_20 | PF00982.9 | -243.6 | Glycosyltransferase family 20 |
| Glycogen_syn | PF05693.2 | -492.3 | Glycogen synthase |
| Glycolytic | PF00274.8 | -158 | Fructose-bisphosphate aldolase class-I |
| Glycos_transf_1 | PF00534.9 | -7.3 | Glycosyl transferases group 1 |
| Glvcos transf 2 | PF00535.14 | 17.6 | Glycosyl transferase family 2 |
| Glyoxalase | PF00903.14 | 12.1 | Glyoxalase/Bleomycin resistance protein/ Dioxygenase superfamily |
| Got1 | PF04178.2 | 25 | Got1-like family |
| Gp_dh_C | PF02800.8 | -64.1 | Glyceraldehyde 3-phosphate dehydrogenase, C-terminal domain |
| Gp_dh_N | PF00044.11 | -74.2 | Glyceraldehyde 3-phospltate dehydrogenase, NAD binding domain |
| HALZ | PF02183.7 | 17 | Homeobox associated leucine zipper |
| HAMP | PF00672.13 | 17 | HAMP domain |
| HATPase_c | PF02518.13 | 22.4 | Histidine kinase-, DNA gyrase B-, and HSP90-like ATPase |
| HEAT | PF02985.9 | 17.6 | HEAT repeat |
| HEM4 | PF02602.5 | -12 | Uroporphyrinogen-III synthase HemD |
| HGTP anticodon | PF03129.9 | -2 | Anticodon binding domain |
| HI0933_like | PF03486.4 | -255.8 | H10933-like protein |

(continued)

| Pfam domain name | accession number | gathering cutoff | domain description |
|---|---|---|---|
| HLH | PF00010.15 | 8.2 | Helix-loop-helix DNA-binding domain |
| HMA | PF00403.14 | 17.4 | Heavv-metal-associated domain |
| HMG box | PF00505.8 | 4.1 | HMG (high mobility group) box |
| HSF_DNA-bind | PF00447.7 | -70 | HSF-type DNA-binding |
| HSP20 | PF00011.9 | 13 | Hsp20/alpha crystallin family |
| H PPase | PF03030.5 | -377 | Inorganic H+ pyrophosphatase |
| Heme_oxygenase | PF01126.10 | -58 | Heme oxygenase |
| Hexapep | PF00132.11 | 20 | Bacterial transferase hexapeptide (three repeats) |
| Hexokinase_1 | PF00349.10 | -110.3 | Hexokinase |
| Hexokinase 2 | PF03727.5 | -131.3 | Hexokinase |
| HisKA | PF00512.13 | 10.2 | His Kinase A (phosphoacceptor) domain |
| Hist deacetvl | PF00850.9 | -71 | Histone deacetvlase domain |
| Histone | PF00125.12 | 17.4 | Core histone H2A/H2B/H3/H4 |
| Homeobox | PF00046.17 | -4.1 | Homeobox domain |
| Hpt | PF01627.1 | 25 | Hpt domain |
| Hydrolase | PF00702.13 | 13.6 | haloacid dehalogenase-like hydrolase |
| ICL | PF00463.9 | -234 | Isocitrate lyase family |
| IF4E | PF01652.8 | -35 | Eukaryotic initiation factor 4E |
| IPK | PF03770.6 | 25 | Inositol polyphosphate kinase |
| IlvC | PF01450.8 | -33.8 | Acetohydroxy acid isomeroreductase, catalytic domain |
| IlvN | PF07991.1 | -75.8 | Acetohydroxy acid isomeroreductase, catalytic domain |
| Inhibitor_129 | PF08246.1 | 4.9 | Cathepsin propeptide inhibitor domain (129) |
| Ion trans | PF00520.18 | -4.5 | Ion transport protein |
| Isoamylase N | PF02922.7 | -6.5 | Isoamylase N terminal domain |
| Jacalin | PF01419.6 | 20 | Jacalin-like lectin domain |
| JmjC | PF02373.11 | -8 | JmjC domain |
| JmjN | PF02375.6 | 25 | jmjN domain |
| K-box | PF01486.7 | 0 | K-box region |
| KA1 | PF02149.9 | 25 | Kinase associated domain 1 |
| KH_1 | PF00013.17 | 8.1 | KH domain |
| Kelch_1 | PF01344.13 | 20 | Kelch motif |
| Kelch 2 | PF07646.4 | 20 | Kelch motif |
| Ketoacyl-synt_C | PF02801.10 | -54.9 | Beta-ketoacyl synthase, C-terminal domain |
| Kunitz_legume | PF00197.8 | -32 | Trypsin and protease inhibitor |
| LEA 5 | PF00477.7 | 25 | Small hydrophilic plant seed protein |
| LIM | PF00412.10 | 0 | LIM domain |

(continued)

| Pfam domain name | accession number | gathering cutoff | domain description |
|---|---|---|---|
| LRR 2 | PF07723.2 | 8.7 | Leucine Rich Repeat |
| Lactamase B | PF00753.15 | 22.3 | Metallo-beta-lactamase superfamily |
| Ldh_1_C | PF02866.6 | -13 | lactate/malate dehydrogenase, alpha/beta C-terminal domain |
| Ldh_1_N | PF00056.11 | -31.3 | lactate/malate dehydrogenase, NAD binding domain |
| Lectin_legA | PF00138.7 | 19 | Legume lectins alpha domain |
| Lectin_legB | PF00139.9 | -77 | Legume lectins beta domain |
| Lig_chan | PF00060.16 | 8.2 | Ligand-gated ion channel |
| Lipase_GDSL | PF00657.11 | 10.9 | GDSL-like Lipase/Acylhydrolase |
| M20_dimer | PF07687.3 | 12 | Peptidase dimerisation domain |
| MAP1_LC3 | PF02991.5 | -18.8 | Microtubule associated protein 1A/1B, light chain 3 |
| MFMR | PF07777.1 | -46.7 | G-box binding protein MFMR |
| MFS_1 | PF07690.4 | 23.5 | Major Facilitator Superfamily |
| MIP | PF00230.8 | -62 | Major intrinsic protein |
| Malic M | PF03949.4 | -143.9 | Malic enzyme, NAD binding domain |
| MatE | PF01554.8 | 59.6 | MatE |
| Metallophos | PF00149.16 | 22 | Calcineurin-like phosphoesterase |
| Metallothio_2 | PF01439.7 | -3 | Metallothionein |
| Meth_synt_1 | PF08267.1 | -167.8 | Cobalamin-independent synthase, N-terminal domain |
| Meth_synt_2 | PF01717.7 | -155 | Cobalamin-independent synthase, Catalytic domain |
| Methyltransf_11 | PF08241.1 | 17.1 | Methyltransferase domain |
| Methyltransf_12 | PF08242.1 | 21.4 | Methyltransferase domain |
| Methyltransf_2 | PF00891.7 | -103.8 | 0-methyltransferase |
| Methyltransf_3 | PF01596.7 | -120.6 | O-methyltransferase |
| Mpv17_PMP22 | PF04117.2 | -5.4 | Mpv17 / PMP22 family |
| MtN3_slv | PF03083.5 | -0.8 | MtN3/saliva family |
| Myb_DNA-binding | PF00249.18 | 19.1 | Myb-like DNA-binding domain |
| NAC | PF01849.6 | 0 | NAC domain |
| NAD_binding_4 | PF07993.1 | -87.7 | Male sterility protein |
| NAF | PF03822.4 | 25 | NAF domain |
| NAM | PF02365.5 | -19 | No apical meristem (NAM) protein |
| NDK | PF00334.8 | -59.9 | Nucleoside diphosphate kinase |
| NIF | PF03031.7 | -81 | NL1 interacting factor-like phosphatase |
| NPH3 | PF03000.4 | 25 | NPH3 family |
| NTF2 | PF02136.10 | 6 | Nuclear transport factor 2 (NTF2) domain |

(continued)

| Pfam domain name | accession number | gathering cutoff | domain description |
|---|---|---|---|
| NTP transferase | PF00483.12 | -90.5 | Nucleotidyl transferase |
| NUDIX | PF00293.16 | 0 | NUDIX domain |
| Na Ca ex | PF01699.12 | 25 | Sodium/calcium exchanger protein |
| NifU_N | PF01592.6 | -13 | NifU-like N terminal domain |
| NmrA | PF05368.2 | -90.6 | NmrA-like family |
| Orn_Arg_deC_N | PF02784.6 | -76 | Pyridoxal-dependent decarboxylase, pyridoxal binding domain |
| Orn_DAP_Arg_deC | PF00278.11 | -34.9 | Pyridoxal-dependent decarboxylase, C-terminal sheet domain |
| Oxidored_FMN | PF00724.8 | -147.7 | NADH:flavin oxidoreductase / NADH oxidase family |
| PA | PF02225.10 | 13 | PA domain |
| PAD_porph | PF04371.4 | -180.8 | Porphyromonas-type peptidyl-arginine deiminase |
| PARP | PF00644.9 | -55.5 | Poly(ADP-ribose) polymerase catalytic domain |
| PAS | PF00989.12 | 20 | PAS fold |
| PB1 | PF00564.12 | 12.1 | PB1 domain |
| PBD | PF00786.16 | 12.1 | P21-Rho-binding domain |
| PCI | PF01399.14 | 25 | PCI domain |
| PDZ | PF00595.11 | 12.1 | PDZ domain (Also known as DHR or GLGF) |
| PEP-utilizers | PF00391.12 | 10 | PEP-utilising enzyme, mobile domain |
| PEP-utilizers C | PF02896.7 | -173 | PEP-utilising enzyme, TIM barrel domain |
| PEPcase | PF00311.7 | 25 | Phosphoenolpyruvate carboxylase |
| PGAM | PF00300.11 | -3 | Phosphoglycerate mutase family |
| PHD | PF00628.16 | 25.9 | PHD-finger |
| PK | PF00224.10 | -244 | Pyruvate kinase, barrel domain |
| PK C | PF02887.5 | -44 | Pyruvate kinase, alpha/beta domain |
| PMSR | PF01625.9 | -62 | Peptide methionine sulfoxide reductase |
| PP2C | PF00481.10 | -44 | Protein phosphatase 2C |
| PPDK N | PF01326.8 | -87 | Pyruvate phosphate dikinase, PEP/pyruvate binding domain |
| PRA1 | PF03208.8 | 25 | PRA1 family protein |
| PSI_PsaF | PF02507.5 | 25 | Photosystem 1 reaction centre subunit III |
| PTR2 | PF00854.11 | -50 | POT family |
| PUA | PF01472.8 | 2.2 | PUA domain |
| Peptidase_A22B | PF04258.3 | -137.3 | Signal peptide peptidase |
| Peptidase_C1 | PF00112.11 | -115.8 | Papain family cysteine protease |
| Peptidase_C15 | PF01470.7 | -100 | Pyroglutamyl peptidase |
| Peptidase M20 | PF01546.16 | -14.4 | Peptidase family M20/M25/M40 |

(continued)

| Pfam domain name | accession number | gathering cutoff | domain description |
|---|---|---|---|
| Peptidase_S10 | PF00450.11 | -198 | Serine carboxypeptidase |
| Peptidase_S41 | PF03572.7 | -25.8 | Peptidase family S41 |
| PfkB | PF00294.12 | -67.8 | pfkB family carbohydrate kinase |
| Phytochrome | PF00360.9 | 11 | Phytochrome region |
| Pkinase | PF00069.14 | -70.8 | Protein kinase domain |
| Pkinase C | PF00433.11 | 14 | Protein kinase C terminal domain |
| Pkinase_Tyr | PF07714.4 | 65 | Protein tyrosine kinase |
| Polysacc_synt_2 | PF02719.5 | -176 | Polysaccharide biosynthesis protein |
| Pro CA | PF00484.8 | -45 | Carbonic anhydrase |
| Pro dh | PF01619.7 | -120.5 | Proline dehydrogenase |
| Pyr redox | PF00070.16 | 5 | Pyridine nucleotide-disulphide oxidoreductase |
| Pyr_redox_2 | PF07992.2 | -20 | Pyridine nucleotide-disulphide oxidoreductase |
| Pyr_redox_dim | PF02852.11 | -13 | Pyridine nucleotide-disulphide oxidoreductase, dimerisation domain |
| Pyridoxal_deC | PF00282.8 | -158.6 | Pyridoxal-dependent decarboxylase conserved domain |
| RHD3 | PF05879.2 | 25 | Root hair defective 3 GTP-binding protein (RHD3) |
| R1O1 | PFO1163.11 | -89.1 | RIO1 family |
| RRM_1 | PF00076.10 | 15.2 | RNA recognition motif. (a.k.a. RRM, RBD, or RNP domain) |
| RTC | PF01137.11 | -36.9 | RNA 3'-terminal phosphate cyclase |
| RTC insert | PF05189.3 | 25 | RNA 3'-terminal phosphate cyclase (RTC), insert domain |
| RWP-RK | PF02042.5 | 25 | RWP-RK domain |
| RanBP1 | PF00638.8 | -38 | RanBP1 domain |
| Ras | PF00071.11 | 18 | Ras family |
| Remorin C | PF03763.3 | 25 | Remorin, C-terminal region |
| Response_reg | PF00072.11 | -14.4 | Response regulator receiver domain |
| Reticulon | PF02453.7 | -40 | Reticulon |
| Ribonuclease T2 | PF00445.8 | -53 | Ribonuclease T2 family |
| Ribosomal_L1 | PF00687.10 | -101 | Ribosomal protein L1p/L10e family |
| Ribosomal_L10e | PF00826.7 | 25 | Ribosomal L1 0 |
| Ribosomal L 12 | PF00542.8 | 25 | Ribosomal protein L7/L12 C-terminal domain |
| Ribosomal_L19e | PF01280.9 | -28 | Ribosomal protein L19e |
| Ribosomal L39 | PF00832.9 | 25 | Ribosomal L39 |
| Ribosomal L7Ae | PF01248.13 | 6 | Ribosomal protein L7Ae/L30e/S 12e/Gadd45 family |
| Ribosomal_S11 | PF00411.7 | -4 | Ribosomal protein S11 |
| Ribosomal_S17 | PF00366.9 | 1.7 | Ribosomal protein S 17 |

(continued)

| Pfam domain name | accession number | gathering cutoff | domain description |
|---|---|---|---|
| Ribosomal_S2 | PF00318.9 | -22 | Ribosomal protein S2 |
| Ribosomal_S27 | PF01599.8 | 50 | Ribosomal protein S27a |
| Rieske | PF00355.15 | -7 | Rieske [2Fe-2S] domain |
| RmID_sub_bind | PF04321.6 | -171.8 | RmID substrate binding domain |
| RuBisCO_small | PF00101.9 | -20.1 | Ribulose bisphosphate carboxylase, small chain |
| Rubrerythrin | PF02915.7 | -4.8 | Rubrerythrin |
| SAM_1 | PF00536.17 | 11.3 | SAM domain (Sterile alpha motif) |
| SAM_2 | PF07647.5 | 20 | SAM domain (Sterile alpha motif) |
| SPC25 | PF06703.1 | 25 | Microsomal signal peptidase 25 kDa subunit (SPC25) |
| SPX | PF03105.9 | -20 | SPX domain |
| SRF-TF | PF00319.8 | 11 | SRF-type transcription factor (DNA-binding and dimerisation domain) |
| START | PF01852.8 | 25 | START domain |
| SapB_1 | PF05184.4 | 20 | Saposin-like type B; region 1 |
| SapB_2 | PF03489.5 | 20 | Saposin-like type B, region 2 |
| SecY | PF00344.9 | -210 | eubacterial secY protein |
| SelR | PF01641.8 | -66.5 | SelR domain |
| Sigma70_r1_2 | PF00140.9 | 25 | Sigma-70 factor, region 1.2 |
| Sigma70_r2 | PF04542.3 | 11 | Sigma-70 region 2 |
| Sigma70_r3 | PF04539.4 | 10 | Sigma-70 region 3 |
| Sigma70_r4 | PF04545.5 | 20.7 | Sigma-70, region 4 |
| Sina | PF03145.6 | -48.4 | Seven in absentia protein family |
| Steroid_dh | PF02544.6 | -44.7 | 3-oxo-5-alpha-steroid 4-dehydrogenase |
| Suc_Fer-like | PF06999.2 | -42.4 | Sucrase/ferredoxin-like |
| Succ_DH_flav_C | PF02910.9 | -42 | Fumarate reductase/succinate dehydrogenase flavoprotein C-terminal domain |
| Sucrose_synth | PF00862.9 | -134 | Sucrose synthase |
| Sugar_tr | PF00083.12 | -85 | Sugar (and other) transporter |
| Synaptobrevin | PF00957.9 | 25 | Synaptobrevin |
| TPP_enzyme_C | PF02775.9 | 19.7 | Thiamine pyrophosphate enzyme, C-terminal TPP binding domain |
| TPP_enzyme_M | PF00205.11 | -23.9 | Thiamine pyrophosphate enzyme, central domain |
| TPP_enzyme_N | PF02776.7 | -70 | Thiamine pyrophosphate enzyme, N-terminal TPP binding domain |
| Thiolase_C | PF02803.6 | -30.7 | Thiolase, C-terminal domain |
| Thiolase_N | PF00108.11 | -129.5 | Thiolase, N-terminal domain |
| Thioredoxin | PF00085.8 | -25.7 | Thioredoxin |
| Tic22 | PF04278.2 | 25 | Tic22-like family |

(continued)

| Pfam domain name | accession number | gathering cutoff | domain description |
|---|---|---|---|
| Transaldolase | PF00923.8 | -49 | Transaldolase |
| Transferase | PF02458.5 | -161.2 | Transferase family |
| Transket pyr | PF02779.12 | -50 | Transketolase, pyridine binding domain |
| Transketolase C | PF02780.9 | -15.5 | Transketolase, C-terminal domain |
| Transketolase_N | PF00456.10 | -98 | Transketolase, thiamine diphosphate binding domain |
| Trehalase | PF01204.8 | 25 | Trehalase |
| Trehalase_Ca-bi | PF07492.1 | 20 | Neutral trehalase Ca2+ binding domain |
| Trehalose_PPase | PF02358.6 | -49.4 | Trehalose-phosphatase |
| Trp_Tyr_perm | PF03222.3 | -232.6 | Tryptophan/tyrosine permease family |
| Trp_syntA | PF00290.10 | -149.8 | Tryptophan synthase alpha chain |
| Trypsin | PF00089.13 | -33.2 | Trypsin |
| Tub | PF01167.7 | -98 | Tub family |
| Tubulin | PF00091.14 | -55.7 | Tubulin/FtsZ family, GTPase domain |
| Tubulin_C | PF03953.6 | -10 | Tubulin/FtsZ family, C-terminal domain |
| UBA | PF00627.18 | 20.5 | UBA/TS-N domain |
| UDPGP | PF01704.7 | -265.2 | UTP--glucose-1-phosphate uridylyltransferase |
| UDPGT | PF00201.8 | -151 | UDP-glucoronosyl and UDP-glucosyl transferase |
| UPF0057 | PF01679.7 | 25 | Uncharacterized protein family UPF0057 |
| UbiA | PF01040.8 | -45 | UbiA prenyltransferase family |
| Ubie_methyltran | PF01209.8 | -117 | ubiE/COQ5 methyltransferase family |
| Usp | PF00582.15 | 25.7 | Universal stress protein family |
| VHS | PF00790.8 | -13.2 | VHS domain |
| VQ | PF05678.3 | 25 | VQ motif |
| W2 | PF02020.7 | 25 | eIF4-gamma/eIF5/eIF2-epsilon |
| WD40 | PF00400.19 | 21.4 | WD domain, G-beta repeat |
| WHEP-TRS | PF00458.9 | 10 | WHEP-TRS domain |
| WRKY | PF03106.5 | 25 | WRKY DNA -binding domain |
| Wzy_C | PF04932.4 | 25 | O-Antigen Polymerase |
| XET_C | PF06955.2 | 11.4 | Xyloglucan endo-transglycosylase (XET) C-terminus |
| Xan_ur_permease | PF00860.10 | -151.2 | Permease family |
| YL1 | PF05764.3 | 25 | YL1 nuclear protein |
| YL1_C | PF08265.1 | 18.6 | YL1 nuclear protein C-terminal domain |
| YTH | PF04146.5 | 25 | YT521-B-like family |
| Yippee | PF03226.4 | 25 | Yippee putative zinc-binding protein |
| YjeF_N | PF03853.3 | 25 | YjeF-related protein N-terminus |
| ZF-HD_dimer | PF04770.2 | 25 | ZF-HD protein dimerisation region |

(continued)

| Pfam domain name | accession number | gathering cutoff | domain description |
|---|---|---|---|
| Zip | PF02535.10 | -28 | ZIP Zinc transporter |
| adh short | PF00106.13 | -46.6 | short chain dehydrogenase |
| bZIP_1 | PF00170.10 | 16.5 | bZIP transcription factor |
| bZIP_2 | PF07716.4 | 15 | Basic region leucine zipper |
| cNMP_binding | PF00027.17 | 20.6 | Cyclic nucleotide-binding domain |
| cobW | PF02492.8 | -10 | CobW/HypB/UreG, nucleotide-binding domain |
| efhand | PF00036.19 | 17.5 | EF hand |
| ketoacyl-synt | PF00109.14 | -73.6 | Beta-ketoacyl synthase, N-terminal domain |
| malic | PF00390.8 | 25 | Malic enzyme, N-terminal domain |
| p450 | PF00067.11 | -105 | Cytochrome P450 |
| peroxidase | PF00141.12 | -10 | Peroxidase |
| tRNA-synt_2b | PF00587.14 | -40.5 | tRNA synthetase class II core domain (G, H, P, S and T) |
| ubiquitin | PF00240.12 | 19.4 | Ubiquitin family |
| zf-B_box | PF00643.13 | 11.1 | B-box zinc finger |
| zf-C2H2 | PF00096.14 | 19 | Zinc finger, C2H2 type |
| zf-C3HC4 | PF00097.12 | 16.9 | Zinc finger, C3HC4 type (RING finger) |
| zf-Dof | PF02701.5 | 25 | Dof domain, zinc finger |
| zf-LSD1 | PF06943.2 | 25 | LSDI zinc finger |

**Example 10. Selection of transgenic plants with enhanced agronomic trait(s)**

This example illustrates the preparation and identification by selection of transgenic seeds and plants derived from transgenic plant cells of this invention where the plants and seed are identified by screening a having an enhanced agronomic trait imparted by expression of a protein selected from the group including the homologous proteins identified in Example 6. Transgenic plant cells of corn, soybean, cotton, canola, wheat and rice are transformed with recombinant DNA for expressing each of the homologs identified in Example 6. Plants are regenerated from the transformed plant cells and used to produce progeny plants and seed that are screened for enhanced water use efficiency, enhanced cold tolerance, increased yield, enhanced nitrogen use efficiency, enhanced seed protein and enhanced seed oil. Plants are identified exhibiting enhanced traits imparted by expression of the homologous proteins.

**Table 9**

SEQ ID NO: homolog SEQ ID NOs

| | | | | | | | | | | | | |
|---|---|---|---|---|---|---|---|---|---|---|---|---|
| 359: | 15388 | 15489 | 16365 | 2107 | 21500 | 26627 | 18101 | 18135 | 13765 | 25122 | 12440 | 29925 |
| | 6428 | 3190 | 14698 | 25119 | 17914 | 18387 | 18386 | 18391 | 20215 | 20218 | 19318 | 18066 |
| | 15176 | 2666 | 6544 | 14909 | 9844 | 20838 | 18698 | 17943 | 14038 | 21112 | 29786 | 10294 |
| | 12219 | 27775 | 16431 | 11572 | 10948 | 3889 | 1306 | 23921 | 6231 | 8604 | 884 | 11714 |
| | 23937 | 4313 | 4102 | 21832 | 16311 | 17151 | 16371 | 21013 | 14362 | 25930 | 5884 | 23008 |
| | 25840 | 21413 | 19180 | 20888 | 16059 | 27488 | 27574 | 21290 | 29636 | 28956 | 28590 | 10923 |
| | 15929 | 27109 | 7543 | 19966 | 28904 | 20749 | 13872 | 30099 | 22627 | 28493 | 19151 | 10431 |
| | 18685 | 24165 | 12053 | 13856 | 3743 | 17579 | 26245 | 16500 | 1536 | 7727 | 16765 | 7367 |
| | 13797 | 24029 | 20328 | 16955 | 25630 | 17551 | 16841 | 21197 | 14982 | 27165 | 21540 | 11863 |
| | 13512 | 20114 | 28808 | 28507 | 9971 | 4477 | 22841 | 23609 | 2163 | 21824 | 21460 | 1292 |
| | 19281 | 17196 | 29874 | 24151 | 26995 | 25060 | 25341 | 7991 | 20901 | 25518 | 9047 | 1700 |
| | 6605 | 15337 | 18891 | 18517 | 23662 | 23388 | 22394 | 27233 | 19649 | 19618 | 12492 | 6978 |
| | 7025 | 28348 | 2773 | 11388 | 1575 | 24449 | 5811 | 23438 | 18689 | 6142 | 6403 | 3525 |
| | 26807 | 5185 | 23295 | 26548 | 30003 | 20388 | 8323 | 12445 | 20756 | 9452 | 24167 | 11314 |
| | 30016 | 9759 | 23769 | 11570 | 1850 | 26778 | 23621 | 17070 | 18113 | 15858 | 28136 | 19497 |
| | 6817 | 25259 | 14324 | 8799 | 6865 | 14282 | 10768 | 18152 | 9989 | 19540 | 22487 | 1489 |
| | 23326 | 23644 | 29174 | 14974 | 5150 | 25462 | 2938 | 2941 | 2628 | 8826 | 29202 | 8802 |
| | 8801 | 8804 | 26441 | 1714 | 30021 | 15771 | 11984 | 24021 | 9123 | 8828 | 8829 | 7601 |
| | 10231 | 17158 | 2069 | 29190 | 5703 | 24403 | 10695 | 2526 | 3927 | 7964 | 10136 | 17234 |
| | 28909 | 8910 | 11028 | 21313 | 22419 | 26107 | 23873 | 21464 | 3489 | 14826 | 3513 | 12472 |
| | 6921 | 5477 | 28981 | 13625 | 13501 | 16361 | 25688 | 3880 | 25402 | 8953 | 16960 | 22707 |
| | 20329 | 10871 | | | | | | | | | | |
| 360: | 25845 | 26147 | 2800 | 23493 | 24582 | 10313 | 18751 | 28146 | 16590 | 16554 | 16550 | 16588 |
| | 16586 | 16617 | 16544 | 16655 | 18938 | 18934 | 18896 | 18890 | 17686 | 17724 | 16619 | 16652 |
| | 16654 | 4952 | 14984 | 30014 | 13318 | 17456 | 17457 | 17426 | 17421 | 17419 | 22044 | 23300 |
| | 17027 | 17004 | 4753 | 18859 | 4708 | 17382 | 17393 | 17386 | 17388 | 25978 | 14019 | 13135 |
| | 24545 | 17251 | 17274 | 17271 | 19323 | 19250 | 19252 | 19345 | 19329 | 28224 | 28228 | 28226 |
| | 14026 | 15191 | 15219 | 15248 | 15222 | 15221 | 2758 | 2754 | 2782 | 28149 | 8932 | 19771 |
| | 28964 | 28144 | 28143 | 14867 | 2787 | 2784 | 2790 | 2690 | 2692 | 2696 | 2718 | 2727 |
| | 2729 | 10226 | 26133 | 19366 | 25253 | 25233 | 21308 | 7319 | 6333 | 14136 | 3918 | 14508 |
| | 11299 | 14030 | 3648 | 19321 | 19257 | 18501 | 18498 | 18495 | 19302 | 19325 | 19288 | 22025 |
| | 3823 | 3166 | 7930 | 28962 | 10793 | 24894 | 26571 | 17469 | 17464 | 26640 | 20552 | 14913 |
| | 20330 | 23678 | 8298 | 28408 | 9828 | 29747 | 18344 | 2419 | 20173 | 11997 | 11910 | 17617 |

SEQ ID NO: homolog SEQ ID NOs

| | | | | | | | | | | | |
|---|---|---|---|---|---|---|---|---|---|---|---|
| 24126 | 27287 | 1603 | 23932 | 24435 | 16318 | 28868 | 23922 | 10924 | 16702 | 19294 | 19296 |
| 19290 | 1752 | 14094 | 9661 | 6148 | 22479 | 3169 | 7572 | 28344 | 18105 | 21423 | 16207 |
| 19960 | 2681 | 11397 | 3515 | 9623 | 17243 | 17217 | 17245 | 7269 | 7547 | 21722 | 10697 |
| 28531 | 11816 | 15850 | 8768 | 2581 | 21921 | 1305 | 21664 | 12381 | 23471 | 20578 | 7628 |
| 1431 | 1429 | 16121 | 25594 | 22437 | 29881 | 5140 | 15518 | 5360 | 26035 | 23579 | 12722 |
| 21986 | 16174 | 5695 | 29899 | 15753 | 12360 | 30211 | 22524 | 3862 | 10915 | 23443 | 21351 |
| 21247 | 11618 | 27531 | 15180 | 15182 | 19370 | 10749 | 27036 | 12999 | 17731 | 17735 | 22652 |
| 11360 | 25602 | 23072 | 4357 | 5364 | 28567 | 18382 | 3005 | 11822 | 11821 | 26402 | 6381 |
| 17814 | 16656 | 14185 | 2792 | 5183 | 3197 | 25540 | 15282 | 1902 | 15217 | 8156 | 22571 |
| 3793 | 5756 | 28554 | 1101 | 24456 | 24458 | 17819 | 7455 | 6482 | 30314 | 13505 | 15795 |
| 22096 | 18423 | 18644 | 16615 | 15719 | 6479 | 4727 | 4728 | 17208 | 14596 | 26006 | 25649 |
| 6525 | 17214 | 17212 | 17211 | 9192 | 9190 | 18731 | 18732 | 13698 | 30164 | 17278 | 17275 |
| 17308 | 17283 | 17311 | 17309 | 14598 | 4735 | 24089 | 24094 | 10552 | 25121 | 28515 | 30234 |
| 9944 | 10918 | 30171 | 16662 | 11549 | 6555 | 12052 | 24431 | 1374 | 12375 | 18739 | 26927 |
| 6500 | 25964 | 9205 | 5515 | 5488 | 3473 | 24183 | 10699 | 15104 | 26973 | 14505 | 29308 |
| 26972 | 7360 | 21124 | 27706 | 22455 | 23541 | 16817 | 29875 | 29177 | 21402 | 24717 | 19765 |
| 2697 | 24132 | 16966 | 14580 | 12908 | 25017 | 21068 | 29253 | 29277 | 29256 | 9301 | 18915 |
| 24421 | 16451 | 8788 | 12525 | 3183 | 6885 | 13623 | 20605 | 23156 | 4686 | 4688 | 17535 |
| 5647 | 6156 | 9200 | 7252 | 27063 | 5291 | 10519 | 10522 | 4345 | 5287 | 5646 | 18266 |
| 2994 | 2961 | 1756 | 30252 | 14994 | 4509 | 28722 | 11699 | 29547 | 2614 | 20734 | 17801 |
| 18649 | 4699 | 18854 | 17765 | 26214 | 13009 | 7722 | 7725 | 23416 | 3100 | 13043 | 2723 |
| 7340 | 8065 | 605 | 12300 | 17639 | 18361 | 24438 | 3555 | 9461 | 13096 | 6533 | 22992 |
| 26737 | 4252 | 26306 | 26315 | 26314 | 26309 | 19541 | 14942 | 5061 | 1400 | 1408 | 30273 |
| 27307 | 2703 | 21086 | 22171 | 12675 | 2126 | 25502 | 4889 | 14758 | 17408 | 29617 | 7457 |
| 17533 | 20898 | 29618 | 14541 | 24619 | 5744 | 14445 | 15969 | 9079 | 4298 | 16855 | 23670 |
| 16284 | 11636 | 29043 | 2368 | 30187 | 5133 | 7414 | 6345 | 14927 | 5689 | 14057 | 2011 |
| 9965 | 3998 | 2103 | 1278 | 27735 | 3643 | 16939 | 4152 | 16387 | 18065 | 12503 | 4178 |
| 13348 | 9163 | 9159 | 9164 | 9161 | 8403 | 6501 | 28203 | 28198 | 28175 | 9131 | 9128 |
| 9156 | 9125 | 4703 | 2664 | 17360 | 2710 | 2706 | 22870 | 22871 | 16546 | 23806 | 23780 |
| 28173 | 28171 | 28168 | 5579 | 9575 | 9576 | 14540 | 15750 | 15766 | 18655 | 16659 | 15755 |
| 18652 | 18678 | 17396 | 17415 | 17417 | 6461 | 28204 | 6497 | 28219 | 28205 | 28221 | 6396 |
| 5355 | 5352 | 18886 | 22866 | 6504 | 4697 | 4707 | 4724 | 17620 | 4733 | 2760 | 2268 |
| 5114 | 12139 | 25979 | 14825 | 24909 | 21178 | 21821 | 2233 | 18262 | 18259 | 17468 | 17463 |

SEQ ID NO: homolog SEQ ID NOs

| | | | | | | | | | | | | |
|---|---|---|---|---|---|---|---|---|---|---|---|---|
| | 14629 | 17459 | 18256 | 5307 | 26127 | 6413 | 14751 | 27963 | 15186 | 28552 | 20689 | 8090 |
| | 1540 | 28523 | 20132 | 6510 | 25352 | 25975 | 2716 | 16539 | 15758 | 17763 | 18881 | 17767 |
| | 17580 | 17648 | 17643 | 17624 | 17721 | 20148 | 17696 | 17688 | 20147 | 20151 | 17692 | 20144 |
| | 20183 | 20153 | 17690 | 20176 | 17642 | 17717 | 17715 | 17650 | 17652 | 12121 | 16613 | 18849 |
| | 16819 | 19570 | 26711 | 5974 | 2694 | 5793 | 11039 | 23795 | 10254 | 26019 | | |
| 361: | 4067 | 4066 | 23179 | 2893 | 26178 | 26177 | 22312 | 4095 | 27151 | 18729 | 21244 | 15423 |
| | 2457 | 18357 | 10440 | 18621 | 608 | 9467 | 14846 | 25494 | 16021 | 22213 | 23437 | 14031 |
| | 5323 | 23183 | 29766 | 28372 | 10564 | 10315 | 2724 | 13465 | 4284 | 22567 | 18966 | 10585 |
| | 25368 | 19280 | 19283 | 24840 | 13374 | 13372 | 5510 | 15296 | 15471 | 18485 | 13207 | 7978 |
| | 21743 | 4580 | 10593 | 18615 | 14003 | 2037 | 13047 | 8865 | 18833 | 16670 | 18837 | |
| 362: | 4067 | 4066 | 23179 | 2893 | 26178 | 26177 | 22312 | 4095 | 27151 | 18729 | 21244 | 15423 |
| | 2457 | 18357 | 10440 | 18621 | 608 | 9467 | 14846 | 25494 | 16021 | 22213 | 23437 | 14031 |
| | 5323 | 23183 | 29766 | 28372 | 10564 | 10315 | 2724 | 13465 | 4284 | 22567 | 18966 | 10585 |
| | 25368 | 19280 | 19283 | 24840 | 13374 | 13372 | 5510 | 15296 | 15471 | 18485 | 13207 | 7978 |
| | 21743 | 4580 | 10593 | 18615 | 14003 | 2037 | 13047 | 8865 | 18833 | 16670 | 18837 | |
| 363: | 4066 | 4067 | 23179 | 23470 | 2893 | 23891 | 10491 | 9562 | 22312 | 4095 | 18729 | 21244 |
| | 14987 | 15423 | 9245 | 18357 | 18621 | 10440 | 608 | 9467 | 14846 | 25494 | 16021 | 6032 |
| | 22213 | 23437 | 5323 | 14031 | 23183 | 29766 | 10564 | 28372 | 10315 | 11057 | 23962 | 23700 |
| | 8399 | 15600 | 18966 | 25368 | 10585 | 24840 | 13374 | 13372 | 5510 | 15471 | 25722 | 17495 |
| | 9536 | 10593 | 6019 | 24672 | 2037 | 23987 | 13047 | 8865 | 18807 | | | |
| 364: | 17583 | 3837 | 25131 | 9424 | 21018 | 1330 | 1248 | 14590 | 17982 | 25546 | 10828 | 21014 |
| | 22786 | 6365 | 21922 | 18588 | 2174 | 24886 | 1032 | 13329 | 13081 | 13199 | 14304 | 2300 |
| | 28026 | 2317 | 15201 | 6269 | 11143 | 15365 | 15367 | 18238 | 2476 | 27444 | 18589 | 16267 |
| | 2378 | 23530 | 18403 | 24287 | 27200 | 22363 | 17902 | 18422 | 29655 | 23711 | 29739 | 9293 |
| | 21281 | 16857 | 9046 | 14882 | 7525 | 11465 | 16572 | 17302 | 13620 | 26825 | 10785 | 10850 |
| | 10511 | 4302 | 26130 | 6657 | 22809 | 21809 | 18944 | 8945 | 24203 | 16044 | 23350 | 5321 |
| | 8171 | 6257 | 6320 | 5984 | 17552 | 21563 | 20817 | 17377 | 2132 | 28721 | 5122 | 1866 |
| | 14397 | 2187 | 9236 | 9235 | 9238 | 14893 | 7693 | 16230 | 18174 | 23435 | 24316 | 20423 |
| | 23211 | 23103 | 18740 | 3078 | 5497 | 24852 | 17572 | 19821 | 14412 | 27750 | 29915 | 27592 |
| | 29300 | 26698 | 23669 | 28565 | 24571 | 24838 | 22463 | 24533 | 6785 | 4435 | 11044 | 12954 |
| | 6322 | 17905 | 6928 | 15307 | 3240 | 12217 | 3996 | 28268 | 27715 | 27623 | 6690 | 17716 |
| | 8385 | 18599 | 25379 | 9359 | 2249 | 9670 | 29729 | 23418 | 8070 | 6547 | 24849 | 13584 |
| | 22097 | 30116 | 30263 | 24745 | 4426 | 16379 | 27817 | 30117 | 27231 | 16949 | 25019 | 19018 |

(continued)

SEQ ID NO: homolog SEQ ID NOs

| | | | | | | | | | | | | |
|---|---|---|---|---|---|---|---|---|---|---|---|---|
| | 14435 | 27373 | 13875 | 3614 | 1404 | 12794 | 27073 | 13550 | 5580 | 6932 | 12909 | 20402 |
| | 13494 | 15527 | 17659 | 6406 | 9251 | 3011 | 16390 | 11463 | | | | |
| 365: | 4412 | 20720 | 18385 | 25343 | 26648 | 25179 | 20491 | 20763 | 27851 | 30025 | 13589 | 28443 |
| | 18645 | 29627 | 29669 | 29713 | 29708 | 29572 | 29542 | 29537 | 29566 | 29517 | 29673 | 28891 |
| | 28859 | 13680 | 16437 | 4986 | 14602 | 13947 | 4990 | 26117 | 16200 | 2565 | 18535 | 14202 |
| | 25627 | 27413 | 7164 | 17969 | 21723 | 19455 | 3267 | 11561 | 16058 | 18453 | 23329 | 21126 |
| | 15529 | 17538 | 10319 | 20549 | 2464 | 27351 | 18318 | 16246 | 23726 | 4127 | 5816 | 24415 |
| | 8588 | 2375 | 14798 | 8749 | 10479 | 24135 | 3494 | 26208 | 4498 | 11762 | 25843 | 12115 |
| | 13498 | 19394 | 26779 | 23925 | 27234 | 8050 | 21249 | 13828 | 16115 | 15531 | 7908 | 26538 |
| | 11853 | 20895 | 20004 | 16034 | 18618 | 24454 | 29630 | 28930 | 17367 | 28454 | 21659 | 28780 |
| | 22148 | 8095 | 13899 | 29213 | 25195 | 7522 | 7429 | 16151 | 28936 | 1737 | 13641 | 12677 |
| | 3782 | 13911 | 14085 | 23875 | 10656 | 10507 | 13643 | 9841 | 14448 | 7358 | 20081 | 6809 |
| | 788 | 27035 | 5398 | 29735 | 13383 | 25997 | 11587 | 8315 | 9787 | 23409 | 26789 | 7961 |
| | 18183 | 3887 | 18659 | 22754 | 7196 | 2110 | 12177 | 8795 | 14843 | 25615 | 12452 | 27123 |
| | 29551 | 15927 | 12957 | 24003 | 19777 | 30152 | 21867 | 20904 | 22092 | 14870 | 29589 | 18917 |
| | 16811 | 22658 | 21417 | 11208 | 10210 | 8879 | 25504 | 26516 | 15422 | 3325 | 25912 | 20421 |
| | 17698 | 15799 | 20971 | 5876 | 14781 | 21187 | 24883 | 16127 | 15488 | 10122 | 22681 | 24020 |
| | 12056 | 28487 | 6705 | 1334 | 20655 | 2674 | 13593 | 8590 | 17074 | 3854 | 2965 | 17322 |
| | 8881 | 15552 | 23800 | 21788 | 25268 | 28327 | 11215 | 6853 | 27677 | 28858 | 19925 | 17613 |
| | 28074 | 17220 | 14775 | 3784 | 10729 | 8743 | 10526 | 11156 | 23150 | 9773 | 21991 | 26881 |
| | 15679 | 17373 | 26447 | 13478 | 22646 | 5587 | 3208 | 4044 | 4191 | 2134 | 2277 | 4637 |
| | 15832 | 27041 | 5101 | 26373 | 2283 | 9630 | 22493 | 10427 | 9080 | 16595 | 5886 | 18019 |
| | 28392 | 4010 | 27138 | 18435 | 10672 | 20080 | 22777 | 20970 | 26677 | 28284 | 25576 | 23994 |
| | 6145 | 12430 | 27537 | 29603 | 22217 | 2128 | 25364 | 13591 | 2508 | 25816 | 7216 | 10030 |
| | 17680 | 25818 | 28350 | 27142 | 14708 | 11227 | 14674 | 12739 | 14677 | 1740 | 10054 | 23199 |
| | 28604 | 28844 | 27306 | 28861 | 8194 | 30260 | 17647 | 22684 | 23735 | 25283 | 15955 | 7518 |
| | 24540 | 16219 | 26884 | 6045 | 10165 | 3906 | 2858 | 28831 | 9544 | 12782 | 17894 | 4038 |
| | 13845 | 25254 | 7349 | 23724 | 4563 | 19353 | 2507 | 10903 | 10650 | 25977 | 15377 | 2410 |
| | 10085 | 15994 | 7305 | 27618 | 4077 | 27369 | 3277 | 23646 | 28338 | 26959 | 13331 | 16330 |
| | 2836 | 24197 | 4700 | 4159 | 25819 | 10173 | 14914 | 23141 | 27629 | 7244 | 15141 | 22675 |
| 366: | 4412 | 20720 | 18385 | 25343 | 26648 | 25179 | 20491 | 20763 | 27851 | 30025 | 13589 | 28443 |
| | 18645 | 29627 | 29669 | 29713 | 29708 | 29572 | 29542 | 29537 | 29566 | 29517 | 29673 | 28891 |
| | 28859 | 13680 | 16437 | 4986 | 14602 | 13947 | 4990 | 26117 | 16200 | 2565 | 18535 | 14202 |

SEQ ID NO: homolog SEQ ID NOs

| | | | | | | | | | | | | |
|---|---|---|---|---|---|---|---|---|---|---|---|---|
| | 25627 | 27413 | 7164 | 17969 | 21723 | 19455 | 3267 | 11561 | 16058 | 18453 | 23329 | 21126 |
| | 15529 | 17538 | 10319 | 20549 | 2464 | 27351 | 18318 | 16246 | 23726 | 4127 | 5816 | 24415 |
| | 8588 | 2375 | 14798 | 8749 | 10479 | 24135 | 3494 | 26208 | 4498 | 11762 | 25843 | 12115 |
| | 13498 | 19394 | 26779 | 23925 | 27234 | 8050 | 21249 | 13828 | 16115 | 15531 | 7908 | 26538 |
| | 11853 | 20895 | 20004 | 16034 | 18618 | 24454 | 29630 | 28930 | 17367 | 28454 | 21659 | 28780 |
| | 22148 | 8095 | 13899 | 29213 | 25195 | 7522 | 7429 | 16151 | 28936 | 1737 | 13641 | 12677 |
| | 3782 | 13911 | 14085 | 23875 | 10656 | 10507 | 13643 | 9841 | 14448 | 7358 | 20081 | 6809 |
| | 788 | 27035 | 5398 | 29735 | 13383 | 25997 | 11587 | 8315 | 9787 | 23409 | 26789 | 7961 |
| | 18183 | 3887 | 18659 | 22754 | 7196 | 2110 | 12177 | 8795 | 14843 | 25615 | 12452 | 27123 |
| | 29551 | 15927 | 12957 | 24003 | 19777 | 30152 | 21867 | 20904 | 22092 | 14870 | 29589 | 18917 |
| | 16811 | 22658 | 21417 | 11208 | 10210 | 8879 | 25504 | 26516 | 15422 | 3325 | 25912 | 20421 |
| | 17698 | 15799 | 20971 | 5876 | 14781 | 21187 | 24883 | 16127 | 15488 | 10122 | 22681 | 24020 |
| | 12056 | 28487 | 6705 | 1334 | 20655 | 2674 | 13593 | 8590 | 17074 | 3854 | 2965 | 17322 |
| | 8881 | 15552 | 23800 | 21788 | 25268 | 28327 | 11215 | 6853 | 27677 | 28858 | 19925 | 17613 |
| | 28074 | 17220 | 14775 | 3784 | 10729 | 8743 | 10526 | 11156 | 23150 | 9773 | 21991 | 26881 |
| | 15679 | 17373 | 26447 | 13478 | 22646 | 5587 | 3208 | 4044 | 4191 | 2134 | 2277 | 4637 |
| | 15832 | 27041 | 5101 | 26373 | 2283 | 9630 | 22493 | 10427 | 9080 | 16595 | 5886 | 18019 |
| | 28392 | 4010 | 27138 | 18435 | 10672 | 20080 | 22777 | 20970 | 26677 | 28284 | 25576 | 23994 |
| | 6145 | 12430 | 27537 | 29603 | 22217 | 2128 | 25364 | 13591 | 2508 | 25816 | 7216 | 10030 |
| | 17680 | 25818 | 28350 | 27142 | 14708 | 11227 | 14674 | 12739 | 14677 | 1740 | 10054 | 23199 |
| | 28604 | 28844 | 27306 | 28861 | 8194 | 30260 | 17647 | 22684 | 23735 | 25283 | 15955 | 7518 |
| | 24540 | 16219 | 26884 | 6045 | 10165 | 3906 | 2858 | 28831 | 9544 | 12782 | 17894 | 4038 |
| | 13845 | 25254 | 7349 | 23724 | 4563 | 19353 | 2507 | 10903 | 10650 | 25977 | 15377 | 2410 |
| | 10085 | 15994 | 7305 | 27618 | 4077 | 27369 | 3277 | 23646 | 28338 | 26959 | 13331 | 16330 |
| | 2836 | 24197 | 4700 | 4159 | 25819 | 10173 | 14914 | 23141 | 27629 | 7244 | 15141 | 22675 |
| 367: | 4412 | 20720 | 18385 | 25343 | 26648 | 25179 | 20491 | 20763 | 27851 | 30025 | 13589 | 28443 |
| | 18645 | 29627 | 29669 | 29713 | 29708 | 29572 | 29542 | 29537 | 29566 | 29517 | 29673 | 28891 |
| | 28859 | 13680 | 16437 | 4986 | 14602 | 13947 | 4990 | 26117 | 16200 | 2565 | 18535 | 14202 |
| | 25627 | 27413 | 7164 | 17969 | 21723 | 19455 | 3267 | 11561 | 16058 | 18453 | 23329 | 21126 |
| | 15529 | 17538 | 10319 | 20549 | 2464 | 27351 | 18318 | 16246 | 23726 | 4127 | 5816 | 24415 |
| | 8588 | 2375 | 14798 | 8749 | 10479 | 24135 | 3494 | 26208 | 4498 | 11762 | 25843 | 12115 |
| | 13498 | 19394 | 26779 | 23925 | 27234 | 8050 | 21249 | 13828 | 16115 | 15531 | 7908 | 26538 |
| | 11853 | 20895 | 20004 | 16034 | 18618 | 24454 | 29630 | 28930 | 17367 | 28454 | 21659 | 28780 |

SEQ ID NO: homolog SEQ ID NOs

| | | | | | | | | | | | |
|---|---|---|---|---|---|---|---|---|---|---|---|
| 22148 | 8095 | 13899 | 29213 | 25195 | 7522 | 7429 | 16151 | 28936 | 1737 | 13641 | 12677 |
| 3782 | 13911 | 14085 | 23875 | 10656 | 10507 | 13643 | 9841 | 14448 | 7358 | 20081 | 6809 |
| 788 | 27035 | 5398 | 29735 | 13383 | 25997 | 11587 | 8315 | 9787 | 23409 | 26789 | 7961 |
| 18183 | 3887 | 18659 | 22754 | 7196 | 2110 | 12177 | 8795 | 14843 | 25615 | 12452 | 27123 |
| 29551 | 15927 | 12957 | 24003 | 19777 | 30152 | 21867 | 20904 | 22092 | 14870 | 29589 | 18917 |
| 16811 | 22658 | 21417 | 11208 | 10210 | 8879 | 25504 | 26516 | 15422 | 3325 | 25912 | 20421 |
| 17698 | 15799 | 20971 | 5876 | 14781 | 21187 | 24883 | 16127 | 15488 | 10122 | 22681 | 24020 |
| 12056 | 28487 | 6705 | 1334 | 20655 | 2674 | 13593 | 8590 | 17074 | 3854 | 2965 | 17322 |
| 8881 | 15552 | 23800 | 21788 | 25268 | 28327 | 11215 | 6853 | 27677 | 28858 | 19925 | 17613 |
| 28074 | 17220 | 14775 | 3784 | 10729 | 8743 | 10526 | 11156 | 23150 | 9773 | 21991 | 26881 |
| 15679 | 17373 | 26447 | 13478 | 22646 | 5587 | 3208 | 4044 | 4191 | 2134 | 2277 | 4637 |
| 15832 | 27041 | 5101 | 26373 | 2283 | 9630 | 22493 | 10427 | 9080 | 16595 | 5886 | 18019 |
| 28392 | 4010 | 27138 | 18435 | 10672 | 20080 | 22777 | 20970 | 26677 | 28284 | 25576 | 23994 |
| 6145 | 12430 | 27537 | 29603 | 22217 | 2128 | 25364 | 13591 | 2508 | 25816 | 7216 | 10030 |
| 17680 | 25818 | 28350 | 27142 | 14708 | 11227 | 14674 | 12739 | 14677 | 1740 | 10054 | 23199 |
| 28604 | 28844 | 27306 | 28861 | 8194 | 30260 | 17647 | 22684 | 23735 | 25283 | 15955 | 7518 |
| 24540 | 16219 | 26884 | 6045 | 10165 | 3906 | 2858 | 28831 | 9544 | 12782 | 17894 | 4038 |
| 13845 | 25254 | 7349 | 23724 | 4563 | 19353 | 2507 | 10903 | 10650 | 25977 | 15377 | 2410 |
| 10085 | 15994 | 7305 | 27618 | 4077 | 27369 | 3277 | 23646 | 28338 | 26959 | 13331 | 16330 |
| 2836 | 24197 | 4700 | 4159 | 25819 | 10173 | 14914 | 23141 | 27629 | 7244 | 15141 | 22675 |
| 368: 16255 | 22265 | 15081 | 24173 | 29320 | 21530 | 25245 | 6634 | 15567 | 22512 | 13725 | 1366 |
| 13728 | 13646 | 26305 | 1137 | 1176 | 23164 | 12013 | 14052 | 1923 | 3017 | 6039 | 28110 |
| 2269 | 12862 | 14650 | 26860 | 24246 | 26349 | 6230 | 13423 | 28379 | 24327 | 22335 | 27059 |
| 5252 | 3512 | 11145 | 13509 | 17491 | 24640 | 19537 | 6040 | 6064 | 14762 | 22972 | 27268 |
| 19230 | 20315 | 3922 | 27400 | 25859 | 4202 | 9549 | 10046 | 15900 | 27079 | 29702 | 19917 |
| 18269 | 25185 | 7661 | 17257 | 17240 | 3719 | 13492 | 6635 | 24046 | 24600 | 7986 | 18415 |
| 5491 | 12481 | 29019 | 24347 | 6888 | 11801 | 21963 | 25757 | 5003 | 15580 | 26580 | 15611 |
| 13675 | 14450 | 20107 | 22929 | 6560 | 14512 | 8777 | 6845 | 4758 | 9593 | 11697 | 21489 |
| 3395 | 5145 | 28692 | 17342 | 26970 | 7063 | 27354 | 21164 | 20214 | 23709 | 21089 | 20871 |
| 22705 | 23847 | 30036 | 4155 | 9143 | 3600 | 23151 | 6299 | 5552 | 10012 | 27722 | 28232 |
| 22273 | 27148 | 14883 | 18355 | 8460 | 26598 | 17781 | 24324 | 11484 | 1626 | 14890 | 3902 |
| 24684 | 23264 | 15852 | 15857 | 8083 | 27508 | 28244 | 6444 | 23032 | 3421 | 28103 | 20319 |
| 24322 | 16204 | 9149 | 14884 | 14910 | 25311 | 23570 | 11989 | 24080 | 12083 | 29922 | 14801 |

(continued)

SEQ ID NO: homolog SEQ ID NOs

| SEQ ID NO: | | | | | | | | | | | | |
|---|---|---|---|---|---|---|---|---|---|---|---|---|
| | 30209 | 1916 | 3919 | 5228 | 7888 | 7756 | 1950 | 11605 | 22366 | 18828 | 5942 | 4311 |
| | 4234 | 16821 | 7190 | 9928 | 28928 | 20678 | 13547 | 6526 | 22520 | 14376 | 12307 | 24837 |
| | 10845 | 18487 | | | | | | | | | | |
| 369: | 13170 | 23347 | 18716 | 10866 | 14771 | 23343 | 9418 | 19015 | 9403 | 11257 | 11289 | 11260 |
| | 4351 | 7119 | 18253 | 2213 | 13316 | 4199 | 7149 | 7151 | 1717 | 25457 | 14944 | 25463 |
| | 25491 | 24250 | 21760 | 29726 | 23084 | 29448 | 11979 | 14733 | 14766 | 7092 | 29827 | 29835 |
| | 7200 | 26496 | 2652 | 2645 | 28309 | 21090 | 26255 | 23367 | 4648 | 22135 | 12428 | 28138 |
| | 19267 | 1303 | 13335 | 4454 | 24546 | 30326 | 25458 | 4413 | 29698 | 6201 | 29974 | 2000 |
| | 2004 | 18330 | 25804 | 27979 | 25383 | 5805 | 1136 | 19287 | 16541 | 982 | 23403 | 23817 |
| | 6852 | 14133 | 15684 | 24608 | 25262 | 26579 | | | | | | |
| 370: | 12753 | 12731 | 12728 | 11403 | 22388 | 27738 | 28210 | 18436 | 5054 | 1888 | 12994 | 13000 |
| | 24466 | 27576 | 1320 | 8979 | 7160 | 8981 | 5482 | 27562 | 17524 | 9039 | 13737 | 20944 |
| | 24036 | 23292 | 20040 | 10403 | 22550 | 12916 | 2165 | 22967 | 26853 | 936 | 30163 | 20887 |
| | 3761 | 11217 | 4322 | 1215 | 26633 | 24957 | 7421 | 24155 | 1080 | 10806 | 26478 | 30322 |
| | 3866 | 8861 | 14703 | 24120 | 5318 | 23124 | 6596 | 2064 | 6491 | 18458 | 7357 | 18700 |
| | 22683 | 11073 | | | | | | | | | | |
| 371: | 20445 | 2217 | 20270 | 4554 | 14594 | 3117 | 22113 | 26956 | 13458 | 29996 | 23762 | 27167 |
| | 16001 | 24338 | 5485 | 18889 | 20404 | 15313 | 24472 | | | | | |
| 372: | 20445 | 2217 | 20270 | 4554 | 14594 | 3117 | 22113 | 26956 | 13458 | 29996 | 23762 | 27167 |
| | 16001 | 24338 | 5485 | 18889 | 20404 | 15313 | 24472 | | | | | |
| 373: | 28810 | 28813 | 26634 | 28811 | 17236 | 20813 | 2597 | 24300 | 3969 | 19544 | 29130 | 19170 |
| | 14664 | 27056 | 3932 | 25962 | 7932 | 19048 | 12523 | 6656 | 23273 | 17673 | 28574 | 9081 |
| | 28807 | 29229 | 11689 | 11685 | 14096 | 5030 | 26606 | 20159 | 30304 | 21507 | 4790 | 27880 |
| | 1747 | | | | | | | | | | | |
| 374: | 1997 | 12632 | 24857 | 27956 | 2924 | 27991 | 25427 | 22615 | 26561 | 2484 | 3588 | 3606 |
| | 17084 | 19450 | 15066 | 7815 | 12090 | 3082 | 25660 | 5237 | 28991 | 25385 | 15696 | 16078 |
| | 15290 | 15465 | 23640 | | | | | | | | | |
| 375: | 2052 | 14301 | 15310 | 19682 | 12244 | 1711 | 10145 | 17287 | 25104 | 20800 | 14168 | 11278 |
| | 15892 | 8185 | 871 | 18661 | 25589 | 21660 | 24122 | 29541 | 10643 | 28589 | 19031 | 14777 |
| | 1660 | 20957 | 21918 | 20894 | 18742 | 4045 | 23837 | 2662 | 25061 | 20101 | 23046 | 25684 |
| | 1710 | 17836 | 25631 | 26350 | 2023 | 23277 | 23243 | 20619 | 1601 | 7583 | 29436 | 27776 |
| | 9217 | 11134 | 20290 | 10380 | 29123 | | | | | | | |
| 376: | 2052 | 14301 | 15310 | 19682 | 12244 | 1711 | 10145 | 17287 | 25104 | 20800 | 14168 | 11278 |

(continued)

SEQ ID NO: homolog SEQ ID NOs

| | | | | | | | | | | | | |
|---|---|---|---|---|---|---|---|---|---|---|---|---|
| | 15892 | 8185 | 871 | 18661 | 25589 | 21660 | 24122 | 29541 | 10643 | 28589 | 19031 | 14777 |
| | 1660 | 20957 | 21918 | 20894 | 18742 | 4045 | 23837 | 2662 | 25061 | 20101 | 23046 | 25684 |
| | 1710 | 17836 | 25631 | 26350 | 2023 | 23277 | 23243 | 20619 | 1601 | 7583 | 29436 | 27776 |
| | 9217 | 11134 | 20290 | 10380 | 29123 | | | | | | | |
| 377: | 2052 | 14301 | 15310 | 19682 | 12244 | 1711 | 10145 | 17287 | 25104 | 20800 | 14168 | 11278 |
| | 15892 | 8185 | 871 | 18661 | 25589 | 21660 | 24122 | 29541 | 10643 | 28589 | 19031 | 14777 |
| | 1660 | 20957 | 21918 | 20894 | 18742 | 4045 | 23837 | 2662 | 25061 | 20101 | 23046 | 25684 |
| | 1710 | 17836 | 25631 | 26350 | 2023 | 23277 | 23243 | 20619 | 1601 | 7583 | 29436 | 27776 |
| | 9217 | 11134 | 20290 | 10380 | 29123 | | | | | | | |
| 378: | 23140 | 25000 | 26947 | 28724 | 23889 | 3221 | 26051 | 5557 | 25493 | 16279 | 17118 | 24070 |
| | 26204 | | | | | | | | | | | |
| 379: | 28296 | 2698 | 12421 | 27951 | 2336 | 30028 | 5453 | 16175 | 23688 | 8115 | 15431 | 1569 |
| | 1511 | 1952 | 1238 | 9517 | 9483 | 17742 | 22869 | 13147 | 27080 | 6172 | 7760 | 17732 |
| | 24149 | 4338 | 5279 | 16406 | 18875 | 13151 | 20727 | 844 | 20551 | 16562 | 1800 | 825 |
| | 2045 | 3203 | 5089 | 27947 | 7388 | 23567 | 18356 | 3000 | 4140 | 10665 | 29686 | 29810 |
| | 11547 | 1996 | 18123 | 12864 | 27240 | 857 | 12136 | 19282 | 18905 | 26012 | 20870 | 22878 |
| | 16959 | 21306 | 29268 | 10239 | 12089 | 14414 | 3526 | 12135 | 11632 | 8073 | 23310 | 26628 |
| | 23972 | 8093 | 15105 | 21565 | 13160 | 27030 | 5244 | 9850 | 18082 | 17748 | 19176 | 13637 |
| | 14111 | 3728 | 8961 | 7564 | 14610 | 2524 | 29301 | 18773 | 14899 | 9693 | 10870 | 13429 |
| | 22595 | 8941 | 8956 | 8375 | 2202 | 12599 | 23672 | 9547 | 13495 | 24659 | 5648 | 28877 |
| | 26904 | 17937 | 18858 | 2739 | 15624 | 29164 | 9504 | 16016 | 17672 | 10707 | 21724 | 25763 |
| | 10773 | 25592 | 4415 | 1174 | 29151 | 19419 | 25057 | 19101 | 15943 | 5057 | 15655 | 21208 |
| | 12192 | 24991 | 9505 | 24997 | 23657 | 24977 | 23612 | 23686 | 9704 | 28090 | 17978 | 17981 |
| | 11114 | 7242 | 11097 | 11205 | 23897 | 25084 | 1354 | 22555 | 10356 | 19904 | 21467 | 10293 |
| | 14483 | 13057 | 10115 | 22159 | 21256 | 22190 | 9893 | 15801 | 4032 | 25905 | 12176 | 7171 |
| | 7615 | 8507 | 6677 | 27962 | 3708 | 16231 | 17678 | 4644 | 24218 | 3852 | 11206 | 9375 |
| | 5841 | 19709 | 29153 | 20442 | 9475 | 16413 | 24732 | 14071 | 8798 | 29906 | 7333 | 25443 |
| | 8133 | 7277 | 27473 | 29015 | 7212 | 6771 | 6837 | 28876 | 15813 | 25460 | 17503 | 10859 |
| | 26936 | 15399 | 26471 | 11806 | 8742 | 24631 | 9555 | 29061 | 8611 | 6152 | 20070 | 25884 |
| | 24484 | 19645 | 17930 | 14710 | 10116 | 27844 | 22939 | 8800 | 8846 | 29567 | 26872 | 10467 |
| | 10375 | 9456 | 26454 | 29926 | 7603 | 20203 | 21429 | 17441 | 9083 | 13448 | 28316 | 5457 |
| | 15001 | 10760 | 28591 | 22887 | 22225 | 18530 | 15693 | 19050 | 29258 | 26410 | 7192 | 7193 |
| | 12624 | 18606 | 20584 | 2719 | 20589 | 17597 | 19362 | 920 | 2695 | 17028 | 29742 | 17056 |

SEQ ID NO: homolog SEQ ID NOs

| | | | | | | | | | | | |
|---|---|---|---|---|---|---|---|---|---|---|---|
| | 2455 | 10032 | 28345 | 9546 | 14659 | 2241 | 19539 | 11345 | 30202 | 12416 | 5937 | 28287 |
| | 11385 | 3986 | 6313 | 14790 | 7891 | 4823 | 13382 | 27959 | 791 | 16747 | 19146 | 9659 |
| | 3612 | 29755 | 3791 | 19343 | 18559 | 15277 | 7425 | 6972 | 18246 | 21010 | 5735 | 17030 |
| | 9933 | 23529 | 23526 | 19807 | 19804 | 15475 | 17422 | 3663 | 20810 | 20808 | 16101 | 3018 |
| | 15207 | 20508 | 2456 | 907 | 21786 | 9624 | 18838 | 27470 | 22375 | 12600 | 6137 | 14444 |
| | 10851 | 20238 | 12596 | 23196 | 26304 | 11688 | 24696 | 8550 | 27051 | 15022 | 18231 | 20742 |
| | 19842 | 21165 | 16829 | 29790 | 1405 | 20649 | 26249 | 607 | 3908 | 22099 | 606 | 4057 |
| | 6606 | 21066 | 23652 | 22318 | 3425 | 11110 | 19962 | 25740 | 9777 | 12012 | 28182 | 5808 |
| | 24054 | 13314 | 1456 | 8667 | 8695 | 4447 | 13521 | 24592 | 21534 | 8877 | 5672 | 22060 |
| | 3806 | 23982 | 2366 | 18009 | 18074 | 14518 | 12837 | 12086 | 28725 | 11755 | 27502 | 19531 |
| | 14879 | 2409 | 28307 | 23951 | 23904 | 21560 | 7377 | 4463 | 17352 | 17284 | 13345 | 13343 |
| | 13100 | 9341 | 6646 | 28894 | 6611 | 13431 | 13425 | 2460 | 8819 | 30168 | 6195 | 978 |
| | 23832 | 8596 | 11448 | 8168 | 10789 | | | | | | | |
| 380: | 2827 | 21648 | 22239 | 20396 | 6761 | 6766 | 6765 | 5350 | 18594 | 13414 | 3105 | 3122 |
| | 10761 | 21096 | 1570 | 8290 | 24441 | 1819 | 7703 | 17446 | 15026 | 11413 | 16074 | 28826 |
| | 8926 | 2985 | 23827 | 18789 | 21796 | 1100 | 27681 | 12292 | 25281 | 6132 | 29085 | 1437 |
| | 17574 | 20269 | 22049 | 1861 | 15353 | 9151 | 16462 | 29950 | 30298 | 8628 | 4670 | 29916 |
| | 22022 | 20351 | 6265 | 26983 | 1260 | 29757 | 11581 | 1116 | 11626 | 25314 | 12647 | 21487 |
| | 22732 | 8349 | 9194 | 5078 | 28322 | 23446 | 29598 | 28404 | 29768 | 27807 | 1435 | 22280 |
| | 24172 | 12973 | 28461 | 21035 | 29323 | 8678 | 26140 | 3550 | 1842 | 27577 | 21267 | 29953 |
| | 2722 | 12906 | 20886 | 10787 | 9494 | 20065 | 17225 | 28472 | 14449 | 4088 | 13827 | 4769 |
| | 20362 | 6736 | 4217 | 9914 | 13158 | 22017 | 16750 | 14583 | 14800 | 13660 | 27988 | 6695 |
| | 22432 | 28758 | 14595 | 1389 | 22126 | 2553 | 7024 | 17822 | 2289 | 6487 | 6484 | 11479 |
| | 6229 | 6502 | 14051 | 16467 | 21170 | 8907 | 8887 | 28847 | 3948 | 11527 | 13632 | 5217 |
| | 1071 | 12831 | 21444 | 28123 | 5960 | 29252 | 10600 | 1560 | 15102 | 21836 | 21517 | 1767 |
| | 16340 | 15215 | 19213 | 13163 | 7408 | 5989 | 1250 | 14864 | 2145 | 20980 | 20786 | 25489 |
| | 19890 | 10154 | 8406 | 3192 | 29520 | 21938 | 25996 | 1922 | 19108 | 2311 | 3944 | 26383 |
| | 25937 | 12706 | 23547 | 19501 | 19503 | 19415 | 13681 | 21632 | 22326 | 21619 | 21916 | 6584 |
| | 23359 | 28375 | 22553 | 23759 | 1138 | 18646 | 12204 | 23294 | 1286 | 18001 | 9694 | 21865 |
| | 3196 | 20557 | 27271 | 16962 | 2957 | 2952 | 15708 | 3560 | 5363 | 5347 | 11222 | 30129 |
| | 7897 | 3361 | 26621 | 22294 | 21939 | 28384 | 17873 | 10472 | 23061 | 9577 | 4111 | 22934 |
| | 12654 | 16339 | 4257 | 6797 | 12457 | 13756 | 24205 | 15796 | 27883 | 16773 | 28772 | 1538 |
| | 18102 | 18360 | 6318 | 3483 | 21978 | 13730 | 12004 | 29583 | 23641 | 22268 | 4137 | 1144 |

(continued)

SEQ ID NO: homolog SEQ ID NOs

|  | | | | | | | | | | | |
|---|---|---|---|---|---|---|---|---|---|---|---|
| 11555 | 24121 | 28029 | 5706 | 10829 | 980 | 29194 | 13198 | 9303 | 20202 | 12102 | 3583 |
| 29251 | 3381 | 18011 | 4119 | 9637 | 20873 | 29840 | 21443 | 29657 | 2501 | 4589 | 13013 |
| 18432 | 4806 | 10739 | 8738 | 11788 | 26334 | 1398 | 6712 | 4292 | 7784 | 744 | 27037 |
| 12145 | 10990 | 3307 | 23088 | 28782 | 2612 | 12058 | 18178 | 25391 | 1561 | 24068 | 23106 |
| 20067 | 29929 | 5270 | 21145 | 29080 | 28832 | 9542 | 12968 | 7842 | 5783 | 16238 | 7338 |
| 19959 | 28042 | 13790 | 4801 | 7485 | 1430 | 9377 | 3050 | 12433 | 10545 | 12519 | 13723 |
| 9393 | 26727 | 27628 | 12352 | 17120 | 381 | 28393 | 23274 | 13906 | | | |

| 381: | | | | | | | | | | | |
|---|---|---|---|---|---|---|---|---|---|---|---|
| 2827 | 21648 | 22239 | 20396 | 6761 | 6766 | 6765 | 5350 | 18594 | 13414 | 3105 | 3122 |
| 10761 | 21096 | 1570 | 8290 | 24441 | 1819 | 7703 | 17446 | 15026 | 11413 | 16074 | 28826 |
| 8926 | 2985 | 23827 | 18789 | 21796 | 1100 | 27681 | 12292 | 25281 | 6132 | 29085 | 1437 |
| 17574 | 20269 | 22049 | 1861 | 15353 | 9151 | 16462 | 29950 | 30298 | 8628 | 4670 | 29916 |
| 22022 | 20351 | 6265 | 26983 | 1260 | 29757 | 11581 | 1116 | 11626 | 25314 | 12647 | 21487 |
| 22732 | 8349 | 9194 | 5078 | 28322 | 23446 | 29598 | 28404 | 29768 | 27807 | 1435 | 22280 |
| 24172 | 12973 | 28461 | 21035 | 29323 | 8678 | 26140 | 3550 | 1842 | 27577 | 21267 | 29953 |
| 2722 | 12906 | 20886 | 10787 | 9494 | 20065 | 17225 | 28472 | 14449 | 4088 | 13827 | 4769 |
| 20362 | 6736 | 4217 | 9914 | 13158 | 22017 | 16750 | 14583 | 14800 | 13660 | 27988 | 6695 |
| 22432 | 28758 | 14595 | 1389 | 22126 | 2553 | 7024 | 17822 | 2289 | 6487 | 6484 | 11479 |
| 6229 | 6502 | 14051 | 16467 | 21170 | 8907 | 8887 | 28847 | 3948 | 11527 | 13632 | 5217 |
| 1071 | 12831 | 21444 | 28123 | 5960 | 29252 | 10600 | 1560 | 15102 | 21836 | 21517 | 1767 |
| 16340 | 15215 | 19213 | 13163 | 7408 | 5989 | 1250 | 14864 | 2145 | 20980 | 20786 | 25489 |
| 19890 | 10154 | 8406 | 3192 | 29520 | 21938 | 25996 | 1922 | 19108 | 2311 | 3944 | 26383 |
| 25937 | 12706 | 23547 | 19501 | 19503 | 19415 | 13681 | 21632 | 22326 | 21619 | 21916 | 6584 |
| 23359 | 28375 | 22553 | 23759 | 1138 | 18646 | 12204 | 23294 | 1286 | 18001 | 9694 | 21865 |
| 3196 | 20557 | 27271 | 16962 | 2957 | 2952 | 15708 | 3560 | 5363 | 5347 | 11222 | 30129 |
| 7897 | 3361 | 26621 | 22294 | 21939 | 28384 | 17873 | 10472 | 23061 | 9577 | 4111 | 22934 |
| 12654 | 16339 | 4257 | 6797 | 12457 | 13756 | 24205 | 15796 | 27883 | 16773 | 28772 | 1538 |
| 18102 | 18360 | 6318 | 3483 | 21978 | 13730 | 12004 | 29583 | 23641 | 22268 | 4137 | 1144 |
| 11555 | 24121 | 28029 | 5706 | 10829 | 980 | 29194 | 13198 | 9303 | 20202 | 12102 | 3583 |
| 29251 | 3381 | 18011 | 4119 | 9637 | 20873 | 29840 | 21443 | 29657 | 2501 | 4589 | 13013 |
| 18432 | 4806 | 10739 | 8738 | 11788 | 26334 | 1398 | 6712 | 4292 | 7784 | 744 | 27037 |
| 12145 | 10990 | 3307 | 23088 | 28782 | 2612 | 12058 | 18178 | 25391 | 1561 | 24068 | 23106 |
| 20067 | 29929 | 5270 | 21145 | 29080 | 28832 | 9542 | 12968 | 7842 | 5783 | 16238 | 7338 |
| 19959 | 28042 | 13790 | 4801 | 7485 | 1430 | 9377 | 3050 | 12433 | 10545 | 12519 | 13723 |

SEQ ID NO: homolog SEQ ID NOs

| | | | | | | | | | | | | |
|---|---|---|---|---|---|---|---|---|---|---|---|---|
| | 9393 | 26727 | 27628 | 12352 | 17120 | 380 | 28393 | 23274 | 13906 | | | |
| 382: | 22849 | 28698 | 3926 | 29319 | 19044 | 12315 | 13233 | 4943 | 26735 | 21017 | 29626 | 14707 |
| | 20033 | 24228 | 23082 | 16942 | 24863 | 11996 | 24875 | 2940 | 27044 | 10513 | 12869 | 25976 |
| | 29218 | 16383 | 18278 | 19118 | 20856 | 15743 | 15668 | 19559 | 14494 | 14144 | 14348 | 28910 |
| | 26422 | 11900 | 27010 | 6294 | 8803 | 8055 | 25725 | 21709 | 3758 | 8246 | 26742 | 19244 |
| | 20439 | 4449 | 27504 | 14836 | 21148 | 15885 | 5686 | 24529 | 20199 | 20822 | 7819 | 26136 |
| | 16052 | 10385 | 21803 | 28755 | 7098 | 2013 | 18658 | 12396 | 10047 | 30146 | 4531 | 9165 |
| | 17108 | 20325 | 25687 | 29538 | 25064 | 17961 | 12998 | 14878 | 4055 | 5532 | 9114 | 4462 |
| | 11568 | 22801 | 22814 | 13598 | 7413 | 8520 | 29543 | 29068 | 28478 | 15218 | 4329 | 8257 |
| | 25107 | 3629 | 2575 | 2930 | 10065 | 16575 | 11121 | 3293 | 16298 | 10000 | 20307 | 26570 |
| | 12003 | 14217 | 6796 | 7370 | 21316 | 12023 | 9743 | 19779 | 28347 | 30239 | 17241 | 29695 |
| | 13804 | 13590 | 17622 | 1480 | 17824 | 1551 | 18779 | 30188 | 25152 | 26297 | 21180 | 29000 |
| | 19328 | 13759 | 24492 | 14076 | 22587 | 16492 | 14325 | 17910 | 26741 | 22123 | 9958 | 21252 |
| | 18277 | 26751 | 21520 | 5285 | 22250 | 9013 | 11063 | 23451 | 12978 | 24923 | 3764 | 2441 |
| | 5760 | 9531 | 22807 | 23708 | 1244 | 26813 | 18323 | 18447 | 26592 | 20474 | 5605 | 24848 |
| | 20661 | 12223 | 20501 | 3861 | 6687 | 24084 | 27671 | 26018 | 2610 | 5972 | 9568 | 26961 |
| | 15742 | 19254 | 21446 | 862 | 18686 | 870 | 26423 | 28240 | 13770 | 15084 | 25239 | 18097 |
| | 14770 | 23101 | 13650 | 5797 | 7830 | 23402 | 24790 | 24051 | 7463 | 4760 | 9181 | 25539 |
| | 22474 | 28798 | 6759 | 21411 | 20842 | 26057 | 29733 | 6838 | 994 | 25118 | 6443 | 7484 |
| | 16653 | 27468 | 10383 | 18601 | 20567 | 22812 | 29691 | 22817 | 22129 | 26672 | 1913 | 9878 |
| | 20485 | 957 | 26149 | 16283 | 4641 | 13016 | 23684 | 9518 | 7785 | 21621 | 11164 | 7436 |
| | 7864 | 7438 | 19439 | 19405 | 27135 | 22693 | 19243 | 13073 | 23276 | 9304 | 8019 | 8435 |
| | 17722 | 4682 | 14532 | 11451 | 14761 | 6665 | 6660 | 18424 | 24738 | 22791 | 2743 | 15136 |
| | 17797 | 2361 | 4425 | 2527 | 9929 | 20780 | 2635 | 18477 | 25855 | 9389 | 17539 | 15468 |
| | 16892 | 23705 | 29688 | 4262 | 4468 | 8933 | 24131 | 27566 | 11777 | 13693 | 13762 | 9308 |
| | 21427 | 7514 | 2990 | 5742 | | | | | | | | |
| 383: | 21751 | 25909 | 12584 | 2097 | 16744 | 18713 | 8832 | 17452 | 11499 | 3388 | 6401 | 3001 |
| | 5699 | 2993 | 2998 | 7765 | 3459 | 12559 | 22928 | 13197 | 25040 | 5190 | 16885 | 5189 |
| | 27694 | 27229 | 3547 | 1172 | 3403 | 1998 | 19270 | 29387 | 3263 | | | |
| 384: | 21751 | 25909 | 12584 | 2097 | 16744 | 18713 | 8832 | 17452 | 11499 | 3388 | 6401 | 3001 |
| | 5699 | 2993 | 2998 | 7765 | 3459 | 12559 | 22928 | 13197 | 25040 | 5190 | 16885 | 5189 |
| | 27694 | 27229 | 3547 | 1172 | 3403 | 1998 | 19270 | 29387 | 3263 | | | |
| 385: | 13968 | 1825 | 21329 | 13274 | 14367 | 28380 | 25070 | 9472 | 5541 | 22356 | 8404 | 19228 |

SEQ ID NO: homolog SEQ ID NOs

| SEQ ID NO | | | | | | | | | | | | |
|---|---|---|---|---|---|---|---|---|---|---|---|---|
| | 13783 | 20684 | 12911 | 7142 | 10098 | 22443 | 4472 | 24839 | 28986 | 22053 | 24289 | 22753 |
| | 2152 | 29227 | 3394 | 22820 | 6648 | 3979 | 22224 | 12965 | 17995 | 18469 | 12772 | 25452 |
| | 10598 | 27647 | 10157 | 10381 | 6448 | 14214 | 28617 | 23813 | 15137 | 21908 | 22043 | 2625 |
| | 17773 | 20709 | 30223 | 13443 | 6438 | 6420 | | | | | | |
| 386: | 13968 | 1825 | 21329 | 13274 | 14367 | 28380 | 25070 | 9472 | 5541 | 22356 | 8404 | 19228 |
| | 13783 | 20684 | 12911 | 7142 | 10098 | 22443 | 4472 | 24839 | 28986 | 22053 | 24289 | 22753 |
| | 2152 | 29227 | 3394 | 22820 | 6648 | 3979 | 22224 | 12965 | 17995 | 18469 | 12772 | 25452 |
| | 10598 | 27647 | 10157 | 10381 | 6448 | 14214 | 28617 | 23813 | 15137 | 21908 | 22043 | 2625 |
| | 17773 | 20709 | 30223 | 13443 | 6438 | 6420 | | | | | | |
| 387: | 3201 | 4339 | 29374 | 18752 | 16428 | 27487 | 6729 | 5611 | 29493 | 9432 | 12344 | 8113 |
| | 22610 | 12927 | 7764 | | | | | | | | | |
| 388: | 3201 | 4339 | 29374 | 18752 | 16428 | 27487 | 6729 | 5611 | 29493 | 9432 | 12344 | 8113 |
| | 22610 | 12927 | 7764 | | | | | | | | | |
| 389: | 1223 | 8875 | 27741 | 10907 | 11594 | 27784 | 27736 | 27779 | 27732 | 9969 | 16232 | 16226 |
| | 10002 | 4243 | 14569 | 19275 | 24585 | 10024 | 4920 | 9942 | 9973 | 9979 | 9784 | 9945 |
| | 16188 | 29944 | 4150 | 23660 | 2162 | 4488 | 17190 | 19481 | 17848 | 27848 | 29075 | 21228 |
| | 26113 | 20639 | 14981 | 27816 | 21712 | 22291 | 12027 | 27264 | 4117 | 24044 | 23618 | 15076 |
| | 14975 | 17926 | 28124 | 6363 | 20097 | 3568 | 23729 | 12030 | 4546 | 2847 | 3763 | 10604 |
| | 2728 | 6582 | 8900 | 10640 | 23123 | 1657 | 28783 | 27093 | 23440 | 24722 | 16222 | 30089 |
| | 5723 | 10895 | 20598 | 17164 | 29403 | 26217 | 18145 | 24490 | 25395 | 7458 | 11924 | 14387 |
| | 20577 | 21262 | 16719 | 14977 | 17971 | 21220 | 7422 | 12629 | 25113 | 19575 | 19613 | 11579 |
| | 22823 | 28656 | 24510 | 11180 | 27885 | 20360 | 24503 | 26266 | 19313 | 13539 | 18984 | 19057 |
| | 21600 | 3724 | 5635 | 22697 | 16934 | 23148 | 18887 | 9132 | 28438 | 27066 | 4422 | 24271 |
| | 23713 | 17877 | 18540 | 12884 | 14589 | 29278 | 21721 | 25219 | 16369 | 14978 | 22746 | 9975 |
| | 26176 | 23868 | 28547 | 24591 | 21196 | 17618 | 17589 | 21505 | 4960 | 28727 | 28795 | 20615 |
| | 26100 | 25258 | 24323 | 20966 | 11395 | 7099 | 14337 | 26868 | 13289 | 11862 | 21121 | 3961 |
| | 1180 | 25849 | 24418 | 2307 | 6150 | 855 | 9282 | 22325 | 22329 | 18864 | 29069 | 25401 |
| | 21150 | 20044 | 21222 | 6947 | 27845 | 15338 | 16227 | 15341 | 13039 | 29081 | 26614 | 16357 |
| | 895 | 7681 | 10055 | 9939 | 26731 | 11716 | 6460 | 1558 | 28632 | 25786 | 16515 | 16513 |
| | 16510 | 4533 | 25063 | 4197 | 24589 | 926 | 9982 | 1235 | 1445 | 1446 | 1448 | 1466 |
| | 14985 | 16417 | 9806 | 7766 | 15008 | 7999 | 27749 | 14952 | 11220 | 9934 | 1611 | 3828 |
| | 14973 | 2387 | 15604 | 20124 | 2816 | 10896 | 18470 | | | | | |
| 390: | 1223 | 8875 | 27741 | 10907 | 11594 | 27784 | 27736 | 27779 | 27732 | 9969 | 16232 | 16226 |

(continued)

SEQ ID NO: homolog SEQ ID NOs

| | | | | | | | | | | | |
|---|---|---|---|---|---|---|---|---|---|---|---|
| | 10002 | 4243 | 14569 | 19275 | 24585 | 10024 | 4920 | 9942 | 9973 | 9979 | 9784 | 9945 |
| | 16188 | 29944 | 4150 | 23660 | 2162 | 4488 | 19386 | 17190 | 19481 | 17848 | 27848 | 29075 |
| | 21228 | 26113 | 20639 | 14981 | 27816 | 21712 | 22291 | 12027 | 27264 | 4117 | 24044 | 23618 |
| | 15076 | 14975 | 17926 | 28124 | 6363 | 20097 | 3568 | 23729 | 12030 | 4546 | 2847 | 3763 |
| | 10604 | 2728 | 6582 | 8900 | 23123 | 10640 | 1657 | 28783 | 27093 | 17641 | 23440 | 24722 |
| | 16222 | 30089 | 5723 | 10895 | 20598 | 17164 | 29403 | 26217 | 18145 | 24490 | 25395 | 7458 |
| | 11924 | 14387 | 20577 | 21262 | 16719 | 14977 | 17971 | 21220 | 7422 | 12629 | 25113 | 19575 |
| | 19613 | 11579 | 22823 | 28656 | 24510 | 11180 | 27885 | 20360 | 24503 | 26266 | 19313 | 13539 |
| | 18984 | 19057 | 21600 | 3724 | 5635 | 22697 | 16934 | 23148 | 18887 | 9132 | 28438 | 27066 |
| | 4422 | 24271 | 23713 | 17877 | 18540 | 12884 | 14589 | 29278 | 21721 | 25219 | 16369 | 14978 |
| | 22746 | 9975 | 26176 | 23868 | 28547 | 24591 | 21196 | 17618 | 17589 | 21505 | 4960 | 28727 |
| | 26100 | 25258 | 28795 | 20615 | 24323 | 20966 | 11395 | 7099 | 14337 | 26868 | 1180 | 25849 |
| | 24418 | 9282 | 22325 | 22329 | 18864 | 29069 | 25401 | 21150 | 6947 | 27845 | 13039 | 29081 |
| | 26614 | 16357 | 895 | 7681 | 10055 | 9939 | 26731 | 11716 | 6460 | 1558 | 28632 | 25786 |
| | 25063 | 4197 | 24589 | 926 | 9982 | 1235 | 1445 | 1446 | 1448 | 1466 | 14985 | 16417 |
| | 9806 | 7766 | 15008 | 7999 | 27749 | 14952 | 11220 | 9934 | 1611 | 3828 | 14973 | 2387 |
| | 15604 | 20124 | 6542 | 29138 | 1689 | 6203 | | | | | | |
| 391: | 2827 | 21648 | 22239 | 20396 | 6761 | 6766 | 6765 | 5350 | 18594 | 13414 | 3105 | 3122 |
| | 10761 | 21096 | 1570 | 8290 | 24441 | 1819 | 7703 | 17446 | 15026 | 11413 | 16074 | 28826 |
| | 8926 | 2985 | 18789 | 23827 | 21796 | 1100 | 27681 | 12292 | 25281 | 6132 | 29085 | 1437 |
| | 17574 | 20269 | 22049 | 1861 | 15353 | 9151 | 16462 | 29950 | 30298 | 8628 | 4670 | 29916 |
| | 22022 | 20351 | 6265 | 26983 | 1260 | 29757 | 11581 | 1116 | 11626 | 25314 | 12647 | 21487 |
| | 22732 | 8349 | 9194 | 5078 | 28322 | 23446 | 29598 | 28404 | 29768 | 27807 | 1435 | 22280 |
| | 24172 | 12973 | 21035 | 28461 | 29323 | 8678 | 26140 | 3550 | 1842 | 27577 | 21267 | 2722 |
| | 29953 | 12906 | 10787 | 20886 | 9494 | 20065 | 17225 | 28472 | 4088 | 14449 | 13827 | 20362 |
| | 4769 | 6736 | 4217 | 9914 | 13158 | 22017 | 16750 | 14583 | 14800 | 13660 | 27988 | 6695 |
| | 22432 | 28758 | 14595 | 1389 | 22126 | 2553 | 7024 | 17822 | 2289 | 6487 | 6484 | 11479 |
| | 6502 | 6229 | 14051 | 16467 | 21170 | 8907 | 8887 | 28847 | 3948 | 11527 | 13632 | 5217 |
| | 1071 | 12831 | 21444 | 28123 | 5960 | 29252 | 10600 | 1560 | 15102 | 21836 | 21517 | 1767 |
| | 16340 | 15215 | 19213 | 13163 | 5989 | 7408 | 1250 | 14864 | 2145 | 20980 | 20786 | 25489 |
| | 19890 | 10154 | 8406 | 3192 | 29520 | 21938 | 25996 | 1922 | 19108 | 2311 | 25937 | 12706 |
| | 23547 | 19503 | 19501 | 19415 | 13681 | 21632 | 22326 | 21619 | 21916 | 6584 | 23359 | 28375 |
| | 22553 | 23759 | 1138 | 18646 | 12204 | 23294 | 1286 | 18001 | 9694 | 21865 | 3196 | 20557 |

(continued)

SEQ ID NO: homolog SEQ ID NOs

| | | | | | | | | | | | | |
|---|---|---|---|---|---|---|---|---|---|---|---|---|
| | 27271 | 16962 | 2957 | 2952 | 15708 | 3560 | 5363 | 5347 | 11222 | 30129 | 7897 | 3361 |
| | 26621 | 22294 | 21939 | 28384 | 17873 | 23061 | 9577 | 4111 | 22934 | 16339 | 12654 | 4257 |
| | 6797 | 12457 | 13756 | 24205 | 15796 | 27883 | 16773 | 28407 | 1538 | 18102 | 6318 | 3483 |
| | 14531 | 28251 | 23641 | 10408 | 24774 | 27457 | 24121 | 28029 | 5706 | 17605 | 10829 | 980 |
| | 1674 | 3583 | 29251 | 3381 | 18011 | 8457 | 4806 | 27733 | 16489 | 12058 | 18178 | 25391 |
| | 1561 | 24068 | 23106 | 20067 | 29929 | 5270 | 21145 | 29080 | 28832 | 9542 | 12968 | 7842 |
| | 5783 | 16238 | 7338 | 19959 | 28042 | 13790 | 4801 | 7485 | 1430 | 9377 | 3050 | 12433 |
| | 12519 | 10545 | 13723 | 9393 | 26727 | 27628 | 12352 | 17120 | | | | |
| 392: | 4335 | 26371 | 12156 | 6292 | 28709 | 4712 | 12072 | 6693 | 12622 | 9633 | 11161 | 5715 |
| | 9656 | 8847 | 6791 | 16598 | 3391 | 16281 | 9675 | 21773 | 15068 | 15042 | 5758 | 29199 |
| | 24947 | 21917 | 6755 | 11071 | 18699 | 13058 | 12051 | 2448 | 18240 | 29145 | 21954 | 24471 |
| | 16612 | 19392 | 23188 | 9648 | 16669 | 28511 | 22138 | 12521 | 13697 | 8202 | 7554 | |
| 393: | 22012 | 22009 | 23097 | 17935 | 17145 | 17150 | 10289 | 17148 | 17155 | 2619 | 7598 | 9584 |
| | 13051 | 12602 | 6890 | 14726 | 16853 | 17191 | 18766 | 9943 | 15167 | 24924 | 7057 | 19743 |
| | 15143 | 26223 | 25967 | 24936 | 25508 | 22918 | 15164 | 15169 | 24927 | 18104 | 19791 | 11642 |
| | 11639 | 18799 | 29059 | 21815 | 11929 | 25942 | 27466 | 11551 | 19055 | 18873 | 14426 | 29911 |
| | 14427 | 14430 | 13640 | 13635 | 27450 | 6681 | 3753 | 11874 | 13847 | 13850 | 3559 | 22144 |
| | 3585 | 11673 | 23723 | 21800 | 21802 | 21828 | 14934 | 8409 | 2257 | 21941 | 810 | 809 |
| | 2038 | 27747 | 22504 | 3705 | 18996 | 18922 | 10266 | | | | | |
| 394: | 21856 | 24164 | 29620 | 1784 | 21478 | 1345 | 12589 | 8615 | 1433 | 29039 | 22405 | 6104 |
| | 4741 | 6153 | 27383 | 1053 | 20878 | 24111 | 24163 | 24908 | 27607 | 16730 | 3089 | 28969 |
| | 25762 | 7048 | 19683 | 17842 | 12038 | 12075 | 25442 | 24302 | 13983 | 14086 | 17606 | 3484 |
| | 2512 | 4483 | 16937 | 12730 | 10048 | 4151 | 19556 | 22226 | 18488 | 10543 | 6235 | 27285 |
| | 6911 | 8299 | 6366 | 1887 | 6922 | 27319 | 28070 | 29608 | 13672 | 3921 | 28033 | 28581 |
| | 9539 | 9634 | 9723 | 27695 | 22700 | 16442 | 2518 | 26084 | 24025 | 11174 | 19518 | 11894 |
| | 7949 | 10852 | 12776 | 12747 | 22813 | 17143 | 18331 | 18368 | 11320 | 12331 | 13177 | 16086 |
| | 9791 | 8880 | 8616 | 4945 | 9638 | 5090 | 2777 | 12555 | 17749 | 25468 | 17794 | 3652 |
| | 22640 | 30174 | 30197 | 25256 | 7125 | 27227 | 14842 | 27230 | 3888 | 7203 | 3886 | 15415 |
| | 14263 | 27253 | 15364 | 28633 | 9807 | 23176 | 10234 | 2048 | 8987 | 22752 | 9718 | 22980 |
| | 22117 | 29993 | 18370 | 23586 | 14855 | 7884 | 6597 | 16986 | 10722 | 8147 | 13098 | 13121 |
| | 7380 | 2799 | 1001 | 19996 | 12133 | 21072 | 12991 | 21289 | 18803 | 24860 | 24010 | 10401 |
| | 10720 | 25947 | 10751 | 13834 | 17532 | 26069 | 26348 | 26044 | 7529 | 27594 | 21975 | 3897 |
| | 23172 | 20321 | 14323 | 3959 | 2609 | 9324 | 16247 | 24744 | 5158 | 15265 | 11192 | 8508 |

(continued)

SEQ ID NO: homolog SEQ ID NOs

| | | | | | | | | | | | |
|---|---|---|---|---|---|---|---|---|---|---|---|
| 15916 | 16362 | 15481 | 3729 | 3937 | 21440 | 20281 | 9882 | 1462 | 15542 | 9677 | 12648 |
| 3068 | 1277 | 11302 | 25813 | 25637 | 3802 | 26281 | 19752 | 28449 | 12786 | 24765 | 3313 |
| 4156 | 4875 | 29932 | 5127 | 6118 | 4153 | 4314 | 28060 | 8850 | 4213 | 3438 | 743 |
| 14317 | 17660 | 22689 | 4054 | 21488 | 9845 | 21342 | 20379 | 18509 | 27074 | 22048 | 21000 |
| 20994 | 18250 | 3931 | 8242 | 8922 | 28444 | 19822 | 3736 | 22263 | 860 | 15264 | 9470 |
| 14355 | 10744 | 11675 | 10808 | 22906 | 22882 | 3900 | 24944 | 29376 | 1528 | 7511 | 1695 |
| 22725 | 25713 | 13583 | 17888 | 8639 | 6915 | 11854 | 30017 | 3397 | 20222 | 18169 | 19301 |
| 15990 | 5086 | 2885 | 25110 | 29616 | 19605 | 15328 | 18518 | 14627 | 5570 | 10207 | 6790 |
| 24693 | 10137 | 5630 | 14649 | 15009 | 4031 | 6698 | 10638 | 8699 | 15043 | 13864 | 24453 |
| 1563 | 30308 | 29999 | 3488 | 28199 | 2539 | 16425 | 2955 | 4729 | 26719 | 1069 | 29461 |
| 6996 | 13736 | 25297 | 2179 | 10887 | 15809 | 29704 | 13678 | 8895 | 13778 | 11964 | 29981 |
| 1002 | 15097 | 20701 | 10558 | 11233 | 6455 | 15140 | 1503 | 10400 | 11132 | 14827 | 20141 |
| 11907 | 15733 | 22989 | 20027 | 10977 | 11283 | 19545 | 22177 | 18150 | 2373 | 2209 | 8439 |
| 10464 | 25109 | 10334 | 21708 | 11349 | 2436 | 21212 | 10583 | 27908 | 6536 | 13711 | 14607 |
| 4576 | 11558 | 5429 | 15650 | 4544 | 8440 | 20939 | 5029 | 4617 | 11005 | 5276 | 16804 |
| 10160 | 18479 | 10658 | 3957 | 10669 | 1602 | 15351 | 15356 | 14307 | 11178 | 14220 | 13049 |
| 14179 | 13819 | 5120 | 6963 | 2056 | 29331 | 28281 | 5677 | 29397 | 1222 | 18628 | 20457 |
| 8219 | 16881 | 15738 | 27878 | 19773 | 5470 | 14225 | 2884 | 14492 | 991 | 6832 | 29600 |
| 20482 | 29119 | 22447 | 15424 | 26291 | 18187 | 25657 | 12787 | 22122 | 27541 | 26600 | 3644 |
| 29342 | 6756 | 23936 | 23634 | 7701 | 20694 | 3875 | 13655 | 14696 | 1280 | 15704 | 10417 |
| 2669 | 27195 | 20016 | 8701 | 3251 | 28762 | 25034 | 28538 | 27139 | 13907 | 28222 | 24150 |
| 4131 | 6086 | 24519 | 28696 | 26453 | 28128 | 10942 | 15495 | 3681 | 17081 | 15455 | 15461 |
| 3172 | 1886 | 16758 | 11352 | 12336 | 3104 | 25700 | 20443 | 13157 | 8447 | 15591 | 22137 |
| 2151 | 24933 | 18702 | 10897 | 2992 | 6919 | 25069 | 19125 | 22552 | 28569 | 22682 | 24511 |
| 2926 | 15979 | 17682 | 5070 | 21160 | 14796 | 25980 | 24091 | 9846 | 30052 | 5095 | 13480 |
| 7224 | 27440 | 17736 | 5193 | 4283 | 13738 | 3995 | 24736 | 3419 | 21059 | 21605 | 4289 |
| 13079 | 22170 | 4978 | 19113 | 11930 | 14083 | 17305 | 20235 | 12162 | 26556 | 23545 | 17543 |
| 25861 | 18044 | 5118 | 11160 | 17863 | 878 | 25646 | 10201 | 24633 | 20135 | 917 | 17944 |
| 24002 | 22082 | 16741 | 1179 | 8003 | 25902 | 24066 | 23752 | 9976 | 23589 | 2934 | 23839 |
| 12687 | 11870 | 15125 | 9071 | 8694 | 28541 | 22460 | 14157 | 15051 | 22490 | 803 | 18373 |
| 10178 | 19738 | 19670 | 13985 | 15797 | 23144 | 13685 | 26543 | 21114 | 3773 | 18216 | 2438 |
| 10832 | 14101 | 21968 | 17866 | 28867 | 14951 | 22902 | | | | | |
| 395: 25567 | 29156 | 19375 | 15945 | 28234 | 1930 | 12662 | 1162 | 10005 | 7044 | 1185 | 26036 |

(continued)

SEQ ID NO: homolog SEQ ID NOs

| | | | | | | | | | | | | |
|---|---|---|---|---|---|---|---|---|---|---|---|---|
| | 23538 | 23977 | 11290 | 7524 | 8204 | 23778 | 7260 | 1469 | | | | |
| 396: | 15550 | 21146 | 14612 | 27344 | 26910 | 19266 | 17014 | 28784 | 7362 | 28427 | 4076 | 27144 |
| | 18402 | 27484 | 20828 | 2950 | 28864 | 5408 | 27693 | 18626 | | | | |
| 397: | 15550 | 21146 | 14612 | 27344 | 26910 | 19266 | 17014 | 28784 | 7362 | 28427 | 4076 | 27144 |
| | 18402 | 27484 | 20828 | 2950 | 28864 | 5408 | 27693 | 18626 | | | | |
| 398: | 20783 | 13450 | 22393 | 29284 | 11496 | 20556 | 21762 | 1987 | 14172 | 23552 | 17413 | 10482 |
| | 13253 | 19913 | 24463 | 22718 | 9008 | 26604 | 22408 | 28778 | 28796 | 18916 | 24241 | 13171 |
| | 17226 | 17223 | 23385 | 19119 | 19094 | 15582 | 11188 | 11183 | 6598 | 19769 | 11262 | 18462 |
| | 17826 | 2299 | 29425 | 9949 | 6728 | 18710 | 5280 | 2348 | 9026 | 2221 | 12828 | 8683 |
| | 8763 | 15413 | 23424 | 15227 | 19187 | 16566 | 26593 | 19506 | 15363 | 25607 | 2781 | 29670 |
| | 4061 | 7971 | 4060 | 22255 | 28113 | 28115 | 13960 | 4634 | 11439 | 11437 | 18272 | 6803 |
| | 11813 | 24821 | 20526 | 7488 | 23499 | 10045 | 25456 | 13060 | 16886 | 13041 | 13604 | 13602 |
| | 5266 | 6561 | 14663 | 16418 | 28276 | 30159 | 3071 | 3069 | 15829 | 15834 | 29783 | 27545 |
| | 27547 | 6633 | 5877 | 5882 | 24447 | 7951 | 11377 | 11378 | 29701 | 23797 | 23824 | 14008 |
| | 5174 | 5890 | 5338 | 6954 | 24967 | 24970 | 3984 | 21553 | 8064 | 8082 | 20705 | 16785 |
| | 8060 | 11794 | 11793 | 18260 | 11235 | 18056 | 17235 | 3574 | 29965 | 14689 | 29008 | 22201 |
| | 26619 | 16254 | 27212 | 18690 | 29985 | 29983 | 26379 | 7323 | 28057 | 28076 | 22178 | 1476 |
| | 1499 | 21816 | 21817 | 23693 | 23697 | 7663 | 26999 | 16904 | 12076 | 22349 | 27631 | 27635 |
| | 3646 | 29216 | 3774 | 1037 | 21377 | 10361 | 1465 | 13657 | 29091 | 9530 | 10438 | 17215 |
| | 15985 | 7122 | 12656 | 12663 | 8482 | 23745 | 11301 | 25560 | 26336 | 26335 | 12233 | 26676 |
| | 26679 | 23718 | 18165 | 17256 | 2845 | 26709 | 2452 | 2468 | 3833 | 4992 | 18677 | 18676 |
| | 6821 | 8228 | 24527 | 4326 | 12763 | 29658 | 8270 | 27358 | 27361 | 19198 | 25232 | 13350 |
| | 12673 | 12604 | 19134 | 19131 | 6879 | 6876 | 23049 | 15891 | 9275 | 16475 | 13447 | 6872 |
| | 22206 | 5694 | 5697 | 2521 | 2519 | 2576 | 27448 | 14912 | 7686 | 26770 | 2483 | 20264 |
| | 21259 | 22789 | 22783 | 25455 | 1491 | 14351 | 24437 | 12355 | 25910 | 28440 | 28419 | 18499 |
| | 19880 | 25524 | 15791 | 15792 | 13807 | 19186 | 23554 | 25939 | 23639 | 23665 | 26002 | 19013 |
| | 1949 | 16345 | 7172 | 8561 | 15612 | 23888 | 21202 | 16774 | 3540 | 16890 | 8889 | 1654 |
| | 838 | 16797 | 28732 | 8443 | 27825 | 5867 | 8959 | 14408 | 28626 | 21725 | 13297 | 26749 |
| | 24604 | 16022 | 27101 | 25116 | 14737 | 6866 | 12276 | 30206 | 1906 | 6048 | 1644 | 15666 |
| | 16375 | 16377 | 27896 | 27906 | 7853 | 4269 | 18184 | 20542 | 16800 | 7163 | 3171 | 29556 |
| | 28213 | 28264 | 4626 | 14525 | 27907 | 4896 | 14415 | 15267 | 18015 | | | |
| 399: | 7481 | 7482 | 21594 | 21912 | 19603 | 11906 | 21562 | 10052 | 17209 | 16856 | 1018 | 12039 |
| | 26126 | 15002 | 25101 | 25991 | 28165 | 28882 | 26327 | 11923 | 27238 | 27247 | 17755 | 28301 |

EP 2 540 831 A2

(continued)

SEQ ID NO: homolog SEQ ID NOs

| | | | | | | | | | | | | |
|---|---|---|---|---|---|---|---|---|---|---|---|---|
| | 8815 | 7223 | 29812 | 13704 | 10475 | 14022 | 25767 | 20465 | 24769 | 19435 | 24674 | 18725 |
| | 15030 | 22301 | 15107 | 7444 | 10078 | 21253 | 19638 | 7979 | 16463 | 28526 | 2840 | 19485 |
| | 15440 | 24235 | 17154 | 1397 | 7592 | 15352 | 10017 | 12846 | 14558 | 14557 | 4745 | 16276 |
| | 28550 | 18302 | 15033 | 17843 | 18737 | 24891 | 4009 | 24663 | 4951 | 18544 | 29771 | 29792 |
| | 12620 | 10529 | 25363 | 10692 | 1011 | 25394 | 11253 | 11089 | 26523 | 20555 | 18801 | 4467 |
| | 21240 | 26753 | 26771 | 887 | 22346 | 18459 | 8640 | 13336 | 16731 | 3502 | 12856 | 3605 |
| | 4104 | 12132 | 15324 | 10083 | 17505 | 23224 | 7611 | 7613 | 24971 | 25092 | 25090 | 9606 |
| | 15210 | 3195 | 18062 | 16686 | 4022 | 16376 | 29189 | 27590 | 30283 | 29064 | 10369 | 8496 |
| | 21529 | 20851 | 17996 | 14524 | 1323 | 27821 | 27824 | 10140 | 6047 | 3057 | 29187 | 4569 |
| | 10143 | 30151 | 23021 | 5561 | 30120 | 6923 | 749 | 919 | 23563 | 28593 | 25604 | 846 |
| | 22632 | 1856 | 22657 | 24366 | 28212 | 22047 | 26475 | 22407 | 12593 | 831 | 22373 | 16081 |
| | 804 | 806 | 1630 | 832 | 17701 | 25214 | 11203 | 4409 | 4059 | 4063 | 4037 | 19350 |
| | 15297 | 16495 | 16414 | 24073 | 23796 | 16412 | 4570 | 25052 | 21704 | 28609 | 8654 | 28610 |
| | 16041 | 14339 | 7588 | 7605 | 2480 | 3849 | 7834 | 6898 | 29421 | 5786 | 1808 | 3189 |
| | 4782 | 10148 | 3144 | 7609 | 12395 | 9867 | 12925 | 2337 | 13594 | 28383 | 2908 | 1090 |
| | 4919 | 12369 | 13557 | 8145 | 15451 | 7430 | 16830 | 1553 | 12805 | | | |
| 400: | 7481 | 7482 | 21594 | 21912 | 19603 | 11906 | 21562 | 10052 | 17209 | 16856 | 1018 | 12039 |
| | 26126 | 15002 | 25101 | 25991 | 28165 | 28882 | 26327 | 11923 | 27238 | 27247 | 17755 | 28301 |
| | 8815 | 7223 | 29812 | 13704 | 10475 | 14022 | 25767 | 20465 | 24769 | 19435 | 24674 | 18725 |
| | 15030 | 22301 | 15107 | 7444 | 10078 | 21253 | 19638 | 7979 | 16463 | 28526 | 2840 | 19485 |
| | 15440 | 24235 | 17154 | 1397 | 7592 | 15352 | 10017 | 12846 | 14558 | 14557 | 4745 | 16276 |
| | 28550 | 18302 | 15033 | 17843 | 18737 | 24891 | 4009 | 24663 | 4951 | 18544 | 29771 | 29792 |
| | 12620 | 10529 | 25363 | 10692 | 1011 | 25394 | 11253 | 11089 | 26523 | 20555 | 18801 | 4467 |
| | 21240 | 26753 | 26771 | 887 | 22346 | 18459 | 8640 | 13336 | 16731 | 3502 | 12856 | 3605 |
| | 4104 | 12132 | 15324 | 10083 | 17505 | 23224 | 7611 | 7613 | 24971 | 25092 | 25090 | 9606 |
| | 15210 | 3195 | 18062 | 16686 | 4022 | 16376 | 29189 | 27590 | 30283 | 29064 | 10369 | 8496 |
| | 21529 | 20851 | 17996 | 14524 | 1323 | 27821 | 27824 | 10140 | 6047 | 3057 | 29187 | 4569 |
| | 10143 | 30151 | 23021 | 5561 | 30120 | 6923 | 749 | 919 | 23563 | 28593 | 25604 | 846 |
| | 22632 | 1856 | 22657 | 24366 | 28212 | 22047 | 26475 | 22407 | 12593 | 831 | 22373 | 16081 |
| | 804 | 806 | 1630 | 832 | 17701 | 25214 | 11203 | 4409 | 4059 | 4063 | 4037 | 19350 |
| | 15297 | 16495 | 16414 | 24073 | 23796 | 16412 | 4570 | 25052 | 21704 | 28609 | 8654 | 28610 |
| | 16041 | 14339 | 7588 | 7605 | 2480 | 3849 | 7834 | 6898 | 29421 | 5786 | 1808 | 3189 |
| | 4782 | 10148 | 3144 | 7609 | 12395 | 9867 | 12925 | 2337 | 13594 | 28383 | 2908 | 1090 |

(continued)

SEQ ID NO: homolog SEQ ID NOs

| | | | | | | | | | | | | |
|---|---|---|---|---|---|---|---|---|---|---|---|---|
| | 4919 | 12369 | 13557 | 8145 | 15451 | 7430 | 16830 | 1553 | 12805 | | | |
| 401: | 9600 | 17233 | 10457 | | | | | | | | | |
| 402: | 15827 | 19317 | 28794 | 3324 | 24498 | 25946 | 6458 | | | | | |
| 403: | 1755 | 8758 | 20447 | 30242 | 14946 | 15928 | 29995 | 8401 | 9344 | 10451 | 10062 | 4571 |
| | 20053 | 6770 | 14377 | 2112 | 10571 | 15211 | 10544 | 10562 | 16567 | 18109 | 10370 | 10367 |
| | 12190 | 25303 | 27426 | 26566 | 21755 | 4980 | 20104 | 30144 | 10357 | 21079 | 28169 | 24217 |
| | 23259 | 2865 | 8448 | 10468 | 16282 | 20406 | 18671 | 13037 | 9395 | 21191 | 19346 | 21036 |
| | 19825 | 5411 | 18566 | 14072 | 24141 | 25361 | 3355 | 11048 | 15566 | 12947 | 27746 | 19152 |
| | 28483 | 26226 | 13476 | 17665 | 21084 | 17743 | 2543 | 16503 | 10263 | 6261 | 15838 | 4224 |
| | 25212 | 7781 | 1812 | 1815 | 17816 | 17776 | 24893 | 12987 | 22185 | 29801 | | |
| 404: | 19675 | 17331 | 17871 | 15703 | 18124 | 13896 | 28376 | 3942 | 20802 | 17221 | 14950 | 14078 |
| | 6794 | 26900 | 29122 | 13528 | 8545 | 9695 | 906 | 911 | 15683 | 785 | 17525 | 24528 |
| | 22782 | 20466 | 5156 | 4212 | 10980 | 4394 | 914 | 18481 | 25078 | 3207 | 3413 | 3370 |
| | 7996 | 11258 | 17857 | 27249 | 27239 | 18791 | 11113 | 15220 | 4585 | 28303 | 8812 | 9646 |
| | 8972 | 7219 | 1758 | 4916 | 10176 | 10177 | 29813 | 26886 | 15036 | 15063 | 2498 | 9495 |
| | 10476 | 17623 | 8316 | 3898 | 20967 | 24565 | 22850 | 19641 | 4643 | 29721 | 27075 | 14036 |
| | 18431 | 18404 | 25765 | 16215 | 20467 | 16335 | 29415 | 24771 | 18129 | 16402 | 4831 | 14642 |
| | 15030 | 22300 | 22303 | 10305 | 21250 | 9604 | 7724 | 18285 | 17941 | 10097 | 28479 | 28524 |
| | 15421 | 15443 | 13986 | 24251 | 27185 | 15378 | 5300 | 12117 | 10015 | 22933 | 13818 | 21611 |
| | 26396 | 19997 | 2536 | 16303 | 1319 | 28174 | 16280 | 1153 | 28571 | 16138 | 18305 | 3690 |
| | 10822 | 17846 | 16027 | 17849 | 24657 | 11486 | 11469 | 18738 | 27834 | 15763 | 9445 | 18723 |
| | 3881 | 28975 | 3748 | 25738 | 9545 | 11539 | 18546 | 13618 | 8893 | 27228 | 12623 | 12929 |
| | 10714 | 15054 | 15154 | 8016 | 8120 | 8164 | 9619 | 5929 | 6614 | 4818 | 28391 | 27294 |
| | 1076 | 29612 | 10693 | 23304 | 11773 | 8797 | 15805 | 15497 | 2882 | 1013 | 22649 | 22189 |
| | 22330 | 20150 | 20907 | 11960 | 12066 | 14814 | 16287 | 16264 | 25392 | 11254 | 27860 | 8908 |
| | 11093 | 3384 | 6377 | 30178 | 19393 | 17913 | 13026 | 14346 | 19905 | 19975 | 26288 | 17253 |
| | 21670 | 16542 | 15812 | 13928 | 18367 | 15347 | 2377 | 13870 | 15898 | 26750 | 26754 | 26769 |
| | 29391 | 3817 | 2504 | 4677 | 1046 | 18463 | 7660 | 16538 | 7687 | 9084 | 16319 | 15658 |
| | 8607 | 2502 | 17492 | 15524 | 3503 | 23883 | 23703 | 8279 | 15006 | 1509 | 14546 | 23648 |
| | 1147 | 3544 | 5327 | 2549 | 8302 | 25387 | 22242 | 24417 | 1849 | 25619 | 3321 | 24292 |
| | 17000 | 21410 | 24969 | 29191 | 23677 | 21609 | 21612 | 27588 | 22898 | 3148 | 3094 | 18882 |
| | 25531 | 29053 | 4900 | 28763 | 7914 | 8498 | 29074 | 10147 | 6595 | 27684 | 24413 | 7107 |
| | 17997 | 27656 | 22691 | 1762 | 7238 | 1065 | 23074 | 3563 | 5597 | 11722 | 28431 | 7801 |

SEQ ID NO: homolog SEQ ID NOs

| | | | | | | | | | | | |
|---|---|---|---|---|---|---|---|---|---|---|---|
| 15649 | 7907 | 23561 | 24501 | 20609 | 22655 | 26099 | 24365 | 15027 | 29581 | 26459 | 22278 |
| 22046 | 13862 | 21392 | 21412 | 21415 | 20847 | 9367 | 27322 | 17216 | 7940 | 12447 | 1542 |
| 25160 | 28288 | 18472 | 26209 | 14087 | 18214 | 2408 | 21255 | 19247 | 7911 | 22973 | 24140 |
| 4899 | 9533 | 24223 | 1159 | 13833 | 6128 | 16155 | 8501 | 23702 | 23602 | 23828 | 23636 |
| 23632 | 23792 | 23857 | 23783 | 23814 | 23821 | 23749 | 23747 | 29888 | 16005 | 24411 | 22468 |
| 26589 | 24381 | 1272 | 3298 | 7202 | 28903 | 2975 | 6897 | 19271 | 19263 | 19269 | 7957 |
| 5945 | 8645 | 3306 | 10080 | 3205 | 3142 | 9507 | 30066 | 19546 | 6117 | 17103 | 7258 |
| 15989 | 3896 | 12924 | 8411 | 10772 | 9558 | 15754 | 1895 | 28386 | 23717 | 23135 | 2067 |
| 2911 | 4290 | 4915 | 4264 | 4288 | 12092 | 9443 | 6955 | 8144 | 15452 | 15069 | 23924 |
| 405: 9350 | 12493 | 14487 | 18668 | 18726 | 8331 | 12912 | 8539 | 27193 | 8397 | 13264 | 14797 |
| 21101 | 17062 | 20407 | 15417 | 28573 | 23583 | 16321 | 18906 | 22260 | 13605 | 21928 | 9963 |
| 8992 | 2050 | 14187 | 1380 | 3119 | 3452 | 28602 | 16297 | 5162 | 6689 | 18568 | 9135 |
| 6198 | 26072 | 1164 | 8540 | 1898 | 24942 | 29472 | 24560 | 14371 | 8153 | 7376 | 13091 |
| 30229 | 22603 | 21470 | 17213 | 16259 | 20009 | 13201 | 4829 | 3756 | 24072 | 29913 | 1293 |
| 14857 | 20294 | 4459 | 15060 | 20028 | 14727 | 7352 | 26768 | 28470 | 17485 | 28972 | 11068 |
| 7337 | 18783 | 1167 | 25027 | 21736 | 10801 | 13209 | 15987 | 11804 | 22229 | 3426 | 6581 |
| 11427 | 7284 | 13243 | 16858 | 13029 | 22296 | 23308 | 11118 | 26938 | 11767 | 24702 | 29297 |
| 8357 | 2819 | 5299 | 1329 | 27153 | 3518 | 3883 | 16645 | 21277 | 26734 | 2386 | 2125 |
| 2349 | 2262 | 2255 | 23811 | 21055 | 2936 | 17975 | 8902 | 14806 | 20438 | 16875 | 23423 |
| 1017 | 26935 | 25158 | 26906 | 16976 | 6007 | 20545 | 22448 | 18493 | 17416 | 28645 | 15368 |
| 25641 | 17631 | 19617 | 1582 | 20775 | 4665 | 4430 | 10035 | 8830 | 14258 | 1984 | 23574 |
| 775 | 14370 | 10685 | 10623 | 6733 | 11444 | 15334 | 19425 | 854 | 9419 | 19787 | 9503 |
| 29087 | 1000 | 8044 | 1035 | 13858 | 28999 | 1031 | 7774 | 20339 | 22389 | 16995 | 20012 |
| 17195 | 14617 | 21103 | 9070 | 1347 | 29991 | 12296 | 11323 | 20610 | 11556 | 17808 | 3521 |
| 5340 | 16689 | 18665 | 14203 | 6569 | 23862 | 10574 | 12208 | 15198 | 8151 | 7176 | 28665 |
| 28132 | 21576 | 28630 | 3831 | 27730 | 16199 | 15067 | 18315 | 13405 | 19304 | 7180 | 12766 |
| 17481 | 3084 | 3200 | 15548 | 16061 | 10219 | 7535 | 3973 | 4517 | 30287 | 29928 | 4157 |
| 30182 | 13820 | 9863 | 7633 | 1751 | 8489 | 24648 | 17107 | 26480 | 11324 | 6043 | 8696 |
| 16314 | 24877 | 19209 | 20194 | 23879 | 27520 | 8402 | 11162 | 2685 | 16331 | 815 | 26106 |
| 6147 | 7170 | 21745 | 5712 | 11038 | 17621 | 7478 | 6252 | 29166 | 16012 | 18081 | 16832 |
| 20695 | 3767 | 14970 | 30103 | 17072 | 12489 | 25691 | 19653 | 12621 | 12618 | 9758 | 29515 |
| 12616 | 28752 | 27643 | 21508 | 2721 | 8526 | 1799 | 20883 | 13652 | 1881 | 7827 | 16990 |
| 13169 | 4263 | 19109 | 8990 | 22270 | 10724 | 25507 | 25506 | 25447 | 25473 | 4511 | 13237 |

(continued)

SEQ ID NO: homolog SEQ ID NOs

| SEQ ID NO | | | | | | | | | | | | |
|---|---|---|---|---|---|---|---|---|---|---|---|---|
| | 12237 | 8247 | 29012 | 14628 | 6352 | 5243 | 13609 | 1095 | 4839 | 21730 | 13702 | 12313 |
| | 23245 | | | | | | | | | | | |
| 406: | 7952 | 7758 | 2121 | 4417 | 1732 | 19878 | 17889 | 24642 | 14721 | | | |
| 407: | 7838 | 6850 | 8388 | 14593 | 11246 | 3199 | 22149 | 29934 | 29338 | 21420 | 26822 | 8735 |
| | 12273 | 12966 | 18963 | 12922 | 4941 | 22605 | | | | | | |
| 408: | 24669 | 1502 | 5941 | 16943 | 28055 | 24504 | 20000 | 23056 | 11933 | 3851 | 2632 | 19121 |
| | 24158 | 7861 | 22901 | 24333 | 23073 | 5920 | 5221 | 3947 | 3713 | 27191 | 12793 | 18206 |
| | 23479 | 25635 | 23086 | 22620 | 28354 | 11322 | 2164 | | | | | |
| 409: | 12329 | 18631 | 6390 | 24914 | 3920 | 3892 | 4321 | 1217 | 9257 | 9256 | 26355 | 7555 |
| | 18640 | 750 | 28065 | 3857 | 19572 | 3672 | 15502 | 5026 | 12741 | 11031 | 17019 | 13004 |
| | 6579 | 6580 | 6652 | 3447 | 12099 | 1520 | 19963 | 9050 | 10016 | 12661 | 26802 | 12649 |
| | 27820 | 17269 | 12655 | 12664 | 22491 | 25899 | 21810 | 7552 | 13567 | 8159 | 6840 | 23331 |
| | 3669 | 25847 | 7467 | 23734 | 23732 | 23730 | 20543 | 2815 | 26075 | 7263 | 29352 | 25149 |
| | 27828 | 808 | 19884 | 6680 | 29811 | 7210 | 7246 | 11006 | 3217 | 1221 | 1214 | 6093 |
| | 6862 | 19833 | 19863 | 2342 | 23041 | 16993 | 27445 | 11691 | 24376 | 18290 | 741 | 16455 |
| | 16449 | 5509 | 16460 | 16923 | 16452 | 30091 | 30069 | 20347 | 21360 | 26976 | 17312 | 25750 |
| | 3304 | 3340 | 26219 | 8160 | 19802 | 9742 | 19770 | 19774 | 19772 | 9741 | 1878 | 23831 |
| | 23768 | | | | | | | | | | | |
| 410: | 27996 | 12880 | 19256 | 16644 | 3981 | 14654 | 1028 | 5657 | 3101 | 6630 | 1999 | 15110 |
| | 17972 | 24751 | 25544 | 1578 | 1574 | 3566 | | | | | | |
| 411: | 19448 | 26177 | 4242 | 22312 | 4095 | 4793 | 18729 | 15673 | 14335 | 14987 | 25338 | 15423 |
| | 9245 | 18357 | 13832 | 29961 | 16555 | 5104 | 6344 | 3595 | 16517 | 6032 | 23254 | 1615 |
| | 1616 | 22213 | 10500 | 23406 | 21091 | 10315 | 2724 | 4210 | 14446 | 14453 | 8111 | 11057 |
| | 18384 | 18410 | 1641 | 1645 | 9211 | 28432 | 22567 | 8502 | 11865 | 11866 | 6567 | 8399 |
| | 19280 | 24840 | 15296 | 1646 | 1649 | 1650 | 15471 | 17495 | 24590 | 7802 | 14003 | 16551 |
| | 2037 | 8313 | 23987 | 13047 | 8865 | | | | | | | |
| 412: | 10094 | 11657 | 15956 | 24211 | 20580 | 6613 | 9925 | 16582 | 24295 | 26427 | 25014 | 14332 |
| | 20436 | 16391 | 8198 | 29112 | 16055 | 5108 | 27447 | 24536 | 17809 | 1135 | 8038 | 27067 |
| | 4439 | 11611 | 22324 | 23323 | 14069 | | | | | | | |
| 413: | 22285 | 1061 | 3004 | 2963 | 21032 | 16178 | 8280 | 8312 | 995 | 29419 | 869 | 904 |
| | 974 | 29793 | 738 | 20089 | 1036 | 13202 | 17050 | 29648 | 24742 | 15272 | 29276 | 1027 |
| | 1055 | 1005 | 1094 | 5734 | 3843 | 5916 | 9055 | 11992 | 7394 | 18986 | 856 | 14178 |
| | 15133 | 6604 | 7982 | 27232 | 23069 | 6981 | 22286 | 22328 | 22331 | 22281 | 22283 | 7148 |

(continued)

SEQ ID NO: homolog SEQ ID NOs

| SEQ ID NO: | | | | | | | | | | | | |
|---|---|---|---|---|---|---|---|---|---|---|---|---|
| | 2627 | 20581 | 29336 | 4982 | 8792 | 7132 | 11103 | 9848 | 11303 | 16433 | 28245 | 23611 |
| | 20168 | 16717 | 2959 | 3010 | 3014 | 20424 | 14461 | 23381 | 10362 | 16531 | 2585 | 12347 |
| | 6943 | 19986 | 15770 | 6541 | 10601 | 20758 | 3511 | 13076 | 28181 | 16767 | 18363 | 9869 |
| | 25926 | 28066 | 20049 | 7442 | 9792 | 29949 | 25386 | 18191 | 7526 | 19610 | 18301 | 30251 |
| | 4282 | 22593 | 17798 | 2861 | 29597 | 14021 | 18028 | 13328 | 18771 | 7435 | 26697 | 22482 |
| | 27836 | 6116 | 29188 | 6113 | 28679 | 7575 | 5222 | 3432 | 22719 | 2810 | 21587 | 9559 |
| | 19124 | 3299 | 15039 | 7541 | 15464 | 27162 | 27178 | 27863 | 23067 | 25803 | 15209 | 20299 |
| | 7546 | 11869 | 11474 | 25744 | 1527 | 11396 | 13394 | 9284 | 29159 | 1068 | 15972 | 17471 |
| | 2206 | 9612 | 14473 | 23349 | 16954 | 26362 | 16795 | 11098 | 25742 | 6012 | 26922 | 1082 |
| | 1291 | 977 | 22568 | 29546 | 5109 | 26295 | 18309 | 20200 | 25172 | | | |
| 414: | 10782 | 1242 | 17179 | 12771 | 14060 | 22410 | 30281 | 7685 | 10804 | 19915 | 13221 | 28293 |
| | 16268 | 13852 | 14829 | 27960 | 23091 | 22480 | 18438 | 14591 | | | | |
| 415: | 27127 | 21128 | 27256 | 19231 | 4532 | 8433 | 2673 | 3106 | 16438 | 16307 | 19857 | 15663 |
| | 21746 | 21122 | 5080 | 23257 | 10478 | 8773 | 5720 | 29291 | 17270 | 1600 | 28310 | 1371 |
| 416: | 10595 | 4522 | 14874 | 27350 | 21200 | 9749 | 23024 | 29619 | 14287 | 28984 | 12016 | 29060 |
| 417: | 1516 | 21172 | 21168 | 25858 | 11937 | 27257 | 8001 | 22977 | 30220 | 7665 | 7790 | 5290 |
| | 27459 | 25772 | 20532 | 10992 | 16906 | 15283 | 25124 | 29489 | 3003 | 23861 | 15087 | 13189 |
| | 17938 | 16918 | 28319 | 5223 | 7696 | 29487 | 1668 | 13491 | 13767 | 14490 | 14489 | 17502 |
| | 14785 | 12813 | 21336 | 22204 | 3118 | 28415 | 22608 | 13812 | 23656 | 28046 | 23856 | 30313 |
| | 24446 | 20430 | 12709 | 25599 | 21687 | 10754 | 23960 | 23727 | 28899 | 22221 | 22351 | 24192 |
| | 6558 | 26987 | 19596 | 23434 | 2301 | 3319 | 1309 | 21346 | 15202 | 28779 | 29002 | 28099 |
| | 10518 | 24874 | 9338 | 14865 | 22529 | 3870 | 3796 | 4625 | 1854 | 19019 | 23376 | 14034 |
| | 26878 | 13025 | 29563 | 13838 | 19700 | 17948 | 12511 | 5437 | 11920 | 14273 | 12199 | 19845 |
| | 21654 | 3110 | 21944 | 24780 | 9761 | 6059 | 14947 | 16308 | 9649 | 17958 | 20974 | 22298 |
| | 10919 | 14844 | 23038 | 9027 | 28032 | 14048 | 28436 | 11124 | 4508 | 19867 | 8007 | 4245 |
| | 12106 | 15840 | 18091 | 12248 | 25310 | 9144 | 23733 | 29880 | 3771 | 4596 | 13282 | 27155 |
| | 27975 | 23755 | 11404 | 17545 | 6684 | 30122 | | | | | | |
| 418: | 21385 | 29298 | 29299 | 10715 | 1142 | 3589 | 28838 | 15694 | 21970 | 21509 | 18339 | 18275 |
| | 10348 | 26736 | 11312 | 22418 | 29526 | 26405 | 24294 | 23653 | 2246 | 24557 | 22744 | 22106 |
| | 16839 | 7137 | 22680 | 12130 | 13825 | 6546 | 27632 | 23630 | 6194 | 5436 | 26590 | 25785 |
| | 11449 | 28866 | 14159 | 3242 | 11935 | 28540 | 26565 | 9220 | 21270 | 21750 | 29142 | 4356 |
| | 13112 | 7791 | 27338 | 7797 | 15315 | 16490 | 25598 | 11641 | 8466 | 28388 | 28737 | 18484 |
| | 10682 | 1818 | 3557 | 9099 | 19141 | 28442 | 6627 | 19724 | 7496 | 11857 | 10790 | 2232 |

(continued)

SEQ ID NO: homolog SEQ ID NOs

|  |  |  |  |  |  |  |  |  |  |  |  |  |
|---|---|---|---|---|---|---|---|---|---|---|---|---|
|  | 10028 | 10180 |  | 4798 | 15933 | 28646 | 21340 | 16738 |  |  |  |  |
| 419: | 6625 | 7268 |  | 5702 | 7052 | 29148 | 10258 | 19858 | 21331 | 12028 | 5359 | 20059 | 3390 |
|  | 13271 | 4895 |  | 5891 | 10622 | 14200 | 24090 | 6754 | 15695 | 16578 | 21792 | 26438 | 1836 |
| 420: | 1297 | 4984 |  | 23087 | 13397 | 23270 | 18439 | 1925 | 16126 | 6519 | 29445 | 2060 | 30047 |
|  | 22766 | 29488 |  | 13782 | 7574 | 10014 | 24270 | 717 | 18217 | 29808 | 3615 | 25047 | 13843 |
|  | 1130 | 29423 |  | 13075 | 13357 | 15528 |  |  |  |  |  |  |  |
| 421: | 1297 | 4984 |  | 23087 | 13397 | 23270 | 18439 | 1925 | 16126 | 6519 | 29445 | 2060 | 30047 |
|  | 22766 | 29488 |  | 13782 | 7574 | 10014 | 24270 | 717 | 18217 | 29808 | 3615 | 25047 | 13843 |
|  | 1130 | 29423 |  | 13075 | 13357 | 15528 |  |  |  |  |  |  |  |
| 422: | 10202 | 10205 |  | 10208 | 845 | 9383 | 14922 | 27915 | 24053 | 24030 | 1675 | 5698 | 22526 |
|  | 22625 | 3178 |  | 5487 | 19354 | 19289 | 29499 | 14741 | 3930 | 29057 | 29027 | 29034 | 29026 |
|  | 29029 | 29033 |  | 12597 | 5581 | 6349 | 6347 | 12692 | 12689 | 5870 | 5829 | 5838 | 5800 |
|  | 19322 | 15669 |  | 10788 | 15764 | 8395 | 22457 | 12707 | 23820 | 28086 | 2205 | 23826 | 8647 |
|  | 2208 | 8600 |  | 8608 | 14812 | 12715 | 23830 | 8499 | 8425 | 12710 | 2185 | 8400 | 8392 |
|  | 27987 | 27982 |  | 27981 | 28012 | 27984 | 27977 | 29719 | 20507 | 8034 | 10627 | 22498 | 20309 |
|  | 15151 | 15331 |  | 19022 | 28024 | 18497 | 24123 | 18642 | 22069 | 6858 | 9999 | 4266 | 13347 |
|  | 15835 | 16521 |  | 22609 | 3960 | 8687 | 10432 | 8918 | 1608 | 26718 | 12588 | 27112 | 29955 |
|  | 13772 | 13902 |  | 13132 | 27485 | 7600 | 23030 | 21279 | 20941 | 8978 | 874 | 29680 | 760 |
|  | 26895 | 24630 |  | 29935 | 2651 | 17306 | 14138 | 7023 | 4445 | 8756 | 25374 | 29683 | 4405 |
|  | 5528 | 29687 |  | 20003 | 1947 | 28985 | 2826 | 24862 | 21531 | 14088 | 2059 | 22336 | 23342 |
|  | 19339 | 26490 |  | 29133 | 17510 | 16822 | 26119 | 2905 | 25802 | 28510 | 16843 | 29989 | 9388 |
|  | 10125 | 5348 |  | 10128 | 14329 | 8483 | 10126 | 10129 | 21399 | 2161 | 2451 | 23593 | 6057 |
|  | 18013 | 23568 |  | 1871 | 29105 | 10120 | 21152 | 20255 | 3699 | 6091 | 23143 | 20463 | 20403 |
|  | 30058 | 19726 |  | 22685 | 8329 | 29341 | 29788 | 7755 | 1830 | 16762 | 7568 | 26967 | 891 |
|  | 13779 | 10341 |  | 8605 | 14213 | 12064 | 5875 | 27489 | 10246 | 26934 | 2160 | 23846 | 26522 |
|  | 10209 | 24820 |  | 6505 | 5442 | 17170 | 24079 | 5821 | 21268 | 14098 | 25334 | 5254 | 1960 |
|  | 14413 | 10738 |  | 18137 | 3722 | 24730 | 24847 | 25330 | 9497 | 2471 | 6492 | 19424 | 28363 |
|  | 5259 | 10736 |  |  |  |  |  |  |  |  |  |  |  |
| 423: | 7181 | 24599 |  | 15499 | 24372 | 26492 | 17584 | 26574 | 22924 | 14238 | 18467 | 4947 | 15293 |
|  | 4973 | 22369 |  | 1873 | 7651 | 10624 | 2540 | 23809 | 6469 | 19939 | 19749 | 3847 | 20789 |
|  | 20198 | 7694 |  | 3641 | 20599 | 28267 | 16723 | 6798 | 6802 | 21254 | 13306 | 30231 | 6752 |
|  | 2968 | 23146 |  | 10812 | 6612 | 10087 | 21469 | 426 | 425 | 424 | 4256 | 10392 | 25986 |
|  | 29672 | 6552 |  | 3085 | 6628 | 28495 | 11473 | 8594 | 11562 |  |  |  |  |

120

(continued)

SEQ ID NO: homolog SEQ ID NOs

| 424: | 7181 | 24599 | 15499 | 24372 | 26492 | 17584 | 26574 | 22924 | 14238 | 18467 | 4947 | 15293 |
|---|---|---|---|---|---|---|---|---|---|---|---|---|
| | 4973 | 22369 | 1873 | 10306 | 7651 | 10624 | 423 | 9065 | 23716 | 912 | 19939 | 2540 |
| | 6469 | 23809 | 19749 | 3847 | 7694 | 28267 | 16723 | 6798 | 6802 | 21254 | 13306 | 30231 |
| | 6752 | 2968 | 23146 | 10087 | 21469 | 5165 | 29211 | 11473 | 8594 | 11562 | 4256 | |
| 425: | 7181 | 24599 | 15499 | 24372 | 26492 | 17584 | 26574 | 22924 | 14238 | 18467 | 4947 | 15293 |
| | 4973 | 22369 | 1873 | 10306 | 7651 | 10624 | 423 | 9065 | 23716 | 912 | 19939 | 2540 |
| | 6469 | 23809 | 19749 | 3847 | 7694 | 28267 | 16723 | 6798 | 6802 | 21254 | 13306 | 30231 |
| | 6752 | 2968 | 23146 | 10087 | 21469 | 5165 | 29211 | 11473 | 8594 | 11562 | 4256 | |
| 426: | 7181 | 24599 | 15499 | 24372 | 26492 | 17584 | 26574 | 22924 | 14238 | 18467 | 4947 | 15293 |
| | 4973 | 22369 | 1873 | 10306 | 7651 | 10624 | 423 | 9065 | 23716 | 912 | 19939 | 2540 |
| | 6469 | 23809 | 19749 | 3847 | 7694 | 28267 | 16723 | 6798 | 6802 | 21254 | 13306 | 30231 |
| | 6752 | 2968 | 23146 | 10087 | 21469 | 5165 | 29211 | 11473 | 8594 | 11562 | 4256 | |
| 427: | 6244 | 19181 | 8461 | 10033 | 10777 | 24690 | 27283 | 18693 | 9727 | 16902 | 24272 | 18513 |
| | 1131 | 25431 | 4543 | 26000 | 4732 | | | | | | | |
| 428: | 22000 | 21734 | 18132 | 18161 | 18156 | 18155 | 20166 | 1228 | 5998 | 5995 | 5967 | 5961 |
| | 5971 | 1612 | 1614 | 27654 | 1316 | 3276 | 3280 | 3286 | 24082 | 24104 | 21698 | 21732 |
| | 21768 | 1839 | 1112 | 15070 | 23223 | 12644 | 1315 | 1275 | 1333 | 11002 | 19238 | 1344 |
| | 7593 | 17341 | 27118 | 853 | 11978 | 29435 | 29163 | 19558 | 19583 | 19586 | 19591 | 19592 |
| | 16381 | 12560 | 24201 | 21669 | 24728 | 4084 | 4107 | 8131 | 22790 | 22380 | 18802 | 15628 |
| | 4989 | 14403 | 13133 | 13125 | 12314 | 13110 | 13131 | 12308 | 12285 | 12260 | 13108 | 13104 |
| | 12255 | 12311 | 12254 | 12376 | 13102 | 12221 | 12316 | 12257 | 12374 | 17737 | 24783 | 1956 |
| | 4613 | 4610 | 4615 | 5335 | 5337 | 5339 | 4609 | 5333 | 5334 | 8305 | 18168 | 1359 |
| | 27430 | 24797 | 29108 | 15071 | 17338 | 19650 | 15112 | 15108 | 21825 | 21083 | 18769 | 8231 |
| | 11776 | 12484 | 14127 | 25513 | 14211 | 22297 | 23445 | 28629 | 28625 | 4918 | 16692 | 18197 |
| | 11464 | 23204 | 23187 | 11609 | 28662 | 7217 | 3303 | 8103 | 5241 | 14894 | 15047 | 30039 |
| | 2428 | 3185 | 13558 | 7704 | 28879 | 5911 | 10698 | 29470 | 25393 | 23152 | 11029 | 28815 |
| | 28071 | 740 | 15697 | 14998 | 17547 | 23517 | 23519 | 23522 | 23525 | 21726 | 4562 | 11117 |
| | 1772 | 17423 | 29690 | 15159 | 19171 | 13708 | 4013 | 8836 | 23256 | 28207 | 12685 | 13250 |
| | 16125 | 17100 | 5721 | 22127 | 22130 | 23943 | 23956 | 12903 | 22247 | 27269 | 23242 | 18940 |
| | 15114 | 3651 | 3510 | 1026 | 25326 | 15064 | 4075 | 26122 | 21752 | 16132 | 17825 | 9665 |
| | 2580 | 7751 | 14169 | 13205 | 29267 | 29264 | 1449 | 29854 | 29857 | 25690 | 9036 | 24063 |
| | 2670 | 10578 | 6329 | 17242 | 21907 | 18328 | 4130 | 20353 | 13917 | 21967 | 6619 | 9006 |
| | 12841 | 2879 | 4740 | 28047 | 2798 | 27946 | 5490 | 5526 | 10131 | 29678 | 7715 | 19043 |

SEQ ID NO: homolog SEQ ID NOs

| | | | | | | | | | | | |
|---|---|---|---|---|---|---|---|---|---|---|---|
| 29646 | 26708 | 6672 | 5542 | 9089 | 18162 | 6264 | 4536 | 29795 | 20355 | 19759 | 8224 |
| 3223 | 15674 | 29951 | 1358 | 7787 | 10879 | 8426 | 27998 | 25704 | 23794 | 5025 | 13247 |
| 9804 | 24026 | 18332 | 1764 | 11970 | 18166 | 3161 | 21754 | 11659 | 20909 | 12029 | 10309 |
| 18034 | 9829 | 12174 | 7989 | 10256 | 21373 | 25412 | 4096 | 27463 | 2461 | 8661 | 5083 |
| 19173 | 25357 | 19138 | 24803 | 29533 | 10444 | 22033 | 11593 | 17210 | 20520 | 16734 | 2071 |
| 5261 | 14634 | 30213 | 22264 | 14241 | 5328 | 22254 | 29197 | 9044 | 28414 | 14139 | 6603 |
| 11135 | 27609 | 15385 | 24429 | 24088 | 9993 | 19095 | 6588 | 27664 | 25938 | 21524 | 5312 |
| 26992 | 25949 | 3914 | 4768 | 8762 | 11514 | 11543 | 8008 | 25088 | 5682 | 28787 | 6417 |
| 8360 | 11962 | 15091 | 27171 | 12358 | 12363 | 18078 | 18076 | 18073 | 18071 | 18012 | 12337 |
| 12330 | 18046 | 18041 | 12390 | 12365 | 18068 | 12333 | 18037 | 1254 | 1251 | 9650 | 8581 |
| 20691 | 22367 | 28094 | 2079 | 3246 | 3367 | 9366 | 27563 | 1605 | 11012 | 13080 | 14227 |
| 13128 | 17885 | 18365 | 9744 | 6002 | 1870 | 8532 | 3707 | 18774 | 25003 | 23115 | 16798 |
| 12926 | 12274 | 27192 | 10466 | 25553 | 14776 | 17125 | 22337 | 29682 | 16552 | 4789 | 10388 |
| 23201 | 22232 | 25270 | 18106 | 19671 | 20933 | 2341 | 23633 | 23638 | 2856 | 9537 | 25541 |
| 25780 | 27004 | 728 | 22319 | 27721 | 26021 | 24107 | 24109 | 20911 | 2077 | 20231 | 29591 |
| 6917 | 30184 | 18093 | 18596 | 3826 | 18441 | 18446 | 18443 | 1651 | 22469 | 6085 | 7832 |
| 11212 | 11789 | 15237 | 20728 | 9213 | 25918 | 8239 | 20171 | 20169 | 20145 | 6472 | 8123 |
| 9883 | 22611 | 894 | 29496 | 24313 | 19648 | 15003 | 18192 | 17674 | 17669 | 7537 | 3650 |
| 27355 | 27353 | 1556 | 1577 | 1573 | 27357 | 1552 | 28450 | 23415 | 18296 | 18291 | 18295 |
| 1613 | 1664 | 6437 | 26483 | 7835 | 6814 | 15571 | 26558 | 29763 | 20591 | 12847 | 5731 |
| 934 | 10349 | 27262 | 27428 | 583 | 14108 | 13516 | 13496 | 909 | 27337 | 10358 | 10404 |
| 10530 | 10458 | 10452 | 10527 | 10498 | 7462 | 28777 | 3420 | 22360 | 24221 | 1370 | 28580 |
| 17633 | 5016 | 11852 | 11850 | 2382 | 27425 | 1313 | 16317 | 25676 | 3065 | 30271 | 14744 |
| 17750 | 3752 | 9919 | 28921 | 28924 | 28927 | 28948 | 28951 | 25166 | 2204 | 12798 | 14871 |
| 3935 | 24817 | 5885 | 28682 | 20105 | 5012 | 19416 | 20712 | 21756 | 8591 | 11745 | 11505 |
| 14095 | 11536 | 13342 | 19662 | 948 | 11001 | 29485 | 29502 | 29505 | 29506 | 29509 | 29522 |
| 29525 | 29527 | 17570 | 4134 | 16728 | 3458 | 11752 | 19879 | 10952 | 10978 | 10954 | 23205 |
| 25890 | 24099 | 17973 | 30076 | 18251 | 2084 | 27398 | 25721 | 25388 | 10251 | 29447 | 27395 |
| 14542 | 21735 | 20952 | 10222 | 14538 | 21741 | 6941 | 29871 | 10661 | 10199 | 5796 | 22712 |
| 19291 | 7759 | 15301 | 27392 | 8075 | 23128 | 4481 | 1145 | 27702 | 4799 | 27318 | 27327 |
| 27325 | 27356 | 16228 | 7580 | 11010 | 27726 | 26408 | 10249 | 13035 | 12992 | 5637 | 25369 |
| 25332 | 25296 | 25370 | 27764 | 25329 | 25293 | 17136 | 17134 | 4975 | 25335 | 6645 | 5052 |
| 25367 | 25252 | 5019 | 25339 | 25290 | 25285 | 5053 | 5047 | 5009 | 5021 | 25282 | 25365 |

(continued)

SEQ ID NO: homolog SEQ ID NOs

| SEQ ID NO: | | | | | | | | | | | | |
|---|---|---|---|---|---|---|---|---|---|---|---|---|
| | 25325 | 5017 | 5051 | 14956 | 9510 | 12269 | 13001 | 13006 | 13005 | 12995 | 13003 | 10216 |
| | 10363 | 10351 | 10326 | 11275 | 11272 | 11268 | 10494 | 10492 | 10487 | 18883 | 18855 | 29529 |
| | 10323 | 10465 | 10461 | 18850 | 9191 | 29532 | 10433 | 18861 | 18848 | 18816 | 18811 | 30255 |
| | 10185 | 18744 | 9152 | 27993 | 11279 | 18813 | 18888 | 18913 | 5738 | 21738 | 20959 | 14537 |
| | 12325 | 11916 | 15823 | | | | | | | | | |
| 429: | 22000 | 21734 | 18132 | 18161 | 18156 | 18155 | 20166 | 1228 | 5998 | 5995 | 5967 | 5961 |
| | 5971 | 1612 | 1614 | 27654 | 1316 | 3276 | 3280 | 3286 | 24082 | 24104 | 21698 | 21732 |
| | 21768 | 1839 | 1112 | 15070 | 23223 | 12644 | 1315 | 1275 | 1333 | 11002 | 19238 | 1344 |
| | 7593 | 17341 | 27118 | 853 | 11978 | 29435 | 29163 | 19558 | 19583 | 19586 | 19591 | 19592 |
| | 16381 | 12560 | 24201 | 21669 | 24728 | 4084 | 4107 | 8131 | 22790 | 22380 | 18802 | 15628 |
| | 4989 | 14403 | 13133 | 13125 | 12314 | 13110 | 13131 | 12308 | 12285 | 12260 | 13108 | 13104 |
| | 12255 | 12311 | 12254 | 12376 | 13102 | 12221 | 12316 | 12257 | 12374 | 17737 | 24783 | 1956 |
| | 4613 | 4610 | 4615 | 5335 | 5337 | 5339 | 4609 | 5333 | 5334 | 8305 | 18168 | 1359 |
| | 27430 | 24797 | 29108 | 15071 | 17338 | 19650 | 15112 | 15108 | 21825 | 21083 | 18769 | 8231 |
| | 11776 | 12484 | 14127 | 25513 | 14211 | 22297 | 23445 | 28629 | 28625 | 4918 | 16692 | 18197 |
| | 11464 | 23204 | 23187 | 11609 | 28662 | 7217 | 3303 | 8103 | 5241 | 14894 | 15047 | 30039 |
| | 2428 | 3185 | 13558 | 7704 | 28879 | 5911 | 10698 | 29470 | 25393 | 23152 | 11029 | 28815 |
| | 28071 | 740 | 15697 | 14998 | 17547 | 23517 | 23519 | 23522 | 23525 | 21726 | 4562 | 11117 |
| | 1772 | 17423 | 29690 | 15159 | 19171 | 13708 | 4013 | 8836 | 23256 | 28207 | 12685 | 13250 |
| | 16125 | 17100 | 5721 | 22127 | 22130 | 23943 | 23956 | 12903 | 22247 | 27269 | 23242 | 18940 |
| | 15114 | 3651 | 3510 | 1026 | 25326 | 15064 | 4075 | 26122 | 21752 | 16132 | 17825 | 9665 |
| | 2580 | 7751 | 14169 | 13205 | 29267 | 29264 | 1449 | 29854 | 29857 | 25690 | 9036 | 24063 |
| | 2670 | 10578 | 6329 | 17242 | 21907 | 18328 | 4130 | 20353 | 13917 | 21967 | 6619 | 9006 |
| | 12841 | 2879 | 4740 | 28047 | 2798 | 27946 | 5490 | 5526 | 10131 | 29678 | 7715 | 19043 |
| | 29646 | 26708 | 6672 | 5542 | 9089 | 18162 | 6264 | 4536 | 29795 | 20355 | 19759 | 8224 |
| | 3223 | 15674 | 29951 | 1358 | 7787 | 10879 | 8426 | 27998 | 25704 | 23794 | 5025 | 13247 |
| | 9804 | 24026 | 18332 | 1764 | 11970 | 18166 | 3161 | 21754 | 11659 | 20909 | 12029 | 10309 |
| | 18034 | 9829 | 12174 | 7989 | 10256 | 21373 | 25412 | 4096 | 27463 | 2461 | 8661 | 5083 |
| | 19173 | 25357 | 19138 | 24803 | 29533 | 10444 | 22033 | 11593 | 17210 | 20520 | 16734 | 2071 |
| | 5261 | 14634 | 30213 | 22264 | 14241 | 5328 | 22254 | 29197 | 9044 | 28414 | 14139 | 6603 |
| | 11135 | 27609 | 15385 | 24429 | 24088 | 9993 | 19095 | 6588 | 27664 | 25938 | 21524 | 5312 |
| | 26992 | 25949 | 3914 | 4768 | 8762 | 11514 | 11543 | 8008 | 25088 | 5682 | 28787 | 6417 |
| | 8360 | 11962 | 15091 | 27171 | 12358 | 12363 | 18078 | 18076 | 18073 | 18071 | 18012 | 12337 |

(continued)

SEQ ID NO: homolog SEQ ID NOs

| | | | | | | | | | | | |
|---|---|---|---|---|---|---|---|---|---|---|---|
| | 12330 | 18046 | 18041 | 12390 | 12365 | 18068 | 12333 | 18037 | 1254 | 1251 | 9650 | 8581 |
| | 20691 | 22367 | 28094 | 2079 | 3246 | 3367 | 9366 | 27563 | 1605 | 11012 | 13080 | 14227 |
| | 13128 | 17885 | 18365 | 9744 | 6002 | 1870 | 8532 | 3707 | 18774 | 25003 | 23115 | 16798 |
| | 12926 | 12274 | 27192 | 10466 | 25553 | 14776 | 17125 | 22337 | 29682 | 16552 | 4789 | 10388 |
| | 23201 | 22232 | 25270 | 18106 | 19671 | 20933 | 2341 | 23633 | 23638 | 2856 | 9537 | 25541 |
| | 25780 | 27004 | 728 | 22319 | 27721 | 26021 | 24107 | 24109 | 20911 | 2077 | 20231 | 29591 |
| | 6917 | 30184 | 18093 | 18596 | 3826 | 18441 | 18446 | 18443 | 1651 | 22469 | 6085 | 7832 |
| | 11212 | 11789 | 15237 | 20728 | 9213 | 25918 | 8239 | 20171 | 20169 | 20145 | 6472 | 8123 |
| | 9883 | 22611 | 894 | 29496 | 24313 | 19648 | 15003 | 18192 | 17674 | 17669 | 7537 | 3650 |
| | 27355 | 27353 | 1556 | 1577 | 1573 | 27357 | 1552 | 28450 | 23415 | 18296 | 18291 | 18295 |
| | 1613 | 1664 | 6437 | 26483 | 7835 | 6814 | 15571 | 26558 | 29763 | 20591 | 12847 | 5731 |
| | 934 | 10349 | 27262 | 27428 | 583 | 14108 | 13516 | 13496 | 909 | 27337 | 10358 | 10404 |
| | 10530 | 10458 | 10452 | 10527 | 10498 | 7462 | 28777 | 3420 | 22360 | 24221 | 1370 | 28580 |
| | 17633 | 5016 | 11852 | 11850 | 2382 | 27425 | 1313 | 16317 | 25676 | 3065 | 30271 | 14744 |
| | 17750 | 3752 | 9919 | 28921 | 28924 | 28927 | 28948 | 28951 | 25166 | 2204 | 12798 | 14871 |
| | 3935 | 24817 | 5885 | 28682 | 20105 | 5012 | 19416 | 20712 | 21756 | 8591 | 11745 | 11505 |
| | 14095 | 11536 | 13342 | 19662 | 948 | 11001 | 29485 | 29502 | 29505 | 29506 | 29509 | 29522 |
| | 29525 | 29527 | 17570 | 4134 | 16728 | 3458 | 11752 | 19879 | 10952 | 10978 | 10954 | 23205 |
| | 25890 | 24099 | 17973 | 30076 | 18251 | 2084 | 27398 | 25721 | 25388 | 10251 | 29447 | 27395 |
| | 14542 | 21735 | 20952 | 10222 | 14538 | 21741 | 6941 | 29871 | 10661 | 10199 | 5796 | 22712 |
| | 19291 | 7759 | 15301 | 27392 | 8075 | 23128 | 4481 | 1145 | 27702 | 4799 | 27318 | 27327 |
| | 27325 | 27356 | 16228 | 7580 | 11010 | 27726 | 26408 | 10249 | 13035 | 12992 | 5637 | 25369 |
| | 25332 | 25296 | 25370 | 27764 | 25329 | 25293 | 17136 | 17134 | 4975 | 25335 | 6645 | 5052 |
| | 25367 | 25252 | 5019 | 25339 | 25290 | 25285 | 5053 | 5047 | 5009 | 5021 | 25282 | 25365 |
| | 25325 | 5017 | 5051 | 14956 | 9510 | 12269 | 13001 | 13006 | 13005 | 12995 | 13003 | 10216 |
| | 10363 | 10351 | 10326 | 11275 | 11272 | 11268 | 10494 | 10492 | 10487 | 18883 | 18855 | 29529 |
| | 10323 | 10465 | 10461 | 18850 | 9191 | 29532 | 10433 | 18861 | 18848 | 18816 | 18811 | 30255 |
| | 10185 | 18744 | 9152 | 27993 | 11279 | 18813 | 18888 | 18913 | 5738 | 21738 | 20959 | 14537 |
| | 12325 | 11916 | 15823 | | | | | | | | | |
| 430: | 22000 | 21734 | 18132 | 18161 | 18156 | 18155 | 20166 | 1228 | 5998 | 5995 | 5967 | 5961 |
| | 5971 | 1612 | 1614 | 27654 | 1316 | 3276 | 3280 | 3286 | 24082 | 24104 | 21698 | 21732 |
| | 21768 | 1839 | 1112 | 15070 | 23223 | 12644 | 1315 | 1275 | 1333 | 11002 | 19238 | 1344 |
| | 7593 | 17341 | 27118 | 853 | 11978 | 29435 | 29163 | 19558 | 19583 | 19586 | 19591 | 19592 |

(continued)

| SEQ ID NO: | homolog SEQ ID NOs | | | | | | | | | | |
|---|---|---|---|---|---|---|---|---|---|---|---|
| 16381 | 12560 | 24201 | 21669 | 24728 | 4084 | 4107 | 8131 | 22790 | 22380 | 18802 | 15628 |
| 4989 | 14403 | 13133 | 13125 | 12314 | 13110 | 13131 | 12308 | 12285 | 12260 | 13108 | 13104 |
| 12255 | 12311 | 12254 | 12376 | 13102 | 12221 | 12316 | 12257 | 12374 | 17737 | 24783 | 1956 |
| 4613 | 4610 | 4615 | 5335 | 5337 | 5339 | 4609 | 5333 | 5334 | 8305 | 18168 | 1359 |
| 27430 | 24797 | 29108 | 15071 | 17338 | 19650 | 15112 | 15108 | 21825 | 21083 | 18769 | 8231 |
| 11776 | 12484 | 14127 | 25513 | 14211 | 22297 | 23445 | 28629 | 28625 | 4918 | 16692 | 18197 |
| 11464 | 23204 | 23187 | 11609 | 28662 | 7217 | 3303 | 8103 | 5241 | 14894 | 15047 | 30039 |
| 2428 | 3185 | 13558 | 7704 | 28879 | 5911 | 10698 | 29470 | 25393 | 23152 | 11029 | 28815 |
| 28071 | 740 | 15697 | 14998 | 17547 | 23517 | 23519 | 23522 | 23525 | 21726 | 4562 | 11117 |
| 1772 | 17423 | 29690 | 15159 | 19171 | 13708 | 4013 | 8836 | 23256 | 28207 | 12685 | 13250 |
| 16125 | 17100 | 5721 | 22127 | 22130 | 23943 | 23956 | 12903 | 22247 | 27269 | 23242 | 18940 |
| 15114 | 3651 | 3510 | 1026 | 25326 | 15064 | 4075 | 26122 | 21752 | 16132 | 17825 | 9665 |
| 2580 | 7751 | 14169 | 13205 | 29267 | 29264 | 1449 | 29854 | 29857 | 25690 | 9036 | 24063 |
| 2670 | 10578 | 6329 | 17242 | 21907 | 18328 | 4130 | 20353 | 13917 | 21967 | 6619 | 9006 |
| 12841 | 2879 | 4740 | 28047 | 2798 | 27946 | 5490 | 5526 | 10131 | 29678 | 7715 | 19043 |
| 29646 | 26708 | 6672 | 5542 | 9089 | 18162 | 6264 | 4536 | 29795 | 20355 | 19759 | 8224 |
| 3223 | 15674 | 29951 | 1358 | 7787 | 10879 | 8426 | 27998 | 25704 | 23794 | 5025 | 13247 |
| 9804 | 24026 | 18332 | 1764 | 11970 | 18166 | 3161 | 21754 | 11659 | 20909 | 12029 | 10309 |
| 18034 | 9829 | 12174 | 7989 | 10256 | 21373 | 25412 | 4096 | 27463 | 2461 | 8661 | 5083 |
| 19173 | 25357 | 19138 | 24803 | 29533 | 10444 | 22033 | 11593 | 17210 | 20520 | 16734 | 2071 |
| 5261 | 14634 | 30213 | 22264 | 14241 | 5328 | 22254 | 29197 | 9044 | 28414 | 14139 | 6603 |
| 11135 | 27609 | 15385 | 24429 | 24088 | 9993 | 19095 | 6588 | 27664 | 25938 | 21524 | 5312 |
| 26992 | 25949 | 3914 | 4768 | 8762 | 11514 | 11543 | 8008 | 25088 | 5682 | 28787 | 6417 |
| 8360 | 11962 | 15091 | 27171 | 12358 | 12363 | 18078 | 18076 | 18073 | 18071 | 18012 | 12337 |
| 12330 | 18046 | 18041 | 12390 | 12365 | 18068 | 12333 | 18037 | 1254 | 1251 | 9650 | 8581 |
| 20691 | 22367 | 28094 | 2079 | 3246 | 3367 | 9366 | 27563 | 1605 | 11012 | 13080 | 14227 |
| 13128 | 17885 | 18365 | 9744 | 6002 | 1870 | 8532 | 3707 | 18774 | 25003 | 23115 | 16798 |
| 12926 | 12274 | 27192 | 10466 | 25553 | 14776 | 17125 | 22337 | 29682 | 16552 | 4789 | 10388 |
| 23201 | 22232 | 25270 | 18106 | 19671 | 20933 | 2341 | 23633 | 23638 | 2856 | 9537 | 25541 |
| 25780 | 27004 | 728 | 22319 | 27721 | 26021 | 24107 | 24109 | 20911 | 2077 | 20231 | 29591 |
| 6917 | 30184 | 18093 | 18596 | 3826 | 18441 | 18446 | 18443 | 1651 | 22469 | 6085 | 7832 |
| 11212 | 11789 | 15237 | 20728 | 9213 | 25918 | 8239 | 20171 | 20169 | 20145 | 6472 | 8123 |
| 9883 | 22611 | 894 | 29496 | 24313 | 19648 | 15003 | 18192 | 17674 | 17669 | 7537 | 3650 |

SEQ ID NO: homolog SEQ ID NOs

| | | | | | | | | | | | | |
|---|---|---|---|---|---|---|---|---|---|---|---|---|
| | 27355 | 27353 | 1556 | 1577 | 1573 | 27357 | 1552 | 28450 | 23415 | 18296 | 18291 | 18295 |
| | 1613 | 1664 | 6437 | 26483 | 7835 | 6814 | 15571 | 26558 | 29763 | 20591 | 12847 | 5731 |
| | 934 | 10349 | 27262 | 27428 | 583 | 14108 | 13516 | 13496 | 909 | 27337 | 10358 | 10404 |
| | 10530 | 10458 | 10452 | 10527 | 10498 | 7462 | 28777 | 3420 | 22360 | 24221 | 1370 | 28580 |
| | 17633 | 5016 | 11852 | 11850 | 2382 | 27425 | 1313 | 16317 | 25676 | 3065 | 30271 | 14744 |
| | 17750 | 3752 | 9919 | 28921 | 28924 | 28927 | 28948 | 28951 | 25166 | 2204 | 12798 | 14871 |
| | 3935 | 24817 | 5885 | 28682 | 20105 | 5012 | 19416 | 20712 | 21756 | 8591 | 11745 | 11505 |
| | 14095 | 11536 | 13342 | 19662 | 948 | 11001 | 29485 | 29502 | 29505 | 29506 | 29509 | 29522 |
| | 29525 | 29527 | 17570 | 4134 | 16728 | 3458 | 11752 | 19879 | 10952 | 10978 | 10954 | 23205 |
| | 25890 | 24099 | 17973 | 30076 | 18251 | 2084 | 27398 | 25721 | 25388 | 10251 | 29447 | 27395 |
| | 14542 | 21735 | 20952 | 10222 | 14538 | 21741 | 6941 | 29871 | 10661 | 10199 | 5796 | 22712 |
| | 19291 | 7759 | 15301 | 27392 | 8075 | 23128 | 4481 | 1145 | 27702 | 4799 | 27318 | 27327 |
| | 27325 | 27356 | 16228 | 7580 | 11010 | 27726 | 26408 | 10249 | 13035 | 12992 | 5637 | 25369 |
| | 25332 | 25296 | 25370 | 27764 | 25329 | 25293 | 17136 | 17134 | 4975 | 25335 | 6645 | 5052 |
| | 25367 | 25252 | 5019 | 25339 | 25290 | 25285 | 5053 | 5047 | 5009 | 5021 | 25282 | 25365 |
| | 25325 | 5017 | 5051 | 14956 | 9510 | 12269 | 13001 | 13006 | 13005 | 12995 | 13003 | 10216 |
| | 10363 | 10351 | 10326 | 11275 | 11272 | 11268 | 10494 | 10492 | 10487 | 18883 | 18855 | 29529 |
| | 10323 | 10465 | 10461 | 18850 | 9191 | 29532 | 10433 | 18861 | 18848 | 18816 | 18811 | 30255 |
| | 10185 | 18744 | 9152 | 27993 | 11279 | 18813 | 18888 | 18913 | 5738 | 21738 | 20959 | 14537 |
| | 12325 | 11916 | 15823 | | | | | | | | | |
| 431: | 20746 | 17015 | 30033 | 4309 | 18561 | 23059 | 12561 | 17517 | 19268 | 13283 | 21644 | 27626 |
| | 26950 | 2485 | 30082 | 24762 | 26244 | 19000 | 18997 | 30088 | 14388 | 7962 | 10644 | 4872 |
| | 26874 | 21493 | 4766 | 7626 | 5275 | 22573 | 23320 | 2558 | 24960 | 14009 | 19387 | 24916 |
| | 16708 | 27608 | 6692 | 13381 | 3182 | 27187 | 2808 | 2557 | 4821 | 14520 | 28337 | 6044 |
| | 25510 | 29634 | 27070 | 25852 | 8026 | 21994 | 23775 | 16486 | 5258 | 20707 | 10580 | 19860 |
| | 27522 | 1894 | 30212 | 13203 | 24892 | 7713 | 20613 | 23400 | 11309 | | | |
| 432: | 20746 | 17015 | 30033 | 4309 | 18561 | 23059 | 12561 | 17517 | 19268 | 13283 | 21644 | 27626 |
| | 26950 | 2485 | 30082 | 24762 | 26244 | 19000 | 18997 | 30088 | 14388 | 7962 | 10644 | 4872 |
| | 26874 | 21493 | 4766 | 7626 | 5275 | 22573 | 23320 | 2558 | 24960 | 14009 | 19387 | 24916 |
| | 16708 | 27608 | 6692 | 13381 | 3182 | 27187 | 2808 | 2557 | 4821 | 14520 | 28337 | 6044 |
| | 25510 | 29634 | 27070 | 25852 | 8026 | 21994 | 23775 | 16486 | 5258 | 20707 | 10580 | 19860 |
| | 27522 | 1894 | 30212 | 13203 | 24892 | 7713 | 20613 | 23400 | 11309 | | | |
| 433: | 20746 | 17015 | 30033 | 4309 | 18561 | 23059 | 12561 | 17517 | 19268 | 13283 | 21644 | 27626 |

SEQ ID NO: homolog SEQ ID NOs

| | | | | | | | | | | | | |
|---|---|---|---|---|---|---|---|---|---|---|---|---|
| | 26950 | 2485 | 30082 | 24762 | 26244 | 19000 | 18997 | 30088 | 14388 | 7962 | 10644 | 4872 |
| | 26874 | 21493 | 4766 | 7626 | 5275 | 22573 | 23320 | 2558 | 24960 | 14009 | 19387 | 24916 |
| | 16708 | 27608 | 6692 | 13381 | 3182 | 27187 | 2808 | 2557 | 4821 | 14520 | 28337 | 6044 |
| | 25510 | 29634 | 27070 | 25852 | 8026 | 21994 | 23775 | 16486 | 5258 | 20707 | 10580 | 19860 |
| | 27522 | 1894 | 30212 | 13203 | 24892 | 7713 | 20613 | 23400 | 11309 | | | |
| 434: | 20746 | 17015 | 30033 | 4309 | 18561 | 23059 | 12561 | 17517 | 19268 | 13283 | 21644 | 27626 |
| | 26950 | 2485 | 30082 | 24762 | 26244 | 19000 | 18997 | 30088 | 14388 | 7962 | 10644 | 4872 |
| | 26874 | 21493 | 4766 | 7626 | 5275 | 22573 | 23320 | 2558 | 24960 | 14009 | 19387 | 24916 |
| | 16708 | 27608 | 6692 | 13381 | 3182 | 27187 | 2808 | 2557 | 4821 | 14520 | 28337 | 6044 |
| | 25510 | 29634 | 27070 | 25852 | 8026 | 21994 | 23775 | 16486 | 5258 | 20707 | 10580 | 19860 |
| | 27522 | 1894 | 30212 | 13203 | 24892 | 7713 | 20613 | 23400 | 11309 | | | |
| 435: | 3320 | 29292 | 10456 | 8974 | 20829 | 3120 | 23408 | 15895 | 1713 | 16348 | 28935 | 9056 |
| | 12899 | 26160 | 4229 | 3965 | 22857 | 27122 | 16211 | 11315 | 22241 | 29909 | 15344 | 24040 |
| | 25056 | 7632 | 29316 | 2786 | 24152 | 20769 | 15412 | 9444 | 2297 | 2115 | 19941 | 3955 |
| 436: | 15820 | 10706 | 23100 | 14963 | 18090 | 27714 | 7934 | 2958 | 17916 | 15037 | 28572 | 2470 |
| | 8807 | 27412 | 6146 | 10214 | 9520 | 26510 | 25234 | 22444 | 14599 | 20568 | 1089 | 15980 |
| | 16484 | 27665 | 28627 | 27446 | 23791 | 8275 | 27653 | 29555 | 3711 | 28025 | 12810 | 16828 |
| | 1980 | 29610 | 19441 | 2119 | 6018 | 5517 | | | | | | |
| 437: | 21127 | 23891 | 24018 | 22087 | 11917 | 23145 | 22312 | 4095 | 27151 | 28346 | 28343 | 26857 |
| | 14335 | 25338 | 15423 | 9245 | 2457 | 16555 | 22213 | 10500 | 12258 | 10315 | 21190 | 14453 |
| | 4210 | 14446 | 8111 | 11057 | 9211 | 16611 | 10833 | 3593 | 6567 | 11866 | 11865 | 15600 |
| | 24840 | 29855 | 28498 | 1649 | 20109 | 14988 | 3755 | 2611 | 13600 | 24016 | 13886 | 12195 |
| | 15174 | 29149 | 3323 | 4208 | 21291 | 21350 | 15536 | 6369 | 16551 | 2037 | 7308 | 14983 |
| | 23987 | 8865 | 25790 | | | | | | | | | |
| 438: | 9383 | 7030 | 29458 | 27519 | 26610 | 12956 | 16802 | 18947 | 1990 | 4837 | 18641 | 25096 |
| | 7356 | 23459 | 1052 | 939 | 3668 | 16399 | 16400 | 29072 | 24735 | 21727 | 7327 | 18077 |
| | 25414 | 4207 | 22227 | 3946 | 12613 | 21317 | 14442 | | | | | |
| 439: | 9147 | 21706 | 15578 | 14421 | 4343 | 26238 | 15690 | 13454 | 20022 | 27804 | 15759 | 24234 |
| | 28642 | 1010 | 24050 | 20670 | 19685 | 17911 | 3364 | 5295 | 16135 | 20262 | 11827 | 28967 |
| | 12384 | 3091 | 8135 | 29132 | 11890 | 18624 | 1201 | 1203 | 2429 | 3164 | 13234 | 21209 |
| | 10071 | 12328 | 9093 | 14374 | 12431 | | | | | | | |
| 440: | 6828 | 17035 | 17420 | 17040 | 9383 | 7030 | 29458 | 27519 | 26610 | 26494 | 22218 | 14711 |
| | 4837 | 18641 | 24382 | 26641 | 26437 | 7356 | 28209 | 1052 | 939 | 11795 | 15775 | 12955 |

(continued)

SEQ ID NO: homolog SEQ ID NOs

| SEQ ID NO | | | | | | | | | | | | |
|---|---|---|---|---|---|---|---|---|---|---|---|---|
| | 22357 | 30218 | 15257 | 7106 | 11200 | 5994 | 14732 | 25693 | 15145 | 24639 | 29785 | 7650 |
| | 8444 | 7327 | | | | | | | | | | |
| 441: | 11875 | 12917 | 13167 | 4305 | 28825 | 10632 | 16073 | 26989 | 20848 | 16068 | 27493 | 21295 |
| | 28584 | 16931 | 18057 | 7549 | 13846 | 10891 | 17109 | 27290 | 11325 | 4983 | 14960 | 26965 |
| | 14626 | 14603 | 26788 | 9241 | 1791 | 25516 | 11336 | 8852 | 3990 | 21367 | 1955 | 3569 |
| | 11967 | 10575 | 15745 | 20130 | 25177 | 22959 | 28349 | 12126 | 5514 | 28907 | 8916 | 15190 |
| | 28362 | 17879 | 25712 | 22734 | 25356 | 9094 | 16423 | | | | | |
| 442: | 14425 | 6734 | 11368 | 6577 | 26767 | 6319 | 10997 | 28469 | 820 | 4101 | 12699 | |
| 443: | 29266 | 9439 | 10912 | 4674 | 798 | 20641 | 20178 | 7975 | 8386 | 1178 | 6200 | 15126 |
| | 12608 | 13694 | 19164 | 2584 | 20057 | 787 | 26781 | 9148 | 25086 | 19197 | 9433 | 5707 |
| | 2306 | 7692 | 24407 | 10106 | 11238 | 10765 | 23647 | 12988 | 21319 | 27957 | 28500 | 9657 |
| | 25039 | 11504 | 2897 | 4800 | 11293 | 20350 | 3710 | 10537 | 8750 | 23173 | 10556 | 1759 |
| | 11837 | 5538 | 14861 | 2285 | 4161 | 3176 | 6994 | 14409 | 1152 | 10272 | 6078 | 16479 |
| | 28121 | 18672 | 27543 | 9580 | 28906 | 14585 | 24181 | 29640 | 10018 | 11921 | 24470 | 2618 |
| | 17713 | 28374 | 14636 | 5039 | 20498 | 9797 | 15996 | 2101 | 19606 | 28476 | 27159 | 8816 |
| | 3871 | 21005 | 3978 | 12504 | 10411 | 1407 | 20181 | 12495 | 4836 | 28843 | 29607 | 24356 |
| | 16704 | 12434 | 6927 | 17529 | 28532 | 3476 | 15053 | 9534 | 15124 | | | |
| 444: | 26101 | 17630 | 8051 | 27207 | 23760 | 13312 | 19404 | 5944 | 17218 | 8882 | 8950 | 10469 |
| | 26224 | 29908 | 9258 | 4023 | 28982 | 446 | 6281 | 22591 | 10865 | 12553 | 7783 | 24950 |
| | 1983 | 6178 | 28974 | 22676 | 15255 | | | | | | | |
| 445: | 26684 | 4222 | 22104 | 18340 | 1501 | 14665 | 18337 | 3714 | 5560 | 24522 | 3666 | 30023 |
| | 30026 | 5310 | 24253 | 19551 | 19547 | 13116 | 28708 | 10503 | 10988 | 10878 | 3288 | 15094 |
| | 8465 | 24525 | 20058 | 12054 | 22659 | 24341 | 24369 | 14015 | 24524 | 16017 | 11893 | 4731 |
| | 3660 | 3680 | 25444 | 20772 | 6376 | 6383 | 6386 | 5559 | 20949 | 5564 | 5556 | 5551 |
| | 2617 | 8668 | 14935 | 15374 | 6380 | 20947 | 2715 | 8665 | 2684 | 2711 | 2691 | 15049 |
| | 16861 | 22925 | 18128 | 14831 | 10984 | 8376 | 9464 | 22470 | 19924 | 18225 | 7800 | 29948 |
| | 24117 | 24115 | 17711 | 5134 | 23569 | 960 | 16421 | 1716 | 9215 | 17006 | 784 | 901 |
| | 9952 | 2644 | 2648 | 2654 | 2655 | 2687 | 2657 | 2688 | 2683 | 11024 | 2712 | 11052 |
| | 7348 | 5431 | 14137 | 14162 | 14134 | 14232 | 14228 | 14205 | 14173 | 14109 | 14242 | 14682 |
| | 14647 | 14757 | 14613 | 14239 | 14236 | 14170 | 14201 | 14716 | 14685 | 14679 | 13293 | 13290 |
| | 14077 | 14028 | 13251 | 5812 | 29998 | 12235 | 11259 | 9364 | | | | |
| 446: | 22676 | 15255 | 444 | 12014 | 13312 | 23760 | 17218 | 8882 | 8950 | 10469 | 26224 | 29908 |
| | 9258 | 4023 | 28974 | 28982 | | | | | | | | |

(continued)

SEQ ID NO: homolog SEQ ID NOs

| | | | | | | | | | | | | |
|---|---|---|---|---|---|---|---|---|---|---|---|---|
| 447: | 10235 | 16184 | 7924 | 3156 | 11974 | 29173 | 17875 | 18808 | 18264 | 6980 | 18697 | 10152 |
| | 9420 | 998 | 5864 | 3997 | 6764 | 14862 | 16404 | 20184 | 20115 | 9437 | 7901 | 6987 |
| | 6938 | 28137 | 27034 | 27032 | 27060 | 7175 | 12893 | 16394 | 5357 | 1504 | 15983 | 8453 |
| | 6524 | 13808 | 13973 | 13967 | 20195 | 2096 | 5651 | 16786 | 16849 | 1438 | 11477 | 30035 |
| | 10204 | 15773 | 13378 | 2903 | 25120 | 27691 | 14218 | 29183 | 30254 | 26221 | 17989 | 26358 |
| | 12093 | 17063 | 13687 | 2621 | 10704 | 21131 | 28073 | 2997 | 6986 | 15777 | 1256 | 20837 |
| | 22892 | 26626 | 7786 | 14637 | 23474 | 16478 | 22385 | 16448 | 16443 | 23551 | 21765 | 27690 |
| | 13216 | 11823 | 7041 | 18520 | 26251 | 12792 | 15675 | 17918 | 25799 | 12870 | 11938 | 21260 |
| | 26762 | 11939 | 11912 | 4857 | 24139 | 12872 | 25730 | 17932 | 1731 | 20863 | 3255 | 16684 |
| | 20204 | 17761 | 8294 | 13871 | 12860 | 10123 | 2130 | 5038 | 27527 | 25907 | 10056 | 28095 |
| | 17999 | 22475 | 27388 | 10423 | 15425 | 15554 | 8896 | 8878 | 8894 | 28064 | 21733 | 27573 |
| | 16697 | 17950 | 15127 | 24705 | 9556 | 18988 | 13691 | 28513 | 6892 | 7456 | 5765 | 26087 |
| | 22715 | 22723 | 20996 | 22720 | 27437 | 20937 | 19233 | 25490 | 22687 | 10013 | 27925 | 24812 |
| | 10538 | 11021 | 11023 | 13667 | 1920 | 17828 | 25779 | 17055 | 14151 | 4639 | 2335 | 2345 |
| | 5799 | 13095 | 4715 | 7563 | 24232 | 23271 | 22537 | 25483 | 8704 | 12517 | 3349 | 13599 |
| | 29346 | 29363 | 20920 | 14305 | 14308 | 11284 | 8903 | 10415 | 21523 | 5092 | 7465 | 7495 |
| | 12757 | 16234 | 25403 | 27092 | 759 | 23085 | 7679 | 26326 | 27360 | 7366 | 3227 | 9077 |
| | 21555 | 22108 | 21859 | 28862 | 28863 | 14177 | 6498 | 4438 | 23281 | | | |
| 448: | 26197 | 835 | 18433 | 25305 | 14851 | 4464 | 15106 | 25176 | 10633 | 15206 | 12945 | 2515 |
| | 10342 | 30081 | 12187 | 11495 | 12885 | 9837 | 6760 | 17310 | 9563 | 25205 | 4299 | 11656 |
| | 1940 | 2962 | 1523 | 2867 | 2888 | 5848 | 27190 | 13261 | 17523 | 5116 | 29186 | 15888 |
| | 7298 | 18374 | 29037 | 8585 | 7776 | 1727 | 29893 | 19823 | 28034 | 16573 | 2117 | 4537 |
| | 27905 | 19766 | 22668 | 27250 | 22421 | 17472 | 9673 | 1832 | 15716 | 8721 | 22721 | 6300 |
| | 8368 | 23142 | 1876 | 15261 | 20998 | 16473 | 10551 | 15816 | 14691 | 5229 | 1738 | 6066 |
| | 24196 | 9345 | 20475 | 4702 | 30060 | 18767 | 11204 | 17518 | 23707 | 25073 | 28898 | 766 |
| | 28814 | 4690 | 14328 | 9371 | 25054 | | | | | | | |
| 449: | 19005 | 18978 | 19528 | 2701 | 22686 | 21409 | 7669 | 3135 | 9543 | 23060 | 7624 | 10662 |
| | 30278 | 9072 | 5251 | 7271 | 24570 | 28802 | 3402 | 29236 | 20140 | 12400 | 13993 | 5594 |
| | 23871 | 18307 | 27104 | 27106 | 26168 | 6003 | 22164 | 540 | 26361 | 18768 | 17282 | 4160 |
| | 20118 | 16870 | 1978 | 8624 | 29692 | 13677 | 28473 | 9078 | 8612 | 26080 | 541 | 21194 |
| 450: | 7251 | 6668 | 21839 | 10875 | 19865 | 16396 | 9687 | 9129 | 27774 | 23390 | 12806 | 10350 |
| | 10316 | 25362 | 1273 | 2444 | 2467 | 27222 | 25236 | 20814 | 27692 | 27687 | 8169 | 8901 |
| | 3907 | 20182 | 3911 | 15642 | 9460 | 5986 | 25184 | 27495 | 23169 | 23685 | 19111 | 5184 |

SEQ ID NO: homolog SEQ ID NOs

| | | | | | | | | | | | |
|---|---|---|---|---|---|---|---|---|---|---|---|
| 14965 | 16778 | 16781 | 12791 | 6745 | 26536 | 7561 | 28366 | 25778 | 27761 | 1079 | 15302 |
| 25608 | 18099 | 28587 | 10318 | 10327 | 10320 | 13044 | 3400 | 18770 | 13322 | 15810 | 18772 |
| 13298 | 18764 | 13325 | 13301 | 13172 | 17389 | 16699 | 16677 | 20425 | 23309 | 20211 | 22155 |
| 22004 | 12122 | 15005 | 7926 | 23992 | 8249 | 9621 | 24384 | 19046 | 9959 | 7938 | 25922 |
| 22274 | 14933 | 18706 | 22694 | 3623 | 17891 | 11888 | 11891 | 10311 | 1096 | 11698 | 1715 |
| 4129 | 11796 | 29454 | 7779 | 5554 | 10435 | 18434 | 18455 | 26055 | 6979 | 21739 | 8859 |
| 4714 | 1007 | 18038 | 15698 | 15702 | 15734 | 12711 | 22153 | 19687 | 29577 | 22633 | 12974 |
| 13014 | 834 | 9968 | 8920 | 12389 | 21297 | 23915 | 8068 | 7315 | 25800 | 10412 | 11149 |
| 10961 | 2469 | 7578 | 8883 | 18475 | 23476 | 3811 | 18396 | 21686 | 13750 | 29344 | 12609 |
| 22876 | 21383 | 24100 | 28230 | 27082 | 30124 | 25708 | 25203 | 11828 | 4856 | 5616 | 29295 |
| 16548 | 8364 | 17078 | 8601 | 6730 | 5264 | 21024 | 22320 | 28122 | 11524 | 24542 | 24541 |
| 7635 | 7672 | 7477 | 12475 | 13010 | 26421 | 6895 | 18362 | 10809 | 10814 | 10811 | 10816 |
| 13074 | 17345 | 28998 | 29001 | 29005 | 23553 | 14481 | 8350 | 2744 | 8141 | 8322 | 28068 |
| 7872 | 9793 | 26852 | 28865 | 20046 | 15546 | 20546 | 25292 | 22341 | 21949 | 26804 | 8281 |
| 17766 | 30259 | 29722 | 3424 | 19097 | 20056 | 2726 | 3132 | 8117 | 16867 | 24098 | 30307 |
| 6884 | 19041 | 20534 | 20538 | 20595 | 11343 | 13403 | 14709 | 3505 | 15920 | 3477 | 29090 |
| 10606 | 13881 | 27255 | 4457 | 6993 | 4628 | 24925 | 1814 | 4624 | 12590 | 24729 | 11779 |
| 2844 | 24731 | 8217 | 8213 | 8215 | 3245 | 29115 | 21221 | 29111 | 28588 | 8241 | 27282 |
| 10172 | 23887 | 28618 | 28585 | 23531 | 28615 | 1684 | 23533 | 28612 | 16928 | 13914 | 13179 |
| 11918 | 15924 | 1671 | 3243 | 16006 | 13181 | 13182 | 1670 | 4524 | 11409 | 15751 | 14841 |
| 28723 | 17076 | 7739 | 7741 | 26993 | 28726 | 5496 | 12932 | 19214 | 12935 | 14839 | 8214 |
| 8210 | 2388 | 2239 | 28822 | 5172 | 7469 | 16556 | 29903 | 24153 | 1557 | 27897 | 27900 |
| 21862 | 6572 | 12720 | 26962 | 18349 | 17520 | 12124 | 1681 | 1686 | 1708 | 16096 | 27471 |
| 13063 | 10484 | 28241 | 13124 | 6831 | 23238 | 13123 | 13130 | 28142 | 14638 | 7293 | 23557 |
| 17395 | 1817 | 11826 | 17390 | 3198 | 9589 | 8940 | 15260 | 4883 | 972 | 4676 | 30243 |
| 3039 | 22664 | 27717 | 9872 | 20977 | 18324 | 9816 | 30237 | 30100 | 28605 | 24575 | 22842 |
| 29265 | 10445 | 2472 | 29247 | 22193 | 1039 | 8937 | 23930 | 3273 | 30061 | 26524 | 23466 |
| 21577 | 29497 | 25146 | 8845 | 14020 | 24275 | 26715 | 17810 | 26572 | 26577 | 30045 | 11526 |
| 23842 | 14724 | 19112 | 16469 | 7490 | 29918 | 20560 | 13452 | 14106 | 6846 | 6410 | 30175 |
| 13984 | 27724 | 17815 | 3372 | 24937 | 4497 | 6296 | 27742 | 19388 | 26474 | 21380 | 9862 |
| 1545 | 24387 | 10890 | 3417 | 29327 | 11096 | 21899 | 17325 | 11680 | 10217 | 18579 | 6704 |
| 10134 | 27669 | 1513 | 20020 | 29804 | 27208 | 29946 | 1453 | 19889 | 26890 | 11359 | 9061 |
| 29601 | 27288 | 27160 | 26257 | 10462 | 26061 | 23241 | 23235 | 23230 | 26060 | 23208 | 23240 |

(continued)

SEQ ID NO: homolog SEQ ID NOs

| | | | | | | | | | | | | |
|---|---|---|---|---|---|---|---|---|---|---|---|---|
| | 23228 | 7279 | 29217 | 23335 | 7542 | 2290 | 20982 | 6297 | 12952 | 9740 | 15584 | 15587 |
| | 19126 | 12506 | 10011 | 21011 | 2748 | 26568 | 1240 | 9248 | 5063 | 5065 | 22621 | 12186 |
| | 23330 | 17534 | 21269 | 17992 | 19237 | 17011 | 7689 | 5075 | 18207 | 18211 | 13302 | 29862 |
| | 5430 | 13848 | 2186 | 25628 | 6807 | 6782 | 6780 | 15416 | 8148 | 3037 | 1484 | 25768 |
| | 8544 | 1271 | 23475 | 23472 | 18377 | 29605 | 2090 | 20864 | 20174 | 18050 | 9255 | |
| 451: | 24594 | 19614 | 20308 | 26528 | 1163 | 19593 | 27829 | 18233 | 15723 | 21027 | 25058 | 6741 |
| | 30235 | 19983 | 15588 | 25168 | 20958 | 9264 | 12404 | 15513 | 12665 | 19084 | 25417 | 21772 |
| | 12290 | 25875 | 20088 | 14969 | 5154 | 21667 | 10615 | 1691 | 1129 | 28558 | 21679 | 10379 |
| | 21512 | 17453 | 15300 | 4450 | 27226 | 30150 | 11454 | 5314 | 10023 | 11338 | 16591 | 20030 |
| | 18633 | 21326 | 28918 | 4535 | 20754 | 15263 | 15360 | 10502 | 11441 | 23324 | 20647 | 2276 |
| | 19190 | 29377 | 12592 | 5710 | 3754 | 3134 | 3131 | 24118 | 15940 | 11381 | 15514 | 24047 |
| | 26487 | 16518 | 27801 | 12309 | 20757 | 21974 | 4272 | 10091 | 25062 | 17470 | 20426 | 12321 |
| | 22713 | 9457 | 3953 | 10792 | 3287 | 27371 | 26455 | 26050 | 16917 | 14699 | 15285 | 13553 |
| | 9596 | 10549 | 16896 | 13228 | 27610 | 21302 | 15448 | 11015 | 7359 | 24665 | 27514 | 12094 |
| | 27281 | 11744 | 15041 | 15681 | 28324 | 14029 | 10196 | 27852 | 8686 | 14249 | 23859 | 18836 |
| | 28105 | 15635 | 12025 | 7659 | 10003 | 5225 | 17008 | 13971 | 22081 | 4614 | 27039 | 20125 |
| | 14809 | 21300 | 23147 | 1678 | 24723 | 12543 | 13654 | 7505 | 4391 | 18134 | 4749 | 20839 |
| | 18625 | 10611 | 24703 | 10996 | 23952 | 12704 | 7528 | 11185 | 5705 | 9514 | 25156 | 26901 |
| | 8518 | 22894 | 9948 | 1283 | 10053 | 3077 | 5362 | 19475 | 8252 | 17401 | 905 | 7000 |
| | 16978 | 18388 | 5968 | 7153 | 24505 | 4115 | 9692 | 8864 | 21362 | 4887 | 5743 | 9500 |
| | 15997 | 4255 | 24905 | 20586 | 30241 | 2505 | 7969 | 26082 | 25151 | 16117 | 19627 | 20600 |
| | 27569 | 16639 | 15626 | 14007 | 16037 | 13974 | 19628 | 19906 | 15128 | 11950 | 20013 | 2215 |
| | 28465 | 14468 | 3279 | 29910 | 19502 | 21447 | 9567 | 9381 | 17346 | 11957 | 15242 | 11332 |
| | 19929 | 23742 | 19216 | 22228 | 19258 | 17057 | 26458 | 19834 | 20924 | 3471 | 13987 | 23877 |
| | 5032 | 21272 | 15991 | 24075 | 13894 | 8149 | 9796 | 11329 | 12670 | 23414 | 17197 | 29642 |
| | 4605 | 26840 | 8366 | 14479 | 10192 | 5719 | 25141 | 26752 | 7976 | 5803 | 8509 | 15718 |
| | 21900 | 13634 | 11292 | 21823 | 26182 | 27855 | 9451 | 13386 | 20843 | 23346 | 7186 | 11542 |
| | 8650 | 5820 | 5825 | 22957 | 26205 | 22006 | 1632 | 4006 | 27944 | 7829 | 21372 | 16991 |
| | 11036 | 18405 | 23351 | 14657 | 2967 | 3312 | 12682 | 4627 | 13575 | 6649 | 24195 | 24400 |
| | 11995 | 20054 | 11647 | 18993 | 23596 | 15606 | 24159 | 2765 | 8178 | 8909 | 6811 | 25904 |
| | 4623 | 2523 | 20163 | 27057 | 27723 | 19679 | 2354 | 28259 | 20380 | 3059 | 6183 | 17542 |
| | 19471 | 24822 | 7945 | 3067 | 17023 | 17443 | 21559 | 6514 | 9329 | 12394 | 29248 | 17394 |
| | 19093 | 22545 | 783 | 17802 | 30274 | 25082 | 9207 | 11402 | 14132 | 29558 | 29553 | 16629 |

(continued)

| SEQ ID NO: | homolog SEQ ID NOs | | | | | | | | | | |
|---|---|---|---|---|---|---|---|---|---|---|---|
| 18087 | 9669 | 28353 | 26960 | 23250 | 23023 | 18067 | 24318 | 18611 | 16944 | 26651 | 2738 |
| 17634 | 8567 | 12696 | 23013 | 17988 | 19309 | 2315 | 28747 | 21341 | 26039 | 2874 | 19998 |
| 9766 | 8441 | 8490 | 22522 | 16919 | 2224 | 6108 | 9292 | 23850 | 23318 | 23231 | 29386 |
| 21275 | 11067 | 3234 | 6182 | 21245 | 29705 | 997 | 21169 | 13564 | 6375 | 22763 | 22769 |
| 22740 | 11940 | 11055 | 27886 | 26839 | 21556 | 20561 | 9372 | 7177 | 14310 | 17314 | 3989 |
| 21614 | 27166 | 5390 | 25963 | 3021 | 1986 | 17758 | 11389 | 13011 | 15610 | 12346 | 10957 |
| 26090 | 25438 | 11298 | 983 | 29521 | 28952 | 10029 | 8720 | 9592 | 8841 | 26882 | 7614 |
| 10092 | 26948 | 2672 | 4416 | 18450 | 27542 | | | | | | |
| 452: | | | | | | | | | | | |
| 24594 | 19614 | 20308 | 26528 | 1163 | 19593 | 27829 | 18233 | 15723 | 21027 | 25058 | 6741 |
| 30235 | 19983 | 15588 | 25168 | 20958 | 9264 | 12404 | 15513 | 12665 | 19084 | 25417 | 21772 |
| 12290 | 25875 | 20088 | 14969 | 5154 | 21667 | 10615 | 1691 | 1129 | 28558 | 21679 | 10379 |
| 21512 | 17453 | 15300 | 4450 | 27226 | 30150 | 11454 | 5314 | 10023 | 11338 | 16591 | 20030 |
| 18633 | 21326 | 28918 | 4535 | 20754 | 15263 | 15360 | 10502 | 11441 | 23324 | 20647 | 2276 |
| 19190 | 29377 | 12592 | 5710 | 3754 | 3134 | 3131 | 24118 | 15940 | 11381 | 15514 | 24047 |
| 26487 | 16518 | 27801 | 12309 | 20757 | 21974 | 4272 | 10091 | 25062 | 17470 | 20426 | 12321 |
| 22713 | 9457 | 3953 | 10792 | 3287 | 27371 | 26455 | 26050 | 16917 | 14699 | 15285 | 13553 |
| 9596 | 10549 | 16896 | 13228 | 27610 | 21302 | 15448 | 11015 | 7359 | 24665 | 27514 | 12094 |
| 27281 | 11744 | 15041 | 15681 | 28324 | 14029 | 10196 | 27852 | 8686 | 14249 | 23859 | 18836 |
| 28105 | 15635 | 12025 | 7659 | 10003 | 5225 | 17008 | 13971 | 22081 | 4614 | 27039 | 20125 |
| 14809 | 21300 | 23147 | 1678 | 24723 | 12543 | 13654 | 7505 | 4391 | 18134 | 4749 | 20839 |
| 18625 | 10611 | 24703 | 10996 | 23952 | 12704 | 7528 | 11185 | 5705 | 9514 | 25156 | 26901 |
| 8518 | 22894 | 9948 | 1283 | 10053 | 3077 | 5362 | 19475 | 8252 | 17401 | 905 | 7000 |
| 16978 | 18388 | 5968 | 7153 | 24505 | 4115 | 9692 | 8864 | 21362 | 4887 | 5743 | 9500 |
| 15997 | 4255 | 24905 | 20586 | 30241 | 2505 | 7969 | 26082 | 25151 | 16117 | 19627 | 20600 |
| 27569 | 16639 | 15626 | 14007 | 16037 | 13974 | 19628 | 19906 | 15128 | 11950 | 20013 | 2215 |
| 28465 | 14468 | 3279 | 29910 | 19502 | 21447 | 9567 | 9381 | 17346 | 11957 | 15242 | 11332 |
| 19929 | 23742 | 19216 | 22228 | 19258 | 17057 | 26458 | 19834 | 20924 | 3471 | 13987 | 23877 |
| 5032 | 21272 | 15991 | 24075 | 13894 | 8149 | 9796 | 11329 | 12670 | 23414 | 17197 | 29642 |
| 4605 | 26840 | 8366 | 14479 | 10192 | 5719 | 25141 | 26752 | 7976 | 5803 | 8509 | 15718 |
| 21900 | 13634 | 11292 | 21823 | 26182 | 27855 | 9451 | 13386 | 20843 | 23346 | 7186 | 11542 |
| 8650 | 5820 | 5825 | 22957 | 26205 | 22006 | 1632 | 4006 | 27944 | 7829 | 21372 | 16991 |
| 11036 | 18405 | 23351 | 14657 | 2967 | 3312 | 12682 | 4627 | 13575 | 6649 | 24195 | 24400 |
| 11995 | 20054 | 11647 | 18993 | 23596 | 15606 | 24159 | 2765 | 8178 | 8909 | 6811 | 25904 |

(continued)

SEQ ID NO: homolog SEQ ID NOs

| SEQ ID NO | | | | | | | | | | | | |
|---|---|---|---|---|---|---|---|---|---|---|---|---|
| | 4623 | 2523 | 20163 | 27057 | 27723 | 19679 | 2354 | 28259 | 20380 | 3059 | 6183 | 17542 |
| | 19471 | 24822 | 7945 | 3067 | 17023 | 17443 | 21559 | 6514 | 9329 | 12394 | 29248 | 17394 |
| | 19093 | 22545 | 783 | 17802 | 30274 | 25082 | 9207 | 11402 | 14132 | 29558 | 29553 | 16629 |
| | 18087 | 9669 | 28353 | 26960 | 23250 | 23023 | 18067 | 24318 | 18611 | 16944 | 26651 | 2738 |
| | 17634 | 8567 | 12696 | 23013 | 17988 | 19309 | 2315 | 28747 | 21341 | 26039 | 2874 | 19998 |
| | 9766 | 8441 | 8490 | 22522 | 16919 | 2224 | 6108 | 9292 | 23850 | 23318 | 23231 | 29386 |
| | 21275 | 11067 | 3234 | 6182 | 21245 | 29705 | 997 | 21169 | 13564 | 6375 | 22763 | 22769 |
| | 22740 | 11940 | 11055 | 27886 | 26839 | 21556 | 20561 | 9372 | 7177 | 14310 | 17314 | 3989 |
| | 21614 | 27166 | 5390 | 25963 | 3021 | 1986 | 17758 | 11389 | 13011 | 15610 | 12346 | 10957 |
| | 26090 | 25438 | 11298 | 983 | 29521 | 28952 | 10029 | 8720 | 9592 | 8841 | 26882 | 7614 |
| | 10092 | 26948 | 2672 | 4416 | 18450 | 27542 | | | | | | |
| 453: | 25366 | 18569 | 29531 | 3818 | 21748 | 11043 | 29332 | 19647 | 25887 | 26064 | 23926 | 4105 |
| | 23710 | 25881 | 20298 | 3352 | 3029 | 11690 | 15830 | 3353 | 7008 | 19722 | 2041 | 29083 |
| | 4788 | 10647 | 27130 | 20324 | 3874 | 3090 | 16618 | 29802 | 25389 | 5905 | 25459 | 11142 |
| | 10528 | 26015 | 6090 | 3940 | 17694 | 26826 | 26481 | 18164 | 18186 | 11988 | 29381 | 11803 |
| | 2344 | 24788 | 26720 | 16667 | 24858 | 21087 | 19689 | 28351 | 21692 | 29013 | 15747 | 9007 |
| | 25278 | 10430 | 22832 | 13515 | 25805 | 13115 | 19172 | 13436 | 26040 | 24354 | 6186 | 5091 |
| | 9551 | 7648 | 9645 | 9635 | 3564 | 17666 | 5119 | 7906 | 5138 | 5571 | 23941 | 21051 |
| | 19482 | 5064 | 18288 | 21564 | 4894 | 7714 | 29242 | 23177 | 22295 | 15831 | 17787 | 21234 |
| | 4325 | 14782 | 11859 | 13999 | 29378 | 24298 | 9122 | 10399 | 6155 | 22347 | 4811 | 25512 |
| | 27640 | 28649 | 24692 | 21574 | 13573 | 12997 | 3087 | 26258 | 13639 | 18310 | 18712 | 18025 |
| | 13445 | 14971 | 7647 | 20368 | 13579 | 19818 | 12098 | 28049 | 7569 | 7512 | 8138 | 11655 |
| | 25923 | 22903 | 12815 | 15631 | 17951 | 18413 | 16895 | 9498 | 29237 | 20212 | 7730 | 20927 |
| | 2036 | 8928 | 27390 | 7748 | 11567 | 2295 | 9306 | 823 | 25111 | 22426 | 22985 | 27483 |
| | 28312 | 21766 | 19746 | 14041 | 4777 | 6512 | 30013 | 9043 | 20585 | 4578 | 2588 | 5621 |
| | 11682 | 24775 | 17563 | 13735 | 12744 | 29740 | 17795 | 6221 | 28597 | 20069 | 2399 | 8125 |
| | 26268 | 17752 | 16883 | 26665 | 3285 | 25829 | 14459 | 9591 | 12154 | 8984 | 10762 | 7317 |
| | 18258 | 6015 | 24872 | | | | | | | | | |
| 454: | 15937 | 21204 | 15557 | 7974 | 7113 | 28706 | 28785 | 18797 | 28914 | 25957 | 29623 | 25671 |
| | 26342 | 18614 | 9382 | 8110 | 12236 | 19552 | 19147 | | | | | |
| 455: | 18809 | 6667 | 2294 | 25777 | 4100 | 10690 | 16859 | 30277 | 20583 | 29524 | 13671 | 6937 |
| | 30055 | 16036 | 17239 | 16152 | 15986 | 16070 | 21231 | 26235 | 3318 | 13935 | 24757 | 4685 |
| | 10213 | 8221 | 12631 | | | | | | | | | |

SEQ ID NO: homolog SEQ ID NOs

| 456: | 18809 | 6667 | 2294 | 25777 | 4100 | 10690 | 16859 | 30277 | 20583 | 29524 | 13671 | 6937 |
| | 30055 | 16036 | 17239 | 16152 | 15986 | 16070 | 21231 | 26235 | 3318 | 13935 | 24757 | 4685 |
| | 10213 | 8221 | 12631 | | | | | | | | | |
| 457: | 18809 | 6667 | 2294 | 25777 | 4100 | 10690 | 16859 | 30277 | 20583 | 29524 | 13671 | 6937 |
| | 30055 | 16036 | 17239 | 16152 | 15986 | 16070 | 21231 | 26235 | 3318 | 13935 | 24757 | 4685 |
| | 10213 | 8221 | 12631 | | | | | | | | | |
| 458: | 14297 | 2931 | 1300 | 6353 | 27708 | 16099 | 9335 | 765 | 21909 | 19607 | 17036 | 4611 |
| | 13166 | 13388 | 13056 | 14897 | 25327 | 29552 | 14464 | 15080 | 25242 | 22846 | 3720 | 9000 |
| | 16013 | 23389 | 2157 | 8389 | 24878 | 25161 | 16087 | 29889 | 3895 | 24798 | 20821 | 3181 |
| | 5407 | 24273 | 5525 | 12800 | 25175 | 9680 | 19128 | 2153 | 15050 | 1544 | 2892 | 20288 |
| | 16963 | 9150 | 11416 | 27083 | 22540 | 13380 | | | | | | |
| 459: | 12252 | 27790 | 13565 | 28397 | 4118 | 16671 | 28560 | 23786 | 19577 | 25879 | 27052 | 10191 |
| | 11476 | 21159 | 28020 | 3991 | 9283 | 26595 | 16540 | 12228 | 22074 | 22464 | 22802 | 26261 |
| | 7859 | 26940 | 17139 | 19573 | 23964 | 23812 | 13571 | 11798 | 13355 | 21494 | 22999 | 23658 |
| | 5034 | 24727 | 15782 | 858 | 19068 | 27754 | 6562 | 18600 | 25192 | 11942 | 11462 | 1417 |
| | 29954 | 5656 | 14528 | 9682 | 19326 | 14054 | 15336 | 20830 | 13841 | 10329 | 10702 | 2331 |
| | 873 | 6735 | 4631 | 9922 | 12306 | 14208 | 14752 | 3103 | 13277 | 4003 | 10671 | 8726 |
| | 13453 | 20643 | 22505 | 24436 | 4386 | 7379 | 21979 | 19686 | 14240 | 20721 | 22374 | 14119 |
| | 14070 | 3015 | 11991 | 14789 | 14186 | 13777 | 10602 | 11466 | 21298 | 17878 | 7423 | 26602 |
| | 22576 | 10042 | 28957 | 19060 | 1692 | 4008 | 5192 | 875 | 13891 | 11264 | 3499 | 25959 |
| | 13118 | 25097 | 19315 | 4295 | 9343 | 19193 | 28140 | 26793 | 28548 | 7625 | 10810 | 20160 |
| | 17861 | 922 | 11887 | 17267 | 4319 | 9434 | 24515 | 14919 | 11926 | 25241 | 8730 | 5953 |
| | 19705 | 3516 | 10620 | 19212 | 28869 | 24611 | 14166 | 4956 | 4513 | 7304 | 20932 | 5789 |
| | 6089 | 18427 | 975 | 15847 | 24320 | 12388 | 25469 | 7112 | 12888 | 1274 | 24859 | 797 |
| | 8627 | 26611 | 20023 | 3311 | 11882 | 19024 | 18734 | 22556 | 18293 | 7960 | 22944 | 9075 |
| | 25422 | 23706 | 20484 | 23580 | 27921 | 19695 | 20948 | 17629 | 18987 | 13090 | 20399 | 15045 |
| | 29898 | 15784 | 16164 | 13799 | 3254 | 27791 | 6700 | 23490 | 17058 | 3858 | 30192 | 12736 |
| | 8163 | 21915 | 19054 | 29873 | 19684 | 13086 | 14830 | 29254 | 1340 | 26644 | 25237 | 7977 |
| | 15326 | 12480 | 28790 | 26966 | 25263 | 726 | 28884 | 16712 | 27948 | 23195 | 15129 | 17738 |
| | 26816 | 10733 | 1741 | 28153 | 15000 | 28480 | 28848 | 21025 | 17132 | 20400 | 8033 | 10994 |
| | 19980 | 20226 | 28462 | 7900 | 4312 | 28087 | 9631 | 27891 | 5434 | 14315 | 14184 | 2288 |
| | 2298 | 21482 | 14606 | 9525 | 26337 | 12871 | 1098 | 9392 | 12406 | 15358 | 26780 | 27916 |
| | 27755 | 26760 | 25573 | 23863 | 16929 | 15783 | 27648 | 25998 | 26210 | 13715 | 20349 | 9310 |

SEQ ID NO: homolog SEQ ID NOs

| SEQ ID NO | | | | | | | | | | | | |
|---|---|---|---|---|---|---|---|---|---|---|---|---|
| | 6485 | 10732 | 14484 | 5627 | 17493 | 6038 | 9101 | 18502 | 4046 | 1276 | 11400 | 19358 |
| | 3667 | 3398 | 27018 | 7255 | 23149 | 12010 | 13859 | 26502 | 19619 | 27932 | 12855 | 25307 |
| | 26943 | 768 | 20273 | 29150 | 27029 | 15671 | 29109 | 18653 | 15731 | 1239 | 1241 | 21463 |
| | 15871 | 20514 | 9859 | 23076 | 1910 | 11094 | 20893 | 27197 | 10798 | 16724 | 16690 | 22778 |
| | 3140 | 23293 | 9947 | 29694 | 29696 | 11557 | 2287 | 13814 | 26137 | 5947 | 10044 | 10874 |
| | 28757 | 5240 | 5253 | 29587 | 29586 | 29557 | 29649 | 29693 | 8747 | 13933 | 2279 | 17115 |
| | 15656 | 26730 | 12194 | 24793 | 11009 | 14536 | 2503 | 28429 | 1537 | 959 | 23661 | 1326 |
| | 4354 | 23477 | 4581 | 27535 | 28901 | 14420 | 17203 | 7270 | 22161 | 10442 | 916 | 12807 |
| | 10270 | 12874 | 20440 | 24746 | 17699 | | | | | | | |
| 460: | 15232 | 19071 | 27333 | 11787 | 1124 | 29203 | 13936 | 16751 | 4597 | 28561 | 20896 | 9465 |
| | 3592 | 19921 | 11646 | 20889 | 22582 | | | | | | | |
| 461: | 24419 | 1402 | 25916 | 20137 | 17625 | 22665 | 10568 | 25358 | 26142 | 28311 | 15846 | 24569 |
| | 24598 | 29238 | 13390 | 24563 | 24567 | 15427 | 23189 | 4780 | 19664 | 14896 | 24951 | 830 |
| | 15736 | 23246 | 18932 | 21676 | 4814 | 7320 | 24568 | 3465 | 30108 | 18936 | 6076 | 6951 |
| | 16905 | 10105 | 7586 | 2254 | 22518 | 5303 | 19750 | 10061 | 6701 | 4164 | | |
| 462: | 8277 | 3558 | 16597 | 29654 | 26398 | 6998 | 24578 | 6409 | 5653 | 5600 | 16422 | 1279 |
| 463: | 14436 | 13440 | 20857 | 18542 | 29977 | 4184 | 8955 | 8576 | 3977 | 27464 | 29539 | 8197 |
| | 12240 | 29732 | | | | | | | | | | |
| 464: | 14436 | 13440 | 20857 | 18542 | 29977 | 4184 | 8955 | 8576 | 3977 | 27464 | 29539 | 8197 |
| | 12240 | 29732 | | | | | | | | | | |
| 465: | 22196 | 12071 | 22979 | 22949 | 29573 | 15498 | 15491 | 22425 | 13716 | 22982 | 13666 | 28606 |
| | 17945 | 17976 | 22481 | 21230 | 27637 | 27641 | 19634 | 7854 | 29819 | 12270 | 12272 | 29828 |
| | 29858 | 29872 | 29863 | 29896 | 22911 | 22913 | 30048 | 30102 | 30104 | 12299 | 12275 | 30107 |
| | 29285 | 12278 | 9953 | 12298 | 29310 | 9916 | 30040 | 30074 | 29307 | 29314 | 30041 | 12378 |
| | 30044 | 30064 | 8445 | 30070 | 30098 | 30125 | 30046 | 30109 | 25261 | 12173 | 25265 | 25269 |
| | 12175 | 12179 | 12182 | 29633 | 12301 | 25411 | 24607 | 22941 | 23018 | 27660 | 8052 | 22557 |
| | 6168 | 26743 | 1121 | 27575 | 26131 | 22376 | 22541 | 23421 | 22506 | 25933 | 22427 | 22392 |
| | 22508 | 22485 | 25445 | 22488 | 7322 | 22503 | 22589 | 29279 | 5219 | 22535 | 22501 | 22397 |
| | 3810 | 22583 | 22456 | 22533 | 22453 | 22458 | 7610 | 7433 | 12303 | 15420 | 3532 | 3554 |
| | 3561 | 3556 | 22527 | 13662 | 19532 | 5629 | 19562 | 19560 | 19564 | 19568 | 19601 | 19595 |
| | 19597 | 27672 | 1210 | 13690 | 16783 | 22569 | 22562 | 22586 | 13141 | 27026 | 5908 | 30119 |
| | 24377 | 23015 | 21860 | 12743 | 9891 | 6206 | 8428 | 1078 | 1115 | 2746 | 20692 | 12068 |
| | 12043 | 12045 | 18786 | 7007 | 23439 | 23971 | 8580 | 7064 | 3706 | 6167 | 16999 | 24206 |

(continued)

(continued)

SEQ ID NO: homolog SEQ ID NOs

| SEQ ID NO: | | | | | | | | | | | | |
|---|---|---|---|---|---|---|---|---|---|---|---|---|
| | 23616 | 26322 | 3113 | 25583 | 10155 | 15899 | 25974 | 27928 | 29956 | 22209 | 22143 | 22141 |
| | 22105 | 22174 | 22208 | 22168 | 11219 | 1070 | 20897 | 7667 | 3534 | 13305 | 28084 | 6284 |
| | 6286 | 6280 | 6277 | 6238 | 6211 | 6207 | 6209 | 8146 | 2605 | 2767 | 9822 | 11566 |
| | 2806 | 29430 | 10312 | 4187 | 9197 | 10546 | 27048 | 6275 | 3980 | 24499 | 9748 | 7851 |
| | 900 | 29212 | 12281 | 3075 | 4909 | 6950 | 8109 | 24379 | 9913 | 14539 | 1414 | 9446 |
| | 29963 | 16956 | 6214 | 6138 | 28637 | 6787 | 27913 | 16877 | 19946 | 14632 | 24351 | 24375 |
| | 12901 | 28154 | 24168 | 4171 | 28158 | 19297 | 13958 | 27194 | 6169 | 6171 | 6243 | 6241 |
| | 6245 | 6248 | 6176 | 25796 | 29624 | 1290 | 17980 | 22417 | 16009 | 16046 | 12150 | 2223 |
| | 29676 | 27846 | 1567 | 11241 | 16848 | 6083 | 7179 | 22954 | 2631 | 4550 | 4518 | 7772 |
| | 3054 | 9430 | 21434 | 17476 | 20220 | 13686 | 21104 | 24978 | 23120 | 22983 | 13892 | 22952 |
| | 23328 | 26616 | 22955 | 16685 | 29867 | 30071 | 9946 | 22531 | 22909 | 14293 | 22986 | 23055 |
| | 24110 | 25142 | 17936 | 19371 | 23053 | 8354 | 23093 | 6124 | 21675 | 7721 | 28958 | 1459 |
| | 8339 | 23092 | 23014 | 8595 | 23047 | 27326 | 28970 | 1205 | 25415 | 27129 | 976 | 29897 |
| | 26834 | 2070 | 29820 | 29822 | 22483 | 26284 | 15712 | 2951 | 1813 | 1754 | 28358 | 29596 |
| | 16687 | 7470 | 2212 | 24264 | 25888 | 6136 | 7211 | 15516 | 25413 | 22191 | 22905 | 25969 |
| | 28313 | 22313 | 24627 | 3443 | 22810 | 16947 | 12653 | 4254 | 22422 | 10215 | 10175 | 10150 |
| | 15017 | 18234 | 22919 | 22945 | 22981 | 15717 | 24666 | 9779 | 16752 | 16748 | 16754 | 16718 |
| | 15869 | 15913 | 16746 | 16673 | 16720 | 15844 | 16709 | 16680 | 15914 | 7056 | 7060 | 10179 |
| | 10113 | 10146 | 3335 | 27826 | 18053 | 27858 | 2475 | 19819 | 1029 | 7091 | 11426 | 11431 |
| | 5002 | 29516 | 26866 | 22590 | 23417 | 7061 | 13286 | 15720 | 15724 | 2770 | 4451 | 8127 |
| | 12246 | 24902 | 6226 | 24904 | 2919 | 6315 | 12211 | 12138 | 12143 | 12146 | 23051 | 6307 |
| | 10873 | 25862 | 1120 | 12940 | 25300 | 25304 | 23020 | 23462 | 23494 | 22398 | 7090 | 8226 |
| | 28830 | 27002 | 22400 | 26328 | 12149 | 12172 | 12206 | 12209 | 21858 | 21853 | 23397 | 25317 |
| | 2949 | 21890 | 21887 | 9073 | 13978 | 14089 | 13977 | 13980 | 22459 | 23463 | 8074 | 8779 |
| | 13748 | 15517 | 12065 | 23011 | 9663 | 9703 | 9660 | 9705 | 12239 | 12243 | 12249 | 21893 |
| | 2943 | 2921 | 2945 | 6310 | 6813 | 3692 | 2954 | 11144 | 23050 | 29312 | 16920 | 25717 |
| | 18708 | 5883 | 10211 | 23691 | 19535 | 13745 | 25585 | 26433 | 20779 | 14386 | 22430 | 6140 |
| | 19308 | 1877 | 13379 | 5152 | 12659 | 928 | 22136 | 22843 | 22131 | 22124 | 22128 | 2385 |
| | 17288 | 17290 | 17279 | 17285 | 17313 | 29824 | 2520 | 2542 | 1702 | 14047 | 7898 | 12044 |
| | 8265 | 7931 | | | | | | | | | | |
| 466: | 7892 | 7896 | 26821 | 26837 | 26847 | 26843 | 26902 | 26879 | 26875 | 26841 | 19174 | 18897 |
| | 21557 | 22670 | 12859 | 8977 | 16721 | 20569 | 12157 | 28261 | 16133 | 6471 | 12435 | 20381 |
| 467: | 7892 | 7896 | 26821 | 26837 | 26847 | 26843 | 26902 | 26879 | 26875 | 26841 | 19174 | 18897 |

SEQ ID NO: homolog SEQ ID NOs

| | | | | | | | | | | | | |
|---|---|---|---|---|---|---|---|---|---|---|---|---|
| | 21557 | 22670 | 12859 | 8977 | 16721 | 20569 | 12157 | 28261 | 16133 | 6471 | 12435 | 20381 |
| 468: | 20342 | 14996 | 8410 | 8167 | 9206 | 19489 | 20674 | 9481 | 22930 | 4492 | 24760 | 19342 |
| | 12327 | 26155 | 10723 | 9936 | 7130 | 8464 | 7682 | 6291 | 5379 | 28178 | 3800 | 15918 |
| | 24974 | 13586 | 4261 | 3649 | | | | | | | | |
| 469: | 20342 | 14996 | 8410 | 8167 | 9206 | 19489 | 20674 | 9481 | 22930 | 4492 | 24760 | 19342 |
| | 12327 | 26155 | 10723 | 9936 | 7130 | 8464 | 7682 | 6291 | 5379 | 28178 | 3800 | 15918 |
| | 24974 | 13586 | 4261 | 3649 | | | | | | | | |
| 470: | 24344 | 24370 | 21966 | 4521 | 17232 | 26896 | 23433 | 24102 | 18282 | 29135 | 22984 | 5704 |
| | 25450 | 24724 | 23399 | 6702 | 14794 | 5859 | 5692 | 13990 | 2274 | 28368 | 1897 | 1893 |
| | 1900 | 21873 | 2425 | 10481 | 10027 | 8191 | 8998 | 8976 | 5451 | 15322 | 21995 | 18923 |
| | 29987 | 3336 | 27849 | 28148 | 28378 | 1427 | 14968 | 6816 | 27557 | 26786 | 23911 | 5000 |
| | 9064 | 27980 | 24651 | 9776 | 27877 | 29296 | 11963 | | | | | |
| 471: | 24344 | 24370 | 21966 | 4521 | 17232 | 26896 | 23433 | 24102 | 18282 | 29135 | 22984 | 5704 |
| | 25450 | 24724 | 23399 | 6702 | 14794 | 5859 | 5692 | 13990 | 2274 | 28368 | 1897 | 1893 |
| | 1900 | 21873 | 2425 | 10481 | 10027 | 8191 | 8998 | 8976 | 5451 | 15322 | 21995 | 18923 |
| | 29987 | 3336 | 27849 | 28148 | 28378 | 1427 | 14968 | 6816 | 27557 | 26786 | 23911 | 5000 |
| | 9064 | 27980 | 24651 | 9776 | 27877 | 29296 | 11963 | | | | | |
| 472: | 10705 | 27256 | 19620 | 8931 | 28314 | 5163 | 27954 | 1395 | 1268 | 15281 | 16563 | 26246 |
| | 7487 | 5080 | 3414 | 7929 | 21683 | 10701 | 14972 | 5413 | 12498 | 5626 | 9270 | 1924 |
| | 4961 | 9416 | 1464 | 27016 | 27443 | 7850 | 6820 | 24741 | 7105 | 12778 | 2092 | 28359 |
| | 3297 | 3111 | 29602 | 18701 | 14432 | 21639 | 29329 | 19611 | 3422 | | | |
| 473: | 10705 | 27256 | 19620 | 8931 | 28314 | 5163 | 27954 | 1395 | 1268 | 15281 | 16563 | 26246 |
| | 7487 | 5080 | 3414 | 7929 | 21683 | 10701 | 14972 | 5413 | 12498 | 5626 | 9270 | 1924 |
| | 4961 | 9416 | 1464 | 27016 | 27443 | 7850 | 6820 | 24741 | 7105 | 12778 | 2092 | 28359 |
| | 3297 | 3111 | 29602 | 18701 | 14432 | 21639 | 29329 | 19611 | 3422 | | | |
| 474: | 25474 | 20408 | 4141 | 30024 | 28043 | 7754 | 21038 | 21416 | 24427 | 14231 | 14886 | 19277 |
| | 14114 | 8383 | 30015 | 5733 | 21052 | 1889 | 28176 | 24595 | 6361 | 27887 | 2497 | |
| 475: | 4404 | 3910 | 19786 | 5332 | 10069 | 20258 | 8014 | 28028 | 16620 | 6511 | 23207 | 3124 |
| | 19430 | 21607 | 13903 | 21737 | 9398 | 20157 | 25074 | 4612 | 13497 | | | |
| 476: | 6242 | 26425 | 26448 | 26444 | 13402 | 29414 | 6792 | 13129 | 8015 | 3041 | 18543 | 28037 |
| | 12350 | 3454 | 26854 | 3572 | 29206 | 17499 | 12719 | 28647 | 8631 | 13022 | 10567 | 19713 |
| | 6789 | 11392 | 30269 | 27847 | 9834 | 3529 | 19003 | 18500 | 21843 | 21845 | 9516 | 26863 |
| | 8835 | | | | | | | | | | | |

(continued)

SEQ ID NO: homolog SEQ ID NOs

| SEQ ID NO: | | | | | | | | | | | | |
|---|---|---|---|---|---|---|---|---|---|---|---|---|
| 477: | 6242 | 26425 | 26448 | 26444 | 13402 | 29414 | 6792 | 13129 | 8015 | 3041 | 18543 | 28037 |
| | 12350 | 3454 | 26854 | 3572 | 29206 | 17499 | 12719 | 28647 | 8631 | 13022 | 10567 | 19713 |
| | 6789 | 11392 | 30269 | 27847 | 9834 | 3529 | 19003 | 18500 | 21843 | 21845 | 9516 | 26863 |
| | 8835 | | | | | | | | | | | |
| 478: | 23597 | 12266 | 27078 | 27427 | 11650 | 20652 | 11932 | 7998 | 18758 | 23785 | 8450 | 27455 |
| | 26951 | 9317 | 13219 | 14863 | 1349 | 932 | 25328 | 8024 | 2427 | 10084 | 10026 | 9051 |
| | 26980 | 8359 | 22086 | 11956 | 5791 | 16366 | 4737 | 22737 | 2291 | 22314 | 17858 | 17370 |
| | 22761 | 7102 | 3485 | 26723 | 29968 | 11168 | 14025 | 20737 | 1635 | 2271 | 6697 | 29787 |
| | 25021 | 21203 | 13398 | 29952 | 25533 | 18141 | 6303 | 12595 | 26380 | 16899 | 12934 | 5739 |
| | 2426 | 28707 | 17445 | 8989 | 1967 | 27599 | 20256 | 1763 | 8527 | 21740 | 17983 | 9202 |
| | 899 | 1769 | 1512 | 30094 | 6126 | 28239 | 30267 | 16014 | 9783 | 11861 | 18133 | 26680 |
| | 4174 | 28883 | 20007 | 7412 | 6262 | 2173 | 22828 | 13580 | 10784 | 4125 | 6321 | 18471 |
| 479: | 11266 | 16085 | 10634 | 1555 | 879 | 14706 | 19360 | 1093 | 20240 | 10079 | 4196 | 4771 |
| | 25836 | 6691 | 30169 | 24095 | 21153 | 753 | 7073 | 22741 | 6654 | 13769 | 20489 | 23077 |
| | 1746 | 1993 | 2630 | 2008 | 16190 | 2532 | 11785 | 13795 | 6217 | 16988 | 3239 | 9686 |
| | 12729 | 3616 | 12449 | 22486 | 7081 | 27365 | 16102 | 27404 | 7820 | | | |
| 480: | 30186 | 17317 | 18088 | 11538 | 17942 | 22139 | | | | | | |
| 481: | 26609 | 26654 | 19703 | 3951 | 25123 | 18199 | 24339 | 13212 | 24623 | 25206 | 9216 | 8714 |
| | 7559 | 24224 | 25894 | 16605 | 16166 | 15851 | 26097 | 10391 | 11814 | 11908 | 1444 | 20213 |
| | 30284 | 20444 | 27204 | 27206 | 22306 | 9519 | 8102 | 6901 | 1685 | 13752 | 25886 | 6829 |
| | 9721 | 18724 | 5847 | 18788 | 859 | 3128 | 26108 | 15970 | 15713 | 12725 | 8760 | 18810 |
| | 935 | 24509 | 23297 | 23096 | 12000 | 18049 | 24810 | 1982 | 28062 | 26467 | 26041 | 23105 |
| | 910 | 26434 | 12153 | 16493 | 28156 | 28100 | 6075 | 24706 | 7716 | 12867 | 29675 | 13338 |
| | 20725 | 21784 | 22960 | 25839 | 28289 | 4506 | 28576 | 1865 | 18489 | 8767 | 16312 | 22942 |
| | 29761 | 22205 | 8285 | 10786 | 16637 | 15815 | 25301 | 27786 | 22579 | 12357 | 13168 | 12961 |
| | 6926 | 1779 | 12142 | 6988 | 19344 | 11177 | 2446 | 15241 | 13629 | 12137 | 12510 | 25429 |
| | 16416 | 27560 | 3808 | 8253 | 4181 | 15573 | 9142 | 19808 | 20521 | 21286 | 21615 | 10748 |
| | 22896 | 28686 | 23136 | 4169 | 19201 | 5175 | 8335 | 28738 | 26332 | 18468 | 22091 | 12852 |
| | 16742 | 25850 | 16557 | 11987 | 5693 | 28184 | 9732 | 2245 | 22103 | 3363 | 25041 | 5785 |
| | 5213 | 26367 | 3107 | 26869 | 2353 | 17262 | 3840 | 19505 | 22076 | 23947 | 1253 | 20675 |
| | 16543 | 6871 | 14856 | 2040 | 27798 | 6312 | 8301 | 14849 | 22793 | 24792 | 19272 | 11727 |
| | 5782 | 1778 | 3143 | 7156 | 5015 | 28496 | 6962 | 20511 | 13156 | 23933 | 25578 | 6432 |
| | 8089 | 4772 | 15390 | 10450 | 6063 | 14560 | 29997 | 27810 | 18529 | 5173 | 28214 | 8968 |

138

(continued)

SEQ ID NO: homolog SEQ ID NOs

|  |  |  |  |  |  |  |  |  |  |  |  |  |
|---|---|---|---|---|---|---|---|---|---|---|---|---|
|  | 4383 | 15090 | 27120 | 3879 | 27490 | 20657 | 6664 | 17300 | 30062 | 3028 | 17077 | 16866 |
|  | 29937 | 29031 | 2412 | 11945 | 11608 | 9107 | 20138 | 12087 | 3571 | 18727 | 7188 | 7504 |
|  | 17906 | 2256 | 27892 | 4308 | 15508 | 6528 | 1312 | 28799 | 6912 | 16032 | 8184 | 29411 |
|  | 12167 | 2834 | 13612 |  |  |  |  |  |  |  |  |  |
| 482: | 25909 | 29988 | 29179 | 26691 | 13642 | 21537 | 11008 | 5717 | 12584 | 12215 | 11207 | 7944 |
|  | 11955 | 1440 | 5540 | 9029 | 2097 | 16744 | 9698 | 11715 | 18713 | 8832 | 5493 | 868 |
|  | 18163 | 16864 | 14230 | 17016 | 16910 | 17452 | 11499 | 25943 | 8481 | 24984 | 30215 | 16885 |
|  | 12528 | 19809 | 27694 | 27229 | 4868 | 27460 | 27642 | 10162 | 17130 | 6925 | 1173 | 1172 |
|  | 3158 | 2320 | 1998 | 6675 | 6995 | 26484 | 14314 | 25643 | 14198 | 30253 | 14690 | 17029 |
|  | 30149 | 4776 | 3263 |  |  |  |  |  |  |  |  |  |
| 483: | 29110 | 3237 | 24034 | 11076 | 19009 | 4487 | 11707 | 13817 | 22420 | 24296 | 8085 | 7805 |
|  | 16768 | 19153 | 5889 | 15715 | 28474 | 2594 | 12164 | 25378 | 1105 | 3194 | 17993 | 7925 |
|  | 13127 | 18534 | 30075 | 6112 | 12462 | 10941 |  |  |  |  |  |  |
| 484: | 15012 | 13085 | 22343 | 9583 | 24926 | 20997 | 22606 | 12951 | 16206 | 2817 | 5881 | 13877 |
|  | 4942 | 17827 | 12466 | 26063 |  |  |  |  |  |  |  |  |
| 485: | 17112 | 28735 | 11966 | 1372 | 23155 | 27023 | 24753 | 5685 | 24596 | 18364 | 4267 | 10110 |
|  | 23654 | 25390 | 3816 | 2599 | 9644 | 5388 | 22269 |  |  |  |  |  |
| 486: | 22471 | 5897 | 6462 | 11512 | 6594 | 12605 | 15510 | 6077 | 19410 | 20570 | 10894 | 2802 |
|  | 18204 | 28451 | 15121 | 21173 | 23559 | 29394 | 6042 | 17434 | 19864 | 15013 | 20645 | 9614 |
|  | 12062 | 6356 | 28536 | 10914 | 7712 | 11137 | 28223 | 16796 | 5311 | 9838 | 28481 | 26464 |
|  | 5374 | 18884 | 25876 | 5245 |  |  |  |  |  |  |  |  |
| 487: | 15099 |  |  |  |  |  |  |  |  |  |  |  |
| 488: | 15099 |  |  |  |  |  |  |  |  |  |  |  |
| 489: | 21356 | 21516 | 26037 | 8808 | 6293 | 12585 | 850 | 6072 | 22946 | 18474 | 16769 | 21943 |
|  | 14885 | 1252 | 30051 | 25754 | 27328 | 1022 | 12843 | 5949 | 24530 | 18151 |  |  |
| 490: | 21356 | 21516 | 26037 | 8808 | 6293 | 12585 | 850 | 6072 | 22946 | 18474 | 16769 | 21943 |
|  | 14885 | 1252 | 30051 | 25754 | 27328 | 1022 | 12843 | 5949 | 24530 | 18151 |  |  |
| 491: | 2871 | 7311 | 5550 | 25927 | 13052 | 22762 | 601 | 19587 | 5178 | 4930 | 5622 | 16364 |
|  | 2560 | 28731 | 13645 | 3869 | 25416 | 5988 | 27188 | 24739 | 2872 | 2873 | 4494 | 23288 |
|  | 23572 | 25147 | 25148 | 28911 | 28118 | 1610 | 9782 | 9785 | 1218 | 30008 | 9751 | 9778 |
|  | 14353 | 25707 | 8776 | 18930 | 9864 | 5555 | 9868 | 9871 | 27500 | 25264 | 19061 | 28533 |
|  | 9730 | 5042 | 19829 | 5546 | 9033 | 11085 | 29776 | 4375 | 22499 | 24352 | 28833 | 11082 |
|  | 5518 | 10835 | 15962 | 3457 | 8543 | 5548 | 29707 | 15480 | 7062 | 26583 | 4373 | 6499 |

(continued)

| SEQ ID NO: | homolog SEQ ID NOs | | | | | | | | | | | |
|---|---|---|---|---|---|---|---|---|---|---|---|---|
| 492: | 25865 | 21223 | 10902 | 8772 | 10689 | 893 | 601 | 19587 | 5178 | 4930 | 5622 | 16364 |
| | 2871 | 7311 | 5550 | 25927 | 13052 | 22762 | 27188 | 24739 | 2872 | 2873 | 4494 | 23288 |
| | 2560 | 28731 | 13645 | 3869 | 25416 | 5988 | 9782 | 9785 | 1218 | 30008 | 9751 | 9778 |
| | 23572 | 25147 | 25148 | 28911 | 28118 | 1610 | 9868 | 9871 | 27500 | 25264 | 19061 | 28533 |
| | 14353 | 25707 | 8776 | 18930 | 9864 | 5555 | 29776 | 4375 | 22499 | 24352 | 28833 | 11082 |
| | 9730 | 5042 | 19829 | 5546 | 9033 | 11085 | 29707 | 15480 | 7062 | 26583 | 4373 | 6499 |
| | 5518 | 10835 | 15962 | 3457 | 8543 | 5548 | | | | | | |
| | 25865 | 21223 | 10902 | 8772 | 10689 | 893 | | | | | | |
| 493: | 15893 | 11844 | 1505 | 23546 | 19494 | 4640 | 7577 | 7579 | 20205 | 17931 | 8734 | 1188 |
| | 16179 | 5729 | 20718 | 1468 | 11704 | 8079 | 14344 | 13549 | 950 | 9813 | 18817 | 13267 |
| | 1604 | 12816 | 4486 | 26827 | 12993 | 30030 | 26559 | 22058 | 8260 | 12582 | 19554 | 9312 |
| | 6144 | 11500 | 14245 | 5983 | 14808 | 3317 | 24059 | 9331 | 26894 | 13101 | 17777 | 12263 |
| | 4316 | 12538 | 18694 | 26225 | 4795 | 9193 | 9198 | 27007 | 12601 | 3099 | 23901 | 2238 |
| | 19116 | 3174 | 20066 | 11925 | 17353 | 6518 | 13945 | 18617 | 25114 | 11485 | 4185 | 14110 |
| | 2996 | 7173 | 25638 | 8216 | 14993 | | | | | | | |
| 494: | 16098 | 15226 | 18714 | 24108 | 6350 | 7236 | 7234 | 7191 | 7162 | 7089 | 6309 | 7232 |
| | 7059 | 7118 | 6247 | 7055 | 7047 | 6276 | 6306 | 6285 | 25251 | 20858 | 19676 | 15790 |
| | 14818 | 29560 | 13114 | 24301 | 25287 | 19135 | 2776 | 22433 | 5688 | 12526 | 19907 | 11843 |
| | 14207 | 27516 | 14194 | 23357 | 14012 | 27497 | 13982 | 11825 | 9171 | 12530 | 6571 | 18717 |
| | 16695 | 20592 | 20390 | 4443 | 12907 | 25851 | 8568 | 3343 | 8541 | 8693 | 8660 | 8690 |
| | 8609 | 8700 | 8564 | 8535 | 8649 | 8653 | 3490 | 8542 | 3377 | 3341 | 3351 | 3435 |
| | 3409 | 8657 | 8563 | 3412 | 3492 | 3431 | 8575 | 8537 | 3434 | 3486 | 8610 | 3408 |
| | 3345 | 3487 | 8534 | 3348 | 3386 | 3382 | 3383 | 3497 | 3464 | 3436 | 3456 | 3380 |
| | 8602 | 3410 | 8698 | 8505 | 8506 | 8572 | 8697 | 3439 | 3461 | 3466 | 3463 | 15615 |
| | 29180 | 11326 | 17427 | 10728 | 11025 | 19083 | 10200 | 25284 | 20672 | 29921 | 27651 | 8009 |
| | 15960 | 27164 | 13753 | 14336 | 14175 | 29938 | 9501 | 4194 | 18730 | 21718 | 29184 | 29161 |
| | 8414 | 27452 | 2072 | 15995 | 2423 | 21861 | 9426 | 11075 | 2155 | 1562 | 10490 | 12120 |
| | 24910 | 6861 | 13747 | 13766 | 8635 | 10815 | 20251 | 14219 | 6946 | 22093 | 5132 | 5683 |
| | 5681 | 4678 | 5428 | 4647 | 4650 | 5598 | 5425 | 4684 | 4652 | 5615 | 5620 | 5513 |
| | 4658 | 5507 | 5423 | 5645 | 5590 | 5535 | 5502 | 5649 | 5395 | 5639 | 5539 | 5536 |
| | 5511 | 5544 | 5593 | 5397 | 5618 | 5545 | 5479 | 5475 | 5589 | 5586 | 5614 | 5641 |
| | 25797 | 26573 | 22080 | 21661 | 13741 | 13743 | 18894 | 19030 | 15292 | 2713 | 14470 | 1383 |
| | 21652 | 14608 | 12364 | 20001 | 3815 | 19522 | 1810 | 24635 | 14056 | 13996 | 4126 | 8206 |

(continued)

SEQ ID NO: homolog SEQ ID NOs

| | | | | | | | | | | | | |
|---|---|---|---|---|---|---|---|---|---|---|---|---|
| | 13433 | 18014 | 18920 | 5059 | 29453 | 4668 | 1944 | 18675 | 9413 | 16607 | 10917 | 10920 |
| | 6464 | 3315 | 24361 | 28940 | 22672 | 8739 | 16441 | 20422 | 3500 | 30266 | 20265 | 7762 |
| | 2029 | 25972 | 20241 | 9611 | 16378 | 16372 | 18179 | 23163 | 6763 | 23957 | 23999 | 23126 |
| | 10819 | 23237 | 16693 | 24531 | 25791 | 12619 | 28760 | 2638 | 3170 | 17962 | 21940 | 21728 |
| | 12919 | 30034 | 4898 | 25085 | 4470 | 10509 | 29941 | 18680 | 9175 | 17803 | 29561 | 25194 |
| | 9352 | 20752 | 6359 | 18531 | 5691 | 28683 | 28371 | 2569 | 2902 | 6860 | 28577 | 22804 |
| | 22310 | 28818 | 2813 | 16579 | 28504 | 24882 | 10592 | 17166 | 22428 | 25437 | 15466 | 28925 |
| | 26032 | 10496 | 3703 | 27209 | 28938 | 4014 | 8872 | 23215 | 3046 | 18954 | 8098 | 5854 |
| | 5852 | 21242 | 18394 | 11229 | 20314 | 7054 | 27924 | 15599 | 29920 | 13150 | 6110 | 20394 |
| | 21307 | 19399 | 20796 | 2400 | 16691 | 27169 | 2789 | 9232 | 28499 | 9427 | 5788 | 3038 |
| | 6956 | 1209 | 14002 | 23344 | 11665 | | | | | | | |
| 495: | 14522 | 23157 | 24634 | 18185 | 16967 | 13763 | 27177 | 1547 | 2783 | 9599 | 2491 | 4035 |
| | 13679 | 20158 | 9134 | 9754 | 19567 | 1024 | 12606 | 16384 | 20382 | 21532 | 24833 | 1790 |
| | 19843 | 20476 | 7695 | 5643 | 6653 | 17562 | 5137 | 19977 | 22708 | 30204 | 26103 | 29134 |
| | 26442 | 6513 | 29842 | 8255 | 3964 | 26666 | 12518 | 22709 | 16083 | 9168 | 20207 | 27613 |
| | 11194 | 28341 | 24615 | 5361 | 25162 | 6052 | 29644 | 15007 | 4530 | | | |
| 496: | 14522 | 23157 | 24634 | 18185 | 16967 | 13763 | 27177 | 1547 | 2783 | 9599 | 2491 | 4035 |
| | 13679 | 20158 | 9134 | 9754 | 19567 | 1024 | 12606 | 16384 | 20382 | 21532 | 24833 | 1790 |
| | 19843 | 20476 | 7695 | 5643 | 6653 | 17562 | 5137 | 19977 | 22708 | 30204 | 26103 | 29134 |
| | 26442 | 6513 | 29842 | 8255 | 3964 | 26666 | 12518 | 22709 | 16083 | 9168 | 20207 | 27613 |
| | 11194 | 28341 | 24615 | 5361 | 25162 | 6052 | 29644 | 15007 | 4530 | | | |
| 497: | 22211 | 13210 | 16940 | 22879 | 26998 | 11928 | 3856 | 2634 | 19117 | 24160 | 1909 | 9662 |
| | 7014 | 6717 | 8960 | 22219 | 1493 | 20866 | 11296 | 7229 | 23995 | 29778 | 2062 | 24572 |
| | 7468 | 11265 | 22604 | 21001 | 9120 | 24303 | 20731 | 8513 | 22622 | 13613 | 16269 | 23938 |
| | 29494 | 1247 | | | | | | | | | | |
| 498: | 22211 | 13210 | 16940 | 22879 | 26998 | 11928 | 3856 | 2634 | 19117 | 24160 | 1909 | 9662 |
| | 7014 | 6717 | 8960 | 22219 | 1493 | 20866 | 11296 | 7229 | 23995 | 29778 | 2062 | 24572 |
| | 7468 | 11265 | 22604 | 21001 | 9120 | 24303 | 20731 | 8513 | 22622 | 13613 | 16269 | 23938 |
| | 29494 | 1247 | | | | | | | | | | |
| 499: | 25196 | 26289 | 18998 | 4236 | 9118 | 18490 | 8150 | 13027 | 6848 | 1015 | 12460 | 24776 |
| | 15664 | 2922 | 23746 | 8326 | 29102 | 1730 | 4791 | 22382 | 28835 | 18300 | 7256 | 13616 |
| | 24887 | 25568 | 16916 | 30085 | 26372 | 7330 | 822 | 26299 | 7209 | 6708 | 8656 | 15923 |
| | 8186 | 7275 | 6958 | 29661 | 14570 | 19106 | 10753 | 5588 | 2188 | 21806 | 4145 | 13696 |

(continued)

SEQ ID NO: homolog SEQ ID NOs

| SEQ ID NO | | | | | | | | | | | | |
|---|---|---|---|---|---|---|---|---|---|---|---|---|
| | 10437 | 28885 | 5084 | 27323 | 10856 | | | | | | | |
| 500: | 25196 | 26289 | 18998 | 4236 | 9118 | 18490 | 8150 | 13027 | 6848 | 1015 | 12460 | 24776 |
| | 15664 | 2922 | 23746 | 8326 | 29102 | 1730 | 4791 | 22382 | 28835 | 18300 | 7256 | 13616 |
| | 24887 | 25568 | 16916 | 30085 | 26372 | 7330 | 822 | 26299 | 7209 | 6708 | 8656 | 15923 |
| | 8186 | 7275 | 6958 | 29661 | 14570 | 19106 | 10753 | 5588 | 2188 | 21806 | 4145 | 13696 |
| | 10437 | 28885 | 5084 | 27323 | 10856 | | | | | | | |
| 501: | 27630 | 4128 | 22679 | 9031 | 7426 | 22027 | 16643 | 20229 | 25745 | 8311 | 9557 | 18537 |
| | 22258 | 7363 | 10846 | 4633 | 12513 | 12096 | 10190 | 25917 | 11604 | 20367 | 3070 | 29510 |
| | 28989 | 17964 | 14552 | 14736 | 3258 | 20172 | 19102 | 25035 | 21188 | 11050 | 13485 | 18215 |
| | 29225 | 842 | 8258 | 17244 | 18267 | 13461 | 10877 | 6836 | 26310 | 3109 | 5301 | 12580 |
| | 8175 | 15806 | 13435 | 10321 | 25006 | | | | | | | |
| 502: | 27630 | 4128 | 22679 | 9031 | 7426 | 22027 | 16643 | 20229 | 25745 | 8311 | 9557 | 18537 |
| | 22258 | 7363 | 10846 | 4633 | 12513 | 12096 | 10190 | 25917 | 11604 | 20367 | 3070 | 29510 |
| | 28989 | 17964 | 14552 | 14736 | 3258 | 20172 | 19102 | 25035 | 21188 | 11050 | 13485 | 18215 |
| | 29225 | 842 | 8258 | 17244 | 18267 | 13461 | 10877 | 6836 | 26310 | 3109 | 5301 | 12580 |
| | 8175 | 15806 | 13435 | 10321 | 25006 | | | | | | | |
| 503: | 15432 | 10133 | 4221 | 7022 | 3475 | 26468 | 6308 | 26984 | 21022 | 20656 | 20653 | 21690 |
| | 3565 | 7735 | 25125 | 6550 | 6530 | 2556 | 6521 | 756 | 758 | 21042 | 21046 | 21044 |
| | 18221 | 18223 | 4408 | 13526 | 6940 | 24247 | 9267 | 9781 | 19142 | 9014 | 13300 | 9297 |
| | 9263 | 5516 | 1712 | 2310 | 16714 | 18512 | 25345 | 20285 | 20292 | 25347 | 26712 | 26710 |
| | 29282 | 27076 | 10130 | 11905 | 7018 | 22429 | 3582 | 5050 | 10629 | 29011 | 18154 | 29457 |
| | 18030 | 10303 | 4232 | 11899 | 25633 | 21633 | 23192 | 17577 | 23432 | 10649 | 30257 | 6877 |
| | 13034 | 16957 | 21347 | 22835 | 21937 | 16948 | 28849 | 27707 | 2176 | 21105 | 25748 | 10322 |
| | 18338 | 14929 | 14932 | 14931 | 4602 | 5044 | 24358 | 17315 | 14799 | 14803 | 14754 | 12488 |
| | 13944 | 14717 | 14713 | 7104 | | | | | | | | |
| 504: | 15432 | 10133 | 4221 | 7022 | 3475 | 26468 | 6308 | 26984 | 21022 | 20656 | 20653 | 21690 |
| | 3565 | 7735 | 25125 | 6550 | 6530 | 2556 | 6521 | 756 | 758 | 21042 | 21046 | 21044 |
| | 18221 | 18223 | 4408 | 13526 | 6940 | 24247 | 9267 | 9781 | 19142 | 9014 | 13300 | 9297 |
| | 9263 | 5516 | 1712 | 2310 | 16714 | 18512 | 25345 | 20285 | 20292 | 25347 | 26712 | 26710 |
| | 29282 | 27076 | 10130 | 11905 | 7018 | 22429 | 3582 | 5050 | 10629 | 29011 | 18154 | 29457 |
| | 18030 | 10303 | 4232 | 11899 | 25633 | 21633 | 23192 | 17577 | 23432 | 10649 | 30257 | 6877 |
| | 13034 | 16957 | 21347 | 22835 | 21937 | 16948 | 28849 | 27707 | 2176 | 21105 | 25748 | 10322 |
| | 18338 | 14929 | 14932 | 14931 | 4602 | 5044 | 24358 | 17315 | 14799 | 14803 | 14754 | 12488 |

(continued)

SEQ ID NO: homolog SEQ ID NOs

| | | | | | | | | | | | | |
|---|---|---|---|---|---|---|---|---|---|---|---|---|
| | 13944 | 14717 | | 14713 | 7104 | | | | | | | |
| 505: | 6341 | 23042 | 7983 | 8856 | 20845 | 6914 | 16780 | 21678 | 1958 | 27864 | 16921 | 30232 |
| | 5344 | 29798 | 25007 | 12563 | 25400 | 8503 | 30248 | 3112 | 12900 | 3664 | 26110 | 28761 |
| | 3928 | 27469 | 28342 | 17635 | 4050 | 20544 | 23715 | 6188 | 8646 | 22948 | 17358 | 28959 |
| | 20142 | 1083 | 3226 | 7988 | 27517 | 20039 | 30315 | 4507 | 18180 | 10951 | 13180 | 5027 |
| | 5463 | 25246 | 23029 | 18175 | 12422 | 21693 | 13953 | 15593 | 19887 | 3799 | 4286 | 24475 |
| | 5845 | 25026 | 10244 | 21176 | 28475 | 4186 | 11348 | 26772 | 18942 | 2231 | 21515 | 12727 |
| | 16265 | 24488 | 27685 | 13287 | 4621 | 24660 | 23125 | 12410 | 17054 | | | |
| 506: | 22089 | 5863 | 19520 | 28147 | 30207 | 9025 | 4882 | 25535 | 15019 | 22434 | 7995 | 27952 |
| | 20928 | 18650 | 27297 | 20548 | 7981 | 27580 | 26883 | 16067 | 26529 | 21985 | 21004 | 11961 |
| | 6055 | 29021 | 21065 | 530 | 17882 | 9582 | 17693 | 3698 | 17319 | 19183 | 7228 | 17347 |
| | 16341 | 14303 | 2139 | 15361 | 25273 | 3830 | 11809 | 1835 | 19010 | 8478 | 20964 | 17385 |
| | 11339 | 3302 | 11582 | 22660 | 21637 | 21552 | 16256 | 3687 | 9629 | 13771 | 8784 | 12851 |
| | 3168 | 28364 | 15872 | 8870 | 13094 | 6001 | 26978 | 27150 | 27731 | 22494 | 10353 | 4914 |
| | 3952 | 19157 | 16485 | 21628 | 11277 | 3083 | 7038 | 17806 | 11668 | 5599 | 29815 | 28179 |
| | 25675 | 5934 | 13195 | 8827 | 7231 | 8560 | 4933 | 23272 | 7992 | 5669 | 7923 | 29171 |
| | 2017 | 21237 | 15981 | 22077 | 19785 | 1566 | 23594 | 29794 | 28728 | 22860 | 4453 | 24900 |
| | 25842 | 10448 | 18960 | 24161 | 25992 | 7972 | 25286 | 15018 | 19731 | 20295 | 23617 | 16803 |
| | 28733 | 8737 | 4620 | 14423 | 11548 | 11529 | 14074 | 16915 | 15935 | 22519 | 22733 | 8380 |
| | 12415 | 3052 | 26436 | 3396 | 10517 | 21526 | 29834 | 21264 | 4940 | 26945 | 18051 | 26880 |
| | 15839 | 9272 | 21458 | 28058 | 5358 | 26186 | 9861 | 8764 | 3411 | 29443 | 21646 | 29398 |
| | 26418 | 9176 | 7450 | 26930 | 2430 | 21980 | 7536 | 28235 | 29041 | 11927 | 20722 | 761 |
| | 24718 | 23016 | 26345 | 9493 | 8810 | 19985 | 2538 | 5406 | 21882 | 25769 | 26192 | 17855 |
| | 7792 | 24938 | 4144 | 11971 | 16823 | 20516 | 1490 | 8515 | 27359 | 15463 | 20112 | 1599 |
| | 26681 | 12502 | 6456 | 29349 | 10925 | 4944 | 16945 | 27385 | 23753 | 29914 | 11341 | 10570 |
| | 23918 | 17449 | 22874 | 3975 | 21566 | 925 | 9578 | 16879 | 8189 | 5074 | 22054 | 15065 |
| | 6906 | 14064 | 2596 | 15346 | 20816 | 16790 | 25811 | 14143 | 16969 | 7168 | 14407 | 22052 |
| | 21794 | 5819 | 26333 | 22963 | 27134 | 12351 | 17895 | 8685 | 19930 | 22779 | 16599 | 15788 |
| | 15605 | 14534 | 22440 | 26703 | 17644 | 10377 | 29825 | 17428 | 14815 | 25694 | 2676 | 12536 |
| | 15249 | 15247 | 23518 | 30199 | 12060 | 9442 | 2533 | 2087 | 25857 | | | |
| 507: | 22089 | 5863 | 19520 | 28147 | 30207 | 9025 | 4882 | 25535 | 15019 | 22434 | 7995 | 27952 |
| | 20928 | 18650 | 27297 | 20548 | 7981 | 27580 | 26883 | 16067 | 26529 | 21985 | 21004 | 11961 |
| | 6055 | 29021 | 21065 | 530 | 17882 | 9582 | 17693 | 3698 | 17319 | 19183 | 7228 | 17347 |

SEQ ID NO: homolog SEQ ID NOs

| | | | | | | | | | | | | |
|---|---|---|---|---|---|---|---|---|---|---|---|---|
| | 16341 | 14303 | 2139 | 15361 | 25273 | 3830 | 11809 | 1835 | 19010 | 8478 | 20964 | 17385 |
| | 11339 | 3302 | 11582 | 22660 | 21637 | 21552 | 16256 | 3687 | 9629 | 13771 | 8784 | 12851 |
| | 3168 | 28364 | 15872 | 8870 | 13094 | 6001 | 26978 | 27150 | 27731 | 22494 | 10353 | 4914 |
| | 3952 | 19157 | 16485 | 21628 | 11277 | 3083 | 7038 | 17806 | 11668 | 5599 | 29815 | 28179 |
| | 25675 | 5934 | 13195 | 8827 | 7231 | 8560 | 4933 | 23272 | 7992 | 5669 | 7923 | 29171 |
| | 2017 | 21237 | 15981 | 22077 | 19785 | 1566 | 23594 | 29794 | 28728 | 22860 | 4453 | 24900 |
| | 25842 | 10448 | 18960 | 24161 | 25992 | 7972 | 25286 | 15018 | 19731 | 20295 | 23617 | 16803 |
| | 28733 | 8737 | 4620 | 14423 | 11548 | 11529 | 14074 | 16915 | 15935 | 22519 | 22733 | 8380 |
| | 12415 | 3052 | 26436 | 3396 | 10517 | 21526 | 29834 | 21264 | 4940 | 26945 | 18051 | 26880 |
| | 15839 | 9272 | 21458 | 28058 | 5358 | 26186 | 9861 | 8764 | 3411 | 29443 | 21646 | 29398 |
| | 26418 | 9176 | 7450 | 26930 | 2430 | 21980 | 7536 | 28235 | 29041 | 11927 | 20722 | 761 |
| | 24718 | 23016 | 26345 | 9493 | 8810 | 19985 | 2538 | 5406 | 21882 | 25769 | 26192 | 17855 |
| | 7792 | 24938 | 4144 | 11971 | 16823 | 20516 | 1490 | 8515 | 27359 | 15463 | 20112 | 1599 |
| | 26681 | 12502 | 6456 | 29349 | 10925 | 4944 | 16945 | 27385 | 23753 | 29914 | 11341 | 10570 |
| | 23918 | 17449 | 22874 | 3975 | 21566 | 925 | 9578 | 16879 | 8189 | 5074 | 22054 | 15065 |
| | 6906 | 14064 | 2596 | 15346 | 20816 | 16790 | 25811 | 14143 | 16969 | 7168 | 14407 | 22052 |
| | 21794 | 5819 | 26333 | 22963 | 27134 | 12351 | 17895 | 8685 | 19930 | 22779 | 16599 | 15788 |
| | 15605 | 14534 | 22440 | 26703 | 17644 | 10377 | 29825 | 17428 | 14815 | 25694 | 2676 | 12536 |
| | 15249 | 15247 | 23518 | 30199 | 12060 | 9442 | 2533 | 2087 | 25857 | | | |
| 508: | 22089 | 5863 | 19520 | 28147 | 30207 | 9025 | 4882 | 25535 | 15019 | 22434 | 7995 | 27952 |
| | 20928 | 18650 | 27297 | 20548 | 7981 | 27580 | 26883 | 16067 | 26529 | 21985 | 21004 | 11961 |
| | 6055 | 29021 | 21065 | 530 | 17882 | 9582 | 17693 | 3698 | 17319 | 19183 | 7228 | 17347 |
| | 16341 | 14303 | 2139 | 15361 | 25273 | 3830 | 11809 | 1835 | 19010 | 8478 | 20964 | 17385 |
| | 11339 | 3302 | 11582 | 22660 | 21637 | 21552 | 16256 | 3687 | 9629 | 13771 | 8784 | 12851 |
| | 3168 | 28364 | 15872 | 8870 | 13094 | 6001 | 26978 | 27150 | 27731 | 22494 | 10353 | 4914 |
| | 3952 | 19157 | 16485 | 21628 | 11277 | 3083 | 7038 | 17806 | 11668 | 5599 | 29815 | 28179 |
| | 25675 | 5934 | 13195 | 8827 | 7231 | 8560 | 4933 | 23272 | 7992 | 5669 | 7923 | 29171 |
| | 2017 | 21237 | 15981 | 22077 | 19785 | 1566 | 23594 | 29794 | 28728 | 22860 | 4453 | 24900 |
| | 25842 | 10448 | 18960 | 24161 | 25992 | 7972 | 25286 | 15018 | 19731 | 20295 | 23617 | 16803 |
| | 28733 | 8737 | 4620 | 14423 | 11548 | 11529 | 14074 | 16915 | 15935 | 22519 | 22733 | 8380 |
| | 12415 | 3052 | 26436 | 3396 | 10517 | 21526 | 29834 | 21264 | 4940 | 26945 | 18051 | 26880 |
| | 15839 | 9272 | 21458 | 28058 | 5358 | 26186 | 9861 | 8764 | 3411 | 29443 | 21646 | 29398 |
| | 26418 | 9176 | 7450 | 26930 | 2430 | 21980 | 7536 | 28235 | 29041 | 11927 | 20722 | 761 |

(continued)

SEQ ID NO: homolog SEQ ID NOs

| SEQ ID NO | | | | | | | | | | | | |
|---|---|---|---|---|---|---|---|---|---|---|---|---|
| | 24718 | 23016 | 26345 | 9493 | 8810 | 19985 | 2538 | 5406 | 21882 | 25769 | 26192 | 17855 |
| | 7792 | 24938 | 4144 | 11971 | 16823 | 20516 | 1490 | 8515 | 27359 | 15463 | 20112 | 1599 |
| | 26681 | 12502 | 6456 | 29349 | 10925 | 4944 | 16945 | 27385 | 23753 | 29914 | 11341 | 10570 |
| | 23918 | 17449 | 22874 | 3975 | 21566 | 925 | 9578 | 16879 | 8189 | 5074 | 22054 | 15065 |
| | 6906 | 14064 | 2596 | 15346 | 20816 | 16790 | 25811 | 14143 | 16969 | 7168 | 14407 | 22052 |
| | 21794 | 5819 | 26333 | 22963 | 27134 | 12351 | 17895 | 8685 | 19930 | 22779 | 16599 | 15788 |
| | 15605 | 14534 | 22440 | 26703 | 17644 | 10377 | 29825 | 17428 | 14815 | 25694 | 2676 | 12536 |
| | 15249 | 15247 | 23518 | 30199 | 12060 | 9442 | 2533 | 2087 | 25857 | | | |
| 509: | 19520 | 22089 | 5863 | 25418 | 8136 | 30207 | 28147 | 2111 | 23798 | 9025 | 22730 | 4882 |
| | 25535 | 15019 | 7995 | 27952 | 18650 | 20928 | 21213 | 20548 | 7981 | 23339 | 14195 | 12936 |
| | 8556 | 27580 | 26883 | 16067 | 11961 | 6055 | 21462 | 21065 | 530 | 9582 | 12854 | 25248 |
| | 3905 | 3698 | 17319 | 19183 | 7228 | 17708 | 28553 | 19451 | 5970 | 14452 | 2139 | 15361 |
| | 7668 | 25273 | 3830 | 11809 | 19010 | 7318 | 17385 | 3302 | 11339 | 22660 | 21637 | 21552 |
| | 16256 | 17380 | 16668 | 3687 | 4939 | 9629 | 8784 | 23464 | 14226 | 12851 | 17838 | 10838 |
| | 15436 | 3168 | 28364 | 14918 | 15872 | 2982 | 8870 | 21771 | 27718 | 13094 | 6001 | 26978 |
| | 6323 | 20282 | 27731 | 22494 | 1885 | 10353 | 21749 | 19157 | 4636 | 21628 | 18429 | 28890 |
| | 20529 | 11277 | 3060 | 10198 | 7038 | 3083 | 17806 | 5599 | 29815 | 28179 | 8840 | 6071 |
| | 25675 | 6029 | 12558 | 5934 | 14122 | 1099 | 13195 | 8827 | 4933 | 7992 | 5669 | 7923 |
| | 2661 | 27431 | 8897 | 29171 | 22077 | 5927 | 18876 | 19785 | 1566 | 23594 | 29794 | 28728 |
| | 13099 | 3611 | 22860 | 4453 | 24900 | 25842 | 26400 | 25992 | 24161 | 7972 | 25286 | 23617 |
| | 16803 | 10800 | 28733 | 14423 | 4620 | 7043 | 26747 | 29751 | 11529 | 12334 | 14074 | 16915 |
| | 15935 | 22519 | 8380 | 3052 | 3396 | 26436 | 22179 | 10517 | 21526 | 21264 | 16049 | 9702 |
| | 4940 | 3049 | 5468 | 26945 | 18051 | 24331 | 16089 | 15839 | 16580 | 9272 | 28233 | 15058 |
| | 21458 | 8914 | 7040 | 28058 | 8689 | 5358 | 26186 | 18901 | 2055 | 13556 | 9861 | 27584 |
| | 8764 | 3411 | 14780 | 21646 | 28639 | 16642 | 15494 | 23279 | 29398 | 26418 | 9176 | 7441 |
| | 26930 | 7450 | 2430 | 21980 | 26765 | 7536 | 29041 | 28235 | 11927 | 1908 | 20722 | 761 |
| | 26345 | 20449 | 2538 | 5406 | 26192 | 10925 | 23918 | 17449 | 3975 | 19903 | 8189 | 5074 |
| | 2596 | 25811 | 20558 | 22052 | 16969 | 28839 | 23316 | 27134 | 8685 | 17644 | 17895 | 22220 |
| | 12413 | 817 | 5456 | 7516 | 25694 | 25624 | 8593 | 2675 | 2676 | 12512 | 12536 | 20372 |
| | 15249 | 15247 | 17555 | 28010 | 23518 | 25789 | 27449 | 5342 | 30199 | 24006 | 5771 | 25446 |
| | 9442 | 9361 | 2533 | 2087 | 13670 | 13139 | 25857 | 635 | 510 | 26696 | | |
| 510: | 19520 | 22089 | 5863 | 25418 | 8136 | 30207 | 28147 | 2111 | 23798 | 9025 | 22730 | 4882 |
| | 25535 | 15019 | 7995 | 27952 | 18650 | 20928 | 21213 | 20548 | 7981 | 23339 | 14195 | 12936 |

SEQ ID NO: homolog SEQ ID NOs

| | | | | | | | | | | | | |
|---|---|---|---|---|---|---|---|---|---|---|---|---|
| | 8556 | 27580 | 26883 | 16067 | 11961 | 6055 | 21462 | 21065 | 530 | 9582 | 12854 | 25248 |
| | 3905 | 3698 | 17319 | 19183 | 7228 | 17708 | 28553 | 19451 | 5970 | 14452 | 2139 | 15361 |
| | 7668 | 25273 | 3830 | 11809 | 19010 | 7318 | 17385 | 3302 | 11339 | 22660 | 21637 | 21552 |
| | 16256 | 17380 | 16668 | 3687 | 4939 | 9629 | 8784 | 14226 | 12851 | 17838 | 10838 | 15436 |
| | 3168 | 28364 | 14918 | 15872 | 2982 | 8870 | 21771 | 27718 | 13094 | 6001 | 26978 | 20010 |
| | 6323 | 20282 | 27731 | 22494 | 1885 | 10353 | 21749 | 19157 | 4636 | 21628 | 18429 | 28890 |
| | 20529 | 11277 | 3060 | 10198 | 7038 | 3083 | 17806 | 5599 | 29815 | 28179 | 8840 | 6071 |
| | 25675 | 6029 | 12558 | 5934 | 14122 | 1099 | 13195 | 8827 | 4933 | 7992 | 5669 | 7923 |
| | 2661 | 27431 | 8897 | 29171 | 22077 | 5927 | 18876 | 19785 | 1566 | 23594 | 29794 | 28728 |
| | 13099 | 3611 | 22860 | 4453 | 24900 | 25842 | 26400 | 25992 | 24161 | 7972 | 25286 | 23617 |
| | 16803 | 10800 | 28733 | 14423 | 4620 | 7043 | 26747 | 29751 | 11529 | 12334 | 14074 | 16915 |
| | 15935 | 22519 | 8380 | 3052 | 3396 | 26436 | 22179 | 10517 | 21526 | 21264 | 16049 | 9702 |
| | 4940 | 3049 | 5468 | 26945 | 18051 | 24331 | 16089 | 15839 | 16580 | 9272 | 28233 | 15058 |
| | 21458 | 8914 | 7040 | 28058 | 8689 | 5358 | 26186 | 18901 | 2055 | 13556 | 9861 | 27584 |
| | 8764 | 3411 | 14780 | 21646 | 28639 | 16642 | 15494 | 23279 | 29398 | 26418 | 9176 | 7441 |
| | 26930 | 7450 | 2430 | 21980 | 26765 | 7536 | 29041 | 28235 | 11927 | 1908 | 20722 | 761 |
| | 26345 | 20449 | 2538 | 5406 | 26192 | 10925 | 23918 | 17449 | 3975 | 19903 | 8189 | 5074 |
| | 2596 | 25811 | 20558 | 22052 | 16969 | 28839 | 23316 | 27134 | 8685 | 17644 | 17895 | 19930 |
| | 14534 | 22220 | 12413 | 817 | 5456 | 7516 | 25694 | 25624 | 8593 | 2675 | 2676 | 12512 |
| | 12536 | 20372 | 15249 | 15247 | 17555 | 28010 | 23518 | 25789 | 27449 | 5342 | 30199 | 24006 |
| | 5771 | 25446 | 9442 | 9361 | 2533 | 2087 | 13670 | 13139 | 25857 | 509 | | |
| 511: | 17739 | 2421 | 6224 | 18395 | 16271 | 14852 | 12439 | 26026 | 26687 | 3594 | 22151 | 24353 |
| | 18411 | 1803 | 20587 | 9627 | 12895 | 16464 | 25569 | 19221 | 25711 | 5385 | 1307 | 1622 |
| | 1016 | 4582 | 10659 | 25198 | 15229 | 27474 | 29388 | 17007 | 15821 | 30190 | 21315 | 17090 |
| | 12471 | 23940 | 2809 | 6111 | 1863 | 22920 | 5727 | 22858 | 1411 | 15744 | 18444 | 9820 |
| | 5709 | 22059 | 17990 | 20740 | 13714 | 5917 | 10991 | 24740 | 10541 | 18448 | 10447 | 1139 |
| | 17657 | 9652 | 14105 | 22238 | 9812 | 6193 | 24922 | 24015 | 23848 | 28931 | 8566 | 12171 |
| | 11772 | 22449 | 24979 | 15544 | 26005 | | | | | | | |
| 512: | 19401 | 16568 | 16621 | 16624 | 28556 | 28537 | 1190 | 24558 | 1243 | 1245 | 28557 | 1191 |
| | 1225 | 1224 | 1227 | 18926 | 28900 | 17723 | 17729 | 27213 | 18819 | 17726 | 23113 | 28256 |
| | 5664 | 28260 | 28352 | 21946 | 19656 | 19633 | 19658 | 23206 | 21109 | 29408 | 14966 | 9672 |
| | 18647 | 10389 | 24243 | 15273 | 15271 | 18326 | 20405 | 4526 | 22996 | 1123 | 18567 | 28163 |
| | 10302 | 17066 | 24496 | 1157 | 22701 | 24795 | 13940 | 27027 | 8238 | 14158 | 26143 | 27276 |

SEQ ID NO: homolog SEQ ID NOs

| SEQ ID NO: | | | | | | | | | | | | |
|---|---|---|---|---|---|---|---|---|---|---|---|---|
| | 24895 | 1322 | 22534 | 19049 | 22536 | 18035 | 10059 | 6779 | 10074 | 10077 | 8091 | 17700 |
| | 18681 | 24880 | 23033 | 17869 | 29432 | 29438 | 2775 | 27675 | 2207 | 2214 | 24368 | 5585 |
| | 3867 | 24293 | 13578 | 24606 | 14656 | 9573 | 3048 | 21483 | 29428 | 29653 | 7871 | 7241 |
| | 22932 | 23102 | 27329 | 7917 | 15212 | 14640 | 22348 | 10463 | 21216 | 28514 | 17818 | 2397 |
| | 6268 | 11977 | 16676 | 20767 | 8782 | 20234 | 24733 | 16072 | 25501 | 27534 | 3043 | 8676 |
| | 10038 | 15819 | 12539 | 29093 | 814 | 1081 | 17566 | 17751 | 7878 | 29554 | 30077 | 3713 |
| | 5372 | 17397 | 7895 | 26029 | 9884 | 16251 | 8806 | 17246 | 29651 | 9042 | 20106 | 20126 |
| | 20228 | 20261 | 20260 | 24422 | 4552 | 14820 | 1891 | 19378 | 23502 | 5869 | 21952 | 9138 |
| | 7816 | 6991 | 28980 | 12712 | 20277 | 17292 | 4819 | 29167 | 4180 | 1197 | 4828 | 10471 |
| | 19871 | 3328 | 12515 | 1648 | 27696 | 30123 | 11340 | 27094 | 16860 | 2568 | 22172 | 28700 |
| | 2226 | 20374 | 29756 | 15314 | 10652 | 3712 | 6737 | 1379 | 2343 | 6372 | 13979 | 28439 |
| | 13018 | 8733 | 23973 | 27110 | 5084 | 17536 | 21649 | 1943 | 13008 | 8344 | 30042 | 14850 |
| | 16196 | 2222 | 7742 | 15859 | 27028 | 1304 | 11488 | 18017 | 7879 | 9980 | 7881 | 13855 |
| | 10926 | 10929 | 14779 | | | | | | | | | |
| 513: | 27837 | 22637 | 15934 | 5207 | 12562 | 13851 | 15562 | 1966 | 1766 | 17280 | 2572 | 5504 |
| | 29128 | 16261 | 11128 | 4720 | 4149 | 11049 | 2868 | 2579 | 4826 | 15659 | 12427 | 23540 |
| | 23550 | 4423 | 8196 | 8192 | 20743 | 15020 | 17919 | 4053 | 10803 | 13050 | 17329 | 21503 |
| | 17733 | 2191 | 11468 | 17438 | 12349 | 26585 | 18408 | 13538 | 6159 | 17465 | 17778 | 8851 |
| | 19027 | 8854 | 23202 | 3108 | 8844 | 4976 | 8849 | 8820 | 18931 | 10635 | 22121 | 19696 |
| | 19007 | 22539 | 24644 | 23485 | 19069 | 3577 | 20539 | 5993 | 4970 | 3506 | 10735 | 28013 |
| 514: | 8553 | | | | | | | | | | | |
| 515: | 5207 | 12562 | 13851 | 15562 | 1966 | 1766 | 17280 | 2572 | 6883 | 29128 | 16261 | 11128 |
| | 4720 | 15057 | 19536 | 11049 | 2868 | 4826 | 15659 | 6801 | 12427 | 23540 | 3983 | 20988 |
| | 23550 | 8192 | 8196 | 14622 | 8851 | 17101 | 10962 | 19027 | 8854 | 2466 | 21403 | 23757 |
| | 8844 | 4976 | 8849 | 8820 | 18931 | 10635 | 23534 | 28211 | 22121 | 4860 | 2541 | 19696 |
| | 19007 | 24644 | 23485 | 19069 | 3577 | 20539 | 23704 | 5993 | 4970 | 19588 | | |
| 516: | 5207 | 12562 | 13851 | 15562 | 1966 | 1766 | 17280 | 2572 | 6883 | 29128 | 16261 | 11128 |
| | 4720 | 15057 | 19536 | 11049 | 2868 | 4826 | 15659 | 6801 | 12427 | 23540 | 3983 | 20988 |
| | 23550 | 8192 | 8196 | 14622 | 8851 | 17101 | 10962 | 19027 | 8854 | 2466 | 21403 | 23757 |
| | 8844 | 4976 | 8849 | 8820 | 18931 | 10635 | 23534 | 28211 | 22121 | 4860 | 2541 | 19696 |
| | 19007 | 24644 | 23485 | 19069 | 3577 | 20539 | 23704 | 5993 | 4970 | 19588 | | |
| 517: | 21785 | 24750 | 30072 | 27780 | 19911 | 6192 | 1351 | 8899 | 21904 | 29507 | 25453 | 21147 |
| | 20332 | 25681 | 12469 | 1003 | 19868 | 27126 | 5966 | 21573 | 4090 | 6337 | 4094 | 1141 |

(continued)

| SEQ ID NO: | homolog SEQ ID NOs | | | | | | | | | | | |
|---|---|---|---|---|---|---|---|---|---|---|---|---|
|  | 15460 | 21451 | 24868 | 29050 | 11954 | 1581 | 9351 | 10508 | 26503 | 11740 | 29313 | 26193 |
|  | 25081 | 29869 | 21110 | 28508 | 11914 | 11506 | 29094 | 14044 | 15306 | 22726 | 27415 | 13499 |
|  | 15478 | 9566 | 7757 | 25031 | 10310 | 9450 | 25055 | 18454 | 15992 | 20682 | 4211 | 7321 |
|  | 890 | 28759 | 26347 | 14358 | 14702 | 19312 | 27759 | 26968 | 26537 | 10221 | 23974 | 6239 |
|  | 29274 | 4070 | 5711 | 29129 | 21303 | 1067 | 26463 | 11018 | 15428 | 28328 | 12740 | 5780 |
|  | 20340 | 17227 |  |  |  |  |  |  |  |  |  |  |
| 518: | 25087 | 4466 | 12338 | 19701 | 26953 | 2708 | 5121 | 26381 | 28078 | 25433 | 25404 | 26582 |
|  | 4364 | 29799 | 23905 | 12018 | 1092 | 29124 | 4874 | 11702 | 23431 | 2564 | 16674 | 22235 |
|  | 17064 | 29422 | 7980 | 19210 | 14361 | 27053 | 20488 | 11884 | 4781 | 12487 | 19417 | 3232 |
|  | 10928 | 5534 | 11748 | 22118 | 22837 | 11316 | 9090 | 22387 | 23448 | 5263 | 6549 | 15244 |
|  | 27670 | 15245 | 18555 | 6267 | 10374 | 7782 | 7780 | 6051 | 25741 | 6699 | 25038 | 25181 |
|  | 11435 | 4730 | 21818 | 11000 | 5232 | 5200 | 15866 | 2445 | 2833 | 5894 | 29405 | 20910 |
|  | 18352 | 2086 | 2088 | 27619 | 8177 | 9234 | 5170 | 25798 | 27024 | 7729 | 17632 | 11447 |
|  | 20572 | 2583 | 21235 | 5098 | 5273 | 3785 | 26016 | 21795 | 12857 | 9715 | 20635 | 11751 |
|  | 6440 | 12382 | 24238 | 16569 | 25465 | 27532 | 3428 | 8709 | 15161 | 11835 | 24911 | 2820 |
|  | 3587 | 2956 | 12061 | 24348 | 24428 | 25373 | 10416 | 19400 | 22154 | 1816 | 7503 | 19120 |
|  | 18754 | 11237 | 7740 | 2020 | 28246 | 20495 | 15016 | 23325 | 25683 | 2562 | 898 | 17127 |
|  | 13122 | 13739 | 18979 | 20925 | 17460 | 25436 | 2991 | 4966 | 16253 | 7959 | 3829 | 13727 |
|  | 29548 | 13853 | 15072 | 25030 | 10355 | 8470 | 8531 | 21459 | 18496 | 12679 | 12193 | 11824 |
|  | 23539 | 10938 | 9268 | 29758 | 18977 | 18955 | 17868 | 7597 | 25135 | 7390 | 7795 | 2772 |
|  | 27275 | 8571 | 27564 | 2561 | 14431 | 19910 | 12022 | 2273 | 27911 | 27003 | 9378 | 15561 |
|  | 7886 | 7085 | 15964 | 6953 | 28258 | 21138 | 21163 | 16011 | 9244 | 15618 | 21996 | 28661 |
|  | 24261 | 8554 | 24255 | 5103 | 16300 | 20266 | 17957 | 15667 | 13722 | 14877 | 26215 | 18317 |
|  | 21384 | 25150 | 24101 | 24103 | 3155 | 4028 | 13387 | 5198 | 13107 | 26495 | 19820 | 22338 |
|  | 20006 | 21154 | 2548 | 1386 | 15932 | 26923 | 26861 | 27953 | 3671 | 9480 | 10287 | 18224 |
|  | 25066 | 9002 | 18139 | 25032 | 20267 | 12034 | 7763 | 27235 | 1517 | 10135 | 12695 | 24830 |
|  | 4867 | 29838 | 11511 | 4848 | 13560 | 28445 | 20979 | 25616 | 15476 | 14740 | 7950 | 22826 |
|  | 2663 | 26913 | 27711 | 4016 | 18426 | 24388 | 1915 | 14345 | 27673 | 7169 | 21261 | 18790 |
|  | 12745 | 15078 | 14792 | 19357 | 14507 | 20352 | 19783 | 8898 | 8962 | 5553 | 9024 | 20664 |
|  | 731 | 18728 | 29550 | 5422 | 9032 | 21425 | 25610 | 5946 | 11742 | 17789 | 27743 | 13411 |
|  | 3236 | 27645 | 13442 | 25336 | 12448 | 11070 | 12833 | 13651 | 18584 | 8722 | 6662 | 792 |
|  | 15911 | 848 | 23981 | 14565 | 22574 | 15687 | 1549 | 1451 | 25837 | 19012 | 16170 | 25496 |
|  | 26763 | 29518 | 20596 | 14791 | 18985 | 26010 | 20187 | 26311 | 16171 | 16816 | 11523 | 10855 |

(continued)

SEQ ID NO: homolog SEQ ID NOs

| | | | | | | | | | | | |
|---|---|---|---|---|---|---|---|---|---|---|---|
| | 21769 | 20331 | 11617 | 15632 | 16529 | 29480 | 2973 | 29843 | 18822 | 21829 | 4444 | 2296 |
| | 24649 | 15500 | 15760 | 5836 | 21450 | 17105 | 29245 | 16272 | 5322 | 3783 | 13119 | 4420 |
| | 2830 | 15845 | 10844 | 26451 | 24587 | 22240 | 23341 | 29139 | 21826 | 24624 | 867 | 22764 |
| | 20512 | 9639 | 17900 | 20517 | 29431 | 20515 | 28566 | 28562 | 14011 | 28754 | 25004 | 11760 |
| | 9809 | 8047 | 10985 | 17683 | 23787 | 26542 | 29805 | 6769 | 24537 | 21790 | 824 | 21793 |
| | 12024 | 896 | 13656 | 17998 | 25989 | 22259 | 8966 | 29971 | 2044 | 29303 | 981 | 12478 |
| | 6774 | 6776 | 1088 | 3231 | 11719 | 4086 | 18765 | 5563 | 8455 | 23624 | 28746 | 27835 |
| | 26836 | 27831 | 26870 | 21282 | 15952 | 22026 | 3296 | 4346 | 6583 | 23455 | | |
| 519: | 25087 | 4466 | 12338 | 19701 | 26953 | 2708 | 5121 | 26381 | 28078 | 25433 | 25404 | 26582 |
| | 4364 | 29799 | 23905 | 12018 | 1092 | 29124 | 4874 | 11702 | 23431 | 2564 | 16674 | 22235 |
| | 17064 | 29422 | 7980 | 19210 | 14361 | 27053 | 20488 | 11884 | 4781 | 12487 | 19417 | 3232 |
| | 10928 | 5534 | 11748 | 22118 | 22837 | 11316 | 9090 | 22387 | 23448 | 5263 | 6549 | 15244 |
| | 27670 | 15245 | 18555 | 6267 | 10374 | 7782 | 7780 | 6051 | 25741 | 6699 | 25038 | 25181 |
| | 11435 | 4730 | 21818 | 11000 | 5232 | 5200 | 15866 | 2445 | 2833 | 5894 | 29405 | 20910 |
| | 18352 | 2086 | 2088 | 27619 | 8177 | 9234 | 5170 | 25798 | 27024 | 7729 | 17632 | 11447 |
| | 20572 | 2583 | 21235 | 5098 | 5273 | 3785 | 26016 | 21795 | 12857 | 9715 | 20635 | 11751 |
| | 6440 | 12382 | 24238 | 16569 | 25465 | 27532 | 3428 | 8709 | 15161 | 11835 | 24911 | 2820 |
| | 3587 | 2956 | 12061 | 24348 | 24428 | 25373 | 10416 | 19400 | 22154 | 1816 | 7503 | 19120 |
| | 18754 | 11237 | 7740 | 2020 | 28246 | 20495 | 15016 | 23325 | 25683 | 2562 | 898 | 17127 |
| | 13122 | 13739 | 18979 | 20925 | 17460 | 25436 | 2991 | 4966 | 16253 | 7959 | 3829 | 13727 |
| | 29548 | 13853 | 15072 | 25030 | 10355 | 8470 | 8531 | 21459 | 18496 | 12679 | 12193 | 11824 |
| | 23539 | 10938 | 9268 | 29758 | 18977 | 18955 | 17868 | 7597 | 25135 | 7390 | 7795 | 2772 |
| | 27275 | 8571 | 27564 | 2561 | 14431 | 19910 | 12022 | 2273 | 27911 | 27003 | 9378 | 15561 |
| | 7886 | 7085 | 15964 | 6953 | 28258 | 21138 | 21163 | 16011 | 9244 | 15618 | 21996 | 28661 |
| | 24261 | 8554 | 24255 | 5103 | 16300 | 20266 | 17957 | 15667 | 13722 | 14877 | 26215 | 18317 |
| | 21384 | 25150 | 24101 | 24103 | 3155 | 4028 | 13387 | 5198 | 13107 | 26495 | 19820 | 22338 |
| | 20006 | 21154 | 2548 | 1386 | 15932 | 26923 | 26861 | 27953 | 3671 | 9480 | 10287 | 18224 |
| | 25066 | 9002 | 18139 | 25032 | 20267 | 12034 | 7763 | 27235 | 1517 | 10135 | 12695 | 24830 |
| | 4867 | 29838 | 11511 | 4848 | 13560 | 28445 | 20979 | 25616 | 15476 | 14740 | 7950 | 22826 |
| | 2663 | 26913 | 27711 | 4016 | 18426 | 24388 | 1915 | 14345 | 27673 | 7169 | 21261 | 18790 |
| | 12745 | 15078 | 14792 | 19357 | 14507 | 20352 | 19783 | 8898 | 8962 | 5553 | 9024 | 20664 |
| | 731 | 18728 | 29550 | 5422 | 9032 | 21425 | 25610 | 5946 | 11742 | 17789 | 27743 | 13411 |
| | 3236 | 27645 | 13442 | 25336 | 12448 | 11070 | 12833 | 13651 | 18584 | 8722 | 6662 | 792 |

(continued)

| SEQ ID NO: | homolog SEQ ID NOs | | | | | | | | | | |
|---|---|---|---|---|---|---|---|---|---|---|---|
| 15911 | 848 | 23981 | 14565 | 22574 | 15687 | 1549 | 1451 | 25837 | 19012 | 16170 | 25496 |
| 26763 | 29518 | 20596 | 14791 | 18985 | 26010 | 20187 | 26311 | 16171 | 16816 | 11523 | 10855 |
| 21769 | 20331 | 11617 | 15632 | 16529 | 29480 | 2973 | 29843 | 18822 | 21829 | 4444 | 2296 |
| 24649 | 15500 | 15760 | 5836 | 21450 | 17105 | 29245 | 16272 | 5322 | 3783 | 13119 | 4420 |
| 2830 | 15845 | 10844 | 26451 | 24587 | 22240 | 23341 | 29139 | 21826 | 24624 | 867 | 22764 |
| 20512 | 9639 | 17900 | 20517 | 29431 | 20515 | 28566 | 28562 | 14011 | 28754 | 25004 | 11760 |
| 9809 | 8047 | 10985 | 17683 | 23787 | 26542 | 29805 | 6769 | 24537 | 21790 | 824 | 21793 |
| 12024 | 896 | 13656 | 17998 | 25989 | 22259 | 8966 | 29971 | 2044 | 29303 | 981 | 12478 |
| 6774 | 6776 | 1088 | 3231 | 11719 | 4086 | 18765 | 5563 | 8455 | 23624 | 28746 | 27835 |
| 26836 | 27831 | 26870 | 21282 | 15952 | 22026 | 3296 | 4346 | 6583 | 23455 | | |
| 520: 6825 | 18619 | 25702 | 10973 | 12191 | 11517 | 18480 | 13664 | 7813 | 2142 | 29829 | 29870 |
| 1847 | 2622 | 13530 | 8321 | 21846 | 15362 | 18656 | 18662 | 17021 | 21358 | 8495 | 9685 |
| 27989 | 27990 | 8838 | 9281 | 26271 | 21901 | 4480 | 30140 | 9266 | 19714 | 26726 | 913 |
| 15752 | 1722 | 6270 | 17378 | 17374 | 3693 | 25639 | 23222 | 23220 | 13021 | 7227 | 13246 |
| 22864 | 5097 | 9992 | 5155 | 6205 | 28651 | 15944 | 5893 | 3093 | 21132 | 29772 | 17172 |
| 1208 | 11738 | 11848 | 2100 | 12972 | 11342 | 21491 | 23286 | 28892 | 17928 | 10535 | 14739 |
| 20750 | 27512 | 17031 | 18440 | 16316 | 3645 | 20648 | 6254 | 2324 | 1699 | 10297 | 11833 |
| 11812 | 6839 | 8761 | 9738 | 7031 | 13552 | 7494 | 7500 | 10072 | 2220 | 15685 | 12216 |
| 11141 | 14100 | 4277 | 1497 | 26343 | 15092 | 30006 | 15093 | 16304 | 7009 | 30063 | 24019 |
| 14658 | 13469 | 19669 | 21140 | 8621 | 21719 | 25788 | 12392 | 22064 | 23663 | 25719 | 17303 |
| 17479 | 26563 | 14961 | 12830 | 25538 | 21610 | 25423 | 3624 | 9613 | 15889 | 17461 | 12297 |
| 18416 | 18756 | 19826 | 22968 | 16868 | 14104 | 11721 | 4062 | 27131 | 1506 | 27143 | 5309 |
| 10082 | 9391 | 16080 | 17925 | 3864 | 7532 | 13965 | 9076 | 17404 | 14873 | 30222 | 13407 |
| 7361 | 23620 | 7135 | 11248 | 23675 | 25552 | 8570 | 25015 | 17144 | 1500 | 16907 | 24124 |
| 6587 | 8599 | 7215 | 21972 | 19039 | 13562 | 13563 | 30137 | 21894 | 5568 | 3745 | 16275 |
| 18834 | 11770 | 1995 | 816 | 15953 | 17102 | 2734 | 2732 | 9063 | 955 | 13334 | 6616 |
| 3685 | 25551 | 10060 | 7389 | 21283 | 19513 | 19479 | 6841 | 22815 | 27022 | 20099 | 29400 |
| 16688 | 27873 | 8352 | 18528 | 3618 | 8493 | 18157 | 16315 | 1229 | 22619 | 22702 | 27401 |
| 5763 | 15469 | 5001 | 19548 | 20359 | 20357 | 19507 | 20333 | 19472 | 19557 | 20336 | 3472 |
| 17162 | 1155 | 15350 | 6559 | 5031 | 6056 | 13272 | 21645 | 4825 | 22146 | 3885 | 26065 |
| 26062 | 26588 | 6074 | 27102 | 16210 | 14478 | 24277 | 10940 | 11159 | 11199 | 3123 | 14189 |
| 9866 | 5925 | 8862 | 20232 | 27311 | 22409 | 4176 | 19934 | 29892 | 22794 | 27881 | 18994 |
| 24912 | 16456 | 27540 | 7709 | 10245 | 7937 | 17454 | 17490 | 21461 | 24193 | 30059 | 23504 |

EP 2 540 831 A2

(continued)

SEQ ID NO: homolog SEQ ID NOs

| | | | | | | | | | | | |
|---|---|---|---|---|---|---|---|---|---|---|---|
| | 18749 | 18721 | 20527 | 9722 | 3020 | 20624 | 6714 | 7796 | 22222 | 5268 | 21332 | 6250 |
| | 23401 | 9196 | 13068 | 10281 | 6253 | 4330 | 4360 | 14121 | 20043 | 4265 | 2211 | 11027 |
| | 1232 | 15305 | 15304 | 22706 | 10576 | 6806 | 9938 | 21643 | 20563 | 19374 | 26714 | 11319 |
| | 15583 | 12531 | 22926 | 26216 | 22915 | 18334 | 14692 | 18949 | 22414 | 22411 | 23884 | 13441 |
| | 17862 | 2106 | 22936 | 7533 | 13631 | 24940 | 8919 | 22162 | 29666 | 5033 | 27661 | 5718 |
| | 4593 | 29302 | 7649 | 19225 | 26116 | 2078 | 14369 | 26386 | 2678 | 16732 | 1680 | 6671 |
| | 26701 | 2807 | 4093 | 9316 | 8431 | 15323 | 16291 | 6441 | 16288 | 14653 | 8603 | 14475 |
| | 2506 | 9932 | 12101 | 26093 | 20541 | 27616 | 14889 | 19499 | 19459 | 24434 | 10493 | 18793 |
| | 10489 | 21324 | 24625 | 26404 | 25266 | 25209 | 7042 | 26511 | 26722 | 24664 | 11876 | 6466 |
| | 26173 | 14396 | 8719 | 6050 | 8754 | 21688 | 27600 | 25126 | 4963 | 12305 | 2607 | 29478 |
| | 3034 | 4859 | 8523 | 26399 | 25098 | 13349 | 13531 | 23247 | 9906 | 1882 | 15597 | 28657 |
| | 9299 | 25271 | 20085 | 11878 | 2759 | 8708 | 11644 | 24794 | 20451 | 4803 | 5467 | 19565 |
| | 15538 | 24906 | 5840 | 7916 | 24994 | 26871 | 12714 | 11032 | 9885 | 22650 | 22653 | 29220 |
| | 26659 | 26564 | 3520 | 22517 | 852 | 3735 | 26547 | 27567 | 29609 | 1127 | 24641 | 27585 |
| | 24643 | 22729 | 6141 | 21575 | 26033 | 26587 | 4512 | 23452 | 22634 | 4587 | 8154 | 15228 |
| | 3665 | 5602 | 24482 | 23313 | 6638 | 6637 | 3229 | 28631 | 27830 | 27867 | 28701 | 2433 |
| | 17865 | 17864 | 22642 | 27850 | 9219 | 16358 | 28655 | 29523 | 29439 | 4368 | 1487 | 2547 |
| | 16636 | 4019 | 10842 | 19143 | 16496 | 1020 | 27362 | 29370 | 24806 | 19099 | 26091 | 2983 |
| | 9736 | 17591 | 10587 | 14554 | 14551 | 8174 | 4268 | 20859 | 6531 | 14395 | 9001 | 2915 |
| | 2877 | 10550 | 30143 | 7134 | 6109 | 9970 | 14835 | 7354 | 24367 | 18705 | 3654 | 18159 |
| | 15833 | 27223 | 10830 | 16911 | 6732 | 13801 | 28291 | 525 | 19739 | 11620 | 14672 | 2624 |
| | 3151 | 3832 | 14113 | 5400 | 4114 | 6478 | 23852 | 22020 | 11311 | 13416 | 22083 | 8485 |
| | 28332 | 28525 | 27697 | 7882 | 22173 | 8028 | 23236 | 6436 | 15157 | 15325 | 23496 | 3220 |
| | 10653 | 7402 | 15193 | 770 | 21807 | 19922 | 10495 | 3751 | 19851 | 27805 | 14579 | 6166 |
| | 30176 | 26497 | 16270 | 26662 | 26810 | 8004 | 30115 | 29936 | 17182 | 13240 | 22101 | 5768 |
| | 24697 | 10825 | 21504 | 30130 | 18924 | 18276 | 18874 | 16826 | 13849 | 24866 | 3738 | 6081 |
| | 20630 | 17296 | 10424 | 15299 | 5343 | 20914 | 1419 | 18100 | 1786 | 29774 | 28745 | 6151 |
| | 11390 | 6543 | 12409 | 22921 | 11676 | 12540 | 12348 | 30158 | 8334 | 6100 | 23984 | 1006 |
| | 1086 | 29853 | 23260 | 10791 | 10232 | 19508 | 19515 | 19476 | 19437 | 8442 | 19468 | 776 |
| | 19390 | 19402 | 19462 | 19431 | 773 | 19396 | 27461 | 10300 | 18126 | 11053 | 13040 | 24219 |
| | 9334 | 28873 | 13835 | 11858 | 11034 | 28150 | | | | | | |
| 521: | 29209 | 12533 | 4572 | 16985 | 16884 | 28045 | 6210 | 6213 | 8061 | 29462 | 23549 | 21366 |
| | 11998 | 3682 | 28330 | 21266 | 27929 | 4500 | 29716 | 29741 | 27978 | 8100 | 6532 | 1705 |

151

SEQ ID NO: homolog SEQ ID NOs

| SEQ ID NO | | | | | | | | | | | | |
|---|---|---|---|---|---|---|---|---|---|---|---|---|
| | 19837 | 529 | 528 | 10758 | 19995 | | | | | | | |
| 522: | 29209 | 12533 | 4572 | 16985 | 16884 | 28045 | 6210 | 6213 | 8061 | 29462 | 23549 | 21366 |
| | 11998 | 3682 | 28330 | 21266 | 27929 | 4500 | 29716 | 29741 | 27978 | 8100 | 6532 | 1705 |
| | 19837 | 529 | 528 | 10758 | 19995 | | | | | | | |
| 523: | 29209 | 12533 | 4572 | 16985 | 16884 | 28045 | 6210 | 6213 | 8061 | 29462 | 23549 | 21366 |
| | 11998 | 3682 | 28330 | 21266 | 27929 | 4500 | 29716 | 29741 | 27978 | 8100 | 6532 | 1705 |
| | 19837 | 529 | 528 | 10758 | 19995 | | | | | | | |
| 524: | 11973 | 2129 | 21393 | 10212 | 19901 | 14958 | 8212 | 7013 | 9770 | 2890 | 6509 | 1939 |
| | 30054 | 20034 | 19273 | 12839 | 22386 | 4479 | 13721 | 13143 | 843 | 18213 | 25188 | 3474 |
| | 1054 | 23692 | 3033 | | | | | | | | | |
| 525: | 6825 | 25702 | 10973 | 11517 | 12191 | 18480 | 13664 | 7813 | 2142 | 29829 | 1847 | 2622 |
| | 13530 | 8321 | 21846 | 15362 | 18656 | 18662 | 17021 | 4400 | 4403 | 4407 | 21358 | 8495 |
| | 9685 | 27989 | 8838 | 9281 | 26271 | 21901 | 4480 | 30140 | 9266 | 19714 | 26726 | 15752 |
| | 913 | 6270 | 17378 | 17374 | 3693 | 25639 | 23222 | 13021 | 7227 | 22864 | 13246 | 5097 |
| | 9992 | 5155 | 28651 | 15944 | 5893 | 29772 | 17172 | 1208 | 11738 | 11848 | 2100 | 12972 |
| | 21491 | 23286 | 28892 | 17928 | 10535 | 14739 | 20750 | 27512 | 17031 | 6254 | 2324 | 1699 |
| | 11833 | 8761 | 9738 | 7031 | 13552 | 7494 | 7500 | 2220 | 15685 | 12216 | 11141 | 14100 |
| | 4277 | 1497 | 26343 | 30006 | 15092 | 16304 | 30063 | 7009 | 24019 | 14658 | 13469 | 19669 |
| | 8621 | 21719 | 25788 | 12392 | 22064 | 23663 | 25719 | 17303 | 17479 | 26563 | 14961 | 12830 |
| | 25538 | 21610 | 3624 | 9613 | 17461 | 12297 | 18416 | 18756 | 19826 | 22968 | 16868 | 14104 |
| | 11721 | 4062 | 27131 | 27143 | 5309 | 10082 | 16080 | 17925 | 3864 | 9076 | 13407 | 7361 |
| | 23620 | 11248 | 8570 | 17144 | 1500 | 6587 | 8599 | 7215 | 21972 | 19039 | 13562 | 13563 |
| | 30137 | 21894 | 16275 | 18834 | 1995 | 816 | 15953 | 2734 | 2732 | 9063 | 955 | 13334 |
| | 6616 | 3685 | 25551 | 10060 | 21283 | 19513 | 19479 | 6841 | 22815 | 20099 | 16688 | 8352 |
| | 18528 | 3618 | 8493 | 18157 | 16315 | 1229 | 22619 | 22702 | 27401 | 5763 | 15469 | 5001 |
| | 20359 | 20357 | 20333 | 19548 | 19507 | 19557 | 19472 | 20336 | 3472 | 5031 | 21645 | 3885 |
| | 4825 | 26065 | 22146 | 26062 | 26588 | 6074 | 14478 | 24277 | 10940 | 11159 | 11199 | 3123 |
| | 14189 | 9866 | 5925 | 8862 | 20232 | 27311 | 22409 | 4176 | 19934 | 29892 | 22794 | 27881 |
| | 18994 | 16456 | 27540 | 7709 | 24193 | 30059 | 23504 | 18749 | 18721 | 20527 | 9722 | 22222 |
| | 5268 | 21332 | 23401 | 9196 | 13068 | 10281 | 6253 | 4330 | 4360 | 14121 | 20043 | 4265 |
| | 2211 | 11027 | 1232 | 15305 | 15304 | 6806 | 9938 | 21643 | 19374 | 11319 | 15583 | 22926 |
| | 26216 | 22915 | 18334 | 18949 | 22414 | 22411 | 23884 | 17862 | 2106 | 7533 | 24940 | 22162 |
| | 29666 | 5033 | 27661 | 5718 | 29302 | 4593 | 19225 | 26116 | 2078 | 14369 | 2678 | 6671 |

(continued)

SEQ ID NO: homolog SEQ ID NOs

| | | | | | | | | | | | | |
|---|---|---|---|---|---|---|---|---|---|---|---|---|
| | 26701 | 9316 | 8431 | 15323 | 6441 | 16291 | 16288 | 14653 | 8603 | 14475 | 2506 | 9932 |
| | 26093 | 20541 | 14889 | 19499 | 19459 | 24434 | 10493 | 10489 | 21324 | 24625 | 26404 | 25266 |
| | 25209 | 7042 | 26511 | 26722 | 14396 | 8719 | 21688 | 27600 | 25126 | 12305 | 2607 | 29478 |
| | 3034 | 26399 | 25098 | 13349 | 13531 | 23247 | 9906 | 1882 | 15597 | 28657 | 9299 | 25271 |
| | 20085 | 11878 | 8708 | 2759 | 20451 | 7916 | 5840 | 11032 | 22650 | 22653 | 29220 | 26659 |
| | 26564 | 3520 | 22517 | 852 | 26547 | 27567 | 29609 | 1127 | 24641 | 27585 | 24643 | 6141 |
| | 21575 | 26033 | 26587 | 4512 | 23452 | 22634 | 4587 | 15228 | 24482 | 23313 | 6638 | 3229 |
| | 28631 | 27830 | 2433 | 17865 | 17864 | 22642 | 27850 | 16358 | 28655 | 29523 | 29439 | 4368 |
| | 1487 | 2547 | 16636 | 4019 | 10842 | 19143 | 16496 | 1020 | 27362 | 29370 | 24806 | 19099 |
| | 2983 | 17591 | 10587 | 14554 | 14551 | 8174 | 4268 | 9825 | 520 | 17168 | 11034 | 28150 |
| | 10307 | 12694 | 12684 | 11946 | 3904 | 12708 | 2081 | 9879 | 10934 | 10900 | 29416 | 11709 |
| | 10945 | 11713 | 9189 | 20274 | 18195 | 6531 | 20859 | 9001 | 14395 | 2915 | 27586 | 14183 |
| | 27587 | 2877 | 6109 | 9970 | 14835 | 7354 | 24367 | 18705 | 3654 | 15833 | 18159 | 27223 |
| | 10830 | 16911 | 3151 | 3832 | 14113 | 5400 | 20504 | 23852 | 6478 | 4114 | 22020 | 11311 |
| | 13416 | 22083 | 8485 | 28332 | 27697 | 22173 | 8028 | 15325 | 15157 | 23496 | 19922 | 770 |
| | 3751 | 19851 | 6166 | 30176 | 26497 | 16270 | 26662 | 26810 | 8004 | 4355 | 4353 | 4359 |
| | 18114 | 18075 | 18116 | 18110 | 30115 | 29936 | 17182 | 13240 | 22101 | 5768 | 24697 | 21504 |
| | 30130 | 18924 | 18276 | 18874 | 16826 | 13849 | 24866 | 6081 | 20630 | 10424 | 15299 | 5343 |
| | 20914 | 1419 | 18100 | 1786 | 29774 | 28745 | 6151 | 18170 | 18144 | 18189 | 4428 | 4427 |
| | 4429 | 18042 | 18039 | 18070 | 11390 | 6543 | 12409 | 22921 | 11676 | 12540 | 12348 | 30158 |
| | 8334 | 6100 | 1006 | 23984 | 1086 | 29853 | 10791 | 23260 | 10232 | 8442 | 19476 | 19515 |
| | 19508 | 19402 | 19390 | 19437 | 19468 | 776 | 19431 | 19462 | 773 | 19396 | 27461 | 10300 |
| | 18126 | 11053 | 13040 | 24219 | 4397 | 4363 | 4399 | 9334 | 28873 | 13835 | 11858 | |
| 526: | 7621 | 26546 | 27408 | 16154 | 6289 | 25189 | 21314 | 17952 | 16882 | 23034 | 30020 | 10581 |
| | 4988 | 807 | 30086 | 19631 | 1200 | 20690 | 3798 | 27875 | 6751 | | | |
| 527: | 1899 | 1827 | 16026 | 17088 | 13534 | 22937 | 25072 | 28842 | 29040 | 14837 | 6103 | 17874 |
| | 15397 | 27314 | 6173 | 4271 | 28594 | 2304 | 10076 | 10253 | 21775 | 7326 | 2929 | 22233 |
| | 20113 | 2261 | 16257 | 28035 | 20247 | 19059 | 1311 | 28840 | 20162 | 16635 | 16631 | 5804 |
| | 13989 | 19276 | 29836 | 5574 | 19067 | 24266 | 29226 | 24616 | 10426 | 7187 | 20683 | 17734 |
| | 30225 | 5142 | 23265 | | | | | | | | | |
| 528: | 29209 | 12533 | 4572 | 16985 | 19995 | 523 | 522 | 521 | 28711 | 9956 | 8061 | 29356 |
| | 10882 | 3682 | 21266 | 16847 | 1705 | 19837 | 10758 | 17343 | | | | |
| 529: | 29209 | 12533 | 4572 | 16985 | 19995 | 523 | 522 | 521 | 28711 | 9956 | 8061 | 29356 |

153

(continued)

SEQ ID NO: homolog SEQ ID NOs

| SEQ ID NO | homolog SEQ ID NOs | | | | | | | | | | |
|---|---|---|---|---|---|---|---|---|---|---|---|
| 530: | | | | | | | | | | | |
| 10882 | 3682 | 21266 | 16847 | 1705 | 19837 | 17343 | 10758 | 23798 | 9025 | 22730 | 15614 |
| 19520 | 5863 | 22089 | 25418 | 8136 | 30207 | 2111 | 28147 | 21213 | 27297 | 20548 | 7981 |
| 16519 | 4882 | 25535 | 15019 | 7995 | 27952 | 20928 | 18650 | 26529 | 21004 | 21985 | 11961 |
| 23339 | 14195 | 12936 | 8915 | 8556 | 27580 | 16067 | 26883 | 3905 | 3698 | 17319 | 19183 |
| 6055 | 21462 | 21065 | 17882 | 9582 | 17693 | 25248 | 12854 | 5970 | 14452 | 16341 | 14303 |
| 7228 | 17708 | 508 | 507 | 506 | 28553 | 19451 | 17347 | 19010 | 7318 | 8478 | 20964 |
| 2139 | 15361 | 7668 | 25273 | 3830 | 11809 | 1835 | 20064 | 21552 | 16256 | 17380 | 16668 |
| 17385 | 26462 | 3302 | 11339 | 8077 | 11582 | 21637 | 22660 | 17838 | 10838 | 15436 | 3168 |
| 3687 | 4939 | 9629 | 8784 | 13771 | 23464 | 12851 | 14226 | 6001 | 26978 | 20010 | 27152 |
| 28364 | 14918 | 15872 | 2982 | 8870 | 21771 | 13094 | 27718 | 3952 | 21749 | 19157 | 4636 |
| 27150 | 6323 | 20282 | 27731 | 22494 | 1885 | 4914 | 10353 | 13066 | 7038 | 17806 | 3083 |
| 21628 | 18429 | 28890 | 16485 | 20529 | 11277 | 10198 | 3060 | 25675 | 13577 | 6029 | 5934 |
| 11668 | 11672 | 5599 | 29815 | 9448 | 28179 | 6071 | 8840 | 23272 | 23275 | 7992 | 5669 |
| 12558 | 14122 | 1099 | 13195 | 8827 | 7231 | 8560 | 4933 | 15981 | 21237 | 22077 | 5927 |
| 7923 | 29017 | 2661 | 27431 | 2416 | 8897 | 2017 | 29171 | 3611 | 22860 | 4453 | 24900 |
| 18876 | 19785 | 1566 | 23594 | 20078 | 29794 | 13099 | 28728 | 23617 | 16803 | 10800 | 28733 |
| 25842 | 26400 | 25992 | 24161 | 18960 | 7972 | 6601 | 25286 | 14074 | 16915 | 15935 | 22519 |
| 14423 | 4620 | 8737 | 7043 | 26747 | 29751 | 12334 | 11529 | 10517 | 21526 | 24409 | 21264 |
| 8380 | 22733 | 12415 | 12419 | 3052 | 3396 | 22179 | 26436 | 16089 | 27728 | 15839 | 16580 |
| 16049 | 9702 | 4940 | 3049 | 5468 | 26945 | 24331 | 18051 | 8689 | 26186 | 18901 | 2055 |
| 9272 | 28233 | 15058 | 21458 | 8914 | 7040 | 5358 | 28058 | 21646 | 28639 | 18476 | 27620 |
| 9861 | 13556 | 27584 | 8764 | 3411 | 14780 | 28685 | 29443 | 18533 | 7441 | 26930 | 7450 |
| 5779 | 16642 | 15494 | 23279 | 29398 | 26418 | 30227 | 9176 | 20722 | 761 | 24718 | 26345 |
| 2430 | 21980 | 26765 | 7536 | 29041 | 28235 | 1908 | 11927 | 24938 | 4144 | 17855 | 26192 |
| 20449 | 9493 | 2538 | 5406 | 19985 | 11971 | 7792 | 8810 | 10937 | 14860 | 19023 | 30300 |
| 21882 | 25769 | 1490 | 16823 | 20516 | 23284 | 5196 | 6905 | 26681 | 29349 | 4944 | 16945 |
| 8515 | 10925 | 6456 | 12502 | 1599 | 27359 | 20112 | 15463 | 10570 | 22874 | 925 | 23918 |
| 26601 | 9817 | 9729 | 29914 | 27385 | 23753 | 24826 | 11341 | 29366 | 16879 | 19903 | 22054 |
| 17449 | 3975 | 21566 | 9578 | 26141 | 2717 | 19314 | 10731 | 4437 | 25811 | 16790 | 8158 |
| 15065 | 15346 | 5074 | 6906 | 8189 | 2596 | 14064 | 20816 | 16969 | 5819 | 21668 | 22052 |
| 14143 | 2766 | 1571 | 6412 | 4888 | 3637 | 14407 | 7168 | 27134 | 19930 | 12351 | 22779 |
| 26333 | 21794 | 25145 | 22963 | 11839 | 21475 | 8685 | 15115 | 10377 | 26703 | 7026 | 10943 |
| 17895 | 14534 | 16599 | 29825 | 17644 | 22440 | 23316 | 15788 | | | | |

(continued)

SEQ ID NO: homolog SEQ ID NOs

| SEQ ID NO | | | | | | | | | | | | |
|---|---|---|---|---|---|---|---|---|---|---|---|---|
|  | 27405 | 14815 | 18442 | 28839 | 17428 | 15605 | 635 | 510 | 26696 | 509 | 26716 | 12413 |
|  | 817 | 22220 | 25694 | 5456 | 7516 | 8593 | 2676 | 2675 | 12536 | 12512 | 20372 | 15249 |
|  | 15247 | 17555 | 11119 | 28010 | 23518 | 25789 | 27449 | 5342 | 30199 | 12060 | 5771 | 25446 |
|  | 9442 | 9361 | 2533 | 2087 | 13670 | 13139 | 25857 |  |  |  |  |  |
| 531: | 26814 | 20625 | 7339 | 26389 | 11428 | 15881 | 4595 | 10419 | 10422 | 2988 | 16805 | 2148 |
|  | 2761 | 28518 | 27088 | 23912 | 4971 | 29044 | 27999 | 28325 | 17946 | 10037 | 17189 | 1637 |
|  | 9654 | 4310 | 17927 | 2891 | 2866 | 20008 | 8559 | 16209 | 15239 | 29482 | 11042 | 22990 |
|  | 19623 | 12890 | 19849 | 12232 | 27597 | 14364 | 16980 | 9053 | 8971 | 17544 | 10982 | 14511 |
|  | 18818 | 22776 | 25826 | 26782 | 8405 | 10441 | 25903 | 17071 | 29817 | 30065 | 17193 | 28941 |
|  | 29849 | 26144 | 16110 | 16145 | 19652 | 23558 | 22249 | 25384 | 19813 | 16119 | 10019 | 28390 |
|  | 11150 | 13346 | 24654 | 15340 | 17387 | 21070 | 23781 | 11398 | 11065 | 2835 | 11571 | 9720 |
|  | 5999 | 6875 | 17598 | 7247 | 3865 | 10338 | 17041 | 8059 | 28117 | 13689 | 5204 | 17486 |
|  | 22256 | 1177 | 9962 | 29590 | 29878 | 4135 | 26669 | 11445 | 5684 | 5979 | 8514 | 28502 |
|  | 29357 | 18279 | 29684 | 22377 | 23829 | 25485 | 5745 | 11896 | 7397 | 10921 | 8107 | 7946 |
|  | 2755 | 17156 | 17180 | 15109 | 23750 | 15686 | 25013 | 2454 | 6486 | 30226 | 4972 | 10366 |
|  | 25934 | 28229 | 11681 | 10950 | 11684 | 4955 | 6931 | 25277 | 30029 | 21891 | 24865 | 16262 |
|  | 18366 | 7858 | 11919 | 6666 | 11993 | 16033 | 9028 | 21043 | 28551 | 17756 | 22875 | 24784 |
|  | 26146 | 4069 | 11181 | 2418 | 1625 | 8018 | 5331 | 1584 | 4434 | 8235 | 27678 | 26946 |
|  | 20562 | 14299 | 28915 | 2779 | 12268 | 17892 | 10840 | 9035 | 20127 | 16743 | 15123 | 11775 |
|  | 25036 | 4007 | 24694 | 17474 | 29513 | 6326 | 9711 | 9463 | 15103 | 7905 | 15817 | 23012 |
|  | 26189 | 20923 | 26211 | 1669 | 20366 | 2862 | 3356 | 16854 | 2168 | 27465 | 11408 | 11455 |
|  | 1840 | 3807 | 14805 | 21880 | 12005 | 26445 | 7887 | 7619 | 7656 | 12552 | 15732 | 2895 |
|  | 6670 | 11394 | 19184 | 7604 | 14515 | 2914 | 23738 | 25827 | 3769 | 3795 | 12105 | 27215 |
|  | 8032 | 9764 | 9447 | 13218 | 9441 | 12775 | 10156 | 10916 | 3976 | 28220 | 6006 | 5918 |
|  | 3218 | 26887 | 8317 | 8558 | 13065 | 29830 | 8970 | 10171 | 14738 | 11380 | 16235 | 12611 |
|  | 4608 | 3844 | 26003 | 15391 | 11153 | 3026 | 25299 | 16972 | 22261 | 7798 | 16108 | 5205 |
|  | 1064 | 25127 | 26363 | 2846 | 9438 | 11382 | 29901 | 17793 | 18125 | 9790 | 21238 | 28855 |
|  | 18784 | 21695 | 22327 | 25018 | 25449 | 27561 | 24188 | 26184 | 20248 | 17977 | 12011 | 11802 |
|  | 15688 | 3482 | 9486 | 18798 | 3531 | 18670 | 29902 | 4406 | 21438 | 23130 | 3423 | 29207 |
|  | 24191 | 19523 | 27339 | 10667 | 30205 | 27332 | 22710 | 3327 | 25891 | 19608 | 16359 | 29459 |
|  | 7545 | 13997 | 18043 | 7410 | 29969 | 24896 | 23833 | 18148 | 19114 | 7010 | 18136 | 25695 |
|  | 21673 | 9523 | 28072 | 19718 | 14925 | 4193 | 1060 | 22969 | 28950 | 13426 | 18098 | 7111 |
|  | 17042 | 24027 | 1892 | 23872 | 4173 | 22975 | 7310 | 3581 | 14582 | 11502 | 5963 | 26652 |

SEQ ID NO: homolog SEQ ID NOs

| | | | | | | | | | | | | |
|---|---|---|---|---|---|---|---|---|---|---|---|---|
| | 24617 | 21589 | 4328 | 22853 | 12781 | 25739 | 29410 | 6599 | 3958 | 16109 | 20631 | 23158 |
| | 7636 | 8703 | 9402 | 20192 | 28640 | 14153 | 7116 | 13653 | 5795 | 15676 | 11475 | 3694 |
| | 10621 | 17702 | 18669 | 23379 | 26300 | 26406 | 18968 | 13333 | 8288 | 5624 | 22965 | 16346 |
| | 24814 | 23232 | 30321 | 17268 | 24350 | 21233 | 19363 | 15181 | 10854 | 2745 | 4770 | 19908 |
| | 26700 | 12339 | 10022 | 10268 | 3064 | 2003 | 7708 | 864 | 3253 | 27124 | 15589 | 30247 |
| | 4442 | 10843 | 24156 | 29233 | 21354 | 10884 | 28247 | 27258 | 22987 | 2577 | 20869 | 18505 |
| | 14451 | 12669 | 9515 | 24618 | 11155 | 17786 | 18981 | 21330 | 8886 | 25409 | 18232 | 21868 |
| | 6644 | 1166 | 1436 | 3891 | 15224 | 2323 | 26728 | 8106 | 5157 | 8211 | 4851 | 4071 |
| | 4043 | 27458 | 22767 | 28485 | 26924 | 26914 | 2131 | 30112 | 21337 | 781 | 18877 | 24818 |
| | 19740 | 17821 | 25906 | 26928 | 7821 | 7824 | 5212 | 21598 | 30265 | 5432 | 28192 | 21424 |
| | 1325 | 13905 | 15389 | 14334 | 28416 | 16523 | 10206 | 20666 | 22617 | 11492 | 6573 | 4474 |
| | 19490 | 25555 | 1058 | 8983 | 24986 | 14285 | 13596 | 17129 | 22597 | 8342 | 21881 | 29280 |
| | 5298 | 11674 | 16764 | 21224 | 21710 | 13540 | 29724 | 28490 | 18776 | 1343 | 17383 | 16338 |
| | 24799 | 16065 | 22008 | 24229 | 16040 | 19160 | 20322 | 27011 | 20435 | 12823 | 17541 | 3498 |
| | 17959 | 3399 | 26907 | 25866 | 11498 | 7747 | 25994 | 15878 | 2183 | 27396 | 8377 | 20079 |
| | 10721 | 18040 | 27933 | 27402 | 22119 | 20640 | 27662 | 2203 | 5330 | 22613 | 27649 | 24307 |
| | 2981 | 13533 | 25814 | 12784 | 14567 | 7515 | 6969 | 14568 | 7093 | 7538 | 20452 | 11173 |
| | 23193 | 2357 | 1196 | 3045 | 11634 | 12015 | 4743 | 15873 | 1868 | 8078 | 11170 | 14915 |
| | 25207 | 24363 | 22956 | 5474 | 5441 | 22547 | 27720 | 9683 | 1328 | 13622 | 20704 | 17730 |
| | 16286 | 11531 | 6983 | 17099 | 6506 | 29456 | 23178 | 23588 | 22438 | 29157 | 3322 | 21421 |
| | 14197 | 2757 | 9700 | 12213 | 6997 | 21913 | 5608 | 2699 | 23763 | 29714 | 20681 | 21681 |
| | 25908 | 1077 | 10949 | 9287 | 13229 | 6088 | 22467 | 13901 | 18270 | 13699 | 16217 | |
| 532: | 26814 | 20625 | 7339 | 26389 | 11428 | 15881 | 4595 | 10419 | 10422 | 2988 | 16805 | 2148 |
| | 2761 | 28518 | 27088 | 23912 | 4971 | 29044 | 27999 | 28325 | 17946 | 10037 | 17189 | 1637 |
| | 9654 | 4310 | 17927 | 2891 | 2866 | 20008 | 8559 | 16209 | 15239 | 29482 | 11042 | 22990 |
| | 19623 | 12890 | 19849 | 12232 | 27597 | 14364 | 16980 | 9053 | 8971 | 17544 | 10982 | 14511 |
| | 18818 | 22776 | 25826 | 26782 | 8405 | 10441 | 25903 | 17071 | 29817 | 30065 | 17193 | 28941 |
| | 29849 | 26144 | 16110 | 16145 | 19652 | 23558 | 22249 | 25384 | 19813 | 16119 | 10019 | 28390 |
| | 11150 | 13346 | 24654 | 15340 | 17387 | 21070 | 23781 | 11398 | 11065 | 2835 | 11571 | 9720 |
| | 5999 | 6875 | 17598 | 7247 | 3865 | 10338 | 17041 | 8059 | 28117 | 13689 | 5204 | 17486 |
| | 22256 | 1177 | 9962 | 29590 | 29878 | 4135 | 26669 | 11445 | 5684 | 5979 | 8514 | 28502 |
| | 29357 | 18279 | 29684 | 22377 | 23829 | 25485 | 5745 | 11896 | 7397 | 10921 | 8107 | 7946 |
| | 2755 | 17156 | 17180 | 15109 | 23750 | 15686 | 25013 | 2454 | 6486 | 30226 | 4972 | 10366 |

SEQ ID NO: homolog SEQ ID NOs

| | | | | | | | | | | | |
|---|---|---|---|---|---|---|---|---|---|---|---|
| 25934 | 28229 | 11681 | 10950 | 11684 | 4955 | 6931 | 25277 | 30029 | 21891 | 24865 | 16262 |
| 18366 | 7858 | 11919 | 6666 | 11993 | 16033 | 9028 | 21043 | 28551 | 17756 | 22875 | 24784 |
| 26146 | 4069 | 11181 | 2418 | 1625 | 8018 | 5331 | 1584 | 4434 | 8235 | 27678 | 26946 |
| 20562 | 14299 | 28915 | 2779 | 12268 | 17892 | 10840 | 9035 | 20127 | 16743 | 15123 | 11775 |
| 25036 | 4007 | 24694 | 17474 | 29513 | 6326 | 9711 | 9463 | 15103 | 7905 | 15817 | 23012 |
| 26189 | 20923 | 26211 | 1669 | 20366 | 2862 | 3356 | 16854 | 2168 | 27465 | 11408 | 11455 |
| 1840 | 3807 | 14805 | 21880 | 12005 | 26445 | 7887 | 7619 | 7656 | 12552 | 15732 | 2895 |
| 6670 | 11394 | 19184 | 7604 | 14515 | 2914 | 23738 | 25827 | 3769 | 3795 | 12105 | 27215 |
| 8032 | 9764 | 9447 | 13218 | 9441 | 12775 | 10156 | 10916 | 3976 | 28220 | 6006 | 5918 |
| 3218 | 26887 | 8317 | 8558 | 13065 | 29830 | 8970 | 10171 | 14738 | 11380 | 16235 | 12611 |
| 4608 | 3844 | 26003 | 15391 | 11153 | 3026 | 25299 | 16972 | 22261 | 7798 | 16108 | 5205 |
| 1064 | 25127 | 26363 | 2846 | 9438 | 11382 | 29901 | 17793 | 18125 | 9790 | 21238 | 28855 |
| 18784 | 21695 | 22327 | 25018 | 25449 | 27561 | 24188 | 26184 | 20248 | 17977 | 12011 | 11802 |
| 15688 | 3482 | 9486 | 18798 | 3531 | 18670 | 29902 | 4406 | 21438 | 23130 | 3423 | 29207 |
| 24191 | 19523 | 27339 | 10667 | 30205 | 27332 | 22710 | 3327 | 25891 | 19608 | 16359 | 29459 |
| 7545 | 13997 | 18043 | 7410 | 29969 | 24896 | 23833 | 18148 | 19114 | 7010 | 18136 | 25695 |
| 21673 | 9523 | 28072 | 19718 | 14925 | 4193 | 1060 | 22969 | 28950 | 13426 | 18098 | 7111 |
| 17042 | 24027 | 1892 | 23872 | 4173 | 22975 | 7310 | 3581 | 14582 | 11502 | 5963 | 26652 |
| 24617 | 21589 | 4328 | 22853 | 12781 | 25739 | 29410 | 6599 | 3958 | 16109 | 20631 | 23158 |
| 7636 | 8703 | 9402 | 20192 | 28640 | 14153 | 7116 | 13653 | 5795 | 15676 | 11475 | 3694 |
| 10621 | 17702 | 18669 | 23379 | 26300 | 26406 | 18968 | 13333 | 8288 | 5624 | 22965 | 16346 |
| 24814 | 23232 | 30321 | 17268 | 24350 | 21233 | 19363 | 15181 | 10854 | 2745 | 4770 | 19908 |
| 26700 | 12339 | 10022 | 10268 | 3064 | 2003 | 7708 | 864 | 3253 | 27124 | 15589 | 30247 |
| 4442 | 10843 | 24156 | 29233 | 21354 | 10884 | 28247 | 27258 | 22987 | 2577 | 20869 | 18505 |
| 14451 | 12669 | 9515 | 24618 | 11155 | 17786 | 18981 | 21330 | 8886 | 25409 | 18232 | 21868 |
| 6644 | 1166 | 1436 | 3891 | 15224 | 2323 | 26728 | 8106 | 5157 | 8211 | 4851 | 4071 |
| 4043 | 27458 | 22767 | 28485 | 26924 | 26914 | 2131 | 30112 | 21337 | 781 | 18877 | 24818 |
| 19740 | 17821 | 25906 | 26928 | 7821 | 7824 | 5212 | 21598 | 30265 | 5432 | 28192 | 21424 |
| 1325 | 13905 | 15389 | 14334 | 28416 | 16523 | 10206 | 20666 | 22617 | 11492 | 6573 | 4474 |
| 19490 | 25555 | 1058 | 8983 | 24986 | 14285 | 13596 | 17129 | 22597 | 8342 | 21881 | 29280 |
| 5298 | 11674 | 16764 | 21224 | 21710 | 13540 | 29724 | 28490 | 18776 | 1343 | 17383 | 16338 |
| 24799 | 16065 | 22008 | 24229 | 16040 | 19160 | 20322 | 27011 | 20435 | 12823 | 17541 | 3498 |
| 17959 | 3399 | 26907 | 25866 | 11498 | 7747 | 25994 | 15878 | 2183 | 27396 | 8377 | 20079 |

EP 2 540 831 A2

SEQ ID NO: homolog SEQ ID NOs

| | | | | | | | | | | | | |
|---|---|---|---|---|---|---|---|---|---|---|---|---|
| | 10721 | 18040 | 27933 | 27402 | 22119 | 20640 | 27662 | 2203 | 5330 | 22613 | 27649 | 24307 |
| | 2981 | 13533 | 25814 | 12784 | 14567 | 7515 | 6969 | 14568 | 7093 | 7538 | 20452 | 11173 |
| | 23193 | 2357 | 1196 | 3045 | 11634 | 12015 | 4743 | 15873 | 1868 | 8078 | 11170 | 14915 |
| | 25207 | 24363 | 22956 | 5474 | 5441 | 22547 | 27720 | 9683 | 1328 | 13622 | 20704 | 17730 |
| | 16286 | 11531 | 6983 | 17099 | 6506 | 29456 | 23178 | 23588 | 22438 | 29157 | 3322 | 21421 |
| | 14197 | 2757 | 9700 | 12213 | 6997 | 21913 | 5608 | 2699 | 23763 | 29714 | 20681 | 21681 |
| | 25908 | 1077 | 10949 | 9287 | 13229 | 6088 | 22467 | 13901 | 18270 | 13699 | 16217 | |
| 533: | 26814 | 20625 | 7339 | 26389 | 11428 | 15881 | 4595 | 10419 | 10422 | 2988 | 16805 | 2148 |
| | 2761 | 28518 | 27088 | 23912 | 4971 | 29044 | 27999 | 28325 | 17946 | 10037 | 17189 | 1637 |
| | 9654 | 4310 | 17927 | 2891 | 2866 | 20008 | 8559 | 16209 | 15239 | 29482 | 11042 | 22990 |
| | 19623 | 12890 | 19849 | 12232 | 27597 | 14364 | 16980 | 9053 | 8971 | 17544 | 10982 | 14511 |
| | 18818 | 22776 | 25826 | 26782 | 8405 | 10441 | 25903 | 17071 | 29817 | 30065 | 17193 | 28941 |
| | 29849 | 26144 | 16110 | 16145 | 19652 | 23558 | 22249 | 25384 | 19813 | 16119 | 10019 | 28390 |
| | 11150 | 13346 | 24654 | 15340 | 17387 | 21070 | 23781 | 11398 | 11065 | 2835 | 11571 | 9720 |
| | 5999 | 6875 | 17598 | 7247 | 3865 | 10338 | 17041 | 8059 | 28117 | 13689 | 5204 | 17486 |
| | 22256 | 1177 | 9962 | 29590 | 29878 | 4135 | 26669 | 11445 | 5684 | 5979 | 8514 | 28502 |
| | 29357 | 18279 | 29684 | 22377 | 23829 | 25485 | 5745 | 11896 | 7397 | 10921 | 8107 | 7946 |
| | 2755 | 17156 | 17180 | 15109 | 23750 | 15686 | 25013 | 2454 | 6486 | 30226 | 4972 | 10366 |
| | 25934 | 28229 | 11681 | 10950 | 11684 | 4955 | 6931 | 25277 | 30029 | 21891 | 24865 | 16262 |
| | 18366 | 7858 | 11919 | 6666 | 11993 | 16033 | 9028 | 21043 | 28551 | 17756 | 22875 | 24784 |
| | 26146 | 4069 | 11181 | 2418 | 1625 | 8018 | 5331 | 1584 | 4434 | 8235 | 27678 | 26946 |
| | 20562 | 14299 | 28915 | 2779 | 12268 | 17892 | 10840 | 9035 | 20127 | 16743 | 15123 | 11775 |
| | 25036 | 4007 | 24694 | 17474 | 29513 | 6326 | 9711 | 9463 | 15103 | 7905 | 15817 | 23012 |
| | 26189 | 20923 | 26211 | 1669 | 20366 | 2862 | 3356 | 16854 | 2168 | 27465 | 11408 | 11455 |
| | 1840 | 3807 | 14805 | 21880 | 12005 | 26445 | 7887 | 7619 | 7656 | 12552 | 15732 | 2895 |
| | 6670 | 11394 | 19184 | 7604 | 14515 | 2914 | 23738 | 25827 | 3769 | 3795 | 12105 | 27215 |
| | 8032 | 9764 | 9447 | 13218 | 9441 | 12775 | 10156 | 10916 | 3976 | 28220 | 6006 | 5918 |
| | 3218 | 26887 | 8317 | 8558 | 13065 | 29830 | 8970 | 10171 | 14738 | 11380 | 16235 | 12611 |
| | 4608 | 3844 | 26003 | 15391 | 11153 | 3026 | 25299 | 16972 | 22261 | 7798 | 16108 | 5205 |
| | 1064 | 25127 | 26363 | 2846 | 9438 | 11382 | 29901 | 17793 | 18125 | 9790 | 21238 | 28855 |
| | 18784 | 21695 | 22327 | 25018 | 25449 | 27561 | 24188 | 26184 | 20248 | 17977 | 12011 | 11802 |
| | 15688 | 3482 | 9486 | 18798 | 3531 | 18670 | 29902 | 4406 | 21438 | 23130 | 3423 | 29207 |
| | 24191 | 19523 | 27339 | 10667 | 30205 | 27332 | 22710 | 3327 | 25891 | 19608 | 16359 | 29459 |

(continued)

| SEQ ID NO: | homolog SEQ ID NOs | | | | | | | | | | | |
|---|---|---|---|---|---|---|---|---|---|---|---|---|
| | 7545 | 13997 | 18043 | 7410 | 29969 | 24896 | 23833 | 18148 | 19114 | 7010 | 18136 | 25695 |
| | 21673 | 9523 | 28072 | 19718 | 14925 | 4193 | 1060 | 22969 | 28950 | 13426 | 18098 | 7111 |
| | 17042 | 24027 | 1892 | 23872 | 4173 | 22975 | 7310 | 3581 | 14582 | 11502 | 5963 | 26652 |
| | 24617 | 21589 | 4328 | 22853 | 12781 | 25739 | 29410 | 6599 | 3958 | 16109 | 20631 | 23158 |
| | 7636 | 8703 | 9402 | 20192 | 28640 | 14153 | 7116 | 13653 | 5795 | 15676 | 11475 | 3694 |
| | 10621 | 17702 | 18669 | 23379 | 26300 | 26406 | 18968 | 13333 | 8288 | 5624 | 22965 | 16346 |
| | 24814 | 23232 | 30321 | 17268 | 24350 | 21233 | 19363 | 15181 | 10854 | 2745 | 4770 | 19908 |
| | 26700 | 12339 | 10022 | 10268 | 3064 | 2003 | 7708 | 864 | 3253 | 27124 | 15589 | 30247 |
| | 4442 | 10843 | 24156 | 29233 | 21354 | 10884 | 28247 | 27258 | 22987 | 2577 | 20869 | 18505 |
| | 14451 | 12669 | 9515 | 24618 | 11155 | 17786 | 18981 | 21330 | 8886 | 25409 | 18232 | 21868 |
| | 6644 | 1166 | 1436 | 3891 | 15224 | 2323 | 26728 | 8106 | 5157 | 8211 | 4851 | 4071 |
| | 4043 | 27458 | 22767 | 28485 | 26924 | 26914 | 2131 | 30112 | 21337 | 781 | 18877 | 24818 |
| | 19740 | 17821 | 25906 | 26928 | 7821 | 7824 | 5212 | 21598 | 30265 | 5432 | 28192 | 21424 |
| | 1325 | 13905 | 15389 | 14334 | 28416 | 16523 | 10206 | 20666 | 22617 | 11492 | 6573 | 4474 |
| | 19490 | 25555 | 1058 | 8983 | 24986 | 14285 | 13596 | 17129 | 22597 | 8342 | 21881 | 29280 |
| | 5298 | 11674 | 16764 | 21224 | 21710 | 13540 | 29724 | 28490 | 18776 | 1343 | 17383 | 16338 |
| | 24799 | 16065 | 22008 | 24229 | 16040 | 19160 | 20322 | 27011 | 20435 | 12823 | 17541 | 3498 |
| | 17959 | 3399 | 26907 | 25866 | 11498 | 7747 | 25994 | 15878 | 2183 | 27396 | 8377 | 20079 |
| | 10721 | 18040 | 27933 | 27402 | 22119 | 20640 | 27662 | 2203 | 5330 | 22613 | 27649 | 24307 |
| | 2981 | 13533 | 25814 | 12784 | 14567 | 7515 | 6969 | 14568 | 7093 | 7538 | 20452 | 11173 |
| | 23193 | 2357 | 1196 | 3045 | 11634 | 12015 | 4743 | 15873 | 1868 | 8078 | 11170 | 14915 |
| | 25207 | 24363 | 22956 | 5474 | 5441 | 22547 | 27720 | 9683 | 1328 | 13622 | 20704 | 17730 |
| | 16286 | 11531 | 6983 | 17099 | 6506 | 29456 | 23178 | 23588 | 22438 | 29157 | 3322 | 21421 |
| | 14197 | 2757 | 9700 | 12213 | 6997 | 21913 | 5608 | 2699 | 23763 | 29714 | 20681 | 21681 |
| | 25908 | 1077 | 10949 | 9287 | 13229 | 6088 | 22467 | 13901 | 18270 | 13699 | 16217 | |
| 534: | 4461 | 6014 | 5818 | 8614 | 8618 | 8620 | 16274 | 28056 | 24174 | 18112 | 6034 | 24186 |
| | 27132 | 26391 | 26384 | 26388 | 26390 | 27320 | 1519 | 3270 | 13299 | 20233 | 26316 | 1148 |
| | 3450 | 18341 | 27077 | 24187 | 9775 | 26395 | 3440 | 3543 | 8855 | 3797 | 1097 | 6360 |
| | 8237 | 8000 | 8002 | 27064 | 10533 | 14967 | 17228 | 19381 | 19330 | 20197 | 27225 | 18222 |
| | 6477 | | | | | | | | | | | |
| 535: | 11616 | 18851 | 18953 | 18865 | 18856 | 18868 | 18892 | 18096 | 29990 | 7728 | 21361 | 16223 |
| | 16260 | 11728 | 11586 | 11610 | 11614 | 11619 | 11645 | 11679 | 11687 | 11693 | 11696 | 11730 |
| | 11683 | 18107 | 11734 | 11737 | 18103 | 18895 | 18553 | 9369 | 18556 | 9370 | 7235 | 8193 |

(continued)

SEQ ID NO: homolog SEQ ID NOs

| SEQ ID NO | | | | | | | | | | | | |
|---|---|---|---|---|---|---|---|---|---|---|---|---|
| | 8195 | 8201 | 28942 | 16888 | 6974 | 28274 | 7084 | 3491 | 28793 | 11606 | 13719 | 5141 |
| | 11633 | 16634 | 14118 | 908 | 11623 | 966 | 15634 | 22112 | 7020 | 14688 | 28053 | 24278 |
| | 16191 | 10824 | 29204 | 24254 | 22509 | 17908 | 7814 | 18473 | 961 | 26472 | 24171 | 24177 |
| | 29859 | 29201 | 8424 | 29864 | 18478 | 20565 | 24086 | 27767 | 21381 | 23398 | 11107 | 21404 |
| | 20566 | 21702 | 11104 | 14102 | 14033 | 13266 | 17350 | 7254 | 27391 | 27416 | 10651 | 17359 |
| | 10655 | 27419 | 10674 | 10681 | 27453 | 7261 | 7257 | 10678 | 7285 | 17363 | 10646 | 10657 |
| | 7283 | 27423 | 17355 | 17348 | 10648 | 7292 | 7912 | 16989 | 7291 | 17431 | 25595 | 7288 |
| | 17433 | 25593 | 17405 | 17436 | 25586 | 25620 | 17398 | 25565 | 25590 | 25622 | 25562 | 17439 |
| | 25626 | 25584 | 17402 | 10712 | 4230 | 11732 | 11654 | 11649 | 7623 | 8613 | 7078 | 7080 |
| | 8218 | 6023 | 26239 | 24239 | 23498 | 3939 | 12074 | 26319 | 26317 | 11652 | 10688 | 10684 |
| | 11653 | 18907 | 18902 | 18945 | 18898 | 18862 | 18950 | | | | | |
| 536: | 11616 | 18851 | 18953 | 18865 | 18856 | 18868 | 18892 | 18096 | 29990 | 7728 | 21361 | 16223 |
| | 16260 | 11728 | 11586 | 11610 | 11614 | 11619 | 11645 | 11679 | 11687 | 11693 | 11696 | 11730 |
| | 11683 | 18107 | 11734 | 11737 | 18103 | 18895 | 18553 | 9369 | 18556 | 9370 | 7235 | 8193 |
| | 8195 | 8201 | 28942 | 16888 | 6974 | 28274 | 7084 | 3491 | 28793 | 11606 | 13719 | 5141 |
| | 11633 | 16634 | 14118 | 908 | 11623 | 966 | 15634 | 22112 | 7020 | 14688 | 28053 | 24278 |
| | 16191 | 10824 | 29204 | 24254 | 22509 | 17908 | 7814 | 18473 | 961 | 26472 | 24171 | 24177 |
| | 29859 | 29201 | 8424 | 29864 | 18478 | 20565 | 24086 | 27767 | 21381 | 23398 | 11107 | 21404 |
| | 20566 | 21702 | 11104 | 14102 | 14033 | 13266 | 17350 | 7254 | 27391 | 27416 | 10651 | 17359 |
| | 10655 | 27419 | 10674 | 10681 | 27453 | 7261 | 7257 | 10678 | 7285 | 17363 | 10646 | 10657 |
| | 7283 | 27423 | 17355 | 17348 | 10648 | 7292 | 7912 | 16989 | 7291 | 17431 | 25595 | 7288 |
| | 17433 | 25593 | 17405 | 17436 | 25586 | 25620 | 17398 | 25565 | 25590 | 25622 | 25562 | 17439 |
| | 25626 | 25584 | 17402 | 10712 | 4230 | 11732 | 11654 | 11649 | 7623 | 8613 | 7078 | 7080 |
| | 8218 | 6023 | 26239 | 24239 | 23498 | 3939 | 12074 | 26319 | 26317 | 11652 | 10688 | 10684 |
| | 11653 | 18907 | 18902 | 18945 | 18898 | 18862 | 18950 | | | | | |
| 537: | 3913 | 25208 | 5724 | 1357 | 28477 | 9311 | 2668 | 10779 | 19883 | 23062 | 12152 | 29957 |
| | 21518 | 27882 | 29114 | 28888 | 3470 | 9141 | 8104 | 4871 | 19465 | 11686 | 28542 | 17123 |
| | 4348 | 28695 | 6062 | 16480 | 1189 | 15394 | 20784 | 29261 | 2033 | 22018 | 28874 | 28522 |
| | 22067 | 21898 | 29931 | 17146 | 17339 | 22560 | 27621 | 19529 | 12652 | 27378 | 29791 | 19550 |
| | 4657 | 11603 | 24184 | 24512 | 1109 | 13162 | 22424 | 23535 | 1158 | 25359 | 23467 | 9415 |
| | 25302 | 16894 | 7844 | 10637 | 20852 | 13270 | 17768 | 2390 | 17909 | 9146 | 29966 | 17224 |
| | 19469 | 7587 | 23822 | 12304 | 9506 | 24687 | | | | | | |
| 538: | 19798 | 3913 | 26285 | 5724 | 1357 | 1355 | 10523 | 27744 | 10525 | 28477 | 9328 | 20161 |

EP 2 540 831 A2

(continued)

SEQ ID NO: homolog SEQ ID NOs

| SEQ ID NO: | | | | | | | | | | | | |
|---|---|---|---|---|---|---|---|---|---|---|---|---|
|  | 21495 | 10780 | 19883 | 10395 | 6859 | 8166 | 7401 | 21518 | 18757 | 26692 | 13259 | 13258 |
|  | 4410 | 27882 | 17812 | 26377 | 3470 | 17494 | 4871 | 12458 | 19045 | 19463 | 28542 | 28539 |
|  | 17123 | 17121 | 18445 | 21471 | 28695 | 6062 | 16480 | 1189 | 15394 | 29261 | 19971 | 22018 |
|  | 6228 | 22088 | 22067 | 25534 | 11037 | 29930 | 3916 | 22200 | 17146 | 17147 | 17339 | 17340 |
|  | 22563 | 19529 | 12652 | 29791 | 4659 | 24184 | 24512 | 1110 | 11211 | 22424 | 23535 | 25359 |
|  | 14292 | 23966 | 24844 | 24841 | 15586 | 4327 | 23467 | 9415 | 16091 | 1187 | 8524 | 24928 |
|  | 7844 | 19534 | 24824 | 20518 | 10637 | 10608 | 6187 | 15135 | 3305 | 7287 | 4934 | 15534 |
|  | 16047 | 24440 | 17254 | 2733 | 28466 | 11077 | 14081 | 23337 | 14266 | 9618 | 19053 | 13764 |
|  | 10103 | 9146 | 17567 | 742 | 2528 | 3945 | 7968 | 9909 | 2443 | 8913 | 12474 | 9974 |
|  | 13472 | 12283 | 29360 | 23026 | 2705 | 10181 | 10299 | 28166 | | | | |
| 539: | 23465 | 13317 | 20710 | 25892 | 5188 | 5186 | 30049 | 18666 | 4493 | | | |
| 540: | 19005 | 18978 | 19528 | 2701 | 22686 | 7669 | 3135 | 9543 | 23060 | 7624 | 10662 | 9072 |
|  | 5251 | 7271 | 24570 | 28802 | 20140 | 3402 | 29236 | 736 | 12400 | 11820 | 24781 | 5899 |
|  | 13993 | 5594 | 23871 | 18307 | 14847 | 10081 | 19926 | 27106 | 27104 | 21194 | 449 | 21171 |
|  | 4160 | 29692 | 28473 | 13677 | 9078 | 8612 | 26080 | 541 | | | | |
| 541: | 19005 | 18978 | 19528 | 2701 | 22686 | 21409 | 7669 | 3135 | 9543 | 23060 | 7624 | 10662 |
|  | 30278 | 9072 | 5251 | 7271 | 24570 | 28802 | 3402 | 29236 | 20140 | 12400 | 13993 | 23871 |
|  | 5594 | 14847 | 27104 | 27106 | 26168 | 6003 | 449 | 21171 | 21194 | 22164 | 540 | 26361 |
|  | 18768 | 17282 | 4160 | 20118 | 16870 | 11317 | 29692 | 1978 | 8624 | 13677 | 28473 | 9078 |
|  | 8612 | 26080 | | | | | | | | | | |
| 542: | 26546 | 27408 | 5008 | 16154 | 6289 | 25189 | 17952 | 21314 | 28093 | 12970 | 20934 | 14556 |
|  | 4959 | 4962 | 5146 | 18958 | 16882 | 4999 | 23034 | 30020 | 4988 | 807 | 30086 | 19631 |
|  | 19899 | 2479 | 9201 | | | | | | | | | |
| 543: | 21950 | 21955 | 11393 | 17628 | 20760 | 4575 | 10480 | 24385 | 14043 | 7452 | 12658 | 25323 |
|  | 9346 | 17067 | 17455 | 9357 | 13475 | 20122 | 15329 | 30165 | 14456 | 14496 | 30141 | 5087 |
|  | 13734 | 3009 | 27042 | 7267 | 26432 | 25505 | 15721 | 24778 | 15725 | 27853 | 16029 | 24297 |
|  | 14902 | 20216 | 13963 | 7046 | 16901 | 22098 | 6722 | 2133 | 14084 | 8035 | 6107 | 1927 |
|  | 6237 | 3133 | 3230 | 28216 | 29973 | 29049 | 6240 | 7918 | 24064 | 1390 | 26227 | 27922 |
|  | 3136 | 2192 | 1204 | 1443 | 27220 | 23104 | 8054 | 10057 | 26325 | 6266 | 27219 | 27884 |
|  | 2352 | 24430 | 15804 | 21030 | 6378 | 7189 | 17740 | 14807 | 15737 | 20094 | 6426 | 7843 |
|  | 13628 | 14616 | 16640 | 19103 | 12751 | 17823 | 10778 | 6033 | 19937 | 13744 | 14621 | 12835 |
|  | 4347 | 7920 | 9616 | 7874 | 2907 | 6405 | 13607 | 10913 | 6423 | 3235 | 14619 | 17917 |
|  | 9819 | 15120 | 20999 | 29884 | 4273 | 8857 | 4074 | 20083 | 10428 | 18551 | 18548 | 18785 |

(continued)

SEQ ID NO: homolog SEQ ID NOs

|  | | | | | | | | | | | | |
|---|---|---|---|---|---|---|---|---|---|---|---|---|
|  | 28398 | 3757 | 5416 | 7475 | 2778 | 24487 | 15939 | 17097 | 17727 | 4660 | 3636 | 16725 |
|  | 1627 | 30285 | 18392 | 1607 | 3573 | 26515 | 2709 | 28433 | 1706 | 21933 | 24457 | 18760 |
|  | 19245 | 18565 | 17247 | 18823 | 17556 | 801 | 17949 | 13089 | 19110 | 17513 | 27046 | 30279 |
|  | 15021 | 29401 | 24989 | 17514 | 10858 | 10818 | 3479 | 4143 | 10378 | 4632 | 9178 | 2393 |
|  | 7301 | 1809 | 22510 | 27581 | 21595 | 21511 | 28402 | 18333 | 14149 | 28805 | 8124 | 12756 |
|  | 17753 | 11831 | 26027 | 16476 | 16386 | 26489 | 15163 | 13733 | 3923 | 19642 | 6389 | 6823 |
|  | 25752 | 28598 | 22311 | 5696 | 13576 | 18989 | 18005 | 19035 | 18400 | 27154 | 26011 | 19284 |
|  | 15096 | 17219 | 28458 | 19408 | 29894 | 11092 | 7071 | 14604 | 1929 | 24823 | 27259 | 27345 |
|  | 27313 | 8416 | 28619 | 18451 | 26707 | 29744 | 18719 | 23137 | 22727 | 1066 | 5523 | 16584 |
|  | 6586 | 27578 | 26630 | 23970 | 21781 | 6527 | 28675 | 17515 | 29333 | 8419 | 3154 | 3912 |
|  | 10675 | 3836 | 1463 | 19990 | 5714 | 20221 | 21292 | 19001 | 24907 | 24815 | 17009 | 9981 |
|  | 13030 | 3180 | 3943 | 4081 | 14765 | 7847 | 19853 | 19964 | 20803 | 11362 | 20246 | 19356 |
|  | 3369 | 24552 | 23419 | 9889 | 25522 | 5023 | 4917 | 4950 | 6367 | 20271 | 12114 | 9892 |
|  | 11903 | 6336 | 6394 | 12112 | 9858 | 11819 | 2120 | 16733 | 16735 | 3628 | 9066 | 10260 |
|  | 7602 | 22142 | 26517 | 13111 | 16148 | 16116 | 21294 | 15158 | 1820 | 21776 | 18805 | 2381 |
|  | 29385 | 16328 | 16201 | 13238 | 802 | 8058 | 29637 | 16558 | 6936 | 3794 | 4734 | 12524 |
|  | 8781 | 12548 | 19999 | 19665 | 10099 | 11210 | 29429 | 27393 | 1231 | 10345 | 9815 | 22690 |
|  | 18504 | 24506 | 29503 | 931 | 2857 | 26975 | 2235 | 2939 | 23622 | 26655 | 29582 | 19895 |
|  | 23676 | 22548 | 19719 | 27658 | 21812 | 28195 | 13930 | 18230 | 12690 | 1233 | 10805 | 27312 |
|  | 2923 | 748 | 5787 | 6102 | 2500 | 27156 | 15973 | 15146 | 7620 | 1302 | 8327 | 2586 |
|  | 13724 | 17005 | 16912 | 17784 | 10972 | 11176 | 4238 | 16536 | 17915 | 29720 | 5105 | 5107 |
|  | 8011 | 3610 | 30154 | 4398 | 7309 | 3709 | 7746 | 7789 | 28563 | 14544 | 18612 | 28643 |
|  | 26991 | 14050 | 17791 | 20037 | 24311 | 28172 | 23603 | 21837 | 7506 | 28983 | 15095 | 29412 |
|  | 22345 | 22085 | 17376 | 18715 | 28266 | 13311 | 28112 | 16100 | 23382 | 10867 | 28015 | 18346 |
|  | 13740 | 7259 | 29519 | 8712 | 6622 | 18526 | 19524 | 2737 | 14530 | 10757 | 18893 | 17093 |
|  | 9988 | 18054 | 30228 | 24998 | 26908 | 24747 | 24743 | 15525 | 23364 | 24179 | 24031 | 10238 |
|  | 24035 | 11069 | 7332 | 15150 | 14379 | 14383 | 14385 | 7499 | 12095 | 12155 | 30147 | 15473 |
|  | 22580 | 1926 | 17636 | 3081 | 17017 | 9569 | 20024 | 15185 | 2305 | 11434 | 25250 | 7406 |
|  | 7396 | 11083 | 14429 | 24008 | 29334 | 9226 | 18929 | 23774 | 23006 | 23039 | 23048 | 25951 |
|  | 16647 | 4998 | 1905 | 10276 | 4669 | 1875 | 11784 | 11758 | 6163 | 15240 | 25044 | 22899 |
|  | 8346 | 13706 | 15607 | 29595 | 11743 | 11540 | 28418 | 27434 | 3716 | 2154 | 24827 | 27241 |
|  | 27236 | 27245 | 27266 | 27248 | 13161 | 13113 | 20989 | 23934 | 11066 | 4012 | | |
| 544: | 24344 | 24370 | 21966 | 4555 | 17232 | 26896 | 23433 | 17135 | 18282 | 10906 | 17412 | 13990 |

(continued)

SEQ ID NO: homolog SEQ ID NOs

|  |  |  |  |  |  |  |  |  |  |  |  |  |
|---|---|---|---|---|---|---|---|---|---|---|---|---|
|  | 14832 | 2274 | 22852 | 28368 | 14768 | 27216 | 21085 | 14723 | 14760 | 14728 | 2425 | 1171 |
|  | 12959 | 13213 | 26269 | 7677 | 14834 | 6334 | 8191 | 8998 | 8407 | 5448 | 9550 | 2546 |
|  | 21466 | 14378 | 5981 | 5802 | 10512 | 4106 | 19981 | 29987 | 3336 | 27849 | 29535 | 18515 |
|  | 13242 | 5853 | 8269 | 14968 | 1427 | 28378 | 6816 | 27557 | 26786 | 23911 | 27703 | 5247 |
|  | 20797 | 30224 | 11425 | 18523 | 27140 | 13959 | 22511 | 8378 | 3788 | 18482 | 13951 | 27877 |
|  | 27997 | 5940 | 12773 |  |  |  |  |  |  |  |  |  |
| 545: | 24344 | 24370 | 21966 | 4555 | 17232 | 26896 | 23433 | 17135 | 18282 | 10906 | 17412 | 13990 |
|  | 14832 | 2274 | 22852 | 28368 | 14768 | 27216 | 21085 | 14723 | 14760 | 14728 | 2425 | 1171 |
|  | 12959 | 13213 | 26269 | 7677 | 14834 | 6334 | 8191 | 8998 | 8407 | 5448 | 9550 | 2546 |
|  | 21466 | 14378 | 5981 | 5802 | 10512 | 4106 | 19981 | 29987 | 3336 | 27849 | 29535 | 18515 |
|  | 13242 | 5853 | 8269 | 14968 | 1427 | 28378 | 6816 | 27557 | 26786 | 23911 | 27703 | 5247 |
|  | 20797 | 30224 | 11425 | 18523 | 27140 | 13959 | 22511 | 8378 | 3788 | 18482 | 13951 | 27877 |
|  | 27997 | 5940 | 12773 |  |  |  |  |  |  |  |  |  |
| 546: | 26831 | 9714 | 6812 | 27172 | 2656 | 26835 | 28743 | 20018 | 11231 |  |  |  |
| 547: | 26831 | 9714 | 6812 | 27172 | 2656 | 26835 | 28743 | 20018 | 11231 |  |  |  |
| 548: | 21229 | 21232 | 20242 | 3177 | 26986 | 11630 | 1339 | 2516 | 8522 | 12967 | 5872 | 26078 |
|  | 17856 | 8924 | 21338 | 8930 | 20155 | 21257 | 1119 |  |  |  |  |  |
| 549: | 12222 | 1580 | 23967 | 16149 | 1246 | 3215 | 21371 | 26818 | 9208 | 19987 | 19129 | 17458 |
|  | 18964 | 3139 | 14576 | 30001 | 25644 | 19316 | 2401 | 29116 | 26138 | 7437 | 18319 | 20175 |
|  | 11198 | 3562 | 3538 | 5851 | 1259 | 27021 | 17663 | 22738 | 4228 | 14059 | 20781 | 25520 |
|  | 29564 | 26165 | 21278 | 4499 | 3894 | 7899 | 28549 | 19866 | 4068 | 6961 | 25421 | 27898 |
|  | 1703 | 9067 | 21927 | 8939 | 27756 | 19279 | 22477 | 22736 | 23743 | 18227 | 13929 | 14924 |
|  | 2860 | 25002 | 1785 | 21878 | 14811 | 21896 | 14997 | 25407 | 10274 | 17263 | 5634 | 13710 |
|  | 4622 | 13422 | 4574 | 6882 | 9640 | 7915 | 971 | 10547 | 13621 | 13831 | 27217 | 29905 |
|  | 16007 | 4870 | 25009 | 22197 | 24394 | 989 | 1128 | 17149 | 21449 | 26539 | 19413 | 6340 |
|  | 9186 | 21547 | 14366 | 21293 | 28820 | 13924 | 21049 | 4542 | 30177 | 18683 | 15958 | 23773 |
|  | 2626 | 30233 | 20165 | 26218 | 30310 | 8538 | 24752 | 29147 | 24325 | 20953 | 14720 | 9489 |
|  | 25919 | 2853 | 6781 | 26925 | 11701 | 27729 | 22819 | 26958 | 3275 | 23587 | 29746 | 8911 |
|  | 9632 | 1676 | 15853 | 13071 | 8674 | 2751 | 7094 | 19510 | 26364 | 7691 | 20427 | 13152 |
|  | 17658 | 11873 | 19969 | 5478 | 18419 | 25106 | 29367 | 5215 | 10759 | 24551 | 26955 | 800 |
|  | 22362 | 17110 | 26705 | 22163 | 24695 | 21181 | 24265 | 3523 | 29362 | 24661 | 9068 | 16407 |
|  | 18312 | 1845 | 722 | 26401 | 20890 | 26512 | 22231 | 18292 | 7011 | 14161 | 23131 | 26397 |
|  | 6570 | 3632 | 12984 | 14953 | 1718 | 13173 | 7530 | 4618 | 8672 | 5521 | 1852 | 14823 |

(continued)

| SEQ ID NO: | homolog SEQ ID NOs | | | | | | | | | | | |
|---|---|---|---|---|---|---|---|---|---|---|---|---|
| | 3222 | 18583 | 8842 | 16799 | 28249 | 3842 | 30245 | 7121 | 805 | 26407 | 10872 | 14467 |
| | 6810 | 20149 | 18045 | 23340 | 14898 | 26070 | 27781 | 20478 | 2591 | 10413 | 25419 | 6223 |
| | 11602 | 3496 | 3493 | 3469 | 2392 | 9826 | 13154 | 8516 | 17323 | 23081 | 24267 | 6404 |
| | 25503 | 18696 | 10795 | 10096 | 747 | 26030 | 29982 | 13292 | 20252 | 23365 | 26250 | 19795 |
| | 4113 | 24958 | 29125 | 9240 | 7101 | 22623 | 9466 | 25451 | 27005 | 19716 | 9726 | 8013 |
| | 3963 | 4227 | 8783 | 1768 | 30290 | 3376 | 1150 | 27507 | 2529 | 13486 | 26341 | 5896 |
| | 12996 | 6753 | 18297 | 17695 | 16909 | 3770 | 15236 | 19086 | 24939 | 19179 | 27778 | 10332 |
| | 24544 | 5466 | 6855 | 27210 | 3863 | 13941 | 23002 | 26279 | 27638 | 6651 | 12231 | 10288 |
| | 9017 | 28317 | 25653 | 23744 | 609 | 29440 | 3430 | 10911 | 27659 | 22635 | 21496 | 27943 |
| | 19206 | 21274 | 23058 | 6904 | 14014 | 6822 | 21830 | 13889 | 4489 | 16525 | 27894 | 6177 |
| | 14216 | 29023 | 16082 | 22698 | 7566 | 5965 | 13236 | 20860 | 18218 | | | |
| 550: | 12222 | 1580 | 23967 | 16149 | 1246 | 3215 | 21371 | 26818 | 9208 | 19987 | 19129 | 17458 |
| | 18964 | 3139 | 14576 | 30001 | 25644 | 19316 | 2401 | 29116 | 26138 | 7437 | 18319 | 20175 |
| | 11198 | 3562 | 3538 | 5851 | 1259 | 27021 | 17663 | 22738 | 4228 | 14059 | 20781 | 25520 |
| | 29564 | 26165 | 21278 | 4499 | 3894 | 7899 | 28549 | 19866 | 4068 | 6961 | 25421 | 27898 |
| | 1703 | 9067 | 21927 | 8939 | 27756 | 19279 | 22477 | 22736 | 23743 | 18227 | 13929 | 14924 |
| | 2860 | 25002 | 1785 | 21878 | 14811 | 21896 | 14997 | 25407 | 10274 | 17263 | 5634 | 13710 |
| | 4622 | 13422 | 4574 | 6882 | 9640 | 7915 | 971 | 10547 | 13621 | 13831 | 27217 | 29905 |
| | 16007 | 4870 | 25009 | 22197 | 24394 | 989 | 1128 | 17149 | 21449 | 26539 | 19413 | 6340 |
| | 9186 | 21547 | 14366 | 21293 | 28820 | 13924 | 21049 | 4542 | 30177 | 18683 | 15958 | 23773 |
| | 2626 | 30233 | 20165 | 26218 | 30310 | 8538 | 24752 | 29147 | 24325 | 20953 | 14720 | 9489 |
| | 25919 | 2853 | 6781 | 26925 | 11701 | 27729 | 22819 | 26958 | 3275 | 23587 | 29746 | 8911 |
| | 9632 | 1676 | 15853 | 13071 | 8674 | 2751 | 7094 | 19510 | 26364 | 7691 | 20427 | 13152 |
| | 17658 | 11873 | 19969 | 5478 | 18419 | 25106 | 29367 | 5215 | 10759 | 24551 | 26955 | 800 |
| | 22362 | 17110 | 26705 | 22163 | 24695 | 21181 | 24265 | 3523 | 29362 | 24661 | 9068 | 16407 |
| | 18312 | 1845 | 722 | 26401 | 20890 | 26512 | 22231 | 18292 | 7011 | 14161 | 23131 | 26397 |
| | 6570 | 3632 | 12984 | 14953 | 1718 | 13173 | 7530 | 4618 | 8672 | 5521 | 1852 | 14823 |
| | 3222 | 18583 | 8842 | 16799 | 28249 | 3842 | 30245 | 7121 | 805 | 26407 | 10872 | 14467 |
| | 6810 | 20149 | 18045 | 23340 | 14898 | 26070 | 27781 | 20478 | 2591 | 10413 | 25419 | 6223 |
| | 11602 | 3496 | 3493 | 3469 | 2392 | 9826 | 13154 | 8516 | 17323 | 23081 | 24267 | 6404 |
| | 25503 | 18696 | 10795 | 10096 | 747 | 26030 | 29982 | 13292 | 20252 | 23365 | 26250 | 19795 |
| | 4113 | 24958 | 29125 | 9240 | 7101 | 22623 | 9466 | 25451 | 27005 | 19716 | 9726 | 8013 |
| | 3963 | 4227 | 8783 | 1768 | 30290 | 3376 | 1150 | 27507 | 2529 | 13486 | 26341 | 5896 |

(continued)

SEQ ID NO: homolog SEQ ID NOs

| | | | | | | | | | | | |
|---|---|---|---|---|---|---|---|---|---|---|---|
| | 12996 | 6753 | 18297 | 17695 | 16909 | 3770 | 15236 | 19086 | 24939 | 19179 | 27778 | 10332 |
| | 24544 | 5466 | 6855 | 27210 | 3863 | 13941 | 23002 | 26279 | 27638 | 6651 | 12231 | 10288 |
| | 9017 | 28317 | 25653 | 23744 | 609 | 29440 | 3430 | 10911 | 27659 | 22635 | 21496 | 27943 |
| | 19206 | 21274 | 23058 | 6904 | 14014 | 6822 | 21830 | 13889 | 4489 | 16525 | 27894 | 6177 |
| | 14216 | 29023 | 16082 | 22698 | 7566 | 5965 | 13236 | 20860 | 18218 | | | |
| 551: | 12222 | 1580 | 23967 | 16149 | 1246 | 3215 | 21371 | 26818 | 9208 | 19987 | 19129 | 17458 |
| | 18964 | 3139 | 14576 | 30001 | 25644 | 19316 | 2401 | 29116 | 26138 | 7437 | 18319 | 20175 |
| | 11198 | 3562 | 3538 | 5851 | 1259 | 27021 | 17663 | 22738 | 4228 | 14059 | 20781 | 25520 |
| | 29564 | 26165 | 21278 | 4499 | 3894 | 7899 | 28549 | 19866 | 4068 | 6961 | 25421 | 27898 |
| | 1703 | 9067 | 21927 | 8939 | 27756 | 19279 | 22477 | 22736 | 23743 | 18227 | 13929 | 14924 |
| | 2860 | 25002 | 1785 | 21878 | 14811 | 21896 | 14997 | 25407 | 10274 | 17263 | 5634 | 13710 |
| | 4622 | 13422 | 4574 | 6882 | 9640 | 7915 | 971 | 10547 | 13621 | 13831 | 27217 | 29905 |
| | 16007 | 4870 | 25009 | 22197 | 24394 | 989 | 1128 | 17149 | 21449 | 26539 | 19413 | 6340 |
| | 9186 | 21547 | 14366 | 21293 | 28820 | 13924 | 21049 | 4542 | 30177 | 18683 | 15958 | 23773 |
| | 2626 | 30233 | 20165 | 26218 | 30310 | 8538 | 24752 | 29147 | 24325 | 20953 | 14720 | 9489 |
| | 25919 | 2853 | 6781 | 26925 | 11701 | 27729 | 22819 | 26958 | 3275 | 23587 | 29746 | 8911 |
| | 9632 | 1676 | 15853 | 13071 | 8674 | 2751 | 7094 | 19510 | 26364 | 7691 | 20427 | 13152 |
| | 17658 | 11873 | 19969 | 5478 | 18419 | 25106 | 29367 | 5215 | 10759 | 24551 | 26955 | 800 |
| | 22362 | 17110 | 26705 | 22163 | 24695 | 21181 | 24265 | 3523 | 29362 | 24661 | 9068 | 16407 |
| | 18312 | 1845 | 722 | 26401 | 20890 | 26512 | 22231 | 18292 | 7011 | 14161 | 23131 | 26397 |
| | 6570 | 3632 | 12984 | 14953 | 1718 | 13173 | 7530 | 4618 | 8672 | 5521 | 1852 | 14823 |
| | 3222 | 18583 | 8842 | 16799 | 28249 | 3842 | 30245 | 7121 | 805 | 26407 | 10872 | 14467 |
| | 6810 | 20149 | 18045 | 23340 | 14898 | 26070 | 27781 | 20478 | 2591 | 10413 | 25419 | 6223 |
| | 11602 | 3496 | 3493 | 3469 | 2392 | 9826 | 13154 | 8516 | 17323 | 23081 | 24267 | 6404 |
| | 25503 | 18696 | 10795 | 10096 | 747 | 26030 | 29982 | 13292 | 20252 | 23365 | 26250 | 19795 |
| | 4113 | 24958 | 29125 | 9240 | 7101 | 22623 | 9466 | 25451 | 27005 | 19716 | 9726 | 8013 |
| | 3963 | 4227 | 8783 | 1768 | 30290 | 3376 | 1150 | 27507 | 2529 | 13486 | 26341 | 5896 |
| | 12996 | 6753 | 18297 | 17695 | 16909 | 3770 | 15236 | 19086 | 24939 | 19179 | 27778 | 10332 |
| | 24544 | 5466 | 6855 | 27210 | 3863 | 13941 | 23002 | 26279 | 27638 | 6651 | 12231 | 10288 |
| | 9017 | 28317 | 25653 | 23744 | 609 | 29440 | 3430 | 10911 | 27659 | 22635 | 21496 | 27943 |
| | 19206 | 21274 | 23058 | 6904 | 14014 | 6822 | 21830 | 13889 | 4489 | 16525 | 27894 | 6177 |
| | 14216 | 29023 | 16082 | 22698 | 7566 | 5965 | 13236 | 20860 | 18218 | | | |
| 552: | 20679 | 27095 | 11456 | 6037 | 24678 | 13176 | 20239 | 6454 | 12097 | 14676 | 9690 | 9823 |

SEQ ID NO: homolog SEQ ID NOs

| SEQ ID NO: | | | | | | | | | | | | |
|---|---|---|---|---|---|---|---|---|---|---|---|---|
| | 20310 | 17762 | 22798 | 11860 | 23849 | 6843 | 2284 | 16844 | 24374 | 22669 | 13572 | 2671 |
| | 1012 | 5354 | 8046 | 5492 | 15568 | 12442 | 16350 | 1972 | 27149 | 29271 | 4545 | 20074 |
| | 11133 | 28994 | 19942 | 4584 | 23447 | 14937 | 4123 | 2839 | 10614 | 10636 | 9522 | 7846 |
| | 10727 | 13587 | 13193 | 14380 | 15392 | 9921 | 25143 | 7006 | 13932 | 15672 | 15886 | 25022 |
| | 2054 | 16024 | 6191 | 7449 | 13761 | 1673 | 5992 | 27107 | 21716 | 4002 | 20237 | 22997 |
| | 22465 | 24244 | 23174 | 7836 | 22692 | 5469 | 14992 | 9337 | 10862 | 29809 | 17526 | 9978 |
| | 19859 | 12892 | 10127 | 14331 | 19854 | 19426 | 5904 | 3591 | 19654 | 17052 | 13592 | 26429 |
| | 25525 | 2264 | 18570 | 7028 | 13226 | 9900 | 9896 | 13155 | 9789 | 9757 | 1108 | 1111 |
| | 22575 | 28509 | 1255 | 3347 | 17670 | 16050 | 6065 | 21343 | 3657 | 8049 | 14817 | 17607 |
| | 9155 | 18503 | 4396 | 15312 | 8056 | 7655 | 15075 | 28159 | 9737 | 3027 | 4787 | 27410 |
| | 18637 | 23565 | 29406 | 7935 | 1382 | 1364 | 1384 | 30305 | 12320 | 9322 | 25677 | 26888 |
| | 19020 | 26917 | 8759 | 29663 | 19066 | 13824 | 7407 | 6431 | 8165 | 2347 | 8525 | 24306 |
| | 18115 | 17527 | 24975 | 2704 | 22885 | 21924 | 1938 | 16008 | 17970 | 24450 | 28162 | 29143 |
| | 15692 | 9772 | 16043 | 15665 | 12047 | 15971 | 14816 | 12046 | 1023 | 10373 | 19660 | 15214 |
| | 24268 | 10153 | 28816 | 9478 | 24962 | 20724 | 13490 | 13484 | 13488 | 3274 | 8936 | 8692 |
| | 2026 | 28098 | | | | | | | | | | |
| 553: | 9016 | 25406 | 1477 | 7083 | 12769 | 15993 | 9839 | 9983 | 24060 | 1725 | 28340 | 24917 |
| | 2374 | 29611 | 27071 | 8248 | 16770 | 29730 | 13992 | 21797 | 6355 | 7334 | 25521 | 20509 |
| | 3689 | 24393 | 1720 | 4893 | 14061 | 4590 | 24691 | 13214 | 14680 | 27544 | 20854 | 28428 |
| | 10687 | 21875 | | | | | | | | | | |
| 554: | 17187 | 17178 | 19949 | 18620 | 3265 | 10774 | 7990 | 12490 | 30301 | 19896 | 14529 | 16324 |
| | 29095 | 6483 | 29345 | 26783 | 30210 | 11051 | 19511 | 12626 | 14394 | 13232 | 12746 | 26368 |
| | 21053 | 7345 | 28088 | 3967 | 27296 | 7195 | 19782 | 11786 | 25724 | | | |
| 555: | 17187 | 17178 | 19949 | 18620 | 3265 | 10774 | 7990 | 12490 | 30301 | 19896 | 14529 | 16324 |
| | 29095 | 6483 | 29345 | 26783 | 30210 | 11051 | 19511 | 12626 | 14394 | 13232 | 12746 | 26368 |
| | 21053 | 7345 | 28088 | 3967 | 27296 | 7195 | 19782 | 11786 | 25724 | | | |
| 556: | 16626 | 24956 | 19338 | 2731 | 1009 | 22399 | 5951 | 9998 | 3216 | 14005 | 1281 | 23083 |
| | 20076 | 1415 | 8139 | 22554 | 1084 | 29912 | 8789 | 2677 | 25174 | 7384 | 28963 | 4601 |
| | 3025 | 6683 | 6328 | 14294 | 12829 | 19305 | 22549 | 22703 | 3278 | 11706 | 14503 | 14697 |
| | 17720 | 25117 | 26023 | 2095 | 8963 | 14678 | 13190 | 22202 | 27237 | 19659 | 8691 | 1541 |
| | 17024 | 16236 | 11401 | 29221 | 10711 | 18753 | 20915 | 8472 | 18585 | 9069 | 5317 | 26635 |
| | 23619 | 10889 | 17907 | 5655 | 21174 | 1855 | 1192 | | | | | |
| 557: | 17126 | 17265 | 2172 | 29599 | | | | | | | | |

(continued)

SEQ ID NO: homolog SEQ ID NOs

| SEQ ID NO: | | | | | | | | | | | | |
|---|---|---|---|---|---|---|---|---|---|---|---|---|
| 558: | 23536 | 6864 | 5371 | 21674 | 1672 | 16353 | 4251 | 11436 | 20468 | 5680 | 4547 | 27549 |
| | 5325 | 6433 | 5055 | 4378 | 2303 | | | | | | | |
| 559: | 21015 | 20929 | 10560 | 24586 | 4051 | 3249 | 24052 | 21082 | 2403 | 8688 | 5948 | 9579 |
| | 15620 | 6080 | 23420 | 8770 | 20190 | 2252 | 9805 | 7353 | 8874 | 23190 | 23488 | 30121 |
| | 15860 | 23352 | 7355 | 14073 | 14068 | 20791 | 19149 | 20191 | 8293 | 1834 | 21265 | 12183 |
| | 28083 | 17608 | 1467 | 7670 | 20984 | 18022 | 17248 | 1337 | 26808 | 23772 | 1126 | 16782 |
| | 7016 | 12938 | 17744 | 16182 | 27163 | | | | | | | |
| 561: | 15651 | 2136 | 3530 | 13661 | 25924 | 15894 | 16409 | 7383 | 16880 | 1824 | | |
| 562: | 28236 | 2473 | 12678 | 29986 | 5293 | 25634 | 18962 | 25091 | 18878 | 10109 | 6084 | 9843 |
| | 26460 | 23299 | 15208 | 16701 | 10987 | 9380 | | | | | | |
| 563: | 21582 | 9166 | 28355 | 16301 | 12858 | 27796 | 1263 | 16277 | 23571 | 1202 | 1510 | 13854 |
| | 11351 | 1063 | 17094 | 24199 | 8338 | 1452 | 4372 | 21863 | | | | |
| 564: | 5366 | 11856 | 17610 | 26794 | 20646 | 7420 | 10477 | 30240 | 14368 | 6092 | | |
| 565: | 28601 | 828 | 16028 | 11915 | 1296 | 16755 | 17837 | 20616 | 18682 | | | |
| 566: | 28601 | 828 | 16028 | 11915 | 1296 | 16755 | 17837 | 20616 | 18682 | | | |
| 567: | 14921 | 19877 | 7810 | 30217 | 17337 | 28215 | 24755 | 11482 | 6624 | 12693 | 8705 | 19733 |
| | 27639 | 21076 | 14588 | 9767 | 12411 | 6164 | 7761 | 2953 | 25960 | 3042 | 17921 | 24577 |
| | 27679 | 26931 | 27923 | 13097 | 26207 | 16530 | 3040 | 9541 | 2047 | 5197 | | |
| 568: | 14921 | 19877 | 7810 | 30217 | 17337 | 28215 | 24755 | 11482 | 6624 | 12693 | 8705 | 19733 |
| | 27639 | 21076 | 14588 | 9767 | 12411 | 6164 | 7761 | 2953 | 25960 | 3042 | 17921 | 24577 |
| | 27679 | 26931 | 27923 | 13097 | 26207 | 16530 | 3040 | 9541 | 2047 | 5197 | | |
| 569: | 16930 | 17324 | 12946 | 27625 | 23317 | 24993 | 17619 | 2886 | 23790 | 30087 | 29723 | 19204 |
| | 10132 | 15139 | 29152 | 10861 | | | | | | | | |
| 570: | 16930 | 17324 | 12946 | 27625 | 23317 | 24993 | 17619 | 2886 | 23790 | 30087 | 29723 | 19204 |
| | 10132 | 15139 | 29152 | 10861 | | | | | | | | |
| 571: | 13684 | 1811 | 23804 | 22317 | 17870 | | | | | | | |
| 572: | 13684 | 1811 | 23804 | 22317 | 17870 | | | | | | | |
| 573: | 20434 | 15963 | 9429 | 13802 | 4455 | 3228 | 16468 | 9124 | 3167 | 12501 | 25140 | 5131 |
| | 6968 | | | | | | | | | | | |
| 574: | 20434 | 15963 | 9429 | 13802 | 4455 | 3228 | 16468 | 9124 | 3167 | 12501 | 12057 | 25140 |
| | 5131 | 6968 | | | | | | | | | | |
| 575: | 17357 | 28067 | 7956 | 21835 | 12227 | 26532 | 24683 | 6944 | 23263 | 21225 | 28489 | 28227 |
| | 3954 | 1336 | 15961 | 12509 | 14535 | 20291 | 21078 | 6204 | 22696 | 9349 | 7404 | 16325 |

(continued)

SEQ ID NO: homolog SEQ ID NOs

| | | | | | | | | | | | |
|---|---|---|---|---|---|---|---|---|---|---|---|
| | 719 | 5659 | 17400 | 12286 | 16498 | 13479 | 9110 | 15177 | 27100 | 21199 | 1295 | 8208 |
| | 9408 | 9407 | 25322 | 19994 | 15575 | 21542 | 17805 | 16263 | 24343 | 27304 | 24835 | 8584 |
| | 6493 | 23867 | 21396 | 15223 | 841 | 3204 | 9112 | 4852 | 28968 | 26212 | 16946 | 15877 |
| | 1143 | 15879 | 9574 | 16064 | 21379 | 15711 | 7857 | 19295 | 29924 | 22559 | 2976 | 13991 |
| | 4195 | 13995 | 5169 | 7400 | 28699 | 7141 | 16020 | 22868 | 4881 | 15662 | 10993 | 2679 |
| | 30136 | 11321 | 5383 | 12202 | 24768 | 5414 | 11058 | 6382 | 4259 | 5112 | 8778 | 16663 |
| | 9709 | 9951 | 10228 | 2522 | 29545 | 24058 | 24078 | 27069 | 27596 | 3535 | 20537 | 3536 |
| | 14405 | 12055 | 8822 | 11994 | 20354 | 22359 | 29048 | 3441 | 14941 | 3153 | 24770 | 23823 |
| | 24081 | 8087 | 8114 | 3416 | 17497 | 19555 | 19165 | 3337 | 9368 | 29479 | 30264 | 21439 |
| | 21387 | 21717 | 20513 | 18519 | 14581 | 12450 | 11335 | 21989 | 18609 | 20884 | 18359 | 9296 |
| | 19836 | 26158 | 26188 | 14229 | 16979 | 4491 | 16426 | 12035 | 18108 | 22743 | 12432 | 11622 |
| | 13070 | 23358 | 4549 | 18558 | 15289 | 3407 | 18111 | 23255 | 16203 | 20446 | 12464 | 19154 |
| | 25559 | 26550 | 23209 | 25170 | 24237 | 18919 | 10454 | 13601 | 7513 | 19967 | 17554 | 20608 |
| | 18393 | 21335 | 16499 | 8200 | 23878 | 7723 | 6199 | 4808 | 27917 | 4018 | 21193 | 9747 |
| | 28756 | 15756 | 19260 | 23219 | 14017 | 19663 | 7883 | 11651 | 30167 | 29106 | 17940 | 15941 |
| | 2720 | 30057 | 18069 | 889 | 24961 | 26638 | 2328 | 5858 | 3292 | 1460 | 14125 | 24645 |
| | 21349 | 24713 | 11443 | 9302 | 12188 | 14561 | 25935 | 3620 | 29373 | 29703 | 9098 | 25885 |
| | 23799 | 20943 | 28751 | 23180 | 27407 | 19955 | 24789 | 19991 | 19989 | 27927 | 28750 | 26167 |
| | 5506 | 25945 | 21108 | 7076 | 12570 | 17712 | 3775 | 27161 | 21312 | 10410 | 30170 | 29340 |
| | 5401 | 29656 | 1795 | 14079 | 6288 | 16975 | 12672 | 27603 | 24973 | 25612 | 26541 | 13370 |
| | 13857 | 1918 | 30097 | 23866 | 30080 | 30083 | 23851 | 1912 | 21386 | 20629 | 25068 | 5100 |
| | 6610 | 13822 | 9379 | 12007 | 19088 | 22910 | 19376 | 3519 | 17511 | 22859 | 28023 | 3146 |
| | 2570 | 6422 | 1576 | 4541 | 24574 | 17920 | 15870 | 8094 | 19167 | 18023 | | |
| 576: | 17357 | 28067 | 12227 | 21835 | 27701 | 21833 | 24656 | 12230 | 26532 | 24683 | 15818 | 28489 |
| | 28491 | 19678 | 1896 | 1336 | 28227 | 3954 | 15961 | 14535 | 12509 | 20291 | 21078 | 22696 |
| | 9349 | 7404 | 16325 | 16483 | 15189 | 719 | 5659 | 5662 | 9540 | 17400 | 12286 | 16498 |
| | 23537 | 13479 | 9110 | 27100 | 15549 | 14693 | 4877 | 4876 | 12318 | 3551 | 12496 | 4807 |
| | 9407 | 9408 | 25322 | 15579 | 19994 | 17646 | 21542 | 17805 | 1788 | 24343 | 16263 | 27304 |
| | 8584 | 6493 | 21663 | 15223 | 18982 | 23969 | 16818 | 29379 | 9252 | 841 | 27265 | 4165 |
| | 3204 | 9112 | 4852 | 28968 | 26212 | 26806 | 16946 | 15877 | 1143 | 16064 | 11703 | 19295 |
| | 29924 | 22559 | 13991 | 2976 | 1394 | 7400 | 5169 | 7141 | 21370 | 10802 | 4881 | 21936 |
| | 2150 | 11491 | 10993 | 29288 | 2679 | 11088 | 27843 | 22333 | 11321 | 5383 | 27284 | 6382 |
| | 11058 | 4259 | 5112 | 8778 | 8980 | 16663 | 9709 | 9951 | 6219 | 2522 | 3535 | 27596 |

EP 2 540 831 A2

168

SEQ ID NO: homolog SEQ ID NOs

| | | | | | | | | | | | | |
|---|---|---|---|---|---|---|---|---|---|---|---|---|
| | 20537 | 29580 | 7286 | 12055 | 11994 | 24637 | 9994 | 20354 | 11952 | 22359 | 17852 | 24770 |
| | 23442 | 23823 | 8087 | 8114 | 29140 | 2406 | 17497 | 19555 | 19165 | 9368 | 2046 | 21439 |
| | 21387 | 18343 | 20513 | 12450 | 11335 | 21989 | 18609 | 20884 | 18359 | 9296 | 19836 | 26158 |
| | 26188 | 20052 | 14229 | 16979 | 12035 | 26774 | 11774 | 16813 | 14670 | 7734 | 13070 | 23358 |
| | 28978 | 23486 | 20633 | 4549 | 25793 | 21814 | 11797 | 24890 | 15289 | 3407 | 8309 | 18111 |
| | 16203 | 21791 | 7582 | 20446 | 22396 | 6939 | 12464 | 27556 | 19154 | 25559 | 26550 | 23209 |
| | 24237 | 25170 | 18919 | 10454 | 13601 | 10521 | 19967 | 17554 | 18393 | 20608 | 21335 | 23064 |
| | 23878 | 7723 | 6199 | 9747 | 15756 | 12795 | 18177 | 19663 | 14017 | 11087 | 21527 | 7883 |
| | 19953 | 14559 | 30167 | 29106 | 17940 | 15941 | 17399 | 2720 | 30057 | 5181 | 889 | 20936 |
| | 18069 | 2328 | 5858 | 27479 | 27481 | 7807 | 27689 | 5284 | 24832 | 1572 | 3292 | 14125 |
| | 24645 | 24713 | 21349 | 11443 | 12188 | 9302 | 14561 | 25935 | 3620 | 25885 | 23180 | 20943 |
| | 23799 | 28751 | 24789 | 19955 | 20412 | 10068 | 19989 | 19991 | 27927 | 26195 | 28750 | 22073 |
| | 18871 | 7214 | 10970 | 16975 | 14079 | 24973 | 6288 | 24538 | 6982 | 2525 | 5100 | 6610 |
| | 17237 | 21236 | 3079 | 12007 | 13927 | 22910 | 3519 | 19376 | 12650 | 17511 | 4342 | 22859 |
| | 28023 | 3146 | 2570 | 6422 | 24574 | 27013 | 4784 | 11591 | 3731 | 28767 | 28623 | 17920 |
| | 15870 | 9610 | 18059 | 27814 | 27800 | | | | | | | |
| 577: | 26200 | 15551 | 29219 | 13471 | 9183 | 21569 | 11494 | 28457 | 29084 | 29086 | 10036 | 29079 |
| | 23175 | 29062 | 12271 | 12253 | 20553 | 18242 | 27602 | 27477 | 764 | 8811 | 1907 | 15910 |
| | 1932 | 23075 | 1934 | 12569 | 25289 | 22413 | 5230 | 20117 | 13581 | 14065 | 17477 | 16470 |
| | 8491 | 5642 | 9735 | 13446 | 28021 | 28306 | 21604 | 11430 | 25932 | 6008 | 21603 | 26801 |
| | 5071 | 9734 | 10151 | 10834 | 29263 | 11565 | 11136 | 24065 | 7953 | 5987 | 5324 | 13033 |
| | 24489 | 5302 | 13031 | 16965 | 23489 | 13192 | 19115 | 19085 | 19140 | 15653 | 19203 | 12735 |
| | 12716 | 19200 | 27429 | 6013 | 10089 | 9158 | 9187 | 9188 | 2600 | 9154 | 5982 | 26625 |
| | 19512 | 2791 | 24500 | 2788 | 24476 | 26374 | 2555 | 2554 | 2552 | 2550 | 5985 | 4747 |
| | 4748 | 5220 | 24581 | 10277 | 9763 | 21572 | 18639 | 25527 | 25529 | 9488 | 21273 | 13103 |
| | 12545 | 24014 | 15132 | 1734 | 14673 | 20844 | 19199 | 21568 | 25165 | 24748 | 9769 | 9814 |
| | 9810 | 9003 | 578 | 4116 | 12630 | 12628 | | | | | | |
| 578: | 26200 | 15551 | 29219 | 13471 | 9183 | 21569 | 11494 | 28457 | 29084 | 29086 | 10036 | 29079 |
| | 23175 | 29062 | 12271 | 12253 | 20553 | 18242 | 27602 | 27477 | 764 | 8811 | 1907 | 15910 |
| | 1932 | 23075 | 1934 | 12569 | 25289 | 22413 | 5230 | 20117 | 13581 | 14065 | 17477 | 16470 |
| | 5642 | 8491 | 26915 | 9735 | 13446 | 28021 | 28306 | 21604 | 11430 | 25932 | 6008 | 21603 |
| | 26801 | 5071 | 10151 | 9734 | 10834 | 29263 | 11565 | 11136 | 24065 | 7953 | 5987 | 5324 |
| | 13033 | 24489 | 5302 | 13031 | 16965 | 23489 | 13192 | 19085 | 15653 | 19115 | 19140 | 19203 |

(continued)

SEQ ID NO: homolog SEQ ID NOs

| | | | | | | | | | | | | |
|---|---|---|---|---|---|---|---|---|---|---|---|---|
| | 12735 | 12716 | 19200 | 27429 | 6013 | 10089 | 9158 | 9188 | 9187 | 2600 | 9154 | 5982 |
| | 26625 | 19512 | 2791 | 24500 | 2788 | 26374 | 24476 | 2555 | 2554 | 2552 | 2550 | 5985 |
| | 4747 | 4748 | 5220 | 24581 | 10277 | 9763 | 21572 | 18639 | 25527 | 25529 | 9488 | 21273 |
| | 13103 | 12545 | 24014 | 15132 | 1734 | 14673 | 20844 | 21568 | 25165 | 24748 | 9769 | 9814 |
| | 9810 | 12630 | 12628 | 577 | 19286 | 9003 | | | | | | |
| 579: | 767 | 10169 | 20323 | 3389 | 11583 | 4270 | 28704 | 3679 | 6470 | 25218 | 10460 | 4573 |
| | 2313 | 4568 | 10066 | 12913 | 27421 | 21364 | 21280 | 25257 | 5260 | 17430 | 16908 | 23197 |
| | 10584 | 13385 | 10559 | 17587 | 13500 | 22735 | 11845 | 16682 | 5462 | 23469 | 16840 | 5740 |
| | 2019 | 15419 | 15160 | 2314 | 11350 | 25348 | 3193 | 26275 | 27524 | 12762 | 20287 | 3062 |
| | 5392 | 9774 | 27085 | 22542 | 8170 | 23198 | 5943 | 3777 | 25833 | 4471 | | |
| 580: | 16568 | 24113 | 14954 | 16466 | 23777 | 3160 | 27879 | 12081 | 16380 | 12726 | 8031 | 4667 |
| | 11612 | 13159 | 9899 | 26535 | 20497 | 18176 | 24675 | 8468 | 4680 | 26457 | 18094 | 8267 |
| | 22253 | 9987 | 7418 | 24733 | 15919 | 20026 | 16961 | 3713 | 7895 | 3642 | 24337 | 17069 |
| | 17073 | 25103 | 19052 | 13304 | 22663 | 23645 | 23771 | | | | | |
| 581: | 26639 | 26608 | 22976 | 10144 | 10108 | 24335 | 1423 | 7867 | 2089 | 1195 | 19775 | 29851 |
| | 851 | 1043 | 1161 | 7581 | 28377 | 24467 | 18003 | 20850 | 4332 | 1994 | 1073 | 28019 |
| | 7711 | 4297 | 28730 | 18237 | 27536 | 29770 | 8039 | 14103 | 23810 | 19651 | 17559 | 14399 |
| | 26850 | 22863 | 27509 | 24808 | 25008 | 22751 | 2169 | 13768 | 29885 | 13683 | 4341 | 25028 |
| | 26862 | 6564 | 13196 | 3640 | 16477 | 26172 | 29273 | 6260 | 30219 | 18921 | 10407 | 17328 |
| | 11307 | 25566 | 12520 | 813 | 21571 | 14010 | 4935 | 1025 | 30126 | 10607 | 10742 | 6910 |
| | 9918 | 6942 | 6900 | 8176 | 1687 | 2947 | 2074 | 26905 | 1048 | 17775 | 12591 | 16698 |
| | 21485 | 9808 | 18761 | 10910 | 883 | 24856 | 19319 | 20677 | 25874 | 22502 | 3987 | 23080 |
| | 27967 | 29205 | 26109 | 15707 | 12646 | 16131 | 16753 | 20177 | 970 | 10726 | 13399 | 11030 |
| | 15746 | 24955 | 19892 | 9113 | 27451 | 3437 | 25815 | 1748 | 933 | 19278 | 25288 | 24493 |
| | 5435 | 13146 | 4502 | 27683 | 13588 | 26809 | 12686 | 20815 | 18048 | 12002 | 4166 | 23315 |
| | 15418 | 24811 | 12170 | 20671 | 22013 | 9950 | 8010 | 21813 | 15959 | 14372 | 22051 | 30200 |
| | 11026 | 22252 | 8925 | 11968 | 21198 | 16984 | 15179 | 1709 | 1761 | 14774 | 23079 | 24864 |
| | 2742 | 27705 | 18961 | 19107 | 14171 | 26431 | 28809 | 21764 | 27598 | 13353 | 2647 | 9499 |
| | 24559 | 21391 | 18138 | 7664 | 10596 | 20687 | 11872 | 14563 | 22011 | 4929 | 1798 | 1697 |
| | 5124 | 14092 | 28102 | 28157 | 2190 | 20369 | 7674 | 16019 | 14533 | 754 | 8413 | 30101 |
| | 2076 | 20794 | 21472 | 3462 | 6073 | 10610 | 8434 | 24704 | 19395 | 23668 | 10111 | 1042 |
| | 947 | 26939 | 12683 | 28644 | 12401 | 12123 | 14447 | 9224 | 6891 | 6218 | 23543 | 30037 |
| | 7710 | 16181 | 7947 | 11880 | 10104 | 7416 | 8180 | 11615 | 9453 | 9485 | 26509 | 774 |

(continued)

SEQ ID NO: homolog SEQ ID NOs

| | | | | | | | | | | | | |
|---|---|---|---|---|---|---|---|---|---|---|---|---|
| | 4021 | 25171 | 25397 | 22095 | 5433 | 5111 | 22075 | 28579 | 26253 | 9449 | 8769 | 24396 |
| | 18900 | 1890 | 30296 | 8282 | 13885 | 15780 | 14165 | 13943 | 15861 | 15291 | 24433 | 18327 |
| | 24130 | 12247 | 15814 | 26185 | 23980 | 27785 | 29078 | 29585 | 23679 | 20338 | 2196 | 1393 |
| | 1236 | 3088 | 15298 | 28038 | 22581 | 17488 | 23410 | 24395 | 6496 | 9414 | 13165 | 17447 |
| | 20193 | 6830 | 29393 | 6930 | 15079 | 19089 | 24269 | 27199 | 20931 | 29096 | 21903 | 21353 |
| | 14053 | 15922 | 13994 | 18006 | 17834 | 22308 | 7343 | 10605 | 12508 | 22395 | 26465 | 25914 |
| | 888 | 28788 | 1964 | 2650 | 21016 | 17349 | 11365 | 13225 | 22284 | 6772 | 11712 | 5592 |
| | 3514 | 25941 | 19493 | 8310 | 6992 | 10737 | 5149 | 11817 | 8787 | 4183 | 12799 | 15570 |
| | 9140 | 23501 | 25498 | 7297 | 19805 | 26612 | 22816 | 27411 | 12067 | 21288 | 8473 | 7738 |
| | 2025 | 22895 | 21799 | 24209 | 22016 | 27099 | 26473 | 11421 | 26859 | 19202 | 16163 | 14284 |
| | 19148 | 22476 | 755 | 23161 | 30294 | 1794 | 2843 | 18466 | 11545 | 12398 | 7826 | 11585 |
| | 29255 | 10004 | 10088 | 21227 | 26392 | 8921 | 13053 | 11367 | 23233 | 21442 | 25828 | 19745 |
| | 24532 | 26733 | 7841 | 27937 | 3792 | 27904 | 10007 | 21077 | 8839 | 11573 | 22037 | 3365 |
| | 1421 | 24876 | 21942 | 2175 | 23303 | 25043 | 5810 | 19389 | 1234 | 12915 | 2680 | 12391 |
| | 1365 | 18587 | 28668 | 24208 | 24602 | 17259 | 28673 | 10365 | 9462 | 26525 | 23513 | 26352 |
| | 14164 | 11497 | 14326 | 18029 | 9172 | 22078 | 16509 | 6870 | 14555 | 14778 | 18196 | 18679 |
| | 28543 | 29738 | 26074 | 2042 | 26031 | 27568 | 17813 | 5654 | 28369 | 27570 | 27090 | 3993 |
| | 17728 | 9697 | 6863 | 19300 | 23521 | 28583 | 26645 | 24791 | 29434 | 22808 | 24340 | 22305 |
| | 19274 | 22884 | 26792 | 16737 | 23262 | 7159 | 13366 | 2414 | 15535 | 4540 | 4388 | 13174 |
| | 4395 | 4387 | 27300 | 10594 | 17010 | 4362 | 30183 | 23031 | 8262 | 27186 | 30203 | 13692 |
| | 16825 | 4276 | 16846 | 30318 | 22877 | 5329 | 15890 | 10588 | 17719 | 14911 | 20278 | 18316 |
| | 17829 | 25276 | 8728 | 13923 | 9967 | 24481 | 21207 | 9119 | 6600 | 29471 | 27605 | 16229 |
| | 2592 | 30133 | 24763 | 1226 | 18167 | 7745 | 23043 | 3210 | 28315 | 8746 | 8300 | 21506 |
| | 8876 | 8871 | 17045 | 26725 | 20658 | 705 | 18461 | 9710 | 3310 | 20711 | 21045 | 15429 |
| | 19698 | 7050 | 27364 | 17080 | 15318 | 13149 | 1954 | 24949 | 19734 | 8948 | 6106 | 394 |
| | 20098 | 20415 | 25735 | 22651 | 22739 | 19495 | 20493 | 20490 | 7894 | 2517 | 15841 | 7519 |
| | 18844 | 11384 | 16169 | 26979 | 28715 | 1598 | 14027 | 6694 | 4306 | 5860 | 23756 | 6529 |
| | 30050 | 5781 | 1213 | 1310 | 12103 | 11739 | 7276 | 24829 | 23720 | 18885 | 24455 | 7374 |
| | 18210 | 12280 | 11969 | 11664 | | | | | | | | |
| 582: | 26639 | 26608 | 22976 | 10144 | 10108 | 24335 | 1423 | 7867 | 2089 | 1195 | 19775 | 29851 |
| | 851 | 1043 | 1161 | 7581 | 28377 | 24467 | 18003 | 20850 | 4332 | 1994 | 1073 | 28019 |
| | 7711 | 4297 | 28730 | 18237 | 27536 | 29770 | 8039 | 14103 | 23810 | 19651 | 17559 | 14399 |
| | 26850 | 22863 | 27509 | 24808 | 25008 | 22751 | 2169 | 13768 | 29885 | 13683 | 4341 | 25028 |

(continued)

SEQ ID NO: homolog SEQ ID NOs

| | | | | | | | | | | | |
|---|---|---|---|---|---|---|---|---|---|---|---|
| 26862 | 6564 | 13196 | 3640 | 16477 | 26172 | 29273 | 6260 | 30219 | 18921 | 10407 | 17328 |
| 11307 | 25566 | 12520 | 813 | 21571 | 14010 | 4935 | 1025 | 30126 | 10607 | 10742 | 6910 |
| 9918 | 6942 | 6900 | 8176 | 1687 | 2947 | 2074 | 26905 | 1048 | 17775 | 12591 | 16698 |
| 21485 | 9808 | 18761 | 10910 | 883 | 24856 | 19319 | 20677 | 25874 | 22502 | 3987 | 23080 |
| 27967 | 29205 | 26109 | 15707 | 12646 | 16131 | 16753 | 20177 | 970 | 10726 | 13399 | 11030 |
| 15746 | 24955 | 19892 | 9113 | 27451 | 3437 | 25815 | 1748 | 933 | 19278 | 25288 | 24493 |
| 5435 | 13146 | 4502 | 27683 | 13588 | 26809 | 12686 | 20815 | 18048 | 12002 | 4166 | 23315 |
| 15418 | 24811 | 12170 | 20671 | 22013 | 9950 | 8010 | 21813 | 15959 | 14372 | 22051 | 30200 |
| 11026 | 22252 | 8925 | 11968 | 21198 | 16984 | 15179 | 1709 | 1761 | 14774 | 23079 | 24864 |
| 2742 | 27705 | 18961 | 19107 | 14171 | 26431 | 28809 | 21764 | 27598 | 13353 | 2647 | 9499 |
| 24559 | 21391 | 18138 | 7664 | 10596 | 20687 | 11872 | 14563 | 22011 | 4929 | 1798 | 1697 |
| 5124 | 14092 | 28102 | 28157 | 2190 | 20369 | 7674 | 16019 | 14533 | 754 | 8413 | 30101 |
| 2076 | 20794 | 21472 | 3462 | 6073 | 10610 | 8434 | 24704 | 19395 | 23668 | 10111 | 1042 |
| 947 | 26939 | 12683 | 28644 | 12401 | 12123 | 14447 | 9224 | 6891 | 6218 | 23543 | 30037 |
| 7710 | 16181 | 7947 | 11880 | 10104 | 7416 | 8180 | 11615 | 9453 | 9485 | 26509 | 774 |
| 4021 | 25171 | 25397 | 22095 | 5433 | 5111 | 22075 | 28579 | 26253 | 9449 | 8769 | 24396 |
| 18900 | 1890 | 30296 | 8282 | 13885 | 15780 | 14165 | 13943 | 15861 | 15291 | 24433 | 18327 |
| 24130 | 12247 | 15814 | 26185 | 23980 | 27785 | 29078 | 29585 | 23679 | 20338 | 2196 | 1393 |
| 1236 | 3088 | 15298 | 28038 | 22581 | 17488 | 23410 | 24395 | 6496 | 9414 | 13165 | 17447 |
| 20193 | 6830 | 29393 | 6930 | 15079 | 19089 | 24269 | 27199 | 20931 | 29096 | 21903 | 21353 |
| 14053 | 15922 | 13994 | 18006 | 17834 | 22308 | 7343 | 10605 | 12508 | 22395 | 26465 | 25914 |
| 888 | 28788 | 1964 | 2650 | 21016 | 17349 | 11365 | 13225 | 22284 | 6772 | 11712 | 5592 |
| 3514 | 25941 | 19493 | 8310 | 6992 | 10737 | 5149 | 11817 | 8787 | 4183 | 12799 | 15570 |
| 9140 | 23501 | 25498 | 7297 | 19805 | 26612 | 22816 | 27411 | 12067 | 21288 | 8473 | 7738 |
| 2025 | 22895 | 21799 | 24209 | 22016 | 27099 | 26473 | 11421 | 26859 | 19202 | 16163 | 14284 |
| 19148 | 22476 | 755 | 23161 | 30294 | 1794 | 2843 | 18466 | 11545 | 12398 | 7826 | 11585 |
| 29255 | 10004 | 10088 | 21227 | 26392 | 8921 | 13053 | 11367 | 23233 | 21442 | 25828 | 19745 |
| 24532 | 26733 | 7841 | 27937 | 3792 | 27904 | 10007 | 21077 | 8839 | 11573 | 22037 | 3365 |
| 1421 | 24876 | 21942 | 2175 | 23303 | 25043 | 5810 | 19389 | 1234 | 12915 | 2680 | 12391 |
| 1365 | 18587 | 28668 | 24208 | 24602 | 17259 | 28673 | 10365 | 9462 | 26525 | 23513 | 26352 |
| 14164 | 11497 | 14326 | 18029 | 9172 | 22078 | 16509 | 6870 | 14555 | 14778 | 18196 | 18679 |
| 28543 | 29738 | 26074 | 2042 | 26031 | 27568 | 17813 | 5654 | 28369 | 27570 | 27090 | 3993 |
| 17728 | 9697 | 6863 | 19300 | 23521 | 28583 | 26645 | 24791 | 29434 | 22808 | 24340 | 22305 |

172

(continued)

SEQ ID NO: homolog SEQ ID NOs

| SEQ ID NO: | | | | | | | | | | | | |
|---|---|---|---|---|---|---|---|---|---|---|---|---|
| | 19274 | 22884 | 26792 | 16737 | 23262 | 7159 | 13366 | 2414 | 15535 | 4540 | 4388 | 13174 |
| | 4395 | 4387 | 27300 | 10594 | 17010 | 4362 | 30183 | 23031 | 8262 | 27186 | 30203 | 13692 |
| | 16825 | 4276 | 16846 | 30318 | 22877 | 5329 | 15890 | 10588 | 17719 | 14911 | 20278 | 18316 |
| | 17829 | 25276 | 8728 | 13923 | 9967 | 24481 | 21207 | 9119 | 6600 | 29471 | 27605 | 16229 |
| | 2592 | 30133 | 24763 | 1226 | 18167 | 7745 | 23043 | 3210 | 28315 | 8746 | 8300 | 21506 |
| | 8876 | 8871 | 17045 | 26725 | 20658 | 705 | 18461 | 9710 | 3310 | 20711 | 21045 | 15429 |
| | 19698 | 7050 | 27364 | 17080 | 15318 | 13149 | 1954 | 24949 | 19734 | 8948 | 6106 | 394 |
| | 20098 | 20415 | 25735 | 22651 | 22739 | 19495 | 20493 | 20490 | 7894 | 2517 | 15841 | 7519 |
| | 18844 | 11384 | 16169 | 26979 | 28715 | 1598 | 14027 | 6694 | 4306 | 5860 | 23756 | 6529 |
| | 30050 | 5781 | 1213 | 1310 | 12103 | 11739 | 7276 | 24829 | 23720 | 18885 | 24455 | 7374 |
| | 18210 | 12280 | 11969 | 11664 | | | | | | | | |
| 583: | 22000 | 21734 | 18132 | 18161 | 18156 | 18155 | 20166 | 1228 | 5998 | 5995 | 5967 | 5961 |
| | 5971 | 1612 | 1614 | 27654 | 1316 | 3276 | 3280 | 3286 | 9362 | 24082 | 24104 | 21698 |
| | 21732 | 21768 | 1839 | 1112 | 15070 | 23223 | 12644 | 1315 | 1275 | 1333 | 11002 | 19238 |
| | 1344 | 7593 | 17341 | 27118 | 853 | 11978 | 29435 | 29163 | 19558 | 19583 | 19586 | 19591 |
| | 19592 | 16381 | 12560 | 24201 | 21669 | 27550 | 27498 | 24728 | 4084 | 4107 | 8131 | 22790 |
| | 22380 | 18802 | 15628 | 4989 | 14403 | 13125 | 12260 | 13110 | 13131 | 12255 | 13133 | 12308 |
| | 12285 | 13108 | 13104 | 12311 | 12314 | 12254 | 12376 | 13102 | 12221 | 12316 | 12257 | 12374 |
| | 17737 | 24783 | 1956 | 4613 | 4610 | 4615 | 5335 | 5337 | 5339 | 4609 | 5333 | 5334 |
| | 8305 | 18168 | 1359 | 27430 | 24797 | 29108 | 15071 | 17338 | 19650 | 15112 | 15108 | 21825 |
| | 21083 | 18769 | 8231 | 11776 | 12484 | 14127 | 25513 | 14211 | 22297 | 23445 | 28629 | 28625 |
| | 4918 | 16692 | 18197 | 11464 | 23187 | 23204 | 11609 | 28662 | 7217 | 3303 | 8103 | 5241 |
| | 14894 | 15047 | 30039 | 2428 | 3185 | 13558 | 7704 | 28879 | 5911 | 10698 | 29470 | 25393 |
| | 23152 | 11029 | 28815 | 28071 | 740 | 15697 | 14998 | 17547 | 23517 | 23519 | 23522 | 23525 |
| | 21726 | 4562 | 11117 | 1772 | 17423 | 29690 | 15159 | 19171 | 13708 | 4013 | 16794 | 8836 |
| | 23256 | 28207 | 12685 | 13250 | 16125 | 17100 | 5721 | 22127 | 22130 | 23943 | 12903 | 23956 |
| | 22247 | 27269 | 23242 | 18940 | 15114 | 3651 | 3510 | 1026 | 25326 | 15064 | 4075 | 26122 |
| | 21752 | 16132 | 17825 | 9665 | 2580 | 7751 | 14169 | 13205 | 29267 | 29264 | 1449 | 29854 |
| | 29857 | 25690 | 9036 | 24063 | 2670 | 10578 | 6329 | 17242 | 21907 | 18328 | 4130 | 20353 |
| | 13917 | 21967 | 6619 | 9006 | 12841 | 2879 | 4740 | 28047 | 2798 | 27946 | 5490 | 5526 |
| | 10131 | 29678 | 7715 | 19043 | 29646 | 26708 | 5542 | 6672 | 9089 | 18162 | 6264 | 4536 |
| | 29795 | 20355 | 19759 | 8224 | 3223 | 15674 | 29951 | 1358 | 7787 | 10879 | 8426 | 27998 |
| | 23794 | 25704 | 13247 | 9804 | 24026 | 18332 | 1764 | 11970 | 18166 | 3161 | 1911 | 21754 |

SEQ ID NO: homolog SEQ ID NOs

| | | | | | | | | | | | |
|---|---|---|---|---|---|---|---|---|---|---|---|
| 11659 | 20909 | 12029 | 10309 | 18034 | 9829 | 12174 | 7989 | 10256 | 21373 | 25412 | 4096 |
| 27463 | 2461 | 8661 | 5083 | 19173 | 25357 | 19138 | 20885 | 24803 | 29533 | 10444 | 22033 |
| 11593 | 17210 | 20520 | 16734 | 2071 | 5261 | 14634 | 30213 | 22264 | 14241 | 5328 | 22254 |
| 29197 | 9044 | 28414 | 14139 | 6603 | 11135 | 27609 | 15385 | 24429 | 21636 | 24088 | 9993 |
| 19095 | 6588 | 27664 | 25938 | 21524 | 5312 | 26992 | 25949 | 3914 | 4768 | 8762 | 11514 |
| 11543 | 8008 | 25088 | 5682 | 28787 | 6417 | 8360 | 11962 | 15091 | 27171 | 12363 | 18078 |
| 18071 | 18012 | 12358 | 12330 | 12337 | 18076 | 18073 | 18046 | 12390 | 18041 | 12365 | 18068 |
| 12333 | 18037 | 1254 | 1251 | 9650 | 8581 | 20691 | 22367 | 28094 | 3246 | 3367 | 9366 |
| 27563 | 1605 | 11012 | 13080 | 14227 | 13128 | 17885 | 18365 | 9744 | 6002 | 1870 | 8532 |
| 3707 | 18774 | 25003 | 23115 | 16798 | 1117 | 12926 | 12274 | 27192 | 10466 | 25553 | 10264 |
| 6676 | 14776 | 17125 | 22337 | 29682 | 16552 | 4789 | 10388 | 23201 | 22232 | 25270 | 18106 |
| 19671 | 20933 | 2341 | 23633 | 23638 | 2856 | 9537 | 25541 | 25780 | 27004 | 728 | 22319 |
| 27721 | 14221 | 26021 | 24107 | 24109 | 20911 | 2077 | 17365 | 20231 | 29591 | 6917 | 30184 |
| 18093 | 18596 | 3826 | 18441 | 18446 | 18443 | 1651 | 22469 | 6085 | 7832 | 11212 | 11789 |
| 15237 | 20728 | 9213 | 25918 | 8239 | 20171 | 20169 | 20145 | 6472 | 8123 | 9883 | 22611 |
| 894 | 29496 | 24313 | 19648 | 15003 | 18192 | 17674 | 17669 | 7537 | 3650 | 27355 | 27353 |
| 1556 | 1577 | 1573 | 27357 | 1552 | 28450 | 23415 | 18296 | 18295 | 18291 | 1613 | 1664 |
| 6437 | 26483 | 7835 | 6814 | 15571 | 29763 | 26558 | 20591 | 12847 | 5731 | 10349 | 27262 |
| 27428 | 27337 | 10358 | 10404 | 10452 | 10530 | 10527 | 10498 | 10458 | 7462 | 28777 | 3420 |
| 22360 | 430 | 429 | 428 | 15823 | 24221 | 1370 | 28580 | 17633 | 5016 | 11852 | 11850 |
| 2382 | 27425 | 1313 | 16317 | 25676 | 3065 | 30271 | 14744 | 17750 | 3752 | 9919 | 28921 |
| 28924 | 28927 | 28948 | 28951 | 25166 | 2204 | 12798 | 14871 | 3935 | 24817 | 5885 | 28682 |
| 20105 | 5012 | 19416 | 20712 | 21756 | 8591 | 11745 | 11505 | 14095 | 11536 | 13342 | 19662 |
| 948 | 11001 | 29485 | 29502 | 29505 | 29506 | 29509 | 29522 | 29525 | 29527 | 17570 | 4134 |
| 16728 | 3458 | 11752 | 19879 | 10952 | 10978 | 10954 | 23205 | 25890 | 24099 | 17973 | 30076 |
| 18251 | 2084 | 27398 | 25721 | 25388 | 10251 | 29447 | 27395 | 14542 | 21735 | 20952 | 10222 |
| 14538 | 21741 | 6941 | 29871 | 10661 | 10199 | 5796 | 22712 | 19291 | 7759 | 15301 | 27392 |
| 8075 | 23128 | 4481 | 1145 | 27702 | 4799 | 27318 | 27327 | 27325 | 27356 | 16228 | 7580 |
| 11010 | 27726 | 26408 | 10249 | 13035 | 12992 | 5637 | 25369 | 25332 | 25296 | 27764 | 25370 |
| 25329 | 25293 | 17136 | 17134 | 6645 | 25335 | 4975 | 25367 | 25252 | 25339 | 25290 | 25285 |
| 5052 | 5047 | 5009 | 5021 | 5019 | 25282 | 25365 | 5053 | 25325 | 5017 | 5051 | 14956 |
| 9510 | 12995 | 12269 | 13001 | 13006 | 13005 | 13003 | 10363 | 10351 | 10326 | 11275 | 11272 |
| 10323 | 29529 | 29532 | 10216 | 18883 | 11268 | 10461 | 10433 | 10185 | 9191 | 18861 | 18855 |

SEQ ID NO: homolog SEQ ID NOs

| SEQ ID NO | | | | | | | | | | | | |
|---|---|---|---|---|---|---|---|---|---|---|---|---|
| | 18848 | 18816 | 18811 | 30255 | 18850 | 11279 | 9152 | 10494 | 10492 | 10487 | 27993 | 10465 |
| | 18744 | 18813 | 18888 | 18913 | 5738 | 21738 | 20959 | 14537 | 12325 | 11916 | 14108 | |
| 584: | 2750 | 19612 | 5924 | 15741 | 22353 | 10164 | 22355 | 10188 | 22015 | 4885 | 28600 | 6557 |
| | 29025 | 14338 | 8725 | 11735 | 8954 | 12625 | 29181 | 26899 | 27902 | 5283 | 9199 | 5269 |
| | 4604 | 19385 | | | | | | | | | | |
| 585: | 20774 | 29066 | 965 | 27001 | 18806 | 13506 | 17414 | 6392 | 20481 | 20462 | 21184 | 19340 |
| | 22484 | 7700 | 17705 | 16487 | 12265 | 14193 | 16290 | 11467 | 4441 | 16250 | 19178 | 21884 |
| | 14687 | 16516 | 27062 | 2595 | 28512 | 25083 | 13093 | 28120 | 29530 | 13460 | 2158 | 7237 |
| | 19914 | 15803 | 26346 | 23374 | 16106 | 19136 | 29355 | 28304 | 4377 | 4015 | 25749 | 28367 |
| | 4258 | 3839 | 22079 | 25249 | 6716 | 15625 | 28517 | 21585 | 17603 | 1639 | 10904 | 28880 |
| | 23491 | 20660 | 27650 | 20492 | 11011 | 12853 | 20253 | 3219 | 14373 | 27091 | 29262 | 27652 |
| | 2530 | 9492 | 16863 | 5236 | 27676 | 7644 | 27414 | 26163 | 11045 | 24946 | 24885 | 1140 |
| | 12359 | 14519 | 12844 | 3213 | 25420 | 28079 | 1643 | 27617 | 19215 | 13231 | 20987 | 19920 |
| | 21390 | 1169 | 9898 | 18610 | 27375 | 14046 | 16249 | 12386 | 30270 | 28546 | 1113 | 24805 |
| | 22570 | 8825 | 12291 | 1198 | 18918 | 2700 | 12324 | 1114 | 9586 | 5901 | 16630 | 16446 |
| | 3130 | 10376 | 20877 | 7652 | 6575 | 16628 | 5180 | 18782 | 8319 | 4367 | 21844 | 15523 |
| | 20765 | 12385 | 25877 | 29223 | 3163 | 18832 | 26125 | 2852 | 18532 | 23487 | | |
| 586: | 2182 | 24474 | 30166 | 27861 | 9554 | 2028 | 6424 | 17904 | 6918 | 13786 | 27305 | 19429 |
| | 7378 | 8606 | 9088 | 5182 | 29826 | 2051 | 8408 | 21961 | 12251 | 5144 | 19579 | 19235 |
| | 4458 | 12042 | 20084 | 10505 | 4201 | 3150 | 18514 | 1532 | 25575 | 25601 | 10248 | 24777 |
| | 15280 | 29967 | 23659 | 22588 | 14382 | 14280 | 20782 | 25537 | 23182 | 7185 | 18763 | 5246 |
| | 11191 | 7863 | 29169 | 6434 | 29239 | 27486 | 3233 | 8353 | 3821 | 27833 | 6632 | 20540 |
| | 12070 | 26977 | 12700 | 1265 | 23931 | 18563 | 18576 | 22497 | 12031 | 24097 | 24764 | 8547 |
| | 2831 | 9615 | 5410 | 24310 | 27119 | 24074 | 6909 | 19226 | 27017 | 7097 | 28031 | 20902 |
| | 7752 | 25673 | 19333 | 16094 | 25095 | 29003 | 17968 | | | | | |
| 587: | 4906 | 27043 | 22565 | 9840 | 11127 | 29890 | 16772 | 25911 | 9010 | 7147 | 30325 | 17893 |
| | 28286 | 11405 | 9221 | 15077 | 27103 | 22109 | 7699 | 13483 | 25795 | 21378 | 15251 | 13062 |
| | 24114 | 8227 | 10292 | 19412 | 28829 | 26409 | 22415 | 25479 | 14773 | 30316 | 21301 | 13371 |
| | 6225 | 26856 | 23384 | 6725 | 8152 | 21953 | 19993 | 8096 | 10225 | 15439 | 2551 | 17697 |
| | 28946 | 4683 | 2376 | 11004 | 25396 | 2620 | 22021 | 25211 | 4136 | 6026 | 24252 | 28819 |
| | 3621 | 15942 | 19961 | 22175 | 16363 | 23386 | 8679 | 13294 | 22904 | 20855 | 29814 | 27565 |
| | 1989 | 19026 | 20111 | 26607 | 11590 | 26618 | 29717 | 2616 | 5161 | 27869 | 25517 | 26151 |
| | 25832 | 7498 | 4246 | 19706 | 26981 | 29007 | 9799 | 28506 | 5438 | 25821 | 739 | 27436 |

(continued)

| SEQ ID NO: | homolog SEQ ID NOs | | | | | | | | | | |
|---|---|---|---|---|---|---|---|---|---|---|---|
| | 24972 | 4418 | 5233 | 13548 | 25600 | 7117 | 12627 | 17615 | 11982 | 22039 | 23858 | 11383 |
| | 11478 | 18492 | 14502 | 12226 | 19249 | 15906 | 28231 | 4969 | 9274 | 1282 | 1533 | 12822 |
| | 23009 | 28097 | 19723 | 9780 | 9254 | 21251 | 2864 | 7329 | 15701 | 4235 | 20225 | 17890 |
| | 2114 | 11792 | 13042 | 924 | 7775 | 29576 | 29662 | 17258 | 8848 | 13285 | 4523 | 25913 |
| | 25936 | 14145 | | | | | | | | | | |
| 588: | 24712 | 21369 | 3368 | 29028 | 21642 | 24686 | 26048 | 3812 | 16604 | 20411 | |
| 589: | 28781 | 20092 | 11146 | 1724 | 11598 | 12594 | 3674 | 3678 | 19262 | 19259 | 19253 | 11778 |
| | 22462 | 21477 | 1624 | 21691 | 21697 | 12918 | 28851 | 4421 | 3023 | 28486 | 23506 | 4794 |
| | 14112 | 22158 | 17845 | 10051 | 27889 | 26180 | 13204 | 25582 | 12077 | 22029 | 16851 | 16852 |
| | 26796 | 24550 | 10730 | 24236 | 20087 | 20582 | 15453 | 14926 | 13931 | 26153 | 7253 | 17171 |
| | 2219 | 8946 | 16187 | 26584 | 5199 | 23116 | 22182 | 26919 | 11600 | 9133 | 27582 | 25136 |
| | 23976 | 14126 | 10548 | 19028 | 10443 | 6446 | 6451 | 26264 | 20673 | 27252 | 12896 | 20077 |
| | 14289 | 2109 | 15195 | 2582 | 13948 | 840 | 956 | 1515 | 22964 | 12529 | 6873 | 3360 |
| | 22525 | 28857 | 23789 | 6408 | 3016 | 3019 | 2979 | 3022 | 22473 | 3141 | 7325 | 7565 |
| | 24032 | 17003 | 22461 | 16242 | 1216 | 3448 | 17851 | 9860 | 17133 | 1212 | 5990 | 18379 |
| | 25349 | 24622 | 1597 | 7646 | 1636 | 23027 | 28107 | 11158 | 13464 | 11175 | 17645 | 8304 |
| | 18119 | 6009 | 6395 | 3780 | 22057 | 24128 | 26370 | 26171 | 27009 | 29244 | 22406 | 20554 |
| | 6935 | 24996 | 5286 | 8244 | 8063 | | | | | | | |
| 590: | 28781 | 20092 | 11146 | 1724 | 11598 | 12594 | 3674 | 3678 | 19262 | 19259 | 19253 | 11778 |
| | 22462 | 21477 | 1624 | 21691 | 21697 | 12918 | 28851 | 4421 | 3023 | 28486 | 23506 | 4794 |
| | 14112 | 22158 | 17845 | 10051 | 27889 | 26180 | 13204 | 25582 | 12077 | 22029 | 16851 | 16852 |
| | 26796 | 24550 | 10730 | 24236 | 20087 | 20582 | 15453 | 14926 | 13931 | 26153 | 7253 | 17171 |
| | 2219 | 8946 | 16187 | 26584 | 5199 | 23116 | 22182 | 26919 | 11600 | 9133 | 27582 | 25136 |
| | 23976 | 14126 | 10548 | 19028 | 10443 | 6446 | 6451 | 26264 | 20673 | 27252 | 12896 | 20077 |
| | 14289 | 2109 | 15195 | 2582 | 13948 | 840 | 956 | 1515 | 22964 | 12529 | 6873 | 3360 |
| | 22525 | 28857 | 23789 | 6408 | 3016 | 3019 | 2979 | 3022 | 22473 | 3141 | 7325 | 7565 |
| | 24032 | 17003 | 22461 | 16242 | 1216 | 3448 | 17851 | 9860 | 17133 | 1212 | 5990 | 18379 |
| | 25349 | 24622 | 1597 | 7646 | 1636 | 23027 | 28107 | 11158 | 13464 | 11175 | 17645 | 8304 |
| | 18119 | 6009 | 6395 | 3780 | 22057 | 24128 | 26370 | 26171 | 27009 | 29244 | 22406 | 20554 |
| | 6935 | 24996 | 5286 | 8244 | 8063 | | | | | | | |
| 591: | 7852 | 20628 | 8292 | 12287 | 30027 | 16791 | 25554 | 2989 | 30018 | 28564 | 24884 | 12380 |
| | 13235 | 26206 | 26658 | 1981 | 4774 | 4742 | 1638 | 24954 | 13709 | 29476 | 26286 | 12986 |
| | 13421 | 19624 | 12891 | 15729 | 4706 | 9440 | 27221 | 29942 | 20365 | 22116 | 19816 | 16716 |

(continued)

| SEQ ID NO: | homolog SEQ ID NOs | | | | | | | | | | |
|---|---|---|---|---|---|---|---|---|---|---|---|
|  | 24873 | 11578 | 20401 | 25528 | 14018 | 10849 | 4168 | 28283 | 18080 | 23782 | 11399 |
|  | 6427 | 2327 | 25191 | 8997 | 5227 | 12465 | 27634 | 29685 | 15231 | 23599 | 23825 |
|  | 21544 | 5746 | 2756 | 3872 | 15111 | 18622 | 21394 | 7799 | 17039 | 14192 | 12732 |
|  | 24242 | 8020 | 22234 | 16084 | 3804 | 28356 | 4200 | 16610 | 11420 | 4249 | 9903 |
|  | 20120 | 27000 | 6768 | 1683 | 19229 | 22065 | 17167 | 8423 | 23575 | 15457 | 19957 |
|  | 21798 | 27218 | 16355 | 12532 | 10031 | 7128 | 5096 | 14416 | 11943 | 8088 | 17774 |
|  | 2900 | 24941 | 5382 | 12048 | 29727 | 3509 | 20766 | 18397 | 1237 | 19347 | 28905 |
|  | 17706 | 17886 | 24405 | 1535 | 18605 | 14575 | 20429 | 20180 | 21031 | 24038 | 8430 |
|  | 14615 | 28238 | 4565 | 16419 | 15749 | 12574 | 23671 | 20524 | 21407 | 18667 | 26240 |
|  | 19882 | 24807 | 9511 | 18992 | 19543 | 10304 | 26820 | 3252 | 10285 | 27955 | 12446 |
|  | 4358 | 11533 | 15592 | 23356 | 25260 | 18506 | 6488 | 17770 | 14016 | 27555 | 20686 |
|  | 790 | 22765 | 3584 | 20420 | 10436 | 26054 | 28945 | 21593 | 17018 | 6481 | 17764 |
|  | 11209 | 7641 | 30214 | 9854 | 20146 | 21870 | 2278 | 6005 | 4206 | 20795 | 20417 |
|  | 15459 | 18147 | 4482 | 2984 | 29777 | 8598 | 4296 | 13682 | 964 | 27783 | 28004 |
|  | 19058 | 1771 | 12497 | 20383 | 25526 | 5923 | 28370 | 9581 | 9060 | 27972 | 28636 |
|  | 15748 | 16787 | 17771 | 8732 | 28920 | 6724 | 9991 | 13178 | 25500 | 27087 | 9696 |
|  | 18321 | 21910 | 22978 | 11116 | 11334 | 7411 | 24282 |  |  |  |  |
| 592: | 7331 | 2574 | 18239 | 29467 | 18245 | 23114 | 1936 | 2039 | 5265 | 21473 | 29697 |
|  | 11670 | 8790 | 26637 | 27657 | 10142 | 14854 | 1904 | 12119 | 6924 | 21820 | 16893 |
|  | 17594 | 25867 | 21481 | 3683 | 8373 | 14764 | 11013 | 5151 | 9535 | 14748 | 14788 |
|  | 24964 | 21926 | 1937 | 16683 | 15630 | 29131 | 9526 | 4606 | 22908 | 8791 | 11638 |
|  | 25048 | 9139 | 9803 | 18787 | 25049 | 5195 | 26353 | 26375 | 16661 | 15882 | 30291 |
|  | 10159 | 7129 | 25642 | 15762 | 15765 | 10473 | 10470 | 14609 | 27331 | 19992 | 3850 |
|  | 12203 | 26803 | 25225 | 25230 | 25971 | 3659 | 18378 | 9336 | 22431 | 22332 | 8069 |
|  | 28406 | 27636 | 27526 | 9831 | 7015 | 4337 | 13954 | 22115 | 882 | 6568 | 18058 |
|  | 2944 | 23306 | 1038 | 2113 | 2641 | 17261 | 26009 | 27214 | 23917 | 23239 | 14244 |
|  | 10118 | 7629 | 23045 | 23699 | 14253 | 21753 | 5644 | 30012 | 30011 | 26323 | 22566 |
|  | 30282 | 18383 | 9717 | 9719 | 27748 | 2487 | 17903 | 21886 | 28947 | 24105 | 3999 |
|  | 1056 | 15256 | 19668 | 9384 | 14001 | 6965 | 25756 | 28871 | 8484 | 21554 | 14916 |
| 593: | 28464 | 19453 | 1193 | 22673 | 29731 | 2590 | 30155 | 22257 | 19931 | 7194 | 7510 |
|  | 7199 | 27914 | 1677 | 22988 | 4817 | 12289 | 22125 | 4655 | 19382 | 4696 | 5732 |
|  | 23564 | 14905 | 2032 | 11629 | 5931 | 18636 | 24462 | 1729 | 25659 | 15621 | 19137 |
|  | 14311 | 1831 | 1833 | 1838 | 12668 | 17725 | 7243 | 9468 | 21020 | 6130 | 19691 |

SEQ ID NO: homolog SEQ ID NOs

| 21658 | 24182 | 17883 | 3092 | 7618 | 28529 | 17790 | 12041 | 26969 | 11273 | 30306 | 16891 |
|---|---|---|---|---|---|---|---|---|---|---|---|
| 11836 | 9137 | 1294 | 24328 | 6585 | 12021 | 8162 | 28740 | 14196 | 25441 | 26152 | 24386 |
| 8222 | 13868 | 29475 | 25591 | 28800 | 979 | 14695 | 13826 | 10009 | 17929 | 29359 | 21405 |
| 8993 | 19972 | 10831 | 19443 | 16103 | 28052 | 20638 | 12868 | 12765 | 10247 | 12877 | 8565 |
| 30320 | 1647 | 9394 | 6457 | 16097 | 26576 | 28218 | 23035 | 11849 | 23168 | 5480 | 15230 |
| 8935 | 26689 | 8449 | 20622 | 20627 | 6615 | 18381 | 7778 | 12577 | 17175 | 4504 | 14928 |
| 6602 | 3460 | 13647 | 2027 | 1719 | 6952 | 11800 | 26366 | 26201 | 29710 | 16998 | 20209 |
| 11846 | 25187 | 13055 | 13633 | 5674 | 14517 | 7373 | 7178 | 28027 | 11282 | 28183 | 22947 |
| 18648 | 29269 | 10839 | 9798 | 16356 | 1154 | 14686 | 13024 | 11949 | 3915 | 3374 | 18188 |
| 3073 | 23582 | 12125 | 20460 | 24432 | 6517 | 26555 | 8651 | 12104 | 22042 | 15119 | 11898 |
| 21597 | 19418 | 19421 | 11901 | 7479 | 19336 | 6041 | 20700 | 17238 | 20164 | 17087 | 5500 |
| 18545 | 24919 | 13988 | 16195 | 12850 | 22391 | 26086 | 16221 | 13665 | 17384 | 14892 | 20480 |
| 3982 | 11811 | 6842 | 17841 | 11640 | 4991 | 21418 | 21476 | 16447 | 29311 | 23249 | 20014 |
| 19940 | 23071 | 24083 | 4834 | 1620 | 12875 | 9852 | 25929 | 15778 | 8259 | 6540 | 7342 |
| 7697 | 8677 | 26739 | 27802 | 17923 | 17922 | 8958 | 4047 | 9881 | 25201 | 23258 | 17967 |
| 23413 | 23998 | 13419 | 12676 | 7617 | 20032 | 26842 | 9376 | 6283 | 3934 | 15472 | 5447 |
| 20345 | 24709 | 6459 | 22513 | 22379 | 11552 | 19100 | 7855 | 19454 | 30275 | 24579 | 14147 |
| 21406 | 16424 | 1652 | 27274 | 25313 | 25410 | 9355 | 16334 | 29964 | 4241 | 19748 | 24502 |
| 23296 | 923 | 22001 | 22002 | 18879 | 24245 | 25267 | 15447 | 26632 | 20729 | 17689 | 17506 |
| 17509 | 26234 | 29289 | 29933 | 26303 | 4681 | 18131 | 19303 | 15700 | 28929 | 6723 | 25275 |
| 6024 | 26260 | 28298 | 8973 | 16808 | 10167 | 25587 | 26092 | 10396 | 2122 | 2850 | 27633 |
| 22442 | 7509 | 26531 | 13823 | 3653 | 3008 | 28054 | 27589 | 21243 | 21321 | 14320 | 25295 |
| 6894 | 6476 | 28334 | 10668 | 1220 | 16950 | 5153 | 5277 | 7870 | 21850 | 28202 | 5747 |
| 8488 | 8486 | 8480 | 12883 | 4344 | 19584 | 19461 | 10599 | 7351 | 10121 | 24154 | 10070 |
| 11986 | 2942 | 19948 | 14675 | 12583 | 1030 | 1034 | 12849 | 26647 | 833 | 3662 | 13785 |
| 11493 | 23793 | 19078 | 27809 | 10394 | 7208 | 25716 | 1867 | 13418 | 25298 | 15131 | 7492 |
| 19407 | 20747 | 4048 | 863 | 26001 | 22601 | 7088 | 18775 | 10163 | 27958 | 23312 | 22071 |
| 7698 | 7019 | 12902 | 6475 | 9927 | 9668 | 11904 | 4030 | 2510 | 1522 | 5409 | 8308 |
| 9651 | 25202 | 18000 | 14549 | 27363 | 20186 | 7825 | 10203 | 12975 | 7823 | 22304 | 26066 |
| 5773 | 10382 | 9180 | 19616 | 10119 | 16450 | 22339 | 5842 | 8794 | 9856 | 28881 | 24305 |
| 21149 | 25294 | 10064 | 19139 | 18079 | 17425 | 25835 | 22612 | 8638 | 8805 | 16137 | 4557 |
| 7732 | 7226 | 14063 | 14553 | 19414 | 19420 | 6636 | 3848 | 17451 | 17704 | 2842 | 24162 |
| 3138 | 5079 | 11140 | 16983 | 15706 | 11061 | 28253 | | | | | |

SEQ ID NO: homolog SEQ ID NOs

| 594: | 5955 | 2138 | 24870 | 12345 | 5028 | 7869 | 15905 | 13470 | 22845 | 16208 | 20868 | 28484 |
| | 25636 | 27114 | 4638 | 13816 | 2237 | 4778 | 6707 | 14243 | 15200 | 600 | 599 | 598 |
| | 24426 | 23429 | 27173 | 29852 | 23544 | 14466 | 25360 | 8626 | | | | |
| 595: | 5955 | 2138 | 24870 | 12345 | 5028 | 7869 | 15905 | 13470 | 22845 | 16208 | 20868 | 28484 |
| | 25636 | 27114 | 4638 | 13816 | 2237 | 4778 | 6707 | 14243 | 15200 | 600 | 599 | 598 |
| | 24426 | 23429 | 27173 | 29852 | 23544 | 14466 | 25360 | 8626 | | | | |
| 596: | 5955 | 2138 | 24870 | 12345 | 5028 | 7869 | 15905 | 13470 | 22845 | 16208 | 20868 | 28484 |
| | 25636 | 27114 | 4638 | 13816 | 2237 | 4778 | 6707 | 14243 | 15200 | 600 | 599 | 598 |
| | 24426 | 23429 | 27173 | 29852 | 23544 | 14466 | 25360 | 8626 | | | | |
| 597: | 5955 | 2138 | 24870 | 12345 | 5028 | 7869 | 15905 | 13470 | 22845 | 16208 | 20868 | 28484 |
| | 25636 | 27114 | 4638 | 13816 | 2237 | 4778 | 6707 | 14243 | 15200 | 600 | 599 | 598 |
| | 24426 | 23429 | 27173 | 29852 | 23544 | 14466 | 25360 | 8626 | | | | |
| 598: | 5955 | 2780 | 2138 | 5028 | 7869 | 15905 | 13470 | 22845 | 16208 | 28484 | 25636 | 27114 |
| | 4638 | 13816 | 2237 | 23429 | 27173 | 597 | 596 | 595 | 594 | 25360 | 14466 | 8626 |
| | 29852 | 21965 | 23544 | 15200 | 14243 | | | | | | | |
| 599: | 5955 | 2780 | 2138 | 5028 | 7869 | 15905 | 13470 | 22845 | 16208 | 28484 | 25636 | 27114 |
| | 4638 | 13816 | 2237 | 23429 | 27173 | 597 | 596 | 595 | 594 | 25360 | 14466 | 8626 |
| | 29852 | 21965 | 23544 | 15200 | 14243 | | | | | | | |
| 600: | 5955 | 2780 | 2138 | 5028 | 7869 | 15905 | 13470 | 22845 | 16208 | 28484 | 25636 | 27114 |
| | 4638 | 13816 | 2237 | 23429 | 27173 | 597 | 596 | 595 | 594 | 25360 | 14466 | 8626 |
| | 29852 | 21965 | 23544 | 15200 | 14243 | | | | | | | |
| 601: | 2871 | 7311 | 3598 | 5550 | 25927 | 13052 | 22762 | 19587 | 5178 | 5179 | 4930 | 5622 |
| | 16364 | 15868 | 23066 | 2560 | 28731 | 13645 | 13669 | 3869 | 25416 | 5988 | 27188 | 24739 |
| | 2872 | 2873 | 9708 | 4494 | 23572 | 25147 | 25148 | 28911 | 28118 | 1610 | 9785 | 9782 |
| | 8772 | 10902 | 492 | 491 | 25865 | 27368 | 6711 | 10689 | 21223 | 16987 | 17504 | 21431 |
| | 7381 | 6713 | 6305 | 893 | 1218 | 30008 | 16665 | 9751 | 9778 | 14353 | 25707 | 8776 |
| | 18930 | 9864 | 5555 | 5558 | 5596 | 9868 | 9871 | 25264 | 27500 | 19061 | 28533 | 5042 |
| | 9730 | 7240 | 19829 | 5546 | 29776 | 9033 | 11085 | 4375 | 22499 | 28833 | 24352 | 5518 |
| | 15962 | 10835 | 8543 | 3457 | 5548 | 29707 | 15480 | 7062 | 8142 | 26583 | 27033 | 886 |
| | 14917 | 4373 | 4374 | 29789 | 6499 | 18249 | | | | | | |
| 602: | 7984 | 8450 | 17096 | 8024 | 27384 | 1653 | 28106 | 14024 | 12897 | 12953 | 17858 | 21635 |
| | 11553 | 1903 | 29210 | 9847 | 6295 | 1508 | 16527 | 1829 | 16838 | 7958 | 17335 | 9684 |
| 603: | 30250 | 30114 | 26298 | 20371 | 27558 | 2589 | 25130 | 17668 | 1091 | 27868 | 13241 | 23160 |

(continued)

SEQ ID NO: homolog SEQ ID NOs

| | | | | | | | | | | | | |
|---|---|---|---|---|---|---|---|---|---|---|---|---|
| | 14190 | 24011 | 18967 | 29677 | 5661 | 10612 | 25005 | 12353 | 10817 | 6452 | 23740 | 24903 |
| | 2177 | 27098 | 24077 | 1696 | 6593 | 29243 | 2876 | 28834 | 1377 | 14093 | 13067 | 16323 |
| | 18976 | 2302 | 3405 | 12261 | 8307 | 11521 | 10112 | 9309 | 1663 | 2637 | 1420 | 7654 |
| | 3814 | 7622 | 10848 | 28217 | 3956 | 7818 | 2689 | 14498 | 21680 | 23690 | 15259 | 22674 |
| | 5353 | 11294 | 7120 | 11934 | 21877 | 16444 | 16726 | 24071 | 5880 | 6857 | 13720 | 6411 |
| | 27272 | 21080 | 14441 | 17320 | 21183 | 27341 | 22390 | 13262 | 20642 | 21964 | 22289 | 20717 |
| | 11990 | 5495 | 2492 | 1777 | 21363 | 14853 | 16926 | 13913 | 9750 | 4953 | 19447 | 27740 |
| | 7065 | 18550 | 11625 | 13950 | 21186 | 18630 | 26120 | 26047 | 2633 | 4080 | 17044 | 1301 |
| | 6750 | 6749 | 25665 | 7736 | 21258 | 21700 | 14271 | 11507 | 3284 | 10577 | 18325 | 21088 |
| | 4485 | 23683 | 22759 | 10093 | 24190 | 6976 | 9691 | 1760 | 20963 | 757 | 29821 | 13319 |
| | 14174 | 27176 | 8043 | 6623 | 27758 | 14400 | 10223 | 9011 | 23253 | 2740 | 11179 | 1104 |
| | 17595 | 10630 | 28225 | 15330 | 4073 | 11131 | 21435 | 6835 | 10102 | 4931 | 27854 | 4027 |
| | 1879 | 28273 | 3334 | 12491 | 29032 | 16749 | 20152 | 7158 | 28776 | 819 | 21627 | 11678 |
| | 21685 | 24283 | 26194 | 15056 | 17553 | 1859 | 18990 | 1267 | 4163 | 13463 | 17500 | 18004 |
| | 13148 | 26247 | 3066 | 3860 | 8023 | 17406 | 24461 | 14235 | 7157 | 22873 | 23383 | 11763 |
| | 18908 | 15617 | 25478 | 16507 | 17627 | 22045 | 13893 | 7942 | 18284 | 30230 | 24226 | 2474 |
| | 20899 | 21932 | 6758 | 20086 | 24028 | 17392 | 22271 | 12084 | 8546 | 15728 | 5888 | 25570 |
| | 3576 | 1324 | 16427 | 13611 | 10718 | 23505 | 9496 | 20559 | 12568 | 23854 | 23507 | 18420 |
| | 23354 | 21914 | 25375 | 14419 | 21983 | 24543 | 26278 | 19166 | 17079 | 13028 | 13585 | 28742 |
| | 26756 | 26997 | 14488 | 7415 | 13749 | 19208 | 14652 | 1789 | 18939 | 11621 | 7233 | 21134 |
| | 8824 | 20031 | 17304 | 23154 | 19335 | 1757 | 28277 | 2116 | 20373 | 8172 | 13215 | 13545 |
| | 10968 | 16439 | 15874 | 2282 | 6678 | 6400 | 20867 | 22521 | 28710 | 26721 | 3295 | 26252 |
| | 3846 | 20777 | 27081 | 25548 | 5262 | 8278 | 18972 | 10372 | 24312 | 12161 | 12777 | 14562 |
| | 2785 | 17546 | 29260 | 29734 | 12368 | 10418 | 18593 | 23816 | 24761 | 7115 | 1640 | 27309 |
| | 14574 | 5907 | 14223 | 27674 | 29270 | 20095 | 18664 | 19806 | 23200 | 20219 | 26479 | 14486 |
| | 1495 | 20983 | 28824 | 20358 | 4985 | 28688 | 8263 | 22889 | 17117 | 3528 | 21761 | 9353 |
| | 19196 | 26846 | 3427 | 26848 | 22772 | 11931 | 17128 | 28555 | 11157 | 27168 | 25606 | 16112 |
| | 23005 | 22747 | 8112 | 16974 | 8512 | 8943 | 1184 | 18018 | 20337 | 24836 | 11972 | 25830 |
| | 11951 | 20938 | 1388 | 2514 | 23779 | 23819 | 22056 | 23366 | 3359 | 4148 | 7409 | 17956 |
| | 27789 | 12387 | 12373 | 4797 | 18827 | 8497 | 20825 | 27055 | 10331 | 18086 | 16045 | 9363 |
| 604: | 20849 | 13444 | 3393 | 22192 | 4673 | 22340 | 4133 | 25844 | 2105 | 11530 | 11534 | 11535 |
| | 19442 | 17368 | 11518 | 6767 | 18351 | 17307 | 11271 | 17409 | 16623 | 20832 | 17366 | 29051 |
| | 27158 | 25025 | 28003 | 22636 | 21998 | 23387 | 1342 | 16715 | 12259 | 5393 | 21514 | 15822 |

SEQ ID NO: homolog SEQ ID NOs

| | | | | | | | | | | |
|---|---|---|---|---|---|---|---|---|---|---|
| 15824 | 21021 | 6082 | 5211 | 23040 | 21958 | 22970 | 12671 | 6425 | 16457 | 5809 | 8303 |
| 16459 | 18171 | 24614 | 23520 | 17177 | 14032 | 13377 | 14117 | 10823 | 18235 | 1363 | 1258 |
| 6036 | 22243 | 19098 | 13695 | 13134 | 23803 | 18638 | 5421 | 5427 | 10280 | 11959 | 24378 |
| 10286 | 10314 | 5297 | 15487 | 21060 | 20594 | 16454 | 20472 | 14330 | 24061 | 4440 | 15623 |
| 17792 | 10741 | 25820 | 21971 | 14116 | 19377 | 25487 | 18209 | 21528 | 14465 | 6232 | 3622 |
| 9425 | 7919 | 6233 | 12091 | 12116 | 18995 | 7927 | 21328 | 4034 | 7558 | 20644 | 3658 |
| 28801 | 24610 | 6416 | 12412 | 16606 | 26643 | 13972 | 20334 | 18623 | 16874 | 4901 | 25734 |
| 1057 | 10664 | 22132 | 7382 | 10582 | 13701 | 8666 | 15438 | 12738 | 1270 | 12279 | 10227 |
| 18592 | 18261 | 18524 | 8476 | 19082 | 2906 | 24850 | 2178 | 23162 | 19092 | 19207 | 16577 |
| 29155 | 8641 | 16777 | 20254 | 22961 | 5730 | 28635 | 3760 | 15896 | 14980 | 7638 | 1821 |
| 11624 | 4089 | 14343 | 6234 | 19130 | 1631 | 18294 | 2009 | 20459 | 25758 | 26096 | 11895 |
| 5094 | 19862 | 6747 | 13473 | 30289 | 4822 | 20377 | 7702 | 8132 | 28321 | 29306 | 29866 |
| 8947 | 5991 | 12185 | 12436 | 23737 | 7909 | 9671 | 27136 | 18229 | 11832 | 13773 | 26394 |
| 7004 | 8969 | 7707 | 23298 | 29104 | 28373 | 7576 | 21102 | 2365 | 6650 | 24093 | 19580 |
| 27734 | 2615 | 16336 | 25050 | 18142 | 22647 | 29959 | 16601 | 29844 | 13918 | 17053 | 28329 |
| 23689 | 23362 | 24443 | 18691 | 2563 | 29750 | 13882 | 15807 | 29396 | 15355 | 25792 | 7386 |
| 2987 | 24076 | 25715 | 19847 | 16241 | 3824 | 9421 | 12403 | 29063 | 22342 | 25993 | 27939 |
| 15433 | 17327 | 18121 | 25497 | 8885 | 25751 | 13624 | 15912 | 14743 | 22607 | 22210 | 19380 |
| 18314 | 10863 | 8592 | 27872 | 2935 | 2156 | 4039 | 23004 | 4476 | 30302 | 8716 | 16220 |
| 2260 | 23217 | 24286 | 16057 | 735 | 1040 | 27795 | 29706 | 27986 | 12598 | 7876 | 2918 |
| 5476 | 28188 | 15025 | 21422 | 21359 | 4382 | 21714 | 4379 | 20458 | 11596 | 28922 | 6339 |
| 13700 | 4414 | 4376 | 23478 | 25925 | 15640 | 22781 | 10278 | 10284 | 10252 | 28771 | 28395 |
| 25848 | 30010 | 24576 | 19191 | 26476 | 18608 | 23963 | 25873 | 16173 | 3746 | 19893 | 13017 |
| 25729 | 13191 | 12688 | 25864 | 14601 | 26038 | 24650 | 20410 | 5043 | 25817 | 17356 | 15789 |
| 17199 | 5394 | 5396 | 5424 | 10255 | 10259 | 25100 | 29337 | 10040 | 24721 | 21133 | 15560 |
| 26867 | 19943 | 29865 | 23681 | 17522 | 21957 | 4520 | 4558 | 17512 | 3507 | 8012 | 29407 |
| 5289 | 20036 | 23360 | 17614 | 17592 | 14248 | 8579 | 25312 | 7590 | 2340 | 1781 | 13830 |
| 22897 | 23991 | 9417 | 7507 | 16385 | 24523 | 21339 | 2753 | 17939 | 22507 | 12418 | 26187 |
| 16389 | 24373 | 22528 | 16393 | 12050 | 21960 | 21962 | 29923 | 13092 | 20245 | 8967 | 29420 |
| 7440 | 10966 | 4891 | 27799 | 25812 | 4763 | 4765 | 3749 | 17880 | 23090 | 6025 | 6016 |
| 5210 | 6053 | 6020 | 5214 | 6054 | 6022 | 5206 | 6028 | 6058 | 5617 | 29876 | 1946 |
| 16727 | 20454 | 27211 | 25574 | 6399 | 17585 | 17575 | 27374 | 17581 | 17294 | 11700 | 5755 |
| 5757 | 892 | 1134 | 1802 | 16711 | 6274 | 22834 | 24157 | 17687 | 6139 | 25434 | 23916 |

EP 2 540 831 A2

181

SEQ ID NO: homolog SEQ ID NOs

| | | | | | | | | | | | |
|---|---|---|---|---|---|---|---|---|---|---|---|
| 11274 | 14215 | 26174 | 27020 | 26170 | 1183 | 1186 | 20376 | 14402 | 12322 | 26553 | 2693 |
| 15458 | 14128 | 7454 | 13222 | 17295 | 14309 | 25581 | 25193 | 5159 | 12455 | 25787 | 25557 |
| 25556 | 28300 | 17482 | 7943 | 25532 | 7941 | 19189 | 24231 | 23428 | 23426 | 23430 | 6278 |
| 6279 | 12085 | 25217 | 25291 | 25190 | 30068 | 984 | 14786 | 25223 | 25542 | 30084 | 21930 |
| 29372 | 17375 | 25753 | 22492 | 19973 | 6373 | 1736 | 11091 | 16633 | 27061 | 14669 | 1441 |
| 19306 | 16063 | 11281 | 10282 | 28659 | 27482 | 17371 | 9333 | 11513 | 8630 | 4675 | 23010 |
| 3047 | 10857 | 15387 | 24796 | 17681 | 17677 | 14661 | 17473 | 8463 | 16958 | 13015 | |

605:

| | | | | | | | | | | | |
|---|---|---|---|---|---|---|---|---|---|---|---|
| 25845 | 28085 | 360 | 25766 | 26147 | 2800 | 23493 | 24582 | 10313 | 18751 | 28146 | 16590 |
| 16554 | 16550 | 16586 | 16588 | 16617 | 16544 | 16655 | 18934 | 18896 | 18938 | 18890 | 17686 |
| 17724 | 16652 | 16619 | 16654 | 4952 | 14984 | 30014 | 13318 | 17426 | 17457 | 17456 | 17419 |
| 17421 | 22044 | 23300 | 15543 | 17027 | 25664 | 25658 | 25656 | 25652 | 17004 | 4753 | 18859 |
| 4708 | 17388 | 17386 | 17382 | 17393 | 15515 | 15501 | 15547 | 25978 | 14019 | 13135 | 24545 |
| 17274 | 17251 | 17271 | 19323 | 19250 | 19252 | 19329 | 19345 | 28228 | 28224 | 28226 | 14026 |
| 15191 | 15219 | 15221 | 15222 | 15248 | 2758 | 2754 | 2782 | 28149 | 8932 | 19771 | 28964 |
| 5455 | 6327 | 6324 | 6364 | 28143 | 28144 | 14867 | 2787 | 2784 | 2790 | 2690 | 2752 |
| 2692 | 2696 | 2749 | 2718 | 2727 | 2729 | 10226 | 13396 | 7585 | 14270 | 13223 | 8234 |
| 8230 | 13227 | 13249 | 13430 | 13256 | 13451 | 7521 | 13456 | 13260 | 13296 | 7553 | 13482 |
| 7550 | 13487 | 13321 | 8254 | 7551 | 13489 | 7434 | 13327 | 13511 | 14234 | 14237 | 13360 |
| 13365 | 13367 | 13389 | 26133 | 19366 | 25253 | 25233 | 21308 | 7319 | 6333 | 14136 | 3918 |
| 14508 | 11299 | 14030 | 3648 | 19321 | 19257 | 18495 | 18501 | 18498 | 19302 | 19325 | 19288 |
| 23311 | 22025 | 3166 | 7930 | 28962 | 10793 | 24894 | 26571 | 17464 | 26640 | 20552 | 14913 |
| 20330 | 23678 | 8298 | 28408 | 9828 | 29747 | 18344 | 2419 | 20173 | 11997 | 11910 | 17617 |
| 24126 | 27287 | 1603 | 23932 | 24435 | 16318 | 28868 | 23922 | 10924 | 16702 | 19294 | 19296 |
| 19290 | 1752 | 14094 | 9661 | 25159 | 25157 | 25163 | 25186 | 25183 | 6148 | 22479 | 3169 |
| 7572 | 28344 | 18105 | 21423 | 16207 | 19960 | 2681 | 11397 | 3515 | 9623 | 17243 | 17217 |
| 17245 | 7269 | 7547 | 21722 | 10697 | 28531 | 11816 | 15850 | 8768 | 2581 | 21921 | 1305 |
| 21664 | 12381 | 23471 | 20578 | 7628 | 1431 | 1429 | 16121 | 25594 | 22437 | 29881 | 5140 |
| 15518 | 5360 | 26035 | 23579 | 12722 | 21986 | 16174 | 5695 | 29899 | 15753 | 12360 | 30211 |
| 22524 | 3862 | 10915 | 23443 | 15638 | 21351 | 21247 | 11618 | 27531 | 15180 | 15182 | 19370 |
| 10749 | 27036 | 12999 | 17731 | 17735 | 22652 | 11360 | 25602 | 23072 | 4357 | 5364 | 28567 |
| 18382 | 3005 | 11822 | 11821 | 26402 | 6381 | 17814 | 16656 | 14185 | 2792 | 15482 | 15479 |
| 5183 | 3197 | 25540 | 15282 | 1902 | 15217 | 8156 | 22571 | 3793 | 5756 | 28554 | 1101 |
| 24458 | 17819 | 7455 | 6482 | 30314 | 13505 | 15795 | 22096 | 18423 | 18644 | 16615 | 15719 |

(continued)

SEQ ID NO: homolog SEQ ID NOs

| | | | | | | | | | | | | |
|---|---|---|---|---|---|---|---|---|---|---|---|---|
| | 6479 | 4728 | 4727 | 17208 | 14596 | 25649 | 26006 | 6525 | 17211 | 17212 | 9192 | 9190 |
| | 18732 | 18731 | 13698 | 30164 | 17311 | 17278 | 14598 | 4735 | 24089 | 24094 | 25121 | 10552 |
| | 11549 | 10918 | 9944 | 30234 | 28515 | 30171 | 16662 | 12052 | 6555 | 24431 | 5515 | 5488 |
| | 3473 | 24183 | 10699 | 26973 | 15104 | 5498 | 16817 | 16159 | 15601 | 15639 | 19765 | 2697 |
| | 21402 | 29875 | 24717 | 29177 | 14580 | 12908 | 29253 | 29256 | 9301 | 29277 | 18915 | 24421 |
| | 16451 | 8788 | 12525 | 3183 | 6885 | 13623 | 20605 | 23156 | 4686 | 4688 | 17535 | 9200 |
| | 15474 | 5646 | 18266 | 2994 | 2961 | 1756 | 30252 | 14994 | 4509 | 28722 | 11699 | 29547 |
| | 2614 | 20734 | 17801 | 18649 | 4699 | 18854 | 17765 | 26214 | 13009 | 7725 | 7722 | 23416 |
| | 3100 | 13043 | 6414 | 6421 | 14269 | 13391 | 11286 | 11287 | 11285 | 11255 | 15444 | 2723 |
| | 18399 | 15576 | 15572 | 26306 | 26315 | 26314 | 26309 | 19541 | 5061 | 14942 | 2703 | 21086 |
| | 15643 | 15446 | 15602 | 9159 | 9163 | 9164 | 9161 | 8403 | 6501 | 28198 | 28175 | 28203 |
| | 9156 | 9125 | 9131 | 9128 | 4703 | 17360 | 2710 | 16546 | 23780 | 23806 | 28173 | 28171 |
| | 28168 | 5579 | 9575 | 14540 | 15750 | 15766 | 18655 | 16659 | 15755 | 18652 | 18678 | 17396 |
| | 17415 | 17417 | 6461 | 28204 | 6497 | 28219 | 28205 | 28221 | 6396 | 5355 | 5352 | 18886 |
| | 22866 | 6504 | 4697 | 4707 | 30090 | 4724 | 17620 | 4733 | 2760 | 2268 | 5114 | 12139 |
| | 13513 | 13330 | 7472 | 14272 | 25979 | 14825 | 24909 | 21178 | 21821 | 2233 | 18262 | 17463 |
| | 18259 | 17468 | 18256 | 17459 | 14629 | 5307 | 26127 | 6413 | 14751 | 27963 | 15186 | 28552 |
| | 20689 | 8090 | 1540 | 28523 | 20132 | 6510 | 25352 | 25975 | 2716 | 23307 | 23327 | 23345 |
| | 23248 | 16539 | 15758 | 17763 | 18881 | 17767 | 17580 | 17648 | 17643 | 17624 | 17721 | 20183 |
| | 20153 | 20176 | 20148 | 20147 | 17696 | 17692 | 20151 | 17688 | 20144 | 17690 | 17717 | 17642 |
| | 17715 | 17650 | 17652 | 12121 | 16613 | 18849 | 25274 | 16819 | 19570 | 15541 | 26711 | 5974 |
| | 15506 | 2694 | 21453 | 5793 | 11039 | 23795 | 15574 | 10254 | 26019 | | | |
| 606: | 17578 | 28296 | 2698 | 12421 | 27951 | 2336 | 30028 | 7204 | 1776 | 5453 | 16175 | 8115 |
| | 23688 | 15431 | 1511 | 1569 | 1238 | 1952 | 9517 | 9483 | 17742 | 22038 | 22869 | 13147 |
| | 27080 | 6172 | 7760 | 24149 | 4338 | 5279 | 16406 | 18875 | 13151 | 844 | 20727 | 20551 |
| | 13410 | 16562 | 1800 | 825 | 2045 | 3203 | 5089 | 27947 | 7388 | 23567 | 18356 | 4140 |
| | 3000 | 10665 | 29686 | 29810 | 11547 | 11671 | 1996 | 4564 | 18123 | 20072 | 12864 | 27240 |
| | 28302 | 857 | 12136 | 19282 | 18905 | 26012 | 20870 | 22878 | 16959 | 21306 | 7491 | 29268 |
| | 29534 | 9701 | 10239 | 14414 | 12089 | 3526 | 12135 | 11632 | 8073 | 21666 | 23310 | 26628 |
| | 23972 | 8093 | 15105 | 21565 | 13160 | 27030 | 5815 | 5244 | 9850 | 18082 | 24851 | 17748 |
| | 19176 | 13637 | 12702 | 14111 | 8961 | 3728 | 7564 | 14610 | 2524 | 29301 | 18773 | 14899 |
| | 9693 | 10870 | 13429 | 22595 | 8941 | 8956 | 8375 | 2202 | 12599 | 23672 | 24659 | 9547 |
| | 13495 | 5648 | 28877 | 26904 | 18858 | 17937 | 2739 | 15624 | 29164 | 9504 | 16016 | 17672 |

(continued)

SEQ ID NO: homolog SEQ ID NOs

| SEQ ID NO: | homolog SEQ ID NOs | | | | | | | | | | |
|---|---|---|---|---|---|---|---|---|---|---|---|
| 10707 | 21724 | 25763 | 10773 | 25592 | 4415 | 1174 | 29151 | 19419 | 25057 | 19101 | 15943 |
| 5057 | 15655 | 2200 | 21208 | 12192 | 24991 | 9505 | 15519 | 24997 | 23657 | 24977 | 23612 |
| 23686 | 9704 | 28090 | 17981 | 17978 | 11114 | 7242 | 11097 | 11205 | 23897 | 25084 | 1354 |
| 22555 | 10356 | 14163 | 19904 | 21467 | 14483 | 10293 | 13057 | 10115 | 22159 | 21256 | 22190 |
| 15798 | 15801 | 4032 | 25905 | 12176 | 7171 | 7615 | 6677 | 8507 | 3708 | 4810 | 27962 |
| 16231 | 17678 | 15699 | 24218 | 4644 | 3852 | 11206 | 9375 | 5841 | 19709 | 29153 | 20442 |
| 9475 | 16413 | 24732 | 14071 | 8133 | 27473 | 25443 | 7277 | 7333 | 29906 | 29015 | 8798 |
| 7212 | 6837 | 6771 | 28876 | 379 | 15813 | 17503 | 25460 | 10859 | 26936 | 15399 | 26471 |
| 11806 | 8742 | 24631 | 9555 | 20793 | 29061 | 8611 | 6152 | 20070 | 25884 | 24484 | 19645 |
| 17930 | 14710 | 10116 | 27844 | 22939 | 8800 | 26052 | 8846 | 29567 | 26872 | 10467 | 10375 |
| 9456 | 26454 | 29926 | 7603 | 20203 | 13615 | 21429 | 17441 | 9083 | 13448 | 28316 | 5457 |
| 15001 | 10760 | 28591 | 22887 | 22225 | 18530 | 15693 | 15828 | 19050 | 29258 | 26410 | 7192 |
| 7193 | 18606 | 920 | 14659 | 2241 | 19539 | 11345 | 30202 | 12416 | 5937 | 6313 | 28287 |
| 14790 | 7891 | 11385 | 3986 | 13382 | 27959 | 5735 | 21010 | 17030 | 9933 | 23526 | 23529 |
| 15475 | 17422 | 3663 | 20808 | 16101 | 15207 | 20508 | 2456 | 6137 | 24054 | 13314 | 1456 |
| 8667 | 8695 | 4447 | 13521 | 8877 | 24592 | 21534 | 5672 | 22060 | 23982 | 2366 | 18009 |
| 18074 | 14518 | 12837 | 12086 | 15837 | 28725 | 11755 | 27502 | 19531 | 14879 | 28307 | 23951 |
| 21560 | 23904 | 4463 | 17352 | 17284 | 13345 | 13343 | 13100 | 9341 | 6646 | 28894 | 13431 |
| 6611 | 13425 | 6607 | 23776 | 2460 | 8819 | 30168 | 6195 | 22940 | 978 | 23832 | 8596 |
| 11448 | 8168 | 10194 | 10789 | | | | | | | | |
| 607: 17578 | 28296 | 2698 | 12421 | 27951 | 2336 | 30028 | 7204 | 1776 | 5453 | 16175 | 8115 |
| 23688 | 15431 | 1511 | 1569 | 1238 | 1952 | 9517 | 9483 | 17742 | 22038 | 22869 | 13147 |
| 27080 | 6172 | 7760 | 24149 | 4338 | 5279 | 16406 | 18875 | 13151 | 844 | 20727 | 20551 |
| 13410 | 16562 | 1800 | 825 | 2045 | 3203 | 5089 | 27947 | 7388 | 23567 | 18356 | 4140 |
| 3000 | 10665 | 29686 | 29810 | 11547 | 11671 | 1996 | 4564 | 18123 | 20072 | 12864 | 27240 |
| 28302 | 857 | 12136 | 19282 | 18905 | 26012 | 20870 | 22878 | 16959 | 21306 | 7491 | 29268 |
| 29534 | 9701 | 10239 | 14414 | 12089 | 3526 | 12135 | 11632 | 8073 | 21666 | 23310 | 26628 |
| 23972 | 8093 | 15105 | 21565 | 13160 | 27030 | 5815 | 9850 | 5244 | 18082 | 24851 | 17748 |
| 19176 | 13637 | 12702 | 14111 | 3728 | 8961 | 7564 | 14610 | 2524 | 29301 | 18773 | 14899 |
| 9693 | 10870 | 13429 | 22595 | 8941 | 8375 | 2202 | 12599 | 23672 | 13495 | 9547 | 24659 |
| 5648 | 28877 | 26904 | 18858 | 17937 | 2739 | 15624 | 29164 | 9504 | 16016 | 17672 | 10707 |
| 21724 | 25763 | 10773 | 25592 | 4415 | 1174 | 29151 | 19419 | 25057 | 19101 | 15943 | 15655 |
| 5057 | 2200 | 21208 | 12192 | 24991 | 9505 | 24997 | 23657 | 24977 | 23612 | 23686 | 9704 |

SEQ ID NO: homolog SEQ ID NOs

| | | | | | | | | | | | | |
|---|---|---|---|---|---|---|---|---|---|---|---|---|
| | 28090 | 17981 | 17978 | 11114 | 7242 | 11097 | 11205 | 23897 | 25084 | 1354 | 22555 | 10356 |
| | 14163 | 19904 | 21467 | 14483 | 10293 | 13057 | 10115 | 22159 | 21256 | 22190 | 15798 | 15801 |
| | 4032 | 25905 | 12176 | 7171 | 7615 | 8507 | 6677 | 3708 | 27962 | 4810 | 16231 | 17678 |
| | 15699 | 4644 | 24218 | 3852 | 11206 | 9375 | 5841 | 19709 | 29153 | 20442 | 28320 | 9475 |
| | 16413 | 24732 | 14071 | 8798 | 25443 | 8133 | 7277 | 29906 | 27473 | 7333 | 29015 | 7212 |
| | 6837 | 6771 | 28876 | 379 | 15813 | 25460 | 17503 | 10859 | 26936 | 15399 | 26471 | 11806 |
| | 8742 | 24631 | 9555 | 20793 | 29061 | 8611 | 6152 | 20070 | 25884 | 24484 | 19645 | 17930 |
| | 14710 | 10116 | 27844 | 22939 | 8800 | 26052 | 8846 | 29567 | 26872 | 10467 | 10375 | 9456 |
| | 26454 | 29926 | 7603 | 20203 | 13615 | 21429 | 17441 | 9083 | 13448 | 28316 | 5457 | 15001 |
| | 10760 | 28591 | 22887 | 22225 | 18530 | 15693 | 15828 | 19050 | 29258 | 26410 | 7192 | 7193 |
| | 18606 | 920 | 14659 | 2241 | 19539 | 11345 | 30202 | 12416 | 5937 | 6313 | 7891 | 28287 |
| | 14790 | 3986 | 11385 | 13382 | 27959 | 5735 | 21010 | 17030 | 9933 | 23529 | 23526 | 15475 |
| | 17422 | 3663 | 20808 | 16101 | 15207 | 20508 | 2456 | 12600 | 22375 | 24054 | 13314 | 1456 |
| | 8667 | 8695 | 4447 | 13521 | 8877 | 24592 | 21534 | 5672 | 22060 | 23982 | 2366 | 18009 |
| | 18074 | 14518 | 12837 | 12086 | 15837 | 28725 | 11755 | 27502 | 19531 | 14879 | 19646 | 28307 |
| | 23951 | 23904 | 21560 | 4463 | 17352 | 17284 | 13345 | 13343 | 13100 | 14062 | 9341 | 6646 |
| | 28894 | 6611 | 13425 | 13431 | 6607 | 23776 | 2460 | 8819 | 30168 | 6195 | 22940 | 978 |
| | 23832 | 8596 | 11448 | 8168 | 10194 | 10789 | | | | | | |
| 608: | 4066 | 4067 | 23179 | 21127 | 23470 | 2893 | 26177 | 26178 | 4242 | 22312 | 4095 | 28346 |
| | 28343 | 18729 | 21244 | 2457 | 4937 | 10591 | 19566 | 6032 | 23254 | 22213 | 23405 | 5323 |
| | 14031 | 23183 | 29766 | 10564 | 28372 | 23437 | 10315 | 2724 | 11057 | 13543 | 13465 | 23962 |
| | 4284 | 23700 | 18966 | 10585 | 25368 | 5148 | 24840 | 13372 | 13374 | 5510 | 15296 | 15471 |
| | 18485 | 17783 | 25722 | 13207 | 6467 | 17495 | 7978 | 9536 | 21743 | 7431 | 4580 | 21048 |
| | 1979 | 18807 | 363 | 16670 | 362 | 361 | 12494 | 18833 | 29231 | 21291 | 14003 | 2037 |
| | 8313 | 13047 | 8865 | 25494 | 10440 | 18621 | 14846 | | | | | |
| 609: | 12222 | 1580 | 23967 | 16149 | 1246 | 3215 | 21371 | 26818 | 19987 | 9208 | 19129 | 17458 |
| | 18964 | 3139 | 14576 | 30001 | 25644 | 29116 | 26138 | 7437 | 11198 | 3538 | 3562 | 5851 |
| | 1259 | 27021 | 17663 | 22738 | 4228 | 14059 | 20781 | 10067 | 25520 | 29564 | 26165 | 21278 |
| | 4499 | 7899 | 28549 | 19866 | 4068 | 6961 | 25421 | 9067 | 27898 | 1703 | 27756 | 21927 |
| | 8939 | 19279 | 22477 | 22736 | 23743 | 18227 | 13929 | 14924 | 2860 | 25002 | 1785 | 21878 |
| | 14811 | 21896 | 14997 | 25407 | 10274 | 5634 | 13710 | 4622 | 13422 | 4574 | 6882 | 9640 |
| | 7915 | 971 | 25053 | 10547 | 13621 | 9931 | 13831 | 27217 | 29905 | 16007 | 4870 | 25009 |
| | 22197 | 24394 | 989 | 1128 | 17149 | 26539 | 21449 | 19413 | 6340 | 9186 | 21547 | 14366 |

(continued)

SEQ ID NO: homolog SEQ ID NOs

|  | | | | | | | | | | | |
|---|---|---|---|---|---|---|---|---|---|---|---|
| | 21293 | 28820 | 13924 | 30177 | 18683 | 15958 | 2626 | 30233 | 26218 | 30310 | 8538 | 24752 |
| | 29147 | 24325 | 20953 | 9489 | 25919 | 6781 | 26925 | 11701 | 22819 | 27729 | 3275 | 29746 |
| | 23587 | 8911 | 9632 | 1676 | 15853 | 13071 | 2751 | 7094 | 19510 | 26364 | 7691 | 20427 |
| | 13152 | 17658 | 11873 | 5478 | 18419 | 21819 | 25106 | 29367 | 10759 | 24551 | 800 | 26955 |
| | 22362 | 17110 | 26705 | 22163 | 18292 | 20890 | 26512 | 22231 | 7011 | 6570 | 3523 | 24661 |
| | 14161 | 21181 | 24695 | 24265 | 9068 | 29362 | 3632 | 13173 | 1718 | 14823 | 14953 | 12984 |
| | 18583 | 8842 | 16799 | 14898 | 26070 | 27781 | 805 | 30245 | 28249 | 3842 | 10872 | 7121 |
| | 26407 | 20149 | 6810 | 14467 | 3496 | 3493 | 9826 | 13154 | 2392 | 3469 | 8516 | 29982 |
| | 747 | 26030 | 17323 | 18696 | 23081 | 24267 | 6404 | 25503 | 2529 | 30290 | 3376 | 8013 |
| | 3963 | 4227 | 1768 | 7101 | 9240 | 26250 | 4113 | 29125 | 20252 | 24958 | 23365 | 19795 |
| | 27507 | 22623 | 20250 | 9466 | 25451 | 19716 | 27005 | 25653 | 23002 | 27210 | 3863 | 6753 |
| | 18297 | 17695 | 16909 | 3770 | 15236 | 24939 | 27778 | 13486 | 26341 | 5896 | 19086 | 12996 |
| | 26279 | 19179 | 24544 | 10332 | 23744 | 10911 | 3430 | 27659 | 9412 | 551 | 550 | 549 |
| | 22635 | 21496 | 27943 | 19206 | 21274 | 23058 | 6904 | 14014 | 6822 | 21830 | 13889 | 4489 |
| | 16525 | 27894 | 14216 | 29023 | 16082 | 22698 | 7566 | 5965 | 13236 | 20860 | 18218 | |
| 610: | 28845 | 20068 | 11102 | 24716 | 15862 | 7136 | 4600 | 30201 | 6030 | 27441 | 20139 | 26259 |
| | 1526 | 15648 | 21456 | 14572 | 27700 | 12131 | 13434 | 13082 | 24601 | 4869 | 1401 | |
| 611: | 17550 | 11471 | 24626 | 6115 | 22678 | 23194 | 2063 | 26248 | 11417 | 11418 | 15435 | 1194 |
| | 15637 | 29004 | 10616 | 19812 | 796 | 15410 | 24800 | 7904 | 24554 | 12184 | 26430 | 15445 |
| | 13805 | 1450 | 26190 | 12549 | 9096 | 15430 | 16807 | 3835 | 12634 | 11225 | 20602 | 29737 |
| | 13036 | 3300 | 3159 | 7167 | 4121 | | | | | | | |
| 612: | 8292 | 7852 | 20628 | 5910 | 5830 | 16791 | 25554 | 2989 | 28564 | 16142 | 16815 | 2660 |
| | 23667 | 29176 | 15740 | 2144 | 12380 | 13627 | 21539 | 26206 | 952 | 26658 | 1981 | 4774 |
| | 29162 | 18794 | 4742 | 1638 | 8387 | 24954 | 13709 | 26286 | 29476 | 12986 | 13421 | 23997 |
| | 14209 | 16042 | 18390 | 21945 | 11190 | 26557 | 25216 | 28164 | 1948 | 25102 | 15729 | 15118 |
| | 7161 | 19169 | 8130 | 15689 | 7082 | 4706 | 28360 | 9440 | 27221 | 18825 | 28860 | 20206 |
| | 10969 | 16716 | 24873 | 11578 | 25528 | 14018 | 10849 | 4168 | 28283 | 18080 | 6427 | 2327 |
| | 2837 | 13570 | 5566 | 21452 | 4958 | 4005 | 25191 | 28654 | 8128 | 8997 | 5227 | 12465 |
| | 27634 | 6975 | 19876 | 13415 | 9528 | 30256 | 29685 | 15231 | 30160 | 23825 | 21544 | 23599 |
| | 5746 | 10922 | 6554 | 8108 | 2756 | 22585 | 15982 | 3872 | 15111 | 18622 | 25726 | 22354 |
| | 29240 | 7799 | 14035 | 17039 | 2066 | 2286 | 27475 | 14155 | 14156 | 14192 | 12982 | 12732 |
| | 28385 | 13551 | 1483 | 24242 | 16973 | 12701 | 27381 | 8020 | 15493 | 8814 | 22599 | 22234 |
| | 28878 | 22066 | 2898 | 2863 | 22770 | 16084 | 12109 | 12245 | 3804 | 9713 | 10501 | 9291 |

SEQ ID NO: homolog SEQ ID NOs

| | | | | | | | | | | | | |
|---|---|---|---|---|---|---|---|---|---|---|---|---|
| | 6181 | 26022 | 25228 | 4200 | 16610 | 11420 | 9903 | 4249 | 20120 | 2002 | 4656 | 839 |
| | 9699 | 17897 | 24176 | 1683 | 11564 | 19229 | 22065 | 4085 | 12614 | 11433 | 2964 | 17167 |
| | 22120 | 8423 | 23575 | 21798 | 15456 | 19957 | 14955 | 27218 | 17760 | 8030 | 17835 | 5440 |
| | 16355 | 1447 | 9824 | 27936 | 6727 | 7201 | 12532 | 15735 | 28503 | 10031 | 2236 | 5096 |
| | 26892 | 5741 | 4056 | 11943 | 8088 | 17774 | 11330 | 6893 | 17450 | 11130 | 12362 | 24508 |
| | 23291 | 28394 | 7812 | 18880 | 14976 | 24976 | 3950 | 15407 | 24941 | 16648 | 2797 | 16533 |
| | 3772 | 16935 | 22237 | 17549 | 2667 | 14306 | 14148 | 16258 | 17344 | 25182 | 12048 | 29727 |
| | 3509 | 24549 | 18397 | 20766 | 1237 | 19347 | 25380 | 24726 | 28905 | 17706 | 17886 | 3522 |
| | 12499 | 23955 | 21906 | 24170 | 19029 | 1535 | 27501 | 24405 | 14957 | 23935 | 28893 | 19974 |
| | 23664 | 18605 | 15253 | 14575 | 20429 | 15085 | 20180 | 21031 | 24038 | 8430 | 14615 | 28238 |
| | 8071 | 27530 | 29246 | 4565 | 11631 | 16419 | 21883 | 16289 | 23159 | 26636 | 27523 | 5638 |
| | 15749 | 12574 | 20524 | 15359 | 23696 | 21407 | 23380 | 18667 | 26240 | 1941 | 19882 | 5565 |
| | 24807 | 28544 | 2396 | 10241 | 5216 | 9511 | 18992 | 18824 | 19543 | 2640 | 10304 | 5997 |
| | 7653 | 10285 | 27955 | 12446 | 4358 | 11533 | 15592 | 12878 | 29484 | 8233 | 23356 | 29392 |
| | 29904 | 26819 | 25260 | 18506 | 6488 | 17770 | 14016 | 26929 | 14493 | 27555 | 20686 | 16789 |
| | 790 | 9290 | 19538 | 21650 | 17596 | 28792 | 22765 | 2043 | 3584 | 26811 | 1962 | 20420 |
| | 10436 | 26054 | 28945 | 17018 | 21061 | 6481 | 17764 | 14643 | 22750 | 22618 | 11550 | 7641 |
| | 20986 | 30214 | 25846 | 27201 | 21305 | 21304 | 29984 | 10959 | 13595 | 20146 | 21870 | 25870 |
| | 23761 | 26740 | 29309 | 2278 | 811 | 6005 | 4206 | 15786 | 11300 | 29635 | 2927 | 30156 |
| | 14819 | 20795 | 16214 | 28681 | 24233 | 24143 | 20297 | 20417 | 20378 | 23054 | 20318 | 10025 |
| | 24424 | 24402 | 21601 | 2629 | 28254 | 19897 | 12049 | 8598 | 4296 | 12438 | 9605 | 28333 |
| | 2075 | 7678 | 17276 | 24391 | 18800 | 23287 | 24921 | 18835 | 28370 | 9581 | 29200 | 21107 |
| | 8487 | 15144 | 12910 | 29222 | 1360 | 6973 | 28318 | 11062 | 19355 | 6669 | 8429 | 11947 |
| | 30105 | 29960 | 17194 | 9060 | 27972 | 28636 | 15748 | 13775 | 24931 | 17771 | 6060 | 28920 |
| | 6724 | 20908 | 1425 | 9991 | 25500 | 25112 | 26744 | 25834 | 17001 | 27087 | 11637 | 9696 |
| | 12312 | 21910 | 10336 | 29565 | 29715 | 4317 | 6133 | 29500 | 10291 | 22978 | 11334 | 13842 |
| | 24282 | 2127 | | | | | | | | | | |
| 613: | 7852 | 20628 | 8292 | 5910 | 5830 | 16791 | 25554 | 2989 | 30018 | 28564 | 16142 | 16815 |
| | 2660 | 23667 | 29176 | 2144 | 12380 | 13627 | 21539 | 26206 | 952 | 26658 | 1981 | 4774 |
| | 29162 | 18794 | 4742 | 1638 | 8387 | 24954 | 26286 | 12986 | 13709 | 29476 | 13421 | 23997 |
| | 14209 | 16042 | 18390 | 21945 | 11190 | 26557 | 25216 | 28164 | 1948 | 25102 | 15729 | 15118 |
| | 7161 | 19169 | 8130 | 15689 | 7082 | 4706 | 28360 | 9440 | 27221 | 18825 | 28860 | 20206 |
| | 10969 | 4436 | 20365 | 22116 | 16716 | 24873 | 11578 | 25528 | 14018 | 10849 | 4168 | 28283 |

(continued)

| SEQ ID NO: | homolog SEQ ID NOs | | | | | | | | | |
|---|---|---|---|---|---|---|---|---|---|---|
| 18080 | 6427 | 2327 | 13570 | 5566 | 21452 | 4958 | 4005 | 25191 | 29976 | 28654 |
| 8128 | 8997 | 5227 | 6975 | 27634 | 19876 | 13415 | 9528 | 30256 | 29685 | 15231 |
| 30160 | 23825 | 21544 | 5746 | 10922 | 6554 | 8108 | 2756 | 22585 | 15982 | 3872 |
| 15111 | 18622 | 25726 | 29240 | 7799 | 14035 | 17039 | 2066 | 2286 | 27475 | 14155 |
| 14156 | 14192 | 12982 | 28385 | 13551 | 1483 | 24242 | 16973 | 12701 | 8020 | 15493 |
| 8814 | 22599 | 22234 | 22066 | 16084 | 12109 | 12245 | 3804 | 9713 | 10501 | 9291 |
| 6181 | 26022 | 25228 | 11420 | 16610 | 4249 | 9903 | 20120 | 2002 | 4656 | 839 |
| 9699 | 17897 | 24176 | 11564 | 19229 | 22065 | 4085 | 12614 | 11433 | 2964 | 17167 |
| 22120 | 8423 | 23575 | 15456 | 19957 | 14955 | 27218 | 17760 | 8030 | 17835 | 5440 |
| 16355 | 1447 | 9824 | 7201 | 12532 | 15735 | 28503 | 10031 | 2236 | 5096 | 26892 |
| 5741 | 4056 | 11943 | 17774 | 11330 | 6893 | 11130 | 17450 | 12362 | 24508 | 23291 |
| 28394 | 7812 | 18880 | 24976 | 3950 | 15407 | 24941 | 16648 | 2797 | 3772 | 16533 |
| 16935 | 22237 | 17549 | 30319 | 14584 | 14306 | 14148 | 16258 | 17344 | 25182 | 12048 |
| 29727 | 3509 | 24549 | 18397 | 1237 | 19347 | 25380 | 24726 | 28905 | 17706 | 17886 |
| 3522 | 12499 | 23955 | 24170 | 19029 | 27501 | 1535 | 24405 | 14957 | 23935 | 28893 |
| 19974 | 23664 | 18605 | 14575 | 20429 | 20180 | 15085 | 21031 | 24038 | 8430 | 14615 |
| 28238 | 8071 | 27530 | 4565 | 11631 | 16419 | 21883 | 16289 | 16293 | 23159 | 26636 |
| 27523 | 5638 | 15749 | 20524 | 15359 | 23696 | 21407 | 23380 | 18667 | 26240 | 1941 |
| 19882 | 5565 | 24807 | 2396 | 10241 | 5216 | 9511 | 18992 | 18824 | 19543 | 2640 |
| 10304 | 5997 | 7653 | 12446 | 4358 | 11533 | 15592 | 12878 | 29484 | 8233 | 23356 |
| 29392 | 29904 | 25173 | 25260 | 18506 | 6488 | 17770 | 14016 | 26929 | 27555 | 20686 |
| 16789 | 790 | 9290 | 21650 | 17596 | 28792 | 22765 | 2043 | 3584 | 26811 | 1962 |
| 5939 | 20420 | 10436 | 26054 | 28945 | 17018 | 21061 | 6481 | 17764 | 14643 | 22750 |
| 22618 | 11550 | 7641 | 30214 | 25846 | 27201 | 21305 | 21304 | 29984 | 10959 | 13595 |
| 20146 | 21870 | 25870 | 26740 | 29309 | 2278 | 811 | 6005 | 4206 | 15786 | 11300 |
| 29635 | 2927 | 30156 | 20795 | 16214 | 28681 | 24233 | 24143 | 20297 | 20417 | 20378 |
| 23054 | 20318 | 10025 | 24402 | 21601 | 2629 | 28254 | 19644 | 12420 | 12049 | 8598 |
| 4296 | 12438 | 9605 | 28004 | 27783 | 964 | 2075 | 17276 | 24391 | 18800 | 23287 |
| 21158 | 5701 | 18815 | 21323 | 11115 | 19768 | 26898 | 18263 | 23348 | 26506 | 2143 |
| 9617 | 27823 | 14286 | 19982 | 10296 | 24921 | 18835 | 28370 | 9581 | 29200 | 21107 |
| 8487 | 15144 | 12910 | 1360 | 6973 | 28318 | 11062 | 19355 | 6669 | 8429 | 11947 |
| 30105 | 29960 | 17194 | 27972 | 28636 | 15748 | 13775 | 24931 | 17771 | 6060 | 28920 |
| 6724 | 20908 | 1425 | 25500 | 25112 | 26744 | 25834 | 17001 | 27087 | 11637 | 9696 |

SEQ ID NO: homolog SEQ ID NOs

| | | | | | | | | | | | | |
|---|---|---|---|---|---|---|---|---|---|---|---|---|
| | 12312 | 21910 | 10336 | 29565 | 29715 | 4317 | 6133 | 29500 | 10291 | 22978 | 11334 | 13842 |
| | 24282 | 14701 | 27244 | | | | | | | | | |
| 614: | 7852 | 20628 | 8292 | 5910 | 5830 | 12354 | 16791 | 25554 | 2989 | 30018 | 28564 | 16142 |
| | 16815 | 2660 | 23667 | 29176 | 2144 | 12380 | 13627 | 21539 | 26206 | 952 | 26658 | 1981 |
| | 4774 | 29162 | 18794 | 4742 | 1638 | 8387 | 24954 | 13709 | 26286 | 29476 | 12986 | 13421 |
| | 23997 | 14209 | 16042 | 18390 | 11190 | 21945 | 26557 | 25216 | 28164 | 1948 | 15729 | 15118 |
| | 7161 | 19169 | 8130 | 15689 | 7082 | 4706 | 28360 | 9440 | 27221 | 18825 | 28860 | 20206 |
| | 10969 | 20365 | 22116 | 16716 | 24873 | 11578 | 25528 | 14018 | 10849 | 4168 | 28283 | 18080 |
| | 11399 | 6427 | 2327 | 2837 | 13570 | 5566 | 21452 | 4958 | 4005 | 25191 | 28654 | 8128 |
| | 8997 | 5227 | 12465 | 6975 | 27634 | 19876 | 13415 | 9528 | 30256 | 29685 | 15231 | 30160 |
| | 23825 | 21544 | 23599 | 5746 | 10922 | 6554 | 8108 | 2756 | 22585 | 15982 | 3872 | 15111 |
| | 18622 | 25726 | 22354 | 29240 | 7799 | 14035 | 17039 | 2066 | 2286 | 27475 | 14155 | 14156 |
| | 14192 | 12982 | 12732 | 28385 | 13551 | 1483 | 24242 | 16973 | 12701 | 8020 | 15493 | 8814 |
| | 22599 | 22234 | 28878 | 22066 | 2898 | 22770 | 2863 | 16084 | 12109 | 12245 | 3804 | 9713 |
| | 10501 | 9291 | 6181 | 26022 | 25228 | 14013 | 4200 | 11420 | 16610 | 9903 | 4249 | 20120 |
| | 2002 | 4656 | 6768 | 839 | 9699 | 17897 | 24176 | 1683 | 11564 | 19229 | 22065 | 4085 |
| | 12614 | 11433 | 2964 | 17167 | 22120 | 8423 | 23575 | 21798 | 15456 | 14955 | 27218 | 8030 |
| | 17760 | 17835 | 5440 | 16355 | 1447 | 9824 | 27936 | 6727 | 7201 | 12532 | 15735 | 28503 |
| | 10031 | 2236 | 5096 | 26892 | 5741 | 14497 | 4056 | 11943 | 8088 | 17774 | 11330 | 6893 |
| | 17450 | 11130 | 12362 | 24508 | 23291 | 28394 | 7812 | 18880 | 14976 | 24976 | 3950 | 15407 |
| | 24941 | 16648 | 2797 | 16533 | 3772 | 16935 | 22237 | 17549 | 2667 | 14306 | 14148 | 16258 |
| | 17344 | 25182 | 12048 | 3509 | 24549 | 18397 | 20766 | 1237 | 19347 | 25380 | 24726 | 28905 |
| | 17706 | 17886 | 3522 | 12499 | 21906 | 23955 | 24170 | 19029 | 1535 | 24405 | 27501 | 14957 |
| | 23935 | 28893 | 19974 | 23664 | 18605 | 15253 | 14575 | 20429 | 20180 | 15085 | 21031 | 24038 |
| | 8430 | 14615 | 28238 | 8071 | 27530 | 29246 | 4565 | 11631 | 16419 | 21883 | 16289 | 23159 |
| | 26636 | 27523 | 5638 | 15749 | 12574 | 20524 | 23696 | 15359 | 21407 | 23380 | 18667 | 26240 |
| | 19882 | 1941 | 5565 | 24807 | 28544 | 2396 | 10241 | 5216 | 9511 | 18992 | 18824 | 19543 |
| | 10304 | 2640 | 7653 | 5997 | 10285 | 27955 | 12446 | 4358 | 11533 | 15592 | 12878 | 29484 |
| | 8233 | 23356 | 29392 | 29904 | 25173 | 26819 | 25260 | 18506 | 6488 | 17770 | 14016 | 26929 |
| | 14493 | 10936 | 27555 | 20686 | 16789 | 790 | 9290 | 19538 | 21650 | 17596 | 28792 | 22765 |
| | 2043 | 3584 | 26811 | 1962 | 20420 | 10436 | 26054 | 28945 | 17018 | 21061 | 6481 | 17764 |
| | 14643 | 22750 | 22618 | 11550 | 7641 | 20986 | 30214 | 25846 | 27201 | 21305 | 29984 | 21304 |
| | 10959 | 13595 | 20146 | 21870 | 25870 | 23761 | 26740 | 29309 | 2278 | 811 | 1914 | 6005 |

SEQ ID NO: homolog SEQ ID NOs

| SEQ ID NO | | | | | | | | | | | | |
|---|---|---|---|---|---|---|---|---|---|---|---|---|
| | 4206 | 15786 | 11300 | 25838 | 29635 | 2927 | 30156 | 14819 | 20795 | 16214 | 28681 | 24143 |
| | 24233 | 20297 | 20417 | 20378 | 23054 | 20318 | 10025 | 24424 | 24402 | 21601 | 2629 | 28254 |
| | 15459 | 27765 | 12049 | 8598 | 4296 | 12438 | 9605 | 20588 | 25511 | 4752 | 13682 | 12535 |
| | 28004 | 2075 | 17276 | 24391 | 18800 | 5701 | 26506 | 18263 | 23348 | 27012 | 18815 | 9617 |
| | 24921 | 18835 | 28370 | 9581 | 29200 | 8487 | 15144 | 12910 | 21107 | 1360 | 29222 | 6973 |
| | 28318 | 11062 | 19355 | 6669 | 8429 | 11947 | 30105 | 29960 | 17194 | 27972 | 28636 | 15748 |
| | 13775 | 24931 | 17771 | 6060 | 28920 | 6724 | 20908 | 1425 | 9991 | 25500 | 25112 | 26744 |
| | 25834 | 17001 | 27087 | 11637 | 9696 | 12312 | 21910 | 10336 | 29565 | 29715 | 4317 | 6133 |
| | 29500 | 10291 | 22978 | 11334 | 13842 | 24282 | 14705 | | | | | |
| 615: | 26461 | 19885 | 29850 | 16306 | 11569 | 8420 | 21310 | 11355 | 24200 | 8731 | 21641 | 5833 |
| | 20370 | 13904 | 5529 | 14313 | 4897 | 5737 | 4764 | 6959 | 12825 | 27704 | 15173 | 29765 |
| | 26851 | 15925 | 8617 | 14828 | 26413 | 14655 | 22788 | 24304 | 29330 | 24017 | 6539 | 19177 |
| | 21623 | | | | | | | | | | | |
| 616: | 26461 | 19885 | 29850 | 16306 | 11569 | 8420 | 21310 | 11355 | 24200 | 8731 | 21641 | 5833 |
| | 20370 | 13904 | 5529 | 14313 | 4897 | 5737 | 4764 | 6959 | 12825 | 27704 | 15173 | 29765 |
| | 26851 | 15925 | 8617 | 14828 | 26413 | 14655 | 22788 | 24304 | 29330 | 24017 | 6539 | 19177 |
| | 21623 | | | | | | | | | | | |
| 617: | 16707 | 12477 | 1230 | 5933 | 13002 | 13400 | 15270 | 8986 | 26112 | 15449 | 14545 | |
| 618: | 16707 | 12477 | 1230 | 5933 | 13002 | 13400 | 15270 | 8986 | 26112 | 15449 | 14545 | |
| 619: | 8780 | 3256 | 10813 | 27950 | 28987 | 16505 | 15319 | 12200 | 27971 | 27965 | 20304 | 26877 |
| | 5365 | 19801 | 9049 | 837 | 15275 | 17336 | 16501 | 24276 | 9598 | 14477 | 19945 | 24825 |
| | 1524 | 27890 | 19838 | 28009 | 22402 | 11354 | 21465 | 27612 | 13614 | 27712 | 28559 | 9479 |
| | 19446 | 21902 | 7750 | 26830 | 26845 | 2478 | 22806 | 25670 | 8223 | 26724 | 8092 | 12958 |
| | 28411 | 24048 | 6317 | 26508 | 3740 | 9678 | 27970 | 27137 | 9802 | 12201 | 25484 | |
| 620: | 8780 | 3256 | 10813 | 27950 | 28987 | 16505 | 15319 | 12200 | 27971 | 27965 | 20304 | 26877 |
| | 5365 | 19801 | 9049 | 837 | 15275 | 17336 | 16501 | 24276 | 9598 | 14477 | 19945 | 24825 |
| | 1524 | 27890 | 19838 | 28009 | 22402 | 11354 | 21465 | 27612 | 13614 | 27712 | 28559 | 9479 |
| | 19446 | 21902 | 7750 | 26830 | 26845 | 2478 | 22806 | 25670 | 8223 | 26724 | 8092 | 12958 |
| | 28411 | 24048 | 6317 | 26508 | 3740 | 9678 | 27970 | 27137 | 9802 | 12201 | 25484 | |
| 621: | 6828 | 7030 | 14439 | 14516 | 5046 | 5403 | 27499 | 18641 | 4324 | 7356 | 23459 | 1052 |
| | 16399 | 16400 | 22401 | 23846 | 7650 | 1051 | 7445 | 23269 | 16105 | | | |
| 622: | 17281 | 25588 | 29228 | 13239 | 19881 | 8536 | 27442 | 1733 | 10514 | 12241 | 19242 | 10853 |
| | 17955 | 7314 | 20048 | 25204 | 10613 | 11126 | 16352 | 16850 | 23631 | 8394 | 28051 | 2838 |

EP 2 540 831 A2

(continued)

SEQ ID NO: homolog SEQ ID NOs

| | | | | | | | | | | | |
|---|---|---|---|---|---|---|---|---|---|---|---|
| 6010 | 17747 | 12889 | 29395 | 22288 | 28717 | 26232 | 27438 | 1973 | 24198 | | |
| **623:** 17281 | 25588 | 29228 | 13239 | 19881 | 8536 | 27442 | 1733 | 10514 | 12241 | 19242 | 10853 |
| 17955 | 7314 | 20048 | 25204 | 10613 | 11126 | 16352 | 16850 | 23631 | 8394 | 28051 | 2838 |
| 6010 | 17747 | 12889 | 29395 | 22288 | 28717 | 26232 | 27438 | 1973 | 24198 | | |
| **624:** 14042 | 15802 | 13369 | 17231 | 23610 | 18033 | 24263 | 1721 | 4139 | 19674 | 6903 | 4592 |
| 9626 | 11172 | 14753 | 15317 | 29154 | | | | | | | |
| **625:** 14042 | 15802 | 13369 | 17231 | 23610 | 18033 | 24263 | 1721 | 4139 | 19674 | 6903 | 4592 |
| 9626 | 11172 | 14753 | 15317 | 29154 | | | | | | | |
| **626:** 6795 | 10510 | 29861 | 1848 | 1739 | 22888 | 9326 | 15354 | 12551 | 9681 | 5871 | 8837 |
| 12639 | 25488 | 17703 | 25764 | 25701 | 25611 | 25760 | 25728 | 25640 | 25609 | 25692 | 25672 |
| 25699 | 25696 | 25703 | 25645 | 28689 | 780 | 6175 | 21056 | 22207 | 8569 | 20047 | 29818 |
| 6398 | 4997 | 4954 | 5093 | 13880 | 3597 | 13527 | 30286 | 23628 | 13019 | 7571 | 1019 |
| 26799 | 8289 | 1122 | 16827 | 6945 | 24930 | 3677 | 829 | 26729 | 8475 | 17174 | 1211 |
| 3145 | 3147 | 2228 | 1458 | 14154 | 17487 | 20230 | 23283 | 7290 | 26562 | 1618 | 17034 |
| 4664 | 1707 | 20618 | 16048 | 2180 | 27399 | 17381 | 1619 | 11834 | 12637 | 6784 | 7497 |
| 3127 | 6371 | 26318 | 5832 | 5177 | 11223 | 16508 | 1361 | 30007 | 24214 | 9601 | 24216 |
| 13413 | 19673 | 1356 | 8623 | 6815 | 21720 | 15396 | 25718 | 19480 | 17362 | 14964 | 21613 |
| 3603 | 9609 | 23483 | 13255 | 18016 | 20530 | 13288 | 19824 | 22966 | 15876 | 4065 | 29127 |
| 14868 | 16077 | 16900 | 17924 | 18085 | 28361 | 14959 | 9104 | 22592 | 24881 | 23338 | 17987 |
| 29848 | 13408 | 7127 | 17141 | 17124 | 17153 | 1479 | 29700 | 22644 | 23591 | 4366 | 19145 |
| 4529 | 6590 | 11592 | 7245 | 14651 | 21973 | 28694 | 19457 | 17222 | 28693 | 21892 | 29257 |
| 25486 | 10983 | 18831 | 26942 | 29304 | 812 | 15954 | 5022 | 16776 | 18826 | 22890 | 6000 |
| 11648 | 9059 | 8173 | 954 | 11830 | 26154 | 16090 | 4757 | 2606 | 20818 | 9400 | 10409 |
| 20676 | 17169 | 21284 | 19478 | 22516 | 22811 | 18313 | 6783 | 1439 | 30032 | 28135 | 25426 |
| 16722 | 18547 | 3750 | 7954 | 8494 | 13406 | 6808 | 5562 | 15266 | 17157 | 22436 | 16144 |
| 29604 | 19697 | 6706 | 1765 | 5761 | 28265 | 29504 | 11016 | 6387 | 4996 | 1621 | 15757 |
| 29328 | 27553 | 4689 | 9223 | 13909 | 11171 | 11196 | 12343 | 938 | 4796 | 9712 | 3165 |
| 29584 | 16245 | 2405 | 2383 | 13493 | 21935 | 11195 | 7970 | 14573 | 25472 | 30216 | 4177 |
| 1968 | 19936 | 9658 | 27808 | 19032 | 20950 | 15678 | 8256 | 7448 | 26623 | 5313 | 22818 |
| 11520 | 18812 | 958 | 2195 | 17111 | 18722 | 15400 | 9725 | 6523 | 26757 | 27601 | 11041 |
| 12514 | 16638 | 25515 | 16141 | 25467 | 11256 | 6251 | 4352 | 4365 | 826 | 29101 | 17272 |
| 15366 | 18564 | 6805 | 9941 | 21640 | 3741 | 20733 | 9320 | 27261 | 13395 | 13332 | 14381 |
| 11749 | 12159 | 3901 | 8462 | 22917 | 22795 | 17898 | 18951 | 28280 | 22412 | 29325 | 18549 |

SEQ ID NO: homolog SEQ ID NOs

| | | | | | | | | | | | |
|---|---|---|---|---|---|---|---|---|---|---|---|
| 27688 | 1974 | 11084 | 29860 | 9753 | 24039 | 10927 | 5399 | 14115 | 18024 | 25094 | 15023 |
| 29762 | 27571 | 6368 | 3925 | 23078 | 7939 | 6563 | 9115 | 30161 | 28872 | 28152 | 19764 |
| 14793 | 5577 | 1880 | 7222 | 8504 | 6439 | 7720 | 9912 | 26118 | 5873 | 5767 | 11589 |
| 11244 | 7213 | 14866 | 19458 | 28463 | 18796 | 21499 | 25222 | 19667 | 19856 | 26519 | 13078 |
| 18010 | 23807 | 29383 | 13673 | 8752 | 6070 | 4663 | 3032 | 4662 | 1050 | 23643 | 23117 |
| 5980 | 1976 | 20761 | 3074 | 26745 | 7146 | 16553 | 24953 | 18358 | 28854 | 19571 | 18265 |
| 8203 | 19598 | 14605 | 13474 | 13477 | 29725 | 24257 | 23334 | 23244 | 15048 | 17046 | 13969 |
| 1075 | 1704 | 14458 | 26365 | 28658 | 9300 | 25272 | 11879 | 21981 | 22167 | 28650 | 23896 |
| 885 | 22771 | 25759 | 23122 | 19794 | 4635 | 29098 | 4122 | 22943 | 3202 | 26732 | 7885 |
| 17963 | 16757 | 6453 | 26162 | 19011 | 7344 | 13364 | 21767 | 1125 | 24638 | 2135 | 27065 |
| 22578 | 4473 | 15909 | 9667 | 19474 | 26706 | 29978 | 2363 | 3819 | 8157 | 4588 | 24479 |
| 26360 | 14410 | 4566 | 13339 | 1474 | 20574 | 3225 | 27832 | 2432 | 18643 | 21857 | 1583 |
| 30327 | 9835 | 9957 | 27548 | 30249 | 9228 | 16354 | 25089 | 21977 | 28005 | 29433 | 4370 |
| 27752 | 14715 | 27793 | 8076 | 11747 | 8040 | 7070 | 16494 | 24564 | 29371 | 18002 | 24834 |
| 21192 | 13356 | 26233 | 26683 | 26982 | 10397 | 9360 | 3542 | 5772 | 24995 | 8951 | 13674 |
| 29071 | 3613 | 29442 | 28096 | 11252 | 17759 | 1693 | 30193 | 20296 | 23844 | 25523 | 3686 |
| 14454 | 12100 | 5887 | 7731 | 18578 | 17138 | 10876 | 22584 | 25564 | 27454 | 3552 | 11072 |
| 28410 | 6419 | 4237 | 4240 | 3968 | 3971 | 3966 | 10117 | 10114 | 5419 | 13510 | 18287 |
| 2356 | 25948 | 4548 | 17065 | 2325 | 4390 | 1475 | 7777 | 16887 | 16835 | 29144 | 28297 |
| 16299 | 918 | 941 | 1482 | 13942 | 12921 | 9231 | 9628 | 2035 | 1021 | 16903 | 18417 |
| 5056 | 23880 | 5459 | 23301 | 2462 | 14140 | 24227 | 23481 | 2978 | 12944 | 24889 | 17334 |
| 19661 | 14592 | 14742 | 25577 | 7531 | 6881 | 2001 | 7985 | 26385 | 5202 | 5115 | 24561 |
| 10893 | 5866 | 29326 | 13462 | 25037 | 29803 | 23441 | 20862 | 26340 | 8081 | 14787 | 25966 |
| 17301 | 1381 | 24085 | 28133 | 24968 | 24965 | 22662 | 4824 | 3744 | 24913 | 13809 | 27111 |
| 11276 | 26918 | 26657 | 24843 | 16472 | 28061 | 13401 | 25010 | 21037 | 19846 | 8643 | 26330 |
| 1531 | 3316 | 15442 | 14810 | 28131 | 6011 | 2424 | 12459 | 27321 | 3917 | 3936 | 9386 |
| 25618 | 4385 | 1481 | 13981 | 15148 | 19778 | 22100 | 5801 | 14428 | 9902 | 30276 | 13424 |
| 10597 | 13884 | 18521 | 24831 | 16320 | 3721 | 12949 | 17291 | 10425 | 17741 | 6272 | 10021 |
| 17872 | 12189 | 27893 | 2901 | 30111 | 26356 | 26412 | 26439 | 26416 | 26420 | 26417 | 26387 |
| 9401 | 3988 | 1857 | 16625 | 11595 | 13188 | 8332 | 25662 | 6121 | 9622 | 19438 | 19403 |
| 19516 | 19509 | 10794 | 19464 | 8438 | 19432 | 10233 | 27478 | 23261 | 778 | 19470 | 19391 |
| 19477 | 19398 | 28139 | 11532 | 18127 | 24220 | 10186 | 9332 | 28875 | 13837 | 11855 | |
| 627: 24647 | 13105 | 24787 | 12148 | 10406 | 7324 | 7987 | | | | | |

(continued)

SEQ ID NO: homolog SEQ ID NOs

| 628: | 2827 | 21648 | 22239 | 20396 | 5350 | 18594 | 13414 | 3105 | 3122 | 10761 | 21096 | 1570 |
|---|---|---|---|---|---|---|---|---|---|---|---|---|
| | 8290 | 24441 | 1819 | 7703 | 17446 | 15026 | 11413 | 16074 | 28826 | 8926 | 2985 | 23827 |
| | 18789 | 21796 | 1100 | 27681 | 12292 | 25281 | 6132 | 29085 | 1437 | 17574 | 20269 | 22049 |
| | 1861 | 15353 | 9151 | 16462 | 29950 | 30298 | 8628 | 4670 | 29916 | 22022 | 20351 | 6265 |
| | 26983 | 1260 | 29757 | 11581 | 1116 | 11626 | 25314 | 12647 | 21487 | 22732 | 8349 | 9194 |
| | 5078 | 28322 | 23446 | 29598 | 28404 | 29768 | 27807 | 1435 | 22280 | 24172 | 12973 | 28461 |
| | 21035 | 29323 | 8678 | 26140 | 3550 | 1842 | 27577 | 21267 | 29953 | 2722 | 12906 | 20886 |
| | 10787 | 9494 | 20065 | 17225 | 28472 | 14449 | 4088 | 13827 | 4769 | 20362 | 6736 | 4217 |
| | 13158 | 9914 | 22017 | 16750 | 14583 | 14800 | 13660 | 27988 | 6695 | 22432 | 28758 | 14595 |
| | 1389 | 22126 | 2553 | 7024 | 17822 | 2289 | 6487 | 6484 | 11479 | 6502 | 6229 | 14051 |
| | 16467 | 21170 | 8907 | 8887 | 28847 | 3948 | 11527 | 13632 | 5217 | 1071 | 12831 | 21444 |
| | 28123 | 5960 | 29252 | 10600 | 1560 | 15102 | 21836 | 21517 | 1767 | 16340 | 15215 | 19213 |
| | 13163 | 7408 | 5989 | 1250 | 14864 | 2145 | 20980 | 20786 | 25489 | 19890 | 10154 | 8406 |
| | 3192 | 29520 | 21938 | 25996 | 1922 | 19108 | 2311 | 25937 | 12706 | 23547 | 19501 | 19503 |
| | 19415 | 13681 | 21632 | 22326 | 21619 | 21916 | 6584 | 23359 | 28375 | 22553 | 23759 | 1138 |
| | 18646 | 12204 | 23294 | 1286 | 18001 | 9694 | 21865 | 3196 | 20557 | 27271 | 16962 | 2957 |
| | 2952 | 15708 | 3560 | 5363 | 5347 | 11222 | 30129 | 7897 | 26621 | 3361 | 22294 | 21939 |
| | 28384 | 17873 | 23061 | 9577 | 4111 | 22934 | 16339 | 12654 | 4257 | 6797 | 12457 | 13756 |
| | 24205 | 15796 | 27883 | 16773 | 28772 | 1538 | 18102 | 8929 | 13368 | 17299 | 19533 | 24355 |
| | 3670 | 23641 | 10408 | 24774 | 24121 | 28029 | 5706 | 17605 | 10829 | 980 | 3583 | 3381 |
| | 11958 | 7811 | 3282 | 2598 | 12058 | 18178 | 25391 | 1561 | 24068 | 23106 | 20067 | 29929 |
| | 5270 | 21145 | 29080 | 28832 | 9542 | 12968 | 7842 | 5783 | 16238 | 7338 | 5930 | 19959 |
| | 28042 | 13790 | 4801 | 1430 | 9377 | 3050 | 12433 | 10545 | 12519 | 13723 | 9393 | 26727 |
| | 27628 | 12352 | 17120 | 16729 | 13926 | 630 | 17440 | 18160 | | | | |
| 629: | 2827 | 21648 | 22239 | 20396 | 5350 | 18594 | 13414 | 3105 | 3122 | 10761 | 21096 | 1570 |
| | 8290 | 24441 | 1819 | 7703 | 17446 | 15026 | 11413 | 16074 | 28826 | 8926 | 2985 | 23827 |
| | 18789 | 21796 | 1100 | 27681 | 12292 | 25281 | 6132 | 29085 | 1437 | 17574 | 20269 | 22049 |
| | 1861 | 15353 | 9151 | 16462 | 29950 | 30298 | 8628 | 4670 | 29916 | 22022 | 20351 | 6265 |
| | 26983 | 1260 | 29757 | 11581 | 1116 | 11626 | 25314 | 12647 | 21487 | 22732 | 8349 | 9194 |
| | 5078 | 28322 | 23446 | 29598 | 28404 | 29768 | 27807 | 1435 | 22280 | 24172 | 12973 | 21035 |
| | 28461 | 29323 | 8678 | 26140 | 3550 | 1842 | 27577 | 21267 | 2722 | 29953 | 12906 | 10787 |
| | 20886 | 9494 | 20065 | 17225 | 28472 | 14449 | 4088 | 13827 | 20362 | 4769 | 6736 | 4217 |
| | 9914 | 13158 | 22017 | 16750 | 14583 | 14800 | 13660 | 27988 | 6695 | 22432 | 28758 | 14595 |

SEQ ID NO: homolog SEQ ID NOs

| SEQ ID NO | | | | | | | | | | | | |
|---|---|---|---|---|---|---|---|---|---|---|---|---|
| | 1389 | 22126 | 2553 | 7024 | 17822 | 2289 | 6487 | 6484 | 6502 | 6229 | 11479 | 14051 |
| | 16467 | 21170 | 8907 | 8887 | 28847 | 3948 | 11527 | 13632 | 5217 | 1071 | 12831 | 21444 |
| | 28123 | 5960 | 29252 | 10600 | 1560 | 15102 | 21836 | 21517 | 1767 | 16340 | 15215 | 19213 |
| | 13163 | 5989 | 7408 | 1250 | 14864 | 2145 | 20980 | 20786 | 25489 | 19890 | 10154 | 8406 |
| | 3192 | 29520 | 21938 | 25996 | 1922 | 19108 | 2311 | 3944 | 26383 | 25937 | 12706 | 23547 |
| | 19501 | 19503 | 13681 | 21632 | 22326 | 21619 | 21916 | 6584 | 23359 | 28375 | 22553 | 23759 |
| | 1138 | 18646 | 12204 | 23294 | 1286 | 18001 | 9694 | 21865 | 3196 | 20557 | 27271 | 16962 |
| | 2957 | 2952 | 15708 | 3560 | 5363 | 5347 | 11222 | 30129 | 7897 | 3361 | 26621 | 22294 |
| | 21939 | 28384 | 17873 | 23061 | 9577 | 4111 | 22934 | 12654 | 16339 | 4257 | 6797 | 12457 |
| | 13756 | 24205 | 15796 | 27883 | 16773 | 28772 | 1538 | 18102 | 18360 | 6318 | 3483 | 14531 |
| | 14548 | 8477 | 28995 | 20413 | 18118 | 14384 | 18507 | 28934 | 12262 | 8671 | 23641 | 10408 |
| | 24774 | 11769 | 28029 | 5706 | 17605 | 10829 | 980 | 23855 | 9303 | 20202 | 12102 | 3583 |
| | 3381 | 17326 | 19988 | 5547 | 4806 | 12802 | 20154 | 12058 | 18178 | 25391 | 1561 | 24068 |
| | 23106 | 20067 | 29929 | 5270 | 21145 | 29080 | 28832 | 9542 | 12968 | 7842 | 5783 | 16238 |
| | 7338 | 5930 | 19959 | 28042 | 13790 | 4801 | 7485 | 1430 | 9377 | 3050 | 12433 | 12519 |
| | 10545 | 13723 | 9393 | 26727 | 27628 | 12352 | 17120 | | | | | |
| 630: | 2827 | 21648 | 22239 | 20396 | 6761 | 6766 | 6765 | 5350 | 18594 | 13414 | 3105 | 3122 |
| | 10761 | 21096 | 1570 | 8290 | 24441 | 1819 | 7703 | 17446 | 15026 | 11413 | 16074 | 28826 |
| | 8926 | 2985 | 23827 | 18789 | 21796 | 1100 | 27681 | 12292 | 25281 | 6132 | 29085 | 1437 |
| | 17574 | 20269 | 22049 | 1861 | 15353 | 9151 | 16462 | 29950 | 30298 | 8628 | 4670 | 29916 |
| | 22022 | 20351 | 6265 | 26983 | 1260 | 29757 | 11581 | 1116 | 11626 | 12647 | 25314 | 21487 |
| | 22732 | 8349 | 9194 | 5078 | 28322 | 23446 | 29598 | 28404 | 29768 | 27807 | 1435 | 22280 |
| | 24172 | 12973 | 28461 | 21035 | 29323 | 8678 | 26140 | 3550 | 1842 | 27577 | 21267 | 29953 |
| | 2722 | 12906 | 20886 | 10787 | 9494 | 20065 | 17225 | 28472 | 14449 | 4088 | 13827 | 4769 |
| | 20362 | 6736 | 4217 | 9914 | 13158 | 22017 | 16750 | 14583 | 14800 | 13660 | 27988 | 6695 |
| | 22432 | 28758 | 14595 | 1389 | 22126 | 2553 | 7024 | 17822 | 2289 | 6487 | 6484 | 6229 |
| | 6502 | 11479 | 14051 | 16467 | 21170 | 8907 | 8887 | 28847 | 3948 | 11527 | 13632 | 5217 |
| | 1071 | 12831 | 21444 | 28123 | 5960 | 29252 | 10600 | 1560 | 15102 | 21836 | 21517 | 1767 |
| | 16340 | 15215 | 19213 | 13163 | 7408 | 5989 | 1250 | 14864 | 2145 | 20980 | 20786 | 25489 |
| | 19890 | 10154 | 8406 | 3192 | 29520 | 21938 | 25996 | 1922 | 19108 | 2311 | 3944 | 26383 |
| | 25937 | 12706 | 23547 | 19501 | 19503 | 13681 | 21632 | 22326 | 21619 | 21916 | 6584 | 23359 |
| | 28375 | 22553 | 23759 | 1138 | 18646 | 12204 | 23294 | 1286 | 18001 | 9694 | 21865 | 3196 |
| | 20557 | 27271 | 16962 | 2957 | 2952 | 15708 | 3560 | 5363 | 5347 | 11222 | 30129 | 7897 |

(continued)

SEQ ID NO: homolog SEQ ID NOs

| SEQ ID NO | | | | | | | | | | | | |
|---|---|---|---|---|---|---|---|---|---|---|---|---|
| | 26621 | 3361 | 22294 | 21939 | 28384 | 17873 | 23061 | 9577 | 4111 | 22934 | 16339 | 12654 |
| | 4257 | 6797 | 12457 | 13756 | 24205 | 15796 | 16773 | 27883 | 28772 | 1538 | 18102 | 18360 |
| | 6318 | 3483 | 13368 | 17299 | 8929 | 19533 | 24355 | 3670 | 23641 | 10408 | 24774 | 24121 |
| | 28029 | 5706 | 17605 | 10829 | 980 | 9303 | 20202 | 12102 | 3583 | 3381 | 18011 | 11958 |
| | 20873 | 4806 | 12058 | 18178 | 25391 | 1561 | 24068 | 23106 | 20067 | 29929 | 5270 | 21145 |
| | 29080 | 28832 | 9542 | 12968 | 7842 | 5783 | 16238 | 7338 | 19959 | 28042 | 13790 | 4801 |
| | 7485 | 1430 | 3050 | 12433 | 10545 | 12519 | 13723 | 9393 | 26727 | 27628 | 12352 | 17120 |
| | 18160 | 628 | 13926 | 16729 | | | | | | | | |
| 631: | 2912 | 2796 | 2818 | 2823 | 2824 | 2889 | 2887 | 2881 | 24871 | 5777 | 4318 | 1807 |
| | 7096 | 15887 | 13427 | 13428 | 1396 | 11883 | 8418 | 16565 | 12937 | 16594 | 16574 | 3766 |
| | 1822 | 6631 | 29752 | 26443 | 3859 | 6574 | 4599 | 5652 | 12826 | 10837 | 4539 | 2372 |
| | 4583 | 29275 | 1656 | 28678 | 5667 | 5665 | 22758 | 27668 | 9995 | 22671 | 18616 | 23608 |
| | 23577 | 23605 | 23604 | 28308 | 19952 | 19951 | 23578 | 10034 | 16194 | 16197 | 19886 | 9038 |
| | 23412 | 24945 | 3734 | 21162 | 9553 | 9552 | 9561 | 29562 | 17830 | 10139 | 22862 | 16596 |
| | 14401 | 15073 | 5678 | 10933 | 18508 | 11829 | 11291 | | | | | |
| 632: | 16189 | 25580 | 21911 | 8067 | 4586 | 19811 | 12749 | 7049 | 3358 | 6977 | 15948 | 15946 |
| | 8868 | 9888 | 14075 | 18674 | 16333 | 4908 | 23516 | 4402 | 3501 | 4285 | 937 | 8723 |
| | 1872 | 8022 | 22031 | 23393 | 15710 | 15714 | 18841 | 19831 | 19817 | 21081 | 6316 | 18573 |
| | 19327 | 28194 | 24281 | 29196 | 1565 | 18557 | 2453 | 20385 | 29042 | 17260 | 7138 | 7144 |
| | 2371 | 10073 | 24249 | 14881 | 26773 | 19205 | 26025 | 27912 | 9674 | 6105 | 10716 | 3455 |
| | 29215 | 10641 | 3868 | 27698 | 9294 | 25213 | 16430 | 24134 | 21976 | 14491 | 14495 | 11660 |
| | 24317 | 13514 | 21711 | 24534 | 23089 | 7833 | 20961 | 21982 | 11419 | 24406 | 20726 | 20716 |
| | 20688 | 20719 | 20723 | 24754 | 8589 | 8587 | 7737 | 4738 | 4736 | 4755 | 26897 | 13746 |
| | 17887 | 990 | 26865 | 9972 | 21468 | 6545 | 10250 | 20969 | 11288 | 11346 | 1554 | 6522 |
| | 6516 | 24423 | 27938 | 786 | 28252 | 24425 | 7133 | 7131 | 28664 | 5784 | 4083 | 30196 |
| | 15931 | 8964 | 22024 | 4158 | 18522 | 16018 | 4854 | 6874 | 15936 | 9746 | 9745 | 12220 |
| | 13520 | 20170 | 782 | 16938 | 11423 | 4205 | 26124 | 26129 | 2545 | 2665 | 3586 | 22500 |
| | 21376 | 21375 | 12718 | 12367 | 23566 | 7460 | 22831 | 22829 | 24308 | 12444 | 23282 | 15034 |
| | 15032 | 15035 | 19307 | 1321 | 16982 | 28653 | 15553 | 8724 | 24291 | 18843 | 5077 | 15246 |
| | 23363 | 11109 | 27763 | 3504 | 23044 | 23923 | 23890 | 23892 | 22454 | 1368 | 19815 | 22452 |
| | 3553 | 21008 | 6325 | 20951 | 22450 | 3726 | 11746 | 10709 | 3723 | 23728 | 2848 | 2841 |
| | 2118 | 18673 | 2094 | 2093 | 2061 | 2058 | 2053 | 19810 | 29165 | 11759 | 15670 | 13840 |
| | 12863 | 26954 | 19105 | 2995 | 29492 | 865 | 22661 | 22624 | 15849 | 10459 | 27243 | 8555 |

EP 2 540 831 A2

(continued)

| SEQ ID NO: | homolog SEQ ID NOs | | | | | | | | | | | |
|---|---|---|---|---|---|---|---|---|---|---|---|---|
| | 4932 | 17612 | 4722 | 2602 | 20100 | 27006 | 5533 | 26150 | 1494 | 13417 | 14316 | 5807 |
| | 5794 | 11129 | 2465 | 24262 | 24260 | 25822 | 14904 | 14936 | 14908 | 14876 | 16216 | 7967 |
| | 20840 | 14838 | 14804 | 14869 | 14840 | 14833 | 897 | 15046 | 23482 | 23509 | 23512 | 5962 |
| | 5957 | 5959 | 23377 | 16977 | 17289 | 21296 | | | | | | |
| 633: | 27380 | 28846 | 8116 | 7165 | 10010 | 7973 | 4861 | 1919 | 17582 | 29569 | 29464 | 23355 |
| | 27418 | 17321 | 13844 | 19488 | 17560 | 8017 | 8072 | 8042 | 12451 | 22195 | 10039 | 27389 |
| | 28290 | 22544 | 15854 | 9195 | 5404 | 5292 | 27352 | 13087 | 18539 | 8121 | 8118 | 14188 |
| | 9469 | 13412 | | | | | | | | | | |
| 634: | 2413 | 28000 | 20414 | 6332 | 24867 | 15974 | 2380 | 18219 | 11560 | 7534 | 19891 | 6629 |
| | 13532 | 19894 | 14341 | 17022 | 7718 | 27278 | 3175 | 19850 | 16266 | 5306 | 22626 | 21419 |
| | 21357 | 15646 | 21647 | 2804 | 5817 | 15484 | 18853 | 22602 | 10566 | 22176 | 24037 | 2334 |
| | 24210 | 24802 | 23651 | 10561 | 8577 | 5369 | 4017 | 1454 | 18255 | 18252 | 1951 | 22010 |
| | 15233 | 13860 | 14262 | 26191 | 28248 | 14438 | 8670 | 5066 | 24710 | 6620 | 18031 | 3617 |
| | 16694 | 8048 | 17411 | 26694 | 25784 | 25825 | 13303 | 1455 | 16039 | 25424 | 3878 | |
| 635: | 19520 | 22089 | 5863 | 25418 | 8136 | 30207 | 28147 | 2111 | 23798 | 9025 | 22730 | 4882 |
| | 25535 | 15019 | 7995 | 27952 | 18650 | 20928 | 21213 | 20548 | 7981 | 23339 | 14195 | 12936 |
| | 8556 | 27580 | 26883 | 16067 | 11961 | 6055 | 21462 | 21065 | 530 | 9582 | 12854 | 25248 |
| | 3905 | 3698 | 17319 | 19183 | 7228 | 17708 | 28553 | 19451 | 5970 | 14452 | 2139 | 15361 |
| | 7668 | 25273 | 3830 | 11809 | 19010 | 7318 | 17385 | 3302 | 11339 | 22660 | 21637 | 21552 |
| | 16256 | 17380 | 16668 | 3687 | 4939 | 9629 | 8784 | 14226 | 12851 | 17838 | 10838 | 15436 |
| | 3168 | 28364 | 14918 | 15872 | 2982 | 8870 | 21771 | 27718 | 13094 | 6001 | 26978 | 20010 |
| | 6323 | 20282 | 27731 | 22494 | 1885 | 10353 | 21749 | 19157 | 4636 | 21628 | 18429 | 28890 |
| | 20529 | 11277 | 3060 | 10198 | 7038 | 3083 | 17806 | 5599 | 29815 | 28179 | 8840 | 6071 |
| | 25675 | 6029 | 12558 | 5934 | 14122 | 1099 | 13195 | 8827 | 4933 | 7992 | 5669 | 7923 |
| | 2661 | 27431 | 8897 | 29171 | 22077 | 5927 | 18876 | 19785 | 1566 | 23594 | 29794 | 28728 |
| | 13099 | 3611 | 22860 | 4453 | 24900 | 25842 | 26400 | 25992 | 24161 | 7972 | 25286 | 23617 |
| | 16803 | 10800 | 28733 | 14423 | 4620 | 7043 | 26747 | 29751 | 11529 | 12334 | 14074 | 16915 |
| | 15935 | 22519 | 8380 | 3052 | 3396 | 26436 | 22179 | 10517 | 21526 | 21264 | 16049 | 9702 |
| | 4940 | 3049 | 5468 | 26945 | 18051 | 24331 | 16089 | 15839 | 16580 | 9272 | 28233 | 15058 |
| | 21458 | 8914 | 7040 | 28058 | 8689 | 5358 | 26186 | 18901 | 2055 | 13556 | 9861 | 27584 |
| | 8764 | 3411 | 14780 | 21646 | 28639 | 16642 | 15494 | 23279 | 29398 | 26418 | 9176 | 7441 |
| | 26930 | 7450 | 2430 | 21980 | 26765 | 7536 | 29041 | 28235 | 11927 | 1908 | 20722 | 761 |
| | 26345 | 20449 | 25743 | 4248 | 5969 | 2538 | 5406 | 26192 | 8991 | 26059 | 12370 | 10925 |

SEQ ID NO: homolog SEQ ID NOs

| | | | | | | | | | | | | |
|---|---|---|---|---|---|---|---|---|---|---|---|---|
| | 23918 | 17449 | 3975 | 19903 | 8189 | 5074 | 2596 | 25811 | 20558 | 22052 | 16969 | 28839 |
| | 23316 | 27134 | 8685 | 17644 | 17895 | 19930 | 14534 | 22220 | 12413 | 817 | 5456 | 7516 |
| | 25694 | 25624 | 8593 | 2675 | 2676 | 12512 | 12536 | 20372 | 15249 | 15247 | 17555 | 636 |
| | 28010 | 23518 | 25789 | 27449 | 22466 | 5342 | 30199 | 24006 | 5771 | 25446 | 9442 | 9361 |
| | 2533 | 2087 | 13670 | 13139 | 25857 | 510 | 26696 | 509 | | | | |
| 636: | 6341 | 23042 | 7983 | 8856 | 20845 | 6914 | 16780 | 21678 | 1958 | 27864 | 16921 | 30232 |
| | 5344 | 29798 | 25007 | 12563 | 25400 | 8503 | 30248 | 3112 | 12900 | 3664 | 26110 | 28761 |
| | 3928 | 27469 | 28342 | 17635 | 4050 | 20544 | 23715 | 6188 | 8646 | 22948 | 17358 | 28959 |
| | 20142 | 1083 | 3226 | 7988 | 27517 | 20039 | 30315 | 4507 | 18180 | 10951 | 13180 | 5027 |
| | 5463 | 25246 | 23029 | 18175 | 12422 | 21693 | 13953 | 15593 | 19887 | 3799 | 4286 | 24475 |
| | 5845 | 25026 | 10244 | 21176 | 28475 | 4186 | 11348 | 26772 | 18942 | 2231 | 25743 | 4248 |
| | 5969 | 8991 | 26059 | 12370 | 635 | 21515 | 12727 | 16265 | 24488 | 27685 | 22466 | 13287 |
| | 4621 | 24660 | 23125 | 12410 | 17054 | 505 | | | | | | |
| 637: | 18021 | 11599 | 6049 | 14302 | 19694 | 12326 | 26545 | 29287 | 15776 | 11726 | 10579 | 11886 |
| | 5708 | 20510 | 17364 | 6685 | 20792 | 20790 | 20768 | 4294 | 26081 | 1107 | 19373 | 26653 |
| | 5831 | 13038 | 27604 | 7837 | 27866 | 12407 | 7631 | 10058 | 24148 | 23025 | 2822 | |
| 638: | 3951 | 24339 | 18199 | 13212 | 17561 | 8714 | 28018 | 1074 | 25894 | 16605 | 16166 | 15851 |
| | 26097 | 5640 | 10391 | 11814 | 1444 | 20213 | 20444 | 27204 | 27206 | 22306 | 11308 | 12522 |
| | 13032 | 8102 | 9519 | 6901 | 13752 | 21480 | 9721 | 18724 | 5847 | 859 | 3128 | 26108 |
| | 15713 | 12725 | 8760 | 21318 | 18810 | 9092 | 23297 | 23096 | 12000 | 18049 | 4167 | 24810 |
| | 1982 | 28062 | 26199 | 26467 | 8927 | 26041 | 23105 | 910 | 21763 | 4203 | 28528 | 12153 |
| | 7662 | 16493 | 14564 | 9811 | 6075 | 24706 | 7716 | 24689 | 12867 | 29675 | 26937 | 13338 |
| | 20725 | 14597 | 23372 | 9455 | 22960 | 25839 | 28289 | 4506 | 28576 | 1865 | 22942 | 15183 |
| | 8285 | 10786 | 16637 | 30238 | 4525 | 15815 | 25301 | 22579 | 17489 | 12357 | 13168 | 12961 |
| | 6926 | 3701 | 24605 | 30258 | 12142 | 6988 | 19344 | 11177 | 2446 | 15241 | 12137 | 7608 |
| | 12510 | 9818 | 16416 | 27560 | 3808 | 2653 | 17351 | 22267 | 5595 | 8253 | 4181 | 8786 |
| | 15573 | 9142 | 12547 | 19808 | 20521 | 21286 | 21615 | 10748 | 22896 | 23635 | 23136 | 8510 |
| | 6719 | 22014 | 27097 | 4169 | 19201 | 5175 | 30180 | 28738 | 18468 | 21226 | 26393 | 13054 |
| | 22091 | 12852 | 16742 | 25850 | 16557 | 11987 | 5693 | 28184 | 9732 | 2245 | 3363 | 22629 |
| | 25041 | 5785 | 5213 | 26367 | 3107 | 26869 | 2353 | 17262 | 3840 | 19505 | 23947 | 20675 |
| | 6871 | 14856 | 29402 | 18190 | 18194 | 28770 | 7607 | 27798 | 15616 | 6312 | 8301 | 14849 |
| | 22793 | 24792 | 5782 | 1778 | 3143 | 7156 | 25240 | 5015 | 5879 | 3702 | 1461 | 14321 |
| | 5996 | 8232 | 23322 | 21075 | 10450 | 6063 | 17881 | 17152 | 14424 | 10557 | 8869 | 28160 |

EP 2 540 831 A2

(continued)

SEQ ID NO: homolog SEQ ID NOs

| SEQ ID NO: | | | | | | | | | | | | |
|---|---|---|---|---|---|---|---|---|---|---|---|---|
| | 5173 | 28214 | 21201 | 8968 | 28401 | 29045 | 8597 | 21239 | 16739 | 24392 | 28988 | 20306 |
| | 8873 | 27490 | 20657 | 17300 | 6664 | 30062 | 3028 | 17077 | 16866 | 29937 | 29031 | 2412 |
| | 11945 | 11608 | 9107 | 25733 | 12087 | 3571 | 18727 | 16488 | 7188 | 17906 | 2256 | 27892 |
| | 11074 | 24259 | 30078 | 4308 | 15508 | 1312 | 16032 | 8184 | 11741 | 12167 | 30293 | 6674 |
| 639: | 5666 | 23314 | 25782 | 8659 | 10609 | 11840 | 20523 | 14260 | 24024 | 2814 | 27263 | 23378 |
| | 17832 | 3601 | 1586 | 22784 | 17707 | 16349 | 29088 | 24342 | 12073 | 28126 | 8905 | 7726 |
| | 28996 | 11243 | 14625 | 12218 | 25775 | 9373 | 1617 | 25076 | 5754 | 11313 | | |
| 640: | 21175 | 7387 | 7108 | 10101 | 24142 | 10449 | 4773 | 15437 | 6343 | 16051 | 818 | 3634 |
| | 20025 | 19324 | 15089 | 14681 | 2309 | 19487 | 2513 | 7250 | 18974 | 3453 | 1642 | 26932 |
| | 25990 | 2459 | 28059 | 27518 | 22090 | 22711 | 27492 | 12845 | 26527 | 23722 | | |
| 641: | 15978 | 27182 | 9857 | 5952 | 3309 | 20409 | 14322 | 20210 | 5143 | 10184 | 11059 | 20799 |
| | 14055 | 23627 | 26166 | 1629 | 5067 | 2250 | 17060 | 24992 | 25579 | 2415 | 18554 | 3776 |
| | 7502 | 29660 | 25045 | 4949 | 5950 | 1953 | 22277 | 13524 | 7313 | 19852 | 11708 | 27769 |
| | 28896 | 7849 | 3855 | 8347 | 4103 | 7453 | 20346 | 11280 | 14735 | 8432 | 26466 | 25132 |
| | 29390 | 27270 | 1008 | 23719 | 9595 | 18244 | 11768 | 17831 | 13278 | 12417 | 23210 | 8084 |
| | 22344 | 3291 | | | | | | | | | | |
| 642: | 12755 | 11304 | 3290 | 19742 | 3366 | 10273 | 13326 | 12861 | 5569 | 20547 | 16130 | 11818 |
| | 4903 | 5906 | 1928 | 29413 | 8663 | 23503 | 27551 | 20823 | 17498 | 4020 | 27277 | 11251 |
| | 29632 | 11297 | 18654 | 19369 | 29907 | 12933 | 11295 | 5512 | 4099 | 12425 | 15395 | 7471 |
| | 8371 | 6934 | 8658 | 7087 | 28669 | 29764 | 24410 | 26903 | 15556 | 8356 | 795 | 23212 |
| | 26157 | 3214 | 30262 | 19320 | 22728 | 14037 | 12759 | 10960 | 8276 | 28889 | 23758 | 9571 |
| | 17432 | 6391 | | | | | | | | | | |
| 643: | 20604 | 17230 | 19793 | 9004 | 21929 | 17800 | 11232 | 15332 | 24138 | 27298 | 28990 | 25680 |
| | 20082 | 5612 | 6868 | 5242 | 18465 | 21543 | 7274 | 8220 | 5591 | 6418 | 26265 | 1432 |
| | 1745 | 9855 | 10955 | 6180 | 12163 | 11663 | 25940 | 10619 | 1317 | 10041 | 9390 | 6647 |
| | 24112 | 23121 | 2812 | 13323 | 7207 | 18457 | 26338 | 3813 | 30324 | 20903 | 18273 | 7875 |
| | 3517 | 29146 | 25200 | 23266 | 9021 | 26759 | 27424 | 28092 | 14433 | 9875 | 13020 | 17095 |
| | 5304 | 17192 | 20827 | 8459 | 25794 | 22446 | 29089 | 10554 | 16388 | 14668 | 12008 | 29036 |
| | 6069 | 4927 | 9237 | 3700 | 6718 | 20865 | 11720 | 7594 | 6154 | 5041 | 24701 | 14411 |
| | 12372 | 24460 | 5601 | 22495 | 27128 | 10628 | 19741 | 15349 | 25435 | 14646 | 21744 | 19292 |
| | 3339 | 28295 | 16925 | 28069 | 24022 | 22914 | 29943 | 3768 | 15062 | 4910 | 670 | 669 |
| | 16062 | 19409 | 17264 | 22523 | 13758 | 10261 | 16761 | 23788 | 1338 | 20496 | 1846 | 7303 |
| | 22194 | 2932 | 12405 | 29250 | 8637 | 26139 | 9091 | 8374 | | | | |

SEQ ID NO: homolog SEQ ID NOs

| 644: | 3201 | 4339 | 29374 | 3933 | 2293 | 26994 | 28971 | 28917 | 6854 | 20981 | 940 | 7066 |
|---|---|---|---|---|---|---|---|---|---|---|---|---|
| | 29493 | 25686 | 23510 | 24816 | 20316 | 2573 | 16627 | 3247 | 19096 | 22610 | 12927 | 23524 |
| | 2768 | | | | | | | | | | | |
| 645: | 21117 | 3627 | 1175 | 1701 | 1667 | 29286 | 2141 | 5792 | 1472 | 8041 | 10799 | 929 |
| | 26844 | 12256 | 11364 | 27382 | 24711 | 28269 | 3271 | 14648 | 4393 | 22872 | 10691 | 12674 |
| | 15044 | 30142 | 16066 | 1787 | 18173 | 11948 | 19756 | 7831 | 22368 | 25108 | 13726 | 11112 |
| | 23607 | 10713 | 20703 | | | | | | | | | |
| 646: | 13175 | 8057 | 13962 | 3385 | 6474 | 7596 | 4754 | 4904 | 8474 | 19615 | 10540 | 8629 |
| | 11981 | 24495 | 15926 | 21782 | 16093 | 17754 | 9756 | 28501 | 12905 | 4567 | 5700 | 11577 |
| | 19087 | 13295 | 23876 | 9676 | 3639 | 1659 | 821 | 19246 | 8245 | 23542 | 10020 | 16107 |
| | 28997 | 28141 | | | | | | | | | | |
| 647: | 1352 | 4866 | 25439 | 9321 | 26777 | 6762 | 28923 | 23371 | 27205 | 6120 | 24133 | 16869 |
| | 11897 | 25970 | 4850 | 12587 | 3801 | 4501 | 30297 | 28943 | 21047 | 16471 | 22600 | 25988 |
| | 19077 | 4815 | 26145 | 944 | 8949 | 22384 | 17033 | 5658 | 7375 | 25720 | 18226 | 20487 |
| | 24915 | 4503 | 16532 | 8295 | | | | | | | | |
| 648: | 1352 | 4866 | 25439 | 9321 | 26777 | 6762 | 28923 | 23371 | 27205 | 6120 | 24133 | 16869 |
| | 11897 | 25970 | 4850 | 12587 | 3801 | 4501 | 30297 | 28943 | 21047 | 16471 | 22600 | 25988 |
| | 19077 | 4815 | 26145 | 944 | 8949 | 22384 | 17033 | 5658 | 7375 | 25720 | 18226 | 20487 |
| | 24915 | 4503 | 16532 | 8295 | | | | | | | | |
| 649: | 16571 | 21287 | 27838 | 15172 | 16202 | 22352 | 27969 | 23227 | 13713 | 14694 | 23468 | 8944 |
| | 8988 | 27377 | 28993 | 16664 | 3373 | 18143 | 10124 | 5349 | 7573 | 3803 | 11373 | 15619 |
| | 11379 | 7845 | 5248 | 24465 | 9436 | 11695 | 23404 | 28775 | 15555 | 25432 | 5320 | 3608 |
| | 10390 | 9490 | 6592 | 17020 | 12920 | 27572 | 16305 | 1335 | 2227 | 15386 | 4505 | 24629 |
| | 1151 | 2319 | 21992 | 26046 | 21217 | 25051 | 17002 | 24439 | | | | |
| 650: | 9030 | 2350 | 28197 | 22084 | 15722 | 8706 | 29375 | 21003 | 25476 | 12834 | 13811 | 13007 |
| | 17974 | 25547 | 26115 | | | | | | | | | |
| 651: | 11864 | 9052 | 7997 | 7645 | 9771 | 13569 | 3116 | 17649 | 3266 | 18146 | 12335 | 28856 |
| | 15652 | 24688 | 13359 | 8370 | 24861 | 11771 | 6351 | 11101 | 13337 | 21889 | 2636 | 23979 |
| | 13916 | 10680 | 19902 | | | | | | | | | |
| 652: | 14550 | 21457 | 28599 | 2440 | 29058 | 8583 | 23375 | 5671 | 5613 | 5610 | 5584 | 5582 |
| | 5578 | 5531 | 5499 | 5420 | 4695 | 5609 | 5576 | 5460 | 4698 | 5636 | 5458 | 5418 |
| | 5632 | 4705 | 5465 | 5631 | 5524 | 5461 | 16522 | 15116 | 18311 | 19076 | 25482 | 2015 |
| | 28081 | 7719 | 2022 | 2018 | 28949 | 16408 | 14991 | 13358 | 12134 | 21541 | 18306 | 8863 |

SEQ ID NO: homolog SEQ ID NOs

| | | | | | | | | | | | |
|---|---|---|---|---|---|---|---|---|---|---|---|
| | 11245 | 18203 | 18200 | 18205 | 18983 | 11269 | 8029 | 8025 | 7143 | 4004 | 28676 | 6311 |
| | 19311 | 19332 | 10807 | 13742 | 8866 | 26456 | 17391 | 18872 | 27125 | 27121 | 16706 | 29294 |
| | 11724 | 6017 | 3631 | 11139 | 7002 | 21934 | 30221 | 14474 | 16933 | 3121 | 16932 | 1416 |
| | 12242 | 10193 | 10189 | 24408 | 11187 | 11182 | 8682 | 2248 | 23119 | 30127 | 7278 | 23929 |
| | 29780 | 14948 | 2643 | 15988 | 19527 | 2166 | 2194 | 27045 | 4000 | 13061 | 2193 | 8775 |
| | 3137 | 25464 | 2263 | 3575 | 2259 | 3578 | 17496 | 3162 | 30009 | 13077 | 1743 | 12080 |
| | 17293 | 10364 | 6849 | 11661 | 22532 | 8860 | 27719 | 22251 | 21120 | 5828 | 5824 | 24256 |
| | 24225 | 12342 | 18904 | 18869 | 18866 | 18863 | 28145 | 8530 | 8492 | 25029 | 13542 | 26290 |
| | 26329 | 8143 | 8140 | 14363 | 20916 | 24929 | 26668 | 27737 | 14472 | 7282 | 751 | 752 |
| | 779 | 4995 | 29380 | 26824 | 8372 | 20293 | 8662 | 19636 | 9594 | 19707 | 19640 | 19672 |
| | 8412 | 7612 | 19751 | 5288 | 13344 | 14986 | 16537 | 13244 | 13248 | 1406 | 1264 | 13955 |
| | 5249 | 23095 | 25727 | 18755 | 20393 | 20391 | 29117 | 23285 | 18692 | 18733 | 18735 | 24383 |
| | 17104 | 17098 | 7706 | 4739 | 27710 | 14510 | 10354 | 10360 | 12780 | 24057 | 19578 | 22275 |
| | 3342 | 16820 | 2010 | 10402 | 5326 | 20108 | 13888 | 27340 | 27008 | 22780 | 12507 | 18582 |
| | 17651 | 9509 | 23913 | 12651 | 16740 | 22365 | 4087 | 3590 | 12319 | 6880 | 9842 | 29837 |
| | 16658 | 5759 | 28749 | 13393 | 17424 | 6087 | 7067 | 10939 | 17116 | 25869 | 3715 | 22538 |
| | 23370 | 8337 | 27974 | 3630 | 8511 | 10869 | 6122 | 10183 | 1665 | 13806 | 12866 | 5823 |
| | 7280 | 20051 | 20416 | 7567 | 8345 | 19293 | 27302 | 2855 | 14375 | 3537 | 11635 | 3893 |
| | 28596 | 10279 | 21625 | 12486 | 12483 | 25705 | 5130 | 5129 | 23548 | 4864 | 24556 | 14357 |
| | 16003 | 17947 | 29659 | 24442 | 24621 | 28040 | 17252 | 28039 | 16745 | 24445 | 10958 | 29839 |
| | 25572 | 6298 | 19123 | | | | | | | | | |
| 653: | 14550 | 21457 | 28599 | 2440 | 29058 | 8583 | 23375 | 5671 | 5613 | 5610 | 5584 | 5582 |
| | 5578 | 5531 | 5499 | 5420 | 4695 | 5609 | 5576 | 5460 | 4698 | 5636 | 5458 | 5418 |
| | 5632 | 4705 | 5465 | 5631 | 5524 | 5461 | 16522 | 15116 | 18311 | 19076 | 25482 | 2015 |
| | 28081 | 7719 | 2022 | 2018 | 28949 | 16408 | 14991 | 13358 | 12134 | 21541 | 18306 | 8863 |
| | 11245 | 18203 | 18200 | 18205 | 18983 | 11269 | 8029 | 8025 | 7143 | 4004 | 28676 | 6311 |
| | 19311 | 19332 | 10807 | 13742 | 8866 | 26456 | 17391 | 18872 | 27125 | 27121 | 16706 | 29294 |
| | 11724 | 6017 | 3631 | 11139 | 7002 | 21934 | 30221 | 14474 | 16933 | 3121 | 16932 | 1416 |
| | 12242 | 10193 | 10189 | 24408 | 11187 | 11182 | 8682 | 2248 | 23119 | 30127 | 7278 | 23929 |
| | 29780 | 14948 | 2643 | 15988 | 19527 | 2166 | 2194 | 27045 | 4000 | 13061 | 2193 | 8775 |
| | 3137 | 25464 | 2263 | 3575 | 2259 | 3578 | 17496 | 3162 | 30009 | 13077 | 1743 | 12080 |
| | 17293 | 10364 | 6849 | 11661 | 22532 | 8860 | 27719 | 22251 | 21120 | 5828 | 5824 | 24256 |
| | 24225 | 12342 | 18904 | 18869 | 18866 | 18863 | 28145 | 8530 | 8492 | 25029 | 13542 | 26290 |

(continued)

SEQ ID NO: homolog SEQ ID NOs

| Label | SEQ ID NO | homolog SEQ ID NOs | | | | | | | | | | |
|---|---|---|---|---|---|---|---|---|---|---|---|---|
| | 26329 | 8143 | 8140 | 14363 | 20916 | 24929 | 26668 | 27737 | 14472 | 7282 | 751 | 752 |
| | 779 | 4995 | 29380 | 26824 | 8372 | 20293 | 8662 | 19636 | 9594 | 19707 | 19640 | 19672 |
| | 8412 | 7612 | 19751 | 5288 | 13344 | 14986 | 16537 | 13244 | 13248 | 1406 | 1264 | 13955 |
| | 5249 | 23095 | 25727 | 18755 | 20393 | 20391 | 29117 | 23285 | 18692 | 18733 | 18735 | 24383 |
| | 17104 | 17098 | 7706 | 4739 | 27710 | 14510 | 10354 | 10360 | 12780 | 24057 | 19578 | 22275 |
| | 3342 | 16820 | 2010 | 10402 | 5326 | 20108 | 13888 | 27340 | 27008 | 22780 | 12507 | 18582 |
| | 17651 | 9509 | 23913 | 12651 | 16740 | 22365 | 4087 | 3590 | 12319 | 6880 | 9842 | 29837 |
| | 16658 | 5759 | 28749 | 13393 | 17424 | 6087 | 7067 | 10939 | 17116 | 25869 | 3715 | 22538 |
| | 23370 | 8337 | 27974 | 3630 | 8511 | 10869 | 6122 | 10183 | 1665 | 13806 | 12866 | 5823 |
| | 7280 | 20051 | 20416 | 7567 | 8345 | 19293 | 27302 | 2855 | 14375 | 3537 | 11635 | 3893 |
| | 28596 | 10279 | 21625 | 12486 | 12483 | 25705 | 5130 | 5129 | 23548 | 4864 | 24556 | 14357 |
| | 16003 | 17947 | 29659 | 24442 | 24621 | 28040 | 17252 | 28039 | 16745 | 24445 | 10958 | 29839 |
| | 25572 | 6298 | 19123 | | | | | | | | | |
| 654: | 19918 | 9689 | 11516 | 29399 | 19038 | 7398 | 28036 | 16244 | 27420 | 27934 | 8552 | 16641 |
| | 29318 | 7395 | 10107 | 6450 | 27813 | 7126 | | | | | | |
| 655: | 19918 | 9689 | 11516 | 29399 | 19038 | 7398 | 28036 | 16244 | 27420 | 27934 | 8552 | 16641 |
| | 29318 | 7395 | 10107 | 6450 | 27813 | 7126 | | | | | | |
| 656: | 3549 | 4762 | 12017 | 24658 | 1917 | 4280 | 22775 | 28534 | 6503 | 7302 | 7307 | 7300 |
| | 7306 | 8080 | 21638 | 16526 | 6739 | 9794 | 15537 | 18590 | 8923 | 23213 | 12294 | 8437 |
| | 22614 | | | | | | | | | | | |
| 657: | 3549 | 4762 | 12017 | 24658 | 1917 | 4280 | 22775 | 28534 | 6503 | 7302 | 7307 | 7300 |
| | 7306 | 8080 | 21638 | 16526 | 6739 | 9794 | 15537 | 18590 | 8923 | 23213 | 12294 | 8437 |
| | 22614 | | | | | | | | | | | |
| 658: | 20824 | 11537 | 7640 | 21182 | 18149 | 28456 | 28497 | 26624 | 24677 | 24671 | 24673 | 14509 |
| | 16545 | 21129 | 24999 | 4042 | 12876 | 23369 | 29198 | 24521 | 18910 | 24258 | 1399 | 23869 |
| | 12284 | 9286 | 22188 | 22181 | 19832 | 19761 | 20788 | 14167 | 13083 | 13084 | 19080 | 19051 |
| | 19047 | 19073 | 13088 | 19017 | 19014 | 9924 | 2821 | 29259 | 27367 | 2986 | 28422 | 28387 |
| | 28382 | 27308 | 27267 | 29293 | 2736 | 2735 | 27511 | 27496 | 28516 | 27515 | 13109 | 16212 |
| | 23959 | 25682 | 29055 | 3974 | 3206 | 22891 | 17516 | 2267 | 7034 | 7037 | 7074 | 7072 |
| | 6933 | 6929 | 6967 | 6971 | 18063 | 6101 | 6098 | 6984 | 18347 | 10944 | 6330 | 12317 |
| | 4287 | 17710 | 16585 | 21969 | 8137 | 12310 | 23333 | 5567 | 5282 | 2801 | 29160 | 1742 |
| | 28578 | 28586 | 28690 | 28716 | 28720 | 28748 | 28616 | 28620 | 28648 | 28652 | 28828 | 28791 |
| | 28821 | 28823 | 4098 | 29800 | 14254 | 14251 | 14255 | 14278 | 14274 | 14283 | 14281 | 14279 |

SEQ ID NO: homolog SEQ ID NOs

| | | | | | | | | | | | |
|---|---|---|---|---|---|---|---|---|---|---|---|
| 11736 | 4024 | 14891 | 15486 | 25770 | 24899 | 20453 | 12108 | 17558 | 16824 | 7770 | 10797 |
| 24448 | 7385 | 14312 | 19830 | 19803 | 29549 | 20364 | 20363 | 10965 | 18937 | 10618 | 4719 |
| 5778 | 23234 | 29175 | 15250 | 24332 | 16015 | 14180 | 20708 | 23853 | 28190 | 12026 | 8500 |
| 27926 | 29508 | 12698 | 21344 | 4981 | 14135 | 21334 | 19708 | 19717 | 19712 | 19755 | 19720 |
| 19754 | 19763 | 19767 | 19835 | 19839 | 30236 | 8982 | 5123 | 13554 | 12764 | 5543 | 14611 |
| 6786 | 9512 | 18372 | 6800 | 11169 | 19919 | 11247 | 10946 | 11470 | 1750 | 4907 | 13117 |
| 23068 | 6170 | 24535 | 3938 | 29024 | 4884 | 5978 | 20259 | 15213 | 11936 | 2230 | 2880 |
| 18778 | 26675 | 13603 | 24676 | 25024 | 13836 | 24146 | 25001 | 27777 | 16801 | 16079 | 18354 |
| 11975 | 29022 | 17198 | 1262 | 10683 | 4279 | 26134 | 23650 | 26620 | 9411 | 12463 | 20286 |
| 29354 | 27935 | 20960 | 5850 | 16922 | 22995 | 2764 | 12633 | 8938 | 19349 | 28494 | 10295 |
| 6703 | 20841 | 26656 | 27246 | 14398 | 22302 | 19218 | 30148 | 25944 | 28008 | 8867 | 25868 |
| 29195 | 23218 | 7922 | 6143 | 9904 | 18795 | 15083 | 2082 | 20826 | 7809 | 13409 | 23741 |
| 28492 | 9724 | 14821 | 29449 | 27615 | 29498 | 2730 | 2883 | 2980 | 27403 | 28357 | 27289 |
| 27467 | 27439 | 27293 | 2793 | 28426 | 29321 | 29317 | 2977 | 19079 | 6215 | 4726 | 10290 |
| 6515 | 3809 | 25679 | 13566 | 19800 | 19797 | 19156 | 1963 | 23950 | 23954 | 14700 | 1156 |
| 13732 | 29473 | 29348 | 27865 | 21185 | 16675 | 1606 | 29121 | 19021 | 21119 | 19081 | 21177 |
| 21144 | 21151 | 21179 | 21206 | 21210 | 19789 | 19792 | 28080 | 1593 | 1591 | 18635 | 10269 |
| 10340 | 22246 | 10301 | 10271 | 5257 | 10337 | 10308 | 10242 | 10240 | 10386 | 10343 | 10339 |
| 5007 | 29846 | 21123 | 16570 | 24045 | 22036 | 8912 | 8365 | 18202 | 18781 | 2966 | 13668 |
| 29970 | 27591 | 13760 | 26331 | 17588 | 9243 | 16140 | 14641 | 8942 | 16482 | 13781 | 7077 |
| 7079 | 16561 | 16524 | 9502 | 16502 | 9126 | 28804 | 14501 | 4827 | 5274 | 5271 | 17679 |
| 12166 | 7570 | 12383 | 21808 | 1823 | 12827 | 4948 | 28412 | 20573 | 6447 | 10398 | 29754 |
| 2593 | 12169 | 22416 | 9786 | 20892 | 13404 | 10956 | 26686 | 23523 | 1862 | 28326 | 11270 |
| 6370 | 10050 | 4775 | 10679 | 12680 | 12238 | 8325 | 28396 | 28460 | 28305 | 28275 | 14350 |
| 23907 | 23942 | 24700 | 24680 | 23939 | 24681 | 23864 | 23860 | 23903 | 23900 | 23946 | 23895 |
| 23865 | 23898 | 6157 | 6161 | 6127 | 6125 | 6097 | 26411 | 16809 | 21599 | 21596 | 16514 |
| 17229 | 16481 | 16511 | 16520 | 18720 | 21592 | 17854 | 3114 | 20041 | 30004 | 13964 | 17403 |
| 9102 | 9100 | 9127 | 9130 | 11353 | 9012 | 9009 | 9105 | 11331 | 11333 | 17600 | 21956 |
| 21951 | 21990 | 21848 | 21885 | 21948 | 21988 | 21925 | 21920 | 11111 | 12009 | 12006 | 9041 |
| 9037 | 9040 | 9162 | 9057 | 9106 | 9058 | 27335 | 9062 | 9097 | 15680 | 9145 | 8243 |
| 20985 | 20990 | 12546 | 22757 | 28913 | 26833 | 10359 | 12943 | 3970 | 9474 | 11358 | 12212 |
| 10387 | 12550 | 2243 | 2272 | 2281 | 2247 | 3002 | 2275 | 2251 | 2999 | 2280 | 3007 |
| 2244 | 8225 | 2240 | 17601 | 11662 | 17602 | 17531 | 17569 | 17557 | 17565 | 17530 | 4653 |

(continued)

SEQ ID NO: homolog SEQ ID NOs

| | | | | | | | | | | | |
|---|---|---|---|---|---|---|---|---|---|---|---|
| 15581 | 4649 | 28294 | 29235 | 28530 | 29234 | 28527 | 29232 | 2762 | 2910 | 4642 | 4646 |
| 4651 | 10700 | 11147 | 27994 | 13269 | 13313 | 13310 | 13273 | 13265 | 13307 | 13568 | 14718 |
| 14756 | 14714 | 14759 | 14722 | 5373 | 5376 | 14506 | 13876 | 13873 | 13794 | 13791 | 5281 |
| 11892 | 26963 | 26095 | 9327 | 16657 | 17051 | 26256 | 16104 | 6495 | 11913 | 26164 | 12636 |
| 9410 | 9325 | 27047 | 11306 | 9428 | 27025 | 26229 | 12579 | 9273 | 26198 | 16198 | 13829 |
| 13798 | 13796 | 13800 | 13910 | 13865 | 13839 | 13867 | 13869 | 5412 | 5402 | 13912 | 9887 |
| 9177 | 9182 | 5444 | 5446 | 5439 | 13934 | 5445 | 11356 | 18870 | 8086 | 23368 | 13938 |
| 5484 | 5450 | 12980 | 9874 | 28272 | 1044 | 18220 | 9179 | 22796 | 22838 | 9167 | 28468 |
| 9901 | 17685 | 22489 | 10008 | 25178 | 13630 | 8355 | 28250 | 5977 | 6445 | 27753 | 20387 |
| 7840 | 26949 | | | | | | | | | | |

659:

| | | | | | | | | | | | |
|---|---|---|---|---|---|---|---|---|---|---|---|
| 20824 | 11537 | 7640 | 21182 | 18149 | 28456 | 28497 | 26624 | 24677 | 24671 | 24673 | 14509 |
| 16545 | 21129 | 24999 | 4042 | 12876 | 23369 | 29198 | 24521 | 18910 | 24258 | 1399 | 23869 |
| 12284 | 9286 | 22188 | 22181 | 19832 | 19761 | 20788 | 14167 | 13083 | 13084 | 19080 | 19051 |
| 19047 | 19073 | 13088 | 19017 | 19014 | 9924 | 2821 | 29259 | 27367 | 2986 | 28422 | 28387 |
| 28382 | 27308 | 27267 | 29293 | 2736 | 2735 | 27511 | 27496 | 28516 | 27515 | 13109 | 16212 |
| 23959 | 25682 | 29055 | 3974 | 3206 | 22891 | 17516 | 2267 | 7034 | 7037 | 7074 | 7072 |
| 6933 | 6929 | 6967 | 6971 | 18063 | 6101 | 6098 | 6984 | 18347 | 10944 | 6330 | 12317 |
| 4287 | 17710 | 16585 | 21969 | 8137 | 12310 | 23333 | 5567 | 5282 | 2801 | 29160 | 1742 |
| 28578 | 28586 | 28690 | 28716 | 28720 | 28748 | 28616 | 28620 | 28648 | 28652 | 28828 | 28791 |
| 28821 | 28823 | 4098 | 29800 | 14254 | 14251 | 14255 | 14278 | 14274 | 14283 | 14281 | 14279 |
| 11736 | 4024 | 14891 | 15486 | 25770 | 24899 | 20453 | 12108 | 17558 | 16824 | 7770 | 10797 |
| 24448 | 7385 | 14312 | 19830 | 19803 | 29549 | 20364 | 20363 | 10965 | 18937 | 10618 | 4719 |
| 5778 | 23234 | 29175 | 15250 | 24332 | 16015 | 14180 | 20708 | 23853 | 28190 | 12026 | 8500 |
| 27926 | 29508 | 12698 | 21344 | 4981 | 14135 | 21334 | 19708 | 19717 | 19712 | 19755 | 19720 |
| 19754 | 19763 | 19767 | 19835 | 19839 | 30236 | 8982 | 5123 | 13554 | 12764 | 5543 | 14611 |
| 6786 | 9512 | 18372 | 6800 | 11169 | 19919 | 11247 | 10946 | 11470 | 1750 | 4907 | 13117 |
| 23068 | 6170 | 24535 | 3938 | 29024 | 4884 | 5978 | 20259 | 15213 | 11936 | 2230 | 2880 |
| 18778 | 26675 | 13603 | 24676 | 25024 | 13836 | 24146 | 25001 | 27777 | 16801 | 16079 | 18354 |
| 11975 | 29022 | 17198 | 1262 | 10683 | 4279 | 26134 | 23650 | 26620 | 9411 | 12463 | 20286 |
| 29354 | 27935 | 20960 | 5850 | 16922 | 22995 | 2764 | 12633 | 8938 | 19349 | 28494 | 10295 |
| 6703 | 20841 | 26656 | 27246 | 14398 | 22302 | 19218 | 30148 | 25944 | 28008 | 8867 | 25868 |
| 29195 | 23218 | 7922 | 6143 | 9904 | 18795 | 15083 | 2082 | 20826 | 7809 | 13409 | 23741 |
| 28492 | 9724 | 14821 | 29449 | 27615 | 29498 | 2730 | 2883 | 2980 | 27403 | 28357 | 27289 |

(continued)

SEQ ID NO: homolog SEQ ID NOs

| SEQ ID NO | | | | | | | | | | | |
|---|---|---|---|---|---|---|---|---|---|---|---|
| | 27467 | 27439 | 27293 | 2793 | 28426 | 29321 | 29317 | 2977 | 19079 | 6215 | 4726 | 10290 |
| | 6515 | 3809 | 25679 | 13566 | 19800 | 19797 | 19156 | 1963 | 23950 | 23954 | 14700 | 1156 |
| | 13732 | 29473 | 29348 | 27865 | 21185 | 16675 | 1606 | 29121 | 19021 | 21119 | 19081 | 21177 |
| | 21144 | 21151 | 21179 | 21206 | 21210 | 19789 | 19792 | 28080 | 1593 | 1591 | 18635 | 10269 |
| | 10340 | 22246 | 10301 | 10271 | 5257 | 10337 | 10308 | 10242 | 10240 | 10386 | 10343 | 10339 |
| | 5007 | 29846 | 21123 | 16570 | 24045 | 22036 | 8912 | 8365 | 18202 | 18781 | 2966 | 13668 |
| | 29970 | 27591 | 13760 | 26331 | 17588 | 9243 | 16140 | 14641 | 8942 | 16482 | 13781 | 7077 |
| | 7079 | 16561 | 16524 | 9502 | 16502 | 9126 | 28804 | 14501 | 4827 | 5274 | 5271 | 17679 |
| | 12166 | 7570 | 12383 | 21808 | 1823 | 12827 | 4948 | 28412 | 20573 | 6447 | 10398 | 29754 |
| | 2593 | 12169 | 22416 | 9786 | 20892 | 13404 | 10956 | 26686 | 23523 | 1862 | 28326 | 11270 |
| | 6370 | 10050 | 4775 | 10679 | 12680 | 12238 | 8325 | 28396 | 28460 | 28305 | 28275 | 14350 |
| | 23907 | 23942 | 24700 | 24680 | 23939 | 24681 | 23864 | 23860 | 23903 | 23900 | 23946 | 23895 |
| | 23865 | 23898 | 6157 | 6161 | 6127 | 6125 | 6097 | 26411 | 16809 | 21599 | 21596 | 16514 |
| | 17229 | 16481 | 16511 | 16520 | 18720 | 21592 | 17854 | 3114 | 20041 | 30004 | 13964 | 17403 |
| | 9102 | 9100 | 9127 | 9130 | 11353 | 9012 | 9009 | 9105 | 11331 | 11333 | 17600 | 21956 |
| | 21951 | 21990 | 21848 | 21885 | 21948 | 21988 | 21925 | 21920 | 11111 | 12009 | 12006 | 9041 |
| | 9037 | 9040 | 9162 | 9057 | 9106 | 9058 | 27335 | 9062 | 9097 | 15680 | 9145 | 8243 |
| | 20985 | 20990 | 12546 | 22757 | 28913 | 26833 | 10359 | 12943 | 3970 | 9474 | 11358 | 12212 |
| | 10387 | 12550 | 2243 | 2272 | 2281 | 2247 | 3002 | 2275 | 2251 | 2999 | 2280 | 3007 |
| | 2244 | 8225 | 2240 | 17601 | 11662 | 17602 | 17531 | 17569 | 17557 | 17565 | 17530 | 4653 |
| | 15581 | 4649 | 28294 | 29235 | 28530 | 29234 | 28527 | 29232 | 2762 | 2910 | 4642 | 4646 |
| | 4651 | 10700 | 11147 | 27994 | 13269 | 13313 | 13310 | 13273 | 13265 | 13307 | 13568 | 14718 |
| | 14756 | 14714 | 14759 | 14722 | 5373 | 5376 | 14506 | 13876 | 13873 | 13794 | 13791 | 5281 |
| | 11892 | 26963 | 26095 | 9327 | 16657 | 17051 | 26256 | 16104 | 6495 | 11913 | 26164 | 12636 |
| | 9410 | 9325 | 27047 | 11306 | 9428 | 27025 | 26229 | 12579 | 9273 | 26198 | 16198 | 13829 |
| | 13798 | 13796 | 13800 | 13910 | 13865 | 13839 | 13867 | 13869 | 5412 | 5402 | 13912 | 9887 |
| | 9177 | 9182 | 5444 | 5446 | 5439 | 13934 | 5445 | 11356 | 18870 | 8086 | 23368 | 13938 |
| | 5484 | 5450 | 12980 | 9874 | 28272 | 1044 | 18220 | 9179 | 22796 | 22838 | 9167 | 28468 |
| | 9901 | 17685 | 22489 | 10008 | 25178 | 13630 | 8355 | 28250 | 5977 | 6445 | 27753 | 20387 |
| | 7840 | 26949 | | | | | | | | | | |
| 660: | 15467 | 15629 | 26076 | 15660 | 25235 | 14999 | 16646 | 12402 | 2486 | 12842 | 13220 | 27918 |
| | 9280 | 16038 | 23836 | 1694 | 8134 | 21136 | 8715 | 8745 | 3924 | 13211 | 14872 | 3173 |
| | 4561 | 28201 | 13320 | 22050 | 7206 | 1698 | 5834 | 21050 | 17662 | 3626 | 16411 | 22068 |

SEQ ID NO: homolog SEQ ID NOs

| | | | | | | | | | | | |
|---|---|---|---|---|---|---|---|---|---|---|---|
| 21167 | 10976 | 20029 | 2937 | 20386 | 29736 | 8205 | 26864 | 27964 | 9910 | 15826 | 24398 |
| 24062 | 11440 | 25571 | 21631 | 4219 | 13789 | 22361 | 19411 | 1199 | 9484 | 16812 | 2913 |
| 12527 | 3063 | 21729 | 29570 | 6578 | 9986 | 28739 | 28965 | 8817 | 20632 | 6964 | 14288 |
| 28161 | 20637 | 16420 | 28082 | 2825 | 16302 | 14152 | 18525 | 9289 | 24285 | 16395 | 21408 |
| 4209 | 21218 | 24966 | 23701 | 10488 | 24773 | 1530 | 21113 | 20131 | 18707 | 18061 | 16158 |
| 16992 | 8287 | 15409 | 18581 | 11717 | 27456 | 26308 | 9620 | 4275 | 6743 | 9911 | 21348 |
| 6834 | 5341 | 2270 | 9587 | 17013 | 17176 | 12950 | 9920 | 9641 | 24679 | 9643 | 4786 |
| 26008 | 27346 | 13915 | 8892 | 19553 | 2147 | 20050 | 25229 | 28628 | 1160 | 25771 | 24539 |
| 16589 | 5102 | 6346 | 1518 | 29283 | 28446 | 21135 | 24898 | 1514 | 27433 | 9136 | 5473 |
| 19947 | 15530 | 6227 | 22543 | 28189 | 27480 | 18928 | 29118 | 25563 | 1413 | 2925 | 15086 |
| 16831 | 23894 | 26988 | 28119 | 23843 | 22152 | 30194 | 3570 | 20348 | 11842 | 26276 | 8818 |
| 24486 | 18750 | 6129 | 26419 | 3051 | 3044 | 18999 | 19604 | 28677 | 17586 | 4842 | 16095 |
| 25761 | 21779 | 19760 | 5874 | 11123 | 16632 | 22962 | 24804 | 26085 | 15965 | 23637 | 21205 |
| 9955 | 20249 | 6658 | 19211 | 9278 | 2234 | 30153 | 19486 | 20002 | 26671 | 21759 | 29895 |
| 25398 | 7793 | 10710 | 1957 | 3656 | 22169 | 9655 | 2763 | 5776 | 22030 | 2535 | 3834 |
| 22881 | 25882 | 4469 | 3036 | 25342 | 1106 | 15984 | 13755 | 29919 | 15522 | 5235 | 12774 |
| 18511 | 5294 | 20143 | 28335 | 1385 | 26891 | 17847 | 23584 | 3357 | 1308 | 6566 | 4694 |
| 21731 | 18560 | 10532 | 24280 | | | | | | | | |

661:
| | | | | | | | | | | | |
|---|---|---|---|---|---|---|---|---|---|---|---|
| 20744 | 4214 | 15408 | 12617 | 27910 | 6256 | 6679 | 29745 | 27794 | 8251 | 23191 | 26321 |
| 6507 | 29427 | 12808 | 1269 | 968 | 4381 | 26605 | 23626 | | | | |

662:
| | | | | | | | | | | | |
|---|---|---|---|---|---|---|---|---|---|---|---|
| 10235 | 16184 | 7924 | 3619 | 17709 | 29173 | 11974 | 18264 | 6980 | 18697 | 10152 | 5864 |
| 3997 | 6764 | 14862 | 16404 | 20184 | 20115 | 9437 | 7901 | 6987 | 2024 | 6938 | 28137 |
| 27034 | 27032 | 27060 | 7175 | 4905 | 12893 | 16394 | 5357 | 1504 | 8453 | 6524 | 20195 |
| 2096 | 5651 | 16786 | 16849 | 17640 | 2007 | 3779 | 15773 | 19185 | 15153 | 27691 | 14218 |
| 29183 | 30254 | 12093 | 17063 | 21246 | 13687 | 21131 | 6986 | 2997 | 15777 | 1256 | 26626 |
| 22892 | 20837 | 13976 | 16448 | 16443 | 23551 | 11823 | 25199 | 12147 | 12914 | 20392 | 12792 |
| 15675 | 17653 | 1864 | 21672 | 13871 | 27527 | 25907 | 29168 | 25353 | 25355 | 15554 | 21733 |
| 27573 | 16697 | 15127 | 17950 | 24705 | 9556 | 18988 | 28513 | 1249 | 3152 | 3607 | 11017 |
| 6892 | 6748 | 12643 | 22323 | 23203 | 21034 | 26237 | 22687 | 27925 | 9387 | 20804 | 11021 |
| 9647 | 11023 | 22316 | 13667 | 9356 | 27762 | 27627 | 5168 | 5798 | 4639 | 5799 | 4715 |
| 3349 | 13599 | 29346 | 14305 | 8903 | 7465 | 9174 | 7495 | 25403 | 27092 | 23085 | 7679 |
| 26326 | 27360 | 9077 | 22108 | 21859 | 28862 | 5139 | 6430 | 3209 | 8707 | 24202 | 29614 |
| 4926 | 25698 | 23687 | 7451 | 29137 | 23642 | 29540 | 5603 | 12578 | 4456 | 9330 | 4892 |

(continued)

SEQ ID NO: homolog SEQ ID NOs

| | | | | | | | | | | | | |
|---|---|---|---|---|---|---|---|---|---|---|---|---|
| | 5878 | 29353 | 5835 | 21947 | 26690 | 5167 | 25318 | 7480 | 27376 | 5191 | 20906 | 9210 |
| 663: | 7266 | 11841 | 4389 | 5389 | 10243 | 10694 | 10947 | 24725 | 27295 | 20276 | 5583 | 14940 |
| | 11152 | 28271 | 15808 | 8549 | 15811 | 11163 | 21093 | 11501 | 27871 | 10881 | 11580 | 22667 |
| | 23139 | 4913 | 30162 | | | | | | | | | |
| 664: | 20917 | 4189 | 21653 | 22157 | 18860 | 1332 | 20061 | 17183 | 6282 | 7589 | 26646 | 7045 |
| | 4865 | 10218 | 26758 | 2613 | 25466 | 21713 | 24699 | 13883 | 30132 | | | |
| 665: | 25164 | 25180 | 4579 | 20650 | 6833 | 2702 | 7743 | 16123 | 2805 | 26505 | 5956 | 14107 |
| | 3845 | 24336 | 7866 | 28895 | 26077 | 15863 | 30145 | 8479 | 4510 | 23251 | 20479 | 26491 |
| | 19168 | 2102 | 2437 | 26121 | 12789 | 28116 | 16806 | 16445 | 18412 | 1985 | 8391 | 13788 |
| | 18830 | 17475 | 10049 | 11386 | 21116 | 21805 | 16128 | 20419 | 23968 | 24314 | 9990 | |
| 666: | 29856 | 20063 | 14129 | 14704 | 10666 | 24299 | 14906 | 8888 | 6826 | 9880 | 17994 | 13821 |
| | 22315 | 8652 | 14259 | 8729 | 4231 | 17049 | 6878 | 6358 | 20096 | 3718 | 26293 | 26294 |
| | 26273 | 26274 | 26272 | 16756 | 22451 | 12832 | 25747 | 24042 | 17576 | 14586 | 16233 | 1434 |
| | 3661 | 19175 | 745 | 24779 | 2292 | 26746 | | | | | | |
| 667: | 25425 | 12423 | 12414 | 25080 | 26514 | 27682 | 2351 | 4679 | 12118 | 4616 | 23595 | 21876 |
| 668: | 6663 | 12456 | 24364 | 1901 | 26649 | 1488 | 8027 | 2894 | 3697 | 4607 | 9271 | 5522 |
| | 5368 | 5370 | 27068 | 27713 | 22861 | 13252 | 27397 | 8702 | 5454 | 5452 | 16474 | 7562 |
| | 849 | 10001 | 22287 | 26267 | 28992 | 16053 | 22134 | 9082 | 11214 | 15371 | 13362 | 2146 |
| | 15843 | 9731 | 7489 | 3086 | 8183 | 25016 | 22927 | 3962 | 20745 | 21616 | 4301 | 8582 |
| | 26876 | 23456 | 23514 | 21774 | 1543 | 4721 | 25169 | 29883 | 27175 | 27787 | 24240 | 9529 |
| | 9607 | 16147 | 17255 | 21634 | 21787 | 8207 | 4751 | 19445 | 10901 | 16252 | 29743 | 12426 |
| | 19725 | 22035 | 26079 | 17609 | 28916 | 4863 | 13920 | 12229 | 13922 | 4226 | 4223 | 20045 |
| | 28937 | 18973 | 22133 | 24056 | 5650 | 18957 | 19348 | 19365 | 10344 | 20663 | 16784 | 10971 |
| | 6489 | 24990 | 4041 | 26320 | 23555 | 4079 | 7540 | 4691 | 13315 | 27857 | 29512 | 16924 |
| | 15521 | 19500 | 10974 | 27343 | 29384 | 13245 | 6804 | 24189 | 4182 | 28966 | 28455 | 12635 |
| | 12063 | 1062 | 28613 | 28270 | 15309 | 12264 | 21433 | 7517 | 18575 | 13437 | 14457 | 18428 |
| | 28622 | 28134 | 14995 | 27995 | 28973 | 9917 | 25255 | 12088 | 29784 | 14962 | 3599 | 2649 |
| | 9423 | 12717 | 18274 | 9204 | 15768 | 9907 | 27117 | 19334 | 2422 | 19484 | 24414 | 29389 |
| | 20361 | 25210 | 14264 | 22639 | 9203 | 10405 | 8718 | 24846 | 3096 | 12214 | 2714 | 6818 |
| | 27491 | 5136 | 12482 | 14859 | 26094 | 9253 | 11213 | 26243 | 14500 | 13608 | 6134 | 30019 |
| | 16941 | 18032 | 28933 | 10847 | 13546 | 25440 | 13048 | 8884 | 21919 | 4847 | 28114 | 24636 |
| | 6302 | 24321 | 23450 | 26805 | 28167 | 23170 | 3260 | 14795 | 27870 | 17867 | 2171 | 25319 |
| | 9230 | 12567 | 11228 | 7955 | 24759 | 3442 | 1478 | 29718 | 10725 | 16497 | 993 | 29643 |

(continued)

SEQ ID NO: homolog SEQ ID NOs

| | | | | | | | | | | | | |
|---|---|---|---|---|---|---|---|---|---|---|---|---|
| | 24603 | 24670 | 15156 | 19162 | 19379 | 29358 | 19737 | 902 | 19609 | 19635 | 19632 | 10717 |
| | 12557 | 17204 | 27273 | 27644 | 23109 | 23108 | 20312 | 20311 | 15404 | 15284 | 15274 | 15384 |
| | 15343 | 15380 | 15279 | 15252 | 15345 | 15342 | 15339 | 15308 | 15402 | 15376 | 20550 | 9242 |
| | 17202 | 22019 | 17206 | 21195 | 27342 | 27324 | 27317 | 27315 | 13140 | 5384 | 24069 | 15603 |
| | 21432 | 12282 | 29178 | 13106 | 4460 | 20861 | 1353 | 18577 | 16964 | 20918 | 27181 | 13535 |
| | 29823 | 25354 | 5166 | 29100 | 26911 | 26885 | 6046 | 10166 | 19004 | 7347 | 11138 | 10898 |
| | 13137 | 15398 | 2407 | 2571 | 27254 | 28001 | 4519 | 28684 | 28714 | 16997 | 9422 | 6715 |
| | 1168 | 16329 | 2509 | 26597 | 27349 | 26617 | 26622 | 10170 | 18120 | 13541 | 16622 | 10187 |
| | 16122 | 12942 | | | | | | | | | | |
| 669: | 20604 | 28812 | 1318 | 17230 | 19793 | 9004 | 21929 | 11232 | 24138 | 25680 | 20082 | 6868 |
| | 5242 | 18465 | 21543 | 7274 | 5591 | 6418 | 26265 | 19040 | 1432 | 16382 | 9855 | 10955 |
| | 12163 | 11663 | 25940 | 1317 | 10041 | 9390 | 24112 | 6647 | 23121 | 2812 | 13323 | 18457 |
| | 26338 | 3813 | 20903 | 18273 | 6095 | 7875 | 3517 | 29146 | 9021 | 25200 | 23266 | 26759 |
| | 28092 | 27424 | 14433 | 9875 | 13020 | 5304 | 17192 | 7606 | 8459 | 25794 | 29089 | 10554 |
| | 16388 | 10686 | 14668 | 12008 | 29036 | 6069 | 18925 | 4927 | 7110 | 9237 | 10696 | 3700 |
| | 1045 | 20865 | 11720 | 7145 | 7594 | 6154 | 5041 | 14411 | 24460 | 5601 | 13946 | 22495 |
| | 27128 | 26139 | 8374 | 9091 | 643 | 28151 | 29250 | 3262 | 21398 | 8637 | 19741 | 14275 |
| | 30031 | 15349 | 25435 | 14482 | 28570 | 14646 | 30113 | 21744 | 19292 | 3339 | 28295 | 16925 |
| | 28069 | 24022 | 28016 | 29943 | 3768 | 15062 | 24362 | 19409 | 17264 | 2489 | 26254 | 22523 |
| | 13758 | 10261 | 16761 | 23788 | 20496 | 1846 | 3781 | 24319 | 2932 | 12405 | 4910 | |
| 670: | 20604 | 28812 | 1318 | 17230 | 19793 | 9004 | 21929 | 11232 | 24138 | 25680 | 20082 | 6868 |
| | 5242 | 18465 | 21543 | 7274 | 5591 | 6418 | 26265 | 19040 | 1432 | 16382 | 9855 | 10955 |
| | 12163 | 11663 | 25940 | 1317 | 10041 | 9390 | 24112 | 6647 | 23121 | 2812 | 13323 | 18457 |
| | 26338 | 3813 | 20903 | 18273 | 6095 | 7875 | 3517 | 29146 | 9021 | 25200 | 23266 | 26759 |
| | 28092 | 27424 | 14433 | 9875 | 13020 | 5304 | 17192 | 7606 | 8459 | 25794 | 29089 | 10554 |
| | 16388 | 10686 | 14668 | 12008 | 29036 | 6069 | 18925 | 4927 | 7110 | 9237 | 10696 | 3700 |
| | 1045 | 20865 | 11720 | 7145 | 7594 | 6154 | 5041 | 14411 | 24460 | 5601 | 13946 | 22495 |
| | 27128 | 26139 | 8374 | 9091 | 643 | 28151 | 29250 | 3262 | 21398 | 8637 | 19741 | 14275 |
| | 30031 | 15349 | 25435 | 14482 | 28570 | 14646 | 30113 | 21744 | 19292 | 3339 | 28295 | 16925 |
| | 28069 | 24022 | 28016 | 29943 | 3768 | 15062 | 24362 | 19409 | 17264 | 2489 | 26254 | 22523 |
| | 13758 | 10261 | 16761 | 23788 | 20496 | 1846 | 3781 | 24319 | 2932 | 12405 | 4910 | |
| 671: | 25139 | 7688 | 19900 | 18350 | 21498 | 25854 | 5764 | 15382 | 25614 | 3759 | 13351 | 11628 |
| | 11601 | 22107 | 2080 | 24963 | 23133 | 15540 | 21365 | 8853 | 5315 | 6185 | 18089 | 18418 |

EP 2 540 831 A2

SEQ ID NO: homolog SEQ ID NOs

| SEQ ID NO | | | | | | | | | | | | |
|---|---|---|---|---|---|---|---|---|---|---|---|---|
| | 24698 | 2104 | 14006 | 26941 | 1486 | 20494 | 10429 | 1596 | 8209 | 881 | 18965 | |
| 672: | 16981 | 6273 | 29593 | 24346 | 10149 | 2420 | 21156 | 4779 | 8774 | 19643 | 17442 | 1869 |
| | 22150 | 4147 | 22994 | 12198 | 20876 | 29779 | 17664 | 11261 | 11318 | 25382 | 15278 | 28691 |
| | 13582 | 22749 | 17316 | 24573 | 5663 | 24584 | 9801 | 3467 | 4072 | 6696 | 29441 | 12964 |
| | 23449 | 3696 | 21931 | 5898 | 29639 | 6067 | 1595 | 28011 | 762 | 20879 | 11525 | 16278 |
| | 26324 | 3451 | 2725 | 23165 | 11412 | 20201 | 24609 | 1679 | 1662 | | | |
| 673: | 16981 | 6273 | 29593 | 24346 | 10149 | 2420 | 21156 | 4779 | 8774 | 19643 | 17442 | 1869 |
| | 22150 | 4147 | 22994 | 12198 | 20876 | 29779 | 17664 | 11261 | 11318 | 25382 | 15278 | 28691 |
| | 13582 | 22749 | 17316 | 24573 | 5663 | 24584 | 9801 | 3467 | 4072 | 6696 | 29441 | 12964 |
| | 23449 | 3696 | 21931 | 5898 | 29639 | 6067 | 1595 | 28011 | 762 | 20879 | 11525 | 16278 |
| | 26324 | 3451 | 2725 | 23165 | 11412 | 20201 | 24609 | 1679 | 1662 | | | |
| 674: | 28741 | 22564 | 992 | 16225 | 11391 | 10645 | 27797 | 29103 | 26776 | 13184 | 26775 | 29099 |
| | 27771 | 8005 | 20461 | 963 | 7249 | 7221 | 19261 | 13136 | 17089 | 24357 | 18084 | 12371 |
| | 7753 | 14949 | 14945 | 17780 | 24284 | 17462 | 13254 | 24279 | 3406 | 3115 | 15184 | 14824 |
| | 23461 | 7005 | 5472 | 26798 | 26615 | 26613 | 14750 | 26594 | 14066 | 29350 | 29782 | 29347 |
| | 3375 | 2049 | 8240 | 3350 | 3346 | 15825 | 1387 | 17369 | 21455 | 14671 | 2016 | 17181 |
| | 16415 | 21984 | 26916 | 29900 | 13636 | 18946 | 4411 | 3789 | 18948 | 14895 | 999 | 4036 |
| | 15287 | 29647 | 3281 | 29335 | 10197 | 29324 | 13529 | 29879 | 24756 | 29511 | 8284 | 22572 |
| | 27992 | 10267 | 29014 | 22971 | 7634 | 5049 | 21400 | 10328 | 26581 | 24580 | 6643 | 13257 |
| | 3344 | 19223 | 1588 | 19219 | 16710 | 21322 | 21333 | 25316 | 5058 | 11750 | 24371 | 20017 |
| | 20060 | 7393 | 877 | 20021 | 20019 | 9768 | 22309 | 9185 | 17684 | 16865 | 1146 | 19217 |
| | 27050 | 8314 | 20343 | 14268 | 17200 | 4577 | 9250 | 9277 | 9276 | 19968 | 9279 | 9247 |
| | 9249 | 25860 | 1257 | 7039 | 22785 | 22722 | 22827 | 22822 | 22856 | 19576 | 11148 | 11151 |
| | 11472 | 25856 | 6673 | 26530 | 6385 | 19796 | 3684 | 14360 | 17844 | 11060 | 19715 | 13537 |
| | 25841 | 20882 | 1623 | 20418 | 25220 | 20223 | 7486 | 5113 | 13898 | 9340 | 9015 | 24404 |
| | 25647 | 20601 | 10486 | 14887 | 22055 | 20272 | 3676 | 26912 | 9524 | 25231 | 14000 | 24620 |
| | 7218 | 24612 | 29575 | 15316 | 10868 | 8985 | 4925 | 28206 | 4528 | 5606 | 7893 | 25129 |
| | 7658 | 4452 | 9830 | 15950 | 2771 | 19192 | 28452 | 26098 | 13064 | 20668 | 20736 | 4559 |
| | 5296 | 20992 | 4556 | 7466 | 2395 | 5790 | 8957 | 26933 | 18991 | 15405 | 12397 | 12393 |
| | 6027 | 18591 | 22774 | 17429 | 7839 | 28420 | 14745 | 14784 | 20483 | 1348 | 26440 | 25605 |
| | 16432 | 16429 | 28453 | 28939 | 21139 | 4448 | 21742 | 27108 | 3446 | 26800 | 1422 | 1424 |
| | 1418 | 11983 | 1426 | 23511 | 13439 | 20123 | 12801 | 12803 | 28520 | 17083 | 8673 | 21355 |
| | 17979 | 4725 | 11754 | 11756 | 12761 | 22299 | 21299 | 16650 | 12250 | 23185 | 28130 | 28705 |

SEQ ID NO: homolog SEQ ID NOs

| | | | | | | | | | | | | |
|---|---|---|---|---|---|---|---|---|---|---|---|---|
| | 24215 | 6908 | 30005 | 15520 | 1726 | 20075 | 13574 | 20236 | 17667 | 24494 | 28177 | 2402 |
| | 20134 | 6304 | 5336 | 2493 | 5752 | 26354 | 7771 | 17160 | 20093 | 24412 | 28063 | 6468 |
| | 14979 | 21428 | 8680 | 22800 | 7265 | 12768 | 19928 | 24526 | 15867 | 15865 | 20976 | 4873 |
| | 1804 | 7341 | 19898 | 19923 | 24828 | 7399 | 7439 | 6061 | 24067 | 17833 | 2068 | 22654 |
| | 29230 | 17201 | 6777 | 9153 | 12785 | 23649 | 12037 | 9937 | 6553 | 19042 | 2490 | 20136 |
| | 15370 | 8467 | 26403 | 7788 | 24613 | 12985 | 19236 | 18927 | 27606 | 16994 | 19569 | 28595 |
| | 23184 | 17799 | 4835 | 28674 | 26596 | 29574 | 28187 | 30079 | 10590 | 14004 | 24288 | 21502 |
| | 27819 | 7447 | 13432 | 23515 | 8421 | 2312 | 3704 | 7769 | 4336 | 12341 | 20227 | 9960 |
| | 3338 | 15450 | 22787 | 16076 | 2639 | 8119 | 2031 | 25808 | 23302 | 15901 | 19065 | 16118 |
| | 21426 | 19869 | 12666 | 16458 | 22824 | 8343 | 21677 | 2184 | 23986 | 18353 | 26175 | 11885 |
| | 11226 | 26486 | 3187 | 18464 | 15930 | 24001 | 5660 | 26296 | 11881 | 15772 | 27157 | 22748 |
| | 11718 | 17782 | 22830 | 23766 | 25519 | 2091 | 16837 | 12158 | 16296 | 19299 | 25346 | 6184 |
| | 12129 | 22953 | 25428 | 30000 | 9121 | 14206 | 17850 | 6709 | 26307 | 720 | 14333 | 23615 |
| | 15966 | 29035 | 1770 | 18660 | 12770 | 22114 | 16168 | 11064 | 28634 | 18320 | 21842 | 1392 |
| | 16779 | 11014 | 27840 | 27299 | 22371 | 28091 | 14463 | 11056 | 3899 | 1921 | 29047 | 8573 |
| | 18829 | 28712 | 10860 | 20432 | 16218 | 14755 | 17435 | 3929 | 3061 | 5272 | 22865 | 13525 |
| | 11783 | 28022 | 19194 | 14730 | 28196 | 22923 | 2859 | 1496 | 24983 | 15485 | 8996 | 11079 |
| | 24982 | 12603 | 23166 | 8551 | 15836 | 22958 | 30268 | 9977 | 10746 | 10750 | 10743 | 9966 |
| | 5932 | 5857 | 5922 | 19075 | 1047 | 10986 | 11871 | 26231 | 17286 | 13059 | 6655 | 15483 |
| | 3241 | 15333 | 13046 | 17568 | 22886 | 21897 | 1961 | 29728 | 16833 | 27745 | 23111 | 24444 |
| | 19241 | 27379 | 3362 | 16681 | 5726 | 18552 | 29797 | 2085 | 26102 | 13536 | 16713 | 11815 |
| | 21219 | 24566 | 30323 | 26575 | 28870 | 16010 | 3567 | 22628 | 2411 | 18704 | 26424 | 6799 |
| | 4433 | 4218 | 9358 | 14160 | 4671 | 28257 | 8813 | 13555 | 25247 | 23252 | 22307 | 7461 |
| | 26873 | 7684 | 20714 | 14319 | 7599 | 10230 | 12886 | 20962 | 15268 | 24597 | 15372 | 27859 |
| | 7299 | 21309 | 16273 | 8934 | 8427 | 17354 | 15155 | 25315 | 16326 | 19561 | 5668 | 10755 |
| | 15373 | 3994 | 17086 | 5919 | 21789 | 3730 | 22844 | 18020 | 30261 | 22594 | 5224 | 29945 |
| | 1085 | 7294 | 11643 | 25781 | 3354 | 15915 | 15917 | 10317 | 9870 | 9865 | 9895 | 9940 |
| | 9873 | 9897 | 9923 | 9935 | 9905 | 9930 | 9926 | 5048 | 5849 | 5844 | 5814 | 21074 |
| | 23919 | | | | | | | | | | | |
| 675: | 24485 | 2052 | 847 | 23886 | 16593 | 16547 | 30191 | 11230 | 20289 | 3655 | 18613 | 25381 |
| | 20685 | 20956 | 18956 | 737 | 28641 | 10531 | 10776 | 8129 | 1933 | 8529 | 1935 | 8037 |
| | 27961 | 27901 | 12971 | 15321 | 4097 | 12724 | 29006 | 13561 | 6844 | 11035 | 14600 | 21397 |
| | 9755 | 15238 | 21012 | 2124 | 19827 | 20243 | 10643 | 14900 | 19070 | 2189 | 23028 | 3332 |

SEQ ID NO: homolog SEQ ID NOs

| SEQ ID NO: | | | | | | | | | | | | |
|---|---|---|---|---|---|---|---|---|---|---|---|---|
| | 4384 | 26815 | 9590 | 9521 | 10886 | 3853 | 10892 | 10883 | 10631 | 13731 | 4078 | 951 |
| | 14660 | 6960 | 16679 | 13284 | 28482 | 13281 | 13280 | 27546 | 26493 | 20957 | 2167 | 23556 |
| | 28908 | 21918 | 18586 | 2242 | 29170 | 12111 | 2904 | 8318 | 22641 | 13276 | 15082 | 15040 |
| | 4380 | 5537 | 1965 | 18663 | 21448 | 11669 | 8328 | 1378 | 7114 | 15999 | 22931 | 2916 |
| | 4323 | 5405 | 27818 | 28155 | 27202 | 14295 | 14296 | 3673 | 27472 | 7657 | 18941 | 5865 |
| | 12332 | 28960 | 18516 | 12544 | 29622 | 15800 | 23945 | 18172 | 6618 | 29016 | 29018 | 29172 |
| | 12461 | 12485 | 8684 | 26661 | 5380 | 5381 | 5377 | 5375 | 7272 | 5378 | 14725 | 21490 |
| | 21141 | 14729 | 14731 | 12641 | 14719 | 7058 | 4026 | 14763 | 27286 | 14767 | 14769 | 14802 |
| | 10455 | 14772 | 12645 | 6068 | 12642 | 10434 | 7804 | 15369 | 12454 | 27348 | 22378 | 23817 |
| | 930 | 3825 | 23065 | 15196 | 25279 | 10158 | | | | | | |
| 676: | 21155 | 22072 | 3268 | 16405 | 4154 | 24734 | 20528 | 16156 | 29674 | 22893 | 3742 | 1298 |
| | 30038 | 1579 | 18425 | 866 | 27184 | 9688 | 4478 | 23695 | 19150 | 17991 | 13705 | |
| 677: | 3787 | 5954 | 15152 | 20119 | 11100 | 5209 | 13144 | 29290 | 5713 | 8199 | 27409 | 25372 |
| | 26313 | 29365 | 28255 | 5255 | 7336 | 23216 | 10756 | 3301 | 19521 | 27646 | 28789 | 11166 |
| | 20389 | 1783 | 15327 | 27476 | 27930 | 6609 | 9087 | 23221 | 1874 | 12788 | 22230 | 6123 |
| | 14091 | 26784 | 22648 | 5770 | 27931 | 6793 | 2364 | 19492 | 10161 | 12293 | 16025 | 18228 |
| | 3546 | 10257 | 25597 | 28365 | 25495 | 23394 | 19341 | 23171 | 4516 | 27554 | 12178 | 5508 |
| | 11725 | 27183 | 5837 | 9397 | 27552 | 27014 | 24330 | 24988 | | | | |
| 678: | 25377 | 18083 | 2537 | 27170 | 26858 | 6212 | 24222 | 28048 | 724 | 29975 | 19384 | 24918 |
| | 24518 | 21869 | 24517 | 29361 | 4977 | 3533 | 19064 | 29224 | 28535 | | | |
| 679: | 26546 | 27408 | 16154 | 16310 | 21570 | 29833 | 6948 | 18958 | 16882 | 5004 | 23034 | 12537 |
| | 4993 | 18336 | 21073 | | | | | | | | | |
| 680: | 8236 | 15682 | 14633 | 22404 | 25530 | 3392 | 1375 | 27113 | 8390 | 20470 | 19728 | 20846 |
| | 26699 | 7963 | 29126 | 1072 | 4761 | 29816 | 30118 | 7183 | 7683 | 12566 | 22760 | 26540 |
| | 6642 | 24772 | 26301 | 5203 | 19637 | 24004 | | | | | | |
| 681: | 21701 | 18510 | 1548 | 29712 | 26631 | 8415 | 16616 | 23052 | 27815 | 26926 | 28977 | 27432 |
| | 16872 | 25351 | 23138 | 14277 | 17483 | 15024 | 26838 | 13194 | 18840 | 2330 | 25774 | 16705 |
| | 1182 | 15727 | 15774 | 26053 | 7523 | 4964 | 3379 | 13072 | 24879 | 23454 | 16342 | 19091 |
| | 15654 | 15594 | 19563 | 6314 | 6031 | 12197 | 14422 | 2170 | 5862 | 1837 | 15401 | 12848 |
| | 10524 | 3647 | 4928 | 12979 | 14080 | 7902 | 23893 | 15357 | 2253 | 28764 | 9836 | 17448 |
| | 22423 | 25732 | 4304 | 5267 | 26089 | 26667 | 10998 | 15303 | 20356 | 21684 | 4484 | 2870 |
| | 4979 | 9833 | 4785 | 29773 | 29972 | 24360 | 15052 | 14635 | 15166 | 13908 | 1728 | 15288 |
| | 22616 | 777 | 18970 | 21849 | 7155 | 12697 | 11263 | 4198 | 21715 | 19456 | 16285 | 1988 |

(continued)

SEQ ID NO: homolog SEQ ID NOs

| | | | | | | | | | | | | |
|---|---|---|---|---|---|---|---|---|---|---|---|---|
| | 22063 | 22034 | 18969 | 25689 | 29483 | 15526 | 4011 | 4361 | 14443 | 9374 | 9849 | 10935 |
| | 29992 | 3429 | 17467 | 11415 | 8348 | 25155 | 27919 | 25807 | 24667 | 30299 | 17528 | 9707 |
| | 19002 | 21143 | 12267 | 27985 | 6617 | 19491 | 13023 | 23453 | 25663 | 21872 | 25625 | 7103 |
| | 4923 | 8379 | 8382 | 15645 | 9585 | 8528 | 3827 | 6726 | 19844 | 17540 | 11081 | 7365 |
| | 16936 | 18158 | 23674 | 13468 | 29466 | 4878 | 28041 | 11106 | 12607 | 16897 | 10677 | 19159 |
| | 7965 | 9108 | 7877 | 27196 | 986 | 24166 | 28687 | 11999 | 18008 | 24087 | 23655 | 26369 |
| | 16374 | 22147 | 23267 | 15122 | 21057 | 25893 | 3822 | 12783 | 9572 | 18322 | 18299 | 12196 |
| | 22032 | 4058 | 23411 | 14045 | 7262 | 23834 | 19530 | 23838 | 2769 | 25596 | 10483 | 17853 |
| | 23268 | 16660 | 10347 | 21866 | 22140 | 2601 | 25477 | 29711 | 7828 | 28077 | 13145 | 24786 |
| | 8297 | 28129 | 21584 | 28423 | 7474 | 15769 | 18430 | 26470 | 25430 | 2917 | 22773 | 9564 |
| | 30288 | 3762 | 1793 | 5750 | 9020 | 11588 | 14204 | 2869 | 18491 | 15294 | 6021 | 11576 |
| | 10275 | 26688 | 14462 | 2338 | 1314 | 23882 | 23444 | 15998 | 12069 | 13718 | 29689 | 2774 |
| | 19063 | 17059 | 18092 | 22445 | 26534 | 25543 | 27666 | 13559 | 11563 | 7371 | 22951 | 9565 |
| | 20189 | 11761 | 27686 | 12948 | 6966 | 2439 | 3283 | 11868 | 28827 | 22883 | 23425 | 22596 |
| | 5187 | 28278 | 30195 | 6565 | 29120 | 21111 | 25982 | 3378 | 12453 | 29477 | 17654 | 7140 |
| | 19970 | 10346 | 16614 | 28702 | 27031 | 12032 | 9233 | 13420 | 15477 | 20654 | 16736 | 20946 |
| | 27301 | 1403 | 17599 | 11022 | 15532 | 945 | 26135 | 21071 | 14875 | 21007 | 19036 | 14476 |
| | 8785 | 2511 | 26196 | 19730 | 23606 | 24948 | 17330 | 23841 | 3006 | 6867 | 22266 | 7075 |
| | 20603 | 11541 | 5426 | 11424 | 17266 | 3387 | 11922 | 29628 | 12572 | 3095 | 29841 | 1559 |
| | 24005 | 5208 | 13126 | 19693 | 24943 | 23629 | 28638 | 25227 | 4142 | 29887 | 1942 | 14618 |
| | 18342 | 19265 | 19504 | 8755 | 19467 | 5068 | 11095 | 25244 | 13503 | 17188 | 1796 | 11411 |
| | 21780 | 13866 | 29769 | 18912 | 22290 | 25674 | 27224 | 11122 | 28919 | 26812 | 12969 | 10740 |
| | 28430 | 20268 | 21271 | 6824 | 27842 | 6869 | 22515 | 20965 | 16360 | 7508 | 22478 | 16465 |
| | 9788 | 1369 | 8833 | 4838 | 11902 | 25408 | 18867 | 7427 | 9262 | 7182 | 17966 | 23225 |
| | 27417 | 3635 | 5076 | 11337 | 17691 | 6916 | 21100 | 1529 | 23601 | 29514 | 26790 | 6449 |
| | 13921 | 19976 | 15875 | 15403 | 23134 | | | | | | | |
| 682: | 13939 | 13012 | 7889 | 10237 | 14630 | 12078 | 10236 | 11250 | 22166 | 9800 | 30173 | 11546 |
| | 6819 | 2326 | 10617 | 4369 | 16237 | 28663 | 12040 | 4833 | 25226 | 4750 | 13466 | 12586 |
| | 20806 | 9664 | 16000 | 21414 | 10676 | 23458 | 26713 | 28339 | 23736 | 21852 | 28111 | 29592 |
| | 19600 | 6851 | 19397 | | | | | | | | | |
| 683: | 9117 | 25666 | 22993 | 14191 | 15383 | 27839 | 18792 | 9399 | 5069 | 6290 | 27280 | 22768 |
| | 28237 | 5135 | 4112 | 15739 | 21696 | 1412 | 25987 | 27973 | 30139 | 16136 | 2308 | 6162 |
| | 16602 | 8261 | 16592 | 7403 | | | | | | | | |

(continued)

SEQ ID NO: homolog SEQ ID NOs

| SEQ ID NO: | | | | | | | | | | | |
|---|---|---|---|---|---|---|---|---|---|---|---|
| 684: | 29110 | 3237 | 24034 | 11076 | 19009 | 4487 | 11707 | 13817 | 22420 | 24296 | 8085 | 7805 |
| | 16768 | 19153 | 5889 | 15715 | 28474 | 2594 | 12164 | 25378 | 1105 | 3194 | 17993 | 7925 |
| | 13127 | 18534 | 30075 | 6112 | 12462 | 10941 | | | | | | |
| 685: | 27976 | 20900 | 24274 | 11607 | 13523 | 17876 | 28519 | 16134 | 21368 | 29052 | 29631 | 25960 |
| | 27679 | 18909 | 22145 | 22974 | | | | | | | | |
| 686: | 1594 | 6640 | 3625 | 17508 | 29958 | 24248 | 21436 | 24041 | 25137 | 27822 | 18741 | 8741 |
| | 20320 | 24129 | 20874 | 18821 | 14499 | 24553 | 23576 | 6208 | 1977 | 25921 | 26985 | 25920 |
| | 29882 | 13975 | 17185 | 3330 | 6189 | 772 | 28603 | 14485 | 8417 | 7295 | 6686 | 17593 |
| | 7001 | 15902 | 21125 | 7473 | 12516 | 6742 | 20133 | 28976 | 23808 | 3949 | 7773 | 8765 |
| | 1853 | 28608 | 12734 | 9636 | 12534 | 25509 | 24897 | 16373 | 6608 | 13363 | 7017 | 9653 |
| | 6907 | 18036 | 4858 | 18538 | 1628 | 17140 | 6990 | 19666 | 4424 | 16434 | 16436 | 4446 |
| | 10899 | 22111 | 6827 | 7527 | 27579 | 746 | 12556 | 28729 | 3238 | 25714 | 28435 | 14265 |
| | 28437 | 23480 | 6591 | 14990 | 28672 | 8122 | 12819 | 22836 | 9716 | 12812 | 26024 | 23001 |
| | 18371 | 3188 | 8681 | 2567 | 28075 | 7880 | 9298 | 13517 | 21041 | 29465 | 28697 | 25755 |
| | 29668 | 15031 | 20121 | 19025 | 20891 | 22070 | 26068 | 26351 | 10745 | 15968 | 2747 | 17205 |
| | 16177 | 16205 | 19599 | 20820 | 21510 | 6301 | 16172 | 17484 | 18007 | 15059 | 11559 | 2355 |
| | 27462 | 23094 | 17656 | 13957 | 15074 | 22279 | 8793 | 21023 | 16996 | 15320 | 21608 | 18527 |
| | 14247 | 14250 | 14252 | 13751 | 29671 | 6190 | 5128 | 5099 | 6131 | 4278 | 26058 | 1327 |
| | 12796 | 13208 | 16031 | 14342 | 18820 | 24213 | 10826 | 15234 | 14907 | 13183 | 4792 | 3841 |
| | 8891 | 12723 | 10663 | 23492 | 22998 | 21855 | 23739 | 19590 | 20741 | 26599 | 25801 | 5973 |
| | 19702 | 8036 | 14233 | 3415 | 9476 | 15636 | 4551 | 29409 | 16368 | 16560 | 8446 | 8283 |
| | 7428 | 26964 | 26795 | 1087 | 22821 | 23840 | 8291 | 23802 | 26823 | 27770 | 24854 | 4401 |
| | 4661 | 8557 | 22717 | 29417 | 27510 | 10783 | 10821 | 9851 | 10827 | 14901 | 16035 | 11911 |
| | 10885 | 15585 | 25706 | 3480 | 25561 | 24469 | 2394 | 2369 | 23226 | 20224 | 19406 | 20926 |
| | 22248 | 1219 | 5018 | 28841 | 29641 | 29615 | 23909 | 21783 | 11347 | 13268 | 28667 | 28389 |
| | 5573 | 20341 | 26670 | 8469 | 4718 | 26678 | 943 | 9285 | 28421 | 18193 | 20062 | 14359 |
| | 5501 | 24682 | 28200 | 24380 | 25723 | 28786 | 946 | 27792 | 5619 | 24782 | 9527 | 4832 |
| | 12140 | 7856 | 10095 | 26660 | 15633 | 13757 | 6999 | 29424 | 15705 | 7069 | 25931 | 7239 |
| | 9984 | 17984 | 26685 | 23037 | 14327 | 11522 | 15197 | 18911 | 7476 | 27072 | 23153 | 22321 |
| | 10975 | 2014 | 7392 | 8190 | 23391 | 10330 | 2140 | 21656 | 25023 | 6538 | 6775 | 20110 |
| | 1884 | 16603 | 20055 | 9295 | 4880 | 11422 | 12581 | 16609 | 17788 | 17820 | 16951 | 17859 |
| | 11460 | 16535 | 17746 | 15511 | 12379 | 16332 | 15243 | 11677 | 24708 | 16461 | 19519 | 19483 |
| | 4974 | 8021 | 5125 | 10734 | 5117 | 24507 | 18286 | 21549 | 27422 | 26292 | 7372 | 28050 |

EP 2 540 831 A2

(continued)

SEQ ID NO: homolog SEQ ID NOs

| | | | | | | | | | | | |
|---|---|---|---|---|---|---|---|---|---|---|---|
| 20326 | 21388 | 28459 | 21382 | 6435 | 29322 | 29455 | 7053 | 26028 | 21374 | 25678 | 25514 |
| 1780 | 27086 | 26159 | 7021 | 3638 | 29450 | 5855 | 21987 | 19639 | 7936 | 26513 | 27667 |
| 28607 | 16559 | 13361 | 27038 | 23321 | 24389 | 3404 | 1288 | 26203 | 29097 | 18401 | 10298 |
| 10325 | 23573 | 1590 | 2829 | 8333 | 17626 | 13376 | 15470 | 26642 | 14614 | 22102 | 14667 |
| 28734 | 16397 | 7557 | 28568 | 20819 | 14783 | 28611 | 10589 | 27856 | 6896 | 6174 | 27655 |
| 25499 | 17960 | 26650 | 16322 | 16876 | 21189 | 2258 | 9894 | 14858 | 4192 | 14578 | 26004 |
| 4293 | 3102 | 24315 | 1261 | 9473 | 13481 | 8736 | 11184 | 27622 | 18389 | 21618 | 5106 |
| 8286 | 6357 | 28331 | 4813 | 2608 | 29781 | 2794 | 21838 | 18632 | 16771 | 17676 | 21841 |
| 27347 | 3179 | 2266 | 2229 | 4350 | 29249 | 21095 | 25952 | 14527 | 21098 | 19828 | 6331 |
| 6099 | 26921 | 5415 | 11847 | 12476 | 30292 | 15130 | 15015 | 15375 | 23818 | 19264 | 24935 |
| 7520 | 6335 | 16913 | 6480 | 16111 | 2499 | 16845 | 6397 | 8306 | 9209 | 7184 | 25863 |
| 4190 | 28902 | 21479 | 13354 | 29578 | 26339 | 9269 | 16581 | 29679 | 22803 | 29681 | 11216 |
| 18208 | 2417 | 2332 | 5856 | 20651 | 19496 | 17840 | 8266 | 11020 | | | |

687:

| | | | | | | | | | | | |
|---|---|---|---|---|---|---|---|---|---|---|---|
| 2604 | 16440 | 28953 | 25461 | 25897 | 15507 | 6197 | 8340 | 17043 | 23682 | 22638 | 24480 |
| 13626 | 6119 | 4946 | 16504 | 30110 | 8843 | 4968 | 8796 | 29076 | 1470 | 27827 | 22833 |
| 29474 | 4538 | 12295 | 12667 | 16814 | 18846 | 9886 | 1883 | 20103 | 4146 | 7860 | 5826 |
| 23000 | 21039 | 28671 | 10195 | 13998 | 8831 | 17985 | 11733 | 13949 | 26791 | 27058 | 5082 |
| 24719 | 13519 | 10474 | 7637 | 12128 | 18072 | 28753 | 18657 | 4340 | 14566 | 4553 | 28670 |
| 27611 | 9491 | 3691 | 23527 | 25710 | 10063 | 5088 | 3053 | 30056 | 25020 | 17161 | 14182 |
| 19840 | 1592 | 16075 | 12059 | 8633 | 8632 | 5024 | 16180 | 6913 | 12610 | 24478 | 14587 |
| 19444 | 30317 | 8341 | 7643 | 19574 | 2005 | 6899 | 2006 | 16002 | 16971 | 23978 | 16453 |
| 22551 | 3244 | 21215 | 3333 | 21430 | 6263 | 7673 | 18281 | 30208 | 16183 | 16186 | 19549 |
| 19655 | 15061 | 25059 | 13966 | 23698 | 13897 | 28109 | 17407 | 13518 | 8393 | 11584 | 22714 |
| 28030 | 22991 | 9261 | 4514 | 9532 | 11481 | 8713 | 11197 | 5749 | 8517 | 7642 | 22496 |
| 21497 | 28488 | 20519 | 22203 | 22742 | 23280 | 27966 | 18376 | 17548 | 4654 | 20993 | 11799 |
| 27968 | 24668 | 3786 | 18651 | 14131 | 7036 | 5060 | 4713 | 15577 | 20156 | 20748 | 7873 |
| 26169 | 29832 | 17480 | 17637 | 28180 | 2197 | 13279 | 9048 | 18494 | 13890 | 18747 | 18748 |
| 29468 | 771 | 2316 | 2318 | 12207 | 10555 | 19331 | 9239 | 27876 | 1525 | 9760 | 17965 |
| 18718 | 27812 | 15545 | 11249 | 7910 | 9642 | 7544 | 8320 | 3596 | 25550 | 23353 | 25915 |
| 30135 | 7139 | 24106 | 26971 | 22040 | 29418 | 26344 | 12205 | 27788 | 25603 | 6429 | 4170 |
| 28262 | 14123 | 1498 | 27533 | 11189 | 23336 | 14547 | 27406 | 18852 | 28887 | 11941 | 28944 |
| 16294 | 28575 | 8187 | 15462 | 880 | 12033 | 24809 | 28680 | 15038 | 20564 | 12151 | 17604 |
| 14469 | 8268 | 4092 | 29927 | 19056 | 7391 | 7921 | 16327 | 25831 | 26952 | 11003 | 9305 |

(continued)

SEQ ID NO: homolog SEQ ID NOs

| 688: | 18847 | 14880 | 15781 | 22847 | 9054 | 28850 | 12127 | 28263 | 10841 | 18201 | 1103 | 9487 |
| | 29559 | 23003 | | | | | | | | | | |
| 689: | 18847 | 14880 | 15781 | 22847 | 9054 | 28850 | 12127 | 28263 | 10841 | 18201 | 1103 | 9487 |
| | 29559 | 23003 | | | | | | | | | | |
| 690: | 20809 | 23473 | 4260 | 2435 | 14571 | 14040 | 1341 | 1362 | 7616 | 24869 | 22156 | 14261 |
| | 22799 | 22797 | 28954 | 28955 | 19909 | 16153 | 29753 | 19359 | 20812 | 2495 | 2494 | 23532 |
| | 23528 | 9706 | 20995 | 8062 | 24888 | 16367 | 29193 | 29214 | 15842 | 10820 | 13729 | 15100 |
| | 4853 | 4855 | 15598 | 23585 | 18283 | 18280 | 12575 | 13138 | 17521 | 29486 | 27054 | 27525 |
| | 24758 | 5813 | 7862 | 7865 | 6196 | 29067 | 24175 | 26761 | 8664 | 28243 | 28242 | 26521 |
| | 25481 | 20978 | 19680 | 20531 | 1749 | 7768 | 27015 | 15855 | 9348 | 12760 | 12758 | 26567 |
| | 15411 | 12779 | 16898 | 4138 | 4124 | 21591 | 27279 | 15028 | 12820 | 9222 | 15787 | 28803 |
| | 15426 | 12963 | 4938 | 9679 | 13341 | 7350 | 8711 | 9227 | 17114 | 17113 | 24145 | 25810 |
| | 25783 | 15856 | 21997 | 11731 | 11509 | 2896 | 18572 | 12470 | 12473 | 10565 | 10964 | 10963 |
| | 5958 | 3579 | 3580 | 8363 | 7994 | 3250 | 3248 | 24737 | 13507 | 13508 | 12210 | 12234 |
| | 2210 | 9019 | 9018 | 26277 | 26088 | 6158 | 14349 | 14347 | 27862 | 9985 | 16092 | 10908 |
| | 4967 | 4965 | 13956 | 29665 | 29667 | 21536 | 21538 | 3289 | 2948 | 2946 | 16071 | 21778 |
| | 21804 | 3805 | 26578 | 17839 | 27751 | 22282 | 24901 | 6889 | 4320 | 19133 | 26382 | 24516 |
| | 24514 | 14391 | 1589 | 14392 | 23928 | 1473 | 1744 | 27945 | 17137 | 3820 | 26222 | 26220 |
| | 18247 | 18248 | 3126 | 3129 | 1806 | 942 | 12638 | 2360 | 6508 | 29501 | 22244 | 22245 |
| | 11033 | 28336 | 11490 | 29009 | 21871 | 7676 | 20922 | 20919 | 18254 | 5085 | 24749 | 11877 |
| | 12660 | 12657 | 19984 | 27115 | 27116 | 24013 | 18598 | 18595 | 24399 | 19736 | 6721 | 6720 |
| | 19814 | 2442 | 1735 | 3877 | 23098 | 23063 | 13438 | 20073 | 20071 | 29315 | 29339 | 4025 |
| | 903 | 8562 | 26450 | 26446 | 13793 | 13792 | 20257 | 19234 | 24707 | 23598 | 27040 | 21895 |
| | 15793 | 11438 | 18437 | 22756 | 6348 | 29136 | 27019 | 27903 | 27895 | 21705 | 21703 | 9260 |
| | 17163 | 9259 | 17165 | 2225 | 15004 | 8533 | 25651 | 25648 | 12441 | 12437 | 18452 | 1041 |
| | 17757 | 5062 | 20091 | 10421 | 27145 | 26787 | 26785 | 12703 | 17537 | 24520 | 21758 | 19337 |
| | 19239 | 13648 | 3548 | 4220 | 20279 | 8271 | 3882 | 2531 | 19711 | 23427 | 1841 | 6494 |
| | 27874 | 10414 | 23167 | 7032 | 30131 | 18627 | 10335 | 24555 | 5147 | 4244 | 15379 | 5464 |
| | 18839 | 4239 | 28703 | 18181 | 13863 | 15559 | 4281 | 20755 | 30043 | 988 | 22236 | 5386 |
| | 9023 | 8999 | 9022 | 973 | 17884 | 6202 | 4666 | 17437 | 17466 | 3294 | 6255 | 16836 |
| | 20090 | 5915 | 16927 | 19582 | 23592 | 23590 | 11186 | 8454 | 8452 | 5036 | 20450 | 20448 |
| | 1826 | 21689 | 28836 | 18409 | 13659 | 2544 | 8965 | 23497 | 7501 | 25824 | 2265 | 9961 |
| | 11805 | 9603 | 9602 | 12742 | 14354 | 21999 | | | | | | |

(continued)

SEQ ID NO: homolog SEQ ID NOs

| 691: | 20809 | 23473 | 4260 | 2435 | 14571 | 14040 | 1341 | 1362 | 7616 | 24869 | 22156 | 14261 |
|------|-------|-------|------|------|-------|-------|------|------|------|-------|-------|-------|
| | 22799 | 22797 | 28954 | 28955 | 19909 | 16153 | 29753 | 19359 | 20812 | 2495 | 2494 | 23532 |
| | 23528 | 9706 | 20995 | 8062 | 24888 | 16367 | 29193 | 29214 | 15842 | 10820 | 13729 | 15100 |
| | 4853 | 4855 | 15598 | 23585 | 18283 | 18280 | 12575 | 13138 | 17521 | 29486 | 27054 | 27525 |
| | 24758 | 5813 | 7862 | 7865 | 6196 | 29067 | 24175 | 26761 | 8664 | 28243 | 28242 | 26521 |
| | 25481 | 20978 | 19680 | 20531 | 1749 | 7768 | 27015 | 15855 | 9348 | 12760 | 12758 | 26567 |
| | 15411 | 12779 | 16898 | 4138 | 4124 | 21591 | 27279 | 15028 | 12820 | 9222 | 15787 | 28803 |
| | 15426 | 12963 | 4938 | 9679 | 13341 | 7350 | 8711 | 9227 | 17114 | 17113 | 24145 | 25810 |
| | 25783 | 15856 | 21997 | 11731 | 11509 | 2896 | 18572 | 12470 | 12473 | 10565 | 10964 | 10963 |
| | 5958 | 3579 | 3580 | 8363 | 7994 | 3250 | 3248 | 24737 | 13507 | 13508 | 12210 | 12234 |
| | 2210 | 9019 | 9018 | 26277 | 26088 | 6158 | 14349 | 14347 | 27862 | 9985 | 16092 | 10908 |
| | 4967 | 4965 | 13956 | 29665 | 29667 | 21536 | 21538 | 3289 | 2948 | 2946 | 16071 | 21778 |
| | 21804 | 3805 | 26578 | 17839 | 27751 | 22282 | 24901 | 6889 | 4320 | 19133 | 26382 | 24516 |
| | 24514 | 14391 | 1589 | 14392 | 23928 | 1473 | 1744 | 27945 | 17137 | 3820 | 26222 | 26220 |
| | 18247 | 18248 | 3126 | 3129 | 1806 | 942 | 12638 | 2360 | 6508 | 29501 | 22244 | 22245 |
| | 11033 | 28336 | 11490 | 29009 | 21871 | 7676 | 20922 | 20919 | 18254 | 5085 | 24749 | 11877 |
| | 12660 | 12657 | 19984 | 27115 | 27116 | 24013 | 18598 | 18595 | 24399 | 19736 | 6721 | 6720 |
| | 19814 | 2442 | 1735 | 3877 | 23098 | 23063 | 13438 | 20073 | 20071 | 29315 | 29339 | 4025 |
| | 903 | 8562 | 26450 | 26446 | 13793 | 13792 | 20257 | 19234 | 24707 | 23598 | 27040 | 21895 |
| | 15793 | 11438 | 18437 | 22756 | 6348 | 29136 | 27019 | 27903 | 27895 | 21705 | 21703 | 9260 |
| | 17163 | 9259 | 17165 | 2225 | 15004 | 8533 | 25651 | 25648 | 12441 | 12437 | 18452 | 1041 |
| | 17757 | 5062 | 20091 | 10421 | 27145 | 26787 | 26785 | 12703 | 17537 | 24520 | 21758 | 19337 |
| | 19239 | 13648 | 3548 | 4220 | 20279 | 8271 | 3882 | 2531 | 19711 | 23427 | 1841 | 6494 |
| | 27874 | 10414 | 23167 | 7032 | 30131 | 18627 | 10335 | 24555 | 5147 | 4244 | 15379 | 5464 |
| | 18839 | 4239 | 28703 | 18181 | 13863 | 15559 | 4281 | 20755 | 30043 | 988 | 22236 | 5386 |
| | 9023 | 8999 | 9022 | 973 | 17884 | 6202 | 4666 | 17437 | 17466 | 3294 | 6255 | 16836 |
| | 20090 | 5915 | 16927 | 19582 | 23592 | 23590 | 11186 | 8454 | 8452 | 5036 | 20450 | 20448 |
| | 1826 | 21689 | 28836 | 18409 | 13659 | 2544 | 8965 | 23497 | 7501 | 25824 | 2265 | 9961 |
| | 11805 | 9603 | 9602 | 12742 | 14354 | 21999 | | | | | | |
| 692: | 17899 | 15098 | 17207 | 23332 | 24853 | 19352 | 16775 | 24491 | 1492 | 836 | 7705 | 19594 |
| | 24785 | 2030 | 27303 | 23007 | 4215 | | | | | | | |
| 693: | 17899 | 15098 | 17207 | 23332 | 24853 | 19352 | 16775 | 24491 | 1492 | 836 | 7705 | 19594 |
| | 24785 | 2030 | 27303 | 23007 | 4215 | | | | | | | |

(continued)

SEQ ID NO: homolog SEQ ID NOs

| 694: | 15467 | 25235 | 15660 | 26076 | 14999 | 23694 | 15629 | 16646 | 12402 | 2486 | 12842 | 13220 |
|---|---|---|---|---|---|---|---|---|---|---|---|---|
| | 20713 | 29868 | 27918 | 24147 | 24125 | 23836 | 16038 | 21136 | 8715 | 8745 | 13211 | 3924 |
| | 14872 | 3173 | 4561 | 28201 | 13320 | 22050 | 7206 | 1698 | 5834 | 21050 | 17662 | 3626 |
| | 16411 | 22068 | 14930 | 21167 | 10976 | 17418 | 15609 | 20029 | 2937 | 20386 | 29736 | 8205 |
| | 26864 | 27964 | 9910 | 15826 | 24398 | 24062 | 11440 | 25571 | 21631 | 22361 | 4219 | 13789 |
| | 3063 | 19411 | 1199 | 21729 | 28545 | 2913 | 9484 | 16812 | 12527 | 27841 | 5892 | 15505 |
| | 6578 | 18562 | 9986 | 28965 | 28739 | 8817 | 14288 | 20632 | 28161 | 6964 | 20637 | 16302 |
| | 2825 | 21408 | 28082 | 9289 | 16395 | 4209 | 14152 | 16420 | 24285 | 23701 | 13392 | 24773 |
| | 10488 | 1530 | 20131 | 21113 | 18061 | 18707 | 28425 | 29606 | 16158 | 16992 | 8287 | 15409 |
| | 18581 | 9620 | 26308 | 10520 | 21348 | 6743 | 4275 | 27456 | 11717 | 9911 | 5341 | 2270 |
| | 26302 | 6834 | 20305 | 2099 | 17013 | 9587 | 17176 | 12950 | 9920 | 9641 | 24679 | 9643 |
| | 4786 | 27346 | 13915 | 8892 | 19553 | 2147 | 20050 | 1160 | 25229 | 28628 | 25771 | 24539 |
| | 16589 | 5102 | 6346 | 1518 | 21135 | 29283 | 28446 | 1514 | 27433 | 9136 | 19947 | 5473 |
| | 15530 | 6227 | 22543 | 28189 | 27480 | 29118 | 18928 | 25563 | 1413 | 2925 | 15086 | 16831 |
| | 23894 | 10625 | 22152 | 4049 | 19351 | 28119 | 26988 | 23843 | 25079 | 30194 | 3570 | 20348 |
| | 21854 | 11842 | 4922 | 26276 | 8818 | 14645 | 24486 | 18750 | 6129 | 26419 | 3051 | 3044 |
| | 18999 | 19604 | 28677 | 17586 | 4842 | 16095 | 19760 | 5874 | 11123 | 27920 | 16632 | 22962 |
| | 24804 | 26085 | 15965 | 20313 | 23637 | 3212 | 17333 | 17273 | 17769 | 9955 | 6658 | 8578 |
| | 20533 | 4804 | 20536 | 2234 | 30153 | 19486 | 6641 | 16672 | 14300 | 17131 | 7793 | 10710 |
| | 9103 | 3656 | 5776 | 1957 | 9655 | 22030 | 2763 | 22169 | 3834 | 2535 | 22881 | 25882 |
| | 13925 | 29404 | 29536 | 4469 | 3036 | 25342 | 1106 | 15984 | 29919 | 13755 | 15522 | 27559 |
| | 5235 | 12774 | 18511 | 5294 | 9288 | 20143 | 28335 | 1385 | 26891 | 17847 | 23584 | 3357 |
| | 1308 | 6566 | 4694 | 18536 | 10573 | 17318 | 9034 | 7432 | | | | |
| 695: | 11980 | 14082 | 4274 | 8975 | 26312 | 5895 | 16054 | 8994 | 10642 | 2920 | 10673 | 24819 |
| | 5256 | 14746 | 26132 | 27773 | 26738 | 2057 | 8361 | 27727 | 29158 | 12713 | 16162 | 2370 |
| | 2559 | 7335 | 17912 | 17106 | 1658 | 969 | 9877 | 27941 | 10265 | 3098 | 17714 | 12811 |
| | 27372 | 10703 | 7803 | 13309 | 27251 | | | | | | | |
| 696: | 17986 | 10603 | 1992 | 20753 | 9225 | 11694 | 10771 | 1049 | 26695 | 22840 | 18688 | 22062 |
| | 2098 | 26855 | 19364 | 15504 | 13457 | 7595 | 8710 | 22907 | 6659 | 25536 | 6949 | 5073 |
| | 28926 | 6710 | 5679 | 27105 | 18959 | 24207 | 28521 | 28191 | 11782 | | | |
| 697: | 19954 | 19956 | 19958 | 21067 | 20881 | 20831 | 20905 | 21009 | 21069 | 23902 | 23906 | 23944 |
| | 23958 | 24000 | 21064 | 23993 | 20913 | 23908 | 20835 | 20836 | 20875 | 20880 | 20912 | 20940 |
| | 23949 | 19933 | 19848 | 21864 | 3727 | 6165 | 13784 | 13813 | 19222 | 19224 | 9157 | 23910 |

(continued)

SEQ ID NO: homolog SEQ ID NOs

|  | | | | | | | | | | | |
|---|---|---|---|---|---|---|---|---|---|---|---|
| 4890 | 22630 | 7124 | 14340 | 24513 | 25134 | 16506 | 25880 | 8771 | 4805 | 4802 | 4809 |
| 4845 | 4849 | 4846 | 4879 | 4844 | 8858 | 4812 | 26280 | 4744 | 29994 | 8655 | 1775 |
| 22855 | 1753 | 9160 | 2123 | 24185 | 8744 | 8045 | 5011 | 8751 | 30309 | 25985 | 3725 |
| 25737 | 11154 | 29664 | 12865 | 2021 | 4709 | 14923 | 25953 | 28448 | 8748 | 23666 | 6216 |
| 4912 | 22041 | 14393 | 19932 | 20972 | 29650 | 8324 | 15225 | 15512 | 14210 | 6374 | 7248 |
| 25340 | 15509 | 13815 | 22350 | 19753 | 25371 | 25981 | 25075 | 2065 | 23914 | 26017 | 29446 |
| 24473 | 24980 | 25878 | 25900 | 25950 | 28441 | 25984 | 26569 | 2083 | 4711 | 10220 | 23460 |
| 23457 | 1858 | 25896 | 14460 | 5218 | 20344 | 17075 | 24952 | 26552 | 26551 | 5045 | 28403 |
| 19870 | 5676 | 1391 | 3739 | 18027 | 25958 | 25105 | 25071 | 24987 | 26114 | 26105 | 20955 |
| 25895 | 25898 | 20005 | 25042 | 9915 | 29699 | 15608 | 18634 | 3056 | 14181 | 14246 | 12898 |
| 9184 | 21094 | 21099 | 21834 | 21827 | 13810 | 19938 | 15441 | 29481 | 26013 | 24981 | 723 |
| 26042 | 725 | 20335 | 26049 | 733 | 734 | 21142 | 18804 | 1568 | 6537 | 25011 | 27147 |
| 5725 | 5748 | 5728 | 5753 | 19861 | 10384 | 1507 | 14577 | 24985 | 25077 | 12894 | 26083 |
| 12923 | 30312 | 30311 | 24092 | 6114 | 28400 | 27513 | 28471 | 28413 | 28434 | 28409 | 28417 |
| 28467 | 19220 | 19525 | 22631 | 5722 | 26111 | 20433 | 20525 | 25376 | 25399 | 25954 | 25956 |
| 25012 | 5909 | 21033 | 26067 | 21831 | 21092 | 21002 | 19935 | 21028 | 21029 | 21062 | 19873 |
| 23996 | 4924 | 4921 | 25344 | 27772 | 6379 | 19965 | 20834 | 23899 | 21822 | 19912 | 20968 |
| 21006 | 21106 | 19875 | 23961 | 20975 | 10453 | 18952 | 10654 | 22577 | | | |
| 698: 2447 | 20300 | 1792 | 2137 | 18684 | 19744 | 7483 | 30172 | 3371 | 15533 | 15596 | 6534 |
| 15539 | 15558 | 15590 | 5623 | 15563 | 15595 | 15565 | 15564 | 15569 | 29980 | 18943 | 19556 |
| 22226 | 15904 | 5417 | 7225 | 10333 | 29038 | 17718 | 24932 | 5868 | 29495 | 29491 | 2686 |
| 12836 | 11894 | 6731 | 26560 | 4759 | 24127 | 15117 | 10796 | 14471 | 9095 | 21072 | 10542 |
| 27739 | 17896 | 11692 | 19758 | 7890 | 15761 | 21757 | 12754 | 18271 | 18268 | 15503 | 11791 |
| 2149 | 29932 | 7123 | 4110 | 8182 | 17092 | 29343 | 6035 | 9170 | 8904 | 27782 | 15393 |
| 4746 | 5572 | 2578 | 13597 | 5505 | 15650 | 16804 | 11078 | 23361 | 26156 | 5530 | 12882 |
| 7868 | 10766 | 1844 | 29397 | 16881 | 21486 | 5549 | 9548 | 27709 | 11554 | 7493 | 27394 |
| 26549 | 26526 | 5126 | 4001 | 14513 | 6236 | 29030 | 6086 | 6388 | 10324 | 18709 | 9318 |
| 28621 | 2587 | 2481 | 1485 | 11658 | 17026 | 4179 | 22699 | 2458 | 7230 | 4630 | 8458 |
| 16129 | 25280 | 28765 | 5625 | 5449 | 17478 | 12879 | 12564 | 24464 | 2431 | 12887 | 20954 |
| 11510 | 7166 | 23765 | 3329 | 6246 | 6970 | 28932 | 7296 | 18329 | 19874 | 10100 | 15051 |
| 22490 | 8422 | 7794 | 19310 | 15967 | 29652 | | | | | | |
| 699: 10864 | 3013 | 1367 | 28719 | 6626 | 21097 | 1688 | | | | | |
| 700: 27593 | 28017 | 27334 | 12565 | 1284 | 4233 | 4475 | 4495 | 6362 | 29092 | 11234 | 14291 |

(continued)

SEQ ID NO: homolog SEQ ID NOs

| SEQ ID NO: | | | | | | | | | | | | |
|---|---|---|---|---|---|---|---|---|---|---|---|---|
| | 25067 | 4783 | 25065 | 17410 | 14666 | 26829 | 5345 | 28447 | 19161 | 27189 | 20317 | 7220 |
| | 8574 | 2849 | 11108 | 9821 | 13688 | 19367 | 19368 | 19372 | 29351 | 1132 | 22370 | 22372 |
| | 4710 | 22110 | | | | | | | | | | |
| 701: | 19401 | 29408 | 23777 | 5250 | 20751 | 1123 | 22996 | 27879 | 12081 | 16380 | 20785 | 14624 |
| | 24496 | 4598 | 8031 | 7100 | 13940 | 11612 | 26143 | 27276 | 13217 | 9899 | 20680 | 24895 |
| | 20853 | 2741 | 2775 | 26357 | 9354 | 25046 | 26469 | 16760 | 17566 | 7948 | 29272 | 7895 |
| | 5914 | 5913 | 5912 | 11047 | 17069 | 17073 | 2073 | 12904 | 6957 | 23771 | 10953 | 14903 |
| | 30042 | 953 | 16196 | 14850 | 2222 | 15859 | 27028 | 1304 | 11488 | 15406 | 23373 | 29241 |
| 702: | 19401 | 29408 | 23777 | 5250 | 20751 | 1123 | 22996 | 27879 | 12081 | 16380 | 20785 | 14624 |
| | 24496 | 4598 | 8031 | 7100 | 13940 | 11612 | 26143 | 27276 | 13217 | 9899 | 20680 | 24895 |
| | 20853 | 2741 | 2775 | 26357 | 9354 | 25046 | 26469 | 16760 | 17566 | 7948 | 29272 | 7895 |
| | 5914 | 5913 | 5912 | 11047 | 17069 | 17073 | 2073 | 12904 | 6957 | 23771 | 10953 | 14903 |
| | 30042 | 953 | 16196 | 14850 | 2222 | 15859 | 27028 | 1304 | 11488 | 15406 | 23373 | 29241 |
| 703: | 20590 | 29620 | 15258 | 1433 | 30185 | 29039 | 19950 | 14318 | 21441 | 16914 | 21214 | 22405 |
| | 8188 | 26228 | 26213 | 16766 | 19127 | 987 | 12075 | 24302 | 18060 | 8273 | 12821 | 4315 |
| | 29544 | 24329 | 8740 | 26378 | 20620 | 23305 | 22472 | 730 | 22441 | 15904 | 3478 | 3508 |
| | 1991 | 11705 | 17718 | 9539 | 2518 | 12881 | 19518 | 3545 | 12747 | 10852 | 12776 | 11953 |
| | 18759 | 25623 | 18398 | 13291 | 10229 | 15216 | 26560 | 4840 | 27614 | 1410 | 28186 | 26073 |
| | 7749 | 29490 | 718 | 8330 | 11515 | 12399 | 22645 | 25333 | 3031 | 4723 | 23132 | 19240 |
| | 10090 | 4701 | 26755 | 20739 | 22805 | 15785 | 8717 | 10989 | 16788 | 10722 | 9458 | 4431 |
| | 13459 | 21161 | 21289 | 9005 | 12991 | 10401 | 12020 | 11909 | 17564 | 17277 | 20280 | 23172 |
| | 11692 | 13467 | 6473 | 15976 | 15088 | 3729 | 8821 | 23508 | 11667 | 18304 | 18122 | 7806 |
| | 28014 | 4225 | 18703 | 22403 | 24345 | 15269 | 16239 | 26281 | 14521 | 29709 | 16970 | 21993 |
| | 16666 | 16600 | 7903 | 26957 | 22439 | 18509 | 20455 | 27074 | 20994 | 17953 | 14845 | 2795 |
| | 5936 | 23874 | 732 | 17519 | 19195 | 14352 | 24653 | 25115 | 27803 | 29594 | 25713 | 22643 |
| | 4432 | 8066 | 11854 | 12721 | 18407 | 24497 | 19163 | 28614 | 23870 | 19757 | 15328 | 4693 |
| | 13206 | 9339 | 13373 | 5630 | 5736 | 2646 | 10515 | 13864 | 3314 | 30308 | 28199 | 21513 |
| | 9666 | 6094 | 1931 | 26832 | 7174 | 10931 | 1975 | 11086 | 5020 | 23112 | 10283 | 10138 |
| | 6455 | 13224 | 12541 | 16528 | 18130 | 18026 | 14199 | 9265 | 14827 | 9116 | 8456 | 25093 |
| | 20275 | 25320 | 20027 | 14620 | 18150 | 2209 | 4594 | 6463 | 29831 | 4082 | 17571 | 17501 |
| | 12361 | 2384 | 16696 | 15613 | 9508 | 26990 | 23835 | 16804 | 21241 | 10669 | 9342 | 6963 |
| | 17934 | 22530 | 1655 | 22180 | 28044 | 28381 | 28125 | 15779 | 26520 | 3030 | 26702 | 14492 |
| | 6832 | 3097 | 11090 | 17047 | 13774 | 25492 | 17772 | 28736 | 6756 | 22199 | 23634 | 23500 |

SEQ ID NO: homolog SEQ ID NOs

| | | | | | | | | | | | |
|---|---|---|---|---|---|---|---|---|---|---|---|
| 2707 | 8625 | 22867 | 6740 | 10764 | 19182 | 12941 | 5903 | 1207 | 18629 | 14513 | 4131 |
| 6086 | 10763 | 28002 | 29940 | 25243 | 15495 | 25545 | 5238 | 3681 | 18975 | 15461 | 29760 |
| 22938 | 12554 | 3104 | 3191 | 17611 | 15641 | 7197 | 19888 | 9832 | 18406 | 6096 | 10995 |
| 23484 | 2926 | 17682 | 5070 | 30244 | 22688 | 3211 | 10420 | 30052 | 21166 | 6738 | 4692 |
| 3995 | 26748 | 16834 | 12873 | 17444 | 13352 | 29621 | 17954 | 25955 | 14434 | 15348 | 21851 |
| 3688 | 6393 | 18456 | 14023 | 15147 | 17048 | 4560 | 22935 | 18153 | 22666 | 20801 | 16608 |
| 16952 | 26007 | 26376 | 9976 | 27316 | 7296 | 18329 | 8126 | 2974 | 9482 | 17184 | 19144 |
| 18373 | 13185 | 6342 | 28774 | 25033 | 22546 | 3326 | 30246 | 6338 | 29749 | 14099 | 793 |
| 10747 | 27983 | 14938 | 21905 | 13164 | 19704 | | | | | | |

| | | | | | | | | | | | | |
|---|---|---|---|---|---|---|---|---|---|---|---|---|
| 704: | 20590 | 29620 | 15258 | 1433 | 30185 | 29039 | 19950 | 14318 | 21441 | 16914 | 21214 | 22405 |
| | 8188 | 26228 | 26213 | 16766 | 19127 | 987 | 12075 | 24302 | 18060 | 8273 | 12821 | 4315 |
| | 29544 | 24329 | 8740 | 26378 | 20620 | 23305 | 22472 | 730 | 22441 | 15904 | 3478 | 3508 |
| | 1991 | 11705 | 17718 | 9539 | 2518 | 12881 | 19518 | 3545 | 12747 | 10852 | 12776 | 11953 |
| | 18759 | 25623 | 18398 | 13291 | 10229 | 15216 | 26560 | 4840 | 27614 | 1410 | 28186 | 26073 |
| | 7749 | 29490 | 718 | 8330 | 11515 | 12399 | 22645 | 25333 | 3031 | 4723 | 23132 | 19240 |
| | 10090 | 4701 | 26755 | 20739 | 22805 | 15785 | 8717 | 10989 | 16788 | 10722 | 9458 | 4431 |
| | 13459 | 21161 | 21289 | 9005 | 12991 | 10401 | 12020 | 11909 | 17564 | 17277 | 20280 | 23172 |
| | 11692 | 13467 | 6473 | 15976 | 15088 | 3729 | 8821 | 23508 | 11667 | 18304 | 18122 | 7806 |
| | 28014 | 4225 | 18703 | 22403 | 24345 | 15269 | 16239 | 26281 | 14521 | 29709 | 16970 | 21993 |
| | 16666 | 16600 | 7903 | 26957 | 22439 | 18509 | 20455 | 27074 | 20994 | 17953 | 14845 | 2795 |
| | 5936 | 23874 | 732 | 17519 | 19195 | 14352 | 24653 | 25115 | 27803 | 29594 | 25713 | 22643 |
| | 4432 | 8066 | 11854 | 12721 | 18407 | 24497 | 19163 | 28614 | 23870 | 19757 | 15328 | 4693 |
| | 13206 | 9339 | 13373 | 5630 | 5736 | 2646 | 10515 | 13864 | 3314 | 30308 | 28199 | 21513 |
| | 9666 | 6094 | 1931 | 26832 | 7174 | 10931 | 1975 | 11086 | 5020 | 23112 | 10283 | 10138 |
| | 6455 | 13224 | 12541 | 16528 | 18130 | 18026 | 14199 | 9265 | 14827 | 9116 | 8456 | 25093 |
| | 20275 | 25320 | 20027 | 14620 | 18150 | 2209 | 4594 | 6463 | 29831 | 4082 | 17571 | 17501 |
| | 12361 | 2384 | 16696 | 15613 | 9508 | 26990 | 23835 | 16804 | 21241 | 10669 | 9342 | 6963 |
| | 17934 | 22530 | 1655 | 22180 | 28044 | 28381 | 28125 | 15779 | 26520 | 3030 | 26702 | 14492 |
| | 6832 | 3097 | 11090 | 17047 | 13774 | 25492 | 17772 | 28736 | 6756 | 22199 | 23634 | 23500 |
| | 2707 | 8625 | 22867 | 6740 | 10764 | 19182 | 12941 | 5903 | 1207 | 18629 | 14513 | 4131 |
| | 6086 | 10763 | 28002 | 29940 | 25243 | 15495 | 25545 | 5238 | 3681 | 18975 | 15461 | 29760 |
| | 22938 | 12554 | 3104 | 3191 | 17611 | 15641 | 7197 | 19888 | 9832 | 18406 | 6096 | 10995 |
| | 23484 | 2926 | 17682 | 5070 | 30244 | 22688 | 3211 | 10420 | 30052 | 21166 | 6738 | 4692 |

(continued)

SEQ ID NO: homolog SEQ ID NOs

705:

| SEQ ID NO: | homolog SEQ ID NOs | | | | | | | | | |
|---|---|---|---|---|---|---|---|---|---|---|
| 3995 | 26748 | 16834 | 12873 | 17444 | 13352 | 17954 | 25955 | 14434 | 15348 | 21851 |
| 3688 | 6393 | 18456 | 14023 | 15147 | 17048 | 22935 | 18153 | 22666 | 20801 | 16608 |
| 16952 | 26007 | 26376 | 9976 | 27316 | 7296 | 8126 | 2974 | 9482 | 17184 | 19144 |
| 18373 | 13185 | 6342 | 28774 | 25033 | 22546 | 30246 | 6338 | 29749 | 14099 | 793 |
| 10747 | 27983 | 14938 | 21905 | 13164 | 19704 | | | | | |
| 799 | 29054 | 15864 | 2108 | 28279 | 5278 | 23017 | 1609 | 21054 | 15908 | 18486 |
| 1851 | 16337 | 12990 | 26183 | 26263 | 11446 | 12542 | 29806 | 1102 | 19422 | 3259 |
| 12001 | 13455 | 21026 | 29917 | 15188 | 18414 | 22695 | 4862 | 15029 | 15286 | 17372 |
| 8622 | 26034 | 22292 | 2339 | 4994 | 7913 | 14418 | 22322 | 19383 | 13712 | 19626 |
| 14176 | 12168 | 14747 | 4936 | 23885 | 5234 | 3125 | 30106 | 26045 | 14523 | 18236 |
| 30272 | 22216 | 11046 | 22003 | 6222 | 11328 | 21040 | 16023 | 30022 | 27203 | 10781 |
| 2181 | 5443 | 20042 | 26828 | 4029 | 11711 | 15178 | 23881 | 18449 | 16292 | 24194 |
| 8384 | 30067 | 8336 | 29185 | 2012 | 27146 | 10708 | 13610 | 14067 | 17804 | 19449 |
| 9795 | 12181 | 15113 | 19132 | 6985 | 25308 | 3838 | 23127 | 26161 | 25661 | 28170 |
| 30181 | 19227 | 23801 | 19434 | 26909 | 20188 | 21157 | 13952 | 827 | 18571 | 17038 |
| 14543 | 23983 | 7556 | 26499 | 2496 | 11344 | 16295 | 21137 | 9728 | 23395 | 23985 |
| 9538 | 10770 | 16343 | 14943 | 3058 | 1266 | 13522 | 13776 | 2803 | 18845 | 7733 |
| 7369 | 29010 | 15848 | 20196 | 19188 | 3890 | 7198 | 10836 | 18857 | 4303 | 15644 |
| 17249 | 17250 | 1564 | 8675 | 20611 | 27180 | 11414 | 28582 | 21811 | 18814 | 26242 |
| 29182 | 4902 | 4392 | 4419 | 3790 | 23805 | 2434 | 26241 | 20522 | 28718 | 25133 |
| 20759 | 19677 | 25153 | 26629 | 11453 | 22183 | 18580 | 18607 | 22187 | 22215 | 22184 |
| 18604 | 22214 | 18541 | 14039 | 582 | 581 | 3269 | 28766 | 14406 | 16846 | 30318 |
| 22877 | 5329 | 27336 | 4132 | 18095 | 6856 | 28806 | 4534 | 1373 | 27768 | 6179 |
| 4253 | 24562 | 18289 | 24477 | 5928 | 4527 | 2477 | 18736 | 24767 | 26682 | 8264 |
| 6989 | 15657 | 22745 | 9765 | 23990 | 15730 | 29979 | 2333 | 30189 | 2623 | 13937 |
| 17745 | 4333 | 22561 | 27084 | 2322 | 26920 | 21959 | 18257 | 3495 | 962 | 2463 |
| 3985 | 5604 | 28713 | 29845 | 22023 | 24483 | 12288 | 29877 | 20208 | 26501 | 8097 |
| 26889 | 13606 | 24401 | 3541 | 18055 | 763 | 16213 | 20597 | 5687 | 13449 | 8619 |
| 4886 | 2346 | 27757 | 21401 | 16703 | 19016 | 14097 | 16114 | 23181 | 20327 | 11597 |
| 8381 | 23815 | 29579 | 25668 | 9323 | 14684 | 26287 | 3633 | 20217 | 13970 | 28089 |
| 1546 | 16143 | 20930 | 23290 | 24632 | 22792 | 12962 | 19037 | 20116 | 5766 | 24766 |
| 24009 | 8006 | 10626 | 26426 | 23770 | 6556 | 1521 | 1165 | 22677 | 29528 | 4515 |
| 22704 | 861 | 22272 | 17807 | 28185 | 5670 | 9513 | 25471 | 5900 | 4619 | 25099 |

(continued)

SEQ ID NO: homolog SEQ ID NOs

| | | | | | | | | | | | |
|---|---|---|---|---|---|---|---|---|---|---|---|
| | 20872 | 9409 | 8296 | 8272 | 5822 | 28208 | 9454 | 19062 | 23186 | 17159 | 10504 | 12340 |
| | 27330 | 18140 | 24290 | 11459 | 10497 | 12356 | 28886 | 16088 | 21777 | 5519 | 12840 | 27888 |
| | 13803 | 3072 | 5921 | 30134 | 14989 | 20395 | 12082 | 17616 | 2201 | 5633 | 26544 | 27198 |
| | 11442 | 25337 | 5319 | 1033 | 4767 | 10767 | 15014 | 25309 | 7680 | 13230 | 20798 | 13649 |
| | 5716 | 24934 | 29568 | 15496 | 3080 | 4716 | 22223 | 14437 | 2811 | 29645 | 11267 | 19681 |
| | 27310 | 22061 | 7368 | 2928 | 5171 | 2960 | 29452 | 6160 | 2391 | 18602 | 17082 | 25197 |
| | 6621 | 4816 | 13707 | 4300 | 26482 | 4172 | 13717 | 26202 | 8586 | 10999 | 14257 | 30053 |
| | 29748 | 11040 | 26359 | 22160 | 27174 | 8362 | 7289 | 9315 | 20441 | 11305 | 17661 | 27811 |
| | 18182 | 5239 | 18375 | 28108 | 3445 | 13676 | 12144 | 21352 | 9477 | 15897 | 9459 | 22293 |
| | 7419 | 14142 | 9996 | 2359 | 18212 | 16113 | 29046 | 26262 | 2828 | 23731 | 16056 | 15205 |
| | 1666 | 23712 | 25221 | 12160 | 15883 | 15880 | 21327 | 24547 | 25667 | 25669 | 25549 | 5110 |
| | 9954 | 27505 | 27503 | 27529 | 27528 | 27494 | 3184 | 17298 | 10506 | 769 | 15907 | 15903 |
| | 26554 | 3737 | 3733 | 18914 | 20102 | 5040 | 6576 | 17091 | 1805 | 21590 | 21588 | 26507 |
| | 26500 | 26504 | 26533 | 27909 | 26498 | 7630 | 19978 | 19979 | 11407 | 11372 | 11371 | 11406 |
| | 11458 | 11410 | 11457 | 13045 | 17012 | 15254 | 12165 | 8105 | 11450 | 11452 | 5035 | 5037 |
| | 5010 | 5014 | 5013 | 16862 | 28282 | 24230 | 4987 | 17032 | 1014 | 18460 | 25999 | 9431 |
| | 22755 | 8906 | 9471 | 25350 | 24180 | 6887 | 3261 | 23129 | 20945 | 29073 | 20942 | 12809 |
| | 12939 | 26435 | 15709 | 23107 | 7364 | 25621 | 11310 | 6407 | 7346 | 17142 | 27680 | 21630 |
| | 1471 | 23920 | 1331 | 12573 | 3449 | 9560 | 25154 | 15677 | 3024 | 24855 | 10932 | 10905 |
| | 15262 | 21130 | 24655 | 11193 | 9405 | 17573 | 1690 | 21840 | 10536 | 14631 | 9218 | 3609 |
| | 6490 | 5607 | 2482 | 6551 | 13658 | 23989 | 28592 | 15235 | 26603 | 7767 | 1149 | 8669 |
| | 11766 | 22435 | 5483 | 13142 | 18903 | 6548 | 22912 | 5843 | 7150 | 3602 | 3308 | 25475 |
| | 5194 | 16968 | 21325 | 12977 | 6520 | 9074 | 1534 | 11528 | 7459 | 10224 | 18762 | 27292 |
| | 22851 | 1828 | 18933 | 17186 | 27506 | 27942 | 2972 | 2358 | 18597 | 5160 | 15921 | 2398 |
| | 17901 | 19721 | 9246 | 5489 | 29588 | 14256 | 16576 | 29070 | 19255 | 5346 | 28285 | 1346 |
| | 1633 | 3732 | 8300 | 10563 | 12797 | 3186 | 19034 | 28299 | 23036 | 21058 | 21345 | 24349 |
| | 30096 | 25448 | 17779 | 18777 | 20477 | 29208 | 5006 | 12814 | 24801 | 20486 | 24043 | 10168 |
| | 11461 | 21888 | 5367 | 2659 | 12752 | 28817 | 26693 | 26236 | 394 | 11375 | 11370 | 11374 |
| | 11369 | 11239 | 11236 | 11221 | 11242 | 11240 | 11218 | 3876 | 21263 | 1376 | 25470 | 23407 |
| | 30303 | 23725 | 25215 | 23680 | 25961 | 25965 | 25224 | 23721 | 25968 | 23754 | 23751 | 16398 |
| | 29056 | 5201 | 8890 | 16124 | 11780 | 29426 | 21879 | 20762 | | | | |
| 706: | 2832 | 17119 | 13754 | 26415 | 27949 | 22514 | 19781 | 23613 | 11387 | 29382 | 12748 | 11080 |
| | 7560 | 5935 | 5964 | 5938 | 27366 | 29638 | 8358 | 5902 | 19526 | 12983 | 29020 | 14526 |

SEQ ID NO: homolog SEQ ID NOs

| | | | | | | | | | | | |
|---|---|---|---|---|---|---|---|---|---|---|---|
| 707: | 18052 | 26488 | 12767 | 25138 | 26893 | 5827 | 12036 | 4841 | 1457 | 21501 | 19361 | 29192 |
| | 6535 | 16248 | 11105 | 29613 | 5308 | 24420 | 16004 | 7848 | 8519 | 25238 | 14141 | 23784 |
| | 12408 | | | | | | | | | | | |
| 708: | 18052 | 26488 | 12767 | 25138 | 26893 | 5827 | 12036 | 4841 | 1457 | 21501 | 19361 | 29192 |
| | 6535 | 16248 | 11105 | 29613 | 5308 | 24420 | 16004 | 7848 | 8519 | 25238 | 14141 | 23784 |
| | 12408 | | | | | | | | | | | |
| 709: | 20636 | 10539 | 26020 | 7312 | 19436 | 19428 | 19433 | 24178 | 4204 | 20129 | 20776 | 14120 |
| | 27089 | 24049 | 19622 | 19625 | 28666 | 16842 | 13200 | 25558 | 14480 | 4250 | 14090 | 9045 |
| | 26591 | 29368 | 25617 | 25654 | 20263 | 1774 | 29463 | 6589 | 12424 | 10660 | 8155 | 13153 |
| | 22848 | 13644 | 27133 | 29767 | 26797 | 4465 | 18421 | 1773 | 6249 | 21118 | 10880 | 11976 |
| | 7808 | 16347 | 28837 | 3747 | 14822 | 30138 | 15381 | 4629 | 30093 | 27141 | 3527 | 8369 |
| | 8367 | 7675 | 2404 | 5628 | 19630 | 19629 | 19585 | 19581 | 3765 | 24007 | 19788 | 28292 |
| | 12479 | 21492 | 23673 | 26485 | 7993 | 7068 | 7273 | 10979 | 6415 | 3055 | 10534 | 27096 |
| | 7095 | 7933 | 20398 | 9086 | 19589 | 19621 | 2971 | 22716 | 24593 | 9111 | 15975 | 4830 |
| | 2389 | 3331 | 30092 | 19074 | 16792 | 9762 | 19158 | 11483 | 1004 | 7405 | 12981 | 7316 |
| | 17675 | 24116 | 11361 | 11357 | 24136 | 24137 | 11363 | 11366 | 11432 | 10516 | 20593 | 3468 |
| | 3992 | 23764 | 26283 | 13879 | 7548 | 949 | 19460 | 19440 | 19466 | 29625 | 19542 | 19517 |
| | 19514 | 29305 | 21311 | 27595 | 18711 | 21019 | 24169 | 16224 | 15011 | 12225 | 10182 | 21770 |
| | 16165 | 16161 | 14146 | 13340 | 14058 | 13874 | 21581 | 19657 | 25928 | 4756 | 4957 | 11807 |
| | 16160 | 19762 | 14124 | 5503 | 23214 | 14623 | 29364 | 20659 | 20617 | 19692 | 20623 | 20621 |
| | 21657 | 20469 | 19699 | 21629 | 20473 | 19688 | 21662 | 20502 | 19729 | 20503 | 20579 | 21665 |
| | 20471 | 21651 | 16167 | 21655 | 19690 | 20626 | 20665 | 20500 | 20464 | 20662 | 19735 | 19727 |
| | 20614 | 19732 | 20669 | 20698 | 20693 | 20696 | 20667 | 21525 | 20738 | 21583 | 20805 | 20811 |
| | 20770 | 21548 | 20778 | 20506 | 20576 | 21624 | 20730 | 20505 | 21580 | 21578 | 21620 | 21519 |
| | 20571 | 21546 | 20575 | 20535 | 20807 | 20702 | 20699 | 21626 | 21617 | 21551 | 21521 | 21586 |
| | 16193 | 21522 | 20732 | 21545 | 20735 | 20773 | 21550 | 16192 | 20771 | 25871 | 25872 | 26764 |
| | 26766 | 2159 | 10485 | 16150 | 25823 | 18348 | 18303 | 2933 | 19008 | 23289 | 12571 | 17025 |
| | 20167 | | | | | | | | | | | |
| 710: | 26071 | 26056 | 26014 | 9404 | 19602 | 20301 | 8179 | 19916 | 20128 | 30179 | 17122 | 24023 |
| | 27435 | 28127 | 25454 | 16564 | 14404 | 15168 | 15165 | 4188 | 29759 | 18745 | 19927 | 8398 |
| | 23948 | 16700 | 24055 | 30280 | 26944 | 26181 | 21682 | 25773 | 3873 | 1206 | 21801 | 25685 |
| | 15142 | 15138 | 15134 | 6135 | 21874 | 14639 | 7086 | 10352 | 10393 | 11851 | 7051 | 4645 |
| | 9396 | 1587 | 14939 | 23581 | 25405 | 20038 | 15627 | 8161 | 10075 | 14290 | 16351 | 25650 |

(continued)

SEQ ID NO: homolog SEQ ID NOs

| | | | | | | | | | | | |
|---|---|---|---|---|---|---|---|---|---|---|---|
| 20283 | 8823 | 14049 | 6402 | 11429 | 5527 | 4490 | 721 | 14888 | 25776 | 12079 | 17655 |
| 12928 | 19784 | 8181 | 28193 | 26128 | 11125 | 15335 | 24326 | 24309 | 4349 | 915 | 3884 |
| 8451 | 14504 | 29847 | 8995 | 789 | 20787 | 30198 | 22839 | 2566 | 5675 | 23495 | 14224 |
| 14222 | 17332 | 25331 | 9365 | 11201 | 3539 | 16871 | 16873 | 996 | 23070 | 20833 | 4687 |
| 18603 | 16069 | 16120 | 15977 | 24548 | 21276 | 2642 | 5775 | 21602 | 9752 | 27699 | 1170 |
| 25995 | 22276 | 26586 | 11575 | 23422 | 19452 | 22900 | 20015 | 28853 | 25324 | 3481 | 4291 |
| 23560 | 23562 | 20764 | 25731 | 15204 | 14813 | 17860 | 16651 | 14920 | 14749 | 9588 | 9214 |
| 10499 | 6886 | 6920 | 6149 | 24920 | 19944 | 12705 | 11054 | 10368 | 7029 | 12838 | 13878 |
| 12989 | 29947 | 10262 | 14734 | 23057 | 20035 | 24212 | 25167 | 24119 | 22916 | 22922 | 11765 |
| 9597 | 15938 | 10639 | 4911 | 21561 | 21671 | 19232 | 17507 | 25709 | 14662 | 7035 | 11867 |
| 2218 | 9307 | 28744 | 28769 | 11666 | 7591 | 29113 | 2851 | 17379 | 15492 | 24459 | 3264 |
| 5751 | 21707 | 17085 | 29891 | 7264 | 7744 | 5976 | 22656 | 10909 | 21606 | 20456 | 20612 |
| 7966 | 3717 | 12323 | 4040 | 5774 | 5081 | 21445 | 29807 | 26477 | 26123 | 30128 | 23845 |
| 23436 | 20921 | 1059 | 20384 | 19090 | 22950 | 20935 | 16185 | 3444 | 10043 | 12930 | 15175 |
| 29962 | 794 | 5926 | 26270 | 4175 | 11944 | 1289 | 20428 | 23714 | 1634 | 1661 | 20607 |
| 5351 | 5356 | 24685 | 10006 | 7424 | 19790 | 21063 | 19872 | 16157 | 6902 | 6778 | 26663 |
| 4247 | 26428 | 8644 | 8648 | 21923 | 8471 | 16549 | 11224 | 9085 | 8917 | 20244 | 24416 |
| 1959 | 5471 | 5226 | 6757 | 10439 | 18980 | 10446 | 6773 | 9229 | 17061 | 9347 | 24845 |
| 13375 | 21847 | 13544 | 16176 | 21395 | 16309 | 3076 | 6744 | 23118 | 5575 | 22558 | 5494 |
| 8274 | 7443 | 2970 | 11519 | 4603 | 17297 | 9169 | 1539 | 12468 | 25655 | 21622 | 4108 |
| 21699 | 23614 | 16583 | 4334 | 29107 | 21285 | 22198 | 22262 | 18335 | 1843 | 9570 | 1181 |
| 27806 | 5839 | 3257 | 8753 | 11710 | 11120 | 11099 | 17173 | 19248 | 17811 | 12302 | 11764 |
| 8634 | 8436 | 28961 | 15794 | 24397 | 6465 | 13919 | 25697 | 22598 | 2362 | 14390 | 13324 |
| 19122 | 30073 | 967 | 2199 | 29444 | 23748 | 1350 | 15490 | 27940 | 7584 | 11503 | 8521 |
| 27260 | 15957 | 6271 | 13120 | 10174 | 7033 | 11757 | 21747 | 2658 | 12113 | 12110 | 23767 |
| 4064 | 15454 | 27539 | 1585 | 12505 | 29437 | 26704 | 23392 | 23396 | 25629 | 13887 | 22094 |
| 23099 | 20991 | 24452 | 24451 | 4371 | 29281 | 23022 | 23019 | 16953 | 17037 | 19423 | 29460 |
| 6079 | 11753 | 11723 | 17933 | 19710 | 18308 | 19251 | 12019 | 1285 | 1287 | 13187 | 29886 |
| 7817 | 24204 | 4672 | 20437 | 4591 | 1682 | 13186 | 20499 | 19776 | 19780 | 6287 | 16139 |
| 15010 | 14514 | 13780 | 7464 | 22880 | 16060 | 15170 | 15187 | 15192 | 15171 | 26282 | 5005 |
| 5673 | 18842 | 11985 | 22381 | 14298 | 1409 | 26452 | 11810 | 11808 | 20375 | 20397 | 985 |
| 3778 | 21694 | 17785 | 22854 | 2321 | 18117 | 12615 | 18369 | 28797 | 10752 | 2216 | 1971 |
| 3012 | 26449 | 2682 | 25613 | 16878 | 9313 | 7012 | 15199 | 15194 | 15203 | 15947 | 15162 |

SEQ ID NO: homolog SEQ ID NOs

| | | | | | | | | | | | | | |
|---|---|---|---|---|---|---|---|---|---|---|---|---|---|
| | 15949 | 15951 | | 11574 | 20302 | 27624 | 1945 | 2034 | 15295 | 15661 | 27387 | 11165 | 11202 |
| | 26148 | | | | | | | | | | | | |
| 711: | 6354 | 25983 | | 24334 | 13638 | 24662 | 4162 | 14389 | 14267 | 22724 | 12931 | 25806 | 5305 |
| | 9319 | 2450 | | 19747 | 18935 | 18345 | 24652 | 24359 | 15647 | 28505 | 18380 | 6442 | 4033 |
| | 9435 | 18047 | | 24715 | 9908 | 11613 | 3675 | 6639 | 1118 | 16344 | 3972 | 23278 | 15726 |
| | 8636 | 12277 | | 28104 | 3157 | 10569 | 22005 | 7539 | 14440 | 9997 | 14130 | 13617 | 18899 |
| | 8727 | 5846 | | 17817 | 28323 | 2488 | 29796 | 19104 | 2878 | 11729 | 26230 | 4331 | 10141 |
| | 2449 | 25736 | | 19498 | 27521 | 14683 | 23229 | 11007 | 1442 | 27583 | 25883 | 1970 | 5806 |
| | 22212 | 25144 | | 7717 | 15691 | 29571 | 20431 | 7690 | 18483 | 24033 | 8766 | 21437 | 18243 |
| | 10775 | 4843 | | 4704 | 6788 | 15434 | 8396 | 26996 | 30295 | 5769 | 8952 | 3035 | 9109 |
| | 876 | 1723 | | 15101 | 12733 | 3903 | 26518 | 28424 | 17361 | 2909 | 15055 | 16401 | 6004 |
| | 20973 | 5520 | | 12467 | 28660 | 25973 | 12750 | 21484 | 1133 | 19033 | 3524 | 18746 | 18743 |
| | 25321 | 2875 | | 2899 | 19285 | 25632 | 13384 | 11167 | 11487 | 10930 | 11544 | 28405 | 10086 |
| | 11508 | 27370 | | 9853 | 18971 | 25128 | 15311 | 6661 | 10967 | 19841 | 16313 | 6688 | 14212 |
| | 20011 | 5072 | | | | | | | | | | | |
| 712: | 10670 | 19799 | | 921 | 24842 | 13619 | 17068 | 8809 | 27179 | 11376 | 13895 | 16030 | 5176 |
| | 14365 | 4052 | | 1860 | 9625 | 19072 | 22364 | 25480 | 5164 | 17638 | 26674 | 19427 | |
| 713: | 1550 | 12691 | | 28768 | 27725 | 16491 | 9212 | 16410 | 3909 | 12224 | 9964 | 25746 | 13663 |
| | 4820 | 14644 | | 7671 | 2329 | 19298 | 22731 | 15149 | 9406 | 14417 | 30095 | 7152 | 8834 |
| | 28007 | 28006 | | 26043 | 3433 | 26673 | 7627 | 7027 | 11838 | 30002 | 21454 | 23975 | 11489 |
| | 14150 | 12141 | | 2534 | 21211 | 23988 | 7446 | 20303 | 21320 | 7928 | 27538 | 12960 | 3272 |
| | 10586 | 25853 | | 29939 | 10572 | 24959 | 15622 | 27049 | 4216 | 11019 | 27291 | 6258 | 26849 |
| | 5486 | 5975 | | 8642 | 10719 | 21248 | 11327 | 12818 | 10769 | 6682 | 29141 | 13703 | 29077 |
| | 5762 | 29082 | | 13308 | 2379 | 7205 | 13504 | 11889 | 8250 | 16678 | 12737 | 2198 | 26104 |
| | 22334 | 7003 | | 18064 | 11627 | 19006 | 9876 | 24144 | 16403 | 16889 | 16763 | 4109 | 22165 |
| | 927 | 22028 | | 3224 | 1299 | 4091 | 20715 | 9385 | 16240 | 8101 | 6746 | 2603 | 21579 |
| | 872 | 4496 | | 9739 | 9733 | 26717 | 3695 | 23600 | 14356 | 27766 | 18198 | 21389 | 22383 |
| | 19473 | 10371 | | 12640 | 8757 | 25889 | 27760 | 28852 | 12500 | 5316 | 22358 | 13069 | 3941 |
| | 26817 | 727 | | 16649 | 24588 | 14455 | 13502 | 16759 | 24096 | 28912 | 28399 | 5481 | 29369 |
| | 22007 | 15767 | | 24646 | 12576 | 7639 | 28624 | 6847 | 9314 | 13275 | 10553 | 16512 | 21567 |
| | 29775 | 28897 | | 21535 | 18574 | 1969 | 27716 | 7154 | 8351 | 20606 | 5231 | 12976 | 20697 |
| | 18687 | 4120 | | 21474 | 7822 | 5690 | 3604 | 11480 | 7666 | 20179 | 21558 | 12429 | 19855 |
| | 16146 | 18695 | | 16392 | 26414 | 6259 | 10888 | 12377 | 12824 | 4307 | 1428 | 12817 | 24390 |

EP 2 540 831 A2

(continued)

| SEQ ID NO: | homolog SEQ ID NOs | | | | | | | | | | | |
|---|---|---|---|---|---|---|---|---|---|---|---|---|
| | 11965 | 11790 | 7109 | 17671 | 6384 | 10981 | 23965 | 24720 | 23953 | 24714 | 27386 | 12790 |
| | 13900 | 9608 | 16435 | 12107 | 22825 | 8229 | 6220 | 12443 | 26179 | 20284 | 25901 | 5387 |
| | 9173 | 17796 | | | | | | | | | | |
| 714: | 4717 | 18780 | 21115 | 25306 | 2969 | 30157 | 7417 | 23927 | 8548 | 23625 | 23623 | 3401 |
| | 3418 | 8099 | 2854 | 9890 | 16810 | 2367 | 14848 | 1782 | | | | |
| 715: | 24012 | 12612 | 16793 | 13263 | 729 | 18298 | 26974 | | | | | |
| 716: | 24012 | 12612 | 16793 | 13263 | 729 | 18298 | 26974 | | | | | |

A plant cell nucleus with stably integrated, recombinant DNA, wherein a. said recombinant DNA comprises a promoter that is functional in said plant cell and that is operably linked to a protein coding DNA encoding a protein having an amino acid sequence comprising a Pfam domain module selected from the group consisting of Gp_dh_N::Gp_ dh_ C, Mg_chelatase::VWA, zf-CCCH::zf-CCCH::zf-CCCH::zf-CCCH::zf-CCCH, WD40, tRNA-synt_2b::HGTP_anticodon, RNase_PH::RNase_PH_C, F-box::Kelch_1::Kelch_1, Peptidase_C54, Iso_dh, Metallophos, OTU, Rotamase, Sugar_ tr, Glyoxalase::Glyoxalase, Ras, Brix, S6PP::S6PP_C, PsbR, Pkinase, p450, PP2C, CH::EB1, DUF537, Histone, PPR:: PPR::PPR::PPR::PPR, TFIIS_M::TFIIS_C, DUF751, RRM_1::RRM_1, ETC_C1_ NDUFA4, SRF-TF, CCT, Globin:: FAD_binding_6::NAD_binding_1, FAE1_CUT1_ RppA::ACP_syn_III_C, Frataxin_Cyay, F-box::LRR_2, Tryp_alpha_ amyl, PFK::PFK, Dehydrin, RLI::Fer4::ABC_tran::ABC_tran, CTP_transf_2, GTP_EFTU::GTP_EFTU_ D2::GTP_ EFTU_D3, PfkB, IPT, TPR_1::TPR_2::TPR_1::TPR_2::TPR_1::TPR_1::TPR_ 1::TPR_1::TPR_1, Globin, Porphobil_ deam::Porphobil_deamC, NB-ARC::LRR_1::LRR_ 1::LRR_1, Bromodomain, DUF1365, PTS_2-RNA, Pkinase::UBA:: KA1, MATH::BTB, DUF6::TPT, Cyclin_N::Cyclin_C, zf-AN1, Methyltransf_6, Thioredoxin, DNA_photolyase::FAD_ binding_7, vATP-synt_E, Bac_globin, B_lectin::S_locus_glycop::PAN_ 2::Pkinase_Tyr, Sigma70_r2::Sigma70_r3:: Sigma70_r4, Ribosomal_L10, zf-C3HC4::WD-40::WD40::WD40, PGM_PMM_I::PGM_PMM_II::PGM_PMM_III::PGM_ PMM_IV, Hydrolase, Peptidase_C1, DS, Carotene_hydrox, Aa_trans, Mov34, zf-MYND::UCH, Heme_oxygenase, S6PP, SSB, Peptidase_M16::Peptidase_M16_C, Bet_v_I, Auxin_ inducible, Response_reg, Di19, DUF125, GDC-P, Pyr_redox_2::Fer2_BFD::NIR_SIR_ ferr::NIR_SIR, KOW::eIF-5a, MtN3_slv::MtN3_slv, Ribul_P_3_epim, NPH3, DnaJ:: DnaJ_ C, UQ_con, RRM_1::RRM_1::RRM_1, F-box, CoA_binding::Ligase_CoA, adh_short, Ribosomal_L22, AA_permease, Acyltransferase, AMPKBI, RRM_1, Chalcone, GATase_ 2::Asn_synthase, Peptidase_M24, DUF498, DAGAT, PFK, DUF1677, Glyco_transf_43, zf-DNL, DHBP_synthase::GTP_cyclohydro2, PseudoU_synth_2, Glyoxalase, DUF21::CBS, Ribosomal_S30AE, Glycolytic, Chloroa_b-bind, ZF-HD_dimer, Usp, Ferrochelatase, Pyridoxal_deC, Glyco_transf_8, Pyr_redox_2::Glutaredoxin, Epimerase, UPF0113, RNase_ PH, AIG1, Phi_1, CorA, HD::RelA_SpoT, P-II, GSHPx, PGAM, PGI, DUF868, Lung_7-TM_R, F-box::FBA_1, TPP_enzyme_N::TPP_enzyme_M::TPP_enzyme_ C, DnaJ::zf-CSL, DEAD::Helicase_C, 20G-FeII_Oxy, HMGL-like::LeuA_dimer, VQ, DUF298, DREPP, ketoacyl-synt:: Ketoacyl-synt_C, THF_DHG_CYH::THF_DHG_CYH_C, DNA_pol_E_B, UPF0051, Pkinase::efhand::efhand::efhand:: efhand, malic: :Malic_M, ThiF, Transket_ pyr::Transketolase_C, Ribosomal_L37ae, PEPcase, Glyco_hydro_32N:: Glyco_hydro_32C, GASA, DnaJ, AA_kinase::ACT::ACT, Pkinase_Tyr, Cupid_1, zf-LSD1::zf-LSD1::zf-LSD1, Cupin_3, GAF::HisKA::HATPase_c::Response_reg, Methyltransf_12::Mg-por_mtran_C, DUF516, PTR2, Ammonium_transp, eIF-5a, ECH, Aldedh, zf-C3HC4, SAM_decarbox, X8, Mg_chelatase, PurA, Ribosomal_S6e, Molybdop_Fe4S4::Molybdopterin::Molydop_binding, CP12, Biotin_lipoyl::E3_binding::2-oxoacid_dh, NOI, Tubulin::Tubulin_C, V-SNARE, AP2, ELFV_dehydrog_N::ELFV_dehydrog, Ribosomal_L32e, and FAD_binding_3; b. said recombinant DNA comprises a promoter that is functional in said plant cell and that is operably linked to a protein coding DNA encoding a protein comprising an amino acid sequence with at least 90% identity to a consensus amino acid sequence selected from the group consisting of SEQ ID NO: 30377 through SEQ ID NO: 30418;

c. said recombinant DNA comprises a promoter that is functional in plant cells and that is operably linked to a protein coding DNA encoding a protein comprising an amino acid sequence selected from the group consisting of 395, 553, 640 , and homologs thereof listed in table 9 ; or

d. said recombinant DNA comprises a promoter that is functional in said plant cell and that is operably linked to a protein coding recombinant DNA encoding a protein having an amino acid sequence having at least 70% identity to an amino acid sequence selected from the group consisting of 560;

and wherein said plant cell nucleus is selected by screening a population of transgenic plants that have said recombinant DNA and an enhanced trait as compared to control plants that do not have said recombinant DNA in their nuclei; and wherein said enhanced trait is selected from group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil.

The plant cell nucleus as defined above, wherein said protein coding DNA encodes a protein having an amino acid sequence selected from the group consisting of SEQ ID NO: 350 through SEQ ID NO: 30327.

The plant cell nucleus as defined above, wherein said protein coding DNA encodes a protein having an amino acid sequence with at least 70% identity to SEQ ID NO: 379. The plant cell nucleus as defined above, wherein said protein coding DNA encodes a protein having an amino acid sequence with at least 80% identity to SEQ ID NO: 379. The plant cell nucleus as defined above, wherein said protein coding DNA encodes a protein having an amino acid sequence with at least 90% identity to SEQ ID NO: 379. The plant cell nucleus as defined above further comprising DNA expressing a protein that provides tolerance from exposure to an herbicide applied at levels that are lethal to a wild type of said plant cell.

The plant cell nucleus as defined above, wherein the agent of said herbicide is a glyphosate, dicamba, or glufosinate compound.

A transgenic plant cell or plant comprising a plurality of plant cells with the plant cell nucleus as defined above.

The transgenic plant cell or plant as defined above, which is homozygous for said recombinant DNA.

A transgenic seed comprising a plurality of plant cells with the plant cell nucleus as defined above.

The transgenic seed as defined above, which is selected from a corn, soybean, cotton, canola, alfalfa, wheat or rice plant.

A transgenic pollen grain comprising a haploid derivative of the plant cell nucleus as defined above.

A method for manufacturing non-natural, transgenic seed as defined above that can be used to produce a crop of transgenic plants with an enhanced trait resulting from expression of stably-integrated recombinant DNA wherein said method for manufacturing said transgenic seed comprising:

(a) screening a population of plants for said enhanced trait and said recombinant DNA wherein individual plants in said population can exhibit said trait at a level less than, essentially the same as or greater than the level that said trait is exhibited in control plants which do not express the recombinant DNA, wherein said enhanced trait is selected from the group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil;

(b) selecting from said population one or more plants that exhibit said trait at a level greater than the level that said trait is exhibited in control plants; and

(c) collecting seed from selected plants selected from step b.

The method as defined above, further comprising

(d) verifying that said recombinant DNA is stably integrated in said selected plants; and

(e) analyzing tissue of said selected plant to determine the expression or suppression of a gene that encodes an protein having the function of a protein having an amino acid sequence selected from the group consisting of one of SEQ ID NO:358-716.

A method of producing hybrid corn seed comprising:

(a) acquiring hybrid corn seed from a herbicide tolerant corn plant which also has stably-integrated, recombinant DNA in a nucleus as defined above;

(b) producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA;

(c) selecting corn plants which are homozygous and hemizygous for said recombinant DNA by treating with an herbicide;

(d) collecting seed from herbicide-treated-surviving corn plants and planting said seed to produce further progeny corn plants;

(e) repeating steps (c) and (d) at least once to produce an inbred corn line; and

(f) crossing said inbred corn line with a second corn line to produce hybrid seed.

## Claims

1.   A plant cell with stably integrated, recombinant DNA, wherein said recombinant DNA comprises a promoter that is functional in said plant cell and that is operably linked to a protein coding DNA encoding a protein having an amino acid sequence with at least 60% identity to an amino acid sequence selected from SEQ ID NOs: 546-716, 359-378, and 380-545.

2.   The plant cell of claim 1, wherein said plant cell is selected by screening a population of transgenic plants that have said recombinant DNA and an enhanced trait as compared to control plants that do not have said recombinant DNA in their nuclei; and wherein said enhanced trait is selected from the group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil.

3.   The plant cell of claim 1 or 2, wherein said protein coding DNA encodes a protein having an amino acid sequence with at least 70% identity, preferably with at least 80% identity, most preferably with at least 90% identity to an amino acid sequence selected from SEQ ID NOs: 546-716, 359-378, and 380-545.

4. The plant cell of claim 1 or 2 further comprising DNA expressing a protein that provides tolerance from exposure to an herbicide applied at levels that are lethal to a wild type of said plant cell wherein the agent of said herbicide is preferably a glyphosate, dicamba, or glufosinate compound.

5. A transgenic plant or seed comprising a plurality of plant cells with the plant cell of claim 1 or 2.

6. The transgenic plant or seed of claim 5, wherein said plant is homozygous for said recombinant DNA.

7. The transgenic plant or seed of claim 6, wherein said seed is from a corn, soybean, cotton, canola, alfalfa, wheat or rice plant.

8. A transgenic pollen grain comprising a haploid gamete of the plant cell of claim 1 or 2.

9. A method for manufacturing non-natural, transgenic seed of claim 5 that can be used to produce a crop of transgenic plants with an enhanced trait resulting from expression of stably-integrated recombinant DNA wherein said method for manufacturing said transgenic seed comprising:

(a) screening a population of plants for said enhanced trait and said recombinant DNA wherein individual plants in said population can exhibit said trait at a level less than, essentially the same as or greater than the level that said trait is exhibited in control plants which do not express the recombinant DNA, wherein said enhanced trait is selected from the group of enhanced traits consisting of enhanced water use efficiency, enhanced cold tolerance, enhanced heat tolerance, enhanced resistance to salt exposure, enhanced shade tolerance, increased yield, enhanced nitrogen use efficiency, enhanced seed protein and enhanced seed oil;
(b) selecting from said population one or more plants that exhibit said trait at a level greater than the level that said trait is exhibited in control plants; and
(c) collecting seed from selected plants selected from step b.

10. The method of claim 9 further comprising

(d) verifying that said recombinant DNA is stably integrated in said selected plants; and
(e) analyzing tissue of said selected plant to determine the expression or suppression of a gene that encodes an protein having the function of a protein having an amino acid sequence selected from the group consisting of SEQ ID NOs: 546-716, 359-378, and 380-545.

11. A method of producing hybrid corn seed comprising:

(a) acquiring hybrid corn seed from a herbicide tolerant corn plant which also has stably-integrated, recombinant DNA of claim 1;
(b) producing corn plants from said hybrid corn seed, wherein a fraction of the plants produced from said hybrid corn seed is homozygous for said recombinant DNA, a fraction of the plants produced from said hybrid corn seed is hemizygous for said recombinant DNA, and a fraction of the plants produced from said hybrid corn seed has none of said recombinant DNA;
(c) selecting corn plants which are homozygous and hemizygous for said recombinant DNA by treating with an herbicide;
(d) collecting seed from herbicide-treated-surviving corn plants and planting said seed to produce further progeny corn plants;
(e) repeating steps (c) and (d) at least once to produce an inbred corn line; and
(f) crossing said inbred corn line with a second corn line to produce hybrid seed.

12. A recombinant DNA construct comprising a polynucleotide which encodes a protein having a sequence having at least 60% identity to an amino acid sequence selected from SEQ ID NOs: 546-716, 359-378, and 380-545.

13. The recombinant DNA construct of claim 12 wherein said encoded protein has a sequence having at least 70% identity, preferably at least 80% identity, most preferably at least 90% identity to an amino acid sequence selected from SEQ ID NOs: 546-716, 359-378, and 380-545.

38-21(54146)B  Abad, Mark et al. 08/17/2007
Transgenic plants with enhanced agronomic traits
Figure #1 of 5
Page 1 of 2

```
SEQ ID NO:  homolog SEQ ID NOs
15651        --------MTSSSPTQRTHVSIPPEPGGKSLTQEANEPPVPISVSSPCGKRTRDPEDE-V
2136         FTFVLCKYCSRYCHESIVSCLELNSSGGKSLTQEANEPPVPISVSSPCGKRTRDPEDE-V
3530         --------MTPASPTQGTHVSDPPEPRGKTLTRESNEAALPISDSSACGNRTIDAEDD-V
13661        -----------------------MGPESRSTPISASSPASSSSPSGKRTRDPEDDEV
25924        --------------------------MGSDSNLGGSPSASSPSAKRTRDPEEE-V
15894        --------------------------MGSDSNLGGSPSASSPSAKRTRDPEEE-V
16409        --------------------------MGFDSNLGGSPSSSSPSSKRTRDPEEE-V
7383         --------------------------MGFDSNLGGSPSSSSPTAKRTRDPEEE-V
16880        -------------------MVGGGGGGEAAMSPPS--------GGGKRGRDPEED-V
1824         -------------------MAGGSGEGAAMSPPSSSGVGGGGGGGKRGRDPEED-V
561          -------------------MAGGSGEGAAMSPPSSS------ASGKRGRDPEED-V
consensus    -------------------xxxxxxxxxxxxxxxxxxxxsxssxxxkRtrDpExx-V
30328
```

```
YLDNLRSQKRYLSEIMACSLNGLTVGDSLPVNMLESPARSE---SFLY-----HRDDLS-
YLDNLRSQKRYLSEIMACSLNGLTVGDSLPVNMLESPARSE---SFLY-----HRDDLS-
YLDNLRSHKRYLNEIVACMXNGLTVGDSLPANVXESPERSE---SLLY-----HRDDLS-
YLDNLRSQKRYLSEIMACSLNGLTVGDSLPVNMLDSPSRSD---TLLSPNNHIHRDDLS-
YLDNLRSHKRYLSEIMASSLNGLTVGDSLPDNLMESPARSESMFS-------FRDDMS-
YLDNLRSHKRYLSEIMASSLNGLTVGDSLPDNLMDSPARSESMFS-------FRDDMSF
YVDNLRSHKRYLSEIMASSLNGLTVGDSLPDNLMESPARSESAFS-------LRDDMS-
YVDNLRSHKRYLSEIMASSLNGLTVGDSLPDNLLESPARSESAFS-------LRDDIS-
YVDNLHSHKRYLSEIMASSLNGLSVGDSLVDNIMESPARSENT--------SYFRDEII
YVDNLHSHKRYLSEIMASSLNGLSVGDSLADNIMESPARSESS--------SCIRDEII
YVDNLHSHKRYLSEIMASSLNGLSVGDSLPDNIMESPARSETA--------SCYRDEIL
YxDNLrShKRYLsEImAxslNGLtVGDSLpxNxxeSPaRSexxxxxxx----xxrdDxsx
```

```
LQYSPMSEDSDEARFCEDPTATASTSSSQPESRPTSPVS-PYRYQRPLTSTNSPQPSPTI
LQYSPMSEDSDEARFCEDPTATASTSSSQPESRPTSPVS-PYRYQRPLTSTNSPQPSPTI
LQDSPMTXQSCELIFCEEPTITASTMSSKPESRPTSPVS-PYRYHRPLTSIDSPQPSLIV
LQYSPMSEDSDEARFCEDP-ITSTSSSQQPESRPTSPVS-PYRYHRPLTSTTS-------
LQYSPMSEDSDDLRFCETPVHSCSS---QPDSLPSSPVS-PHRYQRPQNALPS-------
LQYSPMSEDSDDLRFCETPVHSCSS---QLDSLPSSPVS-PHRYQRPQNASSS-------
LQYSPMSEDSDDSRFCETGLHSCSS---HPDSRPGSPVS-PCRYQRSQNTFSS-------
LQYSPMSEDSDDSRFCETPVHSCLT---NLDSRPSSPVS-PCRYQRQQNSFSS-------
SQYSPMSEDSDDYRCYDTQLP-NGSQTDAMVSPSTSPMSSPHRFQKPQSGLLS-------
SQYSPMSEDSDDYRYFDTQLNPNGSQGDAMVSPSTSPMSSPHRFQKPQAGFLP-------
SQYSPMSEDSDDYRCYDTQLNPSGNHPDAMISPSTSPMSSPHRHQRPQSSLLP-------
lQySPMsedSdxxrfcexxxxxxxxxxxxxxxSxpxSPvS-PxRyqrpxxxxxs-------
```

```
LHHSHTCPASMISNAATTTTPQSRQRG-SDTEGRFPSSPSDICHSGDLRRTALLRSVQMR
LHHSHTCPASMISNAATTTTPQSRQRG-SDTEGRFPSSPSDICHSGDLRRTALLRSVQMR
IHHSHTWPASMSSSATSTTTPQSRQRGXSDTGRGSPSSPSDICPSGDLGRTALPRAVQMR
-HHLSNSHSCPASMSTNATTPQSRPRG-SDTEGRFPSSPSDICHSSDLRRTALLRSVQMR
-APSSSSNASY---GSTIACPQPRQRG-SDSEGRFPSSPSDICHSADLRRAALLRSVQMR
-APSSSSNASH---VSTVTCPQPRQRG-SDSEGRFPSSPSDICHSADLRRAALLRSVQMR
-APSTSLNGSHGFSVSAVTCSQPRQRS-SDSDGRFPSSPSDICHSADLRRAALLRSVQMR
-APSTSLNTSHGFPVSAVTCSQPRQRS-SDSDGRFPSSPSDICHSADLRRAALLRSVQMR
------ANPYPLPSCSLSSVVCSNPRRGSENEGRFPSSPNDMCHGGDLRKTALLRSVQMR
------SSPYPLPSCSLSSVACSHPRRGSENEGRFPSSPNDMCHGADLRRTALLRSVQMR
------SNPYPLPSCSLSSVVCSHARRGSDNEGRFPSSPNDMCHVADLRRTALLRSVQMR
-xxxxxxxxxxxxxxxxxxxxxxqxrxRxxSdxegrfPSSPsDiChsxDLrrxALlRsVQMR
```

38-21(54146)B Abad. Mark et al. 08/17/2007
Transgenic plants with enhanced agronomic traits
Figure #1 of 5
Page 2 of 2

```
TQPCGYSSSSGPSNIDG-EERMCSKSMEEDRGYNKGEDIPYAEVSS-KSKSCKALDMRLC
TQPCGYSSSSGPSNIDG-EERMCSKSMEEDRGYNKGEDIPYAEVSS-KSKSCKALDMRLC
TQPCGYSSSSGPSNIDG-EERMCSKSMEEDRGYNKGEDIPYAEVSS-KSKSCKALDMRLC
TQPCGIASTXGTSNVDGGEEKLCFKSMEED---NRGEDVSYTQVSGGKSKSCKALDMRLC
TQPPGPASLDLPFVSNQESAPNIDPEERSCSYMKSLVDEREY-----------------
TQPPSPASLDLPFVSNPESAPNIDPEERSCPYMKSLVDEREY-----------------
THPPGSASLELPFGSGQEPPPNIDPEERPCSYLKSMVDERDYQIEECSPIGIPEPEFNHD
THPPGSASLELPFGSGQEPAPNIDSEDRPCSYMKSLVDERDYQIEECSSIGAPETEFNHD
VQG--PHAYELSFCGRQEQEHAHDHE--DEHQHEHLEGLEGAERSSSHRETISDGVSYQM
VHGX-PHAYDPSFGSRQEQEHVHEHD--DEHGHEHLEDLGGPERPSCRKSIDNEASGYQG
VQG--PHSYDLSFGIRQGQDHAHEHEGEHDHEHEHLEDLEGTEGSSCNKSIVNEVTYNQR
tqpxxxxsxxxxxxxxxxxxxxxxxxxxxxxxxxxxxdxxxxxxxxxxxxxxxxxxxxxx


EK-------------------------------------------------*
EK-------------------------------------------------*
EK-------------------------------------------------*
ER-------------------------------------------------*
--------------------------------------------------*
--------------------------------------------------*
N-KPCRVLNMNPKEADSGG-------------------------------*
NNKPCRVLNMNPKAADSGG-------------------------------*
PENSYGRPEHDIDYIEDCTPHGCLSDLKFKQEDKD-CSKLTSMDKNR*
AENSYGRQEHDIDYIDGCTADDVLSGPKFKQEDDNQGNSDASMDKTR*
RDHDFGRSEHEIDYINNSTSDDCVSDLKFKQEDKSHSKFDIPMHKNS*
xxxxxxxxxxxxxxxxxxxx------------------------------*
```

38-21(54146)B   Abad, Mark et al. 08/17/2007
Transgenic plants with enhanced agronomic traits
Figure #2 of 5

Plasmid map of pMON93039

Plasmid map of pMON93039, pMON93039, SEQ ID NO:30329, 11722 bp

38-21(54146)B   Abad, Mark et al. 08/17/2007
Transgenic plants with enhanced agronomic traits
Figure #3 of 5

38-21(54146)B  Abad, Mark et al. 08/17/2007
Transgenic plants with enhanced agronomic traits
Figure #4 of 5

38-21(54146)B Abad, Mark et al. 08/17/2007
Transgenic plants with enhanced agronomic traits
Figure #5 of 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60838415 B
- US 5858742 A
- US 5322938 A
- US 5641876 A
- US 20020192813 A1
- US 757089 A
- US 706946 A
- US 310 P
- US 370 A
- US 5593874 A
- US 5420034 A
- US 6433252 B
- US 6090627 A
- US 5188642 A
- US 5728925 A
- US 5094945 A
- US 5627061 A
- US 5633435 A
- US 6040497 A
- US 5463175 A
- US 20030083480 A1
- US 20030135879 A1
- US 4810648 A
- US 2003010609 A1
- US 6107549 A
- US 6376754 B
- US 20020112260 A
- US 5250515 A

- US 5880275 A
- US 6506599 A
- US 5986175 A
- US 20030150017 A1
- US 5015580 A
- US 5550318 A
- US 5538880 A
- US 5914451 A
- US 6160208 A
- US 6399861 B
- WO 6153812 A
- US 6365807 B
- US 5159135 A
- US 5824877 A
- US 5463174 A
- US 5591616 A
- WO 6384301 A
- US 7026528 B
- US 6329571 B
- US 4959317 A
- US 5527695 A
- US 6194636 B
- US 6232526 B
- US 5780708 A
- US 6118047 A
- US 6399861 A
- WO 0036911 A
- US 5846797 A

### Non-patent literature cited in the description

- **S.R. EDDY.** Profile Hidden Markov Models. *Bioinformatics,* 1998, vol. 14, 755-763
- **FISCHHOFF et al.** *Plant Mol Biol.,* 1992, vol. 20, 81-93
- **TANIGUCHI et al.** *Plant Cell Physiol.,* 2000, vol. 41 (1), 42-48
- **RUSSELL et al.** *Transgenic Res.,* 1997, vol. 6 (2), 157-166
- **BELANGER et al.** *Genetics,* 1991, vol. 129, 863-872

- **STACY et al.** *Plant Mol Biol.,* 1996, vol. 31 (6), 1205-1216
- **KLEE, H.J. et al.** *MGG,* 1987, vol. 210, 437-442
- **MISAWA et al.** *Plant J.,* 1993, vol. 4, 833-840
- **MISAWA et al.** *Plant J.,* 1994, vol. 6, 481-489
- **SATHASIIVAN et al.** *Nucl. Acids Res.,* 1990, vol. 18, 2188-2193
- **DEBLOCK et al.** *EMBO J.,* 1987, vol. 6, 2513-2519
- *Photosyn Research,* vol. 37, 89-102